# EUROPEAN PATENT APPLICATION

(11) **EP 4 170 347 A1**
(43) Date of publication of application: **26.04.2023**
(21) Application number: 21204592.6
(22) Date of filing: 25.10.2021
(51) Int. Cl.: G01N 33/543, C07K 7/06, C07K 7/08

(54) **GASTRIC INHIBITORY PEPTIDE RECEPTOR LIGANDS**

(71) Applicant: 3B Pharmaceuticals GmbH, 12489 Berlin (DE)
(72) Inventor: The designation of the inventor has not yet been filed
(74) Representative: Bohmann, Armin K.

(57) **Abstract**

The present invention is related to a compound comprising a cyclic peptide of formula (Ia) 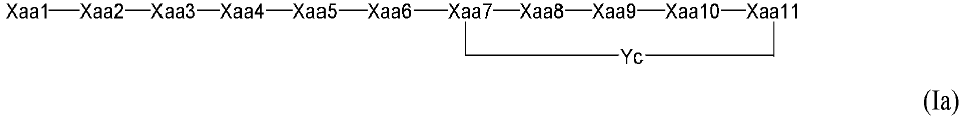 or a cyclic peptide of formula (Ib) 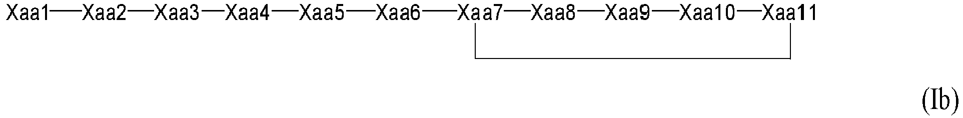 optionally an N-terminal modification group A attached to Xaa1, and optionally a C-terminal group C-term attached to Xaa11.

## Description

### FIELD OF THE INVENTION

The present invention is related to a chemical compound; a peptide; a Gastric Inhibitory Peptide Receptor (GIPR) binding compound; a Gastric Inhibitory Peptide Receptor (GIPR) binding peptide; a composition comprising the compound; a composition comprising the Gastric Inhibitory Peptide Receptor (GIPR) binding compound; a composition comprising the peptide; a composition comprising the Gastric Inhibitory Peptide Receptor (GIPR) peptide; the compound, the Gastric Inhibitory Peptide Receptor (GIPR) binding compound, the peptide, the Gastric Inhibitory Peptide Receptor (GIPR) peptide and the compositions, respectively, for use in a method for the diagnosis of a disease; the compound, the Gastric Inhibitory Peptide Receptor (GIPR) binding compound and the compositions, respectively, for use in a method for the treatment of a disease; the compound, the Gastric Inhibitory Peptide Receptor (GIPR) binding compound, the peptide, Gastric Inhibitory Peptide Receptor (GIPR) peptide and the compositions, respectively, for use in a method of diagnosis and treatment of a disease which is also referred to as "thera(g)nosis" or "thera(g)nostics"; the compound, the Gastric Inhibitory Peptide Receptor (GIPR) binding compound, the peptide, the Gastric Inhibitory Peptide Receptor (GIPR) binding peptide, and the compositions, respectively, for use in a method for delivering a radionuclide to a Gastric Inhibitory Peptide Receptor (GIPR) to a cell, preferably a GIPR overexpressing tumor cell or pancreatic beta cell; a method for the diagnosis of a disease using the compound, Gastric Inhibitory Peptide Receptor (GIPR) binding compound, the peptide, the Gastric Inhibitory Peptide Receptor (GIPR) binding peptide and the compositions, respectively; a method for the treatment of a disease using the compound, the Gastric Inhibitory Peptide Receptor (GIPR) binding compound, the peptide, the Gastric Inhibitory Peptide Receptor (GIPR) binding peptide and the compositions, respectively; a method for the diagnosis and treatment of a disease which is also referred to as "thera(g)nosis" or "thera(g)nostics", using the compound, the Gastric Inhibitory Peptide Receptor (GIPR) binding compound, the peptide, the binding peptide and the compositions, respectively; a method for the delivery of a radionuclide to a Gastric Inhibitory Peptide Receptor (GIPR) expressing tissue using the compound, the Gastric Inhibitory Peptide Receptor (GIPR) binding compound, the peptide, the Gastric Inhibitory Peptide Receptor (GIPR) binding peptide and the compositions, respectively.

### BACKGROUND

The human gastric inhibitory polypeptide (GIP) is a 42 amino acid incretin hormone (H-Tyr-Ala-Glu-Gly-Thr-Phe-Ile-Ser-Asp-Tyr-Ser-Ile-Ala-Met-Asp-Lys-Ile-His-Gln-Gln-Asn-Phe-Val-Asn-Trp-Leu-Leu-Ala-Gln-Lys-Gly-Lys-Lys-Asn-Asp-Trp-Lys-His-Asn-Ile-Thr-Gln-OH) secreted from the enteroendocrine K-cells, which forces diet-related insulin secretion via the GIPR signaling cascade (Cho et al., Vitam Horm, 2010, 84, 111-150). It was first discovered during cholecystokinin studies by John Brown and Raymond Pederson in the 1970s (Brown et al., Scand J Gastroenterol, 1970, 5, 537-541).

The GIPR is a 7-transmembrane spanning class B1 G-protein coupled receptor (GPCR) (Fredriksson et al., Mol Pharmacol, 2003, 63, 1256-1272), which is expressed in the gastrointestinal tract and the pancreatic islet cells (Usdin et al., Endocrinology, 1993, 133, 2861-2870). The extra-cellular N-terminal domain recognizes the ligand on its middle or C-terminal part, followed by the N-terminal part docking into the transmembrane domain of the receptor (Parthier et al., Proc Natl Acad Sci U S A, 2007, 104, 13942-13947). Receptor conformation induces transcription of the pro-insulin gene and insulin release by activating the adenylyl cyclase pathway that increases intracellular cyclic adenosine 3' 5'-monophasphate (cAMP) which is associated with elevated Ca²⁺ influx (Ding et al., Diabetes, 1997, 46, 615-621). Also, proliferative and anti-apoptotic effects were triggered by activation of the mitogen-activated protein kinase (MAPK) pathway and inhibition of caspase 3 (Khan et al., Peptides, 2020, 125, 170201). Both GIP(1-42)NH₂ and GIP(1-30)NH₂ are GIPR agonists and substrates for the dipeptidyl peptidase-4 (DPP4) resulting in potent GIPR inhibitors (GIP(3-42)NH₂; GIP(3-30) NH₂). While N-terminal truncations lead to decreased receptor activation and antagonism (Hansen et al., Br J Pharmacol, 2016, 173, 826-838), C-terminal modifications have only minor impact on stability or tissue distribution.

Recently, the GIPR came into the focus for peptide receptor radionuclide therapy (PRRT). In contrast to non-cancerous tissues, the GIPR is highly expressed in several subgroups of neuroendocrine neoplasms (Waser et al., J Clin Endocrinol Metab, 2012, 97, 482-488).

In 2012, a study showing a high GIP Receptor expression in gastroenteropancreatic and bronchial neuroendocrine tumors was published (Waser et al., J Clin Endocrinol Metab, 2012, 97, 482-488). The authors claimed GIPR represents a novel molecular target for clinical applications such as *in vivo* scintigraphy and targeted radiotherapy. WO 2012/168464 described a method for imaging and therapy of endocrine gastroenteropancreatic tumors and bronchial and thyroid neuroendocrine tumors by targeting GIPR. The receptor's incidence and density in human neuroendocrine tumors and non-neoplastic tissues was analyzed by autoradiography. Of these tumors, functional pancreatic neuroendocrine tumors, including insulinomas, gastrinomas, glucagonomas and vipomas, as well as non-functional pancreatic NETs and ileal NETs, presented highest receptor expression. In non-neoplastic tissues the highest expression was found in islets of the human pancreas. Academic groups published studies in which radiolabelled GIP analogs were used for nuclear medicine imaging applications (Gourni et al., J Nucl Med, 2014, 55, 976-982; Willekens et al., Sci Rep, 2018, 8, 2948). As proof of concept these studies have been successful, however, tumor uptake of the labeled peptides was around 20x lower when compared to kidney uptake.

### DETAILED DESCRIPTION OF THE INVENTION

The problem underlying the present invention is the provision of a compound which is suitable as a diagnostic agent and/or a therapeutic agent, particularly if conjugated to a diagnostically and/or therapeutically active radionuclide.

A further problem underlying the present invention is the provision of a compound which is suitable as a diagnostic agent and/or a therapeutic agent, particularly if conjugated to a diagnostically and/or therapeutically active radionuclide, having a pEC₅₀ of equal to or greater than 8.0 and/or a pIC₅₀ of equal to or greater than 7.5 for Gastric Inhibitory Peptide Receptor (GIPR).

A further problem underlying the present invention is the provision of a compound which is suitable as a diagnostic agent and/or a therapeutic agent, particularly if conjugated to a diagnostically and/or therapeutically active radionuclide, in the diagnosis and/or therapy of a disease where the diseased cells and/or diseased tissues express Gastric Inhibitory Peptide Receptor (GIPR). A still further problem underlying the instant invention is the provision of a compound which is suitable for delivering a diagnostically and/or therapeutically effective radionuclide to a diseased cell and/or diseased tissue, respectively, and more particularly a GIPR-expressing diseased cell and/or diseased tissue, preferably the diseased tissue comprises cancer or tumor cells.

Also, a problem underlying the present invention is the provision of a method for the diagnosis of a disease, of a method for the treatment and/or prevention of a disease, and a method for the combined diagnosis and treatment of a disease; preferably such disease is a disease involving GIPR-expressing cells and/or tissues, more particularly a GIPR-expressing diseased cell and/or diseased tissue, preferably the diseased tissue comprises or contains cancer or tumor cells or pancreatic beta cells.

A still further problem underlying the present invention is the provision of a method for the identification of a subject, wherein the subject is likely to respond or likely not to respond to a treatment of a disease, a method for the selection of a subject from a group of subjects, wherein the subject is likely to respond or likely not to respond to a treatment of a disease; preferably, the disease is cancer, more preferably the disease is a neuroendocrine tumor.

Also, a problem underlying the present invention is the provision of a pharmaceutical composition containing a compound having the characteristics as outlined above. Furthermore, a problem underlying the present invention is the provision of a kit which is suitable for use in any of the above methods.

These and other problems are solved by the subject matter of the attached independent claims; preferred embodiments may be taken from the attached dependent claims.

These and other problems are also solved by the subject matter of the following Embodiments.

Embodiment 1. A compound comprising a cyclic peptide of formula (Ia) 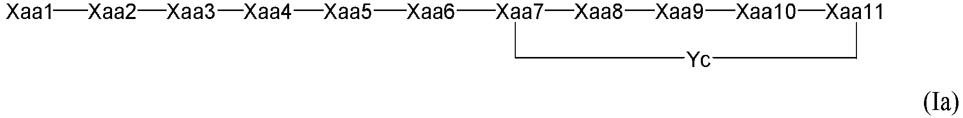 or a cyclic peptide of formula (Ib) 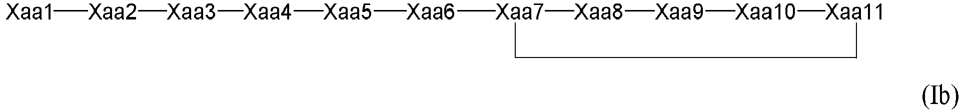
optionally an N-terminal modification group A attached to Xaa1, and
optionally a C-terminal group C-term attached to Xaa11,
wherein
   the peptide sequence is drawn from left to right in N- to C-terminal direction,
   Xaa1 is a residue of an α-amino acid with an acidic or polar group within the side chain or an aliphatic carboxylic acid with an additional acidic or polar group at the ω-position, preferably Xaa1 is a residue of an amino acid of formula (IIa), (IIb) or (IIc), more preferably Xaa1 is a residue of an amino acid formula (IIa), 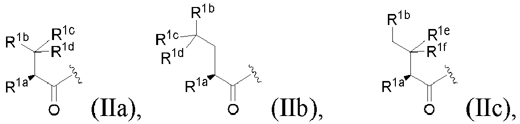 wherein
      - R^{1a}: is selected from the group consisting of NH, H, NH(C1-C4)alkyl, OH, and (C1-C4)alkyl, wherein if R^{1a} is selected from the group consisting of NH and NH(C1-C4)alkyl, the N-terminal modification group is covalently attached to the nitrogen atom of R^{1a}, and if R^{1a} is selected from the group consisting of H, OH or (C1-C4)alkyl the N-terminal modification group is absent,
      - R^{1b}: is selected from the group consisting of CO₂H, SO₃H, OPO₃H₂, CONH₂ and OH,
      - R^{1c}: is selected from the group consisting of H, OH and (C1-C2)alkyl under the proviso that R^{1b} is selected from the group consisting of CO₂H, SO₃H and CONH₂, or is selected from the group consisting of H and (C1-C2)alkyl under the proviso that R^{1b} is selected from the group consisting of OH and OPO₃H₂,
      - R^{1d}: is selected from the group consisting of H and (C1-C2)alkyl; preferably if R^{1c} is (C1-C2)alkyl, R^{1d} is the same (C1-C2)alkyl,
      - R^{1e}: is selected from the group consisting of H, OH and (C1-C2)alkyl,
      - R^{1f}: is selected from the group consisting of H and (C1-C2)alkyl; preferably if R^{1e} is (C1-C2)alkyl, R^{1f} is the same (C1-C2)alkyl,
   the carbonyl group of Xaa1 is covalently attached to the α-nitrogen atom of Xaa2,
   Xaa2 is a residue of an α-amino acid with a substituted or unsubstituted bicyclic aromatic or heteroaromatic side chain, preferably Xaa2 is a residue of an amino acid of formula (IIIa) or (IIIb), more preferably a residue of an amino acid of formula (IIIa), 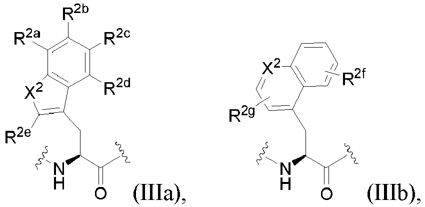 wherein
      - X²: is selected from the group consisting of NH, NCH₃ and S; preferably selected from the group consisting of NH and S, if Xaa2 is an amino acid of formula (IIIa), or is selected from the group consisting of N, CH, CF and C-CH₃; preferably selected from the group consisting of N and CH, if Xaa2 is an amino acid of formula (IIIb),
      - R^{2a}: is selected from the group consisting of H, F, Cl, Br, CH₃ and OCH₃, preferably R^{2a} is selected from the group consisting of H and F,
      - R^{2b}: is selected from the group consisting of H, a halogen, CH₃ and OCH₃, preferably the halogen is selected from the group consisting of Cl and Br, more preferably R^{2b} is selected from the group consisting of H, Cl and Br,
      - R^{2c}: is selected from the group consisting of H, OH, a halogen, CH₃ and OCH₃, preferably the halogen is selected from the group consisting of F, Cl and Br, more preferably R^{2c} is selected from the group consisting of H, F and OH,
      - R^{2d}: is selected from the group consisting of H, a halogen, CH₃ and OCH₃, wherein the halogen is selected from the group consisting of F, Cl and Br, preferably R^{2d} is H,
      - R^{2e}: is selected from the group consisting of H, a halogen and CH₃, preferably the halogen is Cl, more preferably R^{2e} is H,
      - R^{2f}: is selected from the group consisting of H and OCH₃, preferably R^{2f} is H,
      - R^{2g}: is selected from the group consisting of H, OH, CH₃ and OCH₃, preferably R^{2g} is H,
      or
   Xaa2 is a residue of an α-amino acid with a substituted or unsubstituted aromatic or heteroaromatic side chain, wherein Xaa2 is preferably a residue of an amino acid of formula (IIIc) 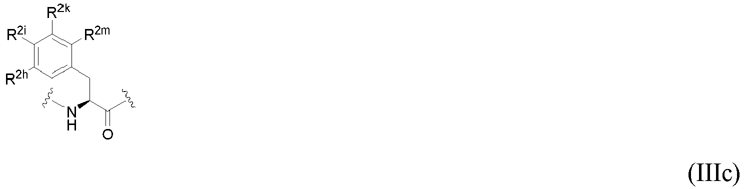 wherein
      - R^{2h}: is selected from the group consisting of H, CH₃, CF₃, OH, OCH₃, OCF₃, CONH₂ and a halogen, preferably the halogen is selected from the group consisting of Cl and Br, more preferably R^{2h} is H, Cl or Br,
      - R²¹: is selected from the group consisting of H, CH₃, C(CH₃)₃, CF₃, OH, OCH₃, OCH₂CH₃, OCF₃, CONH₂, CO₂H and a halogen, preferably the halogen is selected from the group consisting of Cl and Br, more preferably R²ⁱ is selected from the group consisting of H, Cl and Br,
      - R^{2k}: is selected from the group consisting of H, CH₃, CF₃, OH, OCH₃, OCF₃, CONH₂ and a halogen, preferably the halogen is selected from the group consisting of Cl and Br, more preferably R^{2k} is selected from the group consisting of H, Cl and Br,
      - R^{2m}: is selected from the group consisting of H, CH₃, CF₃, OH, OCH₃, OCF₃, CONH₂ and a halogen, preferably the halogen is selected from the group consisting of Cl and Br, more preferably R^{2m} is selected from the group consisting of H, Cl and Br,
   the carbonyl group of Xaa2 is covalently attached to the α-nitrogen atom of Xaa3,
   Xaa3 is an α-amino acid with a substituted or unsubstituted bicyclic aromatic or heteroaromatic side chain, preferably Xaa3 is a residue of an amino acid of formula (IVa) or (IVb), more preferably Xaa3 is a residue of an amino acid of formula (IVa), 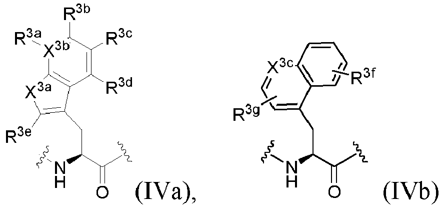 wherein
      - X^{3a}: is selected from the group consisting of NH, NCH₃ and S, preferably selected from the group consisting of NH and S,
      - X^{3b}: is selected from the group consisting of C and N, preferably X^{3b} is C,
      - X^{3c}: is selected from the group consisting of C and N, preferably X^{3c} is C,
      - R^{3a}: is, under the proviso that X^{3b} is C, selected from the group consisting of H, a halogen, CH₃ and OCH₃, preferably the halogen is F, or is absent if X^{3b} is N,
      - R^{3b}: is selected from the group consisting of H, a halogen, CH₃ and OCH₃, preferably the halogen is selected from the group consisting of F and Cl, more preferably R^{3b} is selected from the group consisting of H and F,
      - R^{3c}: is selected from the group consisting of H, a halogen, CH₃ and OCH₃, preferably the halogen is selected from the group consisting of Cl, Br and F, more preferably R^{3c} is selected from the group consisting of Cl and Br,
      - R^{3d}: is selected from the group consisting of H, a halogen, CH₃ and OCH₃, preferably the halogen is F,
      - R^{3e}: is selected from the group consisting of H, a halogen and CH₃, preferably the halogen is Cl,
      - R^{3f}: is selected from the group consisting of H and OCH₃,
      - R^{3g}: is selected from the group consisting of H, F, OH, CH₃ and OCH₃,
      or
   Xaa3 is a residue of an α-amino acid with a substituted or unsubstituted aromatic or heteroaromatic side chain, preferably Xaa3 is a residue of an amino acid of formula (IVc) 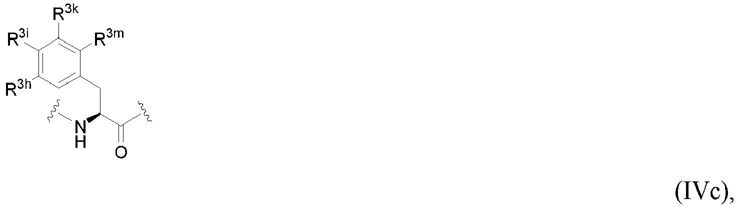 wherein
      - R^{3h}: is selected from the group consisting of H, a halogen, OH, CH₃, CF₃, OCH₃, OCF₃ and CONH₂, preferably the halogen is selected from the group consisting of Cl and Br,
      - R³ⁱ: is selected from the group consisting of H, a halogen, OH, CH₃, CF₃, OCH₃, OCF₃ and CONH₂, preferably the halogen is selected from the group consisting of Cl and Br,
      - R^{3k}: is selected from the group consisting of H, a halogen, OH, CH₃, CF₃, OCH₃, OCF₃ and CONH₂, preferably the halogen is selected from the group consisting of Cl and Br, and
      - R^{3m}: is selected from the group consisting of H, a halogen, OH, CH₃, CF₃, OCH₃, OCF₃ and CONH₂, preferably the halogen is selected from the group consisting of Cl and Br, preferably R^{3m} is selected from the group consisting of H and Cl,
   the carbonyl group of Xaa3 is covalently attached to the α-nitrogen atom of Xaa4,
   Xaa4 is a residue of a cyclic non-aromatic amino acid, preferably Xaa4 is a residue of an amino acid of formula (Va) 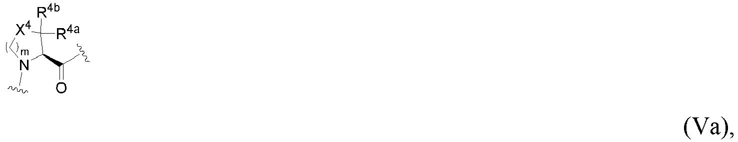 wherein
      - R^{4a}: is selected from the group consisting of H, OH, CH₃ and CF₃
      - R^{4b}: is selected from the group consisting of H and CH₃, preferably R^{4b} is CH₃ if R^{4a} is CH₃,
      - X⁴: is selected from the group consisting of CHR^{4c}, CH₂, S and O, wherein R^{4c} is absent or is selected from the group consisting of NH₂, OH, H, NHR^{4d}, CH₃ and F, wherein R^{4d} is absent or is selected from the group consisting of H, Ac, a Z group, wherein the Z group comprises a bio-distribution modifier with an optional linker, a chelator with an optional linker or a combination thereof, preferably the chelator is DOTA, the bio-distribution modifier is AGLU comprising the linker Glutar
      - m: is 1 or 2, preferably m is 1
      or
   Xaa4 is a residue of an N-alkylated amino acid, preferably Xaa4 is a residue of an amino acid of formula (Vb) 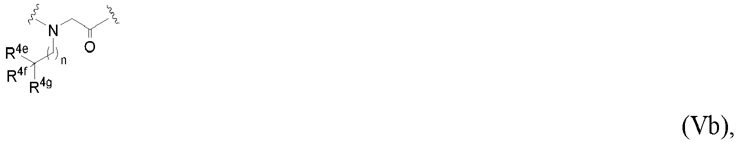 wherein
      - n: is 0, 1, 2, 3, 4, or 5, preferably n is 0, 1 or 3,
      - R^{4e}: is, if n is 0, selected from the group consisting of H, CH₃, a substituted or unsubstituted aromatic residue, COOH, CONH₂ and C(=O)R^{4h}, or is, if n is 1, 2, 3, 4 or 5, selected from the group consisting of H, CH₃, a substituted or unsubstituted aromatic residue, OH, NH₂, COOH, CONH₂ and C(=O)R^{4h},
      - R^{4f}: is selected from the group consisting of H, CH₃ and CH₂CH₃,
      - R^{4g}: is selected from the group consisting of H and CH₃,
      - R^{4h}: is AGLU,
      or
   Xaa4 is a residue of a bicyclic non-aromatic amino acid, preferably Xaa4 is a residue of an amino acid of formula (Vc) 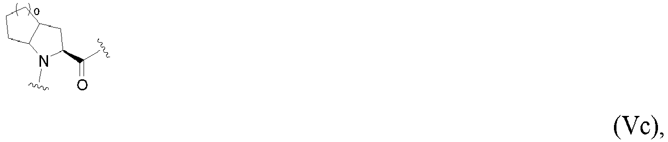 wherein
      o is 1 or 2,
   the carbonyl group of Xaa4 is covalently attached to the α-nitrogen atom of Xaa5,
   Xaa5 a residue of an α-amino acid with a polar, preferably an acidic group within the side chain, preferably Xaa5 is a residue of an amino acid of formula (VIa), (VIb) or (VIc), more preferably Xaa5 is a residue of an amino acid formula (VIa) or (VIb), 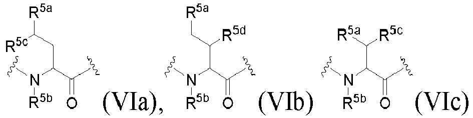 wherein
      - R^{5a}: is selected from the group consisting of COOH, CONH₂, OPO₃H₂, OH and SO₃H,
      - R^{5b}: is selected from the group consisting of H and (C1-C4)alkyl,
      - R^{5c}: is selected from the group consisting of H, OH and CH₃ under the proviso that R^{5a} is selected from the group consisting of COOH, CONH₂, OPO₃H₂ and SO₃H, or is selected from the group consisting of H and CH₃ under the proviso that R^{5a} is selected from the group consisting of COOH, CONH₂, OPO₃H₂, OH and SO₃H,
      - R^{5d}: is selected from the group consisting of H, OH and CH₃
   the carbonyl group of Xaa5 is covalently attached to the α-nitrogen atom of Xaa6,
   Xaa6 is a residue of an α-amino acid with a branched aliphatic side chain, preferably
   Xaa6 is a residue of an amino acid of formula (VIIa) 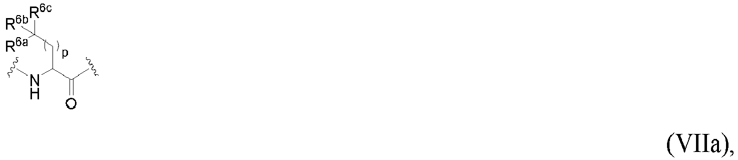 wherein
      - p: is 1, 0 or 2, preferably p is 1,
      - R^{6a}: is selected from the group consisting of CH₃ and CH₂CH₃,
      - R^{6b}: is CH₃,
      - R^{6c}: is H, if p is 0, and is selected from the group consisting of H and CH₃, if p is 1 or 2,
      the carbonyl group of Xaa6 is covalently attached to the α-nitrogen atom of Xaa7,
   Xaa7 is a residue of an amino acid of formula (VIIIa) 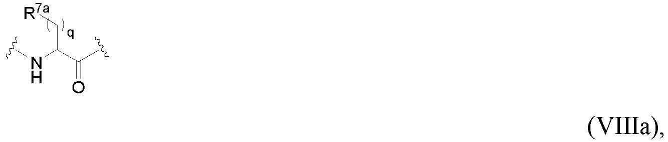 wherein
      - R^{7a}: is selected from the group consisting of SH and NH₂,
      - q: is 1 or 2, preferably q is 1,
   Xaa8 is a residue of an aliphatic cyclic α-amino acid, of an aliphatic heterocyclic α-amino acid or of an aliphatic cyclic α-amino acid with an annulated aromatic ring, preferably Xaa8 is a residue of an amino acid of formula (IXa) or (IXb), more preferably Xaa8 is a residue of an amino acid of formula (IXa), 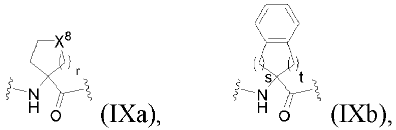 wherein
      - X⁸: is selected from the group consisting of NR^{8a}, O, NH, and CH₂, wherein R^{8a} is a Z group, wherein the Z group comprises a bio-distribution modifier with an optional linker, a chelator with an optional linker or a combination thereof, preferably the bio-distribution modifier is AGLU and the linker is Glutar, the chelator is DOTA, or if AGLU and DOTA are combined the linker is glu, or R^{8a} is acetyl,
      - r: is 2 or 1, preferably r is 2,
      - s: is 1 or 2, preferably s is 1,
      - t: is 1 or 2, preferably t is 1 if s is 1, and t is 2 if s is 2,
      or
   Xaa8 is a residue of an α-amino acid or an α-alkyl-α-amino acid, wherein preferably
   Xaa8 is a residue of an amino acid of formula (IXc), 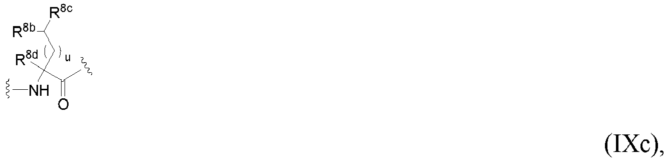 wherein
      - R^{8b}: is selected from the group consisting of H, OH, NH₂, NHR^{8e}, CH₃, CH₂CH₃, COOH, CONH₂, a substituted aromatic ring, an unsubstituted aromatic ring, a substituted bicyclic aromatic ring system, a substituted bicyclic hetero aromatic ring system, an unsubstituted bicyclic aromatic ring system and an unsubstituted bicyclic hetero aromatic ring system, preferably the aromatic ring is phenyl and the bicycylic heteroaromatic ring system is 3-indol, wherein R^{8e} is a Z group, wherein the Z group comprises a bio-distribution modifier with an optional linker, a chelator with an optional linker or a combination thereof,
      - R^{8c}: is selected from the group consisting of H and CH₃, if R^{8b} is selected from the group consisting of H, OH, NH₂, CH₃, CH₂CH₃, COOH, CONH₂, a substituted aromatic ring, an unsubstituted aromatic ring, a substituted bicyclic aromatic system, a substituted bicyclic hetero aromatic ring system, an unsubstituted bicyclic aromatic ring system and an unsubstituted bicyclic hetero aromatic ring system, or is selected from the group consisting of H, CH₃ and OH, if R^{8b} is selected from the group consisting of H, CH₃, CH₂CH₃, COOH, CONH₂, a substituted aromatic ring, an unsubstituted aromatic ring, a substituted bicyclic aromatic ring system, a substituted bicyclic hetero aromatic ring system, an unsubstituted bicyclic aromatic ring system and an unsubstituted bicyclic hetero aromatic ring system,
      - R^{8d}: is selected from the group consisting of H, CH₃ and CH₂CH₃,
      - u: is 0, 1, 2, 3, 4, or 5, preferably u is 0, 1 or 3,
      the carbonyl group of Xaa8 is covalently attached to the α-nitrogen atom of Xaa9,
   Xaa9 is a residue of an α-amino acid, preferably Xaa9 is a residue of an amino acid of formula (Xa) 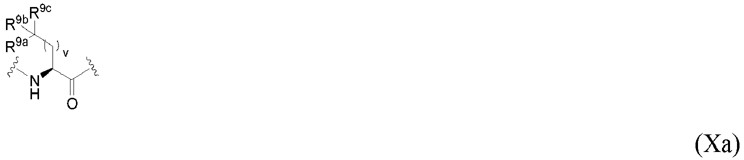 wherein
      - R^{9a}: is selected from the group consisting of CH₃, H, CH₂CH₃, OH, NH₂, NHC(=NH)NH₂, cyclo-hexyl, a substituted aromatic ring, an unsubstituted aromatic ring, a substituted bicyclic aromatic ring system, a substituted bicyclic hetero aromatic ring system, an unsubstituted bicyclic aromatic ring system and an unsubstituted bicyclic hetero aromatic ring system, preferably the aromatic ring is phenyl and the heteroaromatic ring system is 3-indol,
      - R^{9b}: is selected from the group consisting of CH₃ and H,
      - R^{9c}: is selected from the group consisting of H and CH₃,
      - v: is 0, 1 or 2, preferably v is 1,
   the carbonyl group of Xaa9 is covalently attached to the α-nitrogen atom of Xaa10,
   Xaa10 is a residue of an α-amino acid, preferably Xaa10 is a residue of an amino acid of formula (XIa) 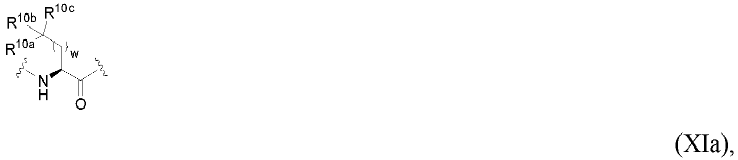 wherein
      - R^{10a}: is selected from the group consisting of CH₂CH₃, CH₃, phenyl, cyclohexyl and OH,
      - R^{10b}: is selected from the group consisting of CH₃ and H,
      - R^{10c}: is selected from the group consisting of H and CH₃,
      - w: is 0 or 1,
      the carbonyl group of Xaa10 is covalently attached to the nitrogen of Xaa11,
   Xaa11 is a residue of an α-amino acid with a nucleophilic group within the side chain or a residue of an aliphatic bis-nucleophile, preferably Xaa11 is a residue of an amino acid of formula (XIIa) 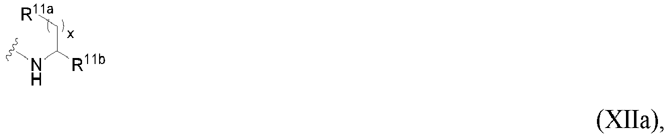 wherein
      - R^{11a}: is selected from the group consisting of SH and NH₂,
      - R^{11b}: is selected from the group consisting of C(=O)R, CONH₂, CO₂H, CH₂OH and H, wherein R is C-term, NH₂ or OH,
      - x: is 1 or 2,
      or
   Xaa11 is a residue of an amino acid of the formula (XIIb) 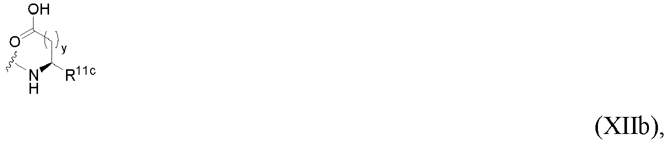 wherein
      - R^{11c}: is selected from the group consisting of H and CH₂CH₂CONH₂,
      - y: is 0 or 1
      wherein
   if R^{1a} in Xaa1 is selected from the group consisting of NH and NH(C1-C4)alkyl, the N-terminal modification group A
      (a) is a Z group or a bio-distribution modifier optionally comprising a linker, wherein the Z group comprises a chelator and optionally a linker,
      (b) is a residue of an amino acid Aaa, wherein an optional linker is interspersed between Aaa and Xaa1,
         wherein the amino acid Aaa is optionally substituted by a Z group, and wherein said Z group comprises a chelator and optionally a linker,
         wherein if Aaa is glu, Aaa is optionally substituted by a bio-distribution modifier,
         or
      (c) is selected from the group consisting of acetyl, a blocking group Abl of a structure of formula (Abl-Ia) or of a structure of formula (Abl-Ib) 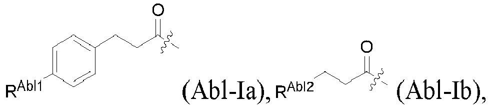 wherein
         - R^{Ab11}: is selected from the group consisting of H, OH and a halogen, preferably the halogen is selected from the group consisting of F and Cl, preferably R^{Ab11} is OH
         - R^{Ab12}: is selected from the group consisting of 1-naphtyl and 2-naphthyl,
         preferably Abl is of formula (Abl-Ia),
   and wherein, if present, the N-terminal modification group A is bound to the α-N atom of R^{1a} in Xaa1,
   wherein
   if Xaa11 is a residue of an amino acid of formula (XIIa), R^{11b} is C(=O)R, and R is C-term,
      the C-terminal group C-term
      (a) is one of more amino acids AA,
         wherein at least one or more of the one or more amino acids AA is optionally substituted by a Z group and/or a bio-distribution modifier optionally comprising a linker, wherein said Z group comprises a chelator and optionally a linker,
         wherein an optional linker is interspersed between Xaa11 and the first of the one or more amino acids AA,
         wherein an optional linker is interspersed between any of the more than one amino acids AA,
         wherein to the last of the one or more amino acids a blocking group Abl is attached, wherein Abl is selected from the group consisting of NH₂ and OH, wherein Abl preferably is NH₂, wherein if AA is glu, AA is optionally substituted by a bio-distribution modifier,
         wherein if AA is Lys, AA is optionally substituted by a chelator,
      (b) is a Z group,
         wherein the Z group comprises a chelator and a linker,
      (c) is a bio-distribution modifier optionally comprising a linker,
         or
      (d) is a blocking group Abl, wherein
         Abl is OH or NH₂,
         preferably Abl is NH₂,
      and wherein, if present, the C-terminal group C-term is bound to the C-atom of R^{11b} in Xaa11,
   wherein
      (a) if the cyclic peptide is of formula (Ia),
         under the proviso that Xaa11 is a residue of formula (XIIa), Yc is a structure of formula (XIIIa) or a structure of formula (XIIIb), more preferably Yc is a structure of formula (XIIIa) 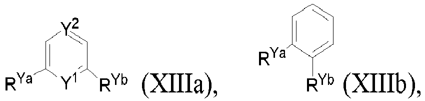 wherein
         - R^{Ya} and R^{Yb}: are each and independently -CH₂-,
         - Y¹: is selected from the group consisting of N and CH,
         - Y²: is selected from the group consisting of N and C-R^{Yc},
         - R^{Yc}: is selected from the group consisting of H and -CH₂-R^{Yd} and
         - R^{Yd}: is a structure of formula (XIIIc), (XIIId) or (XIIIe), 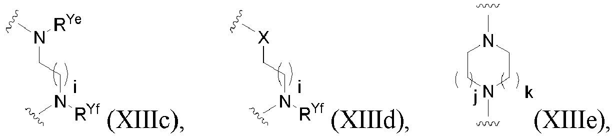
         wherein
         - R^{Ye} and R^{Yf}: are each and independently selected from the group consisting of H and (C1-C4)alkyl
         - i: is 1, 2, 3, 4, 5 or 6, preferably i is 1 or 2,
         - j and k: are each and independently 1, 2 or 3, and
         - X: is O or S, preferably X is S,
         wherein
         in formulae (XIIIc) and (XIIIe)
            one of the two nitrogen atoms is attached to -CH₂- of R^{Yc} and in formula (XIIId)
            -X- is attached to -CH₂- of R^{Yc}
         while the remaining nitrogen atom optionally connects to a Z group comprising a chelator and optionally a linker,
         preferably, if Yc is a structure of formulae (XIIIa), wherein R^{Yc} is -CH₂-R^{Yd}, R^{Yd} is a structure of formulae (XIIId), wherein X is S and R^{Yf} is H,
         and wherein
         one of R^{Ya} and R^{Yb}
            is linked to the S-atom of Xaa7 under the formation of a thioether, if R^{7a} in Xaa7 is SH, or,
            is linked to the N-atom of Xaa7 under the formation of an amine linkage, if R^{7a} in Xaa7 is NH₂,
         and the other one of R^{Ya} and R^{Yb}
            is linked to the S-atom of Xaa11 under the formation of a thioether if R^{11a} in Xaa11 is SH, or,
            is linked to the N-atom of Xaa11 under the formation of an amine linkage if R^{11a} in Xaa11 is NH₂,
         and the resulting generic structural formula corresponds to formula (Ic) 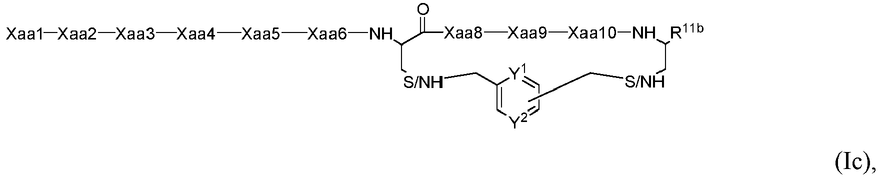 or wherein
      (b) if the cyclic peptide is of formula (Ia),
         under the proviso that
            R^{7a} in Xaa7 is NH₂ and Xaa11 is a residue of formula (XIIa) wherein R^{11a} is SH or NH₂,
            or
               Xaa11 is a residue of formula (XIIa) wherein R^{11a} is NH₂ and R^{7a} in Xaa7 is NH₂ or SH,
         Yc is a structure of formula (XIIIf), 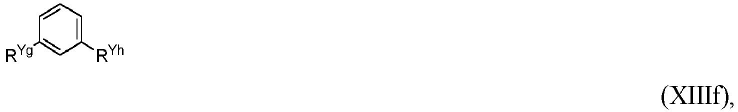 wherein
            - R^{Yg}: is -C(=O)- and
            - R^{Yh}: is -CH₂-,
         and wherein
            if R^{7a} in Xaa7 is NH₂ and Xaa11 is a residue of formula (XIIa) wherein R^{11a} is SH or NH₂,
            R^{Yg} is linked to the N-atom of R^{7a} in Xaa7 under the formation of an amide linkage
            and
            if R^{11a} in Xaa11 is SH, R^{Yh} is linked to said S-atom under the formation of a thioether linkage,
            or
            if R^{11a} in Xaa11 is NH₂, R^{Yh} is linked to said N-atom under the formation of an amine linkage
         or wherein
            if Xaa11 is a residue of formula (XIIa), wherein R^{11a} is NH₂ and R^{7a} in Xaa7 is either SH or NH₂,
            R^{Yg} is linked to the N-atom of R^{11a} in Xaa11 under the formation of an amide linkage
            and
            if R^{7a} in Xaa7 is SH, R^{Yh} is linked to said S-atom under the formation of a thioether linkage,
            or
            if R^{7a} in Xaa7 is NH₂, R^{Yh} is linked to said N-atom under the formation of an amine linkage,
            and the resulting generic structural formula corresponds to formula (Id) or formula (Ie) 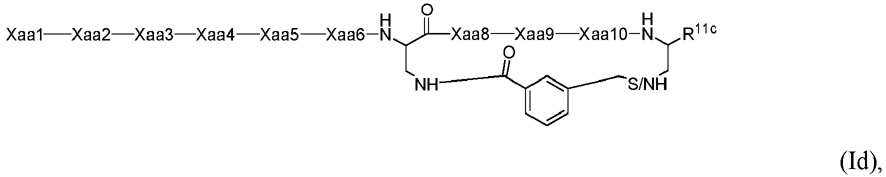 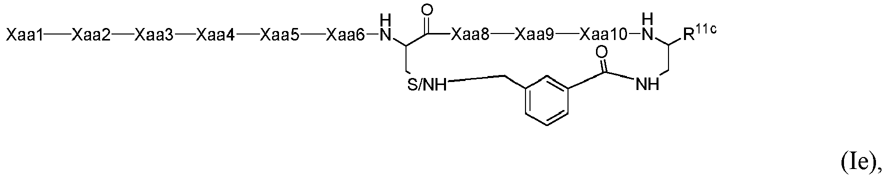
         or wherein
      (c) if the cyclic peptide is of formula (Ia),
         if R^{7a} in Xaa7 is SH and Xaa11 is a residue of formula (XIIa) wherein R^{11a} is SH,
         Yc is a structure of formula (XIIIg), 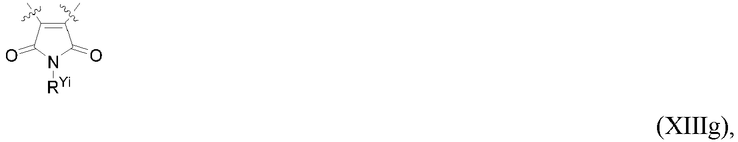 wherein
            R^{Yi} is selected from the group consisting of H and (C1-C6)alkyl and wherein the S-atom of Xaa7 is linked to Yc by a thioether linkage and the S-atom of Xaa11 is linked to Yc by a thioether linkage forming a cyclic structure with a generic formula corresponding to formula (If) 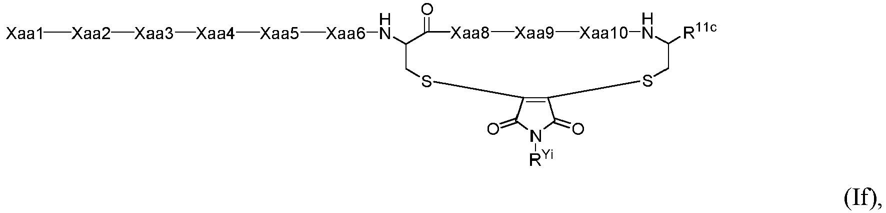
         or wherein
      (d) if the cyclic peptide is of formula (Ia),
         if R^{7a} in Xaa7 is NH₂ and Xaa11 is a residue of an amino acid of the formula (XIIb),
         Yc is a structure of formula (XIIIh), 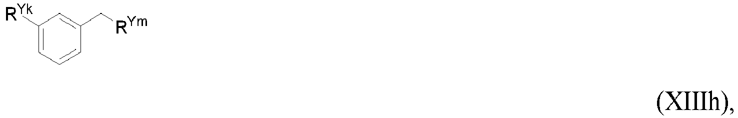 wherein,
            - R^{Yk}: is -C(=O)- and
            - R^{Ym}: is -NH-,
         wherein
            R^{Yk} is linked to the N-atom of R^{7a} in Xaa7 under the formation of an amide bond and R^{Ym} is linked to the carboxylic acid group of Xaa11 under the formation of an amide bond,
         and wherein
            the resulting generic structural formula corresponds to formula (Ig) 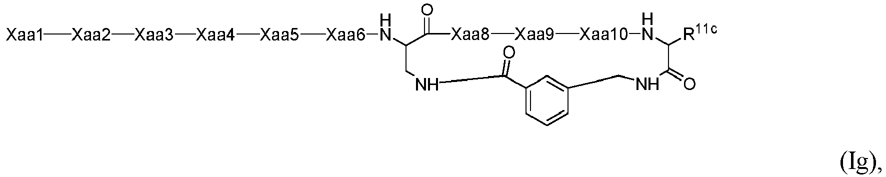
         and wherein
         if the cyclic peptide is of formula (Ib), the S atom of Xaa7 and the S atom of Xaa11 are covalently attached to each other forming a disulfide linkage.

Embodiment 2. The compound of Embodiment 1, wherein Xaa1 is a residue of an α-amino acid with an acidic or polar group within the side chain or an aliphatic carboxylic acid with an additional acidic or polar group at the ω-position, of formula (IId) or (IIe), more preferably Xaa1 is a residue of an amino acid formula (IId), 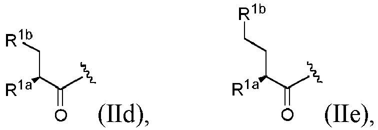 wherein
- R^{1a}: is selected from the group consisting of NH, H or NCH₃,
- R^{1b}: is selected from the group consisting of CO₂H, CONH₂, OH, SO₃H and OPO₃H₂,

under the proviso that R^{1a} is NH, the N-terminal modification group is covalently attached to the nitrogen atom of R^{1a},
the carbonyl group of Xaa1 is covalently attached to the α-nitrogen atom of Xaa2.

Embodiment 3. The compound of any one of Embodiments 1 to 2, wherein Xaa1 is a residue of an α-amino acid with an acidic or polar group within the side chain or an aliphatic carboxylic acid with an additional acidic or polar group at the ω-position, of formula (IId) or (IIe), more preferably Xaa1 is a residue of an amino acid formula (IId), 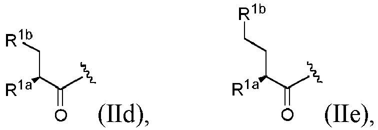 wherein
- R^{1a}: is NH or H,
- R^{1b}: is selected from the group consisting of CO₂H, CONH₂, OH and SO₃H,

under the proviso that R^{1a} is NH, the N-terminal modification group is covalently attached to the nitrogen atom of R^{1a},
the carbonyl group of Xaa1 is covalently attached to the α-nitrogen atom of Xaa2.

Embodiment 4. The compound of any one of Embodiments 1 to 3, wherein Xaa1 is a residue of an α-amino acid with an acidic or polar within the side chain or an aliphatic carboxylic acid with an additional acidic or polar group at the ω-position, of formula (IId) or (IIe), more preferably Xaa1 is a residue of an amino acid of formula (IId), 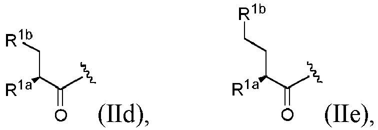 wherein
- R^{1a}: is NH,
- R^{1b}: is CO₂H or CONH₂,

the N-terminal modification group is covalently attached to the α-nitrogen atom of Xaa1,
the carbonyl group of Xaa1 is covalently attached to the α-nitrogen atom of Xaa2.

Embodiment 5. The compound of any one of Embodiment 1 to 2, wherein Xaa1 is an L-amino acid residue selected from the group consisting of Asp, Asn, Glu, Gln, Hse, Cya, Pse, Nmd and Ser, or a dicarboxylic acid residue selected from the group consisting of Succinyl and Glutar.

Embodiment 6. The compound of any one of Embodiments 1 to 3 and 5, preferably of Embodiment 5, wherein Xaa1 is an L-amino acid residue selected from the group consisting of Asp, Asn, Glu, Gln, Hse, Cya, and Ser, or a dicarboxylic acid residue selected from the group consisting of Succinyl and Glutar.

Embodiment 7. The compound of any one of Embodiments 1 to 6, preferably of any one of Embodiments 5 to 6, wherein Xaa1 is an L-amino acid residue selected from the group consisting of Asp, Asn, Glu, and Gln.

Embodiment 8. The compound of any one of Embodiments 1 to 7, of any one of Embodiments 5 to 7, wherein Xaa1 is an Asp residue.

Embodiment 9. The compound of any one of Embodiments 1 to 8, wherein Xaa2 is a residue of an amino acid of formula (IIId), 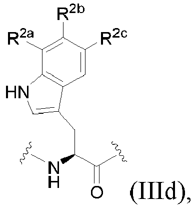 wherein
- R^{2a}: is H or F,
- R^{2b}: is H or Cl,
- R^{2c}: is selected from the group consisting of H, F and OH,
the carbonyl group of Xaa2 is covalently attached to the α-nitrogen atom of Xaa3.

Embodiment 10. The compound of any one of Embodiments 1 to 9, preferably of Embodiment 9,
wherein
- R^{2a}: is H or F,
- R^{2b}: is H,
- R^{2c}: is H or OH,
the carbonyl group of Xaa2 is covalently attached to the α-nitrogen atom of Xaa3.

Embodiment 11. The compound of any one of Embodiments 1 to 8, wherein Xaa2 is an L-amino acid residue selected from the group consisting of Trp, Hyw, 5Fw, 6Clw, 7Fw, Bta, 5Clw, 5Brw and 6Fw.

Embodiment 12. The compound of any one of Embodiments 1 to 8 and 11, preferably of Embodiment 11, wherein Xaa2 is an L-amino acid residue selected from the group consisting of Trp, Hyw, 5Fw, 6Clw, 7Fw, 5Clw, 5Brw and 6Fw.

Embodiment 13. The compound of any one of Embodiments 1 to 9 and preferably the compound of any one of Embodiments 11 to 12, wherein Xaa2 is an _{L}-amino acid residue selected from the group consisting of Trp, Hyw, 5Fw, 6Clw and 7Fw.

Embodiment 14. The compound of any one of Embodiments 1 to 13, preferably the compound of any one of Embodiments 9 to 13, wherein Xaa2 is a Trp residue.

Embodiment 15. The compound of any one of Embodiment 1s to 8, wherein Xaa2 is a 1Qi residue.

Embodiment 16. The compound of any one of Embodiments 1 to 8, wherein Xaa2 is a residue of an amino acid of formula (IIIc) 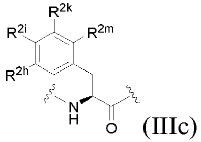 wherein
- R^{2h}: is H or Cl,
- R²ⁱ: is H or Cl,
- R^{2k}: is H or Cl, and
- R^{2m}: is H of Cl,
the carbonyl group of Xaa2 is covalently attached to the α-nitrogen atom of Xaa3.

Embodiment 17. The compound of any one of Embodiments 1 to 8 and 16, preferably the compound of Embodiment 16, wherein Xaa2 is an L-amino acid residue selected from the group consisting of Phe, Ocf, Mcf, Pcf, Eaa, Egm, Egn and Egp.

Embodiment 18. The compound of any one of Embodiments 1 to 17, wherein Xaa3 is a residue of an amino acid of formula (IVd) or (IVe), 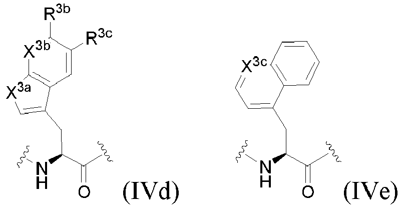 wherein
- R^{3b}: is H or F,
- R^{3c}: is selected from the group consisting of Cl, Br, H and F, and
- X^{3a}: is NH or S,
- X^{3b}: is selected from the group consisting of CH, CF and N,
- X^{3c}: is CH or N, preferably X^{3c} is CH,
the carbonyl group of Xaa3 is covalently attached to the α-nitrogen atom of Xaa4.

Embodiment 19. The compound of any one of Embodiments 1 to 18, preferably of Embodiment 18, wherein Xaa3 is a residue of an amino acid of formula (IVf) 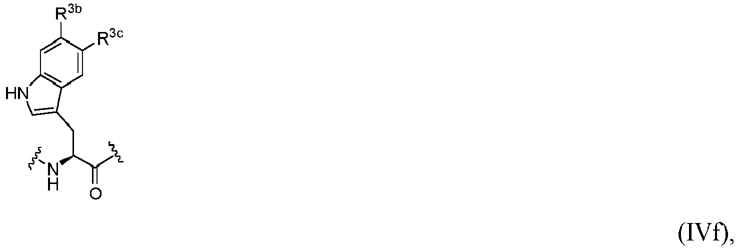 wherein
- R^{3b}: is H or F,
- R^{3e}: is selected from the group consisting of Cl, Br, and H,
the carbonyl group of Xaa3 is covalently attached to the α-nitrogen atom of Xaa4.

Embodiment 20. The compound of any one of Embodiments 1 to 17, wherein Xaa3 is an L-amino acid residue selected from the group consisting of 5Clw, 5Brw, Trp, 1Ni, Bta, 5Fw, 6Fw, 7Fw, 6Clw, 7Nw and 1Qi.

Embodiment 21. The compound of any one of Embodiments 1 to 18 and 20, preferably the compound of any one of Embodiments 18 and 20, wherein Xaa3 is an _{L}-amino acid residue selected from the group consisting of 5Clw, 5Brw, Trp, 1Ni, Bta, 5Fw, 6Fw and 7Fw. Embodiment 22. The compound of any one of Embodiments 1 to 21, preferably the compound of any one of Embodiments 18 to 21, wherein Xaa3 is an L-amino acid residue selected from the group consisting of 5Clw and 5Brw.

Embodiment 23. The compound of any one of Embodiments 1 to 22, preferably the compound of any one of Embodiments 18 to 22, wherein Xaa3 is a 5Clw residue.

Embodiment 24. The compound of any one of Embodiments 1 to 17, wherein Xaa3 is a residue of an amino acid of formula (IVc) 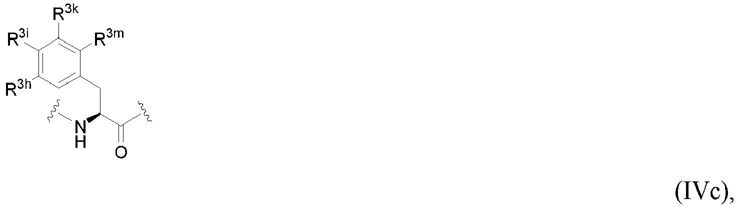 wherein
- R^{3h}: is H or Cl,
- R³ⁱ: is Cl or H,
- R^{3k}: is CI,
- R^{3m}: is H or Cl,
the carbonyl group of Xaa3 is covalently attached to the α-nitrogen atom of Xaa4.

Embodiment 25. The compound of any one of Embodiments 1 to 17 and 24, preferably the compound of Embodiment 24, wherein Xaa3 is a residue of an amino acid of formula (IVd) 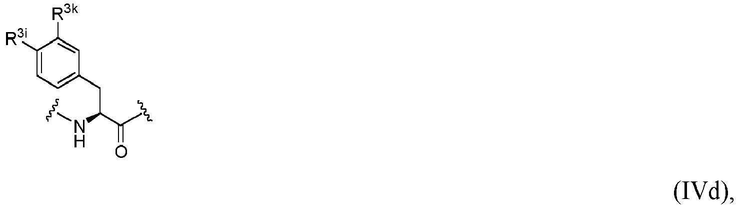 wherein
- R³ⁱ: is CI,
- R^{3k}: is Cl,
the carbonyl group of Xaa3 is covalently attached to the α-nitrogen atom of Xaa4.

Embodiment 26. The compound of any one of Embodiments 1 to 17 and 24, preferably the compound of Embodiment 24, wherein Xaa3 is an _{L}-amino acid residue selected from the group consisting of Eaa, Egn, Egp and Mcf.

Embodiment 27. The compound of any one of Embodiments 1 to 17, 24 and 26, preferably the compound of Embodiment 26, wherein Xaa3 is an _{L}-amino acid residue selected from the group consisting of Eaa, Egn and Egp.

Embodiment 28. The compound of any one of Embodiments 1 to 17 and 24 to 27, preferably the compound of any one of Embodiments 24 to 27, wherein Xaa3 is an Eaa residue.

Embodiment 29. The compound of any one of Embodiments 1 to 28, wherein Xaa4 is a residue of an amino acid of formula (Vd) 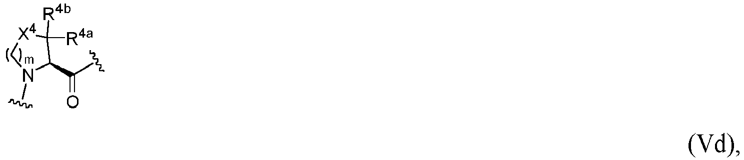 wherein
- m: is 1 or 2, preferably m is 1,
- R^{4a}: is selected from the group consisting of H, OH and CH₃,
- R^{4b}: is either H or CH₃, preferably R^{4b} is CH₃ if R^{4a} is CH₃,
- X⁴: is selected from the group consisting of CHR^{4c}, CH₂, S and O, wherein R^{4c} is selected from the group consisting of NHR^{4d}, NH₂, H, OH, CH₃ and F, wherein R^{4d} is H, Ac or R^{4d} is a chelator, optionally comprising a linker, preferably the chelator is DOTA or R^{4d} is a bio-distribution modifier with an optional linker, preferably the bio-distribution modifier is AGLU with Glutar as linker, the carbonyl group of Xaa4 is covalently attached to the α-nitrogen atom of Xaa5.

Embodiment 30. The compound of any one of Embodiments 1 to 29, preferably the compound of Embodiment 29, wherein Xaa4 is a residue of an amino acid of formula (Ve) 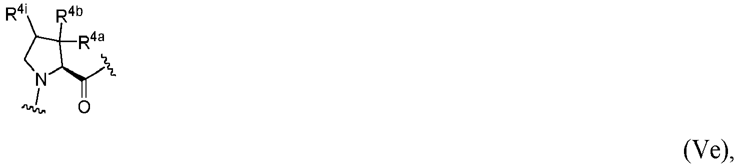 wherein
- R^{4a}: is selected from the group consisting of H, OH and CH₃,
- R^{4b}: is H or CH₃, preferably R^{4b} is CH₃ if R^{4a} is CH₃,
- R⁴ⁱ: is selected from the group consisting of NH₂, OH, NHR^{4d}, H and F, wherein R^{4d} is H or Ac, or
- R^{4d}: is a chelator, preferably the chelator is DOTA, optionally comprising a linker or R^{4d} is a bio-distribution modifier with an optional linker, preferably the bio-distribution modifier is AGLU with Glutar as linker,
the carbonyl group of Xaa4 is covalently attached to the α-nitrogen atom of Xaa5.

Embodiment 31. The compound of any one of Embodiments 1 to 30, preferably the compound of any one of Embodiments 29 and 30, wherein Xaa4 is a residue of an amino acid of formula (Vf) 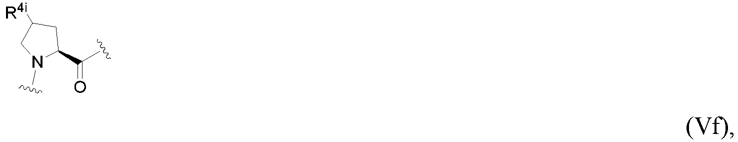 wherein
- R⁴ⁱ: is selected from the group consisting of NH₂, OH and H,
the carbonyl group of Xaa4 is covalently attached to the α-nitrogen atom of Xaa5.

Embodiment 32. The compound of any one of Embodiments 1 to 29, preferably the compound of Embodiment 29, wherein Xaa4 is an _{L}-amino acid residue selected from the group consisting of Tap, Hyp, Pro, 4Ap, 4Ap(Ac), Tap(DOTA), Tap(AGLU-Gutar), 4Tfp, H3p, Eaz, Oxa, Dtc and Pip.

Embodiment 33. The compound of any one of Embodiments 1 to 29 and 32, preferably the compound of any one of Embodiments 29 and 32, wherein Xaa4 is an L-amino acid residue selected from the group consisting of Tap, Hyp, Pro, 4Ap, 4Ap(Ac), Tap(DOTA), Tap(AGLU-Gutar), 4Tfp, H3p, Eaz, Oxa and Dtc.

Embodiment 34. The compound of any one of Embodiments 1 to 30 and 32 to 33, preferably the compound of any of one Embodiments 29 to 30 and 32 to 33, wherein Xaa4 is an _{L}-amino acid residue selected from the group consisting of Tap, Hyp, Pro, 4Ap, 4Ap(Ac), Tap(DOTA), Tap(AGLU-Gutar), 4Tfp and H3p.

Embodiment 35. The compound of any one of Embodiments 1 to 34, preferably the compound of any one of Embodiments 29 to 34, wherein Xaa4 is an _{L}-amino acid residue selected from the group consisting of Tap, Hyp, Pro and 4Ap.

Embodiment 36. The compound of any one of Embodiments 1 to 35, preferably the compound of any one of Embodiment 29 to 35, wherein Xaa4 is a Pro residue.

Embodiment 37. The compound of any one of Embodiments 1 to 35, preferably the compound of any one of Embodiments 29 to 35, wherein Xaa4 is a Hyp residue.

Embodiment 38. The compound of any one of Embodiments 1 to 35, preferably the compound of any one of Embodiment 29 to 35, wherein Xaa4 is a Tap residue.

Embodiment 39. The compound of any one of Embodiments 1 to 28, wherein Xaa4 is a residue of an N-alkylated amino acid of formula (Vg) 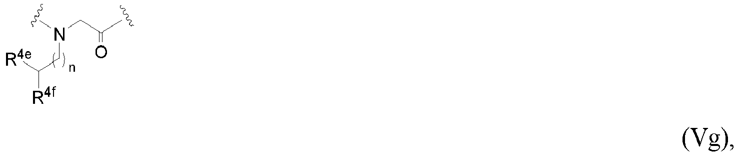 wherein
- n: is 0, 1 or 3,
- R^{4e}: is, if n = 0 is selected from the group consisting of H and phenyl, or is, if n = 3 is NH₂, or is, if n = 1 is selected from the group consisting of CH₃, CO₂H, C(=O)R^{4h}, wherein R^{4h} is a bio-distribution modifier preferably to bio-distribution modifier is AGLU, and
- R^{4f}: is selected from the group consisting of H and CH₃,
the carbonyl group of Xaa4 is covalently attached to the α-nitrogen atom of Xaa5.

Embodiment 40. The compound of any one of Embodiments 1 to 28 and 39, preferably the compound of Embodiment 39, wherein Xaa4 is an N-alkylated amino acid residue selected from the group consisting of Nmg, Nlys, Nleu, Nphe, Nglu and Nglu(AGLU).

Embodiment 41. The compound of any one of Embodiments 1 to 28, wherein Xaa4 is an Oic residue.

Embodiment 42. The compound of any one of Embodiments 1 to 41, wherein Xaa5 is a residue of an amino acid of formula (VId) or (VIe), preferably Xaa5 is a residue of an amino acid of formula (IVd), 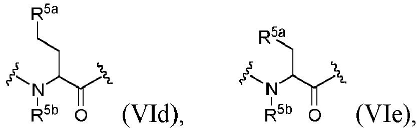 wherein
- R^{5a}: is selected from the group consisting of COOH, CONH₂, OPO₃H₂, OH and SO₃H,
- R^{5b}: is H or CH₃,
the carbonyl group of Xaa5 is covalently attached to the α-nitrogen atom of Xaa6.

Embodiment 43. The compound of any one of Embodiments 1 to 42, preferably the compound of Embodiment 42, wherein Xaa5 is a residue of an amino acid of formula (VIf) or (VIg), preferably Xaa5 is a residue of an amino acid of formula (VIf), 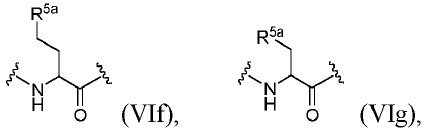 wherein
- R^{5a}: is COOH or CONH₂,
the carbonyl group of Xaa5 is covalently attached to the α-nitrogen atom of Xaa6.

Embodiment 44. The compound of any one of Embodiments 1 to 42, preferably the compound of Embodiment 42, wherein Xaa5 is an amino acid residue selected from the group consisting of Glu, Gln, glu, Nme, Asp, Asn, Pse, Hse and Hca.

Embodiment 45. The compound of any one of Embodiments 1 to 44, preferably the compound of any one of Embodiments 42 to 44, wherein Xaa5 is an amino acid residue selected from the group consisting of Glu, Gln, glu, Asp and Asn.

Embodiment 46. The compound of any one of Embodiments 1 to 45, preferably the compound of any one of Embodiments 42 to 45, wherein Xaa5 is a Glu residue.

Embodiment 47. The compound of any one of Embodiments 1 to 46, wherein Xaa6 is a residue of an amino acid of formula (VIIa) 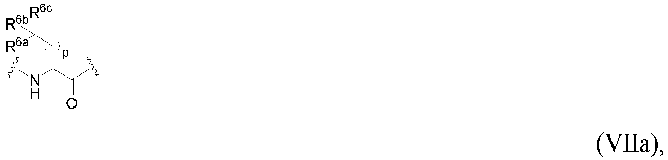 wherein
- p: is 1 or 0, preferably p is 1,
- R^{6a}: is CH₃ or CH₂CH₃,
- R^{6b}: is CH₃,
- R^{6c}: is H if p is 0, and is H or CH₃, if p is 1,
the carbonyl group of Xaa6 is covalently attached to the α-nitrogen atom of Xaa7.

Embodiment 48. The compound of any one of Embodiments 1 to 46, wherein Xaa6 is an amino acid residue selected from the group consisting of leu, Leu, Npg, Val, Ile and Hle.

Embodiment 49. The compound of any one of Embodiments 1 to 48, preferably the compound of any one of Embodiments 47 to 48, wherein Xaa6 is an amino acid residue selected from the group consisting of leu, Leu, Npg, Val and Ile.

Embodiment 50. The compound of any one of Embodiments 1 to 49, preferably the compound of any one of Embodiments 47 to 49, wherein Xaa6 is a leu residue.

Embodiment 51. The compound of any one of Embodiments 1 to 50, wherein Xaa7 is an L-amino acid residue selected from the group of Cys, cys, Hcy and Dap.

Embodiment 52. The compound of any one of Embodiments 1 to 51, preferably the compound of Embodiment 51, wherein Xaa7 is a Cys residue.

Embodiment 53. The compound of any one of Embodiments 1 to 52, wherein Xaa8 is a residue of an amino acid of formula (IXa), 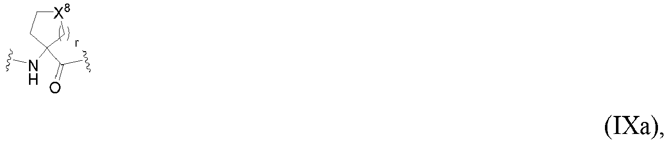 wherein
- r: is 2 or 1, preferably r is 2,
- X⁸: is selected from the group consisting of CH₂, O, NH and NR^{8a},
wherein R^{8a} is a Z group, wherein the Z group comprises a bio-distribution modifier with an optional linker, a chelator with an optional linker or a combination thereof, preferably the bio-distribution modifier is AGLU and the linker is Glutar, the chelator is DOTA, or if AGLU and DOTA are combined the linker is glu, or R^{8a} is acetyl,
the carbonyl group of Xaa8 is covalently attached to the α-nitrogen atom of Xaa9.

Embodiment 54. The compound of any one of Embodiments 1 to 53, preferably the compound of Embodiment 53, wherein Xaa8 is a residue of an amino acid of formula (IXd) 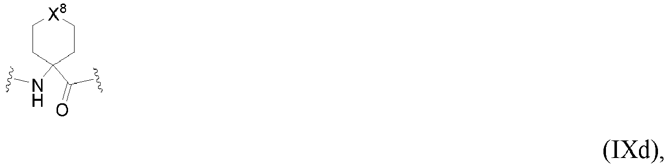 wherein
- X⁸: is selected from the group consisting of NR^{8a}, NH and O, wherein R^{8a} is a Z group, wherein the Z group comprises a bio-distribution modifier with an optional linker, a chelator with an optional linker or a combination thereof, preferably the bio-distribution modifier is AGLU and the linker is Glutar, the chelator is DOTA, or if AGLU and DOTA are combined the linker is glu, or R^{8a} is acetyl,
the carbonyl group of Xaa8 is covalently attached to the α-nitrogen atom of Xaa9.

Embodiment 55. The compound of any one of Embodiments 1 to 54, preferably the compound of any one of Embodiments 53 to 54, wherein
- X⁸: is NR^{8a} or O, wherein R^{8a} is a Z group, wherein the Z group comprises a bio-distribution modifier with an optional linker, a chelator with an optional linker or a combination thereof, preferably the bio-distribution modifier is AGLU and the linker is Glutar, the chelator is DOTA, or if AGLU and DOTA are combined the linker is glu, or R^{8a} is acetyl,
the carbonyl group of Xaa8 is covalently attached to the α-nitrogen atom of Xaa9.

Embodiment 56. The compound of any one of Embodiments 1 to 53, preferably the compound of Embodiment 53, wherein Xaa8 is an amino acid residue selected from the group consisting of Apc(R^{8a}), Apc, Thp, Egz, and Eca, wherein R^{8a} is a Z group, wherein the Z group comprises a bio-distribution modifier with an optional linker, a chelator with an optional linker or a combination thereof, preferably the bio-distribution modifier is AGLU and the linker is Glutar, the chelator is DOTA, or if AGLU and DOTA are combined the linker is glu, or R^{8a} is acetyl.

Embodiment 57. The compound of any one of Embodiments 1 to 54 and 56, preferably the compound of any of Embodiments 53, 54 and 56, wherein Xaa8 is an amino acid residue selected from the group consisting of Apc(R^{8a}), Apc, and Thp, wherein R^{8a} is a Z group, wherein the Z group comprises a bio-distribution modifier with an optional linker, a chelator with an optional linker or a combination thereof, preferably the bio-distribution modifier is AGLU and the linker is Glutar, the chelator is DOTA, or if AGLU and DOTA are combined the linker is glu, or R^{8a} is acetyl.

Embodiment 58. The compound of any one of Embodiments 1 to 57, preferably the compound of any one of Embodiments 53 to 57, wherein Xaa8 is an Apc(R^{8a}) residue, wherein R^{8a} is a Z group, wherein the Z group comprises a chelator with an optional linker, preferably the chelator is DOTA.

Embodiment 59. The compound of any one of Embodiments 1 to 58, preferably the compound of any one Embodiments 53 to 58, wherein the chelator
(a) preferably is selected from the group consisting of DOTA, DOTAGA, DOTAM, DOTP, NOTA, NODAGA, NODA-MPAA, HBED, TETA, CB-TE2A, DTPA, CHX-A"-DTPA, DFO, Macropa, HOPO, TRAP, THP, DATA, NOPO, NOTP, PCTA, sarcophagine, FSC, NETA, NE3TA, H4octapa, pycup, HYNIC, NxS4-x (N4, N2S2, N3S), ^{99m}Tc(CO)3-chelators and their analogs,
(b) more preferably is selected from the group consisting of DOTA, DOTAGA, DOTAM, NOTA, NODAGA, NODA-MPAA, NOPO, HBED, DTPA, CHX-A"-DTPA, CB-TE2A, Macropa, PCTA, N4, and analogs thereof,
   and
(c) most preferably is selected from the group consisting of DOTA, DOTAGA, DOTAM, NOTA, NODAGA, NOPO, Macropa, PCTA and analogs thereof.

Embodiment 60. The compound of any one of Embodiments 1 to 58, preferably the compound of any one Embodiments 53 to 58, wherein the bio-distribution modifier
(a) preferably is selected from the group consisting of AGLU with Glutar as linker, AHOL with Glutar as linker, APOL with Glutar as linker, ABOL with Glutar as linker, APrOL with Glutar as linker, MeO2kDaPEGAc, MeO10kDaPEGAc or MeO36PEG,
   or
(b) more preferably is AGLU with Glutar as linker.

Embodiment 61. The compound of any one of Embodiments 1 to 58, preferably the compound of any one of Embodiments 53 to 58, wherein the combination of chelator and bio-distribution modifier is of formula (IXe)

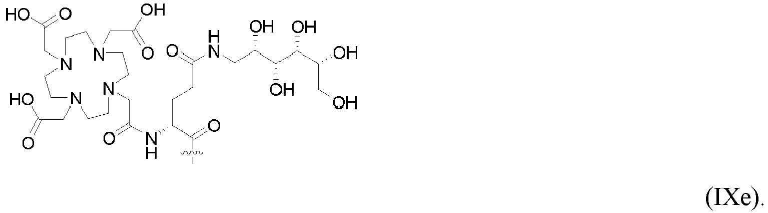

Embodiment 62. The compound of any one of Embodiments 1 to 52, wherein Xaa8 is an Aic residue.

Embodiment 63. The compound of any one of Embodiments 1 to 52, wherein Xaa8 is an amino acid residue selected from the group consisting of Lys, Lys(R^{8e}), lys(R^{8e}), Dab(R^{8e}) and dab(R^{8e}), wherein R^{8e} is a chelator, with an optional linker.

Embodiment 64. The compound of any one of Embodiments 1 to 52 and 63, preferably the compound of Embodiment 63, wherein the chelator
(a) preferably is selected from the group consisting of DOTA, DOTAGA, DOTAM, DOTP, NOTA, NODAGA, NODA-MPAA, HBED, TETA, CB-TE2A, DTPA, CHX-A"-DTPA, DFO, Macropa, HOPO, TRAP, THP, DATA, NOPO, NOTP, PCTA, sarcophagine, FSC, NETA, NE3TA, H4octapa, pycup, HYNIC, NxS4-x (N4, N2S2, N3S), ^{99m}Tc(CO)3-chelators and their analogs,
(b) more preferably is selected from the group consisting of DOTA, DOTAGA, DOTAM, NOTA, NODAGA, NODA-MPAA, NOPO, HBED, DTPA, CHX-A"-DTPA, CB-TE2A, Macropa, PCTA, N4, and analogs thereof,
   and
(c) most preferably is selected from the group consisting of DOTA, DOTAGA, DOTAM, NOTA, NODAGA, NOPO, Macropa, PCTA and analogs thereof.

Embodiment 65. The compound of any one of Embodiments 1 to 52, wherein Xaa8 is an amino acid residue selected from the group consisting of Aib, Deg, Ams, ala, Ala, Gln, Thr, Trp, Phe and Glu.

Embodiment 66. The compound of any one of Embodiments 1 to 65, wherein Xaa9 is a residue of an amino acid of formula (Xa) 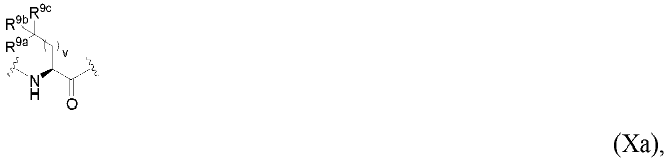 wherein
- R^{9a}: is selected from the group consisting of CH₃, CH₂CH₃, OH, NH(=NH)NH₂, CO₂H, phenyl, 3-indol, cyclo-hexyl and H,
- R^{9b}: is CH₃ or H,
- R^{9c}: is H or CH₃, and
- v: is 0, 1, 2, preferably v is 1,
the carbonyl group of Xaa9 is covalently attached to the α-nitrogen atom of Xaa10.

Embodiment 67. The compound of any one of Embodiments 1 to 66, preferably the compound of Embodiment 66, wherein Xaa9 is a residue of an amino acid of formula (Xa) 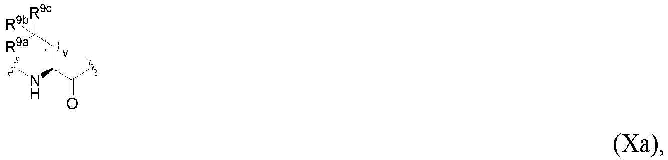 wherein
- R^{9a}: is selected from the group consisting of CH₃, CH₂CH₃, OH, NH(=NH)NH₂, CO₂H, phenyl and 3-indol,
- R^{9b}: is CH₃ or H,
- R^{9c}: is H or CH₃, and
- v: is 0, 1, 2, preferably v is 1,
the carbonyl group of Xaa9 is covalently attached to the α-nitrogen atom of Xaa10.

Embodiment 68. The compound of any one of Embodiments 1 to 66, preferably the compound of any one of Embodiments 66, wherein Xaa9 is an _{L}-amino acid residue selected from the group consisting of Leu, Hle, Ile, Arg, Trp, Glu, Phe, Ser, Cha, Npg, Tie and Ala.

Embodiment 69. The compound of any one of Embodiments 1 to 68, preferably the compound of any one of Embodiments 66 to 68, preferably the compound wherein Xaa9 is an _{L}-amino acid residue selected from the group consisting of Leu, Hle, Ile, Arg, Trp, Glu, Phe, Ser, Npg and Tie.

Embodiment 70. The compound of any one of Embodiments 1 to 69, preferably the compound of any one of Embodiments 66 to 69, wherein Xaa9 is an _{L}-amino acid residue selected from the group consisting of Leu, Hle, Ile, Arg, Trp, Glu, Phe and Ser.

Embodiment 71. The compound of any one of Embodiments 1 to 70, preferably the compound of any one of Embodiments 66 to 70, wherein Xaa9 is a Leu residue.

Embodiment 72. The compound of any one of Embodiments 1 to 71, wherein Xaa10 is a residue of an amino acid of formula (XIa) 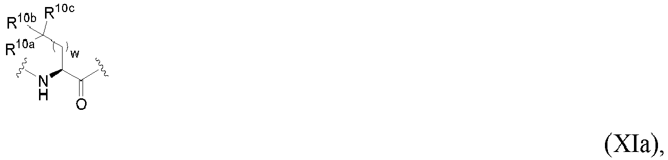 wherein
- R^{10a}: is selected from the group consisting of CH₂CH₃, CH₃, phenyl, OH and cyclo-hexyl,
- R^{10b}: is CH₃ or H,
- R^{10c}: is H or CH₃,
- w: is 0 or 1,
the carbonyl group of Xaa10 is covalently attached to the α-nitrogen atom of Xaa11.

Embodiment 73. The compound of any one of Embodiments 1 to 72, preferably the compound of Embodiment 72, wherein Xaa10 is a residue of an amino acid of formula (XVI) 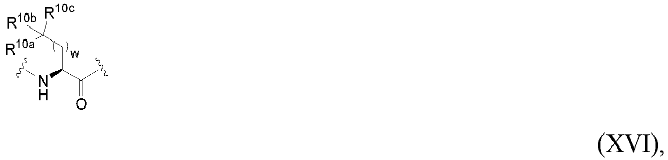 wherein
- R^{10a}: is CH₂CH₃ or CH₃
- R^{10b}: is CH₃ or H,
- R^{10c}: is H or CH₃,
- w: is 0 or 1,
the carbonyl group of Xaa10 is covalently attached to the α-nitrogen atom of Xaa11.

Embodiment 74. The compound of any one of Embodiments 1 to 72, preferably the compound of Embodiment 72, wherein Xaa10 is an _{L}-amino acid residue selected from the group consisting of Ile, Leu, Tie, Val, Phe, Cha and Ser.

Embodiment 75. The compound of any one of Embodiments 1 to 74, preferably the compound of any one of Embodiments 72 to 74, wherein Xaa10 is an _{L}-amino acid residue selected from the group consisting of Ile, Leu, Tie and Val.

Embodiment 76. The compound of any one of Embodiments 1 to 75, preferably the compound of any one of Embodiments 72 to 75, wherein Xaa10 is an _{L}-amino acid residue selected from the group consisting of Ile, Leu and Tie.

Embodiment 77. The compound of any one of Embodiments 1 to 76, preferably the compound of any one of Embodiments 72 to 76, wherein Xaa10 is an Ile residue.

Embodiment 78. The compound of any one of Embodiments 1 to 77, wherein Xaa11 is a residue of an amino acid with a nucleophilic group within the side chain or a residue of an aliphatic bis-nucleophile, preferably Xaa11 is a residue of an amino acid of formula (XIIa) 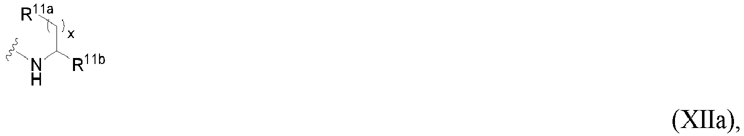 wherein
- R^{11a}: is SH or NH₂,
- R^{11b}: is selected from the group consisting of C(=O)R, CONH₂, CO₂H, CH₂OH and H, wherein R is C-term, NH₂ or OH,
- x: is 1 or 2, preferably 1.

Embodiment 79. The compound of any one of Embodiments 1 to 78, preferably the compound of Embodiment 78, wherein Xaa11 is a residue of an amino acid with a nucleophilic group within the side chain or a residue of an aliphatic bis-nucleophile, preferably Xaa11 is a residue of an amino acid of formula (XIIc) 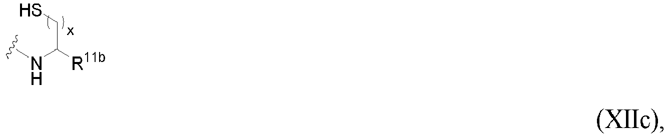 wherein
- R^{11b}: is selected from the group consisting of C(=O)R, CONH₂ and H, wherein R is NH₂ or C-term,
- x: is 1 or 2, preferably 1.

Embodiment 80. The compound of any one of Embodiments 1 to 78, preferably the compound of Embodiment 78, wherein Xaa11 is a residue selected from the group consisting of Cys, cys, Hcy, AET, en and Cysol.

Embodiment 81. The compound of any one of Embodiments 1 to 80, preferably the compound of any one of Embodiments 78 to 80, wherein Xaa11 is a residue selected from the group consisting of Cys, cys, Hcy and AET.

Embodiment 82. The compound of any one of Embodiments 1 to 81, preferably the compound of any one of Embodiments 78 to 81, wherein Xaa11 is a Cys residue.

Embodiment 83. The compound of any one of Embodiments 1 to 82, wherein if R^{1a} in Xaa1 is selected from the group consisting of NH and NHCH₃, the N-terminal modification group A
(a) is a Z group or a bio-distribution modifier optionally comprising a linker, wherein the Z group comprises a chelator and optionally a linker, preferably the bio-distribution modifier is AGLU in combination with Glutar as linker, the chelator is DOTA, the optional linker is selected from the group consisting of Ttds and APAc,
(b) is a residue of an amino acid Aaa, wherein an optional linker is interspersed between Aaa and Xaa1,
   wherein the amino acid Aaa is optionally substituted by an acetyl or a Z group, and wherein said Z group comprises a chelator and optionally a linker,
   wherein if Aaa is glu, Aaa is optionally substituted by a bio-distribution modifier,
   wherein the linker optionally interspersed between Aaa and Xaa1 is selected from the group consisting of O2Oc, PEG6, Ttds and APAc or is an amino acid residue, wherein the amino acid residue is preferably selected from Tyr, tyr, Ala and Met,
   preferably Aaa is glu which is substituted by AGLU, the chelator is DOTA and the linker optionally interspersed between Aaa and Xaa1 is selected from the group consisting of O2Oc, PEG6, Ttds and APAc,
   or
(c) is selected from the group consisting of acetyl or a blocking group Abl of a structure of formula (Abl-Ia) 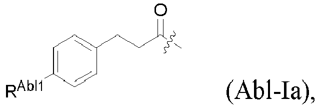 wherein
   - R^{Ab11}: is selected from the group consisting of H, OH and a halogen, preferably the halogen is selected from the group consisting of F and Cl, preferably R^{Ab11} is OH
   preferably Abl is selected from the group consisting of Ac, 40HPhp, 4FPhp, 4ClPhp and Php,
   and wherein, if present, the N-terminal modification group A is bound to the α-N atom of R^{1a} in Xaa1.

Embodiment 84. The compound of any one of Embodiments 1 to 83, preferably the compound of Embodiment 83, wherein if R^{1a} in Xaa1 is selected from the group consisting of NH and NHCH₃, the N-terminal modification group A
(a) is a Z group or a bio-distribution modifier optionally comprising a linker, wherein the Z group comprises a chelator and optionally a linker, preferably the bio-distribution modifier is AGLU in combination with Glutar as linker, the chelator is DOTA, the optional linker is selected from the group consisting of Ttds and APAc,
   or
(b) is a residue of an amino acid Aaa, wherein an optional linker is interspersed between Aaa and Xaa1,
   wherein the amino acid Aaa is optionally substituted by a Z group, and wherein said Z group comprises a chelator and optionally a linker,
   wherein if Aaa is glu, Aaa is optionally substituted by a bio-distribution modifier,
   wherein the linker optionally interspersed between Aaa and Xaa1 is selected from the group consisting of O2Oc, PEG6, Ttds and APAc,
   preferably Aaa is glu which is substituted by AGLU, the chelator is DOTA and the linker optionally interspersed between Aaa and Xaa1 is selected from the group consisting of O2Oc, PEG6, Ttds and APAc,and wherein, if present, the N-terminal modification group A is bound to the α-N atom of R^{1a} in Xaa1.

Embodiment 85. The compound of any one of Embodiments 1 to 83, preferably the compound of any one of Embodiments 83 to 84 wherein if R^{1a} in Xaa1 is selected from the group consisting of NH and NHCH₃, the N-terminal modification group A
(a) is a Z group or a bio-distribution modifier optionally comprising a linker, wherein the Z group comprises a chelator and optionally a linker, preferably the bio-distribution modifier is AGLU in combination with Glutar as linker, the chelator is DOTA, the optional linker is APAc,
   or
(b) is a residue of an amino acid Aaa, wherein an optional linker is interspersed between Aaa and Xaa1,
   wherein the amino acid Aaa is optionally substituted by a Z group, and wherein said Z group comprises a chelator and optionally a linker, wherein if Aaa is glu, Aaa is optionally substituted by a bio-distribution modifier,
   wherein the linker optionally interspersed between Aaa and Xaa1 is selected from the group consisting of O2Oc, PEG6, Ttds and APAc,
   preferably Aaa is glu which is substituted by AGLU, the chelator is DOTA and the linker optionally interspersed between Aaa and Xaa1 is APAc,and wherein, if present, the N-terminal modification group A is bound to the α-N atom of R^{1a} in Xaa1.

Embodiment 86. The compound of any one of Embodiments 1 to 85, preferably the compound of any one of Embodiment 83 to 85, wherein, if in Xaa1 R^{1a} is NH, the N-terminal modification group A is a bio-distribution modifier optionally comprising a linker, most preferably the bio-distribution modifier is AGLU in combination with Glutar as linker.

Embodiment 87. The compound of any one of Embodiments 1 to 85, preferably the compound of any one of Embodiments 83 to 85, wherein if in Xaa1 R^{1a} is NH, the N-terminal modification group A is a residue of an amino acid Aaa,
wherein an optional linker is interspersed between Aaa and Xaa1,
wherein the amino acid Aaa is substituted by a Z group, wherein said Z group comprises a chelator and with an optional linker,
wherein if Aaa is glu, Aaa is substituted by a bio-distribution modifier,
wherein most preferably Aaa is glu,
   which is substituted by AGLU,
   the chelator is DOTA and
   the linker optionally interspersed between Aaa and Xaa1 is APAc.

Embodiment 88. The compound of any one of Embodiments 1 to 87, preferably the compound of any one Embodiments 83 to 87, wherein the chelator
(a) preferably is selected from the group consisting of DOTA, DOTAGA, DOTAM, DOTP, NOTA, NODAGA, NODA-MPAA, HBED, TETA, CB-TE2A, DTPA, CHX-A"-DTPA, DFO, Macropa, HOPO, TRAP, THP, DATA, NOPO, NOTP, PCTA, sarcophagine, FSC, NETA, NE3TA, H4octapa, pycup, HYNIC, NxS4-x (N4, N2S2, N3S), ^{99m}Tc(CO)3-chelators and their analogs,
(b) more preferably is selected from the group consisting of DOTA, DOTAGA, DOTAM, NOTA, NODAGA, NODA-MPAA, NOPO, HBED, DTPA, CHX-A"-DTPA, CB-TE2A, Macropa, PCTA, N4, and analogs thereof,
   and
(c) most preferably is selected from the group consisting of DOTA, DOTAGA, DOTAM, NOTA, NODAGA, NOPO, Macropa, PCTA and analogs thereof.

Embodiment 89. The compound of any one of Embodiments 1 to 87, preferably the compound of any one Embodiments 83 to 87, wherein the bio-distribution modifier
(a) preferably is selected from the group consisting of AGLU with Glutar as linker, AHOL with Glutar as linker, APOL with Glutar as linker, ABOL with Glutar as linker, APrOL with Glutar as linker, MeO2kDaPEGAc, MeO10kDaPEGAc or MeO36PEG,
   or
(b) more preferably is AGLU with Glutar as linker.

Embodiment 90. The compound of any one of Embodiments 1 to 89, wherein if Xaa11 is a residue of an amino acid of formula (XIIa) and R^{11b} therein is C(=O)R, whereby R is C-term,
the C-terminal group C-term
   (a) is one or more amino acids AA,
      wherein at least one or more of the one or more amino acids AA is optionally substituted by a Z group and/or a bio-distribution modifier optionally comprising a linker, wherein said Z group comprises a chelator and optionally a linker,
      wherein an optional linker is interspersed between Xaa11 and the first of the one or more amino acids AA,
      wherein to the last of the one or more amino acids a blocking group Abl is attached, wherein Abl is selected from the group consisting of NH₂ and OH, wherein Abl preferably is NH₂, wherein if AA is glu, AA is optionally substituted by a bio-distribution modifier, preferably the bio-distribution modifier is AGLU,
      wherein if AA is Lys, AA is optionally substituted by a chelator, preferably the chelator is DOTA,
   (b) is a Z group,
      wherein the Z group comprises a chelator and a linker
or
   (c) is a blocking group Abl, wherein
   Abl is OH or NH₂, preferably Abl is NH₂.

Embodiment 91. The compound of any one of Embodiments 1 to 90, preferably the compound of Embodiment 90, wherein, if Xaa11 is a residue of an amino acid of formula (XIIa) and R^{11b} therein is C(=O)R, whereby R is C-term,
the C-terminal group C-term
(a) is one or more amino acids AA,
   wherein at least one or more of the one or more amino acid AA is optionally substituted by a Z group and/or a bio-distribution modifier optionally comprising a linker, wherein said Z group comprises a chelator and optionally a linker,
   wherein an optional linker is interspersed between Xaa11 and the first of the one or more amino acids AA,
   wherein to the last of the one or more amino acids a blocking group Abl is attached, wherein Abl is selected from the group consisting of NH₂ and OH, wherein Abl preferably is NH₂, wherein if AA is glu, AA is optionally substituted by a bio-distribution modifier, preferably the bio-distribution modifier is AGLU,
   wherein if AA is Lys, AA is optionally substituted by a chelator, preferably the chelator is DOTA,
   or
(b) is a Z group,
   wherein the Z group comprises a chelator and a linker.

Embodiment 92. The compound of any one of Embodiments 1 to 91, preferably the compound of any one of Embodiments 90 to 91, wherein, if Xaa11 is a residue of an amino acid of formula (XIIa) and R^{11b} therein is C(=O)R, whereby R is C-term,
the C-terminal group C-term
is one or more amino acids AA,
   wherein at least one of the one or more amino acids AA is substituted with a bio-distribution modifier optionally comprising a linker and at least one of the one or more amino acids AA is substituted with a chelator optionally comprising a linker,
   wherein an optional linker is interspersed between Xaa11 and the first of the one or more amino acids AA,
   wherein to the last of the one or more amino acids NH₂ is attached as a blocking group Abl,
   and
   wherein preferably
   the first of the one or more amino acids is glu with AGLU as bio-distribution modifier attached to the side chain of said glu,
   the second of the one or more amino acids is Lys with DOTA as chelator attachted to the side chain of said Lys and the linker is interspersed between Xaa11 and the first of the one or more amino acids AA is APAc.

Embodiment 93. The compound of any one of Embodiments 1 to 92, preferably the compound of any one Embodiments 90 to 92, wherein, if Xaa11 is a residue of an amino acid of formula (XIIa) and R^{11b} is C(=O)R, whereby R is C-term,
the C-terminal group C-term
is one amino acid AA,
wherein the amino acid AA is substituted with a bio-distribution modifier optionally comprising a linker,
wherein an optional linker is interspersed between Xaa11 and the amino acid AA,
wherein to the amino acid AA NH₂ is attached as a blocking group Abl and
wherein preferably
   the amino acid is glu with AGLU as bio-distribution modifier attached to the side chain of said glu,
   the linker interspersed between Xaa11 and the amino acid is APAc.

Embodiment 94. The compound of any one of Embodiments 1 to 92, preferably the compound of any one of Embodiments 90 to 92, wherein, if Xaa11 is a residue of an amino acid of formula (XIIa) and R^{11b} is C(=O)R, whereby R is C-term,
the C-terminal group C-term
is two amino acid AA,
wherein the first of the amino acids AA is substituted with a bio-distribution modifier optionally comprising a linker,
wherein the second of the amino acids AA is substituted with a chelator optionally comprising a linker,
wherein an optional linker is interspersed between Xaa11 and the amino acid AA,
wherein to the amino acid AA NH₂ is attached as a blocking group Abl and
wherein preferably
   the first amino acid is glu with AGLU as bio-distribution modifier attached to the side chain of said glu,
   the second amino acid is Lys with DOTA as chelator attached to the side chain of said Lys,
   the linker interspersed between Xaa11 and the first amino acid is APAc.

Embodiment 95. The compound of any one of Embodiments 1 to 94, preferably the compound of any one of Embodiments 90 to 94, wherein the chelator
(a) is selected from the group consisting of DOTA, DOTAGA, DOTAM, DOTP, NOTA, NODAGA, NODA-MPAA, HBED, TETA, CB-TE2A, DTPA, CHX-A"-DTPA, DFO, Macropa, HOPO, TRAP, THP, DATA, NOPO, NOTP, PCTA, sarcophagine, FSC, NETA, NE3TA, H4octapa, pycup, HYNIC, NxS4-x (N4, N2S2, N3S), ^{99m}Tc(CO)3-chelators and their analogs,
(b) preferably is selected from the group consisting of DOTA, DOTAGA, DOTAM, NOTA, NODAGA, NODA-MPAA, NOPO, HBED, DTPA, CHX-A"-DTPA, CB-TE2A, Macropa, PCTA, N4, and analogs thereof,
   and
(c) more preferably is selected from the group consisting of DOTA, DOTAGA, DOTAM, NOTA, NODAGA, NOPO, Macropa, PCTA and analogs thereof.

Embodiment 96. The compound of any one of Embodiments 1 to 94, preferably the compound of any one of Embodiments 90 to 94, wherein the bio-distribution modifier
(a) is selected from the group consisting of AGLU with Glutar as linker, AHOL with Glutar as linker, APOL with Glutar as linker, ABOL with Glutar as linker, APrOL with Glutar as linker, MeO2kDaPEGAc, MeO10kDaPEGAc or MeO36PEG,
   or
(b) preferably is AGLU with Glutar as a linker.

Embodiment 97. The compound of any one of Embodiments 1 to 96, wherein the cyclic peptide is a cyclic peptide of formula (Ia), wherein Yc is a structure of formula (XIIIa) 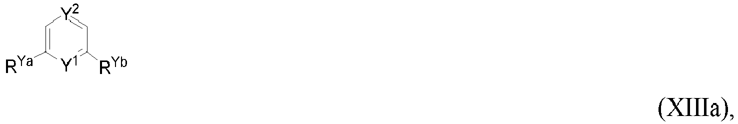 wherein
- R^{Ya} and R^{Yb}: are each and independently -CH₂-,
- Y¹: is N or CH,
- Y²: is CH, N or C-R^{Yc},
- R^{Yc}: is H or -CH₂-R^{Yd}
- and R^{Yd}: is a structure of formula (XIIIi) or (XIIIk), 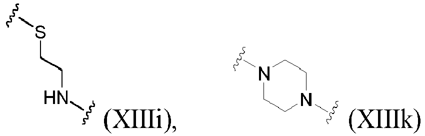
wherein
in formulae (XIIIi) the S-atom is attached to -CH₂- of R^{Yc} and
in formulae (XIIIk) one of the two N-atoms is to -CH₂- of R^{Yc}, while the remaining N-atom connects to a Z group
comprising a chelator with an optional linker,
wherein if R^{Yd} is present, R^{Yd} preferably is a structure of formulae (XIIIi).

Embodiment 98. The compound of any one of Embodiments 1 to 97, preferably the compound of Embodiment 97, wherein the cyclic peptide is a cyclic peptide of formula (Ia), wherein Yc is a structure of formula (XIIIa) 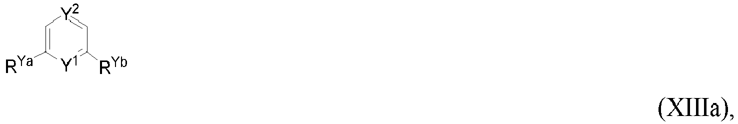 wherein
- R^{Ya} and R^{Yb}: are each and independently -CH₂-,
- Y¹: is N or CH,
- Y²: is CH or N.

Embodiment 99. The compound of any one of Embodiments 1 to 98, preferably the compound of any one of Embodiments 97 to 98, wherein the cyclic peptide is a cyclic peptide of formula (Ia), wherein Yc is a structure of formula (XIIIm) 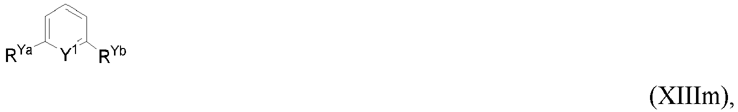 wherein
- R^{Ya}: is -CH₂-,
- R^{Yb}: is -CH₂-,
- Y¹: is N or CH.

Embodiment 100. The compound of any one of Embodiments 1 to 97, preferably the compound of Embodiment 97, wherein the cyclic peptide is a cyclic peptide of formula (Ia), wherein Yc is selected from the group consisting of 2Lut, 3MeBn, 3Lut, tMeBn(DOTA-AET) and tMeBn(DOTA-PP).

Embodiment 101. The compound of any one of Embodiments 1 to 98 and 100, preferably the compound of any one of Embodiments 97, 98 and 100, wherein the cyclic peptide is a cyclic peptide of formula (Ia), wherein Yc is selected from the group consisting of 2Lut, 3Lut and 3MeBn.

Embodiment 102. The compound of any one of Embodiments 1 to 101, preferably the compound of any one of Embodiments 97 to 101, wherein the cyclic peptide is a cyclic peptide of formula (Ia), wherein Yc is selected from the group consisting of 2Lut and 3MeBn.

Embodiment 103. The compound of any one of Embodiments 1 to 102, preferably the compound of any one of Embodiments 97 to 102, wherein the cyclic peptide is a cyclic peptide of formula (Ia), wherein Yc is 2Lut.

Embodiment 104. The compound of any one of Embodiments 1 to 96, wherein under the proviso that either
- Xaa7: is a Dap residue,
- while Xaa11: is selected from the group consisting of Cys, Hcy, AET or Cysol,
or
- Xaa7: is either Cys or Hcy,
- while Xaa11: is an en residue,
the cyclic peptide is a cyclic peptide of formula (Ia), wherein Yc is a structure of formula (XIIIf), 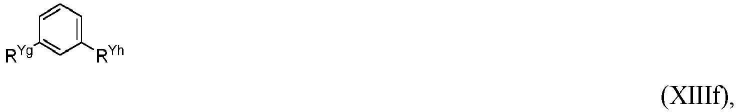 wherein
- R^{Yg}: is -C(=O) - and is connected to said Dap or en residue, and
- R^{Yh}: is -CH₂- and is connected to the sulphur atom of the other residue.

Embodiment 105. The compound of any one of Embodiments 1 to 96 and 104, preferably the compound of Embodiment 104, wherein the cyclic peptide is a cyclic peptide of formula (Ia), wherein Yc is 3MeBz or 3CbBn.

Embodiment 106. The compound of any one of Embodiments 1 to 96, wherein under the proviso that Xaa7 is a Cys or an Hcy residue and Xaa11 is a residue selected from the group consisting of Cys, Hcy, AET and Cysol, the cyclic peptide is a cyclic peptide of formula (Ia), wherein Yc is a structure of formula (XIIIn)

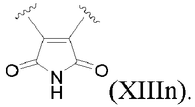

Embodiment 107. The compound of any one of Embodiments 1 to 96, wherein under the proviso that Xaa7 is Cys or Hcy and Xaa11 is a residue selected from the group consisting of Cys, Hcy, AET and Cysol, the cyclic peptide is a cyclic peptide of formula (Ib).

Embodiment 108. The compound of Embodiment 1, wherein
- Xaa1: is Asp, Asn, Glu, Gln, Succinyl, Glutar or Cya,
- Xaa2: is Trp, Hyw, 5Fw, 6Clw or 7Fw,
- Xaa3: is 5Clw, 5Brw, 5Fw, Trp, INi, Bta, 6Fw or Trp,
- Xaa4: is Tap, Hyp, 4Tfp, Pro, H3p, Eaz, Oxa, Dtc, Nmg, Nleu, Nlys, Nphe or Nglu,
- Xaa5: is Glu, glu, Nme, Gln, Asp, Asn or Hse,
- Xaa6: is leu, Leu, Npg, Val or Ile,
- Xaa7: is Cys or Hcy,
- Xaa8: is Apc(DOTA), Thp, Apc, Aib, Deg, Ams, Lys, Thr, Egz, Eca, Ala or ala,
- Xaa9: is Leu, Hle, Ile, Phe, Glu, Arg, Ser or Trp,
- Xaa10: is Ile, Leu, Val or Tie, and
- Xaa11: is Cys, Hcy or AET.

Embodiment 109. The compound of any one of Embodiments 1 and 108, wherein
- Xaa1: is Asp, Glu or Succinyl,
- Xaa2: is Trp or Hyw,
- Xaa3: is 5Clw or 5Brw,
- Xaa4: is Tap, Hyp, or Pro,
- Xaa5: is Glu,
- Xaa6: is leu or Leu,
- Xaa7: is Cys,
- Xaa8: is Apc(DOTA) or Thp,
- Xaa9: is Leu,
- Xaa10: is Ile and
- Xaa11: is Cys.

Embodiment 110. The compound of any one of Embodiments 1, 108 and 109, wherein
- Xaa1: is Asp,
- Xaa2: is Trp,
- Xaa3: is 5Clw,
- Xaa4: is Tap or Hyp,
- Xaa5: is Glu,
- Xaa6: is leu,
- Xaa7: is Cys,
- Xaa8: is Apc(DOTA),
- Xaa9: is Leu,
- Xaa10: is Ile and
- Xaa11: is Cys.

Embodiment 111. The compound of any one of Embodiments 1, 83 and 108, wherein under the proviso that Xaa1 is selected from the group consisting of Asp, Asn, Glu, Gln and Cya,
the N-terminal modification group A
(a) is selected from the group consisting of AGLU-Glutar, AGLU-Glutar-APAc, DOTA, DOTA-Ttds, DOTA-APAc, MeO36PEG-APAc or MeO2kDaPEGAc,
(b) is selected from the group consisting of DOTA-glu(AGLU), DOTA-glu(AGLU)-APAc, DOTA-O2Oc-glu(AGLU), DOTA-glu(AGLU)-O2Oc, DOTA-PEG6-glu(AGLU), DOTA-glu(AGLU)-PEG6, Ac-Tyr, Met-Tyr, Met-tyr or Met-Ala,
   or
(c) is selected from the group consisting of Ac, Php, 4FPhp, 4ClPhp or 4OHPhp, preferably is 4OHPhp,
and wherein the N-terminal modification group A is bound to the α-N atom in Xaa1.

Embodiment 112. The compound of any one of Embodiments 1, 83, 84, 108 and 111, wherein under the proviso that Xaa1 is selected from the group consisting of Asp, Asn, Glu, Gln and Cya,
the N-terminal modification group A
(a) is selected from the group consisting of AGLU-Glutar, AGLU-Glutar-APAc, DOTA, DOTA-Ttds or DOTA-APAc,
   or
(b) is selected from the group consisting of DOTA-glu(AGLU),
   DOTA-glu(AGLU)-APAc, DOTA-O2Oc-glu(AGLU),
   DOTA-glu(AGLU)-O2Oc, DOTA-PEG6-glu(AGLU) or
   DOTA-glu(AGLU)-PEG6,
   and wherein the N-terminal modification group A is bound to the α-N atom in Xaa1.

Embodiment 113. The compound of any one of Embodiments 1, 83, 84 and 108, 111 and 112, wherein under the proviso that Xaa1 is selected from the group consisting of Asp, Asn, Glu, Gln and Cya the N-terminal modification group A is selected from the group consisting of AGLU-Glutar, DOTA-glu(AGLU)-APAc, DOTA and DOTA-Ttds.

Embodiment 114. The compound of any one of Embodiments 1, 83 and 109, wherein under the proviso that Xaa1 is selected from the group consisting of Asp and Glu,
the N-terminal modification group A
(a) is selected from the group consisting of AGLU-Glutar, AGLU-Glutar-APAc, DOTA, DOTA-Ttds, DOTA-APAc, MeO36PEG-APAc or MeO2kDaPEGAc,
(b) is selected from the group consisting of DOTA-glu(AGLU), DOTA-glu(AGLU)-APAc, DOTA-O2Oc-glu(AGLU), DOTA-glu(AGLU)-O2Oc, DOTA-PEG6-glu(AGLU), DOTA-glu(AGLU)-PEG6, Ac-Tyr, Met-Tyr, Met-tyr or Met-Ala,
   or
(c) is selected from the group consisting of Ac, Php, 4FPhp, 4ClPhp or 4OHPhp, preferably is 4OHPhp,
and wherein the N-terminal modification group A is bound to the α-N atom in Xaa1.

Embodiment 115. The compound of any one of Embodiments 1, 83, 84, 109 and 114, wherein under the proviso that Xaa1 is selected from the group consisting of Asp and Glu,
the N-terminal modification group A
(a) is selected from the group consisting of AGLU-Glutar, AGLU-Glutar-APAc, DOTA, DOTA-Ttds or DOTA-APAc,
   or
(b) is selected from the group consisting of DOTA-glu(AGLU),
   DOTA-glu(AGLU)-APAc, DOTA-O2Oc-glu(AGLU),
   DOTA-glu(AGLU)-O2Oc, DOTA-PEG6-glu(AGLU) or
   DOTA-glu(AGLU)-PEG6,
   and wherein the N-terminal modification group A is bound to the α-N atom in Xaa1.

Embodiment 116. The compound of any one of Embodiments 1, 83, 84 and 109, 114 and 115, wherein under the proviso that Xaa1 is selected from the group consisting of Asp and Glu, the N-terminal modification group A is selected from the group consisting of AGLU-Glutar, DOTA-glu(AGLU)-APAc, DOTA and DOTA-Ttds.

Embodiment 117. The compound of any one of Embodiments 1, 83 and 110,
wherein the N-terminal modification group A
(a) is selected from the group consisting of AGLU-Glutar, AGLU-Glutar-APAc, DOTA, DOTA-Ttds, DOTA-APAc, MeO36PEG-APAc or MeO2kDaPEGAc,
(b) is selected from the group consisting of DOTA-glu(AGLU), DOTA-glu(AGLU)-APAc, DOTA-O2Oc-glu(AGLU), DOTA-glu(AGLU)-O2Oc, DOTA-PEG6-glu(AGLU), DOTA-glu(AGLU)-PEG6, Ac-Tyr, Met-Tyr, Met-tyr or Met-Ala,
   or
(c) is selected from the group consisting of Ac, Php, 4FPhp, 4ClPhp or 4OHPhp, preferably is 4OHPhp,
and wherein the N-terminal modification group A is bound to the α-N atom in Xaa1.

Embodiment 118. The compound of any one of Embodiments 1, 83, 84, 110 and 117,
wherein the N-terminal modification group A
(a) is selected from the group consisting of AGLU-Glutar, AGLU-Glutar-APAc, DOTA, DOTA-Ttds or DOTA-APAc,
   or
(b) is selected from the group consisting of DOTA-glu(AGLU),
   DOTA-glu(AGLU)-APAc, DOTA-O2Oc-glu(AGLU),
   DOTA-glu(AGLU)-O2Oc, DOTA-PEG6-glu(AGLU) or
   DOTA-glu(AGLU)-PEG6,
   and wherein the N-terminal modification group A is bound to the α-N atom in Xaa1.

Embodiment 119. The compound of any one of Embodiments 1, 83, 84 and 110, 117 and 118, wherein the N-terminal modification group A is selected from the group consisting of AGLU-Glutar, DOTA-glu(AGLU)-APAc, DOTA and DOTA-Ttds.

Embodiment 120. The compound of any one of Embodiments 1, 83 to 86, 110, and 117 to 119, wherein the N-terminal modification group A is AGLU-Glutar.

Embodiment 121. The compound of any one of Embodiments 1, 83 to 85, 87, 110 and 117 to 119, wherein the N-terminal modification group A is DOTA-glu(AGLU)-APAc.

Embodiment 122. The compound of any one of Embodiments 1, 90, 91 and 108 to 121, wherein under the proviso that Xaa11 is Cys, the C-terminal group C-term
(a) is selected from the group consisting of APAc-glu(AGLU)-NH₂,
   APAc-glu(AGLU)-Lys(DOTA)-NH₂,
   APAc-glu(AGLU)-glu(AGLU)-K(DOTA)-NH₂, APAc-Lys(DOTA)-NH₂,
   Ttds-Lys(DOTA)-NH₂, APAc-lys(MeO2kDaPEGAc)-Lys(DOTA)-NH₂,
   glu(AGLU)-NH₂, Lys(DOTA)-NH₂, lys(DOTA)-NH₂ or
   lys(MeO36PEG)-NH₂,
(b) is Ape-DOTA or PP-DOTA,
   or
(c) is NH₂ or OH, preferably NH₂.

Embodiment 123. The compound of any one of Embodiments 1, 90 to 92 and 108 to 122, wherein under the proviso that Xaa11 is Cys, the C-terminal group C-term is selected from the group consisting of APAc-glu(AGLU)-NH₂, APAc-glu(AGLU)-Lys(DOTA)-NH₂ or APAc-Lys(DOTA)-NH₂.

Embodiment 124. The compound of any one of Embodiments 1, 90 to 93, 108 to 123, wherein under the proviso that Xaa11 is Cys, the C-terminal group C-term is APAc-glu(AGLU)-NH₂.

Embodiment 125. The compound of any one of Embodiments 1, 90 to 92, 94 and 108 to 124, wherein under the proviso that Xaa11 is Cys, the C-terminal group C-term is APAc-glu(AGLU)-Lys(DOTA)-NH₂.

Embodiment 126. The compound of any one of Embodiments 1, 97 to 103 and 108 to 126, wherein the cyclic peptide is a cyclic peptide of formula (Ia), wherein Yc is 3MeBn.

Embodiment 127. The compound of any one of Embodiments 1, 97 to 102 and 108 to 119, wherein the cyclic peptide is a cyclic peptide of formula (Ia), wherein Yc is 2Lut.

Embodiment 128. The compound of any one of Embodiments 1 to 127, wherein the compound comprises one single chelator.

Embodiment 129. The compound of any one of Embodiments 1 to 30, 32 to 34, 42 to 59, 61, 63 to 64, 66 to 88, 90 to 92, 94 to 97, 108 to 119, 121 to 123, 125 and 128, preferably the compound of Embodiment 128, wherein the chelator
(a) is attached to or part of substituent R^{4d},
(b) is attached to or part of substituent R^{8a} under the proviso that Xaa8 is of structure IXa,
(c) is attached to or part of substituent R^{8e} under the proviso that Xaa8 is of structure IXc,
(d) is attached to or part of substituent R^{Yd},
(e) is attached to amino acid Aaa of the N-terminal modification group A,
(f) is attached to amino acid AA of the C-terminal group C-term,
(g) is part of or forms the N-terminal modification group A,
   or
(h) is part of the C-terminal group C-term.

Embodiment 130. The compound of any one of Embodiments 1 to 59, 61, 63 to 64, 66 to 88, 90 to 92, 94 to 119, 121 to 123 and 125 to 129, preferably the compound of any one of Embodiments 128 and 129, wherein the chelator
(a) is attached to or part of substituent R^{8a} under the proviso that Xaa8 is of structure IXa,
(b) is attached to or part of substituent R^{8e} under the proviso that Xaa8 is of structure IXc,
(c) is attached to amino acid Aaa of the N-terminal modification group A,
(d) is attached to amino acid AA of the C-terminal group C-term,
(e) is part of or forms the N-terminal modification group A,
   or
(f) is part of the C-terminal group C-term.

Embodiment 131. The compound of any one of Embodiments 1 to 59, 61, 63 to 64, 66 to 88, 90 to 92, 94 to 119, 121 to 123, 125 to 130, preferably the compound of any one of Embodiments 128 to 130, wherein the chelator
(a) is attached to or part of substituent R^{8a} under the proviso that Xaa8 is of structure IXa,
(b) is attached to or part of substituent R^{8e} under the proviso that Xaa8 is of structure IXc,
(c) is attached to amino acid Aaa of the N-terminal modification group A,
   or
(d) is attached to amino acid AA of the C-terminal group C-term,
preferably the chelator is part of substituent R^{8a}.

Embodiment 132. The compound of any one of Embodiments 1 to 59, 61, 63 to 64 and 66 to 131, preferably the compound of any one of Embodiments 128 to 131, wherein the chelator is attached to or part of substituent R^{8a} under the proviso that Xaa8 is of structure IXa.

Embodiment 133. The compound of any one of Embodiments 1 to 88, 90 to 119 and 121 to 131, preferably the compound of any one of Embodiments 128 to 131, wherein the chelator is attached to amino acid Aaa of the N-terminal modification group A.

Embodiment 134. The compound of any one of Embodiments 1 to 92, 94 to 123, 125 to 131, preferably the compound of any one Embodiments 128 to 131, wherein the chelator is attached to amino acid AA of the C-terminal group C-term.

Embodiment 135. The compound of any one of Embodiments 1 to 127, wherein the compound comprises two of more chelators.

Embodiment 136. The compound of any one of Embodiments 1 to 30, 32 to 34, 42 to 59, 61, 63 to 64, 66 to 88, 90 to 92, 94 to 97, 108 to 119, 121 to 123, 125 and 135, preferably the compound of Embodiment 135, wherein each of the two or more chelators
(a) is attached to or part of substituent R^{4d},
(b) is attached to or part of substituent R^{8a} under the proviso that Xaa8 is of structure IXa,
(c) is attached to or part of substituent R^{8e} under the proviso that Xaa8 is of structure IXc,
(d) is attached to or part of substituent R^{Yd},
(e) is attached to part of substituent amino acid Aaa of the N-terminal modification group A,
(f) is attached to part of substituent amino acid AA of the C-terminal group C-term,
(g) is part of or forms the N-terminal modification group A,
   or
(h) is part of the C-terminal group C-term,
preferably under the proviso that one chelator is attached to or part of one of said substituents and group, respectively, only.

Embodiment 137. The compound of any one of Embodiments 1 to 30, 32 to 34, 42 to 59, 61, 63 to 64, 66 to 88, 90 to 92, 94 to 97, 108 to 119, 121 to 123, 125, 135 and 136, preferably the compound of any one of Embodiments 135 and 136, wherein the compound comprises 2 to 4 chelators, preferably 2 to 3 chelators, more preferably 2 chelators.

Embodiment 138. The compound of any one of Embodiments 1 to 30, 32 to 34, 42 to 59, 61, 63 to 64, 66 to 88, 90 to 92, 94 to 119, 121 to 123, 125 to 127 and 135 to 137, preferably the compound of any one of Embodiments 135 and 136, wherein the compound comprises 4 chelators, wherein
(a) the first of said 4 chelators is attached to or part of substituent R^{4d},
(b) the second of said 4 chelators is attached to or part of substituent R^{8a} or substituent R^{8e},
(c) the third of said 4 chelators is attached to amino acid Aaa of the N-terminal modification group A or is part of or forms the N-terminal modification group A,
   and
(d) the fourth of said 4 chelators is attached to amino acid AA of the C-terminal group C-term or is part of the C-terminal group C-term.

Embodiment 139. The compound of any one of c Embodiments 1 to 59, 61, 63 to 64, 66 to 88, 90 to 92, 94 to 119, 121 to 123, 125 to 127 and 135 to 137, preferably the compound of any one of Embodiments 135 and 136, wherein the compound comprises 3 chelators, wherein
(a) the first of said 3 chelators is attached to or part of substituent R^{8a} or substituent R^{8e},
(b) the second of said 3 chelators is attached to amino acid Aaa of the N-terminal modification group A or is part of or forms the N-terminal modification group A,
   and
(c) the third of said 3 chelators is attached to amino acid AA of the C-terminal group C-term or is part of the C-terminal group C-term.

Embodiment 140. The compound of any one of Embodiments 1 to 59, 61, 63 to 64, 66 to 88, 90 to 92, 94 to 119, 121 to 123, 125 to 127 and 135 to 137, preferably the compound of any one of Embodiments 135 and 136, wherein the compound comprises 2 chelators, wherein
(a) the first of said 2 chelators is attached to or part of substituent R^{8a} or substituent R^{8e},
   and
(b) the second of said 2 chelators is attached to amino acid Aaa of the N-terminal modification group A or is part of or forms the N-terminal modification group A
   or
(c) the second of said 2 chelators is attached to amino acid AA of the C-terminal group C-term or is part of the C-terminal group C-term.

Embodiment 141. The compound of any one of Embodiments 1 to 88, 90 to 92, 94 to 119, 121 to 123, 125 to 127 and 135 to 137, preferably the compound of any one of Embodiments 135 and 136, wherein the compound comprises 2 chelators, wherein
(a) the first of said 2 chelators is attached to amino acid Aaa of the N-terminal modification group A or is part of or forms the N-terminal modification group
   and
(b) the second of said 2 chelators is attached to amino acid AA of the C-terminal group C-term or is part of the C-terminal group C-term.

Embodiment 142. The compound of any one of Embodiments 1 to 141, wherein the compound is selected from the group consisting of
compound AGLU-Glutar-Asp-Trp-5Clw-Tap-Glu-leu-[Cys(2Lut)-Apc(DOTA)-Leu-Ile-Cys]-APAc-glu(AGLU)-NH₂ (GIP-001) of the following formula 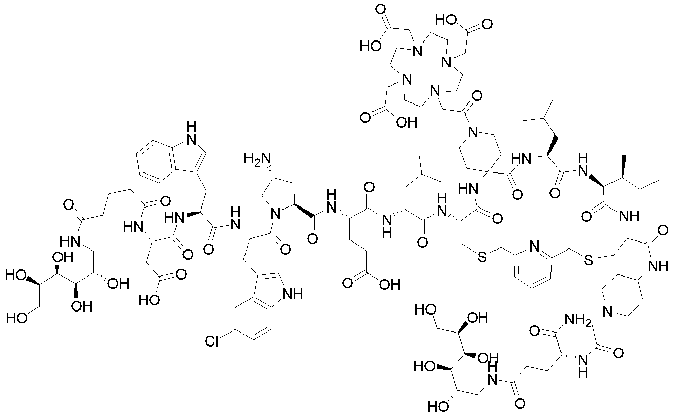
compound AGLU-Glutar-Asp-Trp-5Clw-Hyp-Glu-leu-[Cys(2Lut)-Apc(DOTA)-Leu-Ile-Cys]-APAc-glu(AGLU)-NH₂ (GIP-002) of the following formula 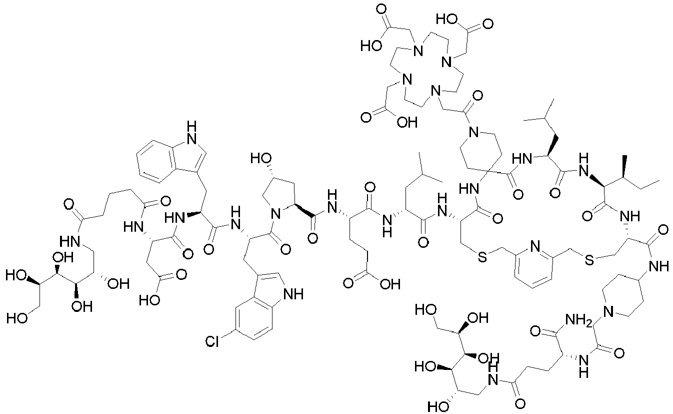
compound DOTA-glu(AGLU)-APAc-Asp-Trp-5Clw-Hyp-Glu-leu-[Cys(2Lut)-Apc(AGLU-Glutar)-Leu-Ile-Cys]-APAc-glu(AGLU)-NH₂ (GIP-003) of the following formula 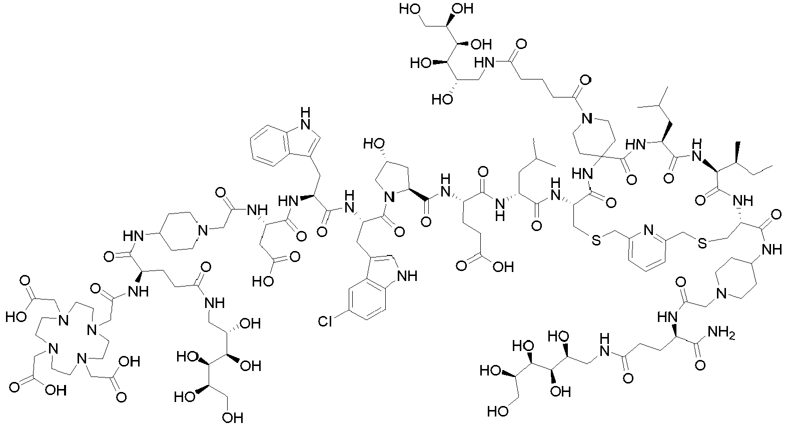
compound AGLU-Glutar-Asp-Trp-5Clw-Hyp-Glu-leu-[Cys(2Lut)-Thp-Leu-Ile-Cys]-APAc-glu(AGLU)-Lys(DOTA)-NH₂ (GIP-004) of the following formula 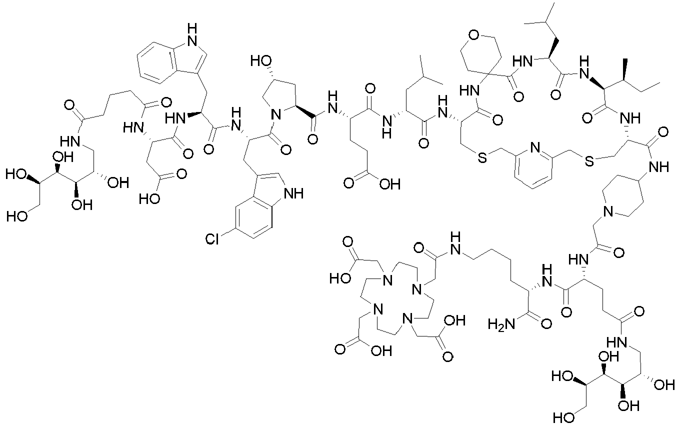
compound DOTA-glu(AGLU)-APAc-Asp-Trp-5Clw-Hyp-Glu-leu-[Cys(2Lut)-Thp-Leu-Ile-Cys]-APAc-glu(AGLU)-NH₂ (GIP-005) of the following formula 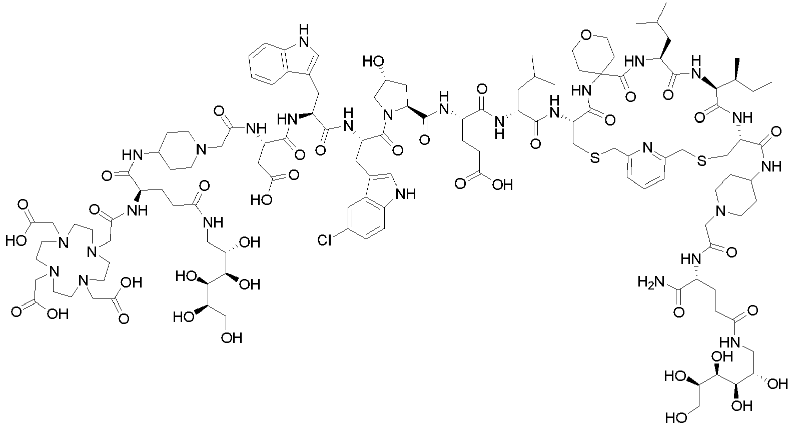
compound AGLU-Glutar-APAc-Asp-Trp-5Clw-Tap(AGLU-Glutar)-Glu-leu-[Cys(2Lut)-Apc(DOTA)-Leu-Ile-Cys]-APAc-glu(AGLU)-NH₂ (GIP-006) of the following formula 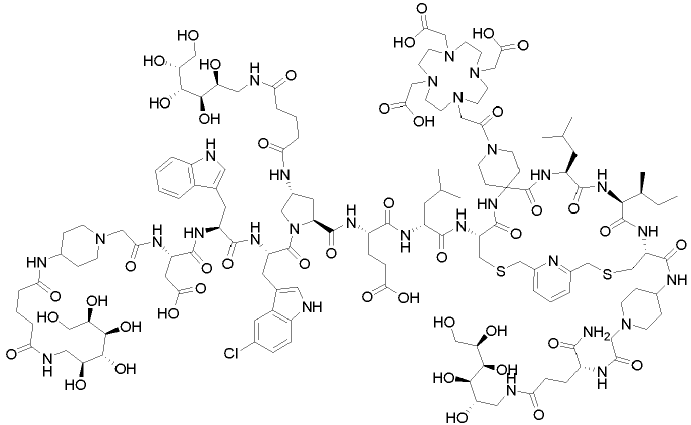
compound AGLU-Glutar-APAc-Asp-Trp-5Clw-Tap(AGLU-Glutar)-Glu-leu-[Cys(2Lut)-Apc(DOTA-glu(AGLU))-Leu-Ile-Cys]-APAc-glu(AGLU)-NH₂ (GIP-007) of the following formula 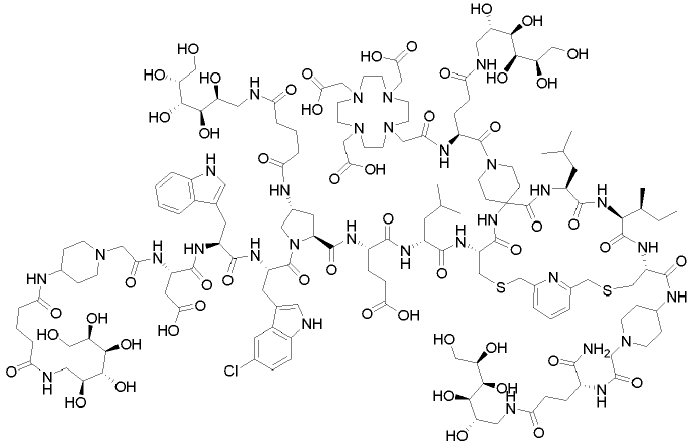
compound AGLU-Glutar-APAc-Asp-Trp-5Clw-Tap-Glu-leu-[Cys(2Lut)-Apc(DOTA-glu(AGLU))-Leu-Ile-Cys]-APAc-glu(AGLU)-NH₂ (GIP-008) of the following formula 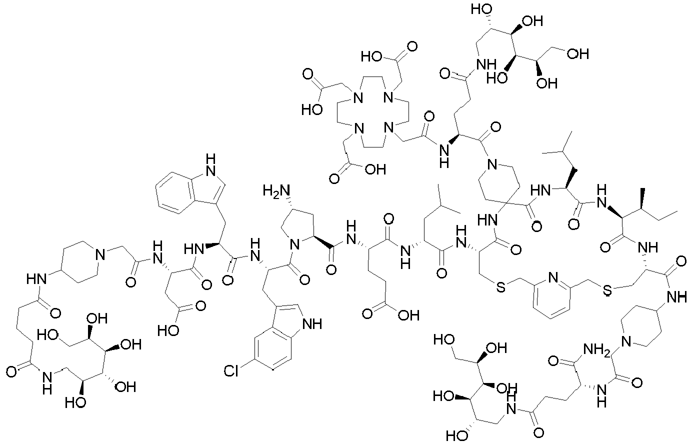
compound DOTA-glu(AGLU)-O2Oc-Asp-Trp-5Clw-Tap-Glu-leu-[Cys(2Lut)-Apc(AGLU-Glutar)-Leu-Ile-Cys]-APAc-glu(AGLU)-NH₂ (GIP-009) of the following formula 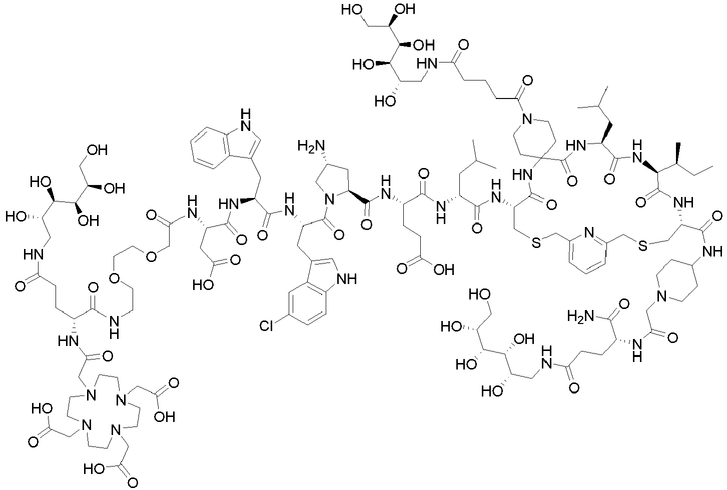
compound AGLU-Glutar-Asp-Trp-5Clw-Hyp-Glu-leu-[Cys(2Lut)-Apc(AGLU-Glutar)-Leu-Ile-Cys]-APAc-Lys(DOTA)-NH₂ (GIP-010) of the following formula 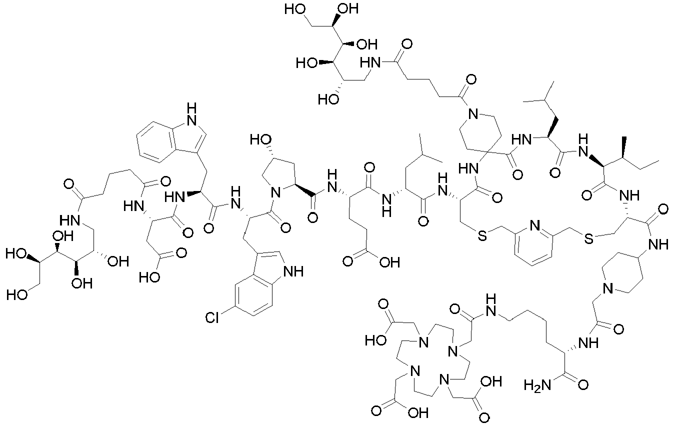
compound AGLU-Glutar-APAc-Asp-Trp-5Clw-Hyp-Glu-leu-[Cys(2Lut)-Apc(DOTA)-Leu-Ile-Cys]-APAc-glu(AGLU)-NH₂ (GIP-011) of the following formula 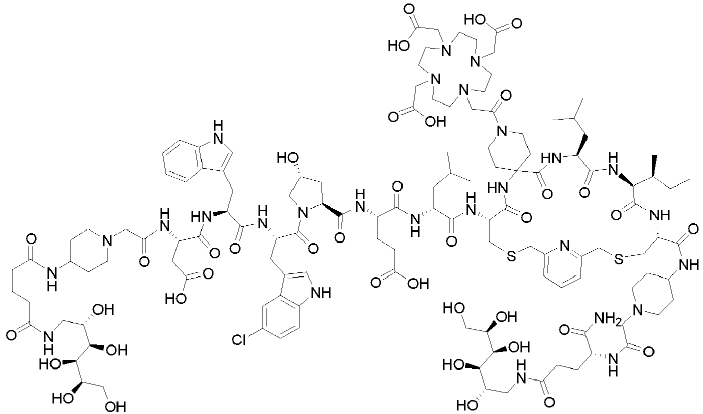
compound DOTA-APAc-Asp-Trp-5Clw-Hyp-Glu-leu-[Cys(2Lut)-Apc(AGLU-Glutar)-Leu-Ile-Cys]-APAc-glu(AGLU)-NH₂ (GIP-012) of the following formula 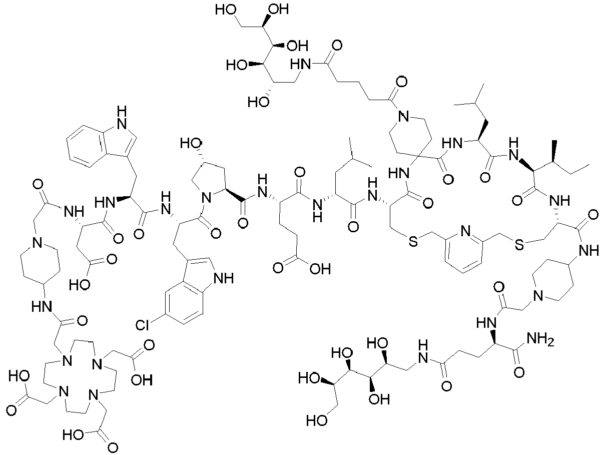
compound AGLU-Glutar-APAc-Asp-Trp-5Clw-Tap-Glu-leu-[Cys(2Lut)-Apc(DOTA)-Leu-Ile-Cys]-APAc-glu(AGLU)-NH₂ (GIP-013) of the following formula 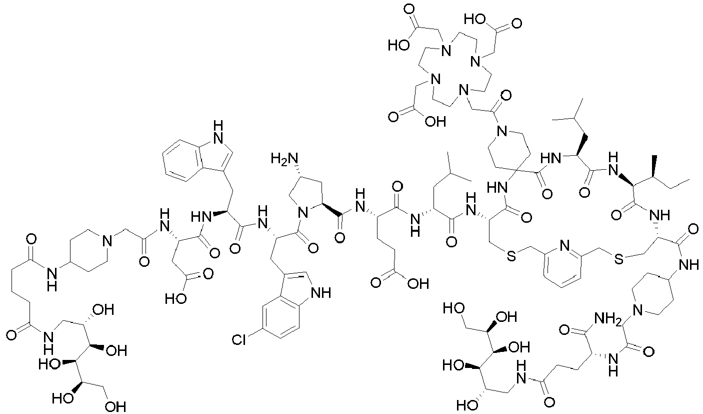
compound AGLU-Glutar-APAc-Asp-Trp-5Brw-Tap-Glu-leu-[Cys(2Lut)-Apc(DOTA)-Leu-Ile-Cys]-APAc-glu(AGLU)-NH₂ (GIP-014) of the following formula 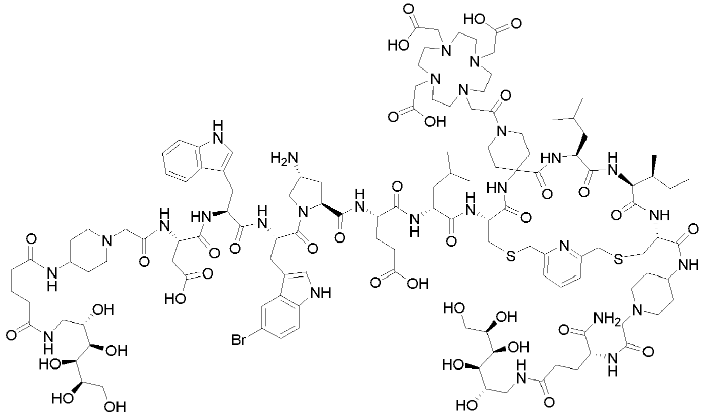
compound AGLU-Glutar-Asp-Trp-5Brw-Tap-Glu-leu-[Cys(2Lut)-Apc(DOTA)-Leu-Ile-Cys]-APAc-glu(AGLU)-NH₂ (GIP-015) of the following formula 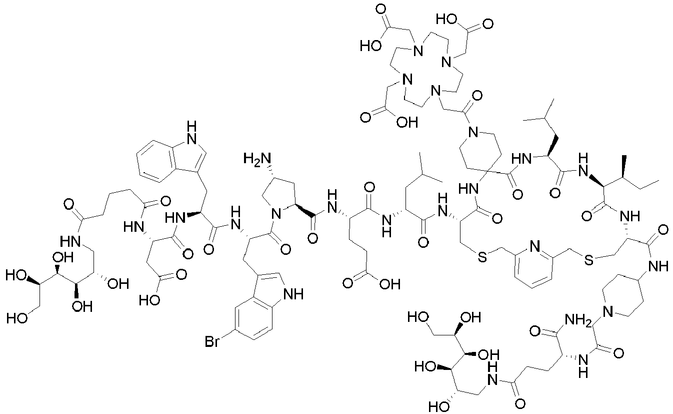
compound AGLU-Glutar-Hse-Trp-5Brw-Tap-Glu-leu-[Cys(2Lut)-Apc(DOTA)-Leu-Ile-Cys]-APAc-glu(AGLU)-NH₂ (GIP-016) of the following formula 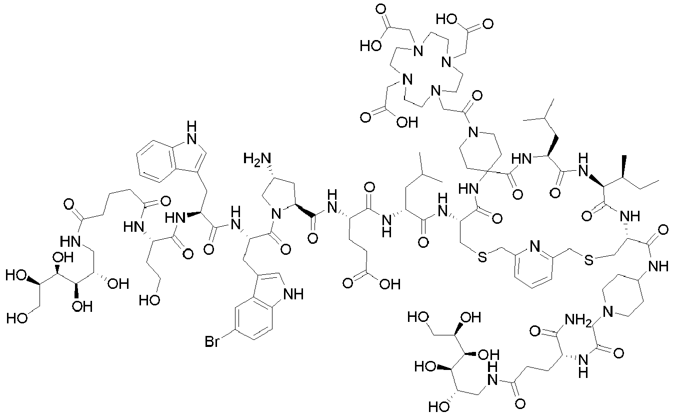
compound DOTA-glu(AGLU)-Asp-Trp-5Clw-Tap-Glu-leu-[Cys(2Lut)-Thp-Leu-Ile-Cys]-APAc-glu(AGLU)-NH₂ (GIP-017) of the following formula 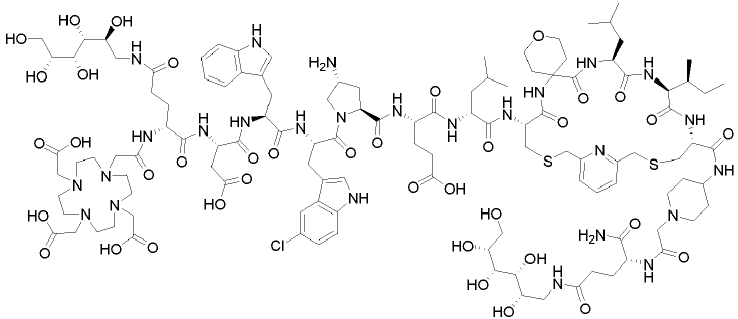
compound DOTA-O2Oc-glu(AGLU)-Asp-Trp-5Clw-Tap-Glu-leu-[Cys(2Lut)-Thp-Leu-Ile-Cys]-APAc-glu(AGLU)-NH₂ (GIP-018) of the following formula 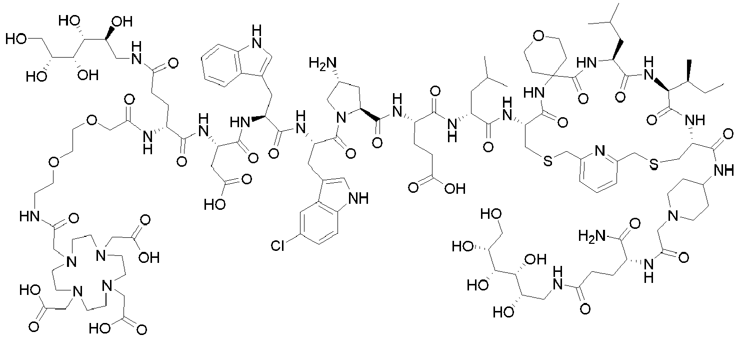
compound DOTA-glu(AGLU)-PEG6-Asp-Trp-5Clw-Tap-Glu-leu-[Cys(2Lut)-Thp-Leu-Ile-Cys]-APAc-glu(AGLU)-NH₂ (GIP-019) of the following formula 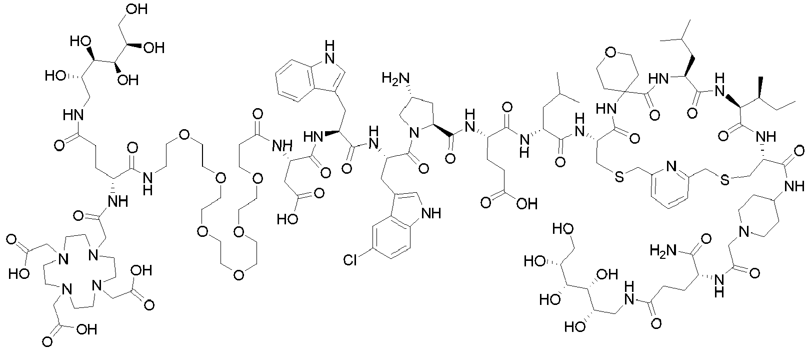
compound DOTA-glu(AGLU)-O2Oc-Asp-Trp-5Clw-Tap-Glu-leu-[Cys(2Lut)-Thp-Leu-Ile-Cys]-APAc-glu(AGLU)-NH₂ (GIP-020) of the following formula 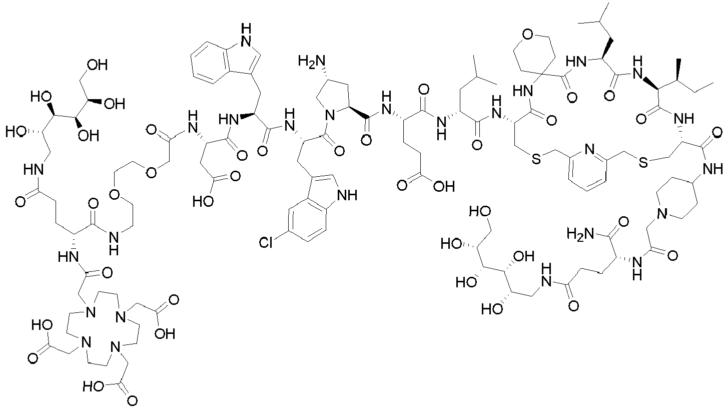
compound HO-Succinyl-Trp-5Clw-Hyp-Glu-leu-[Cys(2Lut)-Thp-Leu-Ile-Cys]-APAc-glu(AGLU)-Lys(DOTA)-NH₂ (GIP-021) of the following formula 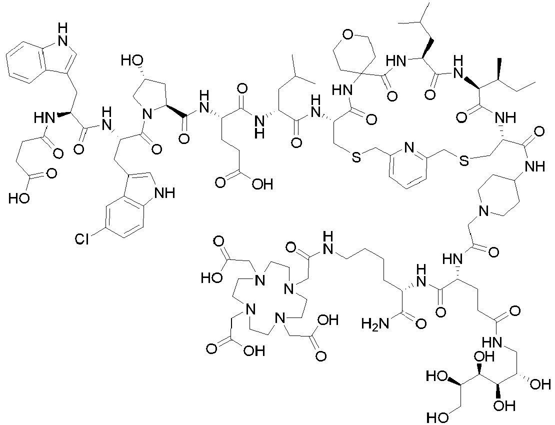
compound HO-Succinyl-Trp-5Clw-Tap-Glu-leu-[Cys(2Lut)-Apc-Leu-Ile-Cys]-APAc-glu(AGLU)-Lys(DOTA)-NH₂ (GIP-022) of the following formula 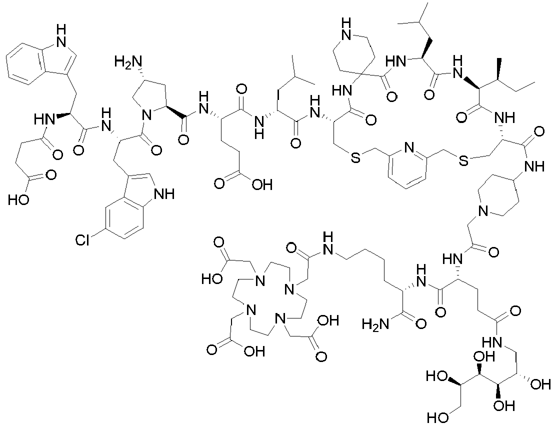
compound HO-Succinyl-Trp-5Clw-Tap-Glu-leu-[Cys(2Lut)-Apc(DOTA)-Leu-Ile-Cys]-APAc-glu(AGLU)-NH₂ (GIP-023) of the following formula 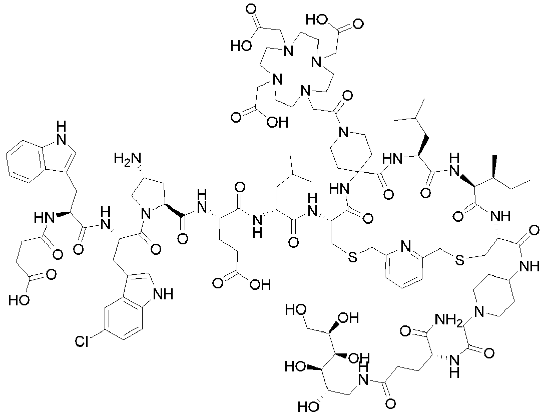
compound AGLU-Glutar-Asp-Trp-5Clw-Tap-Glu-leu-[Cys(2Lut)-Lys(DOTA)-Leu-Ile-Cys]-APAc-glu(AGLU)-NH₂ (GIP-024) of the following formula 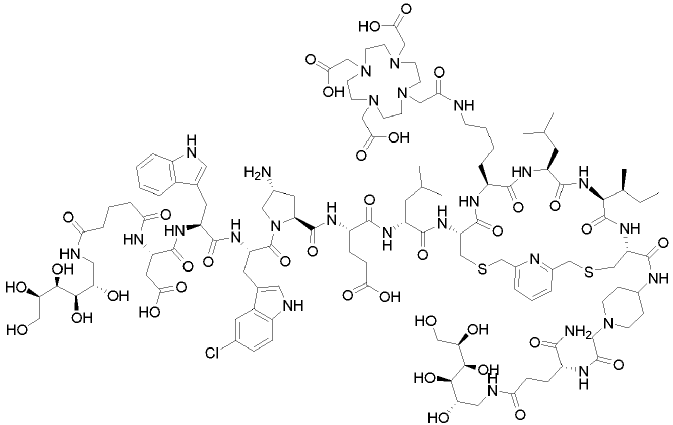
compound AGLU-Glutar-Asp-Trp-5Clw-Tap-Glu-leu-[Cys(2Lut)-lys(DOTA)-Leu-Ile-Cys]-APAc-glu(AGLU)-NH₂ (GIP-025) of the following formula 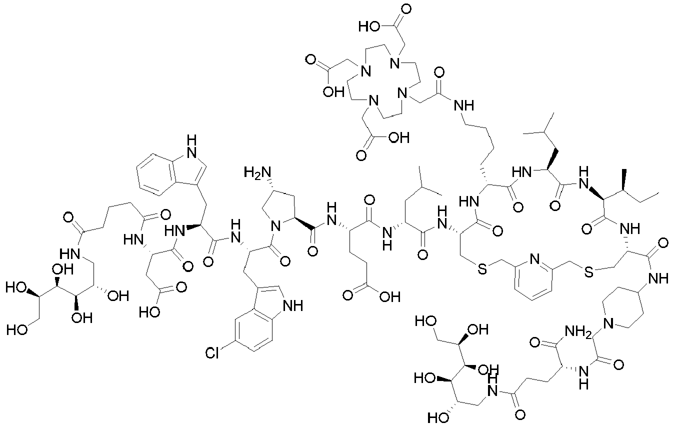
compound AGLU-Glutar-Asp-Trp-5Clw-Tap-Glu-leu-[Cys(2Lut)-Dab(DOTA)-Leu-Ile-Cys]-APAc-glu(AGLU)-NH₂ (GIP-026) of the following formula 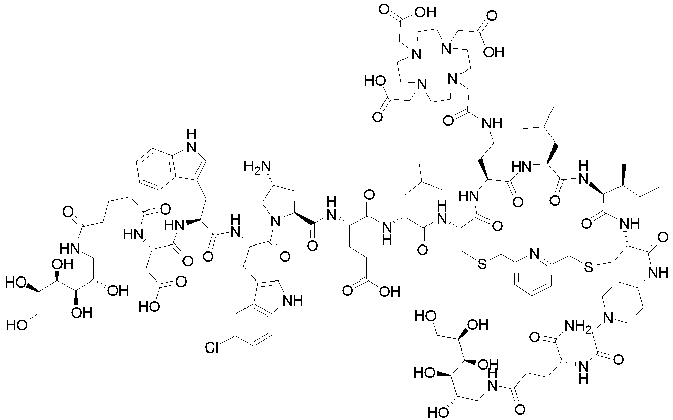
compound AGLU-Glutar-Asp-Trp-5Clw-Tap-Glu-leu-[Cys(2Lut)-dab(DOTA)-Leu-Ile-Cys]-APAc-glu(AGLU)-NH₂(GIP-027) of the following formula 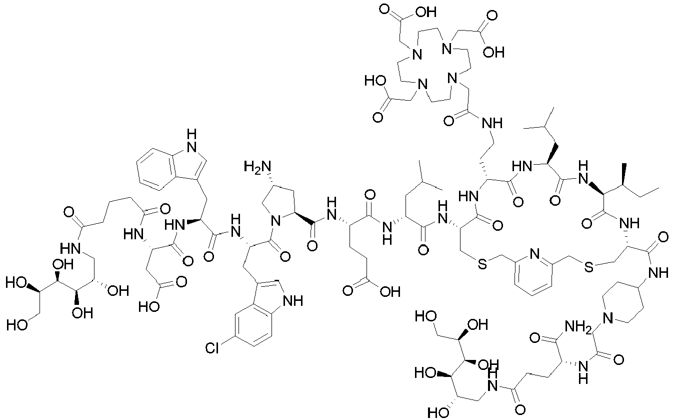
compound AGLU-Glutar-Asp-Trp-5Clw-Tap-Glu-leu-[Cys(2Lut)-Apc(NODAGA)-Leu-Ile-Cys]-APAc-glu(AGLU)-NH₂ (GIP-028) of the following formula 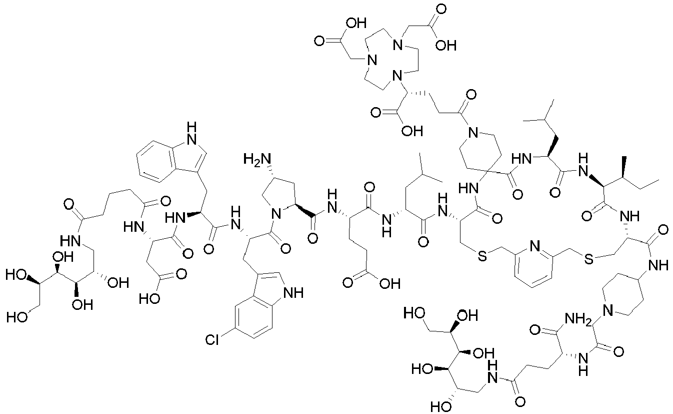
compound AGLU-Glutar-Asp-Trp-5Clw-Tap-Glu-leu-[Cys(2Lut)-Apc(NOPO)-Leu-Ile-Cys]-APAc-glu(AGLU)-NH₂ (GIP-029) of the following formula 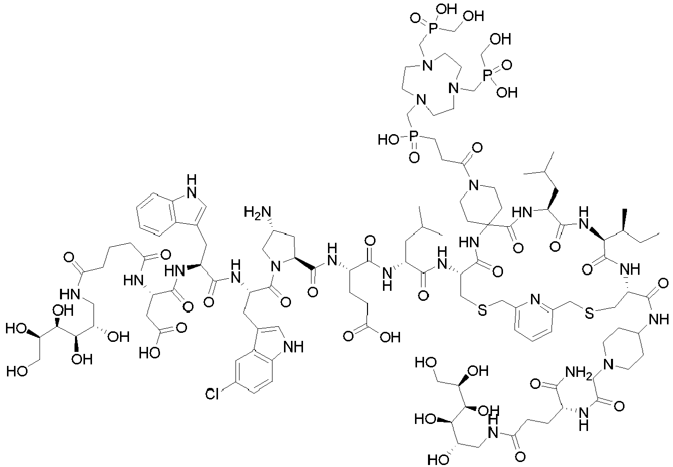
compound AGLU-Glutar-Asp-Trp-5Clw-Tap-Glu-leu-[Cys(2Lut)-Apc(N4Ac)-Leu-Ile-Cys]-APAc-glu(AGLU)-NH₂ (GIP-030) of the following formula 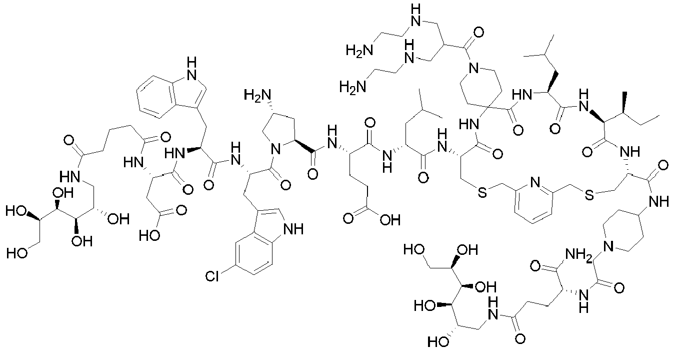
compound AGLU-Glutar-Asp-Trp-5Clw-Tap-Glu-leu-[Cys(2Lut)-Apc(NODAGA-Gly)-Leu-Ile-Cys]-APAc-glu(AGLU)-NH₂ (GIP-031) of the following formula 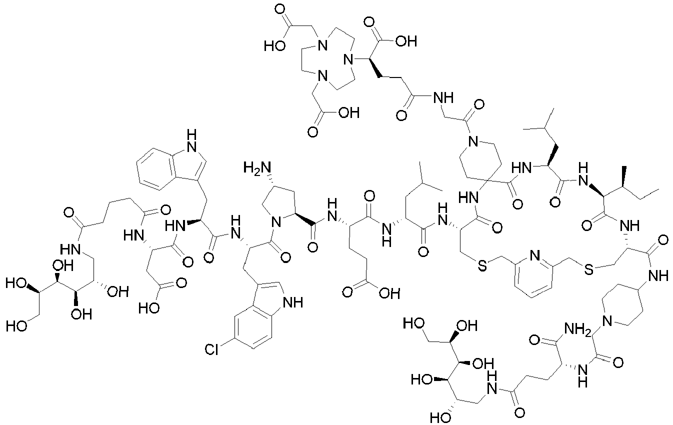
compound HO-Succinyl-Trp-5Clw-Hyp-Glu-leu-[Cys(2Lut)-Thp-Leu-Ile-Cys]-APAc-glu(AGLU)-glu(AGLU)-Lys(DOTA)-NH₂ (GIP-032) of the following formula 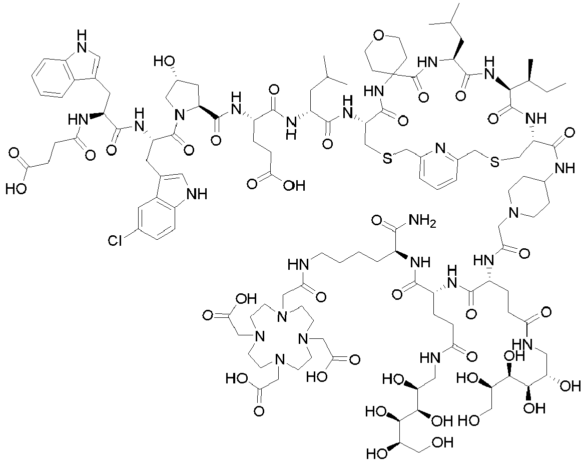
compound AGLU-Glutar-APAc-Asp-Trp-5Clw-Hyp-Glu-leu-[Cys(2Lut)-Apc(DOTA)-Leu-Ile-Cys]-glu(AGLU)-NH₂ (GIP-033) of the following formula 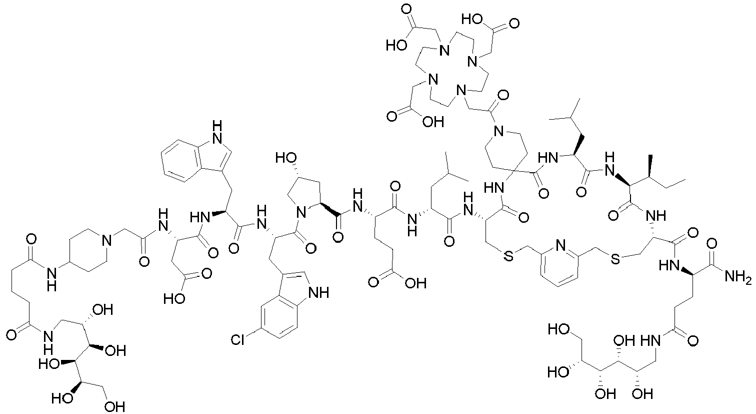
compound AGLU-Glutar-Hse-Trp-5Clw-Tap-Glu-leu-[Cys(2Lut)-Apc(DOTA)-Leu-Ile-Cys]-APAc-glu(AGLU)-NH₂ (GIP-034) of the following formula 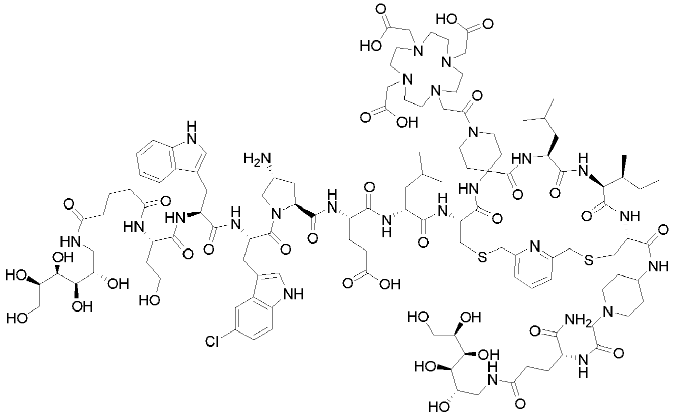
compound AGLU-Glutar-Asp-Trp-5Clw-Tap-Glu-Leu-[Cys(2Lut)-Apc(DOTA)-Leu-Ile-Cys]-APAc-glu(AGLU)-NH₂ (GIP-035) of the following formula 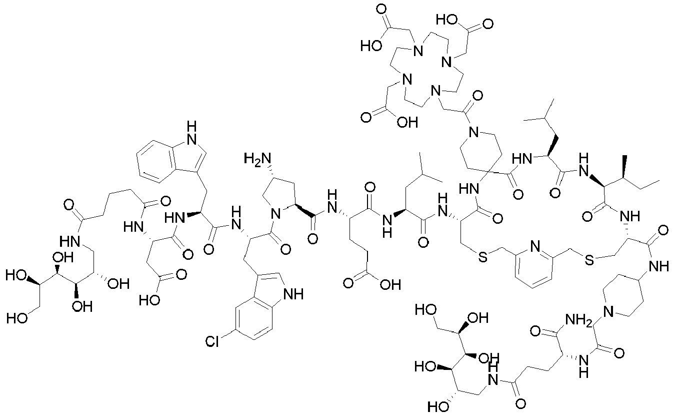
compound DOTA-glu(AGLU)-Asp-Trp-5Clw-Hyp-Glu-leu-[Cys(2Lut)-Thp-Leu-Ile-Cys]-APAc-glu(AGLU)-NH₂ (GIP-036) of the following formula 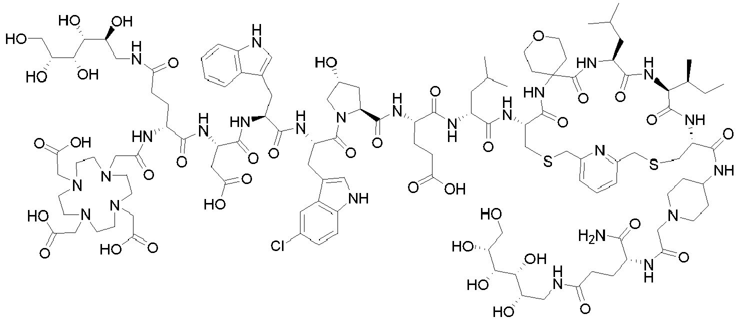
compound DOTA-PEG6-glu(AGLU)-Asp-Trp-5Clw-Tap-Glu-leu-[Cys(2Lut)-Thp-Leu-Ile-Cys]-APAc-glu(AGLU)-NH₂ (GIP-037) of the following formula 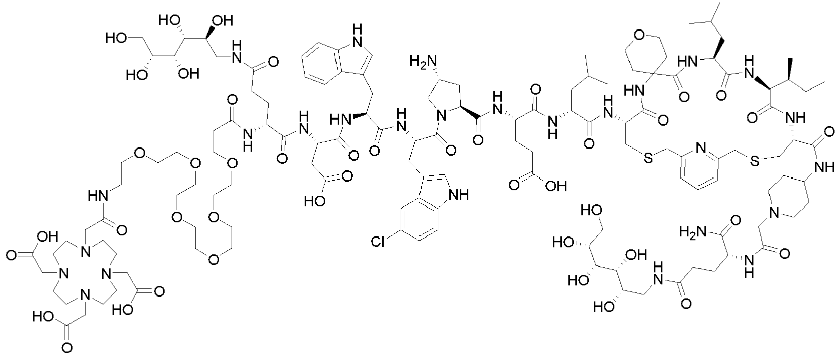
compound AGLU-Glutar-Asp-Trp-5Clw-Tap-Glu-leu-[Cys(2Lut)-Apc(DOTA)-Leu-Ile-cys]-APAc-glu(AGLU)-NH₂ (GIP-038) of the following formula 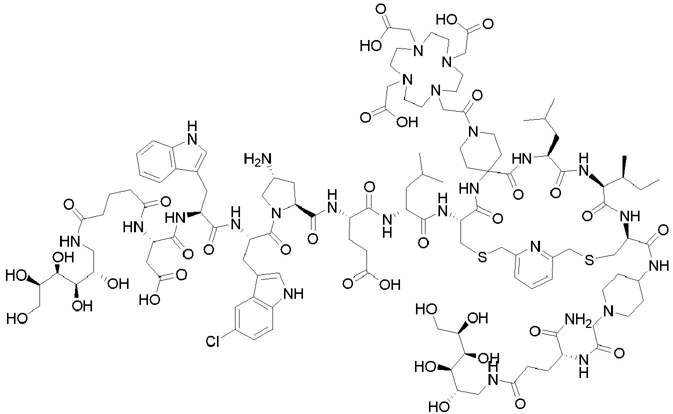
compound AGLU-Glutar-APAc-Asp-Trp-5Clw-Hyp-Glu-leu-[Cys(2Lut)-Apc(DOTA)-Leu-Ile-Cys]-lys(MeO36PEG)-NH₂ (GIP-039) of the following formula 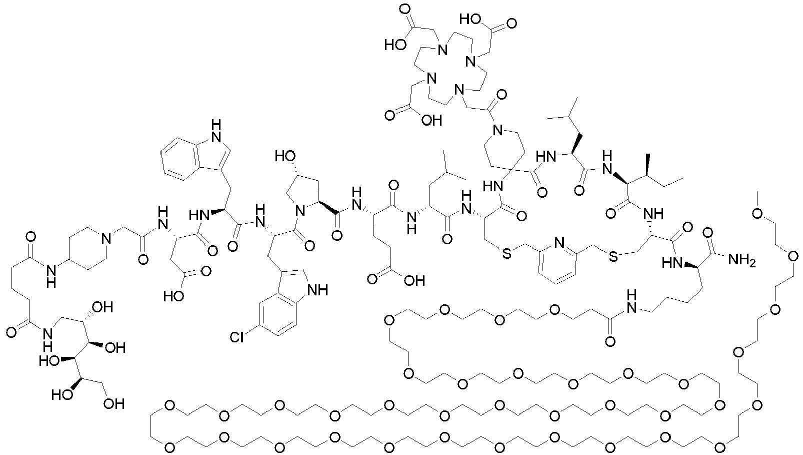
compound MeO36PEG-APAc-Asp-Trp-5Clw-Hyp-Glu-leu-[Cys(2Lut)-Apc(DOTA)-Leu-Ile-Cys]-glu(AGLU)-NH₂ (GIP-040) of the following formula 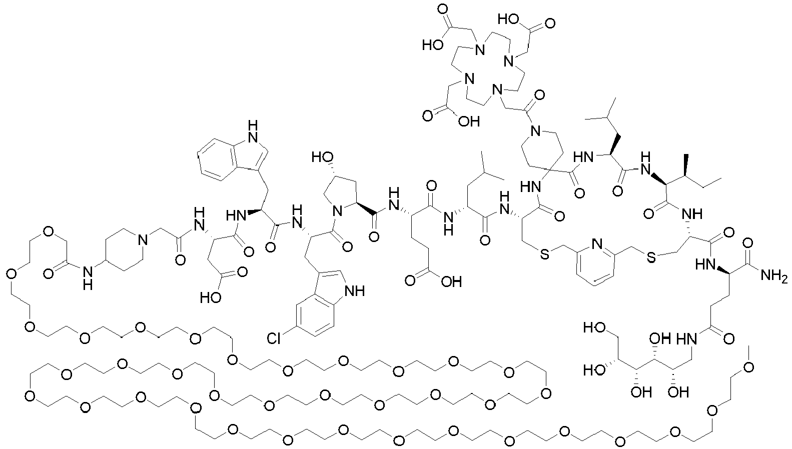
compound MeO36PEG-APAc-Asp-Trp-5Clw-Hyp-Glu-leu-[Cys(2Lut)-Apc(DOTA)-Leu-Ile-Cys]-APAc-glu(AGLU)-NH₂ (GIP-041) of the following formula 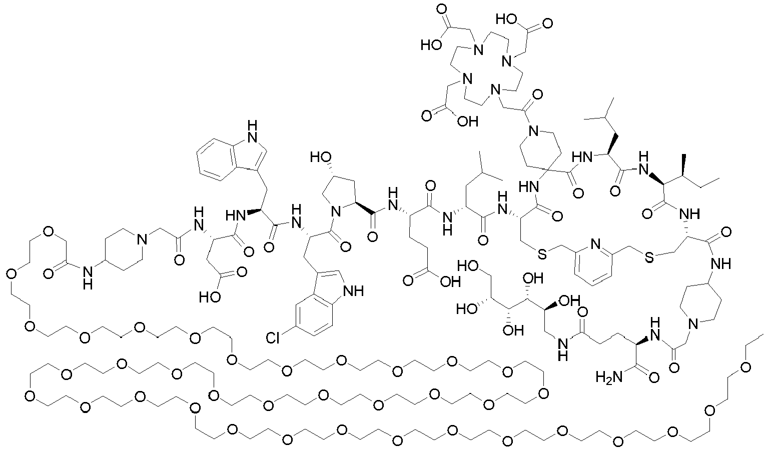
compound MeO36PEG-APAc-Asp-Trp-5Clw-Tap-Glu-leu-[Cys(2Lut)-Apc(DOTA)-Leu-Ile-Cys]-APAc-glu(AGLU)-NH₂ (GIP-042) of the following formula 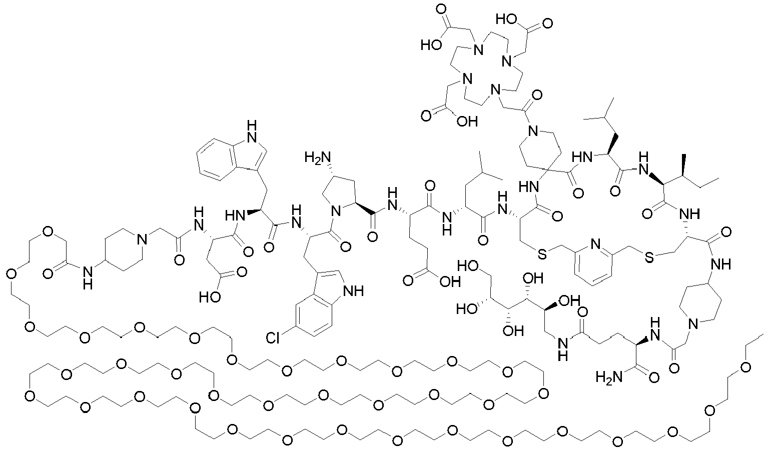
compound AGLU-Glutar-APAc-Asp-Trp-5Clw-Hyp-Glu-leu-[Cys(2Lut)-Apc(DOTA-glu(AGLU))-Leu-Ile-Cys]-APAc-glu(AGLU)-NH₂ (GIP-043) of the following formula 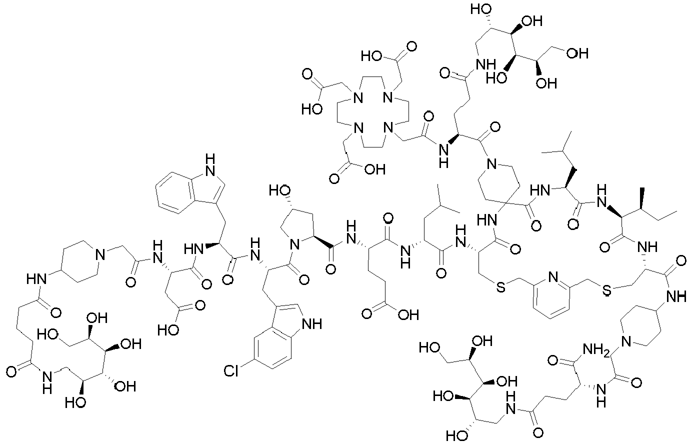
compound DOTA-O2Oc-glu(AGLU)-Asp-Trp-5Clw-Tap-Glu-leu-[Cys(2Lut)-Apc(AGLU-Glutar)-Leu-Ile-Cys]-APAc-glu(AGLU)-NH₂ (GIP-044) of the following formula 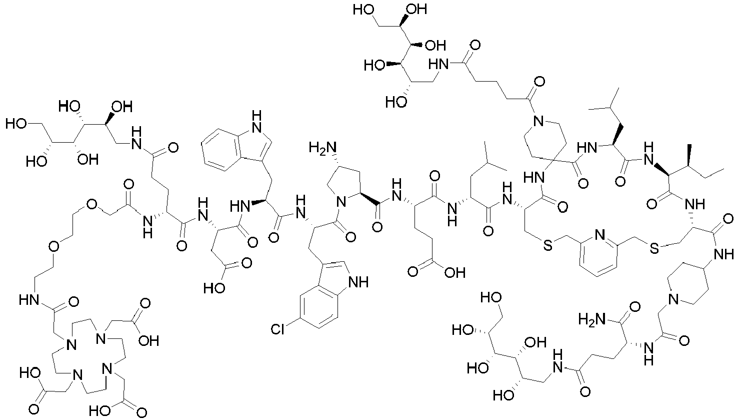
compound DOTA-glu(AGLU)-Asp-Trp-5Clw-Tap-Glu-leu-[Cys(2Lut)-Apc(AGLU-Glutar)-Leu-Ile-Cys]-APAc-glu(AGLU)-NH₂ (GIP-045) of the following formula 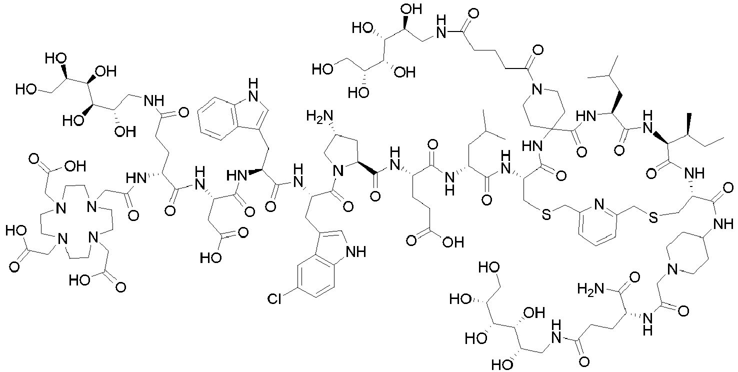
compound AGLU-Glutar-APAc-Ser-Trp-5Clw-Tap(AGLU-Glutar)-Hse-leu-[Cys(2Lut)-Apc(DOTA)-Leu-Ile-Cys]-APAc-glu(AGLU)-NH₂ (GIP-046) of the following formula 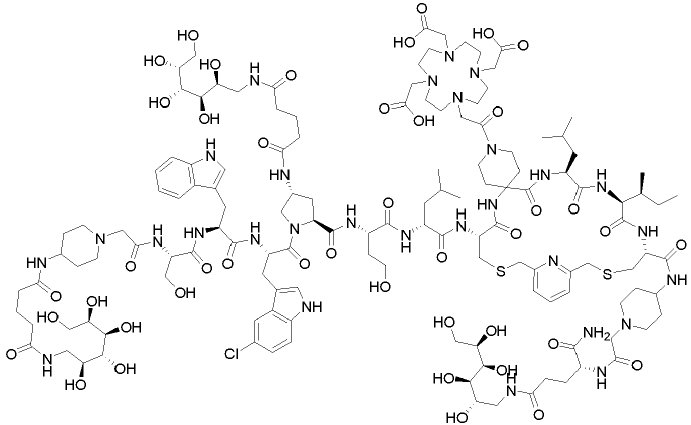
compound AGLU-Glutar-APAc-Hse-Trp-5Clw-Hyp-Glu-leu-[Cys(2Lut)-Apc(DOTA-glu(AGLU))-Leu-Ile-Cys]-APAc-glu(AGLU)-NH₂ (GIP-047) of the following formula 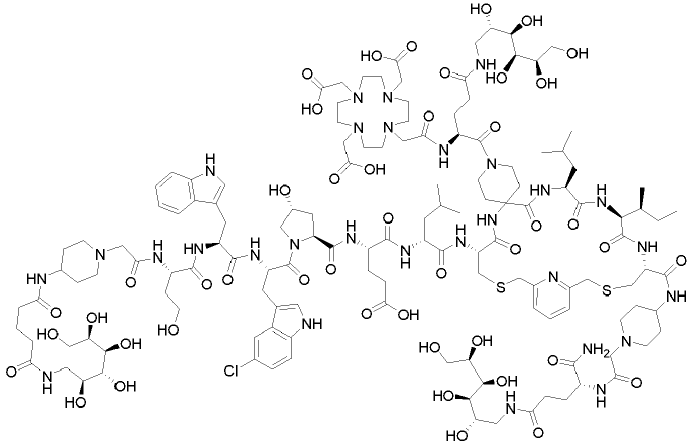
compound AGLU-Glutar-APAc-Ser-Trp-5Clw-Tap-Hse-leu-[Cys(2Lut)-Apc(DOTA)-Leu-Ile-Cys]-APAc-glu(AGLU)-NH₂ (GIP-048) of the following formula 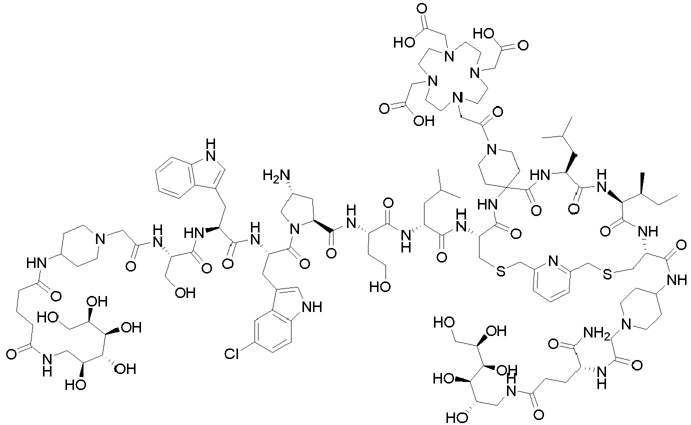
compound AGLU-Glutar-APAc-Hse-Trp-5Clw-Hyp-Glu-leu-[Cys(2Lut)-Apc(DOTA)-Leu-Ile-Cys]-APAc-glu(AGLU)-NH₂ (GIP-049) of the following formula 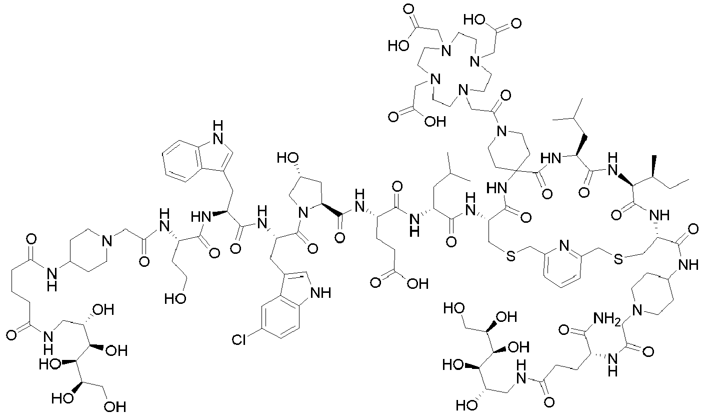
compound AGLU-Glutar-APAc-Hse-Trp-5Clw-Tap-Glu-leu-[Cys(2Lut)-Apc(DOTA)-Leu-Ile-Cys]-APAc-glu(AGLU)-NH₂ (GIP-050) of the following formula 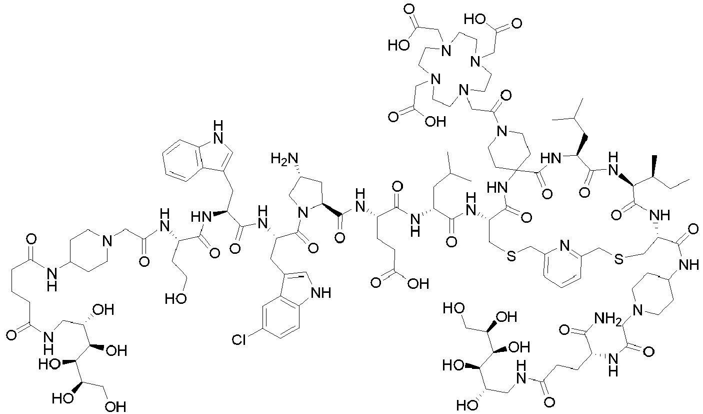
compound AGLU-Glutar-Asp-Trp-5Clw-Tap-Glu-leu-[cys(2Lut)-Apc(DOTA)-Leu-Ile-Cys]-APAc-glu(AGLU)-NH₂ (GIP-051) of the following formula 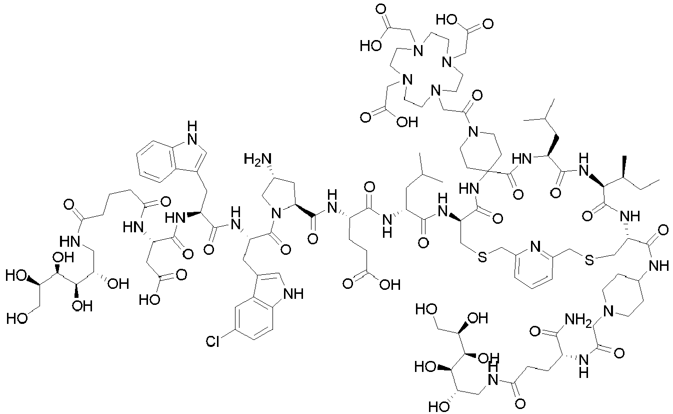
compound AGLU-Glutar-Asp-Trp-5Clw-Tap-Glu-leu-[cys(2Lut)-Apc(DOTA)-Leu-Ile-cys]-APAc-glu(AGLU)-NH₂ (GIP-052) of the following formula 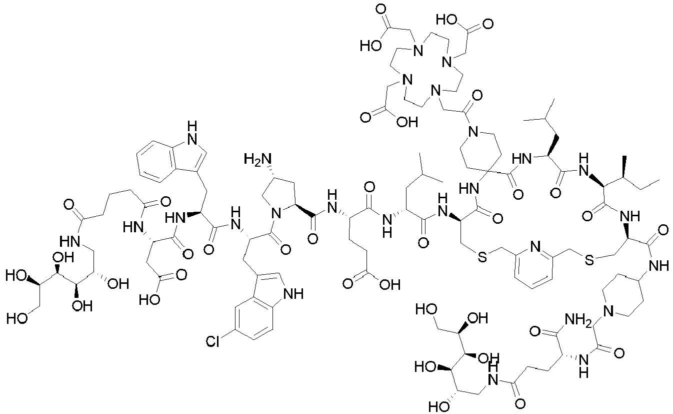
compound DOTA-Ttds-Asp-Trp-5Clw-Pro-Glu-leu-[Cys(3MeBn)-Apc(DOTA)-Leu-Ile-AET] (GIP-053) of the following formula 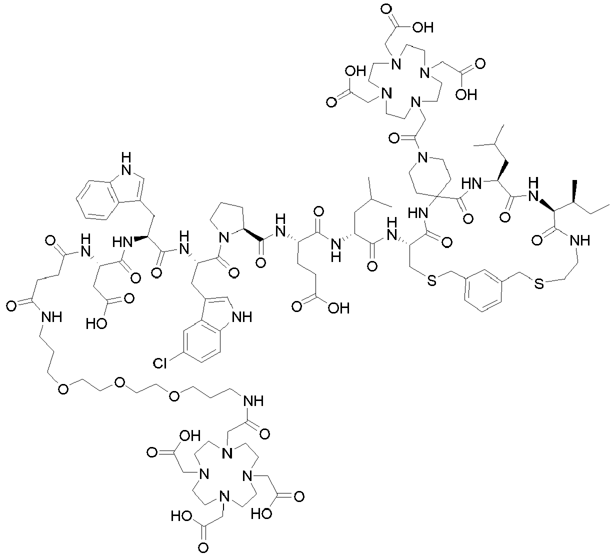
compound DOTA-Ttds-Asp-Trp-5Clw-Hyp-Glu-leu-[Cys(2Lut)-Thp-Leu-Ile-Cys]-Lys(DOTA)-NH₂ (GIP-054) of the following formula 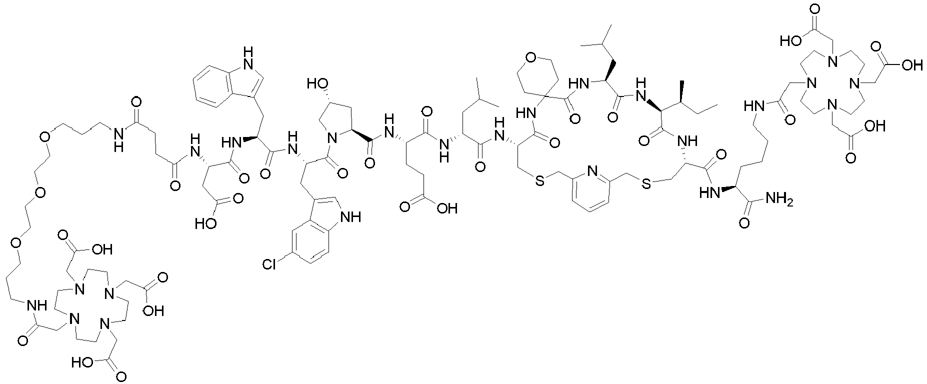
compound DOTA-Ttds-Asp-Trp-5Clw-Hyp-Glu-leu-[Cys(2Lut)-Apc(DOTA)-Leu-Ile-Cys]-Lys(DOTA)-NH₂ (GIP-055) of the following formula 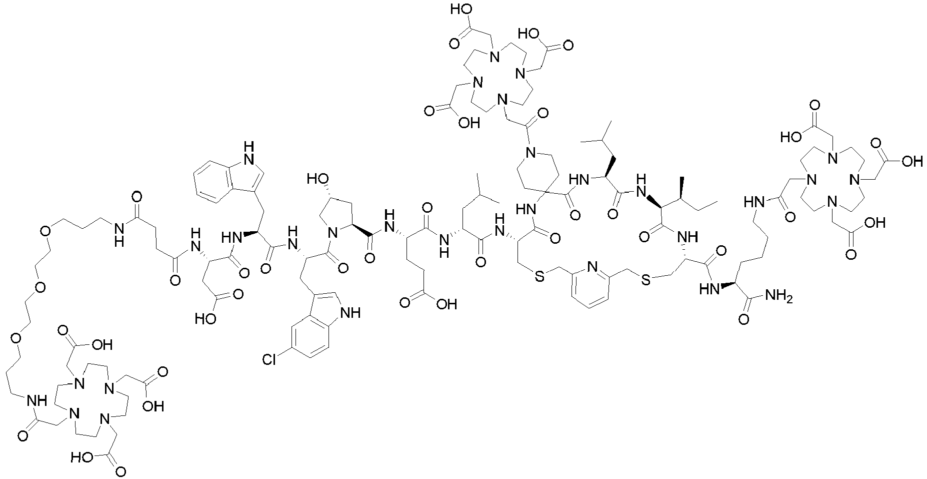
compound DOTA-Ttds-Asp-Trp-5Clw-Tap(DOTA)-Glu-leu-[Cys(2Lut)-Apc(DOTA)-Leu-Ile-Cys]-Lys(DOTA)-NH₂ (GIP-056) of the following formula 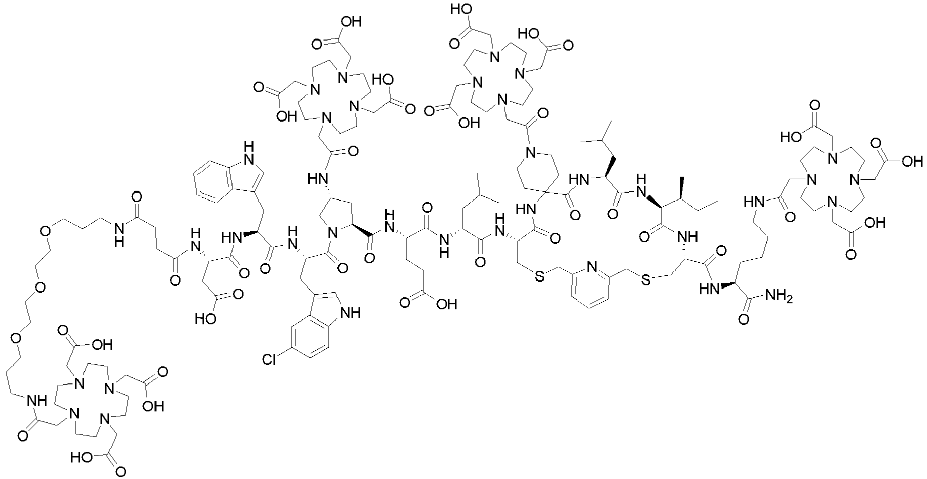
compound H-Met-Tyr-Asp-Trp-Trp-Pro-Glu-Leu-[Cys(3MeBn)-Ala-Leu-Ile-Cys]-Ttds-Lys(Bio)-NH₂ (GIP-057) of the following formula 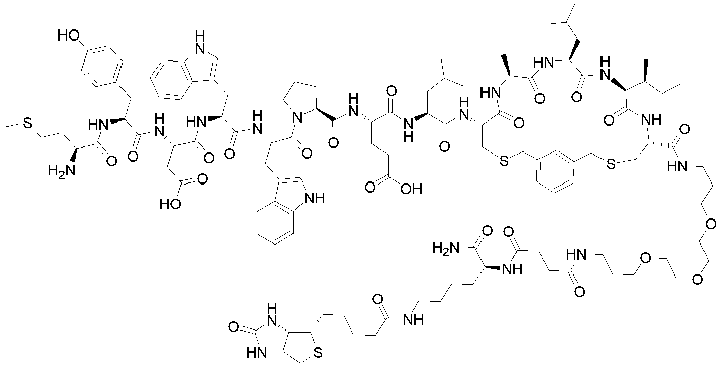
compound H-Met-Tyr-Asp-Trp-Trp-Pro-Glu-Leu-[Cys(3MeBn)-Ala-Leu-Ile-Cys]-Ttds-Lys(Cy5SO3)-NH₂ (GIP-058) of the following formula 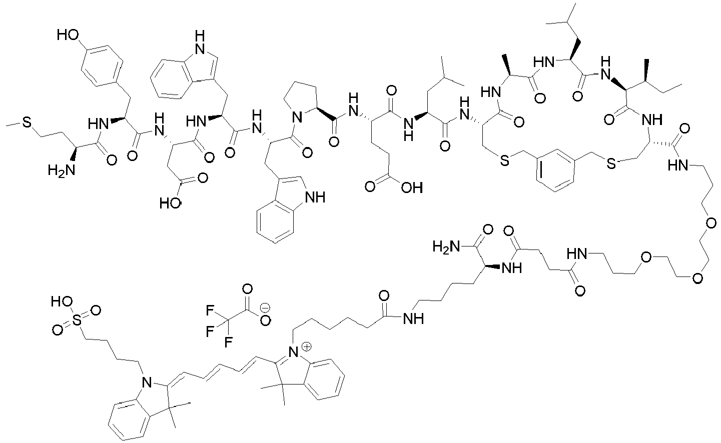
compound AGLU-Glutar-APAc-Asp-Trp-5Clw-Tap-Glu-leu-[Cys(2Lut)-Apc(GaDOTA-glu(AGLU))-Leu-Ile-Cys]-APAc-glu(AGLU)-NH₂ (GIP-059) of the following formula 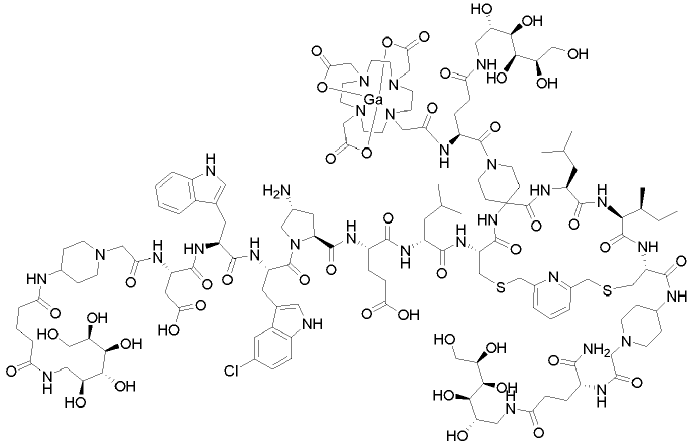
compound AGLU-Glutar-APAc-Asp-Trp-5Clw-Tap-Glu-leu-[Cys(2Lut)-Apc(InDOTA-glu(AGLU))-Leu-Ile-Cys]-APAc-glu(AGLU)-NH₂ (GIP-060) of the following formula 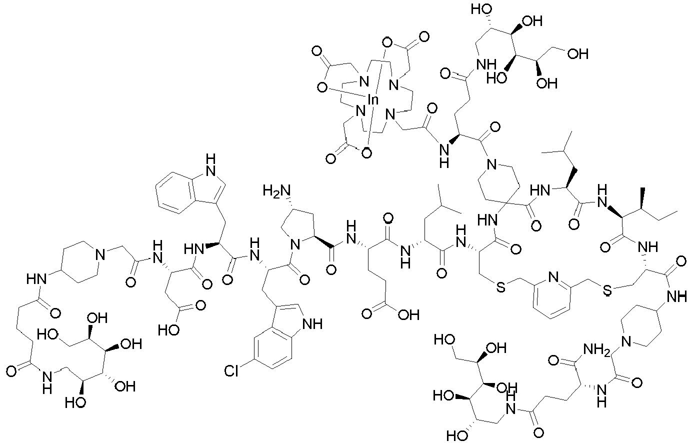
compound AGLU-Glutar-APAc-Asp-Trp-5Clw-Tap-Glu-leu-[Cys(2Lut)-Apc(LuDOTA-glu(AGLU))-Leu-Ile-Cys]-APAc-glu(AGLU)-NH₂ (GIP-061) of the following formula 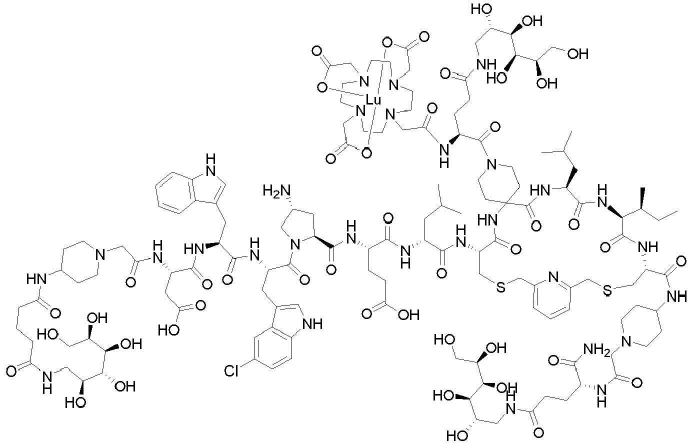
compound LuDOTA-glu(AGLU)-APAc-Asp-Trp-5Clw-Hyp-Glu-leu-[Cys(2Lut)-Thp-Leu-Ile-Cys]-APAc-glu(AGLU)-NH₂ (GIP-062) of the following formula 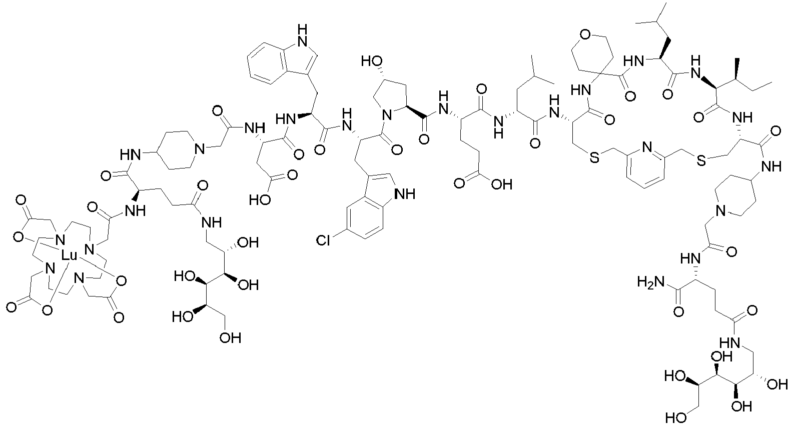
compound InDOTA-glu(AGLU)-APAc-Asp-Trp-5Clw-Hyp-Glu-leu-[Cys(2Lut)-Thp-Leu-Ile-Cys]-APAc-glu(AGLU)-NH₂ (GIP-063) of the following formula 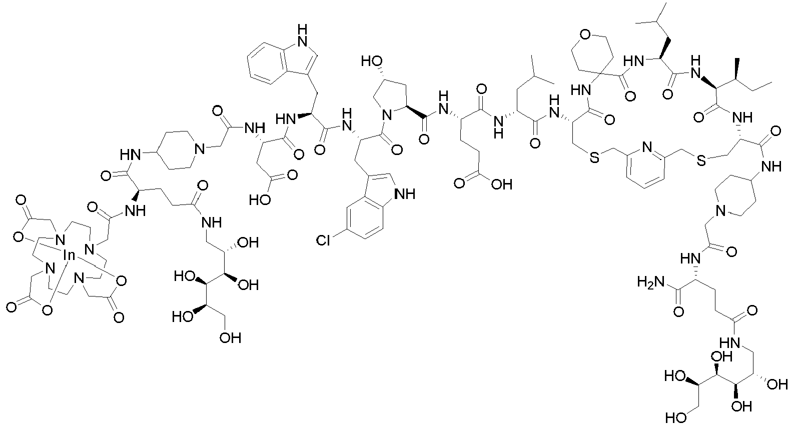
compound EuDOTA-glu(AGLU)-APAc-Asp-Trp-5Clw-Hyp-Glu-leu-[Cys(2Lut)-Thp-Leu-Ile-Cys]-APAc-glu(AGLU)-NH₂ (GIP-064) of the following formula 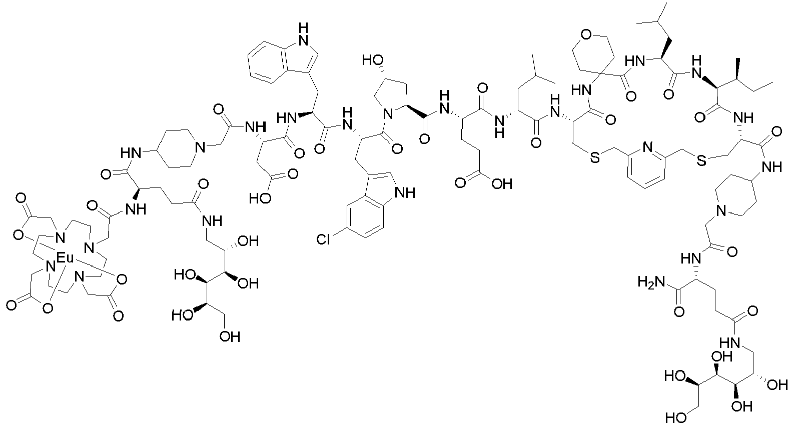
compound AGLU-Glutar-Asp-Trp-5Clw-Hyp-Glu-leu-[Cys(2Lut)-Apc(InDOTA)-Leu-Ile-Cys]-APAc-glu(AGLU)-NH₂ (GIP-065) of the following formula 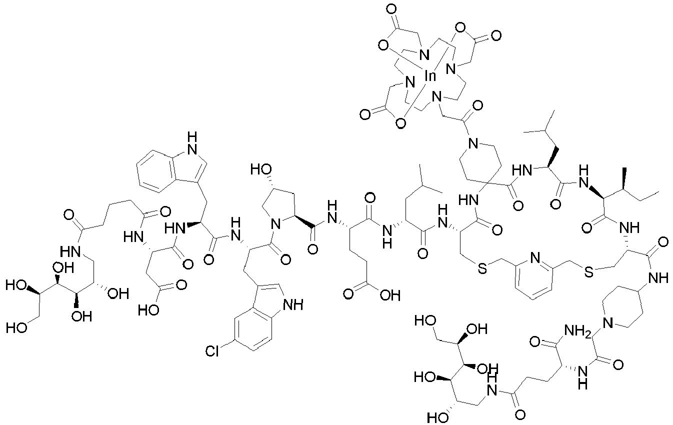
compound AGLU-Glutar-Asp-Trp-5Clw-Hyp-Glu-leu-[Cys(2Lut)-Apc(LuDOTA)-Leu-Ile-Cys]-APAc-glu(AGLU)-NH₂ (GIP-066) of the following formula 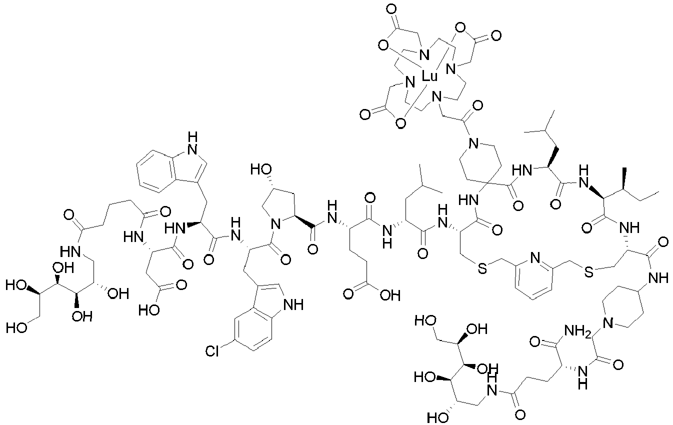
compound AGLU-Glutar-Asp-Trp-5Clw-Tap-Glu-leu-[Cys(2Lut)-Apc(LuDOTA)-Leu-Ile-Cys]-APAc-glu(AGLU)-NH₂ (GIP-067) of the following formula 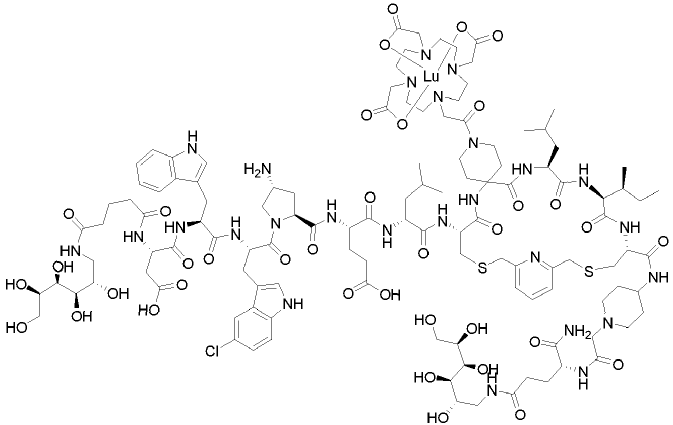
compound AGLU-Glutar-Asp-Trp-5Clw-Tap-Glu-leu-[Cys(2Lut)-Apc(InDOTA)-Leu-Ile-Cys]-APAc-glu(AGLU)-NH₂ (GIP-068) of the following formula 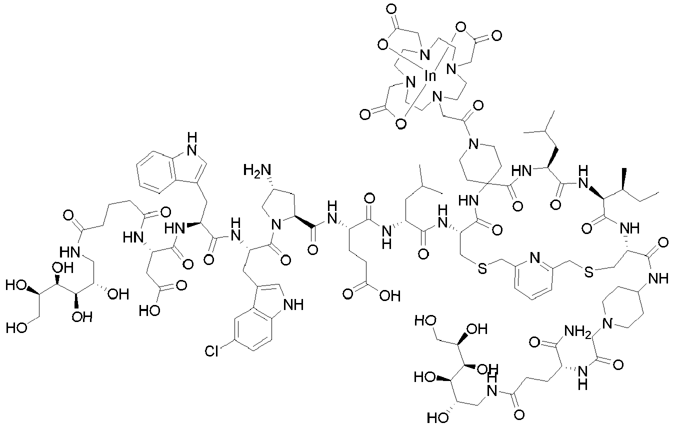
compound AGLU-Glutar-Asp-Trp-5Clw-Tap-Glu-leu-[Cys(2Lut)-Apc(GaDOTA)-Leu-Ile-Cys]-APAc-glu(AGLU)-NH₂ (GIP-069) of the following formula 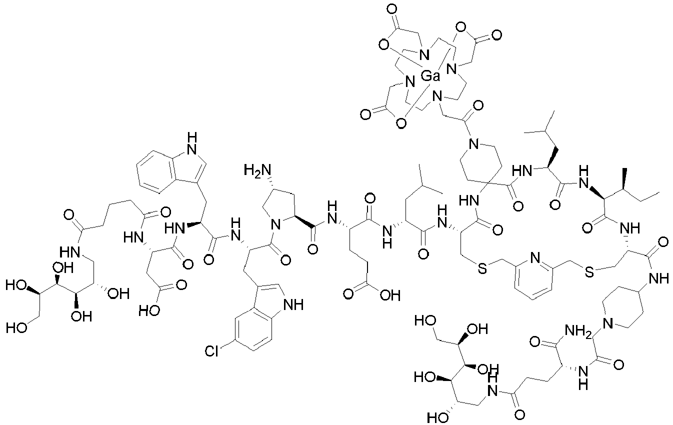
compound AGLU-Glutar-Asp-Trp-5Clw-Hyp-Glu-leu-[Cys(2Lut)-Apc(GaDOTA)-Leu-Ile-Cys]-APAc-glu(AGLU)-NH₂ (GIP-070) of the following formula 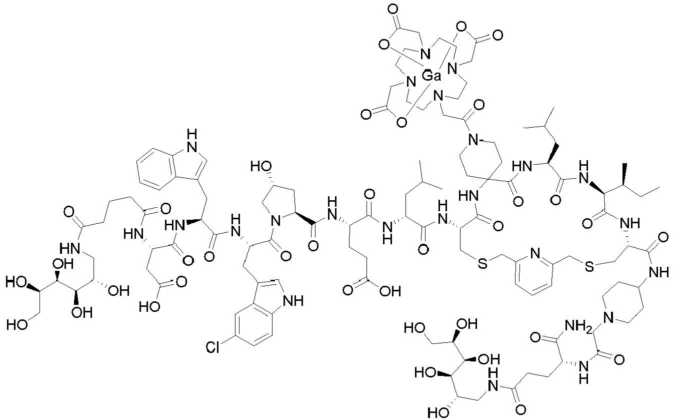
compound AGLU-Glutar-Asp-Trp-5Clw-Tap-Glu-leu-[Cys(2Lut)-Apc(GaNODAGA)-Leu-Ile-Cys]-APAc-glu(AGLU)-NH₂ (GIP-071) of the following formula 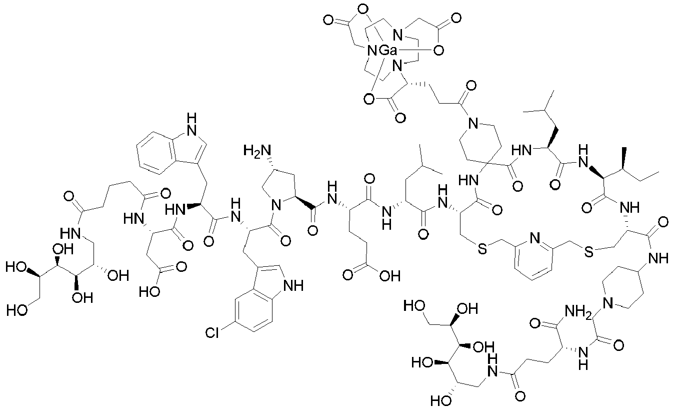
compound AGLU-Glutar-Asp-Trp-5Clw-Hyp-Glu-leu-[Cys(2Lut)-Apc(DOTA)-Leu-Ile-AET] (GIP-072) of the following formula 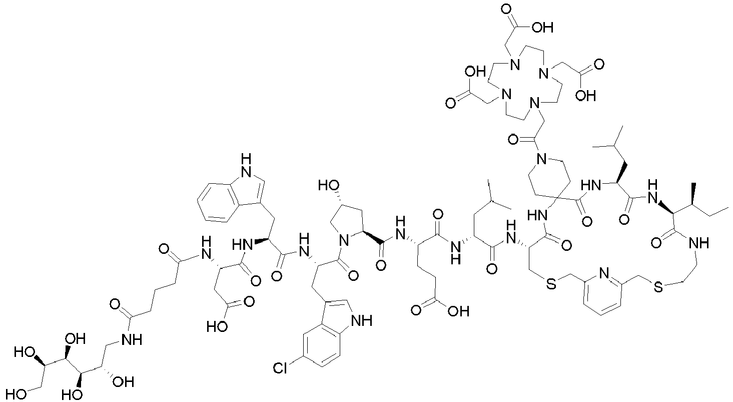
compound HO-Succinyl-Trp-5Clw-Hyp-Glu-leu-[Cys(2Lut)-Apc(AGLU-Glutar)-Leu-Ile-Cys]-APAc-Lys(DOTA)-NH₂ (GIP-073) of the following formula 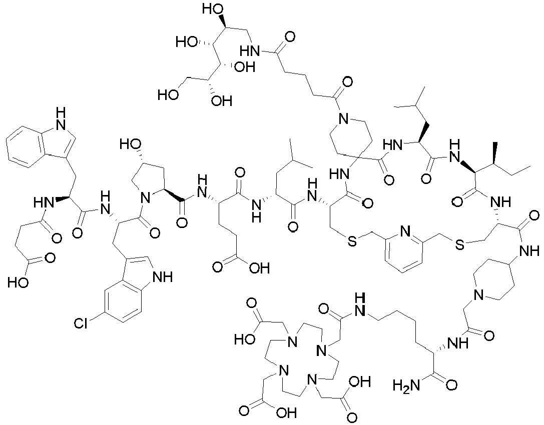
compound Ac-Asp-Trp-1Ni-Pro-Glu-Leu-[Cys(3MeBn)-Aib-Leu-Ile-Cys]-Ttds-Lys(DOTA)-NH₂ (GIP-074) of the following formula 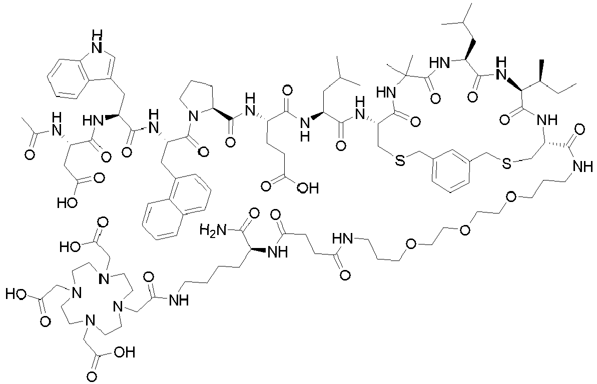
compound Ac-Asp-Trp-Bta-Pro-Glu-Leu-[Cys(3MeBn)-Aib-Leu-Ile-Cys]-Ttds-Lys(DOTA)-NH₂ (GIP-075) of the following formula 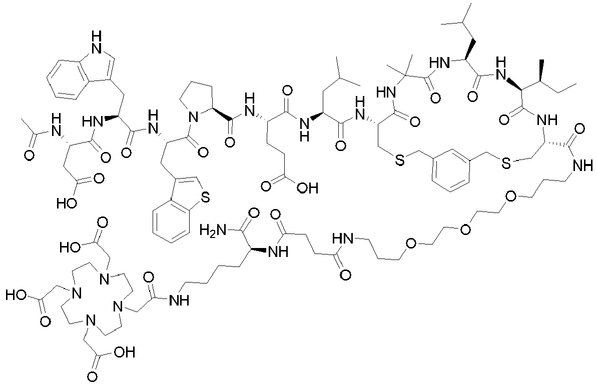
compound Ac-Asp-Trp-5Clw-Pro-Glu-Leu-[Cys(3MeBn)-Aib-Leu-Ile-Cys]-Ttds-Lys(DOTA)-NH₂ (GIP-076) of the following formula 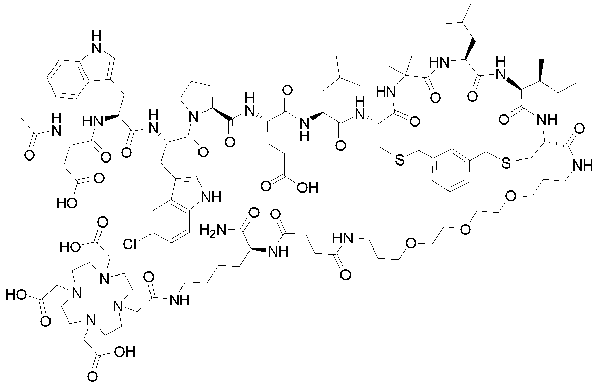
compound DOTA-Ttds-Asp-Trp-5Clw-Pro-Glu-leu-[Cys(3MeBn)-Aib-Leu-Ile-AET] (GIP-077) of the following formula 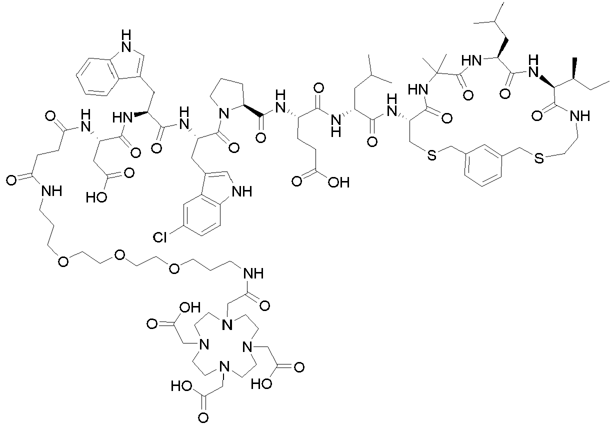
compound Ac-Asp-Trp-1Ni-Pro-Glu-leu-[Cys(3MeBn)-Aib-Leu-Ile-Cys]-Ttds-Lys(DOTA)-NH₂ (GIP-078) of the following formula 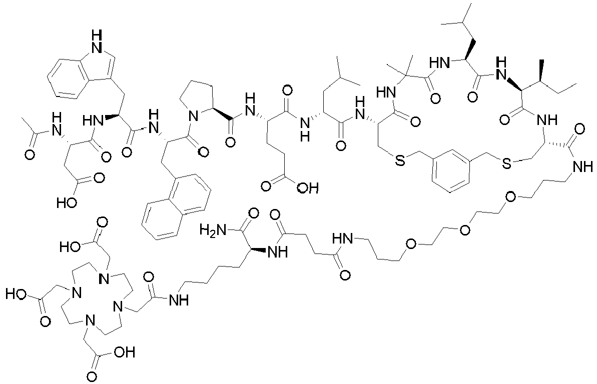
compound Ac-Asp-Trp-5Clw-Pro-Glu-leu-[Cys(3MeBn)-Aib-Leu-Ile-Cys]-Ttds-Lys(DOTA)-NH₂ (GIP-079) of the following formula 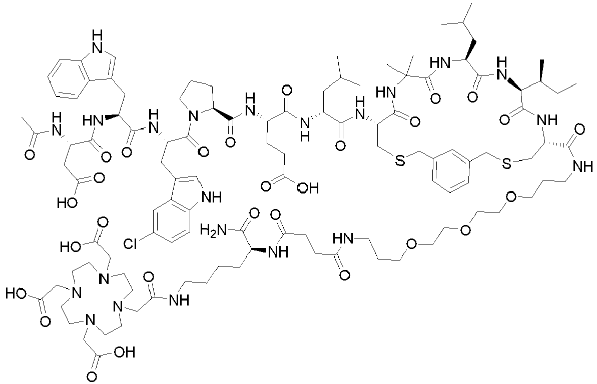
compound Ac-Asp-Trp-1Ni-Pro-Glu-leu-[Cys(3MeBn)-Egz-Leu-Ile-Cys]-Ttds-Lys(DOTA)-NH₂ (GIP-080) of the following formula 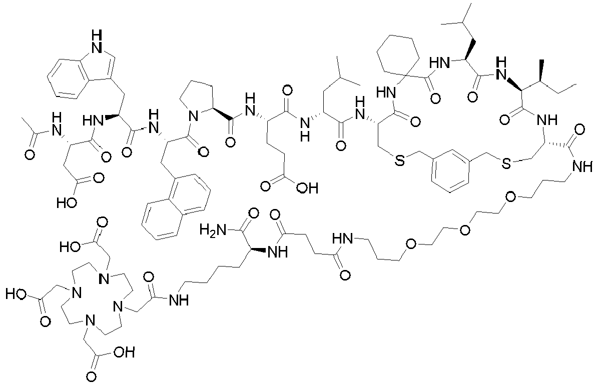
compound Ac-Asp-Trp-5Fw-Pro-Glu-leu-[Cys(3MeBn)-Aib-Leu-Ile-Cys]-Ttds-Lys(DOTA)-NH₂ (GIP-081) of the following formula 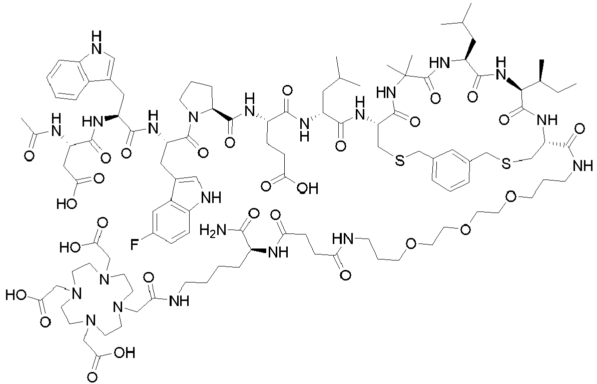
compound Ac-Asp-Trp-5Brw-Pro-Glu-leu-[Cys(3MeBn)-Aib-Leu-Ile-Cys]-Ttds-Lys(DOTA)-NH₂ (GIP-082) of the following formula 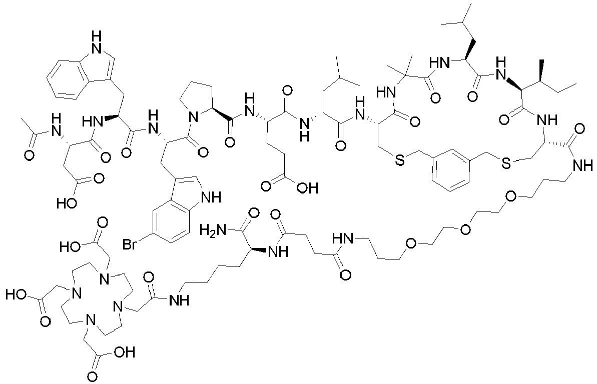
compound Ac-Asp-5Fw-1Ni-Pro-Glu-leu-[Cys(3MeBn)-Aib-Leu-Ile-Cys]-Ttds-Lys(DOTA)-NH₂ (GIP-083) of the following formula 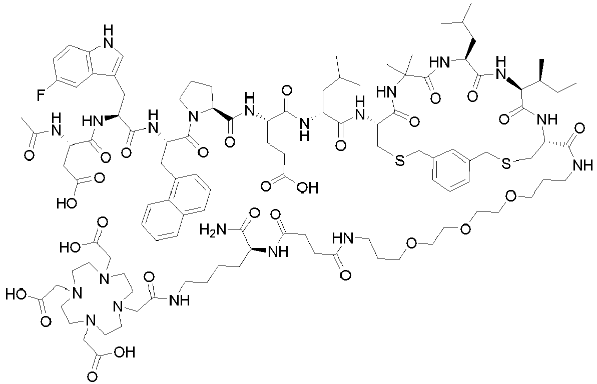
compound Ac-Asp-6Fw-1Ni-Pro-Glu-leu-[Cys(3MeBn)-Aib-Leu-Ile-Cys]-Ttds-Lys(DOTA)-NH₂ (GIP-084) of the following formula 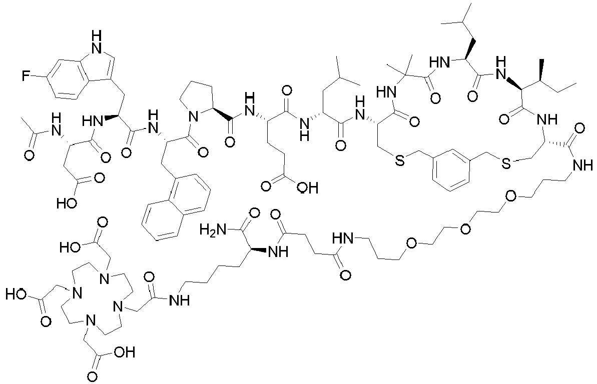
compound HO-Succinyl-Trp-1Ni-Pro-Glu-Leu-[Cys(3MeBn)-Aib-Leu-Ile-Cys]-Ttds-Lys(DOTA)-NH₂ (GIP-085) of the following formula 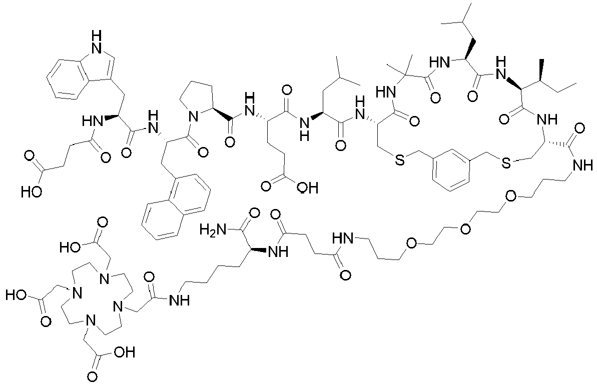
compound DOTA-Ttds-Asp-Trp-5Clw-Pro-Glu-leu-[Cys(3MeBn)-Egz-Leu-Ile-AET] (GIP-086) of the following formula 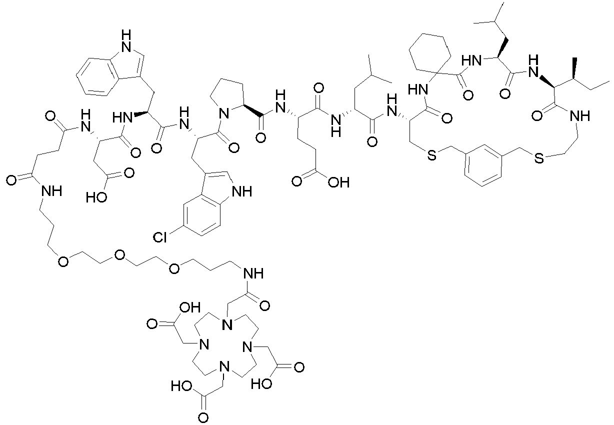
compound DOTA-Ttds-Asp-Trp-5Clw-Pro-Glu-leu-[Cys(3MeBn)-Thp-Leu-Ile-AET] (GIP-087) of the following formula 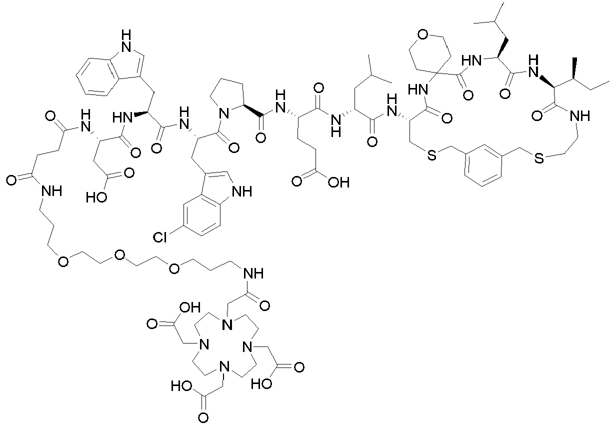
compound DOTA-Ttds-Asp-Trp-5Clw-Pro-Glu-leu-[Cys(3MeBn)-Apc-Leu-Ile-AET] (GIP-088) of the following formula 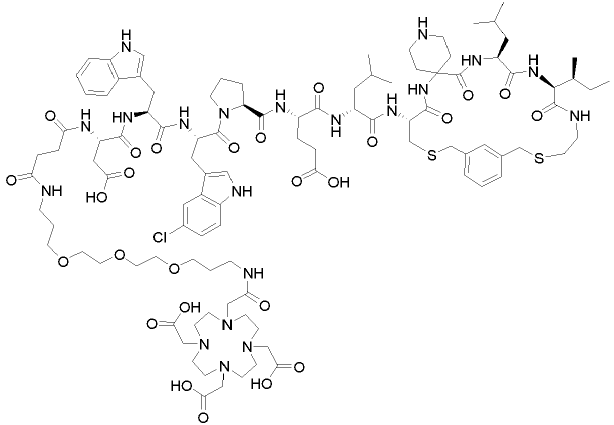
compound DOTA-APAc-Asp-Trp-5Clw-Pro-Glu-leu-[Cys(3MeBn)-Aib-Leu-Ile-AET] (GIP-089) of the following formula 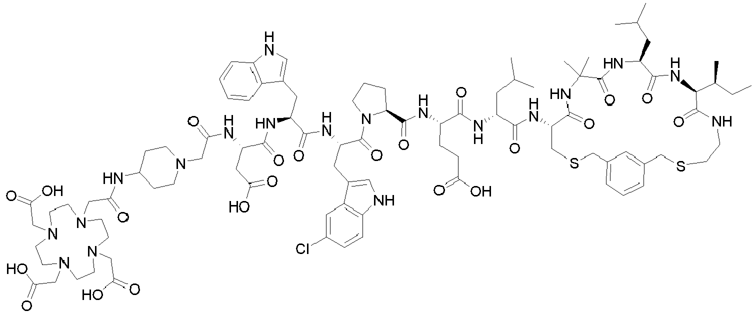
compound DOTA-Ttds-Asp-Trp-5Clw-Pro-Glu-leu-[Cys(2Lut)-Aib-Leu-Ile-AET] (GIP-090) of the following formula 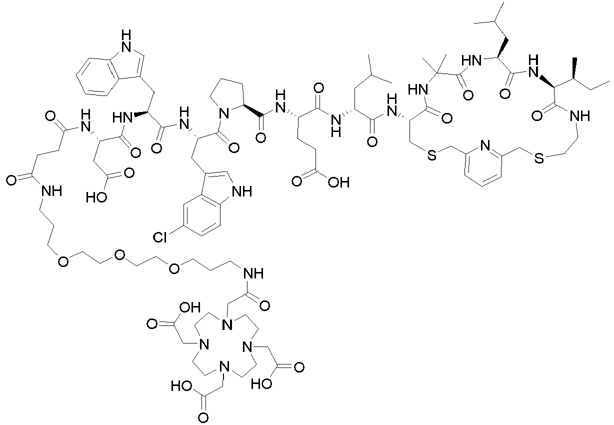
compound DOTA-Ttds-Asp-Trp-5Clw-Pro-Glu-leu-[Cys(3Lut)-Aib-Leu-Ile-AET] (GIP-091) of the following formula 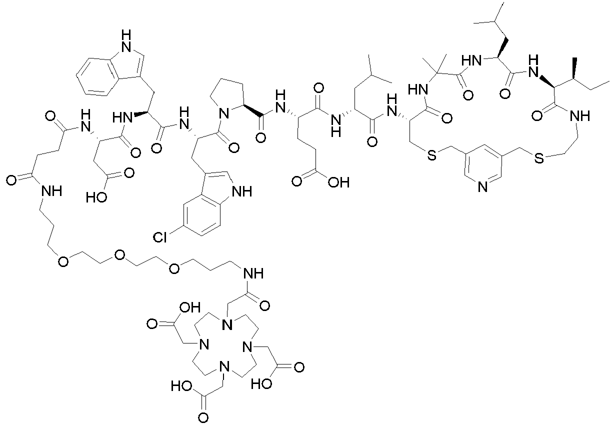
compound DOTA-Ttds-Asp-Trp-5Clw-Pro-Glu-leu-[Dap(3MeBn)-Aib-Leu-Ile-AET] (GIP-092) of the following formula 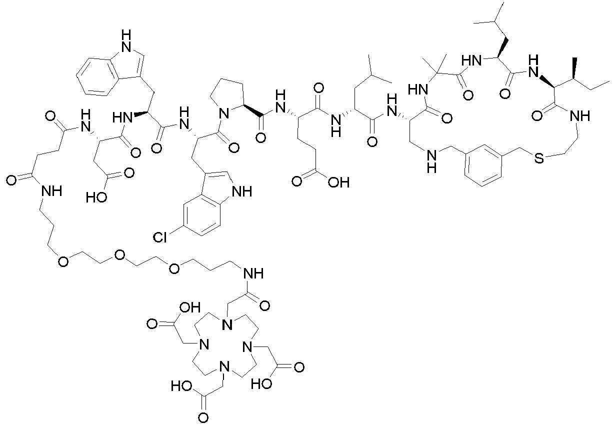
compound DOTA-Ttds-Asp-Trp-5Clw-Pro-Glu-leu-[Cys(3MeBn)-Aib-Leu-Ile-en] (GIP-093) of the following formula 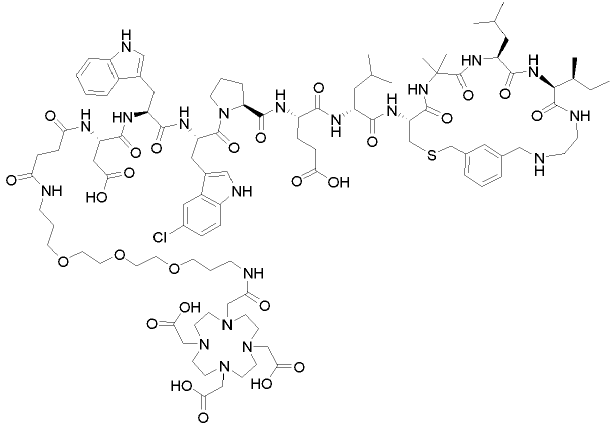
compound DOTA-Ttds-Asp-Trp-5Clw-Hyp-Glu-leu-[Cys(3MeBn)-Aib-Leu-Ile-AET] (GIP-094) of the following formula 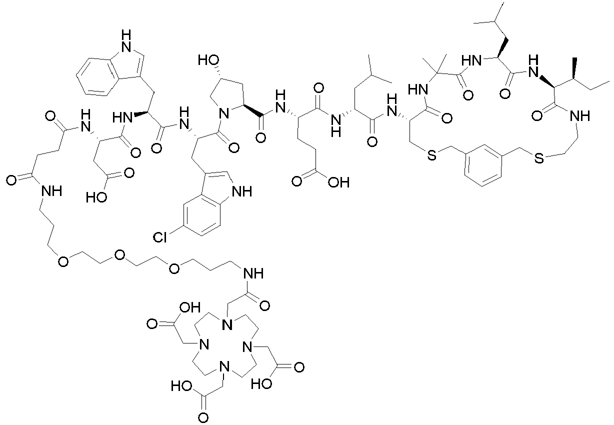
compound DOTA-Ttds-Asp-Trp-5Clw-Tap-Glu-leu-[Cys(3MeBn)-Aib-Leu-Ile-AET] (GIP-095) of the following formula 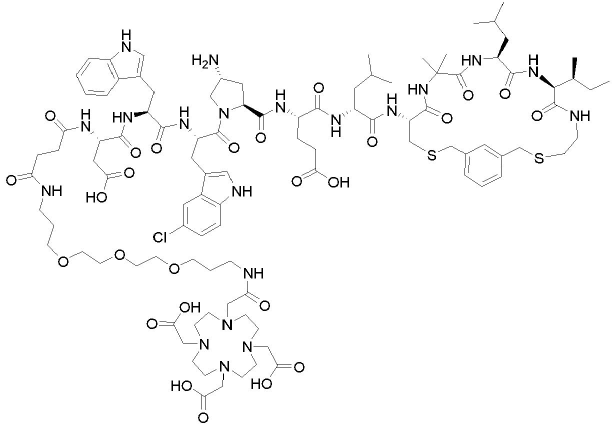
compound DOTA-Ttds-Asp-Trp-5Clw-4Ap-Glu-leu-[Cys(3MeBn)-Aib-Leu-Ile-AET] (GIP-096) of the following formula 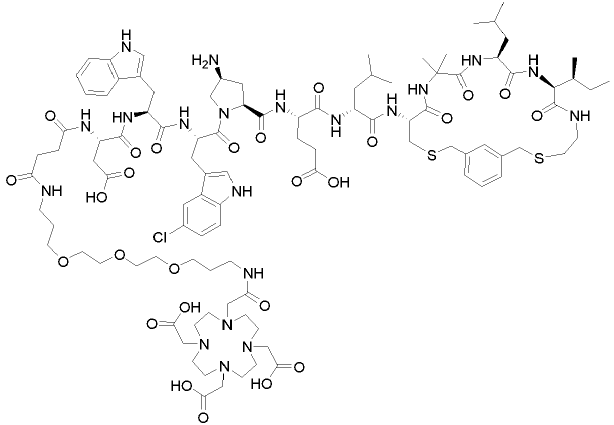
compound DOTA-Ttds-Asp-Trp-5Clw-Pro-Glu-leu-[Cys(3MeBn)-Apc(Ac)-Leu-Ile-AET] (GIP-097) of the following formula 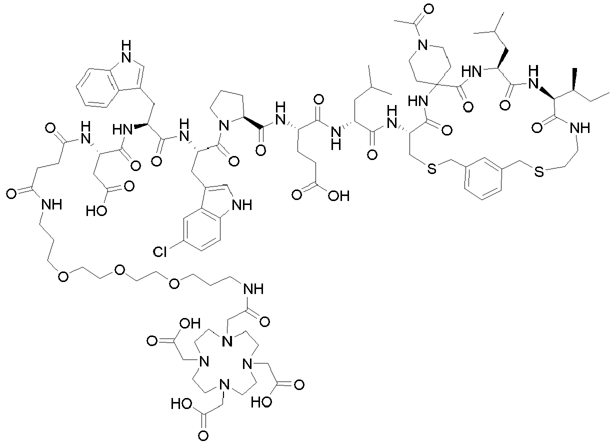
compound DOTA-Ttds-Asp-Trp-5Clw-Pro-Glu-leu-[Dap(3MeBn)-Aib-Leu-Ile-AETO] (GIP-098) of the following formula 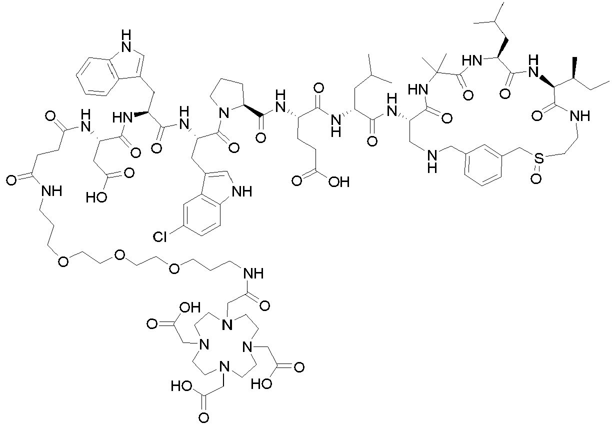
compound DOTA-Ttds-Asp-Trp-5Clw-Pro-Glu-leu-[Cys(3MeBn)-Aib-Hle-Ile-AET] (GIP-099) of the following formula 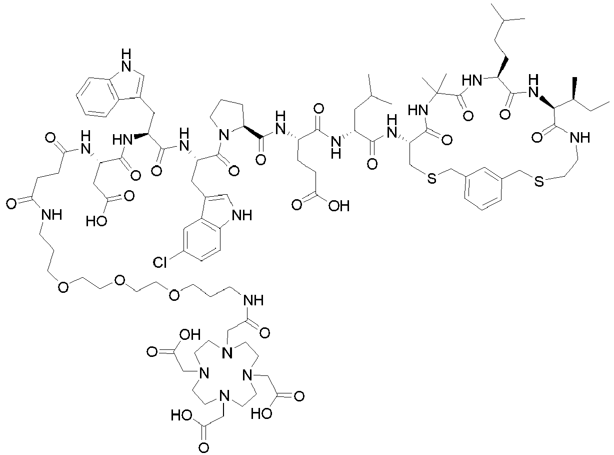
compound DOTA-Ttds-Asp-Trp-5Clw-Nmg-Glu-leu-[Cys(3MeBn)-Aib-Leu-Ile-AET] (GIP-100) of the following formula 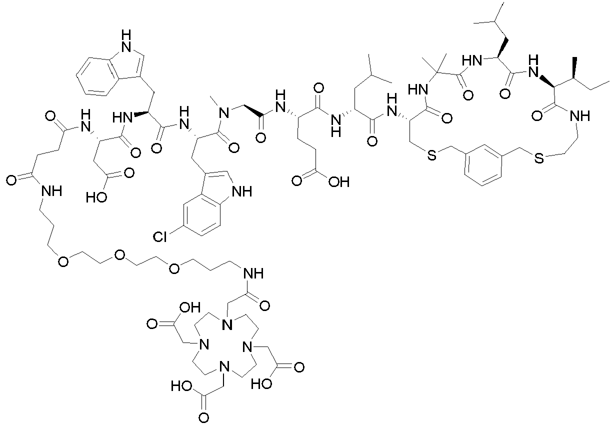
compound DOTA-Ttds-Asp-Trp-5Clw-Pro-Glu-leu-[Cys(3MeBn)-Ams-Leu-Ile-AET] (GIP-101) of the following formula 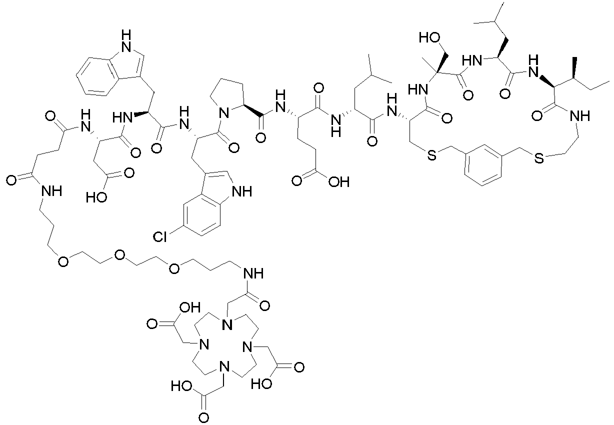
compound DOTA-Ttds-Asp-Trp-5Clw-H3p-Glu-leu-[Cys(3MeBn)-Aib-Leu-Ile-AET] (GIP-102) of the following formula 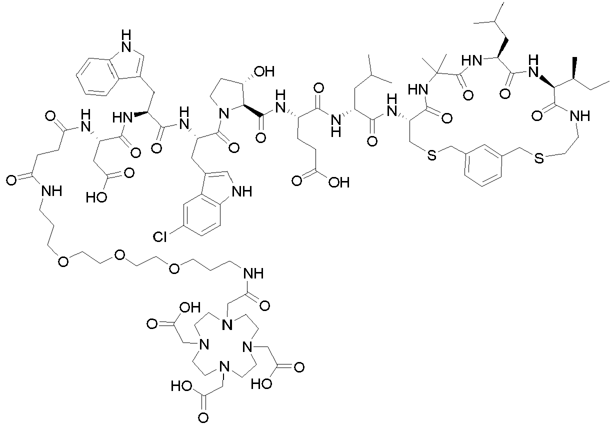
compound DOTA-APAc-Asp-Trp-5Clw-Hyp-Glu-leu-[Cys(2Lut)-Thp-Leu-Ile-AET] (GIP-103) of the following formula 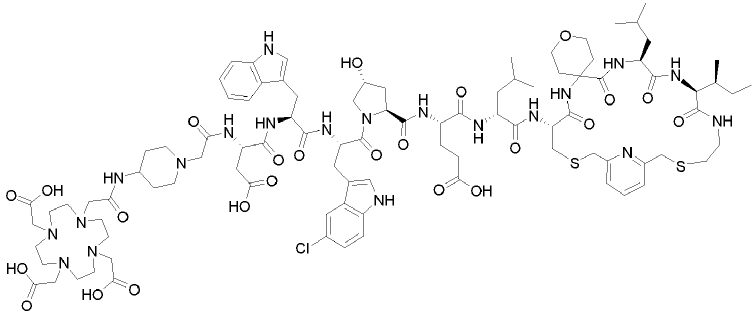
compound DOTA-Ttds-Asp-6Fw-5Clw-Pro-Glu-leu-[Cys(3MeBn)-Aib-Leu-Ile-AET] (GIP-104) of the following formula 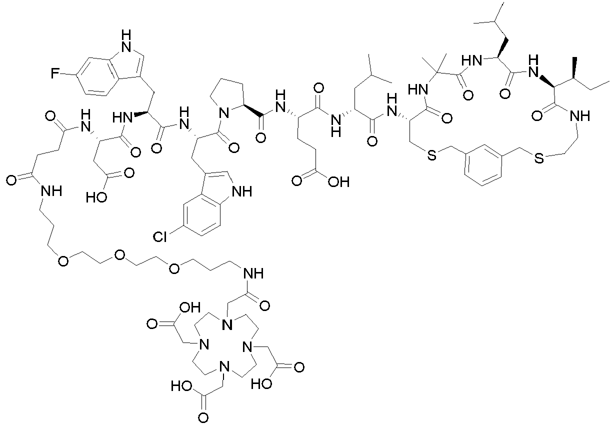
compound DOTA-Ttds-Asp-6Fw-5Brw-Pro-Glu-leu-[Cys(3MeBn)-Aib-Leu-Ile-AET] (GIP-105) of the following formula 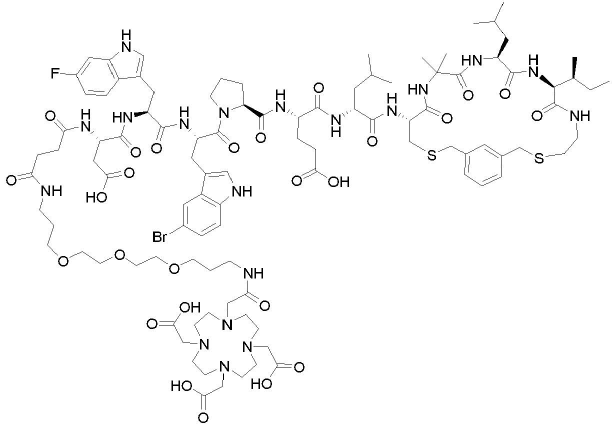
compound DOTA-APAc-Asp-Trp-5Clw-Hyp-Glu-leu-[Dap(2Lut)-Thp-Leu-Ile-AET] (GIP-106) of the following formula 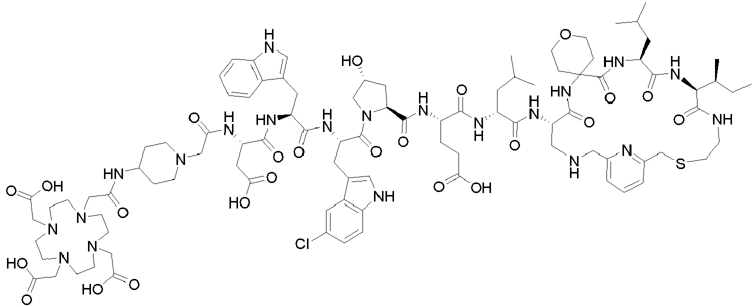
compound DOTA-APAc-Asp-Trp-5Clw-Hyp-Glu-leu-[Dap(3MeBn)-Thp-Leu-Ile-AET] (GIP-107) of the following formula 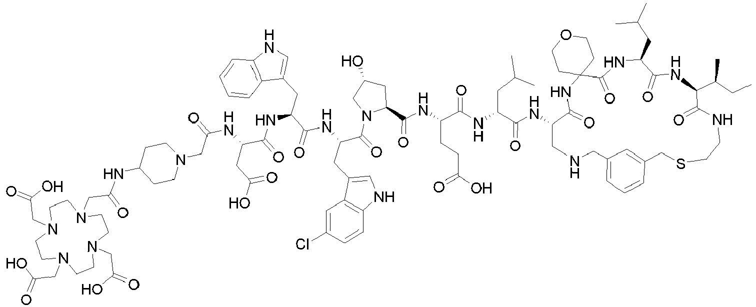
compound HO-Succinyl-Trp-5Clw-Hyp-Glu-leu-[Cys(2Lut)-Thp-Leu-Ile-Cys]-Ttds-Lys(DOTA)-NH₂ (GIP-108) of the following formula 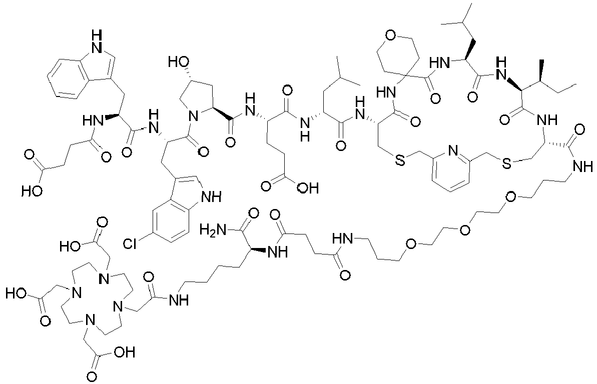
compound HO-Succinyl-Trp-5Clw-Hyp-Glu-leu-[Cys(2Lut)-Thp-Leu-Ile-Cys]-APAc-Lys(DOTA)-NH₂ (GIP-109) of the following formula 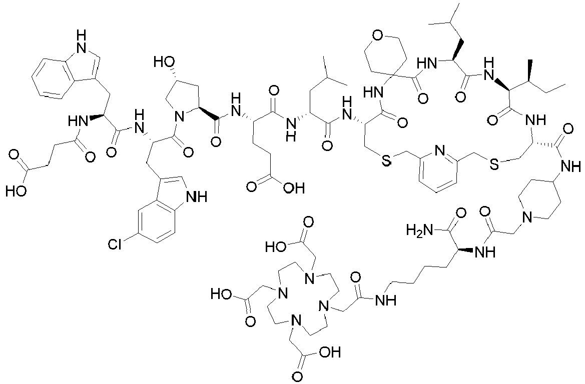
compound HO-Succinyl-Trp-5Clw-Hyp-Glu-leu-[Cys(2Lut)-Apc(DOTA)-Leu-Ile-AET] (GIP-110) of the following formula 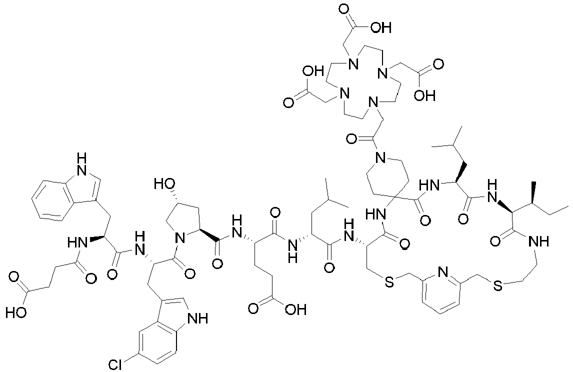
compound DOTA-Ttds-Asp-Trp-5Clw-4Ap(Ac)-Glu-leu-[Cys(3MeBn)-Aib-Leu-Ile-AET] (GIP-111) of the following formula 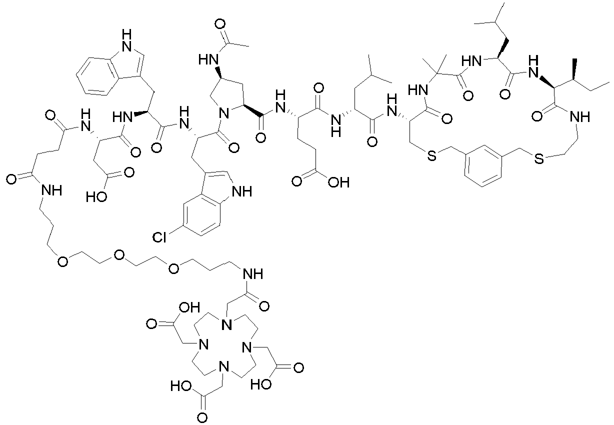
compound DOTA-Ttds-Asp-Trp-5Clw-Tap-Glu-leu-[Cys(2Lut)-Apc-Leu-Ile-AET] (GIP-112) of the following formula 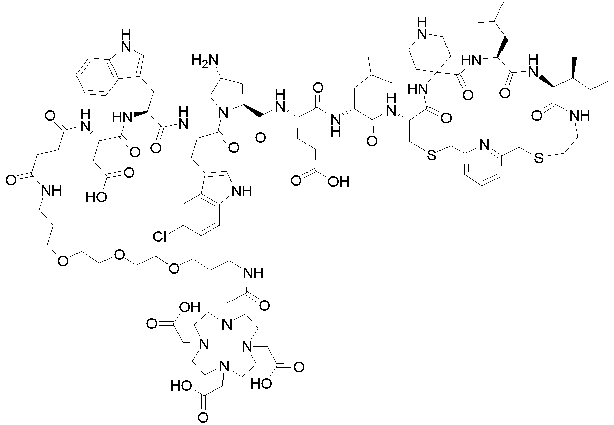
compound DOTA-Ttds-Asp-Trp-5Clw-Tap-Glu-leu-[Dap(2Lut)-Apc-Leu-Ile-AET] (GIP-113) of the following formula 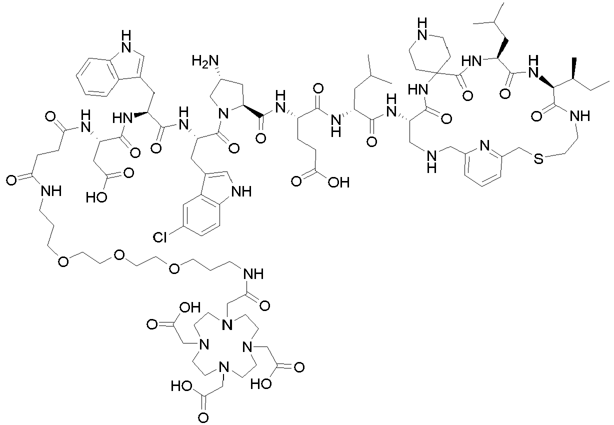
compound HO-Succinyl-Trp-5Clw-Hyp-Glu-leu-[Cys(2Lut)-Apc-Leu-Ile-Cys]-APAc-Lys(DOTA)-NH₂ (GIP-114) of the following formula 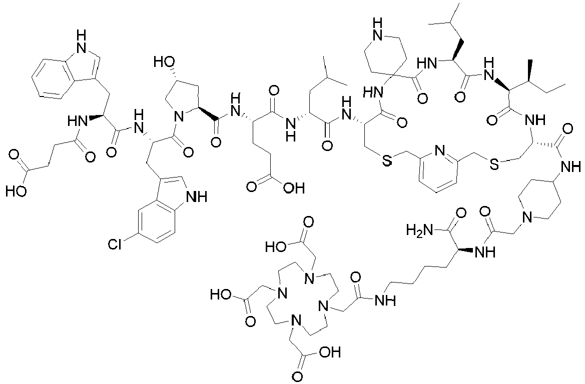
compound HO-Succinyl-Trp-5Clw-Hyp-Glu-leu-[Cys(2Lut)-Thp-Leu-Ile-Cys]-Lys(DOTA)-NH₂ (GIP-115) of the following formula 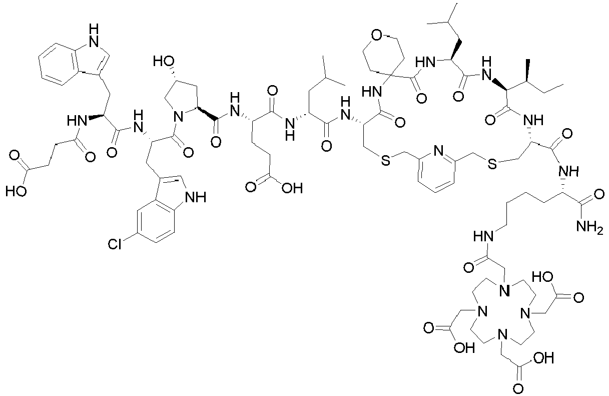
compound HO-Succinyl-Trp-5Clw-Hyp-Glu-leu-[Cys(2Lut)-Thp-Leu-Ile-Cys]-lys(DOTA)-NH₂ (GIP-116) of the following formula 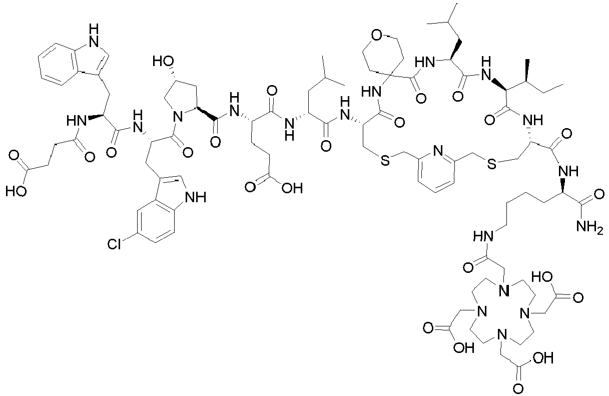
compound HO-Succinyl-Trp-5Clw-Hyp-Glu-leu-[Cys(2Lut)-Thp-Leu-Ile-Cys]-Ape-DOTA (GIP-117) of the following formula 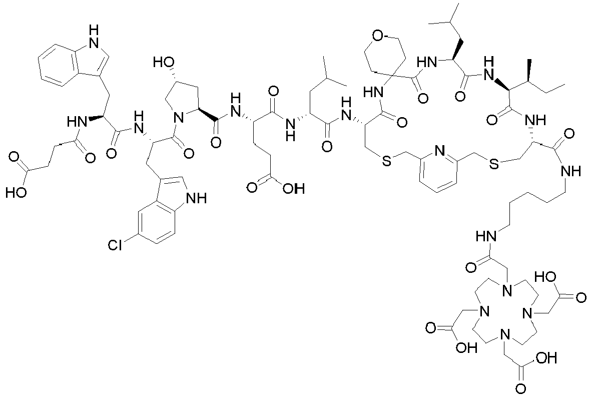
compound HO-Succinyl-Trp-5Clw-Hyp-Glu-leu-[Cys(2Lut)-Thp-Leu-Ile-Cys]-PP-DOTA (GIP-118) of the following formula 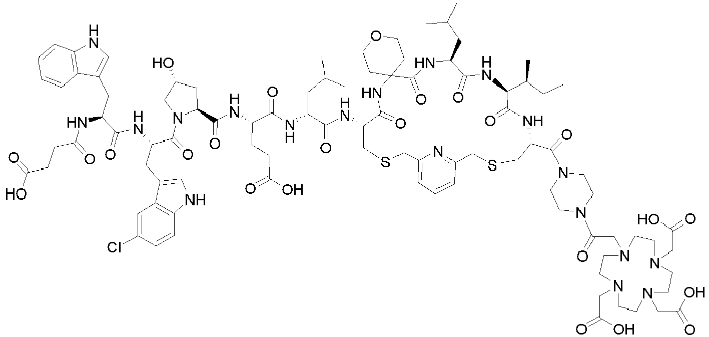
compound DOTA-Ttds-Asp-Trp-5Clw-Pro-Glu-leu-[Cys(2Lut)-Apc-Leu-Ile-AET] (GIP-119) of the following formula 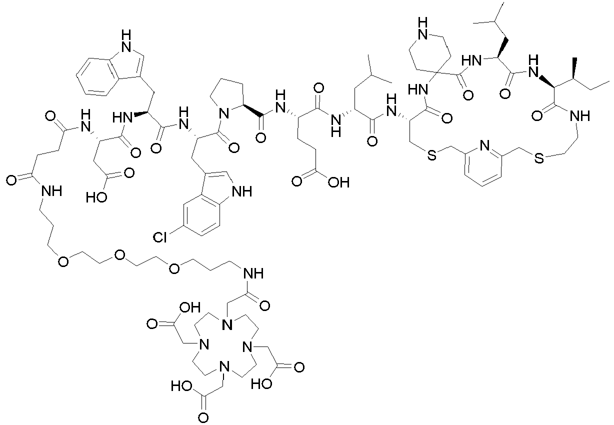
compound MeO2kDaPEGAc-Asp-Trp-5Clw-Hyp-Glu-leu-[Cys(2Lut)-Thp-Leu-Ile-Cys]-lys(DOTA)-NH₂ (GIP-120) of the following formula 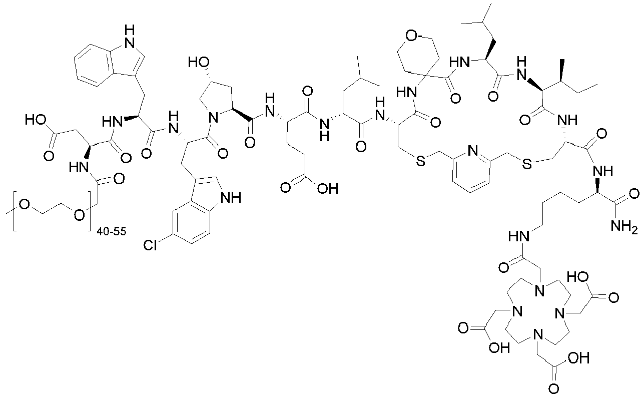
compound Ac-Asp-Trp-5Clw-Tap(DOTA)-Glu-leu-[Cys(2Lut)-Thp-Leu-Ile-Cys]-NH₂ (GIP-121) of the following formula 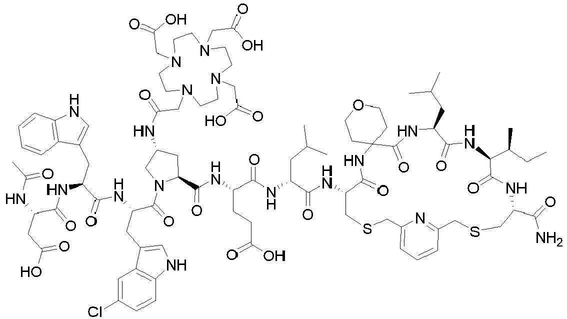
compound Ac-Asp-Trp-5Clw-Hyp-Glu-leu-[Cys(2Lut)-Apc(DOTA)-Leu-Ile-Cys]-NH₂ (GIP-122) of the following formula 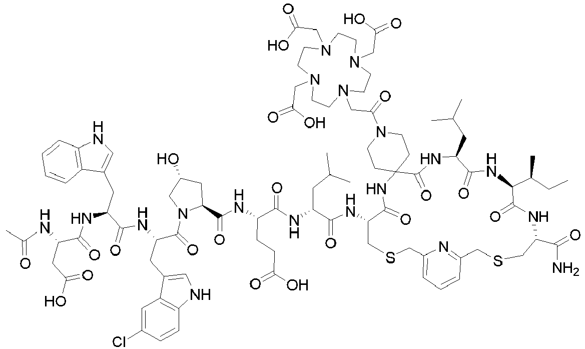
compound Ac-Asp-Trp-5Clw-Tap-Glu-leu-[Cys(2Lut)-Apc(DOTA)-Leu-Ile-Cys]-NH₂ (GIP-123) of the following formula 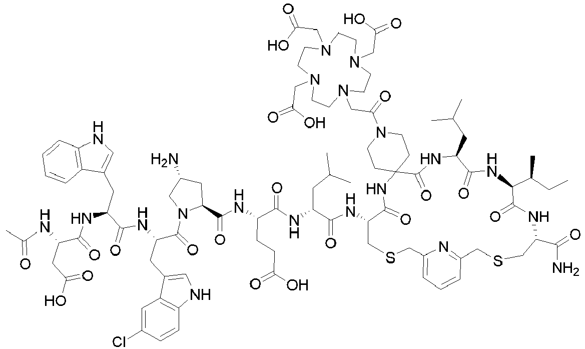
compound Ac-Asp-Trp-5Clw-Hyp-Glu-leu-[Cys(tMeBn(DOTA-AET))-Thp-Leu-Ile-Cys]-NH₂ (GIP-124) of the following formula 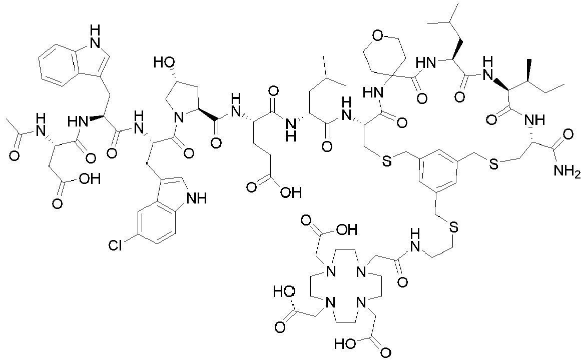
compound DOTA-Asp-Trp-5Clw-Hyp-Glu-leu-[Cys(2Lut)-Thp-Leu-Ile-Cys]-NH₂ (GIP-125) of the following formula 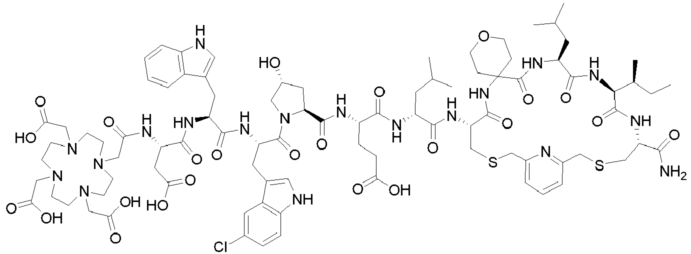
compound DOTA-Ttds-Tyr-Asp-Trp-Trp-Pro-Glu-Leu-[Cys(3MeBn)-Ala-Leu-Ile-Cys]-NH₂ (GIP-126) of the following formula 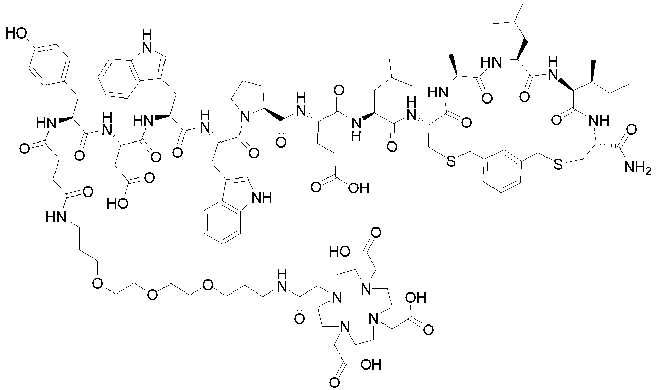
compound Ac-Tyr-Asp-Trp-Trp-Pro-Glu-Leu-[Cys(3MeBn)-Ala-Leu-Ile-Cys]-Ttds-Lys(DOTA)-NH₂ (GIP-127) of the following formula 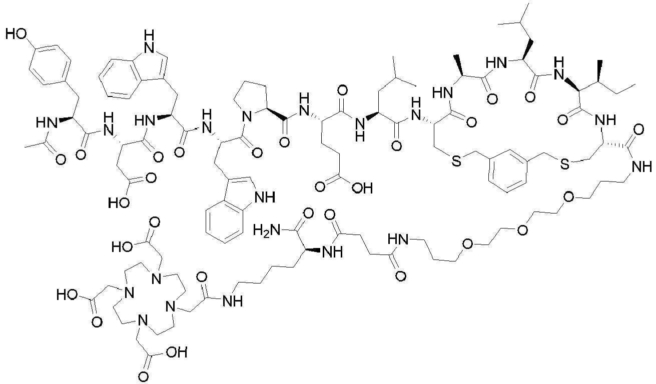
compound DOTA-Ttds-Asp-Trp-Trp-Pro-Glu-leu-[Cys(3MeBn)-Aib-Leu-Ile-Cys]-NH₂ (GIP-128) of the following formula 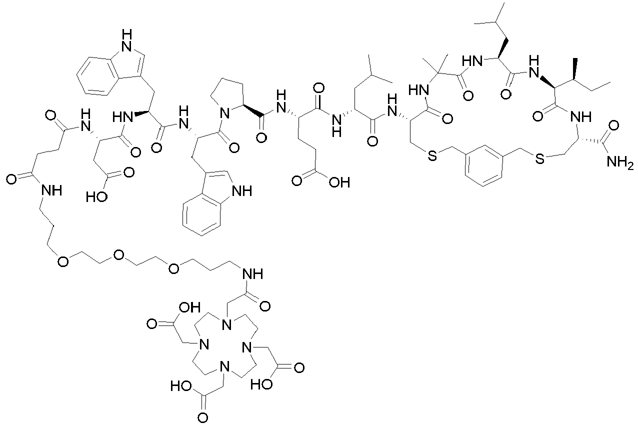
compound DOTA-Ttds-Asp-Trp-Trp-Pro-Glu-leu-[Cys(3MeBn)-Aib-Leu-Ile-AET] (GIP-129) of the following formula 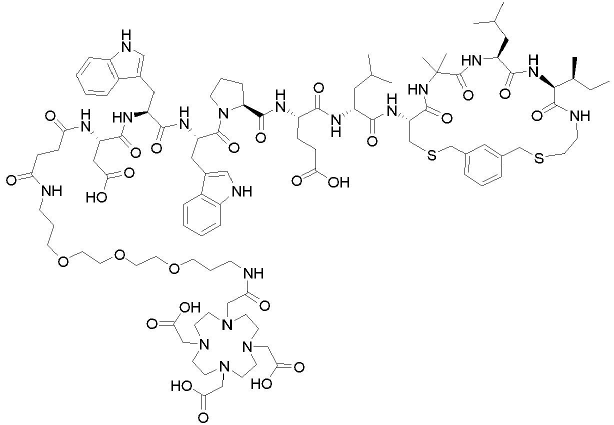
compound DOTA-Ttds-Nmd-Trp-Trp-Pro-Glu-Leu-[Cys(3MeBn)-Aib-Leu-Ile-AET] (GIP-130) of the following formula 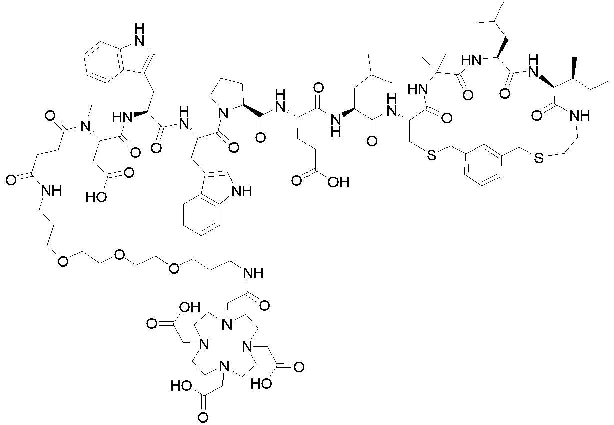
compound Ac-Asp-Trp-Trp-Pro-Glu-Leu-[Cys(3MeBn)-Aib-Leu-Ile-Cys]-Ttds-Lys(DOTA)-NH₂ (GIP-131) of the following formula 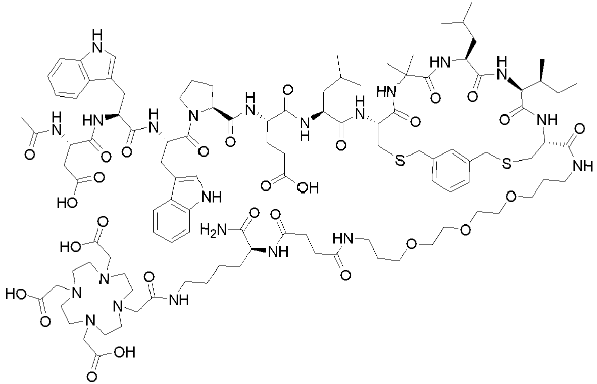
compound Ac-Asp-Trp-Trp-Pro-Glu-Npg-[Cys(3MeBn)-Aib-Leu-Ile-Cys]-Ttds-Lys(DOTA)-NH₂ (GIP-132) of the following formula 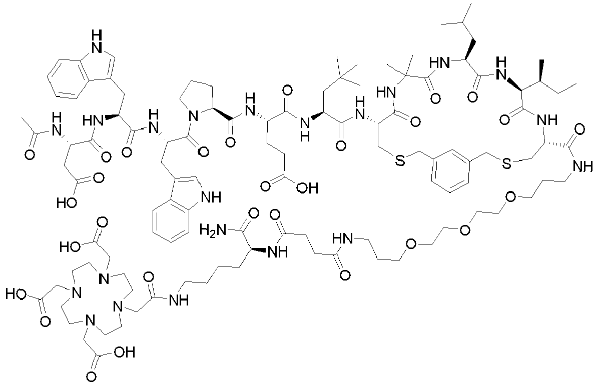
compound Ac-Asp-Trp-Trp-Pro-Glu-Leu-[Cys(3MeBn)-Aib-Npg-Ile-Cys]-Ttds-Lys(DOTA)-NH₂ (GIP-133) of the following formula 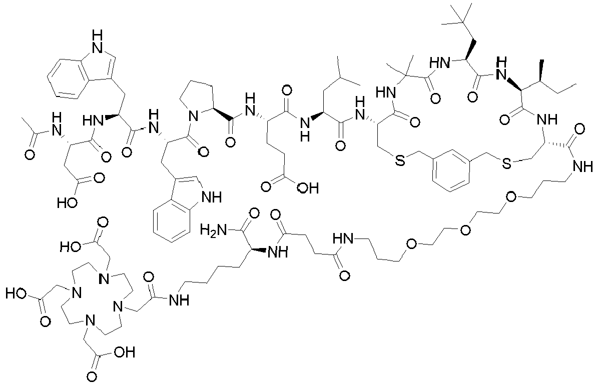
compound Ac-Asp-Trp-Trp-Pro-Glu-Leu-[Cys(3MeBn)-Aib-Cha-Ile-Cys]-Ttds-Lys(DOTA)-NH₂ (GIP-134) of the following formula 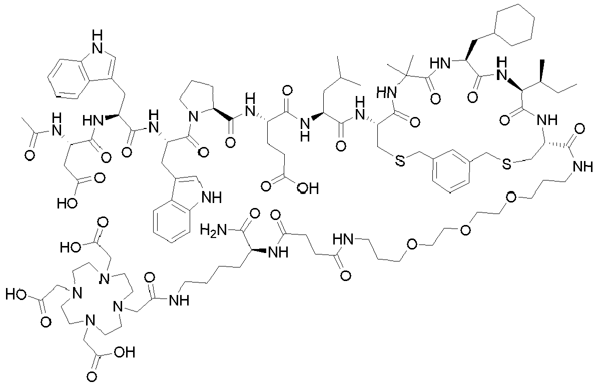
compound Php-Asp-Trp-Trp-Pro-Glu-leu-[Cys(3MeBn)-Aib-Leu-Ile-Cys]-Ttds-Lys(DOTA)-NH₂ (GIP-135) of the following formula 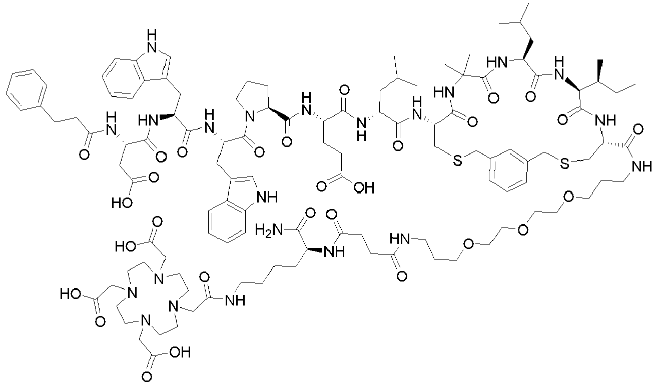
compound 4FPhp-Asp-Trp-Trp-Pro-Glu-leu-[Cys(3MeBn)-Aib-Leu-Ile-Cys]-Ttds-Lys(DOTA)-NH₂ (GIP-136) of the following formula 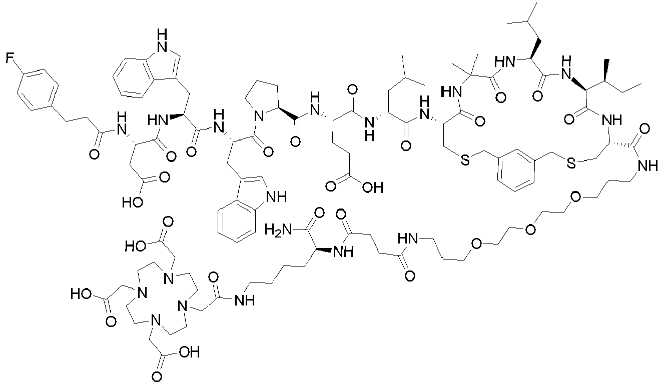
compound 40HPhp-Asp-Trp-Trp-Pro-Glu-leu-[Cys(3MeBn)-Aib-Leu-Ile-Cys]-Ttds-Lys(DOTA)-NH₂ (GIP-137) of the following formula 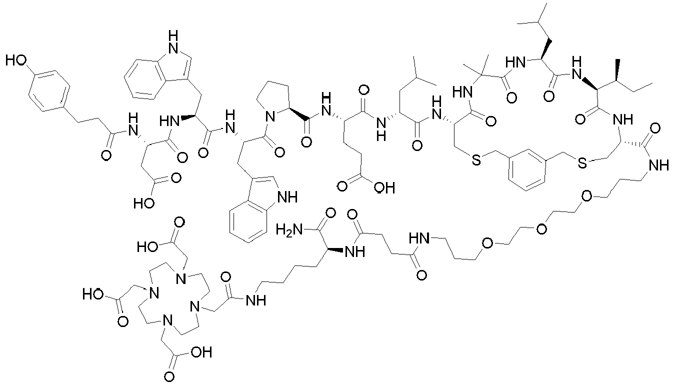
compound Ac-Asp-Trp-1Ni-Pro-Glu-leu-[Cys(3MeBn)-Ala-Leu-Val-Cys]-Ttds-Lys(DOTA)-NH₂ (GIP-138) of the following formula 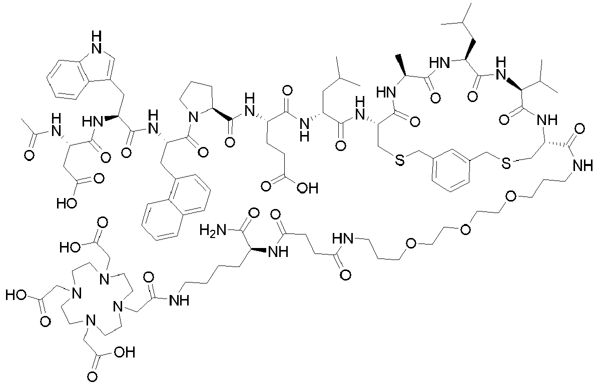
compound Ac-Asp-Trp-1Ni-Pro-Glu-leu-[Cys(3MeBn)-Deg-Leu-Ile-Cys]-Ttds-Lys(DOTA)-NH₂ (GIP-139) of the following formula 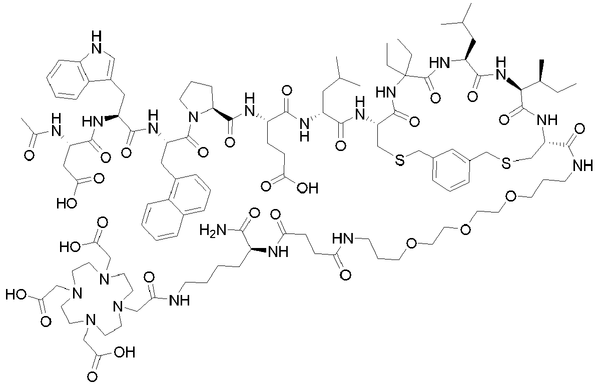
compound Ac-Asp-Trp-6Fw-Pro-Glu-leu-[Cys(3MeBn)-Aib-Leu-Ile-Cys]-Ttds-Lys(DOTA)-NH₂ (GIP-140) of the following formula 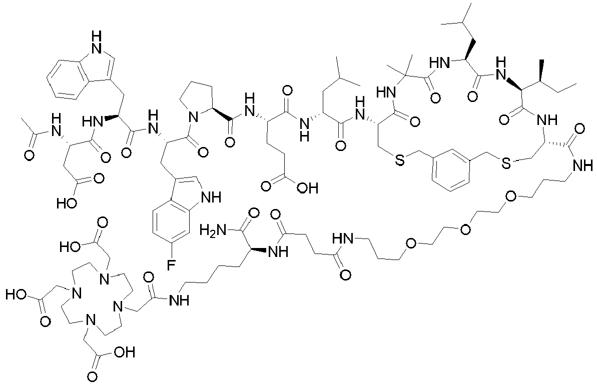
compound DOTA-Ttds-Asp-6Clw-Trp-Pro-Glu-leu-[Cys(3MeBn)-Aib-Leu-Ile-AET] (GIP-141) of the following formula 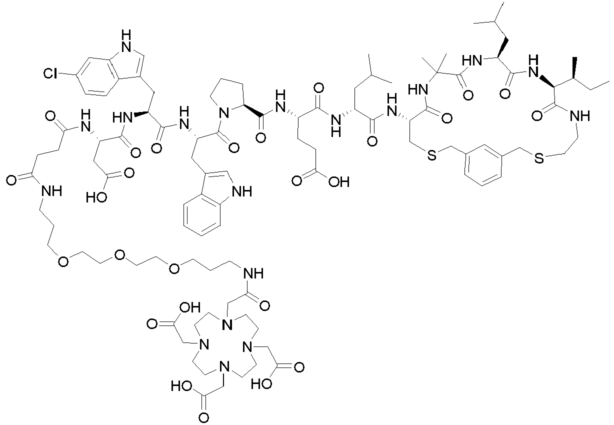
compound HO-Succinyl-Trp-5Clw-Hyp-Glu-leu-[Cys(2Lut)-Apc(MeO10kDaPEGAc)-Leu-Ile-Cys]-APAc-Lys(DOTA)-NH₂ (GIP-142) of the following formula 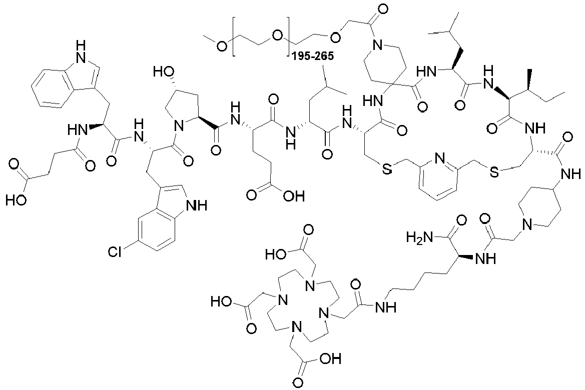
compound HO-Succinyl-Trp-5Clw-Hyp-Glu-leu-[Cys(2Lut)-Apc(MeO2kDaPEGAc)-Leu-Ile-Cys]-APAc-Lys(DOTA)-NH₂ (GIP-143) of the following formula 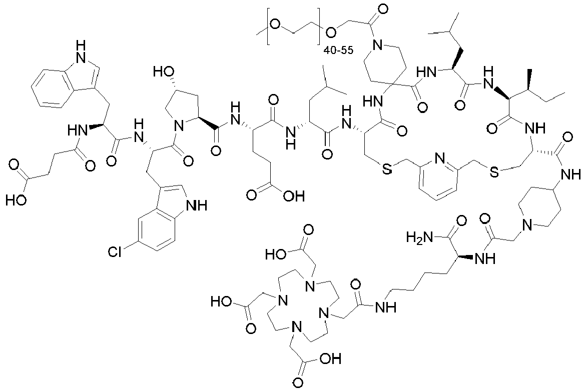
compound HO-Succinyl-Trp-5Clw-Hyp-Glu-leu-[Cys(2Lut)-Thp-Leu-Ile-Cys]-APAclys(MeO2kDaPEGAc)-Lys(DOTA)-NH₂ (GIP-144) of the following formula 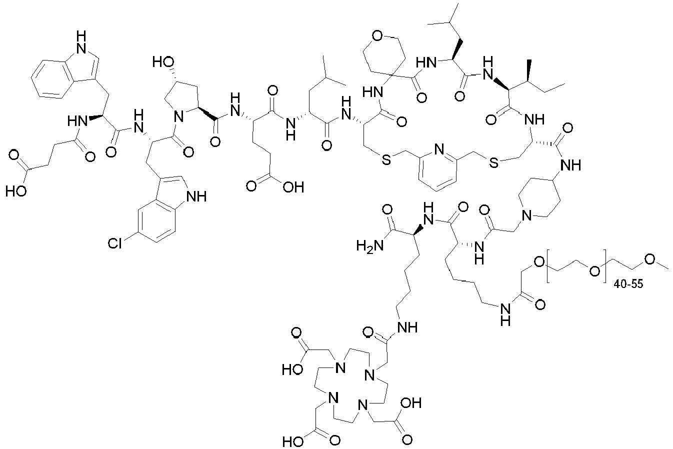
compound Ac-Asp-Trp-5Clw-Hyp-Glu-leu-[Cys(tMeBn(DOTA-PP))-Thp-Leu-Ile-Cys]-NH₂ (GIP-145) of the following formula 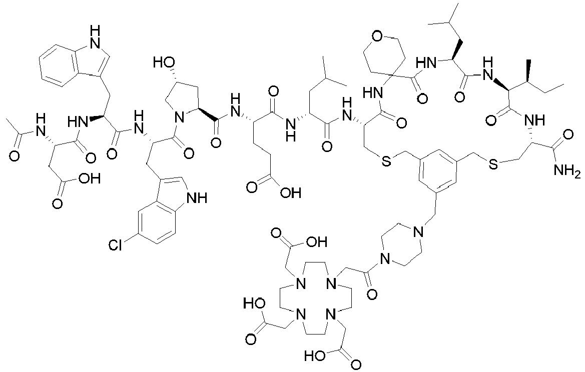
compound AGLU-Glutar-Asp-Trp-5Clw-Hyp-Glu-leu-[Cys(2Lut)-Apc(InDOTA)-Leu-Ile-AET] (GIP-146) of the following formula 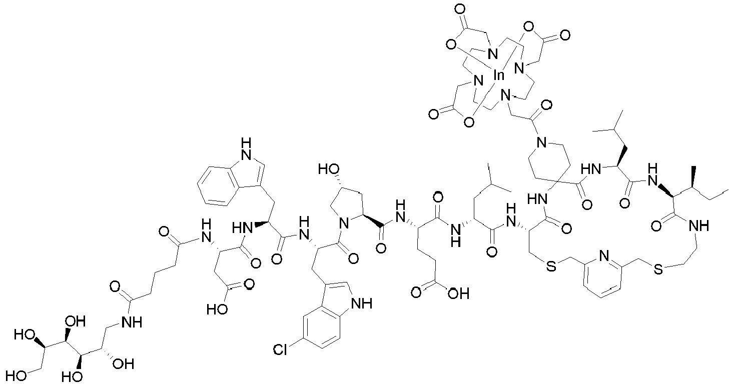
compound AGLU-Glutar-Asp-Trp-5Clw-Hyp-Glu-leu-[Cys(2Lut)-Apc(ZnDOTA)-Leu-Ile-AET] (GIP-147) of the following formula 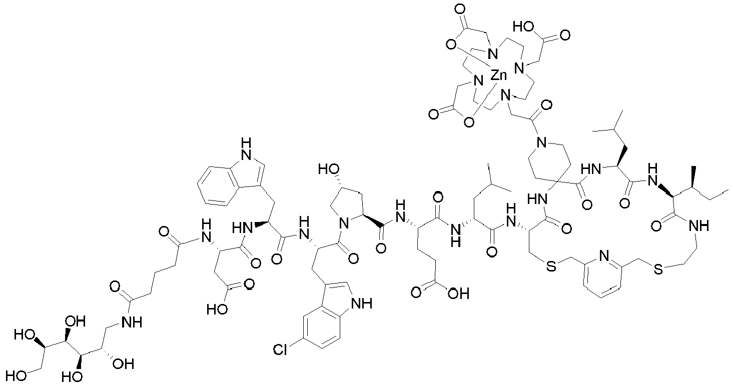
compound InDOTA-Ttds-Asp-Trp-5Clw-Pro-Glu-leu-[Cys(3MeBn)-Aib-Leu-Ile-AET] (GIP-148) of the following formula 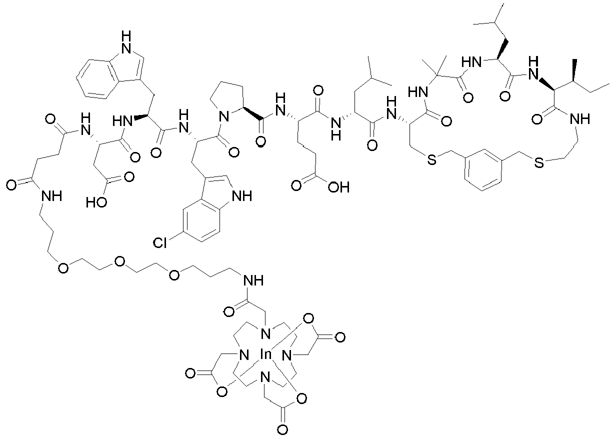
compound InDOTA-Ttds-Asp-Trp-5Clw-Pro-Glu-leu-[Cys(3MeBn)-Ams-Leu-Ile-AET] (GIP-149) of the following formula 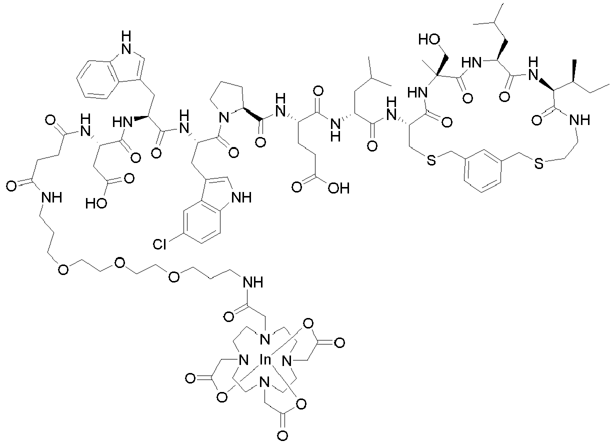
compound InDOTA-Ttds-Asp-Trp-5Clw-Pro-Glu-leu-[Cys(3MeBn)-Thp-Leu-Ile-AET] (GIP-150) of the following formula 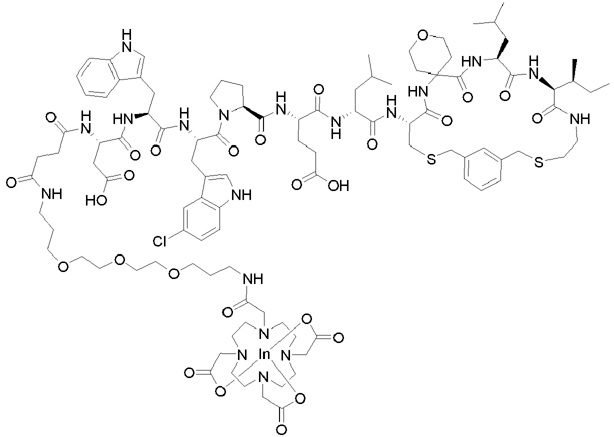
compound InDOTA-Ttds-Asp-Trp-5Clw-Hyp-Glu-leu-[Cys(3MeBn)-Aib-Leu-Ile-AET] (GIP-151) of the following formula 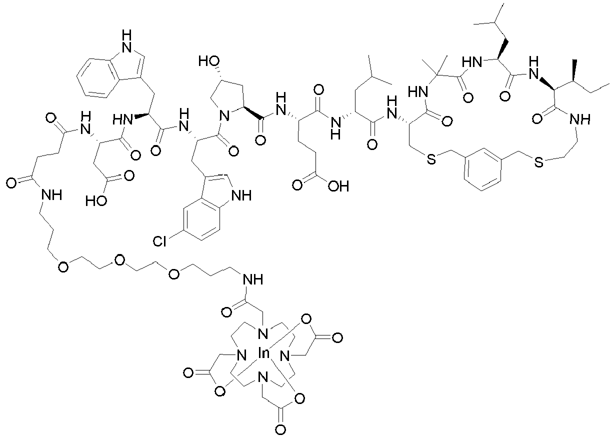
compound InDOTA-Ttds-Asp-Trp-5Clw-Nmg-Glu-leu-[Cys(3MeBn)-Aib-Leu-Ile-AET] (GIP-152) of the following formula 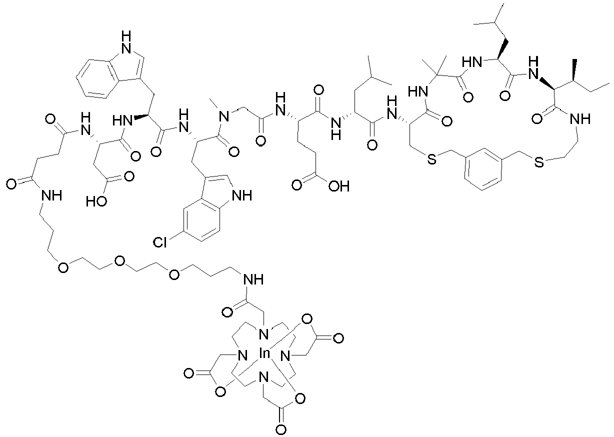
compound InDOTA-APAc-Asp-Trp-5Clw-Pro-Glu-leu-[Cys(3MeBn)-Aib-Leu-Ile-AET] (GIP-153) of the following formula 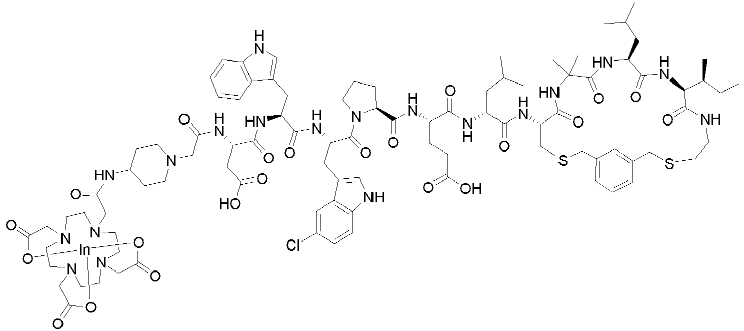
compound InDOTA-Ttds-Asp-Trp-5Clw-Pro-Glu-leu-[Cys(2Lut)-Aib-Leu-Ile-AET] (GIP-154) of the following formula 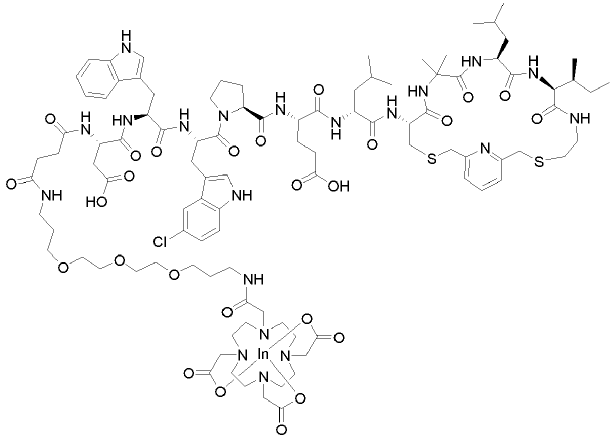
compound InDOTA-APAc-Asp-Trp-5Clw-Hyp-Glu-leu-[Cys(2Lut)-Thp-Leu-Ile-AET] (GIP-155) of the following formula 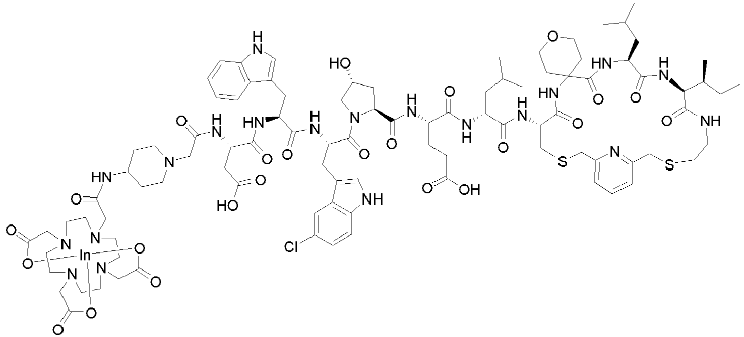
compound Ac-Asp-Trp-5Clw-Pro-Glu-leu-[Cys(3MeBn)-Aib-Leu-Ile-Cys]-Ttds-Lys(LuDOTA)-NH₂ (GIP-156) of the following formula 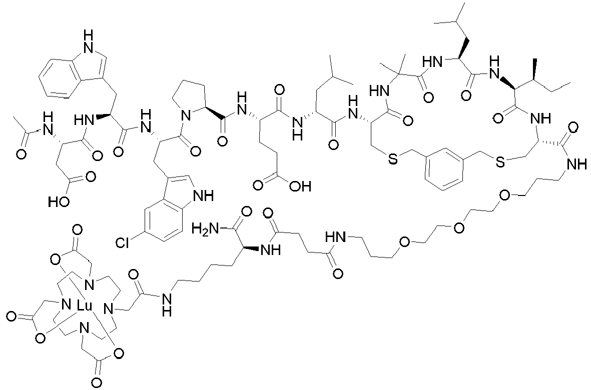
compound LuDOTA-Ttds-Asp-Trp-5Clw-Pro-Glu-leu-[Cys(3MeBn)-Ams-Leu-Ile-AET] (GIP-157) of the following formula 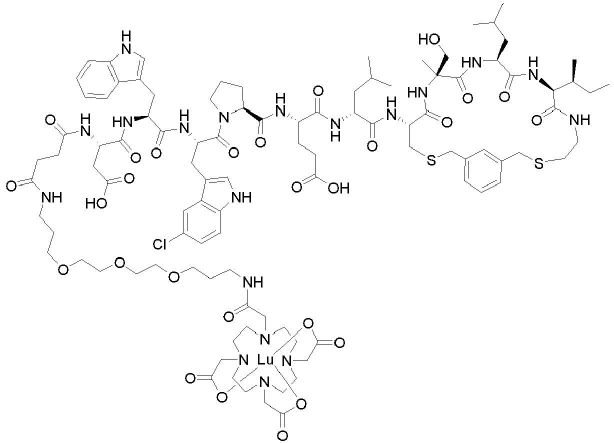
compound LuDOTA-Ttds-Asp-Trp-5Clw-Pro-Glu-leu-[Cys(3MeBn)-Thp-Leu-Ile-AET] (GIP-158) of the following formula 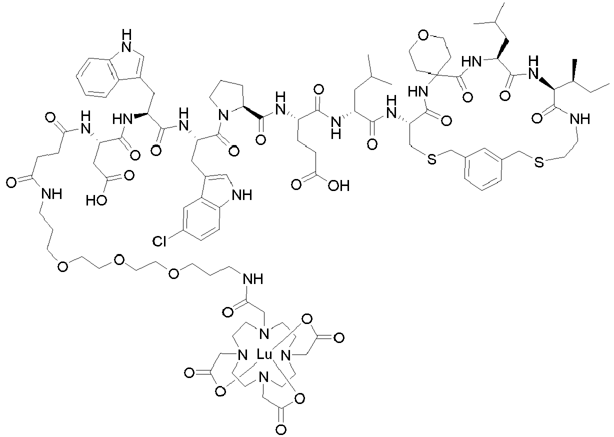
compound LuDOTA-Ttds-Asp-Trp-5Clw-Hyp-Glu-leu-[Cys(3MeBn)-Aib-Leu-Ile-AET] (GIP-159) of the following formula 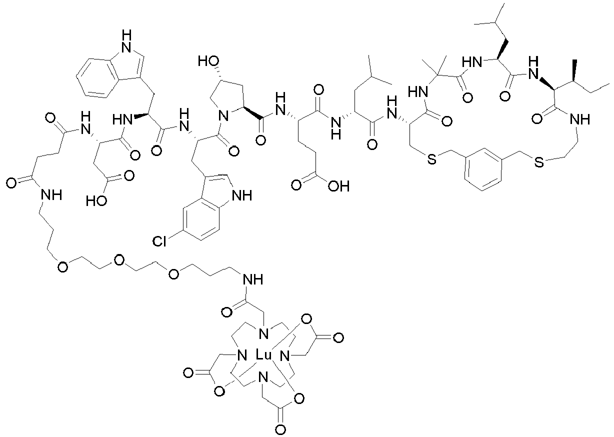
compound LuDOTA-Ttds-Asp-Trp-5Clw-Nmg-Glu-leu-[Cys(3MeBn)-Aib-Leu-Ile-AET] (GIP-160) of the following formula 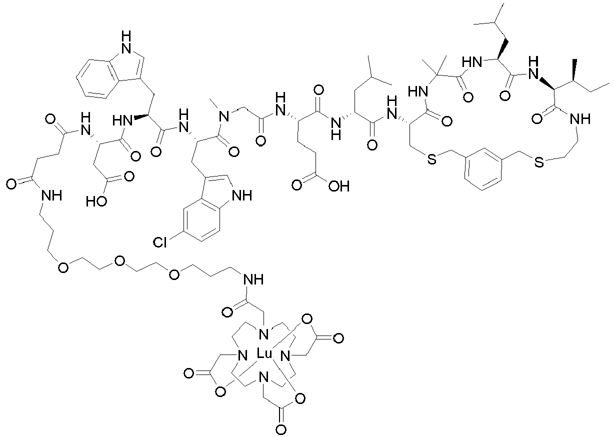
compound LuDOTA-APAc-Asp-Trp-5Clw-Pro-Glu-leu-[Cys(3MeBn)-Aib-Leu-Ile-AET] (GIP-161) of the following formula 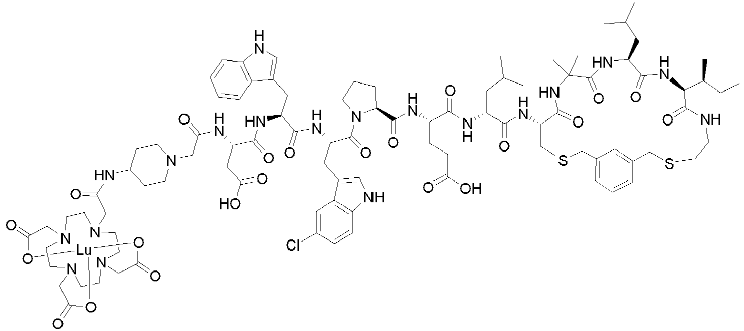
compound LuDOTA-APAc-Asp-Trp-5Clw-Hyp-Glu-leu-[Cys(2Lut)-Thp-Leu-Ile-AET] (GIP-162) of the following formula 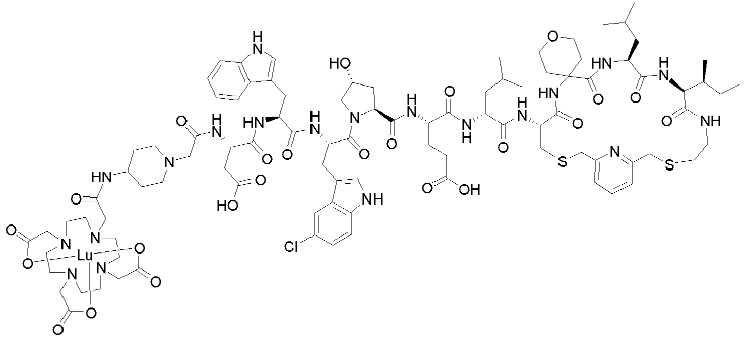
compound LuDOTA-Ttds-Asp-Trp-5Clw-Pro-Glu-leu-[Cys(2Lut)-Aib-Leu-Ile-AET] (GIP-163) of the following formula 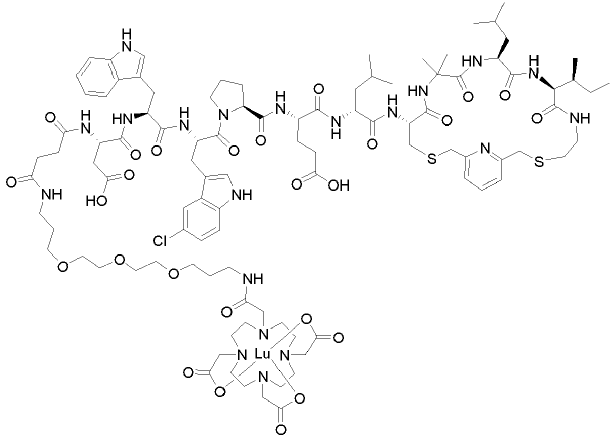
compound Ac-Asp-Trp-5Clw-Pro-Glu-leu-[Cys(3MeBn)-Aib-Leu-Ile-Cys]-Ttds-Lys(InDOTA)-NH₂ (GIP-164) of the following formula 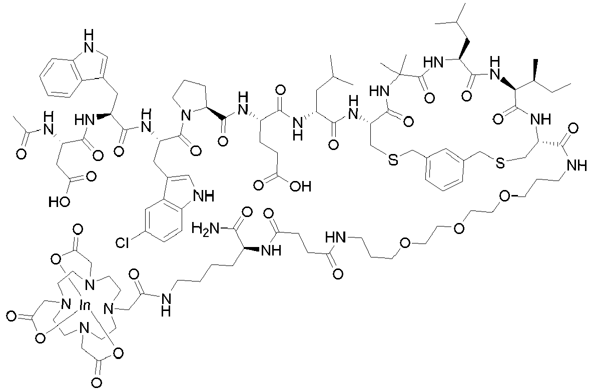
compound Ac-Asp-Trp-1Ni-Pro-Glu-leu-[Cys(3MeBn)-Aib-Leu-Ile-Cys]-Ttds-Lys(InDOTA)-NH₂ (GIP-165) of the following formula 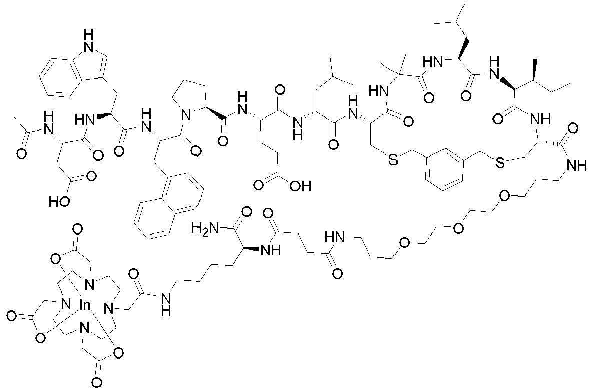
compound Ac-Asp-Trp-lNi-Pro-Glu-leu-[Cys(3MeBn)-Egz-Leu-Ile-Cys]-Ttds-Lys(InDOTA)-NH₂ (GIP-166) of the following formula 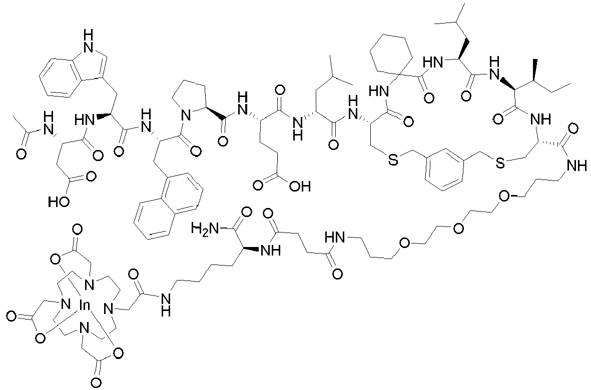
compound LuDOTA-Ttds-Asp-Trp-5Clw-Pro-Glu-leu-[Cys(3MeBn)-Aib-Leu-Ile-AET] (GIP-167) of the following formula 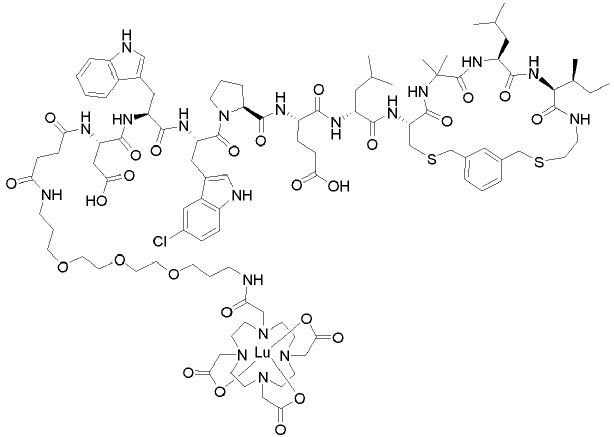
compound HO-Succinyl-Trp-5Clw-Hyp-Glu-leu-[Cys(2Lut)-Thp-Leu-Ile-Cys]-APAc-Lys(InDOTA)-NH₂ (GIP-168) of the following formula 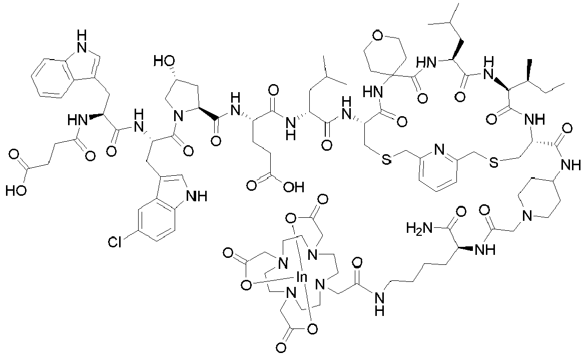
compound HO-Succinyl-Trp-5Clw-Hyp-Glu-leu-[Cys(2Lut)-Apc(InDOTA)-Leu-Ile-AET] (GIP-169) of the following formula 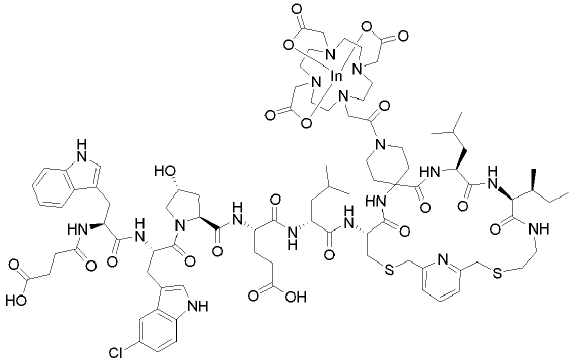
compound HO-Succinyl-Trp-5Clw-Hyp-Glu-leu-[Cys(2Lut)-Thp-Leu-Ile-Cys]-APAc-Lys(LuDOTA)-NH₂ (GIP-170) of the following formula 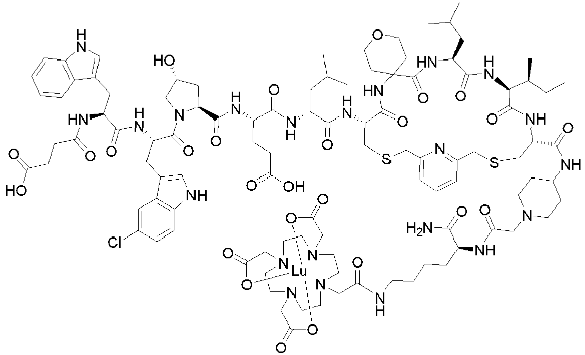
compound HO-Succinyl-Trp-5Clw-Hyp-Glu-leu-[Cys(2Lut)-Apc(LuDOTA)-Leu-Ile-AET] (GIP-171) of the following formula 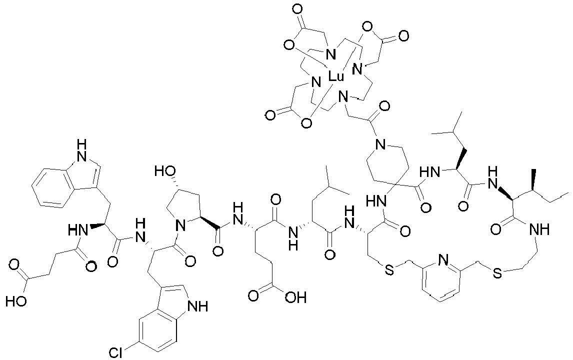
compound Ac-Asp-Trp-1Ni-Pro-Glu-leu-[Cys(3MeBn)-Aib-Leu-Ile-Cys]-Ttds-Lys(LuDOTA)-NH₂ (GIP-172) of the following formula 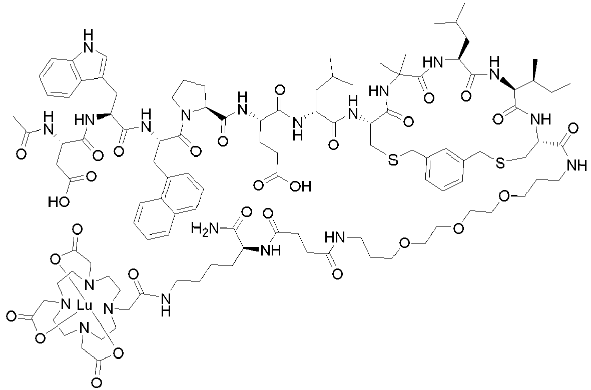
compound Ac-Asp-Trp-1Ni-Pro-Glu-leu-[Cys(3MeBn)-Egz-Leu-Ile-Cys]-Ttds-Lys(LuDOTA)-NH₂ (GIP-173) of the following formula 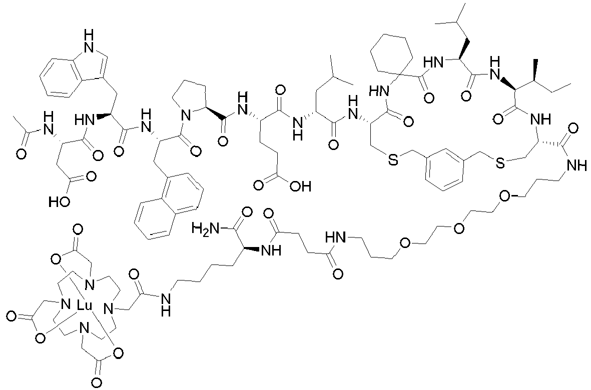
compound AGLU-Glutar-Asp-Trp-5Clw-Hyp-Glu-leu-[Cys(2Lut)-Thp-Leu-Ile-Cys]-APAc-glu(AGLU)-NH₂ (GIP-174) of the following formula 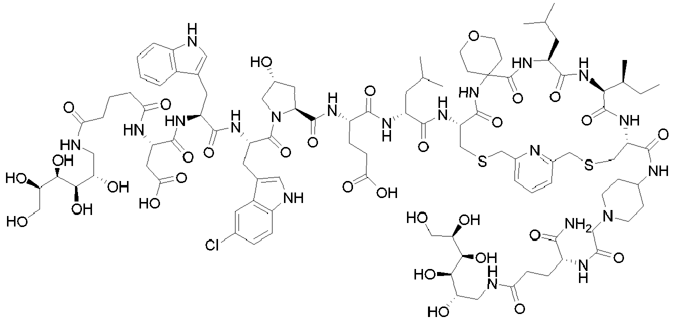
compound Ac-Asp-Trp-5Clw-Tap(AGLU-Glutar)-Glu-leu-[Cys(2Lut)-Thp-Leu-Ile-Cys]-NH₂ (GIP-175) of the following formula 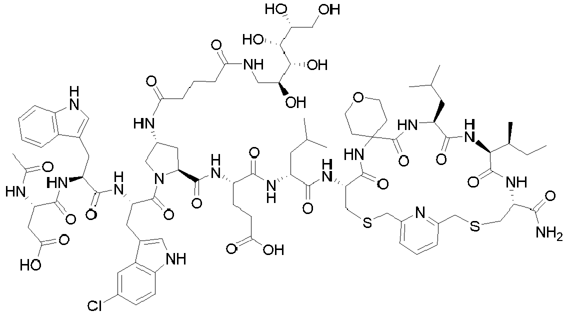
compound Ac-Asp-Trp-5Clw-Nglu(AGLU)-Glu-leu-[Cys(2Lut)-Thp-Leu-Ile-Cys]-NH₂ (GIP-176) of the following formula 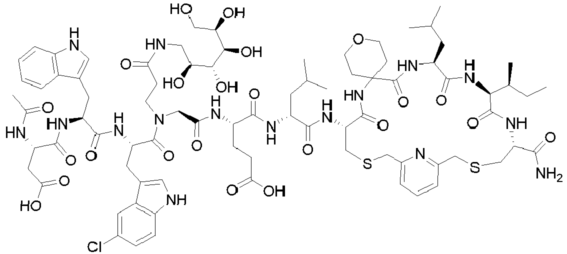
compound Ac-Asp-Trp-5Clw-Hyp-Glu-leu-[Cys(2Lut)-Thp-Leu-Ile-Cys]-APAc-glu(AGLU)-NH₂ (GIP-177) of the following formula 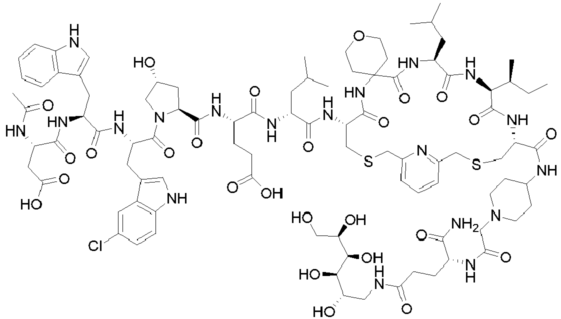
compound AGLU-Glutar-Asp-Trp-5Clw-Hyp-Glu-leu-[Cys(2Lut)-Thp-Leu-Ile-Cys]-NH₂ (GIP-178) of the following formula 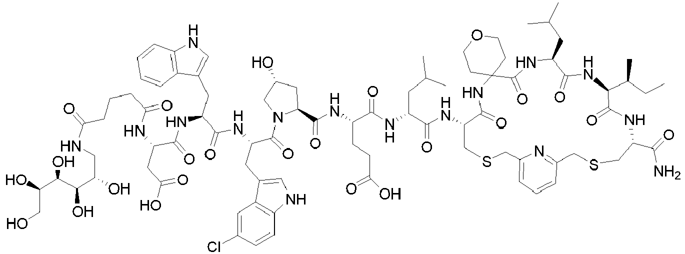
compound Ac-Cya-Trp-5Clw-Hyp-Glu-leu-[Cys(2Lut)-Aib-Leu-Ile-AET] (GIP-179) of the following formula 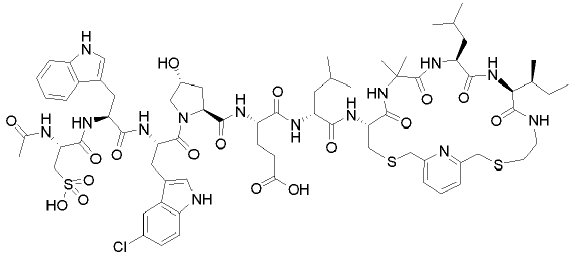
compound Ac-Asp-Trp-5Clw-Hyp-Glu-leu-[Cys(2Lut)-Thp-Leu-Ile-Cys]-NH₂ (GIP-180) of the following formula 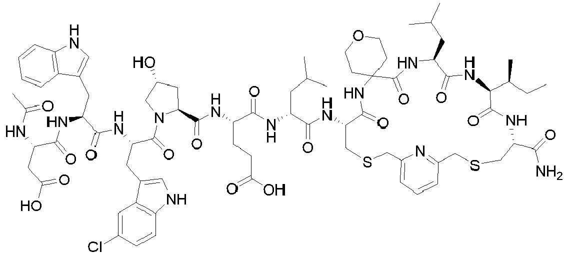
compound Ac-Asp-Trp-5Clw-Hyp-Glu-leu-[Cys(2Lut)-Thp-Leu-Ile-Cys]-OH (GIP-181) of the following formula 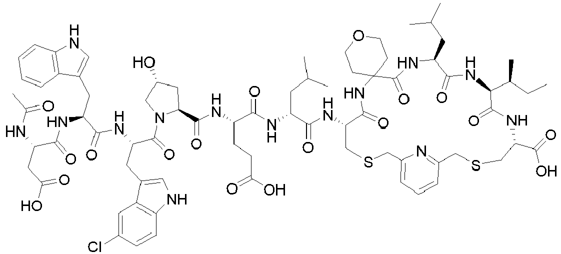
compound Ac-Asp-Trp-5Clw-Hyp-Glu-leu-[Cys(2Lut)-Thp-Leu-Ile-Cysol]-OH (GIP-182) of the following formula 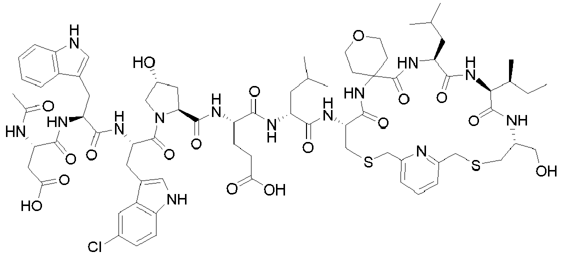
compound Ac-Asp-Trp-5Clw-Nmg-Glu-leu-[Cys(2Lut)-Thp-Leu-Ile-Cys]-NH₂ (GIP-183) of the following formula 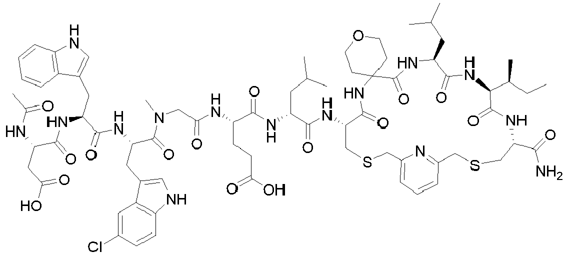
compound Ac-Asp-Trp-5Clw-Nleu-Glu-leu-[Cys(2Lut)-Thp-Leu-Ile-Cys]-NH₂ (GIP-184) of the following formula 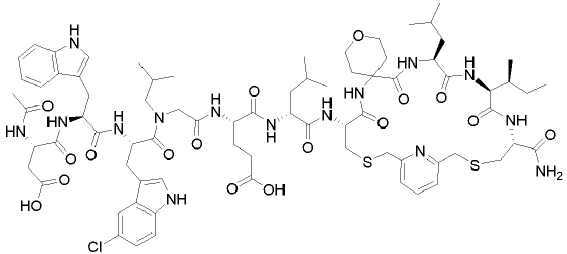
compound Ac-Asp-Trp-5Clw-Nphe-Glu-leu-[Cys(2Lut)-Thp-Leu-Ile-Cys]-NH₂ (GIP-185) of the following formula 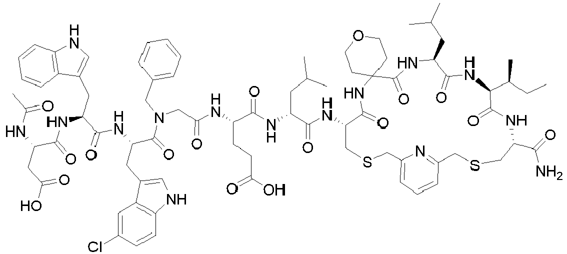
compound Ac-Asp-Trp-5Clw-Nlys-Glu-leu-[Cys(2Lut)-Thp-Leu-Ile-Cys]-NH₂ (GIP-186) of the following formula 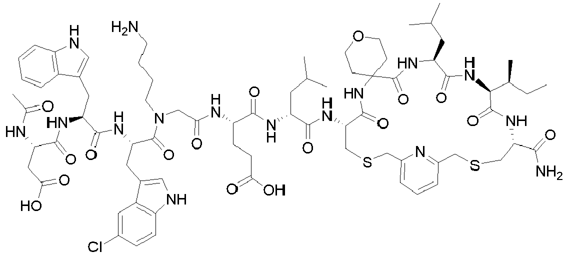
compound Ac-Asp-Trp-5Clw-4Tfp-Glu-leu-[Cys(2Lut)-Thp-Leu-Ile-Cys]-NH₂ (GIP-187) of the following formula 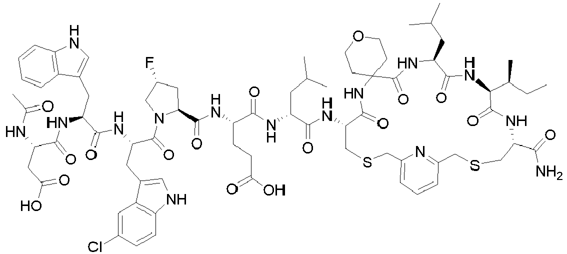
compound Ac-Ser-Trp-5Clw-Hyp-Glu-leu-[Cys(2Lut)-Thp-Leu-Ile-Cys]-NH₂ (GIP-188) of the following formula 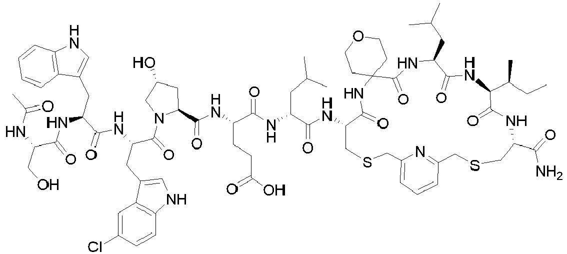
compound Ac-Cya-Trp-5Clw-Hyp-Glu-leu-[Cys(2Lut)-Thp-Leu-Ile-Cys]-NH₂ (GIP-189) of the following formula 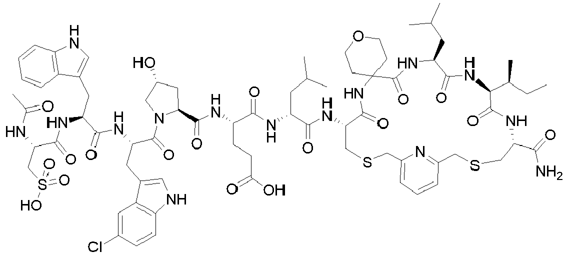
compound Ac-Asn-Trp-5Clw-Hyp-Glu-leu-[Cys(2Lut)-Thp-Leu-Ile-Cys]-NH₂ (GIP-190) of the following formula 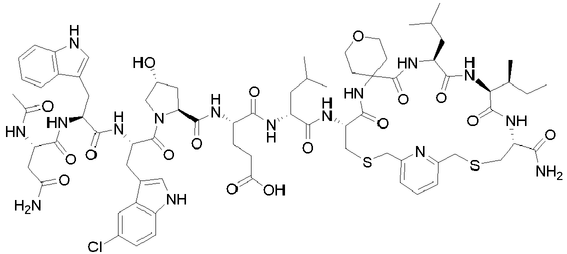
compound Ac-Glu-Trp-5Clw-Hyp-Glu-leu-[Cys(2Lut)-Thp-Leu-Ile-Cys]-NH₂ (GIP-191) of the following formula 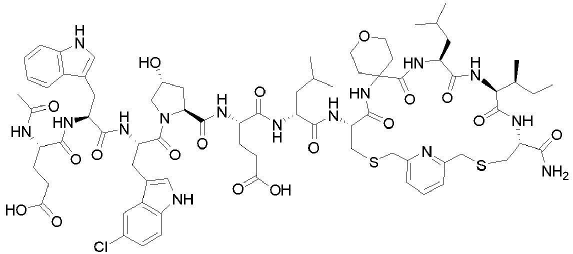
compound Ac-Asp-Trp-5Clw-Hyp-Asp-leu-[Cys(2Lut)-Thp-Leu-Ile-Cys]-NH₂ (GIP-192) of the following formula 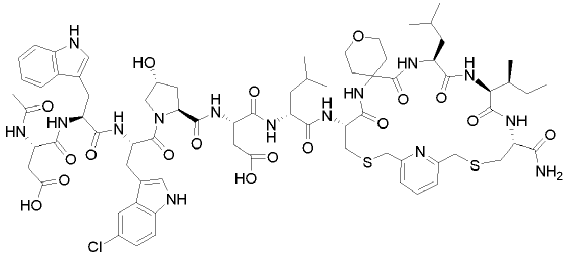
compound Ac-Asp-Trp-5Clw-Hyp-Gln-leu-[Cys(2Lut)-Thp-Leu-Ile-Cys]-NH₂ (GIP-193) of the following formula 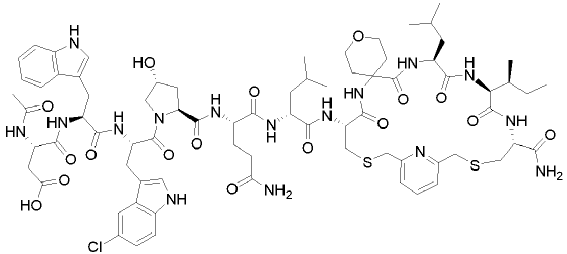
compound Ac-Asp-Trp-5Clw-Hyp-Hse-leu-[Cys(2Lut)-Thp-Leu-Ile-Cys]-NH₂ (GIP-194) of the following formula 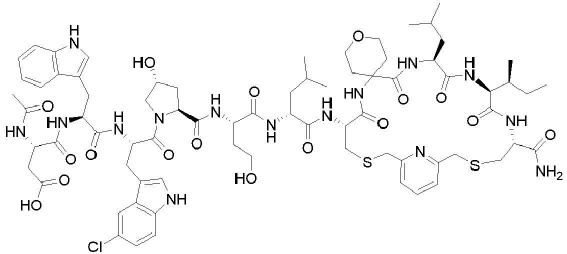
compound Ac-Hse-Trp-5Clw-Hyp-Glu-leu-[Cys(2Lut)-Thp-Leu-Ile-Cys]-NH₂ (GIP-195) of the following formula 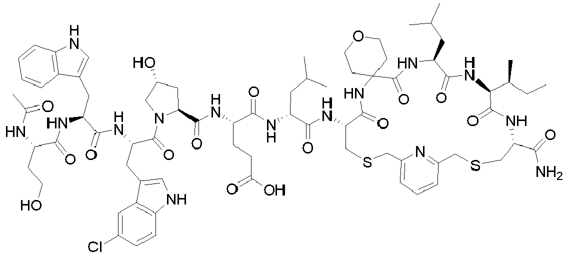
compound Ac-Asp-Trp-5Clw-Nglu-Glu-leu-[Cys(2Lut)-Thp-Leu-Ile-Cys]-NH₂ (GIP-196) of the following formula 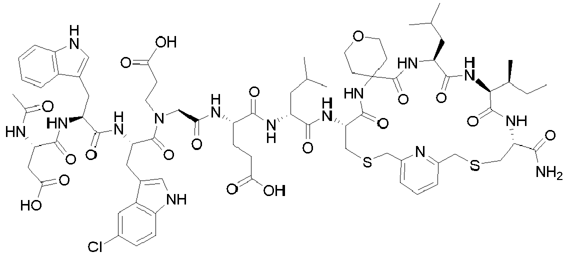
compound Ac-Hse-Trp-5Brw-Hyp-Glu-leu-[Cys(2Lut)-Thp-Leu-Ile-Cys]-NH₂ (GIP-197) of the following formula 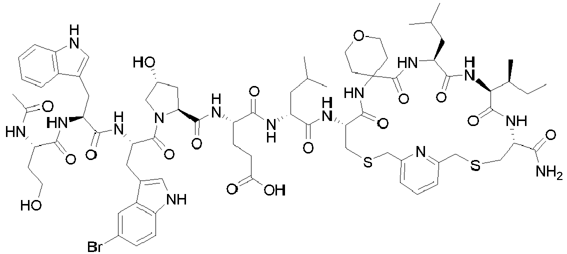
compound HO-Succinyl-Trp-5Clw-Pro-Glu-leu-[Cys(3MeBn)-Aib-Leu-Ile-Cys]-NH₂ (GIP-198) of the following formula 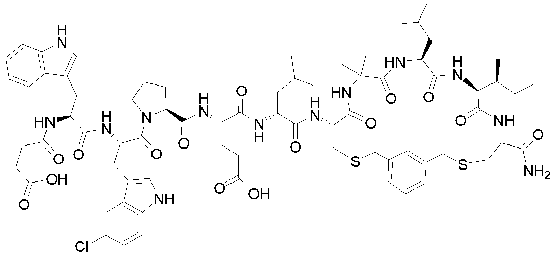
compound HO-Succinyl-Trp-5Clw-Pro-Glu-leu-[Cys(3MeBn)-Thp-Leu-Ile-Cys]-NH₂ (GIP-199) of the following formula 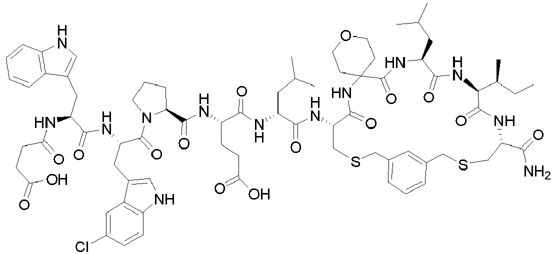
compound Ac-Asp-Trp-5Clw-Hyp-Glu-leu-[Hcy(2Lut)-Thp-Leu-Ile-Cys]-NH₂ (GIP-200) of the following formula 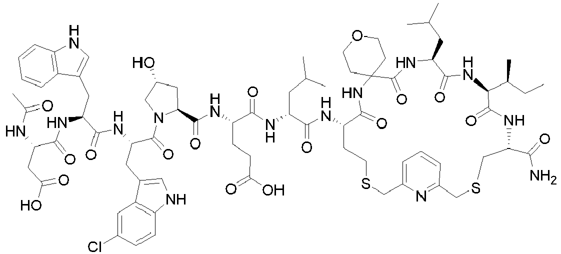
compound Ac-Asp-Trp-5Clw-Hyp-Glu-leu-[Cys(2Lut)-Thp-Leu-Ile-Hcy]-NH₂ (GIP-201) of the following formula 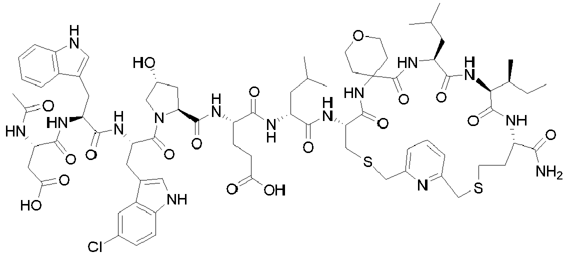
compound Ac-Asp-Trp-5Clw-Hyp-Glu-leu-[Hcy(2Lut)-Thp-Leu-Ile-Hcy]-NH₂ (GIP-202) of the following formula 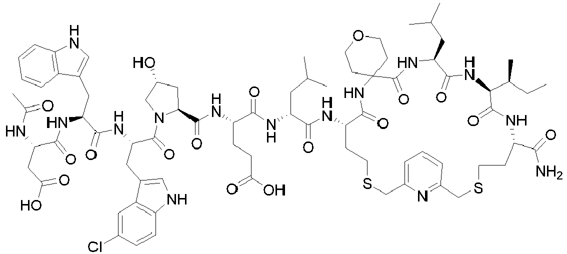
compound Ac-Asp-Trp-5Clw-Hyp-Glu-leu-[Cys(2Lut)-Eca-Leu-Ile-Cys]-NH₂ (GIP-203) of the following formula 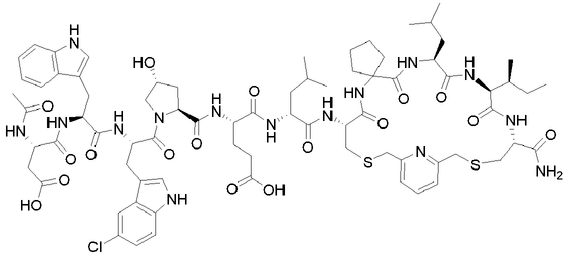
compound Ac-Gln-Trp-5Clw-Hyp-Glu-leu-[Cys(2Lut)-Thp-Leu-Ile-Cys]-NH₂ (GIP-204) of the following formula 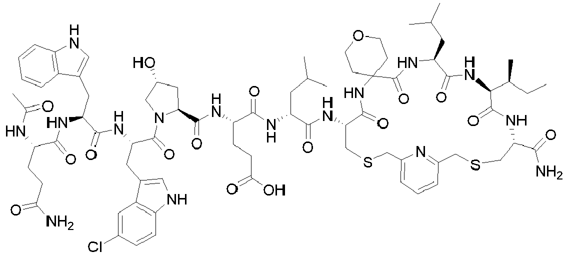
compound Ac-Asp-Trp-5Clw-Hyp-Asn-leu-[Cys(2Lut)-Thp-Leu-Ile-Cys]-NH₂ (GIP-205) of the following formula 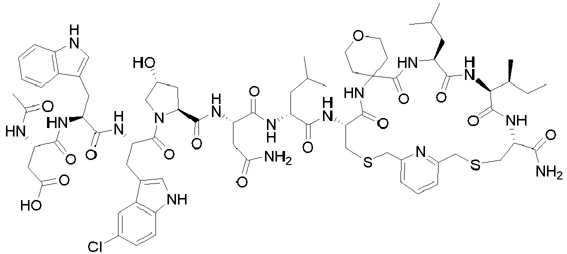
compound Ac-Asp-Trp-5Clw-Hyp-Glu-leu-[Cys(2Lut)-Aic-Leu-Ile-Cys]-NH₂ (GIP-206) of the following formula 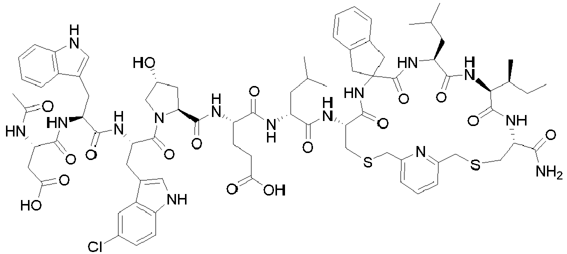
compound Ac-Pse-Trp-5Clw-Hyp-Glu-leu-[Cys(2Lut)-Thp-Leu-Ile-Cys]-NH₂ (GIP-207) of the following formula 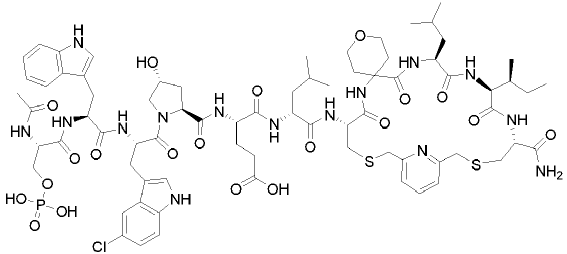
compound Ac-Asp-Trp-5Clw-Hyp-Pse-leu-[Cys(2Lut)-Thp-Leu-Ile-Cys]-NH₂ (GIP-208) of the following formula 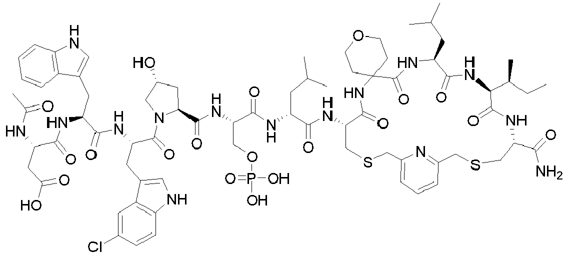
compound Ac-Asp-Mcf-5Clw-Hyp-Glu-leu-[Cys(2Lut)-Thp-Leu-Ile-Cys]-NH₂ (GIP-209) of the following formula 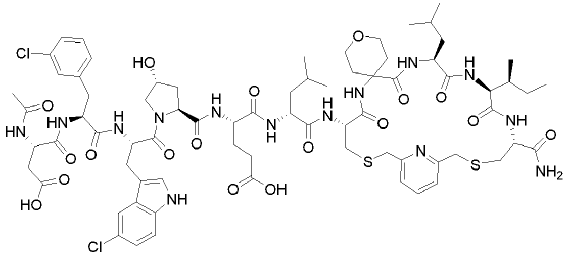
compound Ac-Asp-Pcf-5Clw-Hyp-Glu-leu-[Cys(2Lut)-Thp-Leu-Ile-Cys]-NH₂ (GIP-210) of the following formula 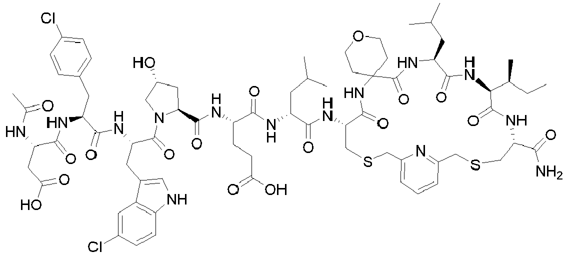
compound Ac-Asp-Eaa-5Clw-Hyp-Glu-leu-[Cys(2Lut)-Thp-Leu-Ile-Cys]-NH₂ (GIP-211) of the following formula 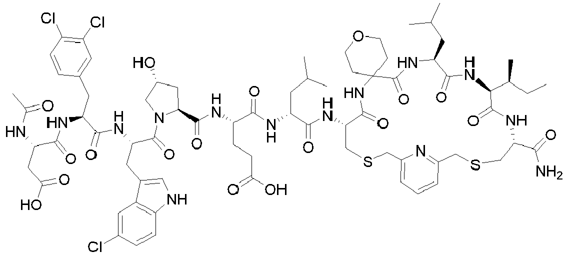
compound Ac-Asp-Egm-5Clw-Hyp-Glu-leu-[Cys(2Lut)-Thp-Leu-Ile-Cys]-NH₂ (GIP-212) of the following formula 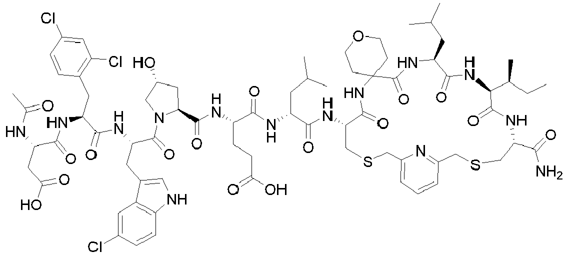
compound Ac-Asp-Trp-Eaa-Hyp-Glu-leu-[Cys(2Lut)-Thp-Leu-Ile-Cys]-NH₂ (GIP-213) of the following formula 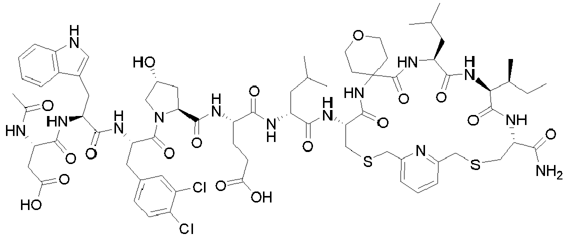
compound HO-Glutar-Trp-5Clw-Hyp-Glu-leu-[Cys(2Lut)-Thp-Leu-Ile-Cys]-NH₂ (GIP-214) of the following formula 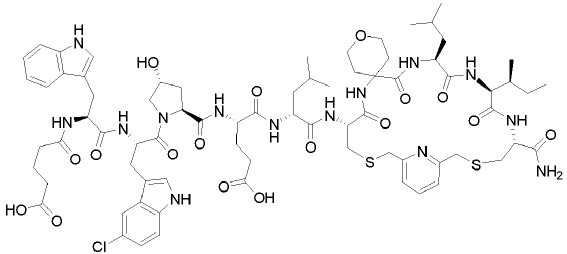
compound Ac-Asp-Trp-5Clw-Dtc-Glu-leu-[Cys(2Lut)-Thp-Leu-Ile-Cys]-NH₂ (GIP-215) of the following formula 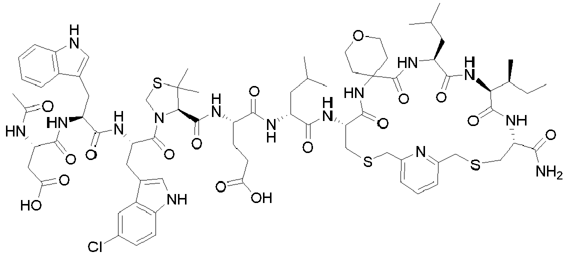
compound Ac-Asp-Trp-5Clw-Oxa-Glu-leu-[Cys(2Lut)-Thp-Leu-Ile-Cys]-NH₂ (GIP-216) of the following formula 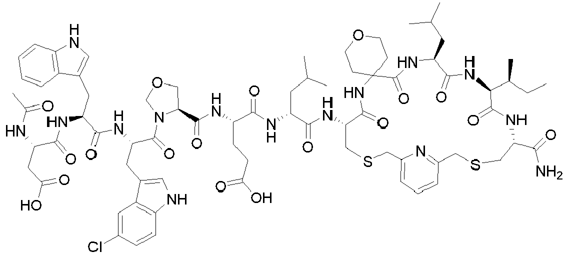
compound Ac-Asp-Trp-5Clw-Hyp-Nme-Leu-[Cys(2Lut)-Thp-Leu-Ile-Cys]-NH₂ (GIP-217) of the following formula 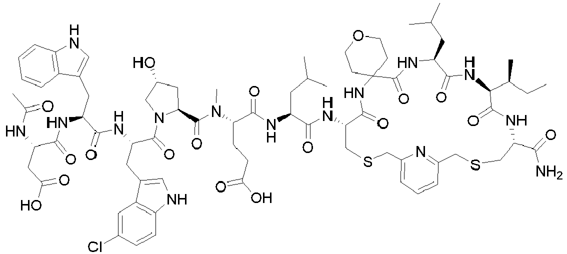
compound Ac-Asp-Trp-5Clw-Hyp-Glu-Ile-[Cys(2Lut)-Thp-Leu-Ile-Cys]-NH₂ (GIP-218) of the following formula 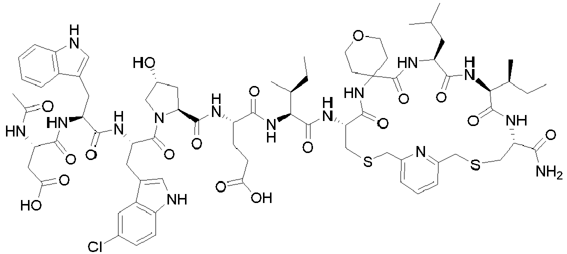
compound Ac-Asp-Trp-5Clw-Hyp-Glu-Val-[Cys(2Lut)-Thp-Leu-Ile-Cys]-NH₂ (GIP-219) of the following formula 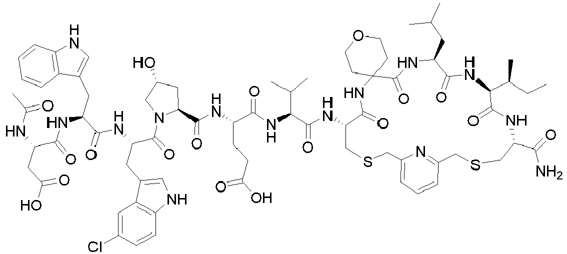
compound Ac-Asp-Hyw-5Clw-Hyp-Glu-leu-[Cys(2Lut)-Thp-Leu-Ile-Cys]-NH₂ (GIP-220) of the following formula 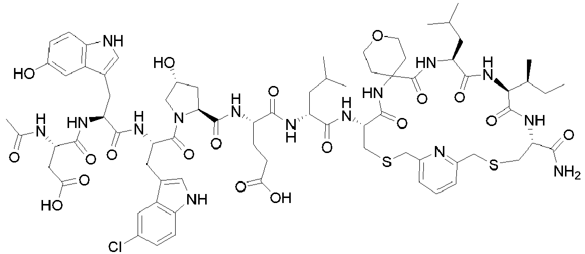
compound Ac-Asp-7Fw-5Clw-Hyp-Glu-leu-[Cys(2Lut)-Thp-Leu-Ile-Cys]-NH₂ (GIP-221) of the following formula 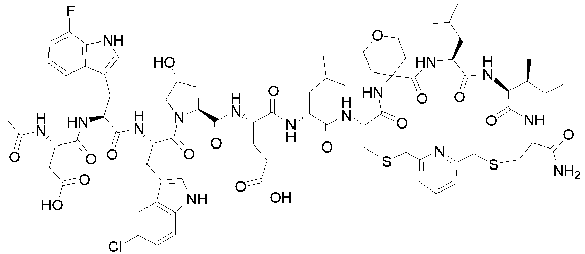
compound Ac-Asp-Trp-5Clw-Eaz-Glu-leu-[Cys(2Lut)-Thp-Leu-Ile-Cys]-NH₂ (GIP-222) of the following formula 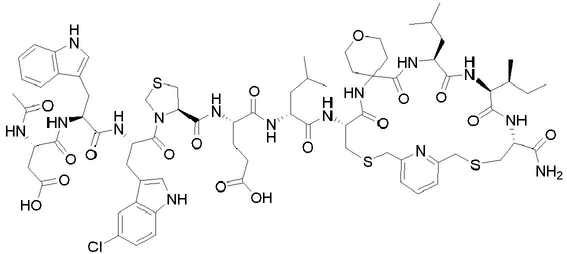
compound Ac-Asp-Trp-5Clw-Hyp-glu-Leu-[Cys(2Lut)-Thp-Leu-Ile-Cys]-NH₂ (GIP-223) of the following formula 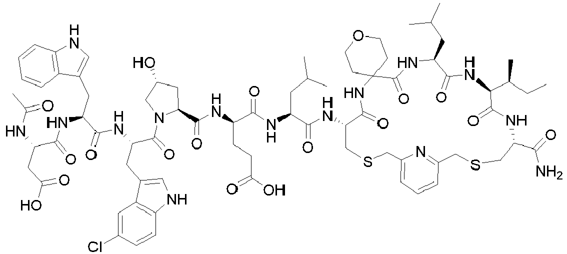
compound Ac-Asp-Phe-5Clw-Hyp-Glu-leu-[Cys(2Lut)-Thp-Leu-Ile-Cys]-NH₂ (GIP-224) of the following formula 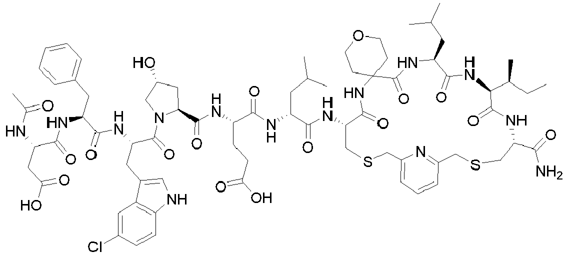
compound Ac-Asp-1Qi-5Clw-Hyp-Glu-leu-[Cys(2Lut)-Thp-Leu-Ile-Cys]-NH₂ (GIP-225) of the following formula 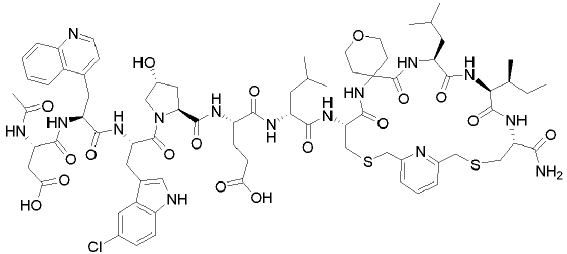
compound Ac-Asp-Egp-5Clw-Hyp-Glu-leu-[Cys(2Lut)-Thp-Leu-Ile-Cys]-NH₂ (GIP-226) of the following formula 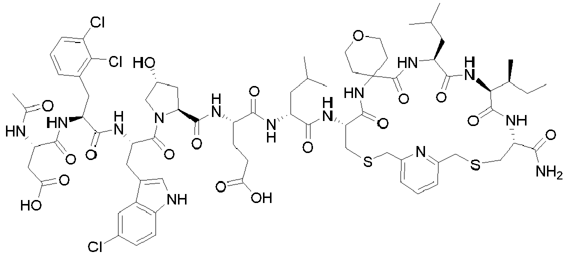
compound Ac-Asp-Trp-7Fw-Hyp-Glu-leu-[Cys(2Lut)-Thp-Leu-Ile-Cys]-NH₂ (GIP-227) of the following formula 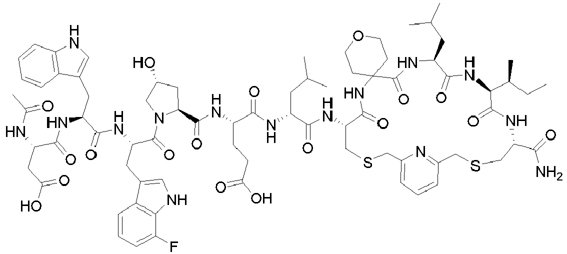
compound Ac-Asp-Trp-5Clw-Hyp-Glu-leu-[Cys(2Lut)-Phe-Leu-Ile-Cys]-NH₂ (GIP-228) of the following formula 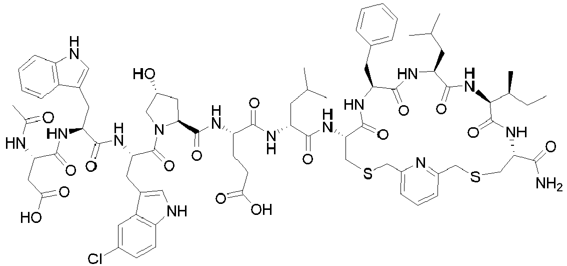
compound Ac-Asp-Trp-5Clw-Hyp-Glu-leu-[Cys(2Lut)-Thp-Ile-Ile-Cys]-NH₂ (GIP-229) of the following formula 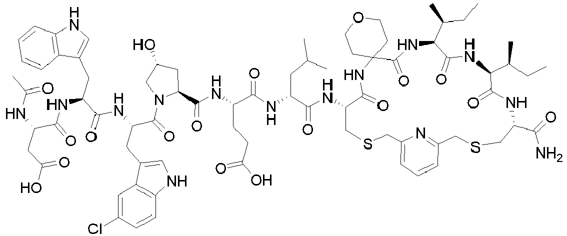
compound Ac-Asp-Trp-5Clw-Hyp-Glu-leu-[Cys(2Lut)-Thp-Arg-Ile-Cys]-NH₂ (GIP-230) of the following formula 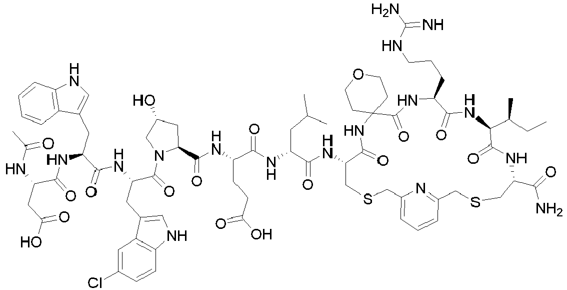
compound Ac-Asp-Trp-5Clw-Hyp-Glu-leu-[Cys(2Lut)-Thp-Glu-Ile-Cys]-NH₂ (GIP-231) of the following formula 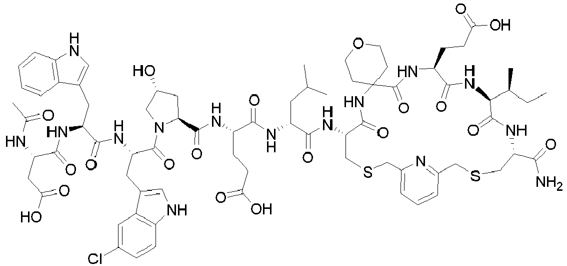
compound Ac-Asp-Trp-5Clw-Hyp-Glu-leu-[Cys(2Lut)-Thp-Phe-Ile-Cys]-NH₂ (GIP-232) of the following formula 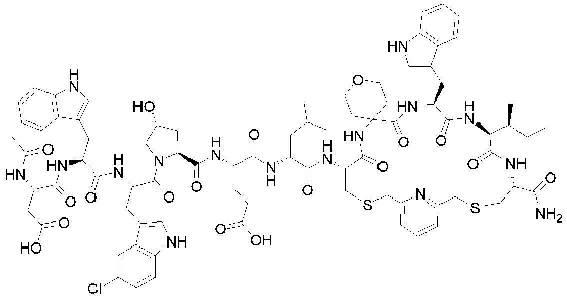
compound Ac-Asp-Trp-5Clw-Hyp-Glu-leu-[Cys(2Lut)-Thp-Trp-Ile-Cys]-NH₂ (GIP-233) of the following formula 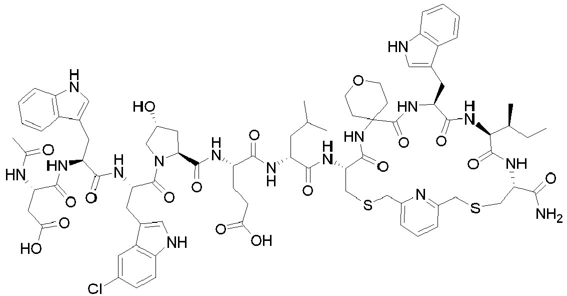
compound Ac-Asp-Trp-5Clw-Hyp-Glu-leu-[Cys(2Lut)-Trp-Leu-Ile-Cys]-NH₂ (GIP-234) of the following formula 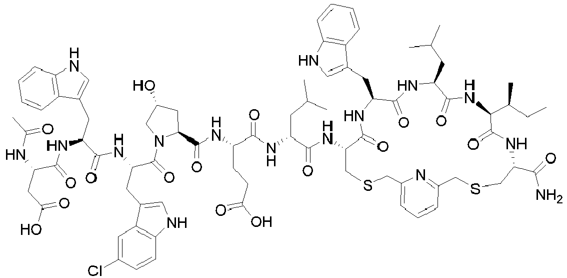
compound Ac-Asp-Trp-5Clw-Hyp-Glu-leu-[Cys(2Lut)-Glu-Leu-Ile-Cys]-NH₂ (GIP-235) of the following formula 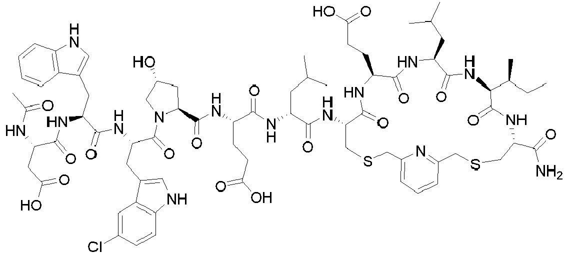
compound Ac-Asp-Trp-5Clw-Hyp-Glu-leu-[Cys(2Lut)-Thp-Ser-Ile-Cys]-NH₂ (GIP-236) of the following formula 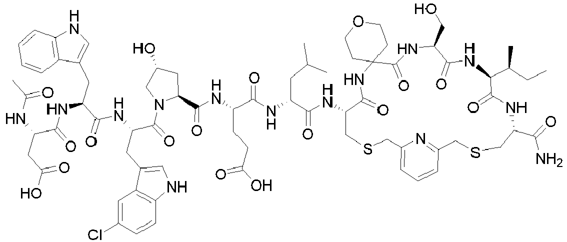
compound Ac-Asp-Trp-5Clw-Hyp-Glu-leu-[Cys(2Lut)-Thp-Leu-Leu-Cys]-NH₂ (GIP-237) of the following formula 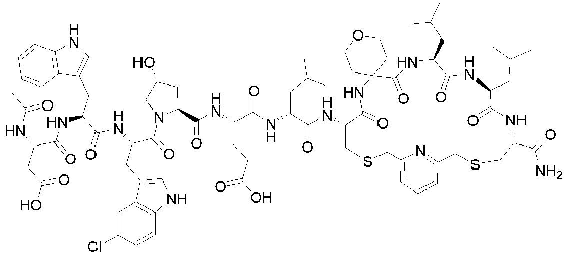
compound Ac-Asp-Trp-5Clw-Hyp-Glu-leu-[Cys(2Lut)-Thp-Leu-Tle-Cys]-NH₂ (GIP-238) of the following formula 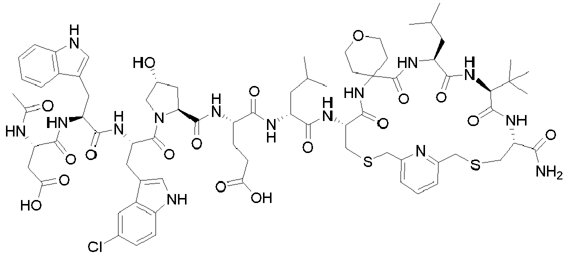
compound Ac-Asp-Trp-5Clw-Tap-Glu-leu-[Cys(2Lut)-Thp-Leu-Ile-Cys]-NH₂ (GIP-239) of the following formula 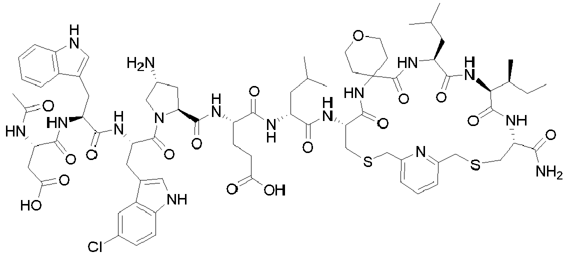
compound Ac-Tyr-Asp-Trp-Trp-Pro-Glu-Leu-[Cys(3MeBn)-Ala-Leu-Ile-Cys]-NH₂ (GIP-240) of the following formula 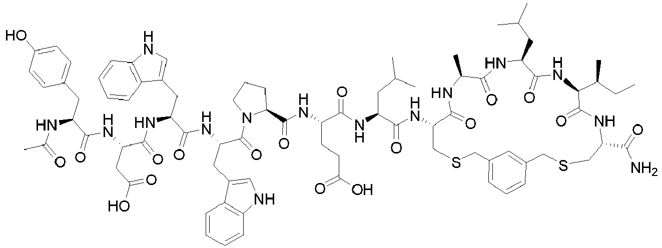
compound H-Met-Tyr-Asp-Trp-Trp-Pro-Glu-Leu-[Cys(3MeBn)-Gln-Leu-Ile-Cys]-NH₂ (GIP-241) of the following formula 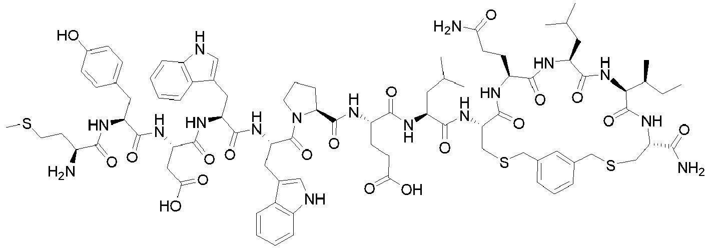
compound H-Met-Tyr-Asp-Trp-Trp-Pro-Glu-Leu-[Cys(3MeBn)-Thr-Leu-Ile-Cys]-NH₂ (GIP-242) of the following formula 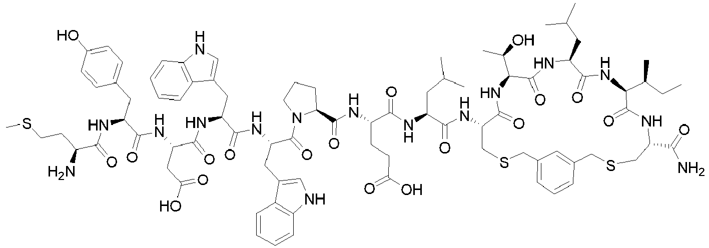
compound H-Met-Tyr-Asp-Trp-Trp-Pro-Glu-Leu-[Cys(3MeBn)-Lys-Leu-Ile-Cys]-NH₂ (GIP-243) of the following formula 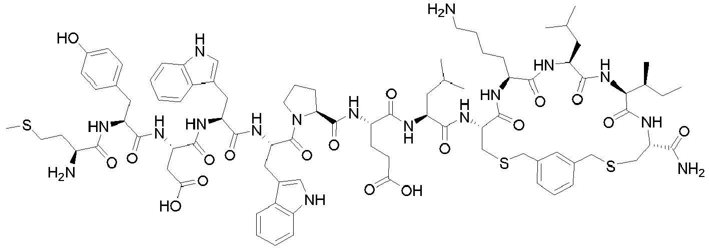
compound Ac-Asp-Trp-Trp-Pro-Glu-Leu-[Cys(3MeBn)-Ala-Leu-Ile-Cys]-NH₂ (GIP-244) of the following formula 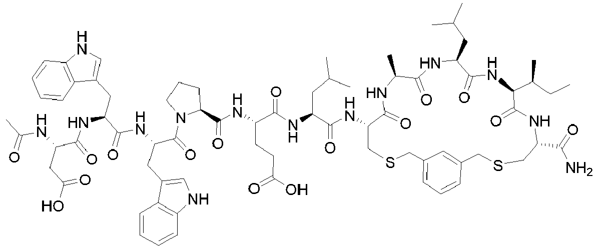
compound H-Met-Tyr-Asp-Trp-Trp-Pro-glu-Leu-[Cys(3MeBn)-Ala-Leu-Ile-Cys]-NH₂ (GIP-245) of the following formula 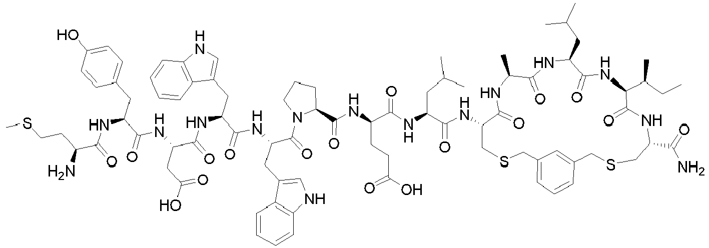
compound H-Met-tyr-Asp-Trp-Trp-Pro-Glu-Leu-[Cys(3MeBn)-Ala-Leu-Ile-Cys]-NH₂ (GIP-246) of the following formula 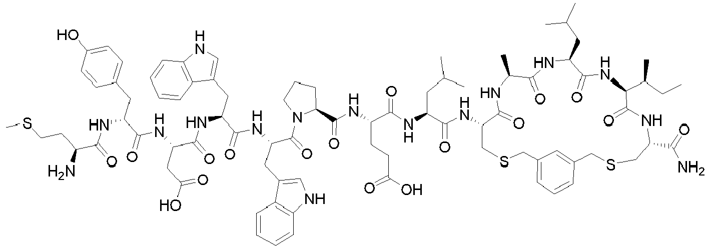
compound H-Met-Tyr-Asp-Trp-Trp-Pro-Glu-leu-[Cys(3MeBn)-Ala-Leu-Ile-Cys]-NH₂ (GIP-247) of the following formula 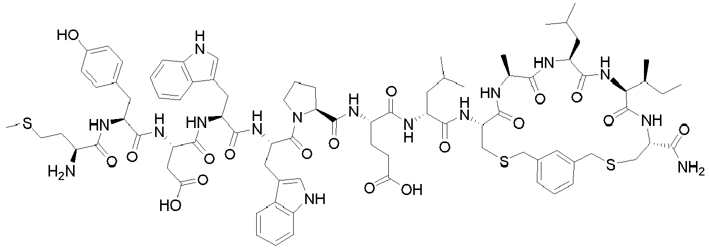
compound H-Met-Tyr-Asp-Trp-Trp-Pro-Glu-Leu-[Cys(3MeBn)-ala-Leu-Ile-Cys]-NH₂ (GIP-248) of the following formula 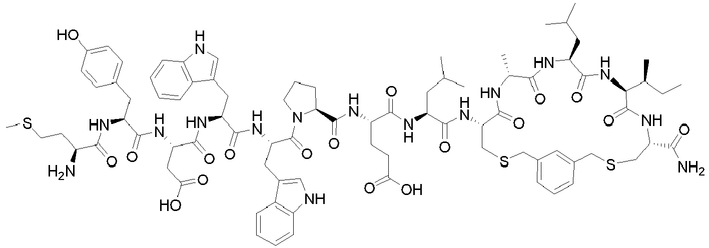
compound H-Met-Ala-Asp-Trp-Trp-Pro-Glu-Leu-[Cys(3MeBn)-Ala-Leu-Ile-Cys]-NH₂ (GIP-249) of the following formula 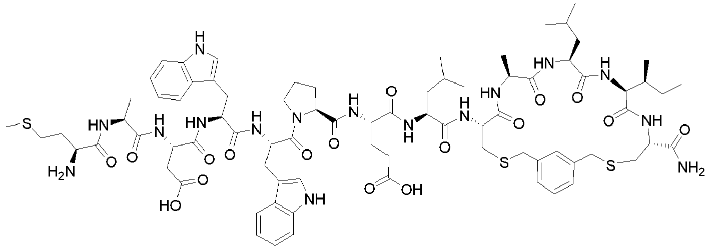
compound Ac-Asp-Trp-Trp-Pro-Glu-Leu-[Cys(3MeBn)-ala-Leu-Ile-AET] (GIP-250) of the following formula 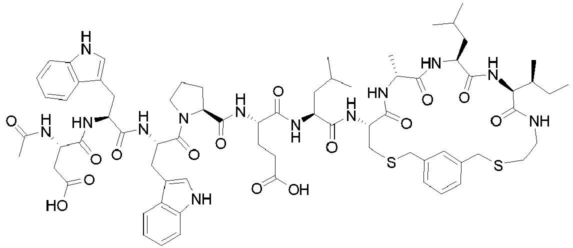
compound Ac-Asp-Trp-Trp-Pro-Glu-Leu-[Cys(3MeBn)-Aib-Leu-Ile-AET] (GIP-251) of the following formula 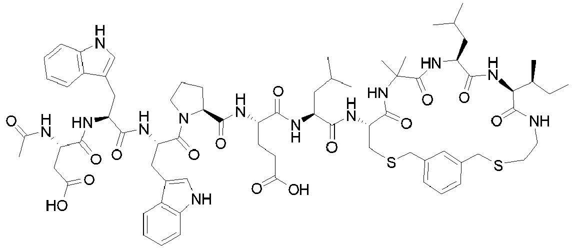
compound Ac-Asp-Trp-1Ni-Oic-Glu-Leu-[Cys(3MeBn)-Aib-Leu-Ile-Cys]-NH₂ (GIP-252) of the following formula 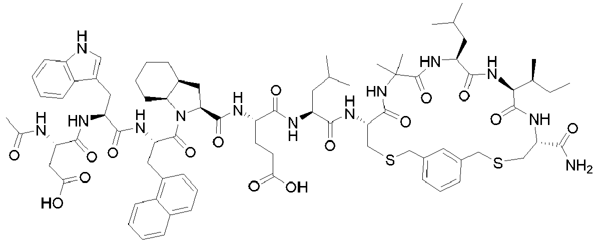
compound Ac-Asp-Trp-1Ni-Pip-Glu-Leu-[Cys(3MeBn)-Aib-Leu-Ile-Cys]-NH₂ (GIP-253) of the following formula 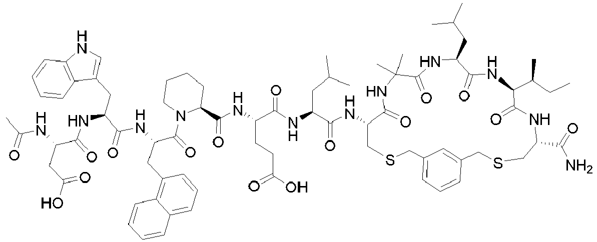
compound H-Met-Tyr-Asp-Trp-Trp-Pro-Glu-Leu-[Cys(3MeBn)-Ala-Leu-Ile-Cys]-NH₂ (GIP-254) of the following formula 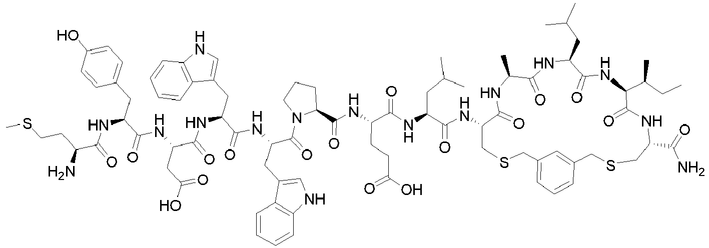
compound HO-Succinyl-Trp-Trp-Pro-Glu-Leu-[Cys(3MeBn)-Ala-Leu-Ile-Cys]-NH₂ (GIP-255) of the following formula 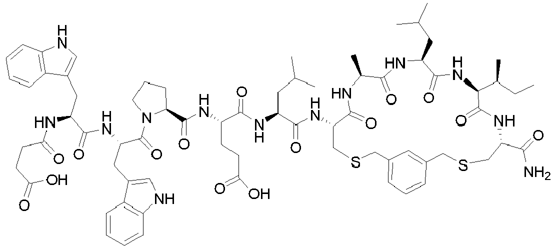
compound HO-Succinyl-Trp-Trp-Pro-Glu-Leu-[Cys(3MeBn)-Ala-Leu-Ile-AET] (GIP-256) of the following formula 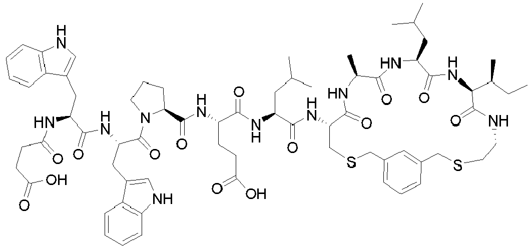
compound HO-Succinyl-Trp-Trp-Pro-Glu-Leu-[Cys(2Lut)-Ala-Leu-Ile-Cys]-NH₂ (GIP-257) of the following formula 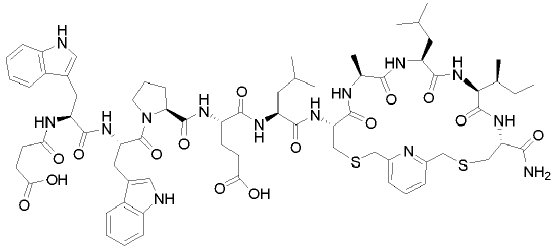
compound HO-Succinyl-Trp-Trp-Pro-Glu-leu-[Cys(3MeBn)-Aib-Leu-Ile-Cys]-NH₂ (GIP-258) of the following formula 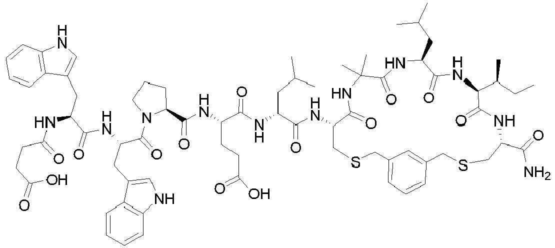
compound HO-Succinyl-Trp-Trp-Pro-Glu-Leu-[Cys(3MeBn)-Aib-Leu-Ile-Cys]-NH₂ (GIP-259) of the following formula 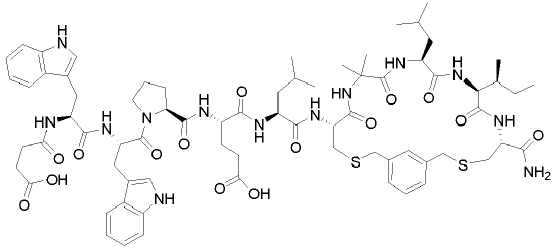
compound HO-Succinyl-Trp-Trp-Pro-Glu-Leu-[Cys(3MeBn)-Aib-Hle-Ile-Cys]-NH₂ (GIP-260) of the following formula 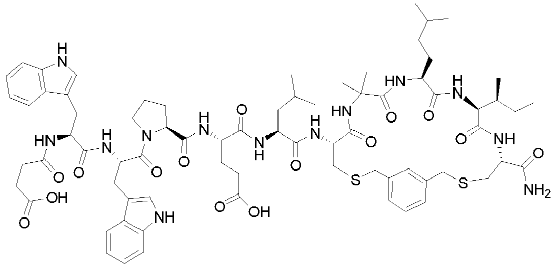
compound HO-Succinyl-Trp-Trp-Pro-Glu-Leu-[Cys(3MeBn)-Thp-Leu-Ile-Cys]-NH₂ (GIP-261) of the following formula 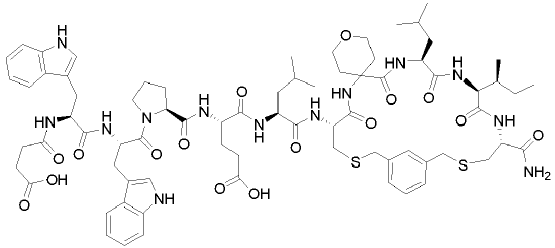
compound Ac-Asp-Ocf-5Clw-Hyp-Glu-leu-[Cys(2Lut)-Thp-Leu-Ile-Cys]-NH₂ (GIP-262) of the following formula 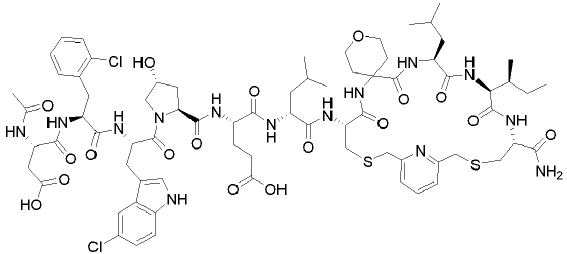
compound Ac-Asp-Egn-5Clw-Hyp-Glu-leu-[Cys(2Lut)-Thp-Leu-Ile-Cys]-NH₂ (GIP-263) of the following formula 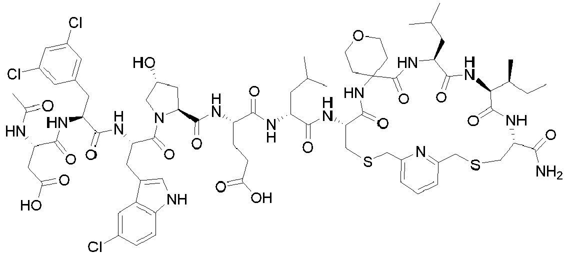
compound Ac-Asp-Trp-Egn-Hyp-Glu-leu-[Cys(2Lut)-Thp-Leu-Ile-Cys]-NH₂ (GIP-264) of the following formula 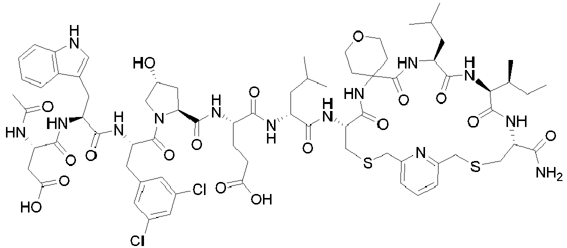
compound Ac-Asp-Trp-Egp-Hyp-Glu-leu-[Cys(2Lut)-Thp-Leu-Ile-Cys]-NH₂ (GIP-265) of the following formula 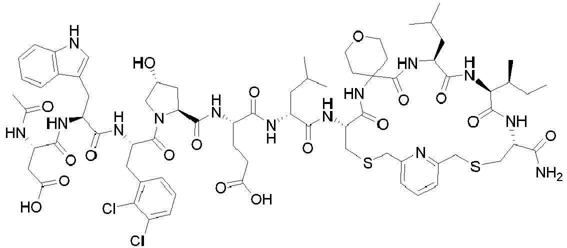
compound Ac-Asp-Trp-5Clw-Hyp-Glu-leu-[Cys(2Lut)-Thp-Leu-Phe-Cys]-NH₂ (GIP-266) of the following formula 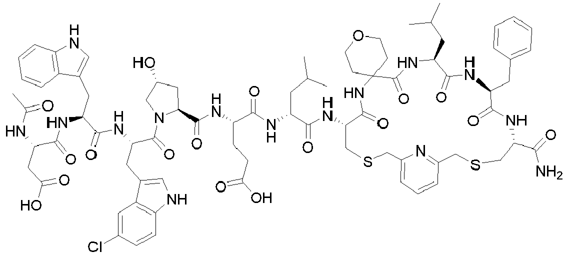
compound Ac-Asp-Trp-7Nw-Hyp-Glu-leu-[Cys(2Lut)-Thp-Leu-Ile-Cys]-NH₂ (GIP-267) of the following formula 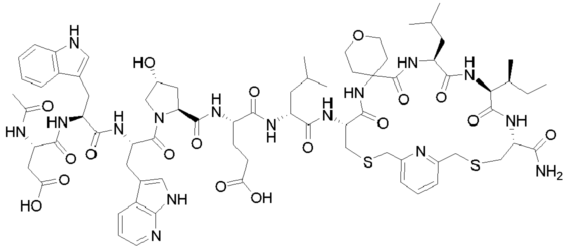
compound DOTA-Ttds-Asp-Trp-Trp-Pro-Glu-leu-[Cys(3MeBn)-ala-Leu-Ile-Cys]-NH₂ (GIP-268) of the following formula 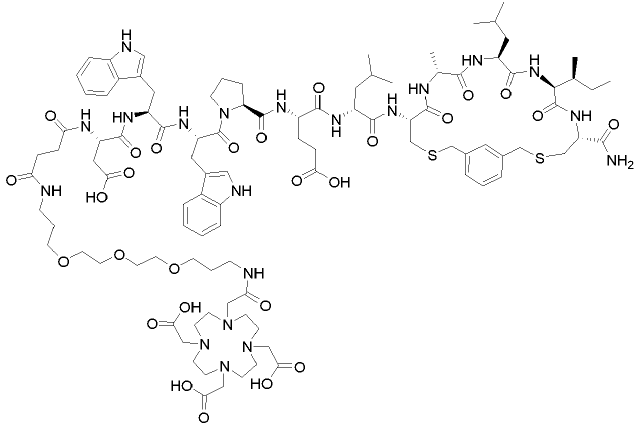
compound DOTA-Ttds-Asp-Trp-Trp-Pro-glu-Leu-[Cys(3MeBn)-ala-Leu-Ile-Cys]-NH₂ (GIP-269) of the following formula 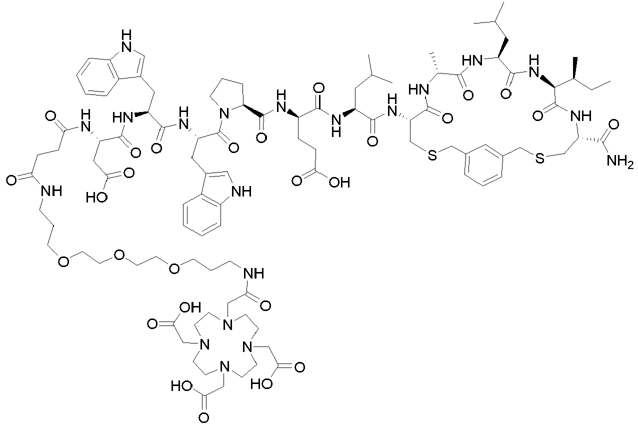
compound DOTA-Ttds-Asp-Trp-Trp-Pro-glu-Leu-[Cys(3MeBn)-Aib-Leu-Ile-Cys]-NH₂ (GIP-270) of the following formula 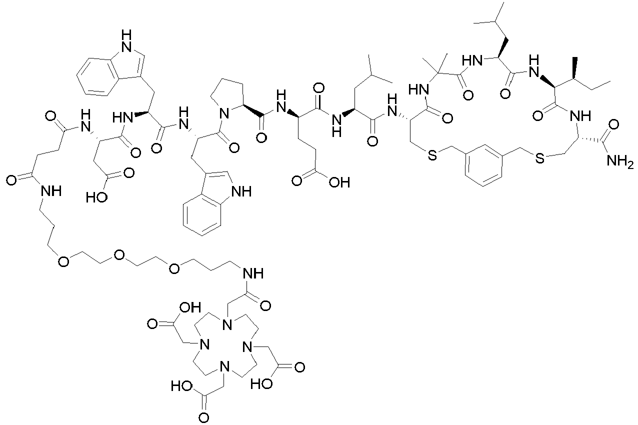
compound DOTA-Ttds-asp-Trp-Trp-Pro-Glu-Leu-[Cys(3MeBn)-ala-Leu-Ile-Cys]-NH₂ (GIP-271) of the following formula 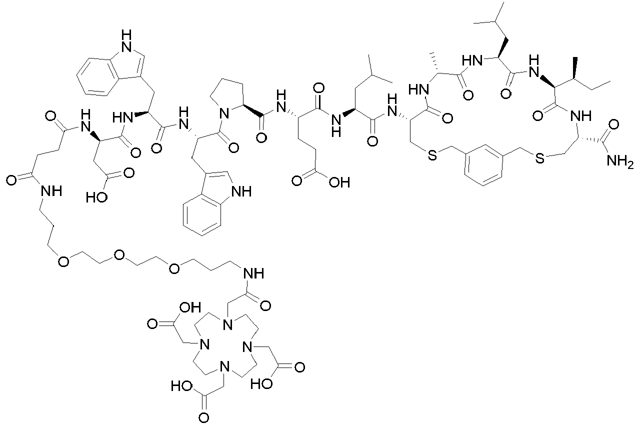
compound Ac-Asp-2Ni-Trp-Pro-Glu-Leu-[Cys(3MeBn)-Aib-Leu-Ile-Cys]-Ttds-Lys(DOTA)-NH₂ (GIP-272) of the following formula 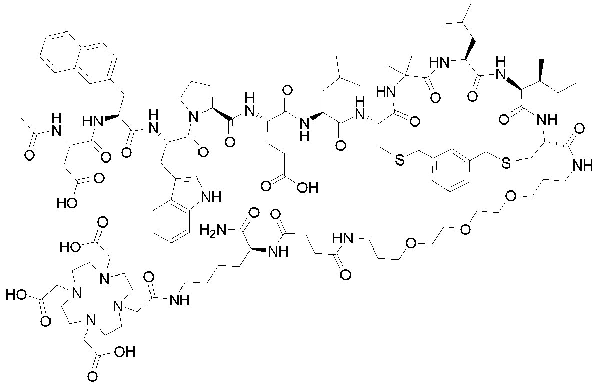
compound Ac-Asp-Bta-Trp-Pro-Glu-Leu-[Cys(3MeBn)-Aib-Leu-Ile-Cys]-Ttds-Lys(DOTA)-NH₂ (GIP-273) of the following formula 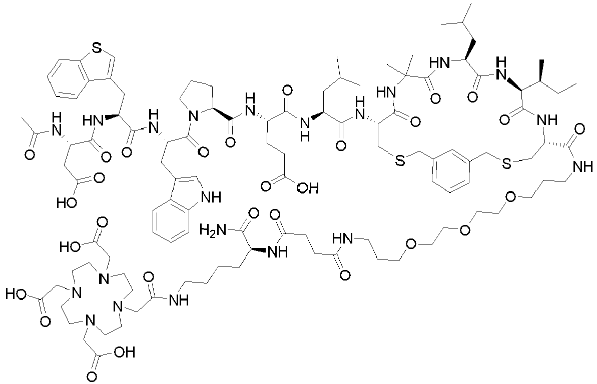
compound Ac-Asp-Trp-Trp-Pro-Nme-Leu-[Cys(3MeBn)-Aib-Leu-Ile-Cys]-Ttds-Lys(DOTA)-NH₂ (GIP-274) of the following formula 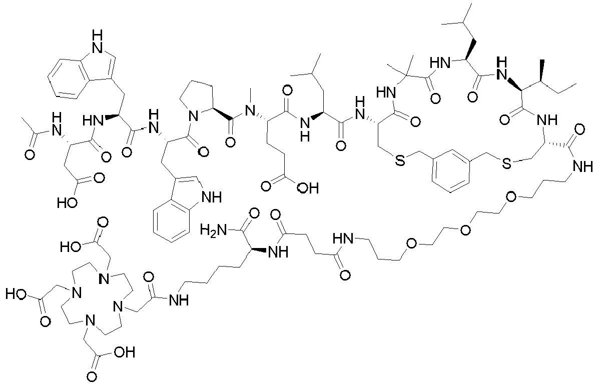
compound Ac-Asp-Trp-Trp-Pro-Glu-Tle-[Cys(3MeBn)-Aib-Leu-Ile-Cys]-Ttds-Lys(DOTA)-NH₂ (GIP-275) of the following formula 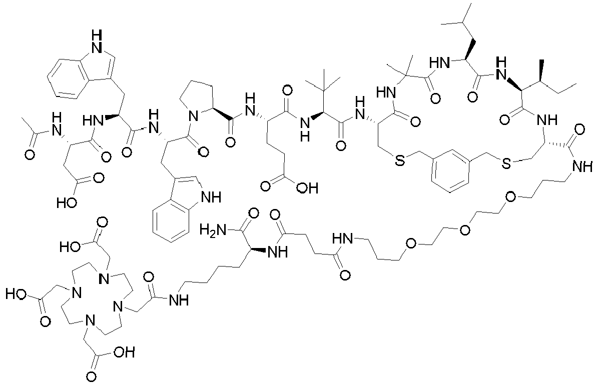
compound Ac-Asp-Trp-Trp-Pro-Glu-Leu-[Cys(3MeBn)-Aib-Tle-Ile-Cys]-Ttds-Lys(DOTA)-NH₂ (GIP-276) of the following formula 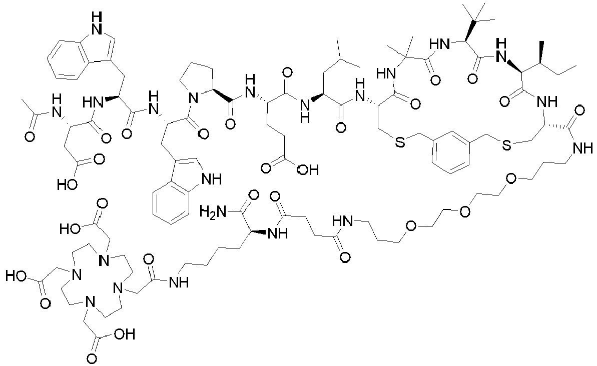
compound Ac-Asp-5Clw-Trp-Pro-Glu-Leu-[Cys(3MeBn)-Aib-Leu-Ile-Cys]-Ttds-Lys(DOTA)-NH₂ (GIP-277) of the following formula 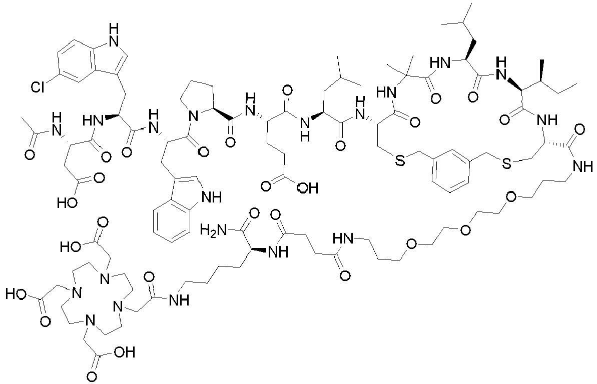
compound Ac-Asp-Trp-Trp-Pro-Glu-Leu-[Cys(3MeBn)-Aib-Leu-Cha-Cys]-Ttds-Lys(DOTA)-NH₂ (GIP-278) of the following formula 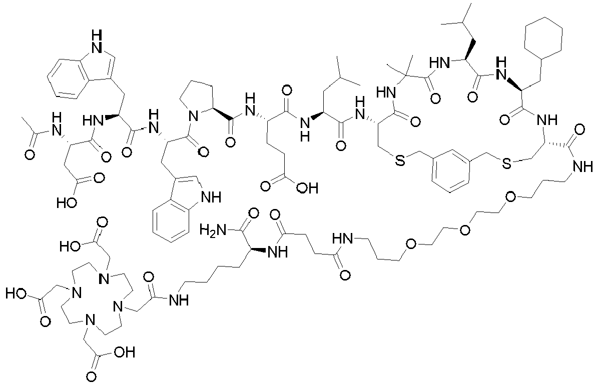
compound 4ClPhp-Asp-Trp-Trp-Pro-Glu-leu-[Cys(3MeBn)-Aib-Leu-Ile-Cys]-Ttds-Lys(DOTA)-NH₂ (GIP-279) of the following formula 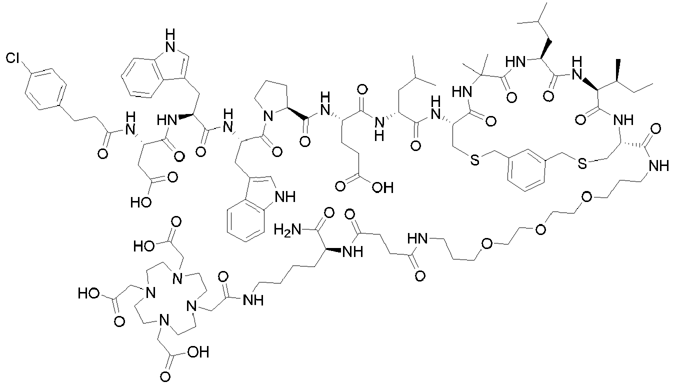
compound Nphlp-Asp-Trp-Trp-Pro-Glu-leu-[Cys(3MeBn)-Aib-Leu-Ile-Cys]-Ttds-Lys(DOTA)-NH₂ (GIP-280) of the following formula 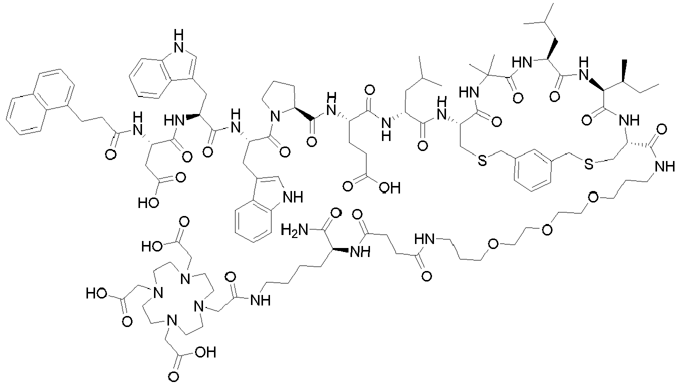
compound Nph2p-Asp-Trp-Trp-Pro-Glu-leu-[Cys(3MeBn)-Aib-Leu-Ile-Cys]-Ttds-Lys(DOTA)-NH₂ (GIP-281) of the following formula 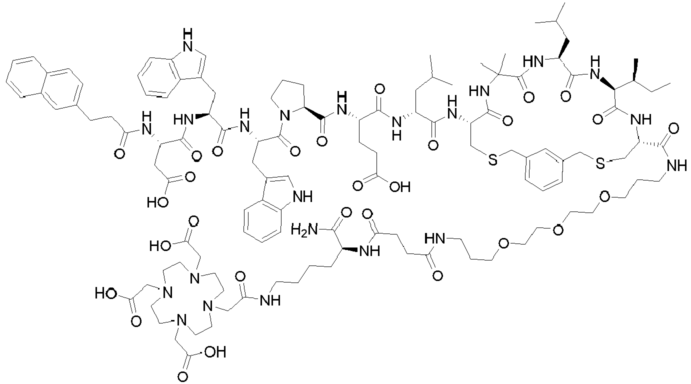
compound Ac-Asp-Trp-6Clw-Pro-Glu-leu-[Cys(3MeBn)-Aib-Leu-Ile-Cys]-Ttds-Lys(DOTA)-NH₂ (GIP-282) of the following formula 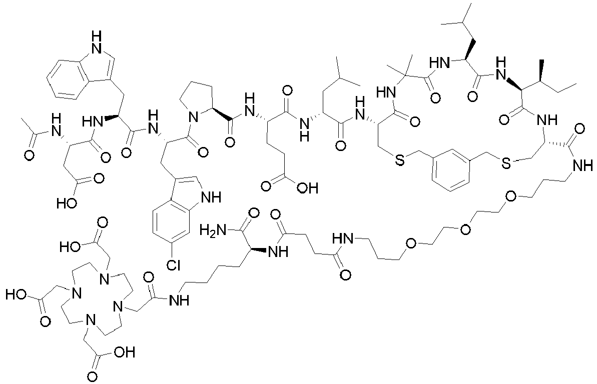
compound DOTA-Ttds-Asp-6Fw-Trp-Pro-Glu-leu-[Cys(3MeBn)-Aib-Leu-Ile-AET] (GIP-283) of the following formula 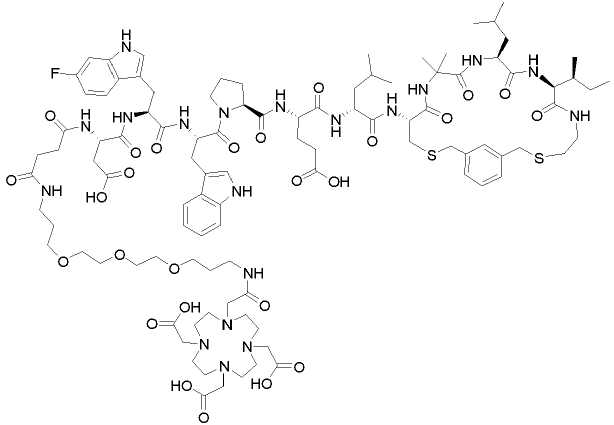
compound DOTA-APAc-Asp-Gly-5Clw-Hyp-Glu-leu-[Cys(2Lut)-Thp-Leu-Ile-AET] (GIP-284) of the following formula 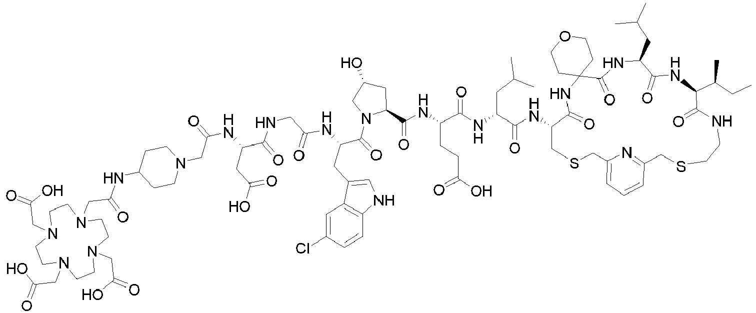
compound DOTA-Ttds-Asp-Trp-Trp-Pro-Glu-leu-[Cys(3MeBn)-ala-Leu-Ile-Cys]-OH (GIP-285) of the following formula 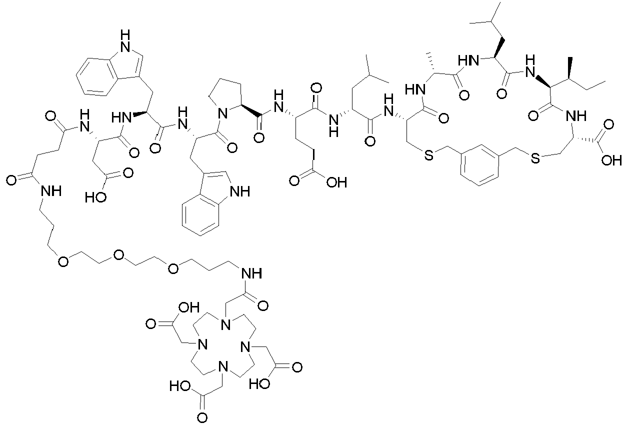
compound HO-Succinyl-Trp-Trp-Pro-Glu-leu-[Cys(tMeBn(DOTA-PP))-ala-Leu-Ile-Cys]-NH₂ (GIP-286) of the following formula 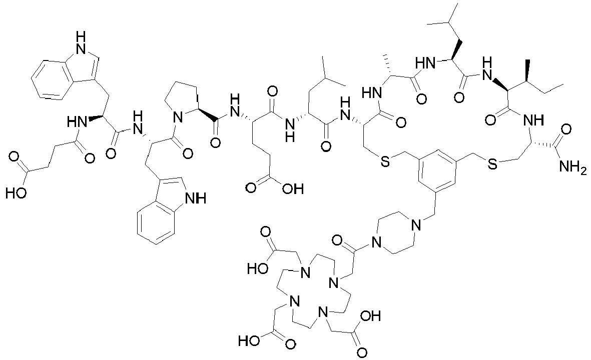
compound HO-Succinyl-Trp-Trp-Pro-Glu-leu-[Cys(tMeBn(DOTA-AET))-ala-Leu-Ile-Cys]-NH₂ (GIP-287) of the following formula 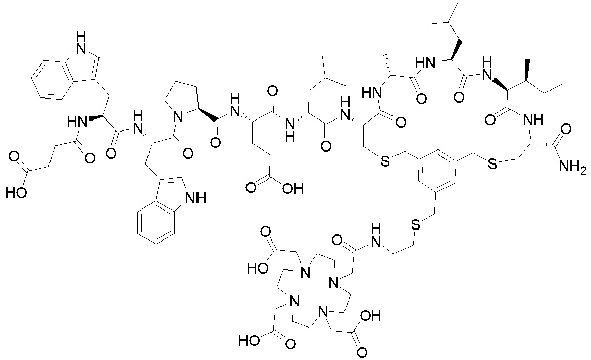
compound HO-Succinyl-Trp-Trp-Pro-Glu-leu-[Cys(3MeBn)-ala-Leu-Ile-Cys]-Ttds-Lys(DOTA)-NH₂ (GIP-288) of the following formula 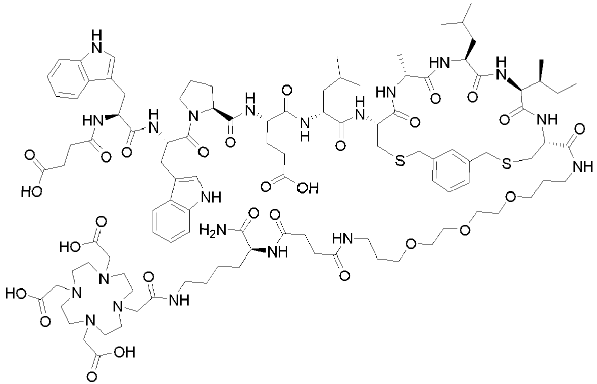
compound Ac-Asp-Trp-Nmw-Pro-Glu-Leu-[Cys(3MeBn)-Aib-Leu-Ile-Cys]-Ttds-Lys(DOTA)-NH₂ (GIP-289) of the following formula 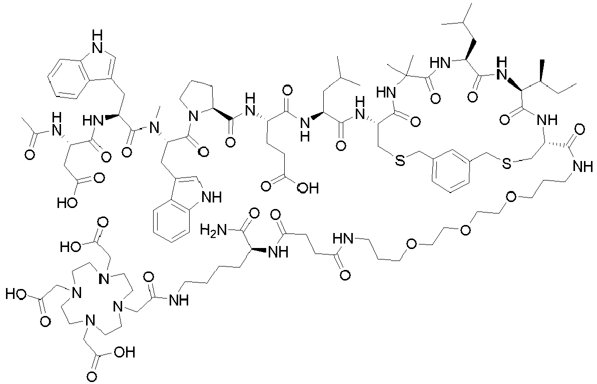
compound Ac-Asp-Trp-Trp-Pro-Glu-Nml-[Cys(3MeBn)-Aib-Leu-Ile-Cys]-Ttds-Lys(DOTA)-NH₂ (GIP-290) of the following formula 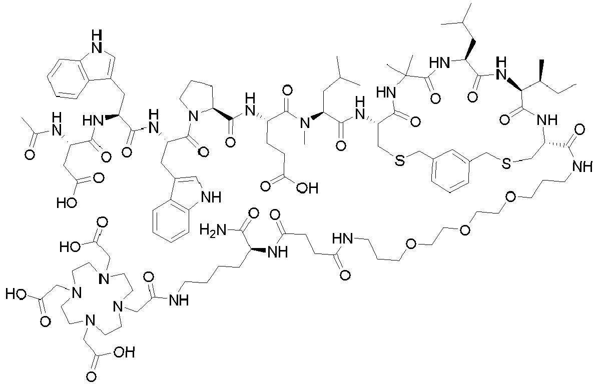
compound Ac-Asp-Trp-Trp-Pro-Glu-Leu-[Nmc(3MeBn)-Aib-Leu-Ile-Cys]-Ttds-Lys(DOTA)-NH₂ (GIP-291) of the following formula 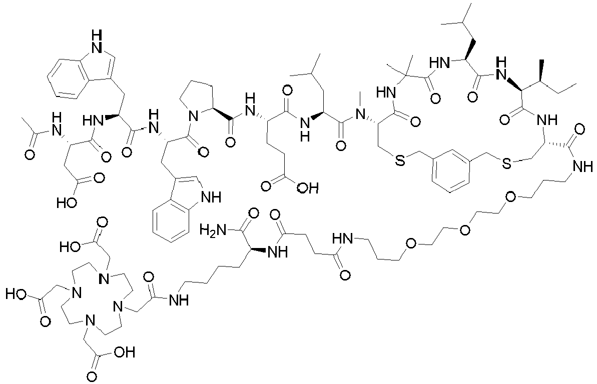
compound Ac-Asp-Trp-Trp-Pro-Glu-Leu-[Cys(3MeBn)-Aib-Leu-Ile-Nmc]-Ttds-Lys(DOTA)-NH₂ (GIP-292) of the following formula 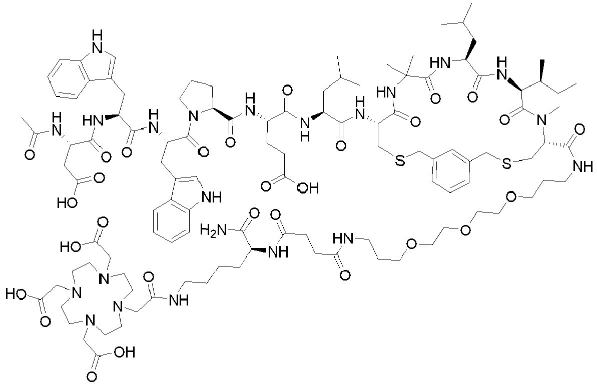
compound Ac-Asp-1Ni-Trp-Pro-Glu-Leu-[Cys(3MeBn)-Aib-Leu-Ile-Cys]-Ttds-Lys(DOTA)-NH₂ (GIP-293) of the following formula 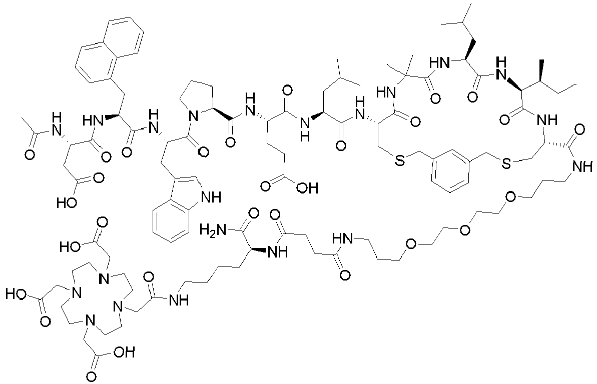
compound Ac-Asp-Trp-2Ni-Pro-Glu-Leu-[Cys(3MeBn)-Aib-Leu-Ile-Cys]-Ttds-Lys(DOTA)-NH₂ (GIP-294) of the following formula 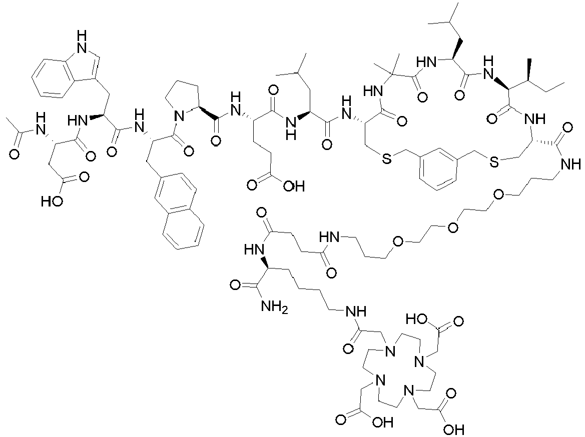
compound Ac-Asp-Trp-Trp-Pro-Glu-Cha-[Cys(3MeBn)-Aib-Leu-Ile-Cys]-Ttds-Lys(DOTA)-NH₂ (GIP-295) of the following formula 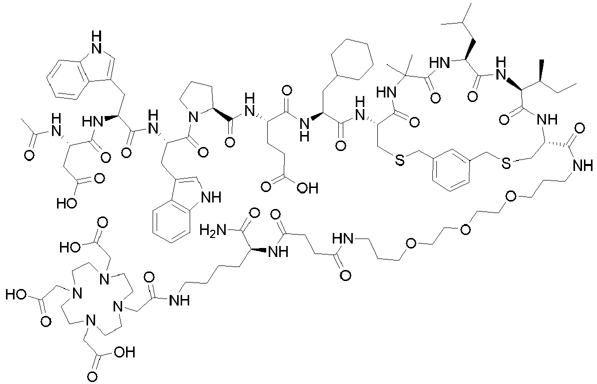
compound Ac-Asp-Trp-Trp-Pro-Glu-Aml-[Cys(3MeBn)-Aib-Leu-Ile-Cys]-Ttds-Lys(DOTA)-NH₂ (GIP-296) of the following formula 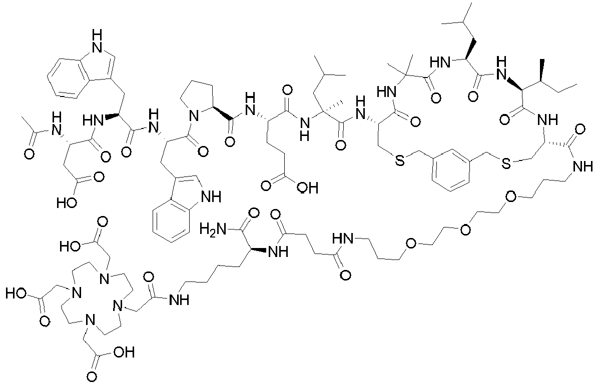
compound Ac-Asp-Nmw-Trp-Pro-Glu-Leu-[Cys(3MeBn)-Aib-Leu-Ile-Cys]-Ttds-Lys(DOTA)-NH₂ (GIP-297) of the following formula 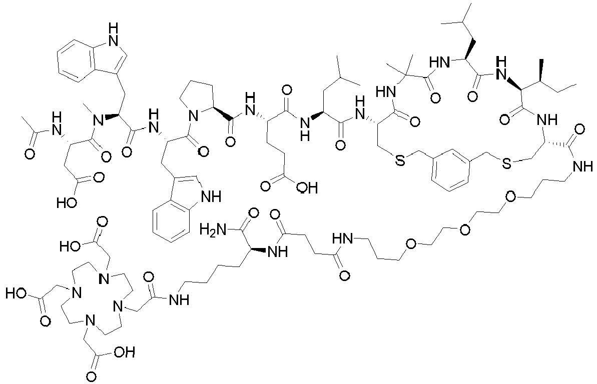
compound Ac-1Ni-Pro-Glu-Leu-[Cys(3MeBn)-Aib-Leu-Ile-Cys]-Ttds-Lys(DOTA)-NH₂ (GIP-298) of the following formula 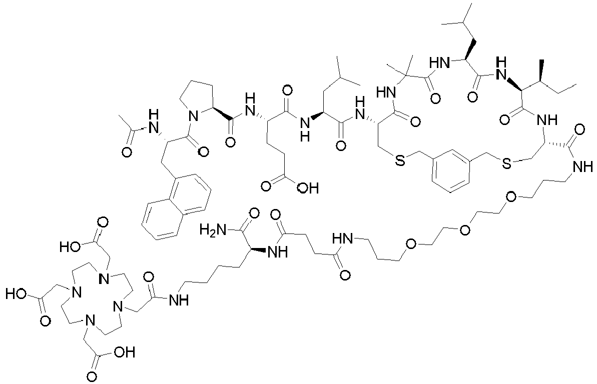
compound Nph1p-Pro-Glu-Leu-[Cys(3MeBn)-Aib-Leu-Ile-Cys]-Ttds-Lys(DOTA)-NH₂ (GIP-299) of the following formula 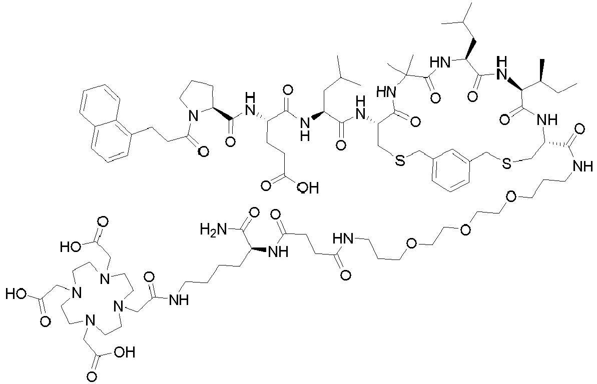
compound Ac-Asp-Trp-1Ni-Pro-Glu-leu-[Cys(3MeBn)-Ala-Leu-val-Cys]-Ttds-Lys(DOTA)-NH₂ (GIP-300) of the following formula 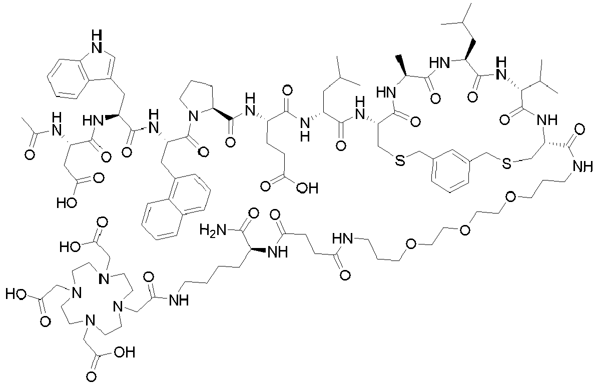
compound Ac-Trp-1Ni-Pro-Glu-Leu-[Cys(3MeBn)-Aib-Leu-Ile-Cys]-Ttds-Lys(DOTA)-NH₂ (GIP-301) of the following formula 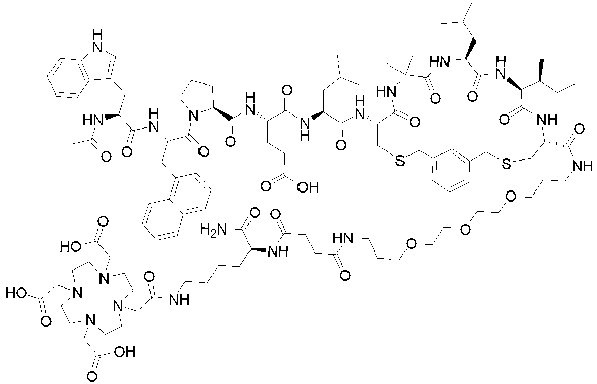
compound Ac-Asp-Trp-Hyw-Pro-Glu-leu-[Cys(3MeBn)-Aib-Leu-Ile-Cys]-Ttds-Lys(DOTA)-NH₂ (GIP-302) of the following formula 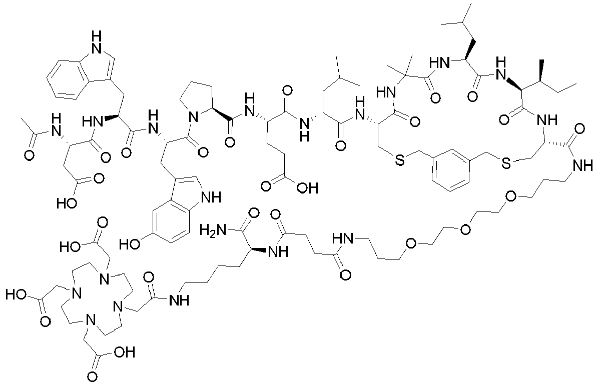
compound Ac-Asp-7Nw-1Ni-Pro-Glu-leu-[Cys(3MeBn)-Aib-Leu-Ile-Cys]-Ttds-Lys(DOTA)-NH₂ (GIP-303) of the following formula 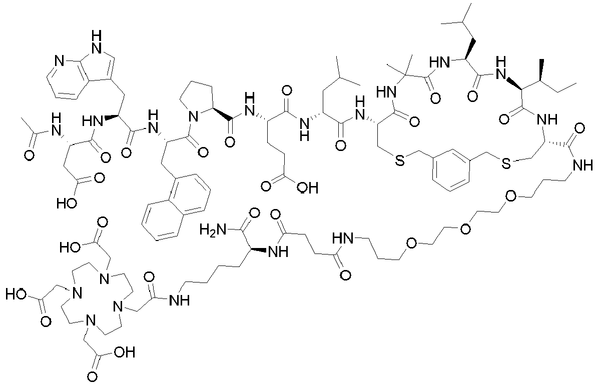
compound DOTA-Ttds-Trp-Asp-Pro-5Clw-leu-Glu-[Cys(3MeBn)-Leu-Ile-Aib-AET] (GIP-304) of the following formula 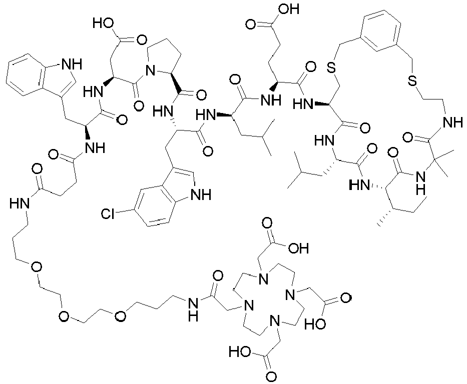
compound DOTA-Ttds-leu-Asp-Pro-5Clw-Trp-Leu-[Cys(3MeBn)-Glu-Aib-Ile-AET] (GIP-305) of the following formula 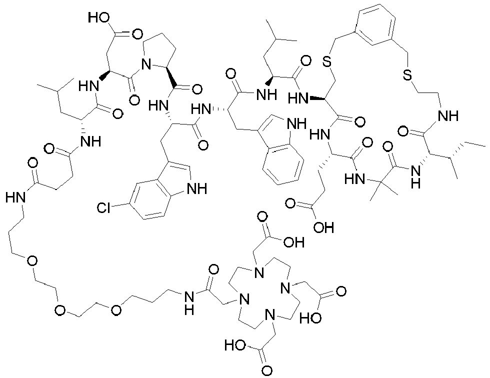
compound DOTA-Ttds-5Clw-Pro-Glu-leu-Asp-Trp-[Cys(3MeBn)-Ile-Leu-Aib-AET] (GIP-306) of the following formula 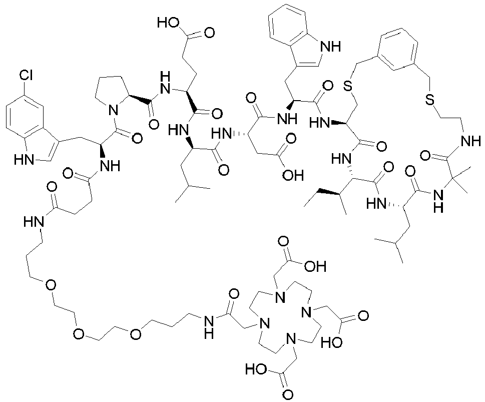
compound H-Met-Tyr-asp-Trp-Trp-Pro-Glu-Leu-[Cys(3MeBn)-Ala-Leu-Ile-Cys]-NH₂ (GIP-307) of the following formula 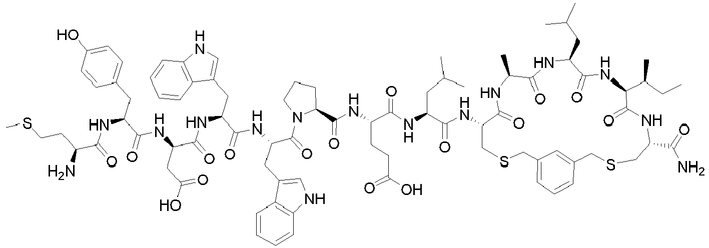
compound H-Met-Tyr-Asp-trp-Trp-Pro-Glu-Leu-[Cys(3MeBn)-Ala-Leu-Ile-Cys]-NH₂ (GIP-308) of the following formula 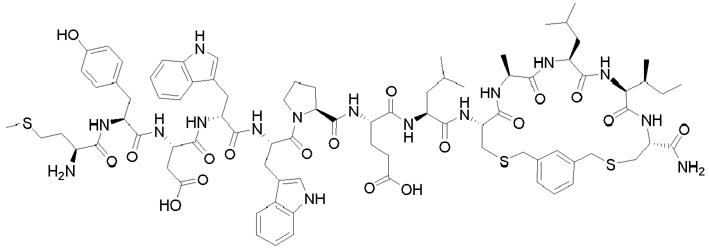
compound H-Met-Tyr-Asp-Trp-Trp-Pro-Glu-Leu-[cys(3MeBn)-Ala-Leu-Ile-Cys]-NH₂ (GIP-309) of the following formula 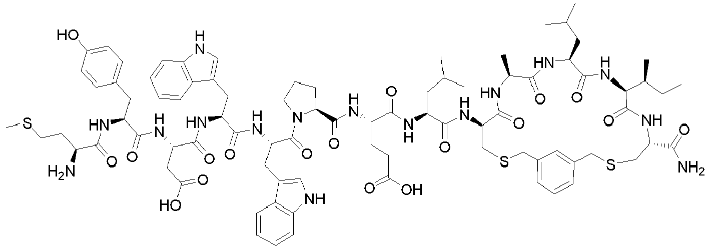
compound H-Met-Tyr-Asp-Trp-Trp-Pro-Glu-Leu-[Cys(3MeBn)-Ala-Leu-Ile-cys]-NH₂ (GIP-310) of the following formula 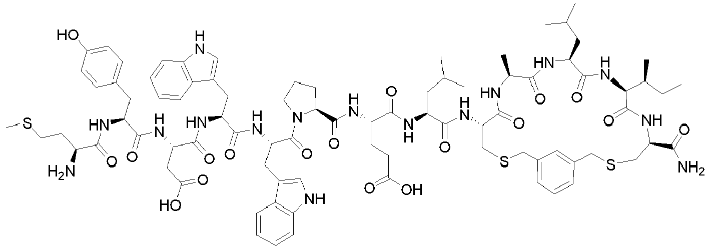
compound H-Met-Tyr-Asp-Trp-Trp-Ala-Glu-Leu-[Cys(3MeBn)-Ala-Leu-Ile-Cys]-NH₂ (GIP-311) of the following formula 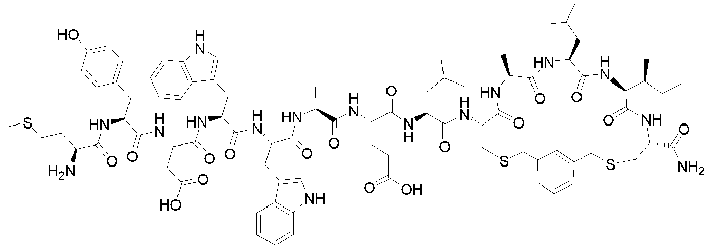
compound H-Met-Tyr-Asp-Trp-Trp-Pro-Glu-Leu-[Cys(3MeBn)-Ala-Ala-Ile-Cys]-NH₂ (GIP-312) of the following formula 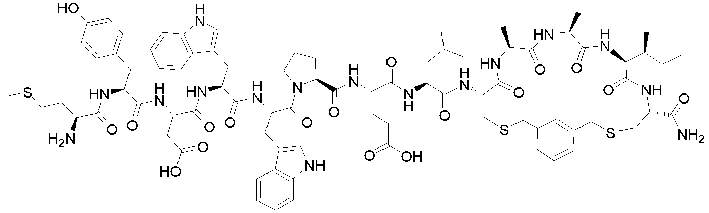
compound H-Met-Tyr-Asp-Trp-Trp-Pro-Glu-Leu-[Cys(2MeBn)-Ala-Leu-Ile-Cys]-NH₂ (GIP-313) of the following formula 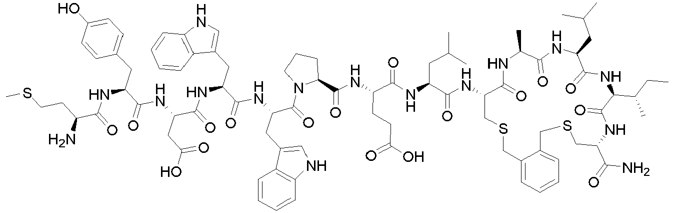
compound HO-Succinyl-Trp-Trp-Pro-Glu-Leu-[Cys(3MeBn)-ala-Leu-Ile-Cys]-NH₂ (GIP-314) of the following formula 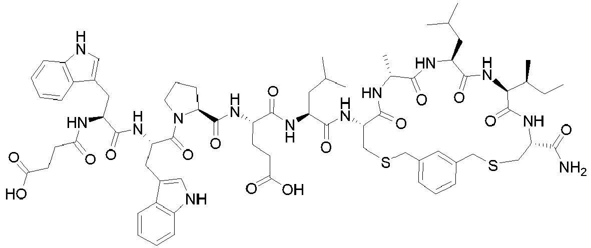
compound Ac-Asp-Trp-1Ni-Pro-Glu-Hle-[Cys(3MeBn)-Aib-Leu-Ile-Cys]-NH₂ (GIP-315) of the following formula 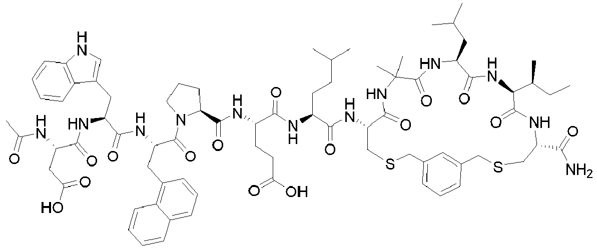
compound Ac-Cya-Gly-5Clw-Hyp-Glu-leu-[Cys(2Lut)-Aib-Leu-Ile-AET] (GIP-316) of the following formula 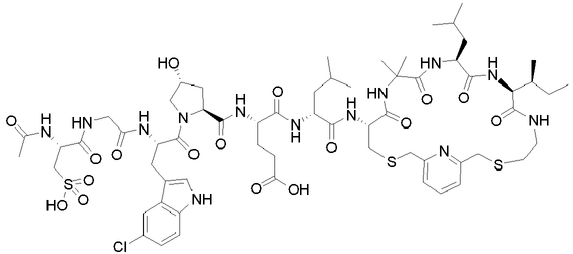
compound Ac-Cya-Trp-Trp-Pro-Glu-Leu-[Cys(3MeBn)-Ala-Leu-Ile-Cys]-NH₂ (GIP-317) of the following formula 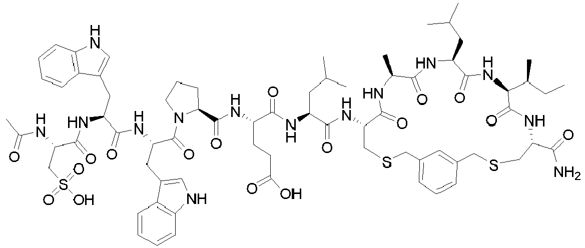
compound HO-Succinyl-Trp-Trp-Pro-Glu-leu-[Cys(3MeBn)-ala-Leu-Ile-Cys]-NH₂ (GIP-318) of the following formula 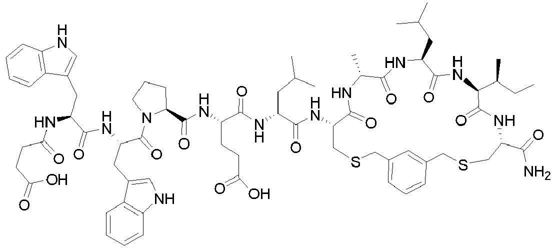
compound HO-Succinyl-Trp-Trp-Hyp-Glu-Leu-[Cys(3MeBn)-Aib-Leu-Ile-Cys]-NH₂ (GIP-319) of the following formula 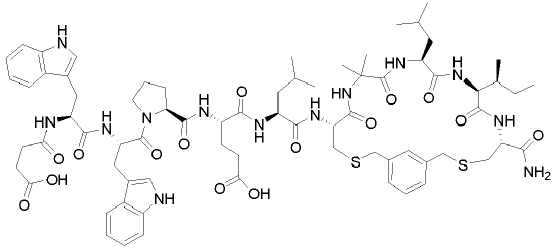
compound Ac-Asp-Trp-Mcf-Hyp-Glu-leu-[Cys(2Lut)-Thp-Leu-Ile-Cys]-NH₂ (GIP-320) of the following formula 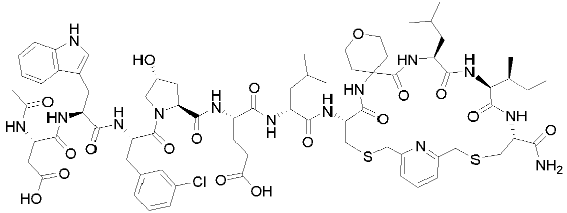
compound Ac-Asp-Trp-1Qi-Hyp-Glu-leu-[Cys(2Lut)-Thp-Leu-Ile-Cys]-NH₂ (GIP-321) of the following formula 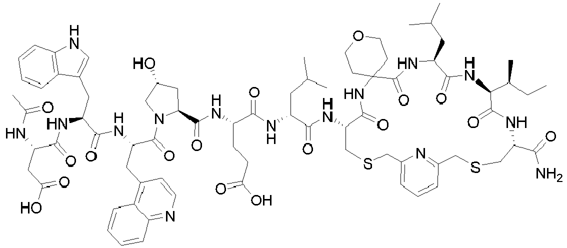
compound Ac-Asp-Trp-5Clw-Hyp-Glu-leu-[Cys(2Lut)-Thp-Leu-Ser-Cys]-NH₂ (GIP-322) of the following formula 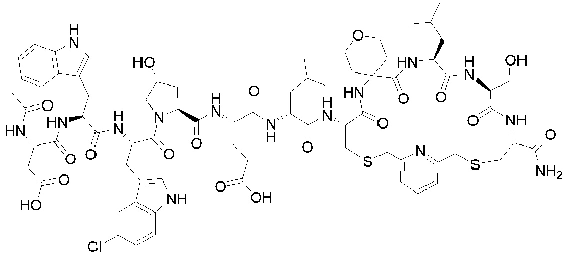
compound Ac-Asp-Eaa-Eaa-Hyp-Glu-leu-[Cys(2Lut)-Thp-Leu-Ile-Cys]-NH₂ (GIP-323) of the following formula 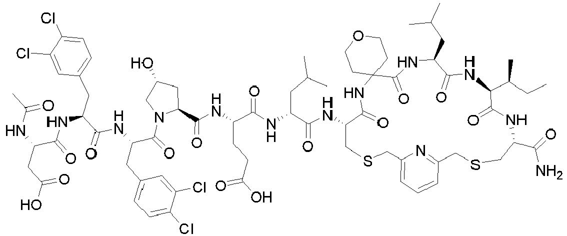
compound Ac-Trp-Trp-Pro-Glu-Leu-[Cys(3MeBn)-Ala-Leu-Ile-Cys]-NH₂ (GIP-324) of the following formula 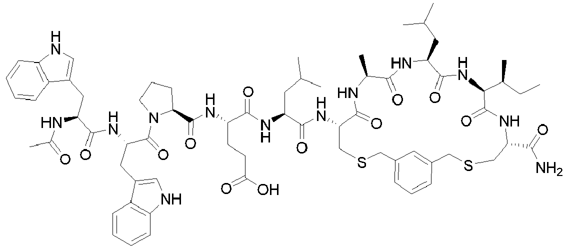
compound Ac-Trp-Pro-Glu-Leu-[Cys(3MeBn)-Ala-Leu-Ile-Cys]-NH₂ (GIP-325) of the following formula 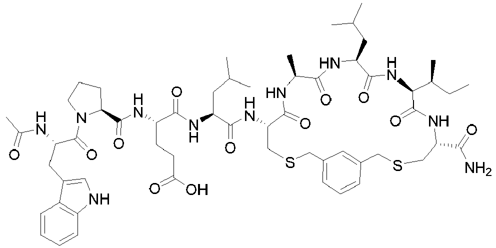
compound Ac-Pro-Glu-Leu-[Cys(3MeBn)-Ala-Leu-Ile-Cys]-NH₂ (GIP-326) of the following formula 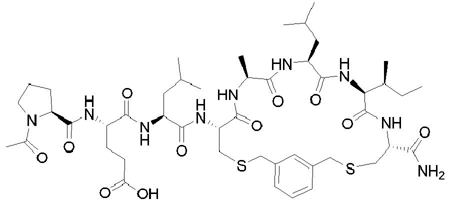
compound Ac-Glu-Leu-[Cys(3MeBn)-Ala-Leu-Ile-Cys]-NH₂ (GIP-327) of the following formula 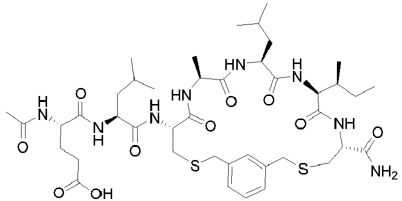
compound H-Met-Tyr-Asp-Trp-Trp-pro-Glu-Leu-[Cys(3MeBn)-Ala-Leu-Ile-Cys]-NH₂ (GIP-328) of the following formula 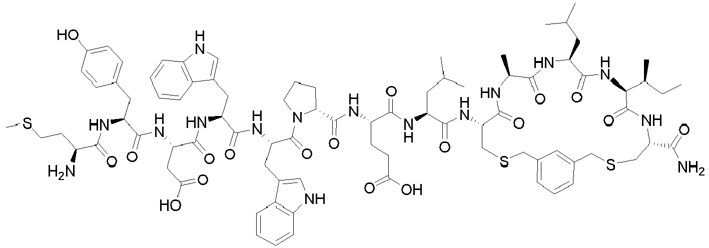
compound H-Met-Tyr-Asp-Trp-trp-Pro-Glu-Leu-[Cys(3MeBn)-Ala-Leu-Ile-Cys]-NH₂ (GIP-329) of the following formula 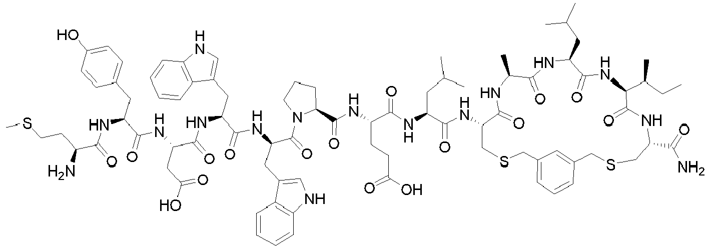
compound H-Met-Tyr-Asp-Trp-Trp-Pro-Glu-Leu-[Cys(3MeBn)-Ala-leu-Ile-Cys]-NH₂ (GIP-330) of the following formula 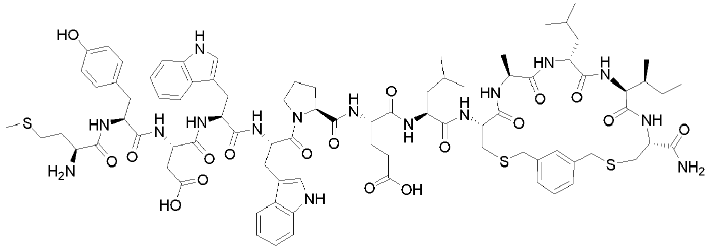
compound H-Met-Tyr-Asp-Trp-Trp-Pro-Glu-Leu-[Cys(4MeBn)-Ala-Leu-Ile-Cys]-NH₂ (GIP-331) of the following formula 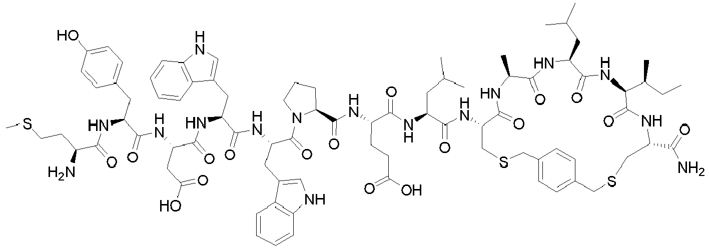
compound H-Met-Tyr-Ala-Trp-Trp-Pro-Glu-Leu-[Cys(3MeBn)-Ala-Leu-Ile-Cys]-NH₂ (GIP-332) of the following formula 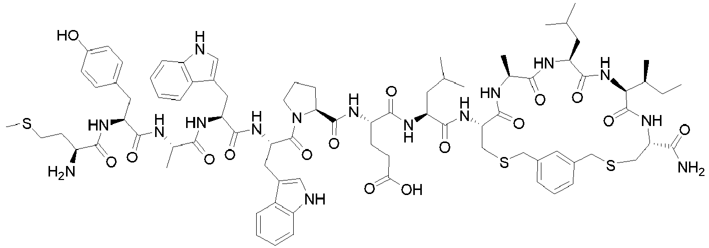
compound H-Met-Tyr-Asp-Ala-Trp-Pro-Glu-Leu-[Cys(3MeBn)-Ala-Leu-Ile-Cys]-NH₂ (GIP-333) of the following formula 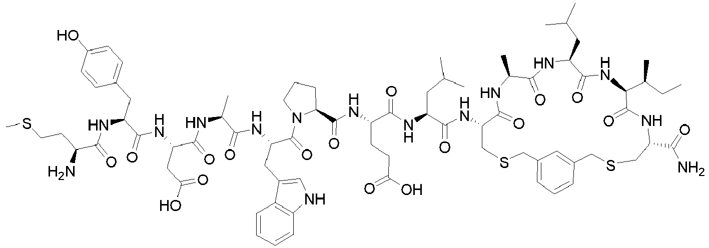
compound H-Met-Tyr-Asp-Trp-Ala-Pro-Glu-Leu-[Cys(3MeBn)-Ala-Leu-Ile-Cys]-NH₂ (GIP-334) of the following formula 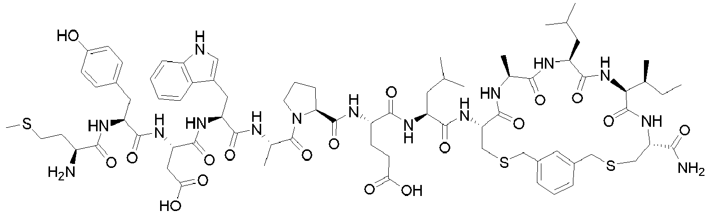
compound H-Met-Tyr-Asp-Trp-Trp-Pro-Ala-Leu-[Cys(3MeBn)-Ala-Leu-Ile-Cys]-NH₂ (GIP-335) of the following formula 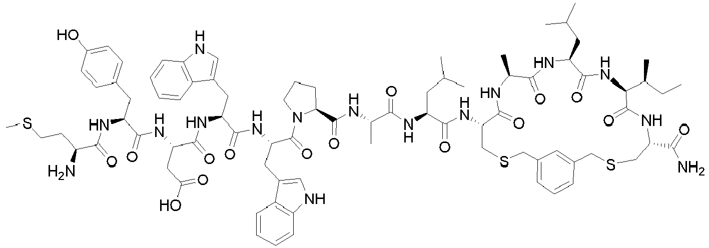
compound H-Met-Tyr-Asp-Trp-Trp-Pro-Glu-Ala-[Cys(3MeBn)-Ala-Leu-Ile-Cys]-NH₂ (GIP-336) of the following formula 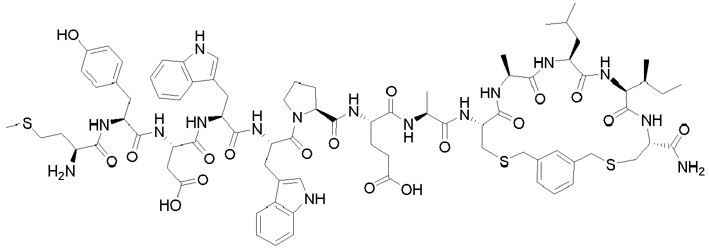
compound H-Met-Tyr-Asp-Trp-Trp-Pro-Glu-Leu-[Cys(3MeBn)-Ala-Leu-Ala-Cys]-NH₂ (GIP-337) of the following formula 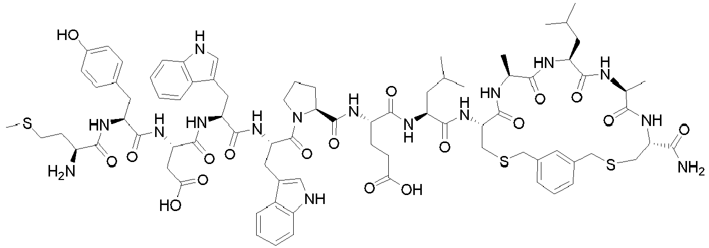
compound Ac-Asp-Trp-Trp-Pro-Glu-Leu-[Pen(3MeBn)-Aib-Leu-Ile-Cys]-NH₂ (GIP-338) of the following formula 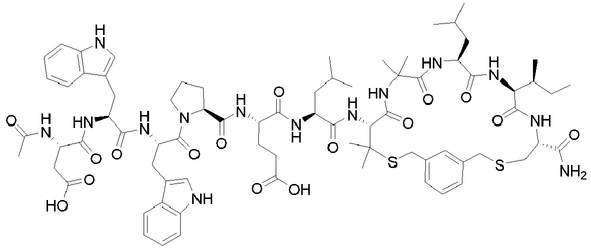
compound Ac-Asp-Trp-Trp-Pro-Glu-Leu-[Cys(3MeBn)-Aib-Leu-Ile-Pen]-NH₂ (GIP-339) of the following formula 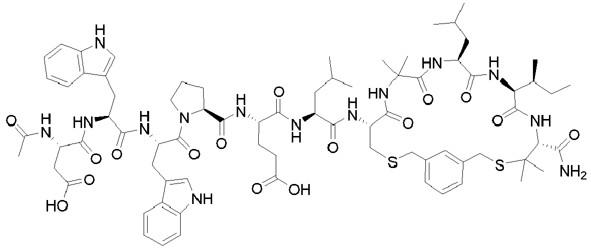
compound Ac-Asp-Trp-1Ni-Pro-Glu-Asn-[Cys(3MeBn)-Aib-Leu-Ile-Cys]-NH₂ (GIP-340) of the following formula 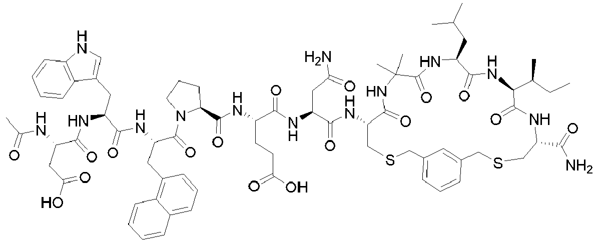
compound Ac-Asp-Trp-1Ni-4Dfp-Glu-Leu-[Cys(3MeBn)-Aib-Leu-Ile-Cys]-NH₂ (GIP-341) of the following formula 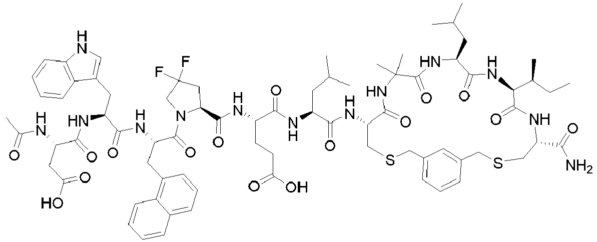
compound Ac-Asp-Trp-1Ni-Rni-Glu-Leu-[Cys(3MeBn)-Aib-Leu-Ile-Cys]-NH₂ (GIP-342) of the following formula 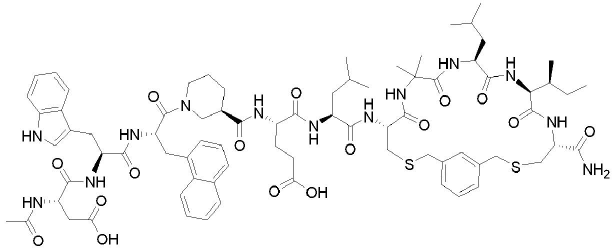
compound Ac-Asp-Trp-1Ni-Pro-Glu-Thi-[Cys(3MeBn)-Aib-Leu-Ile-Cys]-NH₂ (GIP-343) of the following formula 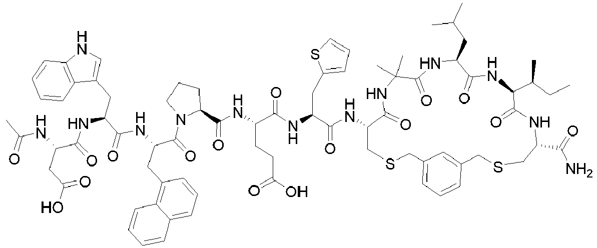
compound Ac-Asp-Trp-1Ni-Pro-Glu-Phe-[Cys(3MeBn)-Aib-Leu-Ile-Cys]-NH₂ (GIP-344) of the following formula 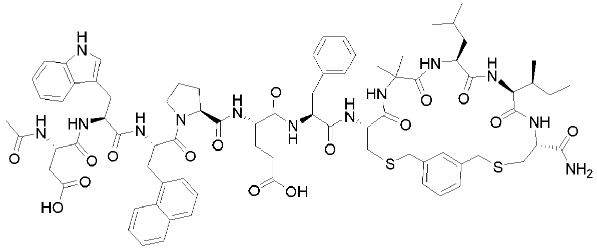
compound Ac-Asp-Trp-1Ni-Pro-Glu-Lys-[Cys(3MeBn)-Aib-Leu-Ile-Cys]-NH₂ (GIP-345) of the following formula 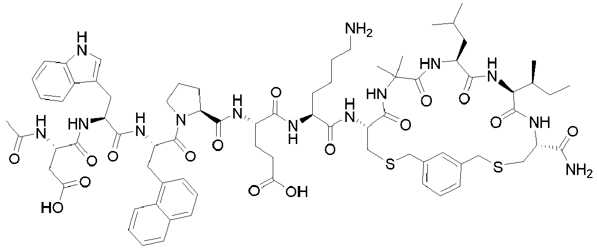
compound Ac-Asp-Trp-1Ni-Pro-Glu-Nle-[Cys(3MeBn)-Aib-Leu-Ile-Cys]-NH₂ (GIP-346) of the following formula 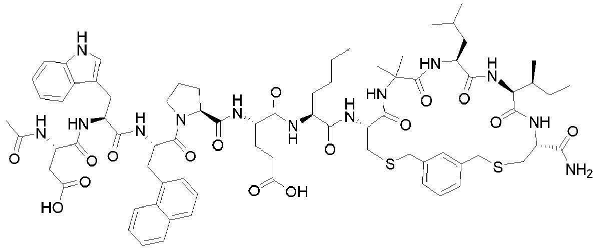
compound Ac-Asp-Trp-1Ni-Pro-Glu-Cit-[Cys(3MeBn)-Aib-Leu-Ile-Cys]-NH₂ (GIP-347) of the following formula 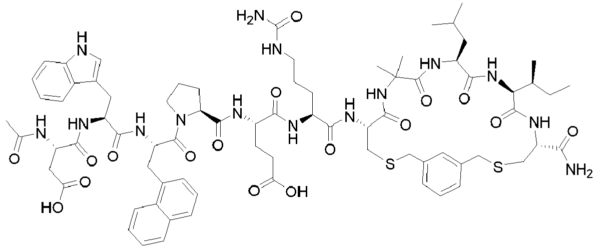
compound Ac-Asp-Trp-1Ni-Pro-Glu-Dap(Ac)-[Cys(3MeBn)-Aib-Leu-Ile-Cys]-NH₂ (GIP-348) of the following formula 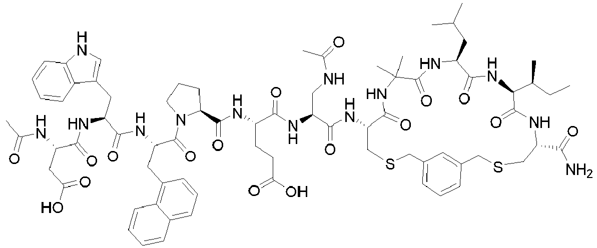
compound Ac-Asp-Trp-Ocf-Hyp-Glu-leu-[Cys(2Lut)-Thp-Leu-Ile-Cys]-NH₂ (GIP-349) of the following formula 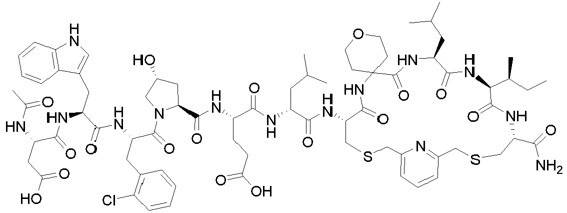
compound Ac-Asp-Trp-Pcf-Hyp-Glu-leu-[Cys(2Lut)-Thp-Leu-Ile-Cys]-NH₂ (GIP-350) of the following formula 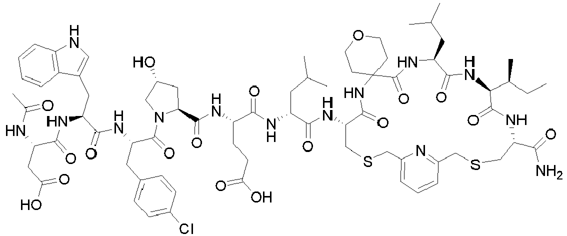
compound Ac-Asp-Trp-Egm-Hyp-Glu-leu-[Cys(2Lut)-Thp-Leu-Ile-Cys]-NH₂ (GIP-351) of the following formula 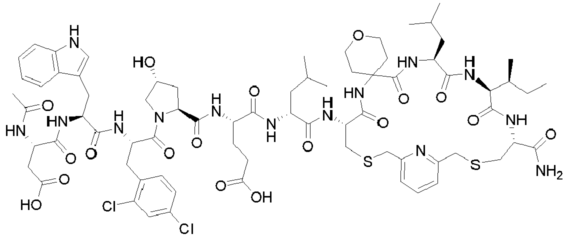
compound Ac-Asp-Trp-Phe-Hyp-Glu-leu-[Cys(2Lut)-Thp-Leu-Ile-Cys]-NH₂ (GIP-352) of the following formula 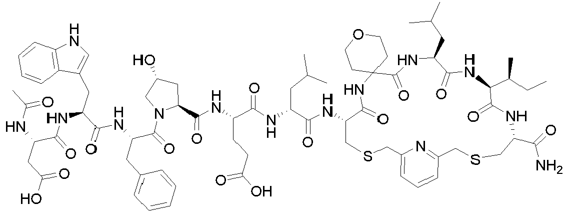
compound Ac-Asp-Trp-5Clw-Hyp-Glu-leu-[Cys(2Lut)-Thp-Leu-Arg-Cys]-NH₂ (GIP-353) of the following formula 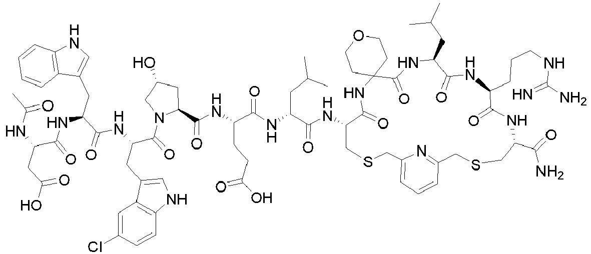
compound Ac-Asp-Trp-5Clw-Hyp-Glu-leu-[Cys(2Lut)-Thp-Leu-Glu-Cys]-NH₂ (GIP-354) of the following formula 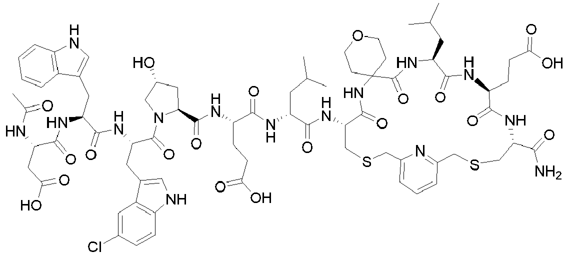
compound Ac-Asp-Trp-5Clw-Hyp-Glu-leu-[Cys(2Lut)-Thp-Leu-Trp-Cys]-NH₂ (GIP-355) of the following formula 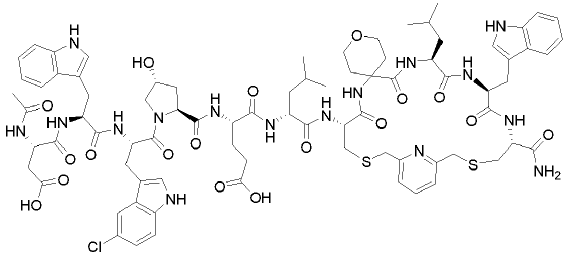
compound Ac-Asp-Eaa-Eaa-Oic-Glu-leu-[Cys(2Lut)-Thp-Leu-Ile-Cys]-NH₂ (GIP-356) of the following formula 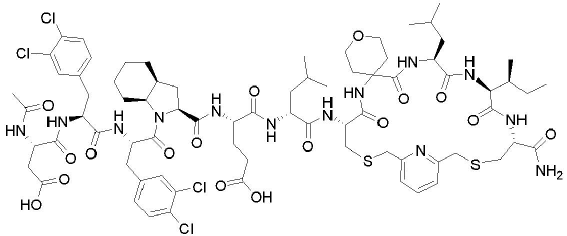
compound Ac-Asp-Eaa-Eaa-Nmg-Glu-leu-[Cys(2Lut)-Thp-Leu-Ile-Cys]-NH₂ (GIP-357) of the following formula 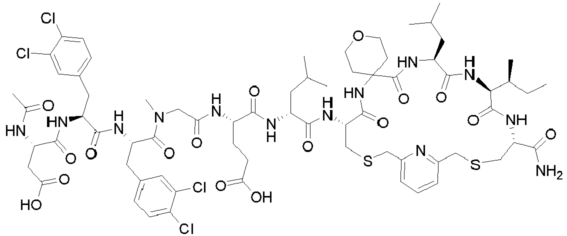
compound DOTA-Ttds-Asp-Trp-5Clw-Pro-Glu-leu-[Dap(3MeBz)-Aib-Leu-Ile-AET] (GIP-358) of the following formula 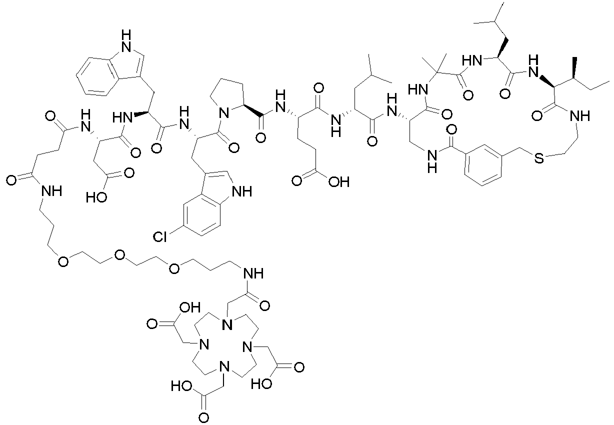
compound DOTA-Ttds-Asp-Trp-5Clw-Pro-Glu-leu-[Cys(3CbBn)-Aib-Leu-Ile-en] (GIP-359) of the following formula 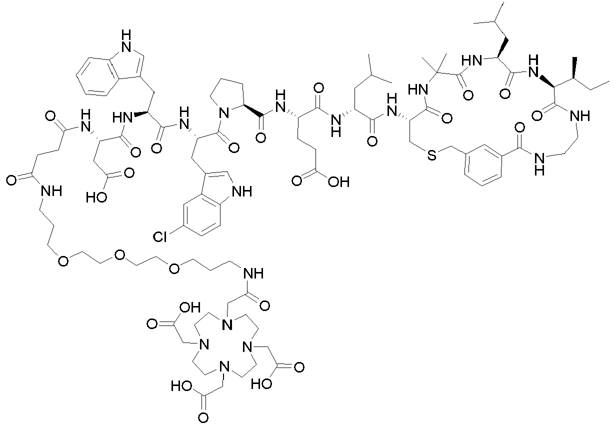
compound Ac-Asp-Trp-5Clw-Hyp-Glu-leu-[Cys(mli)-Thp-Leu-Ile-Cys]-NH₂ (GIP-360) of the following formula 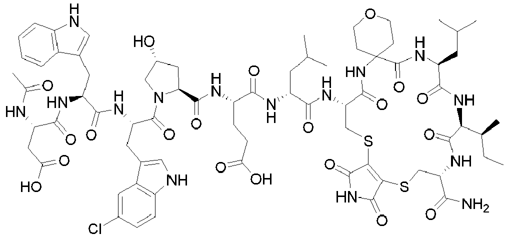
compound Ac-Asp-Trp-5Clw-Hyp-Glu-leu-[Cys(mli)-Thp-Leu-Ile-Hcy]-NH₂ (GIP-361) of the following formula 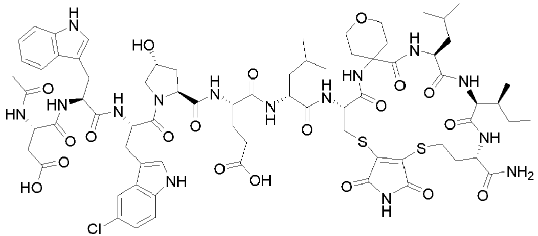
compound Ac-Asp-Trp-5Clw-Hyp-Glu-leu-[Hcy(mli)-Thp-Leu-Ile-Cys]-NH₂ (GIP-362) of the following formula 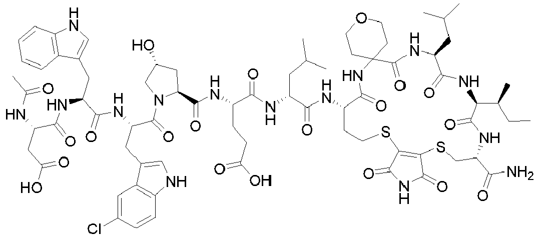
compound Ac-Asp-Trp-5Clw-Hyp-Glu-leu-[Hcy-Thp-Leu-Ile-Hcy]-NH₂ (GIP-363) of the following formula 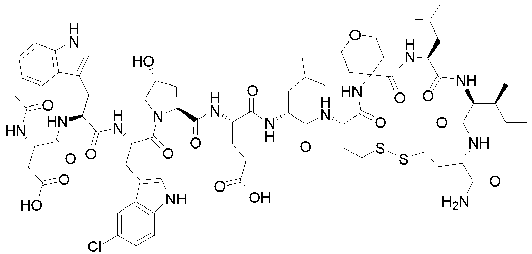
compound Ac-Asp-Trp-5Clw-Hyp-Glu-leu-[Cys-Thp-Leu-Ile-Cys]-NH₂ (GIP-364) of the following formula 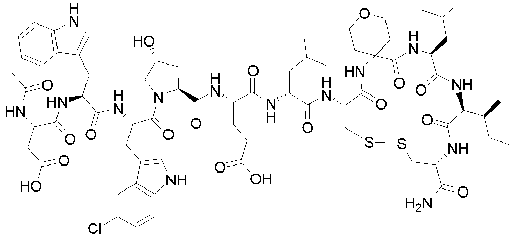
compound DOTA-Ttds-Asp-Trp-5Clw-Pro-Glu-leu-[Dap(Mamb)-Aib-Leu-Ile-Gly] (GIP-365) of the following formula 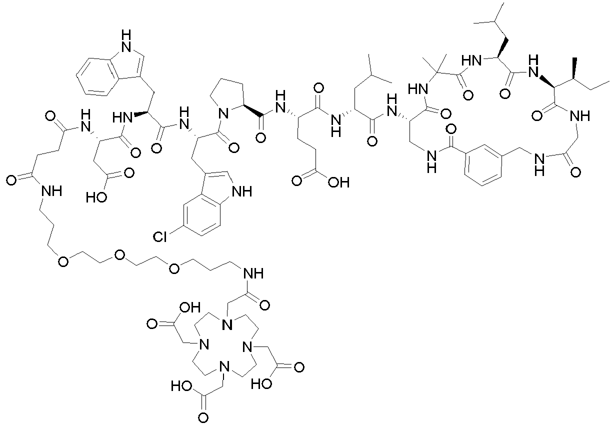
compound DOTA-Ttds-Asp-Trp-5Clw-Pro-Glu-leu-[Dap(Mamb)-Aib-Leu-Ile-gln] (GIP-366) of the following formula 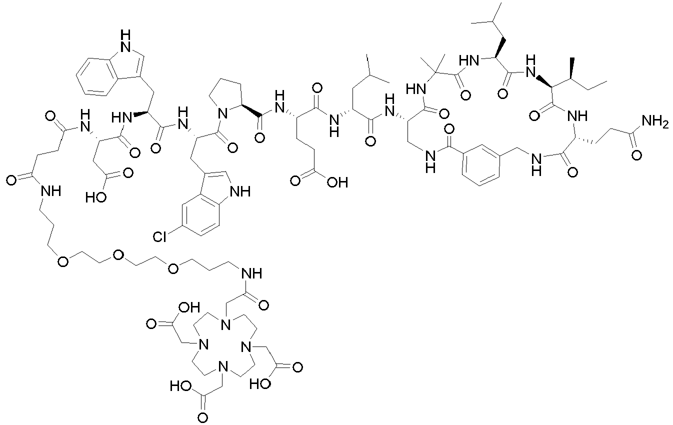
compound DOTA-Ttds-Asp-Trp-5Clw-Pro-Glu-leu-[Dap(Mamb)-Aib-Leu-Ile-Gln] (GIP-367) of the following formula 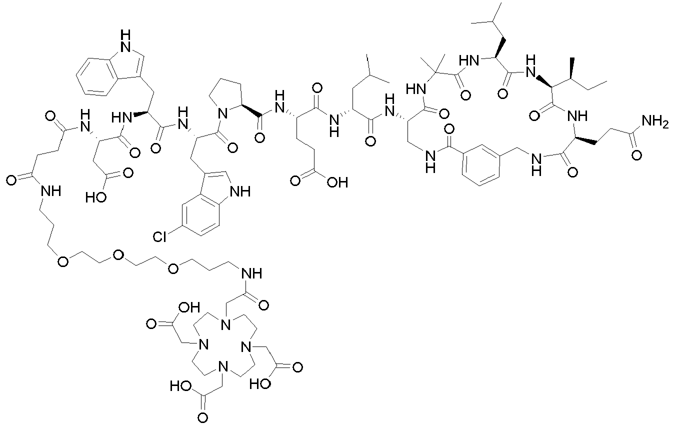
compound H-Met-Tyr-Asp-Trp-Trp-Pro-Glu-Leu-Cys(Bzl)-Ala-Leu-Ile-Smc-NH₂ (GIP-368) of the following formula 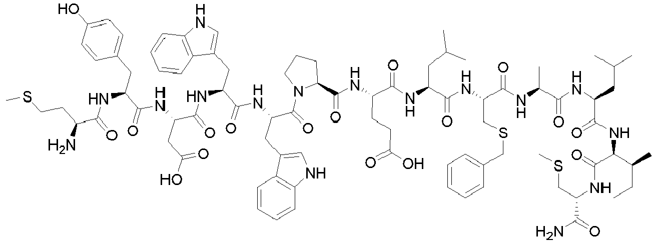
compound H-Met-Tyr-Asp-Trp-Trp-Pro-Glu-Leu-Smc-Ala-Leu-Ile-Cys(Bzl)-NH₂ (GIP-369) of the following formula

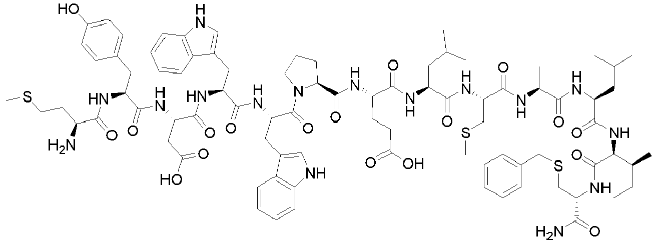

Embodiment 143. The compound of any one of Embodiments 1 to 142, preferably Embodiment 142, wherein the compound
(a) is selected from the group consisting of GIP-001 to GIP-369,
(b) preferably is selected from the group consisting of GIP-001 to GIP-267,
(c) more preferably is selected from the group consisting of GIP-001 to GIP-071, and
(d) most preferably is selected from the group consisting of GIP-001 to GIP-023.

Embodiment 144. The compound of any one Embodiments 1 to 143, preferably any one of Embodiments 142 and 143, wherein the compound
(a) preferably is selected from the group consisting of GIP-001 to GIP-267,
(b) more preferably is selected from the group consisting of GIP-001 to GIP-071, and
(c) most preferably is selected from the group consisting of GIP-001 to GIP-023.

Embodiment 145. The compound of any one Embodiments 1 to 144, preferably any one of Embodiments 142 to 144, wherein the compound
(a) preferably is selected from the group consisting of GIP-001 to GIP-125,
(b) more preferably is selected from the group consisting of GIP-001 to GIP-058, and
(c) most preferably is selected from the group consisting of GIP-001 to GIP-007.

Embodiment 146. The compound of any one Embodiments 1 to 145, wherein any S-atom, which can be oxidized, preferably S atoms of thioether groups, is present as -S-, -S(O)- or -S(O₂)- or mixture thereof.

Embodiment 147. The compound of any one of Embodiments 1 to 146, wherein the compound comprises a diagnostically active radio nuclide, wherein the radio nuclide
(a) is a radio nuclide used for diagnosis,
(b) preferably is selected from the group, but not limited to, comprising ⁴³Sc, ⁴⁴Sc, ⁵¹Mn, ⁵²Mn, ⁶⁴Cu, ⁶⁷Ga, ⁶⁸Ga, ⁸⁶Y, ⁸⁹Zr, ^{94m}Tc, ^{99m}Tc, ¹¹¹In, ¹⁵²Tb, ¹⁵⁵Tb, ¹⁷⁷Lu, ²⁰¹T1, ²⁰³Pb, ¹⁸F ⁷⁶Br, ⁷⁷Br, ¹²³I, ¹²⁴I, and ¹²⁵I,
(c) more preferably is selected from the group comprising ⁴³Sc, ⁴⁴Sc, ⁶⁴Cu, ⁶⁷Ga, ⁶⁸Ga, ⁸⁶Y, ⁸⁹Zr, ¹¹¹In, ¹⁵² Tb, ¹⁵⁵Tb, and ²⁰³Pb,
   and
(d) most preferably is selected from the group comprising ⁶⁴Cu, ⁶⁸Ga, and ¹¹¹In.

Embodiment 148. The compound of any one of Embodiments 1 to 146, wherein the compound comprises a therapeutically active radio nuclide, wherein the radio nuclide
(a) is a radio nuclide used for therapy,
(b) preferably is selected from the group comprising ⁴⁷Sc, ⁶⁷Cu, ⁸⁹Sr, ⁹⁰Y, ¹¹¹In, ¹⁵³Sm, ¹⁴⁹Tb, ¹⁶¹Tb, ¹⁷⁷Lu, ¹⁸⁶Re, ¹⁸⁸Re, ²¹²Pb, ²¹³Bi, ²²³Ra, ²²⁵Ac, ²²⁶Th, ²²⁷Th, ¹³¹I, and ²¹¹At,
(c) more preferably is selected from the group comprising ⁴⁷Sc, ⁶⁷Cu, ⁹⁰Y, ¹⁷⁷Lu, ²¹²Pb, ²¹³Bi, ²²⁵Ac, and ²²⁷Th,
   and
(d) most preferably is selected from the group comprising ⁹⁰Y, ¹⁷⁷Lu, ²²⁵Ac, and ²²⁷Th.

Embodiment 149. The compound of any one of Embodiments 1 to 148 for use in a method of diagnosing a disease.

Embodiment 150. The compound of any one of Embodiments 1 to 148 for use in a method for the treatment of a disease.

Embodiment 151. The compound of any one of Embodiments 1 to 148 for use in a method for the identification of a subject, wherein the subject is likely to respond or likely not to respond to a treatment of a disease, wherein the method for the identification of a subject comprises carrying out a method of diagnosing a disease using a compound of any one of Embodiments 1 to 148.

Embodiment 152. The compound of any one of Embodiments 1 to 148 for use in a method for the selection of a subject from a group of subjects, wherein the subject is likely to respond or likely not to respond to a treatment of a disease, wherein the method for the selection of a subject from a group of subjects comprises carrying out a method of diagnosing a disease using a compound of any one of Embodiments 1 to 148.

Embodiment 153. The compound of any one of Embodiments 1 to 148 for use in a method for the stratification of a group of subjects into subjects which are likely to respond to a treatment of a disease, and into subjects which are not likely to respond to a treatment of a disease, wherein the method for the stratification of a group of subjects comprises carrying out a method of diagnosing a disease using a compound of any one of Embodiments 1 to 148.

Embodiment 154. The compound for use of any one of Embodiments 149 to 153, wherein the disease is cancer.

Embodiment 155. The compound for use of Embodiment 154, wherein the cancer is expressing Gastric Inhibitory Peptide Receptor (GIPR), preferably, the cancer is overexpressing GIPR.

Embodiment 156. The compound for use of any one of Embodiments 154 to 155, wherein the cancer is a neuroendocrine cancer or a neuroendocrine neoplasm.

Embodiment 157. The compound for use of any one of Embodiments 154 to 156, wherein the cancer is selected from the group consisting of medullary thyroid cancer, gastrointestinal neuroendocrine neoplasms, ileal neuroendocrine neoplasms, pancreatic neuroendocrine neoplasms, nonfunctioning neuroendocrine neoplasms, Insulinomas, Gastrinomas, Glucagonomas, VIPomas, Somatostatinoma, ACTHoma, lung neuroendocrine neoplasms, typical carcinoid tumors, atypical carcinoid tumors, small cell carcinoma of the lung, large cell carcinoma of the lung, thymic neuroendocrine tumors, Merkel cell carcinoma, Pheochromocytoma of the adrenal gland, adrenal cancer, parathyroid cancer, paraganglioma, pituitary gland tumors, neuroendocrine tumors of the ovaries and neuroendocrine tumors of the testicles.

Embodiment 158. The compound for use of any one of Embodiments 149 to 153, wherein the disease is a metabolic disease.

Embodiment 159. The compound for use of Embodiment 158, wherein a diseased cell and/or a diseased tissue, preferably a diseased cell and/or a diseased tissue of the metabolic disease expresses GIPR.

Embodiment 160. The compound for use of Embodiment 159, wherein the diseased cell and/or the diseased tissue overexpresses GIPR.

Embodiment 161. The compound for use of any one of Embodiments 159 to 160, wherein the diseased cell is a pancreatic cell.

Embodiment 162. The compound for use of any one of Embodiments 158 to 161, wherein the metabolic disease is selected from the group consisting of type 2 diabetes, type 1 diabetes, metabolic syndrome, insulin resistance, dyslipidemia, impaired fasting glucose, and impaired glucose tolerance.

Embodiment 163. A composition comprising a compound of any one of Embodiments 1 to 148 and a pharmaceutically acceptable excipient.

Embodiment 164. The composition of Embodiment 163, wherein the composition is a pharmaceutical composition.

Embodiment 165. A kit comprising a compound of any one of Embodiments 1 to 148 and one or more optional excipient(s) and optionally one or more device(s).

Embodiment 166. The kit of Embodiment 165, wherein the device(s) is/are selected from the group comprising a labeling device, a purification device, a handling device, a radioprotection device, an analytical device or an administration device.

The problem underlying the present invention is also solved in a first aspect, which is also a first embodiment of the first aspect, by
a compound comprising
a cyclic peptide of formula (Ia) 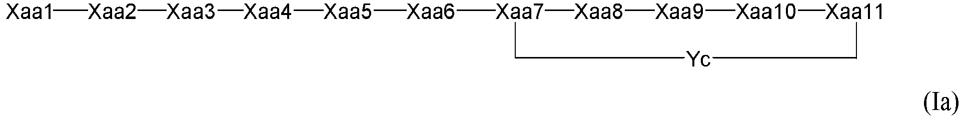
or a cyclic peptide of formula (Ib) 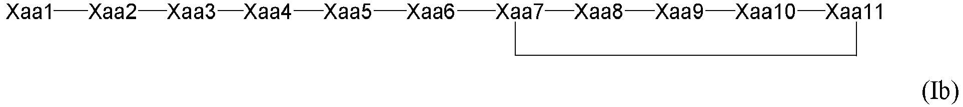
optionally an N-terminal modification group A attached to Xaa1, and
optionally a C-terminal group C-term attached to Xaa11,
wherein
   the peptide sequence is drawn from left to right in N- to C-terminal direction,
   Xaa1 is a residue of an α-amino acid with an acidic or polar group within the side chain or an aliphatic carboxylic acid with an additional acidic or polar group at the ω-position, preferably Xaa1 is a residue of an amino acid of formula (IIa), (IIb) or (IIc), more preferably Xaa1 is a residue of an amino acid formula (IIa), 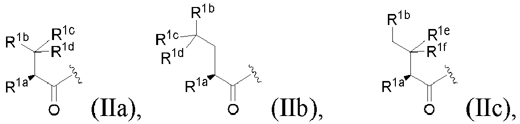 wherein
      - R^{1a}: is selected from the group consisting of NH, H, NH(C1-C4)alkyl, OH, and (C1-C4)alkyl, wherein if R^{1a} is selected from the group consisting of NH and NH(C1-C4)alkyl, the N-terminal modification group is covalently attached to the nitrogen atom of R^{1a}, and if R^{1a} is selected from the group consisting of H, OH or (C1-C4)alkyl the N-terminal modification group is absent,
      - R^{1b}: is selected from the group consisting of CO₂H, SO₃H, OPO₃H₂, CONH₂ and OH,
      - R^{1c}: is selected from the group consisting of H, OH and (C1-C2)alkyl under the proviso that R^{1b} is selected from the group consisting of CO₂H, SO₃H and CONH₂, or is selected from the group consisting of H and (C1-C2)alkyl under the proviso that R^{1b} is selected from the group consisting of OH and OPO₃H₂,
      - R^{1d}: is selected from the group consisting of H and (C1-C2)alkyl; preferably if R^{1c} is (C1-C2)alkyl, R^{1d} is the same (C1-C2)alkyl,
      - R^{1e}: is selected from the group consisting of H, OH and (C1-C2)alkyl,
      - R^{1f}: is selected from the group consisting of H and (C1-C2)alkyl; preferably if R^{1e} is (C1-C2)alkyl, R^{1f} is the same (C1-C2)alkyl,
   the carbonyl group of Xaa1 is covalently attached to the α-nitrogen atom of Xaa2,
   Xaa2 is a residue of an α-amino acid with a substituted or unsubstituted bicyclic aromatic or heteroaromatic side chain, preferably Xaa2 is a residue of an amino acid of formula (IIIa) or (IIIb), more preferably a residue of an amino acid of formula (IIIa), 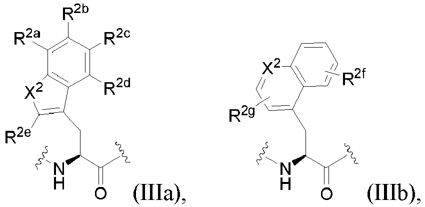 wherein
      - X²: is selected from the group consisting of NH, NCH₃ and S; preferably selected from the group consisting of NH and S, if Xaa2 is an amino acid of formula (IIIa), or is selected from the group consisting of N, CH, CF and C-CH₃; preferably selected from the group consisting of N and CH, if Xaa2 is an amino acid of formula (IIIb),
      - R^{2a}: is selected from the group consisting of H, F, Cl, Br, CH₃ and OCH₃, preferably R^{2a} is selected from the group consisting of H and F,
      - R^{2b}: is selected from the group consisting of H, a halogen, CH₃ and OCH₃, preferably the halogen is selected from the group consisting of Cl and Br, more preferably R^{2b} is selected from the group consisting of H, Cl and Br,
      - R^{2c}: is selected from the group consisting of H, OH, a halogen, CH₃ and OCH₃, preferably the halogen is selected from the group consisting of F, Cl and Br, more preferably R^{2c} is selected from the group consisting of H, F and OH,
      - R^{2d}: is selected from the group consisting of H, a halogen, CH₃ and OCH₃, wherein the halogen is selected from the group consisting of F, Cl and Br, preferably R^{2d} is H,
      - R^{2e}: is selected from the group consisting of H, a halogen and CH₃, preferably the halogen is Cl, more preferably R^{2e} is H,
      - R^{2f}: is selected from the group consisting of H and OCH₃, preferably R^{2f} is H,
      - R^{2g}: is selected from the group consisting of H, OH, CH₃ and OCH₃, preferably R^{2g} is H,
      or
   Xaa2 is a residue of an α-amino acid with a substituted or unsubstituted aromatic or heteroaromatic side chain, wherein Xaa2 is preferably a residue of an amino acid of formula (IIIc) 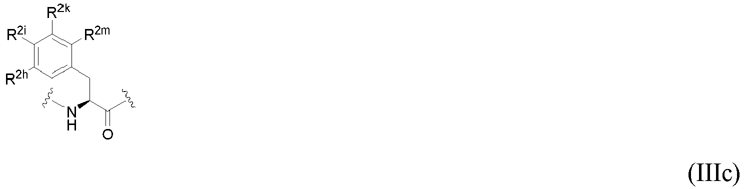 wherein
      - R^{2h}: is selected from the group consisting of H, CH₃, CF₃, OH, OCH₃, OCF₃, CONH₂ and a halogen, preferably the halogen is selected from the group consisting of Cl and Br, more preferably R^{2h} is H, Cl or Br,
      - R²¹: is selected from the group consisting of H, CH₃, C(CH₃)₃, CF₃, OH, OCH₃, OCH₂CH₃, OCF₃, CONH₂, CO₂H and a halogen, preferably the halogen is selected from the group consisting of Cl and Br, more preferably R²¹ is selected from the group consisting of H, Cl and Br,
      - R^{2k}: is selected from the group consisting of H, CH₃, CF₃, OH, OCH₃, OCF₃, CONH₂ and a halogen, preferably the halogen is selected from the group consisting of Cl and Br, more preferably R^{2k} is selected from the group consisting of H, Cl and Br,
      - R^{2m}: is selected from the group consisting of H, CH₃, CF₃, OH, OCH₃, OCF₃, CONH₂ and a halogen, preferably the halogen is selected from the group consisting of Cl and Br, more preferably R^{2m} is selected from the group consisting of H, Cl and Br,
   the carbonyl group of Xaa2 is covalently attached to the α-nitrogen atom of Xaa3,
   Xaa3 is an α-amino acid with a substituted or unsubstituted bicyclic aromatic or heteroaromatic side chain, preferably Xaa3 is a residue of an amino acid of formula (IVa) or (IVb), more preferably Xaa3 is a residue of an amino acid of formula (IVa), 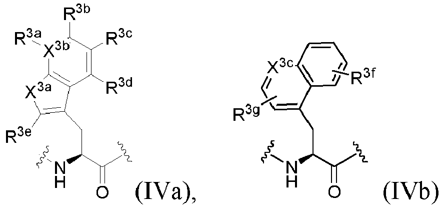 wherein
      - X^{3a}: is selected from the group consisting of NH, NCH₃ and S, preferably selected from the group consisting of NH and S,
      - X^{3b}: is selected from the group consisting of C and N, preferably X^{3b} is C,
      - X^{3c}: is selected from the group consisting of C and N, preferably X^{3c} is C,
      - R^{3a}: is, under the proviso that X^{3b} is C, selected from the group consisting of H, a halogen, CH₃ and OCH₃, preferably the halogen is F, or is absent if X^{3b} is N,
      - R^{3b}: is selected from the group consisting of H, a halogen, CH₃ and OCH₃, preferably the halogen is selected from the group consisting of F and Cl, more preferably R^{3b} is selected from the group consisting of H and F,
      - R^{3c}: is selected from the group consisting of H, a halogen, CH₃ and OCH₃, preferably the halogen is selected from the group consisting of Cl, Br and F, more preferably R^{3c} is selected from the group consisting of Cl and Br,
      - R^{3d}: is selected from the group consisting of H, a halogen, CH₃ and OCH₃, preferably the halogen is F,
      - R^{3e}: is selected from the group consisting of H, a halogen and CH₃, preferably the halogen is Cl,
      - R^{3f}: is selected from the group consisting of H and OCH₃,
      - R^{3g}: is selected from the group consisting of H, F, OH, CH₃ and OCH₃,
   or
   Xaa3 is a residue of an α-amino acid with a substituted or unsubstituted aromatic or heteroaromatic side chain, preferably Xaa3 is a residue of an amino acid of formula (IVc) 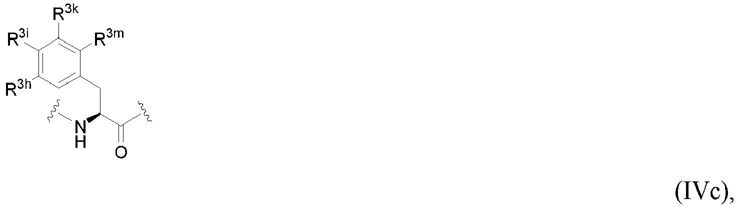 wherein
      - R^{3h}: is selected from the group consisting of H, a halogen, OH, CH₃, CF₃, OCH₃, OCF₃ and CONH₂, preferably the halogen is selected from the group consisting of Cl and Br,
      - R³¹: is selected from the group consisting of H, a halogen, OH, CH₃, CF₃, OCH₃, OCF₃ and CONH₂, preferably the halogen is selected from the group consisting of Cl and Br,
      - R^{3k}: is selected from the group consisting of H, a halogen, OH, CH₃, CF₃, OCH₃, OCF₃ and CONH₂, preferably the halogen is selected from the group consisting of Cl and Br, and
      - R^{3m}: is selected from the group consisting of H, a halogen, OH, CH₃, CF₃, OCH₃, OCF₃ and CONH₂, preferably the halogen is selected from the group consisting of Cl and Br, preferably R^{3m} is selected from the group consisting of H and Cl,
   the carbonyl group of Xaa3 is covalently attached to the α-nitrogen atom of Xaa4,
   Xaa4 is a residue of a cyclic non-aromatic amino acid, preferably Xaa4 is a residue of an amino acid of formula (Va) 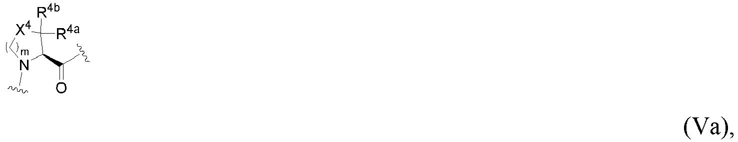 wherein
      - R^{4a}: is selected from the group consisting of H, OH, CH₃ and CF₃
      - R^{4b}: is selected from the group consisting of H and CH₃, preferably R^{4b} is CH₃ if R^{4a} is CH₃,
      - X⁴: is selected from the group consisting of CHR^{4c}, CH₂, S and O, wherein R^{4c} is absent or is selected from the group consisting of NH₂, OH, H, NHR^{4d}, CH₃ and F, wherein R^{4d} is absent or is selected from the group consisting of H, Ac, a Z group, wherein the Z group comprises a bio-distribution modifier with an optional linker, a chelator with an optional linker or a combination thereof, preferably the chelator is DOTA, the bio-distribution modifier is AGLU comprising the linker Glutar
      - m: is 1 or 2, preferably m is 1
   or
   Xaa4 is a residue of an N-alkylated amino acid, preferably Xaa4 is a residue of an amino acid of formula (Vb) 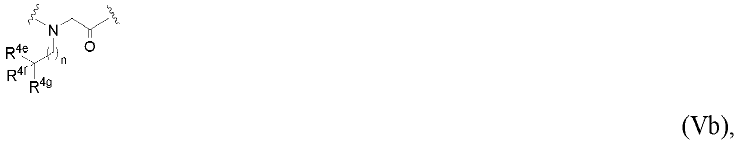 wherein
      - n: is 0, 1, 2, 3, 4, or 5, preferably n is 0, 1 or 3,
      - R^{4e}: is, if n is 0, selected from the group consisting of H, CH₃, a substituted or unsubstituted aromatic residue, COOH, CONH₂ and C(=O)R^{4h}, or is, if n is 1, 2, 3, 4 or 5, selected from the group consisting of H, CH₃, a substituted or unsubstituted aromatic residue, OH, NH₂, COOH, CONH₂ and C(=O)R^{4h},
      - R^{4f}: is selected from the group consisting of H, CH₃ and CH₂CH₃,
      - R^{4g}: is selected from the group consisting of H and CH₃,
      - R^{4h}: is AGLU,
   or
   Xaa4 is a residue of a bicyclic non-aromatic amino acid, preferably Xaa4 is a residue of an amino acid of formula (Vc) 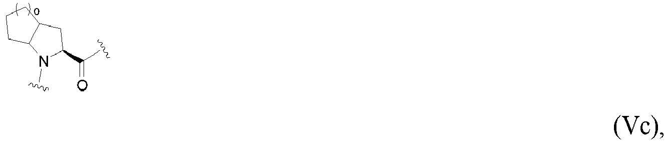 wherein
      - o: is 1 or 2,
   the carbonyl group of Xaa4 is covalently attached to the α-nitrogen atom of Xaa5,
   Xaa5 a residue of an α-amino acid with a polar, preferably an acidic group within the side chain, preferably Xaa5 is a residue of an amino acid of formula (VIa), (VIb) or (VIc), more preferably Xaa5 is a residue of an amino acid formula (VIa) or (VIb), 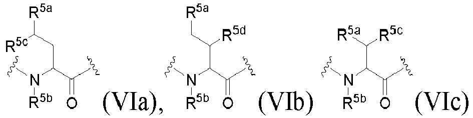 wherein
      - R^{5a}: is selected from the group consisting of COOH, CONH₂, OPO₃H₂, OH and SO₃H,
      - R^{5b}: is selected from the group consisting of H and (C1-C4)alkyl,
      - R^{5c}: is selected from the group consisting of H, OH and CH₃ under the proviso that R^{5a} is selected from the group consisting of COOH, CONH₂, OPO₃H₂ and SO₃H, or is selected from the group consisting of H and CH₃ under the proviso that R^{5a} is selected from the group consisting of COOH, CONH₂, OPO₃H₂, OH and SO₃H,
      - R^{5d}: is selected from the group consisting of H, OH and CH₃
   the carbonyl group of Xaa5 is covalently attached to the α-nitrogen atom of Xaa6,
   Xaa6 is a residue of an α-amino acid with a branched aliphatic side chain, preferably
   Xaa6 is a residue of an amino acid of formula (VIIa) 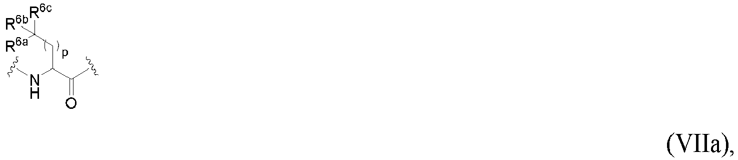 wherein
      - p: is 1, 0 or 2, preferably p is 1,
      - R^{6a}: is selected from the group consisting of CH₃ and CH₂CH₃,
      - R^{6b}: is CH₃,
      - R^{6c}: is H, if p is 0, and is selected from the group consisting of H and CH₃, if p is 1 or 2,
      the carbonyl group of Xaa6 is covalently attached to the α-nitrogen atom of Xaa7,
   Xaa7 is a residue of an amino acid of formula (VIIIa) 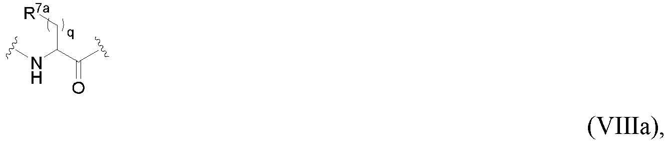 wherein
      - R^{7a}: is selected from the group consisting of SH and NH₂,
      - q: is 1 or 2, preferably q is 1,
   Xaa8 is a residue of an aliphatic cyclic α-amino acid, of an aliphatic heterocyclic α-amino acid or of an aliphatic cyclic α-amino acid with an annulated aromatic ring, preferably Xaa8 is a residue of an amino acid of formula (IXa) or (IXb), more preferably Xaa8 is a residue of an amino acid of formula (IXa), 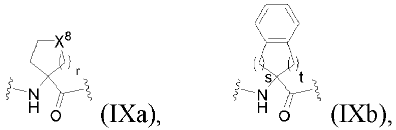 wherein
      - X⁸: is selected from the group consisting of NR^{8a}, O, NH, and CH₂, wherein R^{8a} is a Z group, wherein the Z group comprises a bio-distribution modifier with an optional linker, a chelator with an optional linker or a combination thereof, preferably the bio-distribution modifier is AGLU and the linker is Glutar, the chelator is DOTA, or if AGLU and DOTA are combined the linker is glu, or R^{8a} is acetyl,
      - r: is 2 or 1, preferably r is 2,
      - s: is 1 or 2, preferably s is 1,
      - t: is 1 or 2, preferably t is 1 if s is 1, and t is 2 if s is 2,
   or
   Xaa8 is a residue of an α-amino acid or an α-alkyl-α-amino acid, wherein preferably
   Xaa8 is a residue of an amino acid of formula (IXc), 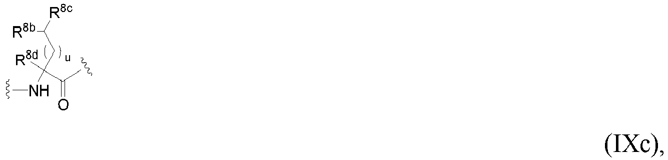 wherein
      - R^{8b}: is selected from the group consisting of H, OH, NH₂, NHR^{8e}, CH₃, CH₂CH₃, COOH, CONH₂, a substituted aromatic ring, an unsubstituted aromatic ring, a substituted bicyclic aromatic ring system, a substituted bicyclic hetero aromatic ring system, an unsubstituted bicyclic aromatic ring system and an unsubstituted bicyclic hetero aromatic ring system, preferably the aromatic ring is phenyl and the bicycylic heteroaromatic ring system is 3-indol, wherein R^{8e} is a Z group, wherein the Z group comprises a bio-distribution modifier with an optional linker, a chelator with an optional linker or a combination thereof,
      - R^{8c}: is selected from the group consisting of H and CH₃, if R^{8b} is selected from the group consisting of H, OH, NH₂, CH₃, CH₂CH₃, COOH, CONH₂, a substituted aromatic ring, an unsubstituted aromatic ring, a substituted bicyclic aromatic system, a substituted bicyclic hetero aromatic ring system, an unsubstituted bicyclic aromatic ring system and an unsubstituted bicyclic hetero aromatic ring system, or
      - R^{8d}: is selected from the group consisting of H, CH₃ and OH, if R^{8b} is selected from the group consisting of H, CH₃, CH₂CH₃, COOH, CONH₂, a substituted aromatic ring, an unsubstituted aromatic ring, a substituted bicyclic aromatic ring system, a substituted bicyclic hetero aromatic ring system, an unsubstituted bicyclic aromatic ring system and an unsubstituted bicyclic hetero aromatic ring system, is selected from the group consisting of H, CH₃ and CH₂CH₃,
      - u: is 0, 1, 2, 3, 4, or 5, preferably u is 0, 1 or 3,
      the carbonyl group of Xaa8 is covalently attached to the α-nitrogen atom of Xaa9,
   Xaa9 is a residue of an α-amino acid, preferably Xaa9 is a residue of an amino acid of formula (Xa) 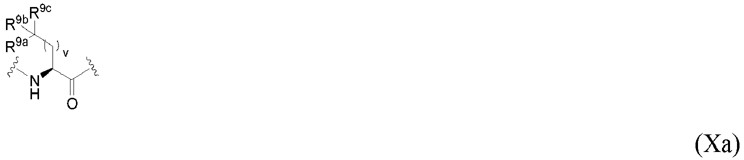 wherein
      - R^{9a}: is selected from the group consisting of CH₃, H, CH₂CH₃, OH, NH₂, NHC(=NH)NH₂, cyclo-hexyl, a substituted aromatic ring, an unsubstituted aromatic ring, a substituted bicyclic aromatic ring system, a substituted bicyclic hetero aromatic ring system, an unsubstituted bicyclic aromatic ring system and an unsubstituted bicyclic hetero aromatic ring system, preferably the aromatic ring is phenyl and the heteroaromatic ring system is 3-indol,
      - R^{9b}: is selected from the group consisting of CH₃ and H,
      - R^{9c}: is selected from the group consisting of H and CH₃,
      - v: is 0, 1 or 2, preferably v is 1,
   the carbonyl group of Xaa9 is covalently attached to the α-nitrogen atom of Xaa10,
   Xaa10 is a residue of an α-amino acid, preferably Xaa10 is a residue of an amino acid of formula (XIa) 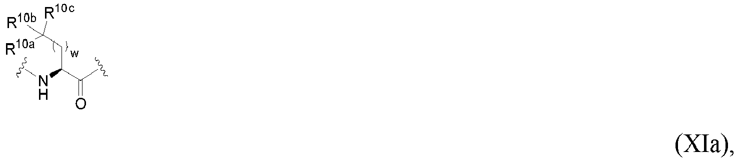 wherein
      - R^{10a}: is selected from the group consisting of CH₂CH₃, CH₃, phenyl, cyclohexyl and OH,
      - R^{10b}: is selected from the group consisting of CH₃ and H,
      - R^{10c}: is selected from the group consisting of H and CH₃,
      - w: is 0 or 1,
      the carbonyl group of Xaa10 is covalently attached to the nitrogen of Xaa11,
   Xaa11 is a residue of an α-amino acid with a nucleophilic group within the side chain or a residue of an aliphatic bis-nucleophile, preferably Xaa11 is a residue of an amino acid of formula (XIIa) 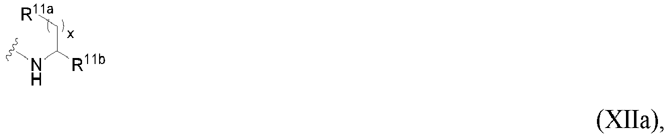 wherein
      - R^{11a}: is selected from the group consisting of SH and NH₂,
      - R^{11b}: is selected from the group consisting of C(=O)R, CONH₂, CO₂H, CH₂OH and H, wherein R is C-term, NH₂ or OH,
      - x: is 1 or 2,
   or
   Xaa11 is a residue of an amino acid of the formula (XIIb) 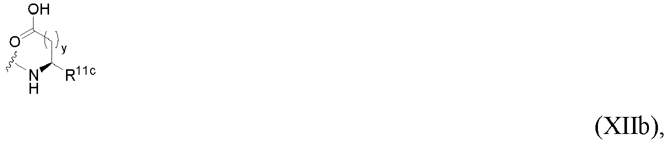 wherein
      - R^{11c}: is selected from the group consisting of H and CH₂CH₂CONH₂,
      - y: is 0 or 1
      wherein if R^{1a} in Xaa1 is selected from the group consisting of NH and NH(C1-C4)alkyl, the N-terminal modification group A
      (d) is a Z group or a bio-distribution modifier optionally comprising a linker, wherein the Z group comprises a chelator and optionally a linker,
      (e) is a residue of an amino acid Aaa, wherein an optional linker is interspersed between Aaa and Xaa1,
         wherein the amino acid Aaa is optionally substituted by a Z group, and wherein said Z group comprises a chelator and optionally a linker,
         wherein if Aaa is glu, Aaa is optionally substituted by a bio-distribution modifier,
   or
      (f) is selected from the group consisting of acetyl, a blocking group Abl of a structure of formula (Abl-Ia) or of a structure of formula (Abl-Ib) 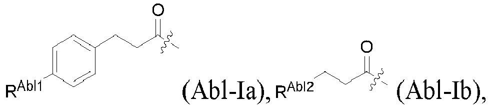 wherein
      - R^{Abl1}: is selected from the group consisting of H, OH and a halogen, preferably the halogen is selected from the group consisting of F and Cl, preferably R^{Abl1} is OH
      - R^{Abl2}: is selected from the group consisting of 1-naphtyl and 2-naphthyl,
      preferably Abl is of formula (Abl-Ia),
   and wherein, if present, the N-terminal modification group A is bound to the α-N atom of R^{1a} in Xaa1,
   wherein
   if Xaa11 is a residue of an amino acid of formula (XIIa), R^{11b} is C(=O)R, and R is C-term,
      the C-terminal group C-term
         (e) is one of more amino acids AA,
            wherein at least one or more of the one or more amino acids AA is optionally substituted by a Z group and/or a bio-distribution modifier optionally comprising a linker, wherein said Z group comprises a chelator and optionally a linker,
            wherein an optional linker is interspersed between Xaa11 and the first of the one or more amino acids AA,
            wherein an optional linker is interspersed between any of the more than one amino acids AA,
            wherein to the last of the one or more amino acids a blocking group Abl is attached, wherein Abl is selected from the group consisting of NH₂ and OH, wherein Abl preferably is NH₂, wherein if AA is glu, AA is optionally substituted by a bio-distribution modifier,
            wherein if AA is Lys, AA is optionally substituted by a chelator,
         (f) is a Z group,
            wherein the Z group comprises a chelator and a linker,
         (g) is a bio-distribution modifier optionally comprising a linker,
      or
         (h) is a blocking group Abl, wherein
         Abl is OH or NH₂,
         preferably Abl is NH₂,
         and wherein, if present, the C-terminal group C-term is bound to the C-atom of R^{11b} in Xaa11,
   wherein
   (e) if the cyclic peptide is of formula (Ia),
   under the proviso that Xaa11 is a residue of formula (XIIa), Yc is a structure of formula (XIIIa) or a structure of formula (XIIIb), more preferably Yc is a structure of formula (XIIIa) 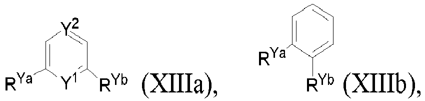 wherein
      - R^{Ya} and R^{Yb}: are each and independently -CH₂-,
      - Y¹: is selected from the group consisting of N and CH,
      - Y²: is selected from the group consisting of N and C-R^{Yc},
      - R^{Yc}: is selected from the group consisting of H and -CH₂-R^{Yd} and
      - R^{Yd}: is a structure of formula (XIIIc), (XIIId) or (XIIIe), 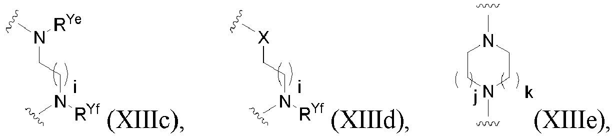
      wherein
      - R^{Ye} and R^{Yf}: are each and independently selected from the group consisting of H and (C1-C4)alkyl
      - i: is 1, 2, 3, 4, 5 or 6, preferably i is 1 or 2,
      - j and k: are each and independently 1, 2 or 3, and
      - X: is O or S, preferably X is S,
      wherein
      in formulae (XIIIc) and (XIIIe)
      one of the two nitrogen atoms is attached to -CH₂- of R^{Yc} and in formula (XIIId)
      -X- is attached to -CH₂- of R^{Yc}
      while the remaining nitrogen atom optionally connects to a Z group comprising a chelator and optionally a linker,
      preferably, if Yc is a structure of formulae (XIIIa), wherein R^{Yc} is -CH₂-R^{Yd}, R^{Yd} is a structure of formulae (XIIId), wherein X is S and R^{Yf} is H,
      and wherein
      one of R^{Ya} and R^{Yb}
         is linked to the S-atom of Xaa7 under the formation of a thioether, if R^{7a} in Xaa7 is SH, or,
         is linked to the N-atom of Xaa7 under the formation of an amine linkage, if R^{7a} in Xaa7 is NH₂,
      and the other one of R^{Ya} and R^{Yb}
         is linked to the S-atom of Xaa11 under the formation of a thioether if R^{11a} in Xaa11 is SH, or,
         is linked to the N-atom of Xaa11 under the formation of an amine linkage if R^{11a} in Xaa11 is NH₂,
      and the resulting generic structural formula corresponds to formula (Ic) 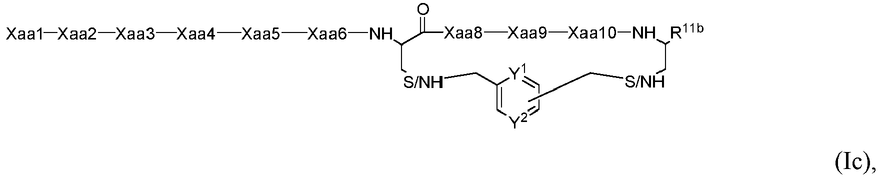 or wherein
   (f) if the cyclic peptide is of formula (Ia),
   under the proviso that
      R^{7a} in Xaa7 is NH₂ and Xaa11 is a residue of formula (XIIa) wherein R^{11a} is SH or NH₂,
      or
      Xaa11 is a residue of formula (XIIa) wherein R^{11a} is NH₂ and R^{7a} in Xaa7 is NH₂ or SH,
   Yc is a structure of formula (XIIIf), 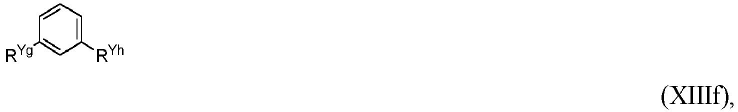 wherein
      - R^{Yg}: is -C(=O)- and
      - R^{Yh}: is -CH₂-,
   and wherein
      if R^{7a} in Xaa7 is NH₂ and Xaa11 is a residue of formula (XIIa) wherein R^{11a} is SH or NH₂,
      R^{Yg} is linked to the N-atom of R^{7a} in Xaa7 under the formation of an amide linkage
      and
      if R^{11a} in Xaa11 is SH, R^{Yh} is linked to said S-atom under the formation of a thioether linkage,
      or
      if R^{11a} in Xaa11 is NH₂, R^{Yh} is linked to said N-atom under the formation of an amine linkage
   or wherein
      if Xaa11 is a residue of formula (XIIa), wherein R^{11a} is NH₂ and R^{7a} in Xaa7 is either SH or NH₂,
      R^{Yg} is linked to the N-atom of R^{11a} in Xaa11 under the formation of an amide linkage
      and
      if R^{7a} in Xaa7 is SH, R^{Yh} is linked to said S-atom under the formation of a thioether linkage,
      or
      if R^{7a} in Xaa7 is NH₂, R^{Yh} is linked to said N-atom under the formation of an amine linkage,
      and the resulting generic structural formula corresponds to formula (Id) or formula (Ie) 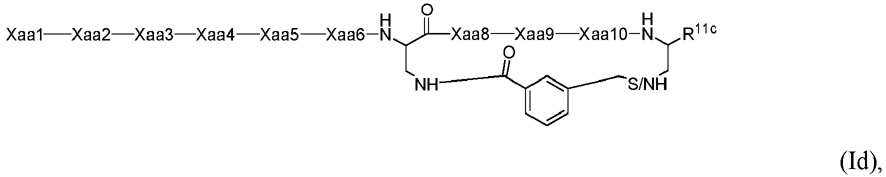 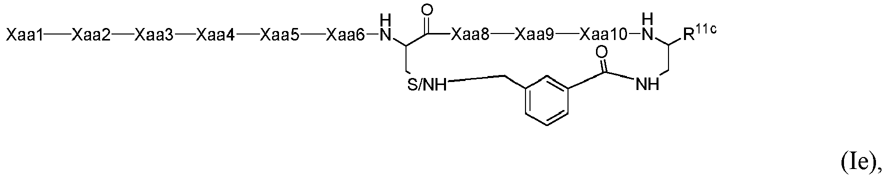 or wherein
   (g) if the cyclic peptide is of formula (Ia),
   if R^{7a} in Xaa7 is SH and Xaa11 is a residue of formula (XIIa) wherein R^{11a} is SH,
   Yc is a structure of formula (XIIIg), 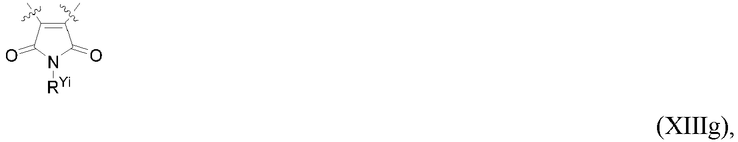 wherein
      R^{Yi} is selected from the group consisting of H and (C1-C6)alkyl and wherein the S-atom of Xaa7 is linked to Yc by a thioether linkage and the S-atom of Xaa11 is linked to Yc by a thioether linkage forming a cyclic structure with a generic formula corresponding to formula (If) 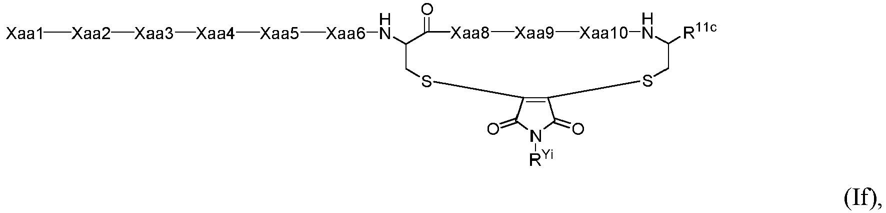 or wherein
   (h) if the cyclic peptide is of formula (Ia),
      if R^{7a} in Xaa7 is NH₂ and Xaa11 is a residue of an amino acid of the formula (XIIb),
      Yc is a structure of formula (XIIIh), 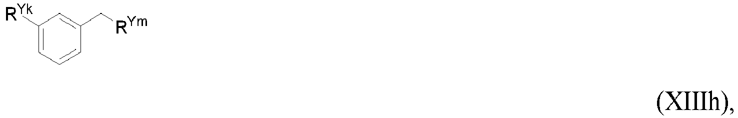 wherein,
         - R^{Yk}: is -C(=O)- and
         - R^{Ym}: is -NH-,
      wherein
         R^{Yk} is linked to the N-atom of R^{7a} in Xaa7 under the formation of an amide bond and R^{Ym} is linked to the carboxylic acid group of Xaa11 under the formation of an amide bond,
      and wherein
         the resulting generic structural formula corresponds to formula (Ig) 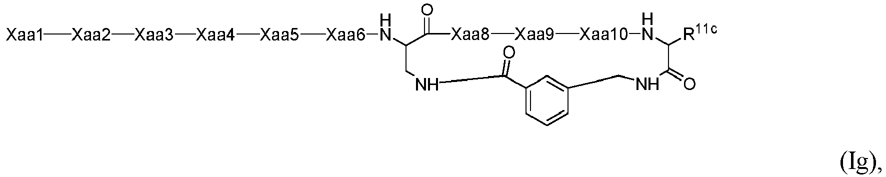
      and wherein
      if the cyclic peptide is of formula (Ib), the S atom of Xaa7 and the S atom of Xaa11 are covalently attached to each other forming a disulfide linkage.

The problem underlying the present invention is solved in a second aspect, which is also a first embodiment of the second aspect, by a peptide a peptide selected from the group consisting of
a cyclic peptide of formula (Ia) 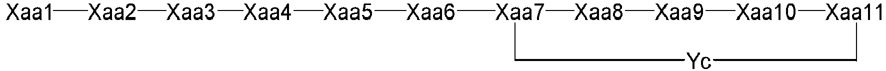 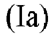
or a cyclic peptide of formula (Ib) 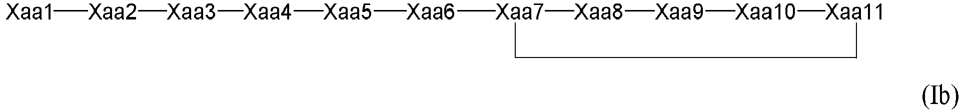
optionally an N-terminal modification group A attached to Xaa1, and
optionally a C-terminal group C-term attached to Xaa11,
wherein
   the peptide sequence is drawn from left to right in N- to C-terminal direction,
   Xaa1 is a residue of an α-amino acid with an acidic or polar group within the side chain or an aliphatic carboxylic acid with an additional acidic or polar group at the ω-position, preferably Xaa1 is a residue of an amino acid of formula (IIa), (IIb) or (IIc), more preferably Xaa1 is a residue of an amino acid formula (IIa), 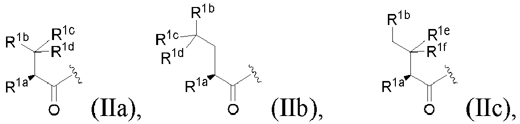 wherein
      - R^{1a}: is selected from the group consisting of NH, H, NH(C1-C4)alkyl, OH, and (C1-C4)alkyl, wherein if R^{1a} is selected from the group consisting of NH and NH(C1-C4)alkyl, the N-terminal modification group is covalently attached to the nitrogen atom of R^{1a}, and if R^{1a} is selected from the group consisting of H, OH or (C1-C4)alkyl the N-terminal modification group is absent,
      - R^{1b}: is selected from the group consisting of CO₂H, SO₃H, OPO₃H₂, CONH₂ and OH,
      - R^{1c}: is selected from the group consisting of H, OH and (C1-C2)alkyl under the proviso that R^{1b} is selected from the group consisting of CO₂H, SO₃H and CONH₂, or is selected from the group consisting of H and (C1-C2)alkyl under the proviso that R^{1b} is selected from the group consisting of OH and OPO₃H₂,
      - R^{1d}: is selected from the group consisting of H and (C1-C2)alkyl; preferably if R^{1c} is (C1-C2)alkyl, R^{1d} is the same (C1-C2)alkyl,
      - R^{1e}: is selected from the group consisting of H, OH and (C1-C2)alkyl,
      - R^{1f}: is selected from the group consisting of H and (C1-C2)alkyl; preferably if R^{1e} is (C1-C2)alkyl, R^{1f} is the same (C1-C2)alkyl,
   the carbonyl group of Xaa1 is covalently attached to the α-nitrogen atom of Xaa2,
   Xaa2 is a residue of an α-amino acid with a substituted or unsubstituted bicyclic aromatic or heteroaromatic side chain, preferably Xaa2 is a residue of an amino acid of formula (IIIa) or (IIIb), more preferably a residue of an amino acid of formula (IIIa), 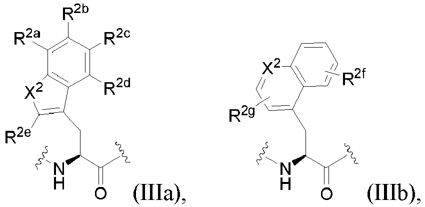 wherein
      - X²: is selected from the group consisting of NH, NCH₃ and S; preferably selected from the group consisting of NH and S, if Xaa2 is an amino acid of formula (IIIa), or is selected from the group consisting of N, CH, CF and C-CH₃; preferably selected from the group consisting of N and CH, if Xaa2 is an amino acid of formula (IIIb),
      - R^{2a}: is selected from the group consisting of H, F, Cl, Br, CH₃ and OCH₃, preferably R^{2a} is selected from the group consisting of H and F,
      - R^{2b}: is selected from the group consisting of H, a halogen, CH₃ and OCH₃, preferably the halogen is selected from the group consisting of Cl and Br, more preferably R^{2b} is selected from the group consisting of H, Cl and Br,
      - R^{2c}: is selected from the group consisting of H, OH, a halogen, CH₃ and OCH₃, preferably the halogen is selected from the group consisting of F, Cl and Br, more preferably R^{2c} is selected from the group consisting of H, F and OH,
      - R^{2d}: is selected from the group consisting of H, a halogen, CH₃ and OCH₃, wherein the halogen is selected from the group consisting of F, Cl and Br, preferably R^{2d} is H,
      - R^{2e}: is selected from the group consisting of H, a halogen and CH₃, preferably the halogen is Cl, more preferably R^{2e} is H,
      - R^{2f}: is selected from the group consisting of H and OCH₃, preferably R^{2f} is H,
      - R^{2g}: is selected from the group consisting of H, OH, CH₃ and OCH₃, preferably R^{2g} is H,
   or
   Xaa2 is a residue of an α-amino acid with a substituted or unsubstituted aromatic or heteroaromatic side chain, wherein Xaa2 is preferably a residue of an amino acid of formula (IIIc) 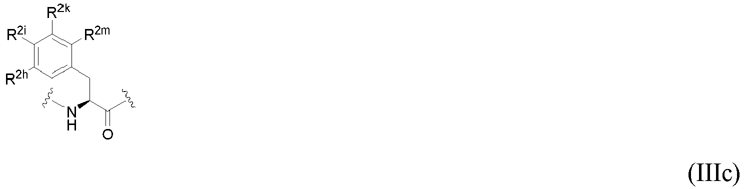 wherein
      - R^{2h}: is selected from the group consisting of H, CH₃, CF₃, OH, OCH₃, OCF₃, CONH₂ and a halogen, preferably the halogen is selected from the group consisting of Cl and Br, more preferably R^{2h} is H, Cl or Br,
      - R²¹: is selected from the group consisting of H, CH₃, C(CH₃)₃, CF₃, OH, OCH₃, OCH₂CH₃, OCF₃, CONH₂, CO₂H and a halogen, preferably the halogen is selected from the group consisting of Cl and Br, more preferably R²¹ is selected from the group consisting of H, Cl and Br,
      - R^{2k}: is selected from the group consisting of H, CH₃, CF₃, OH, OCH₃, OCF₃, CONH₂ and a halogen, preferably the halogen is selected from the group consisting of Cl and Br, more preferably R^{2k} is selected from the group consisting of H, Cl and Br,
      - R^{2m}: is selected from the group consisting of H, CH₃, CF₃, OH, OCH₃, OCF₃, CONH₂ and a halogen, preferably the halogen is selected from the group consisting of Cl and Br, more preferably R^{2m} is selected from the group consisting of H, Cl and Br,
   the carbonyl group of Xaa2 is covalently attached to the α-nitrogen atom of Xaa3,
   Xaa3 is an α-amino acid with a substituted or unsubstituted bicyclic aromatic or heteroaromatic side chain, preferably Xaa3 is a residue of an amino acid of formula (IVa) or (IVb), more preferably Xaa3 is a residue of an amino acid of formula (IVa), 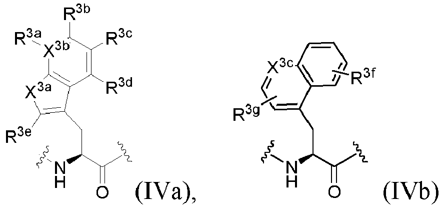 wherein
      - x^{3a}: is selected from the group consisting of NH, NCH₃ and S, preferably selected from the group consisting of NH and S,
      - X^{3b}: is selected from the group consisting of C and N, preferably X^{3b} is C,
      - X^{3c}: is selected from the group consisting of C and N, preferably X^{3c} is C,
      - R^{3a}: is, under the proviso that X^{3b} is C, selected from the group consisting of H, a halogen, CH₃ and OCH₃, preferably the halogen is F, or is absent if X^{3b} is N,
      - R^{3b}: is selected from the group consisting of H, a halogen, CH₃ and OCH₃, preferably the halogen is selected from the group consisting of F and Cl, more preferably R^{3b} is selected from the group consisting of H and F,
      - R^{3c}: is selected from the group consisting of H, a halogen, CH₃ and OCH₃, preferably the halogen is selected from the group consisting of Cl, Br and F, more preferably R^{3c} is selected from the group consisting of Cl and Br,
      - R^{3d}: is selected from the group consisting of H, a halogen, CH₃ and OCH₃, preferably the halogen is F,
      - R^{3e}: is selected from the group consisting of H, a halogen and CH₃, preferably the halogen is Cl,
      - R^{3f}: is selected from the group consisting of H and OCH₃,
      - R^{3g}: is selected from the group consisting of H, F, OH, CH₃ and OCH₃,
   or
   Xaa3 is a residue of an α-amino acid with a substituted or unsubstituted aromatic or heteroaromatic side chain, preferably Xaa3 is a residue of an amino acid of formula (IVc) 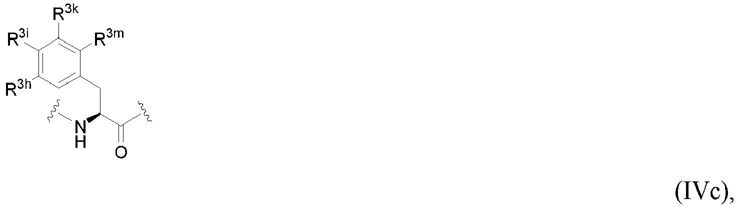 wherein
      - R^{3h}: is selected from the group consisting of H, a halogen, OH, CH₃, CF₃, OCH₃, OCF₃ and CONH₂, preferably the halogen is selected from the group consisting of Cl and Br,
      - R³ⁱ: is selected from the group consisting of H, a halogen, OH, CH₃, CF₃, OCH₃, OCF₃ and CONH₂, preferably the halogen is selected from the group consisting of Cl and Br,
      - R^{3k}: is selected from the group consisting of H, a halogen, OH, CH₃, CF₃, OCH₃, OCF₃ and CONH₂, preferably the halogen is selected from the group consisting of Cl and Br, and
      - R^{3m}: is selected from the group consisting of H, a halogen, OH, CH₃, CF₃, OCH₃, OCF₃ and CONH₂, preferably the halogen is selected from the group consisting of Cl and Br, preferably R^{3m} is selected from the group consisting of H and Cl,
   the carbonyl group of Xaa3 is covalently attached to the α-nitrogen atom of Xaa4,
   Xaa4 is a residue of a cyclic non-aromatic amino acid, preferably Xaa4 is a residue of an amino acid of formula (Va) 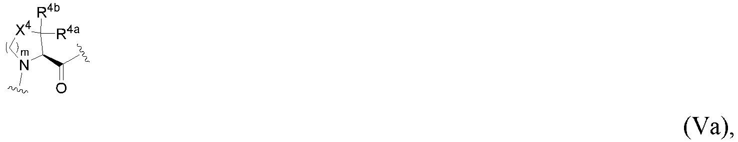 wherein
      - R^{4a}: is selected from the group consisting of H, OH, CH₃ and CF₃
      - R^{4b}: is selected from the group consisting of H and CH₃, preferably R^{4b} is CH₃ if R^{4a} is CH₃,
      - X⁴: is selected from the group consisting of CHR^{4c}, CH₂, S and O, wherein R^{4c} is absent or is selected from the group consisting of NH₂, OH, H, NHR^{4d}, CH₃ and F, wherein R^{4d} is absent or is selected from the group consisting of H, Ac, a Z group, wherein the Z group comprises a bio-distribution modifier with an optional linker, a chelator with an optional linker or a combination thereof, preferably the chelator is DOTA, the bio-distribution modifier is AGLU comprising the linker Glutar
      - m: is 1 or 2, preferably m is 1
   or
   Xaa4 is a residue of an N-alkylated amino acid, preferably Xaa4 is a residue of an amino acid of formula (Vb) 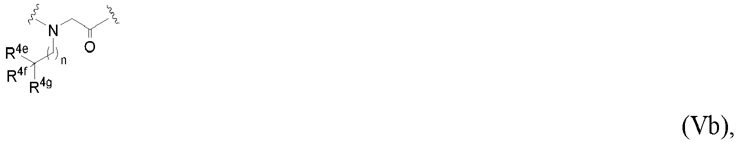 wherein
      - n: is 0, 1, 2, 3, 4, or 5, preferably n is 0, 1 or 3,
      - R^{4e}: is, if n is 0, selected from the group consisting of H, CH₃, a substituted or unsubstituted aromatic residue, COOH, CONH₂ and C(=O)R^{4h}, or is, if n is 1, 2, 3, 4 or 5, selected from the group consisting of H, CH₃, a substituted or unsubstituted aromatic residue, OH, NH₂, COOH, CONH₂ and C(=O)R^{4h},
      - R^{4f}: is selected from the group consisting of H, CH₃ and CH₂CH₃,
      - R^{4g}: is selected from the group consisting of H and CH₃,
      - R^{4h}: is AGLU,
   or
   Xaa4 is a residue of a bicyclic non-aromatic amino acid, preferably Xaa4 is a residue of an amino acid of formula (Vc) 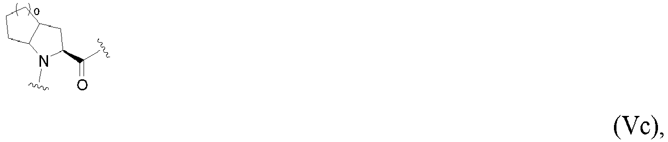 wherein
      - o: is 1 or 2,
   the carbonyl group of Xaa4 is covalently attached to the α-nitrogen atom of Xaa5,
   Xaa5 a residue of an α-amino acid with a polar, preferably an acidic group within the side chain, preferably Xaa5 is a residue of an amino acid of formula (VIa), (VIb) or (VIc), more preferably Xaa5 is a residue of an amino acid formula (VIa) or (VIb), 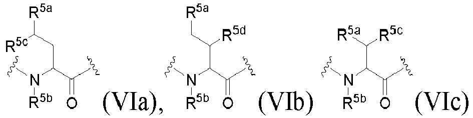 wherein
      - R^{5a}: is selected from the group consisting of COOH, CONH₂, OPO₃H₂, OH and SO₃H,
      - R^{5b}: is selected from the group consisting of H and (C1-C4)alkyl,
      - R^{5c}: is selected from the group consisting of H, OH and CH₃ under the proviso that R^{5a} is selected from the group consisting of COOH, CONH₂, OPO₃H₂ and SO₃H, or is selected from the group consisting of H and CH₃ under the proviso that R^{5a} is selected from the group consisting of COOH, CONH₂, OPO₃H₂, OH and SO₃H,
      - R^{5d}: is selected from the group consisting of H, OH and CH₃
   the carbonyl group of Xaa5 is covalently attached to the α-nitrogen atom of Xaa6,
   Xaa6 is a residue of an α-amino acid with a branched aliphatic side chain, preferably
   Xaa6 is a residue of an amino acid of formula (VIIa) 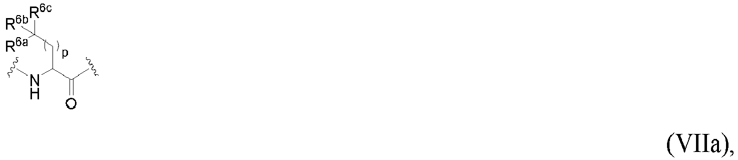 wherein
      - p: is 1, 0 or 2, preferably p is 1,
      - R^{6a}: is selected from the group consisting of CH₃ and CH₂CH₃,
      - R^{6b}: is CH₃,
      - R^{6c}: is H, if p is 0, and is selected from the group consisting of H and CH₃, if p is 1 or 2,
      the carbonyl group of Xaa6 is covalently attached to the α-nitrogen atom of Xaa7,
   Xaa7 is a residue of an amino acid of formula (VIIIa) 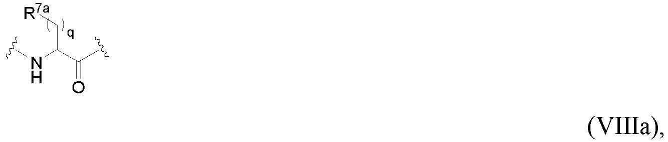 wherein
      - R^{7a}: is selected from the group consisting of SH and NH₂,
      - q: is 1 or 2, preferably q is 1,
   Xaa8 is a residue of an aliphatic cyclic α-amino acid, of an aliphatic heterocyclic α-amino acid or of an aliphatic cyclic α-amino acid with an annulated aromatic ring, preferably Xaa8 is a residue of an amino acid of formula (IXa) or (IXb), more preferably Xaa8 is a residue of an amino acid of formula (IXa), 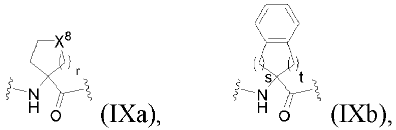 wherein
      - X⁸: is selected from the group consisting of NR^{8a}, O, NH, and CH₂, wherein R^{8a} is a Z group, wherein the Z group comprises a bio-distribution modifier with an optional linker, a chelator with an optional linker or a combination thereof, preferably the bio-distribution modifier is AGLU and the linker is Glutar, the chelator is DOTA, or if AGLU and DOTA are combined the linker is glu, or R^{8a} is acetyl,
      - r: is 2 or 1, preferably r is 2,
      - s: is 1 or 2, preferably s is 1,
      - t: is 1 or 2, preferably t is 1 if s is 1, and t is 2 if s is 2,
   or
   Xaa8 is a residue of an α-amino acid or an α-alkyl-α-amino acid, wherein preferably
   Xaa8 is a residue of an amino acid of formula (IXc), 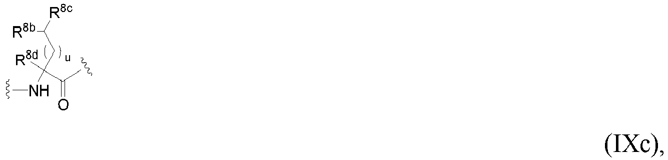 wherein
      - R^{8b}: is selected from the group consisting of H, OH, NH₂, NHR^{8e}, CH₃, CH₂CH₃, COOH, CONH₂, a substituted aromatic ring, an unsubstituted aromatic ring, a substituted bicyclic aromatic ring system, a substituted bicyclic hetero aromatic ring system, an unsubstituted bicyclic aromatic ring system and an unsubstituted bicyclic hetero aromatic ring system, preferably the aromatic ring is phenyl and the bicycylic heteroaromatic ring system is 3-indol, wherein R^{8e} is a Z group, wherein the Z group comprises a bio-distribution modifier with an optional linker, a chelator with an optional linker or a combination thereof,
      - R^{8c}: is selected from the group consisting of H and CH₃, if R^{8b} is selected from the group consisting of H, OH, NH₂, CH₃, CH₂CH₃, COOH, CONH₂, a substituted aromatic ring, an unsubstituted aromatic ring, a substituted bicyclic aromatic system, a substituted bicyclic hetero aromatic ring system, an unsubstituted bicyclic aromatic ring system and an unsubstituted bicyclic hetero aromatic ring system, or is selected from the group consisting of H, CH₃ and OH, if R^{gb} is selected from the group consisting of H, CH₃, CH₂CH₃, COOH, CONH₂, a substituted aromatic ring, an unsubstituted aromatic ring, a substituted bicyclic aromatic ring system, a substituted bicyclic hetero aromatic ring system, an unsubstituted bicyclic aromatic ring system and an unsubstituted bicyclic hetero aromatic ring system,
      - R^{8d}: is selected from the group consisting of H, CH₃ and CH₂CH₃,
      - u: is 0, 1, 2, 3, 4, or 5, preferably u is 0, 1 or 3,
      the carbonyl group of Xaa8 is covalently attached to the α-nitrogen atom of Xaa9,
   Xaa9 is a residue of an α-amino acid, preferably Xaa9 is a residue of an amino acid of formula (Xa) 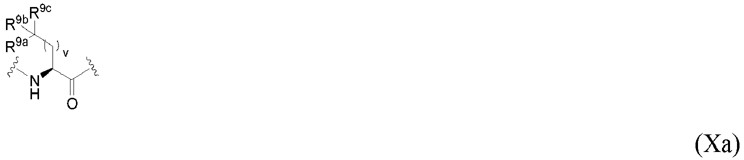 wherein
      - R^{9a}: is selected from the group consisting of CH₃, H, CH₂CH₃, OH, NH₂, NHC(=NH)NH₂, cyclo-hexyl, a substituted aromatic ring, an unsubstituted aromatic ring, a substituted bicyclic aromatic ring system, a substituted bicyclic hetero aromatic ring system, an unsubstituted bicyclic aromatic ring system and an unsubstituted bicyclic hetero aromatic ring system, preferably the aromatic ring is phenyl and the heteroaromatic ring system is 3-indol,
      - R^{9b}: is selected from the group consisting of CH₃ and H,
      - R^{9c}: is selected from the group consisting of H and CH₃,
      - v: is 0, 1 or 2, preferably v is 1,
   the carbonyl group of Xaa9 is covalently attached to the α-nitrogen atom of Xaa10,
   Xaa10 is a residue of an α-amino acid, preferably Xaa10 is a residue of an amino acid of formula (XIa) 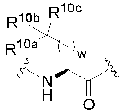 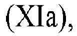 wherein
      - R^{10a}: is selected from the group consisting of CH₂CH₃, CH₃, phenyl, cyclohexyl and OH,
      - R^{10b}: is selected from the group consisting of CH₃ and H,
      - R^{10c}: is selected from the group consisting of H and CH₃,
      - w: is 0 or 1,
      the carbonyl group of Xaa10 is covalently attached to the nitrogen of Xaa11,
   Xaa11 is a residue of an α-amino acid with a nucleophilic group within the side chain or a residue of an aliphatic bis-nucleophile, preferably Xaa11 is a residue of an amino acid of formula (XIIa) 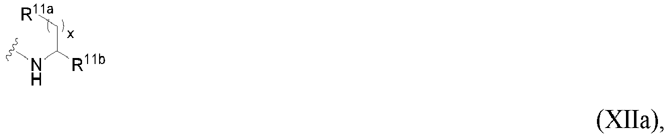 wherein
      - R^{11a}: is selected from the group consisting of SH and NH₂,
      - R^{11b}: is selected from the group consisting of C(=O)R, CONH₂, CO₂H, CH₂OH and H, wherein R is C-term, NH₂ or OH,
      - x: is 1 or 2,
   or
   Xaa11 is a residue of an amino acid of the formula (XIIb) 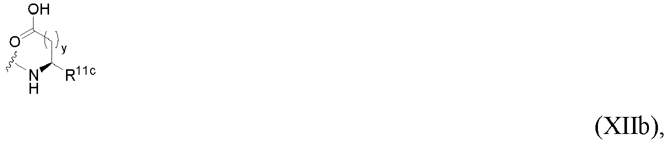 wherein
      - R^{11c}: is selected from the group consisting of H and CH₂CH₂CONH₂,
      - y: is 0 or 1
   wherein
   if R^{1a} in Xaa1 is selected from the group consisting of NH and NH(C1-C4)alkyl, the N-terminal modification group A
      (g) is a Z group or a bio-distribution modifier optionally comprising a linker, wherein the Z group comprises a chelator and optionally a linker,
      (h) is a residue of an amino acid Aaa, wherein an optional linker is interspersed between Aaa and Xaa1,
         wherein the amino acid Aaa is optionally substituted by a Z group, and wherein said Z group comprises a chelator and optionally a linker,
         wherein if Aaa is glu, Aaa is optionally substituted by a bio-distribution modifier,
      or
      (i) is selected from the group consisting of acetyl, a blocking group Abl of a structure of formula (Abl-Ia) or of a structure of formula (Abl-Ib) 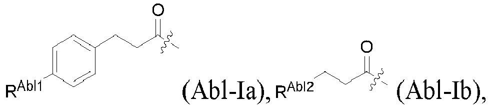 wherein
      - R^{Ab11}: is selected from the group consisting of H, OH and a halogen, preferably the halogen is selected from the group consisting of F and Cl, preferably R^{Ab11} is OH
      - R^{Ab12}: is selected from the group consisting of 1-naphtyl and 2-naphthyl,
      preferably Abl is of formula (Abl-Ia),
   and wherein, if present, the N-terminal modification group A is bound to the α-N atom of R^{1a} in Xaa1,
   wherein
   if Xaa11 is a residue of an amino acid of formula (XIIa), R^{11b} is C(=O)R, and R is C-term,
      the C-terminal group C-term
         (i) is one of more amino acids AA,
            wherein at least one or more of the one or more amino acids AA is optionally substituted by a Z group and/or a bio-distribution modifier optionally comprising a linker, wherein said Z group comprises a chelator and optionally a linker,
            wherein an optional linker is interspersed between Xaa11 and the first of the one or more amino acids AA,
            wherein an optional linker is interspersed between any of the more than one amino acids AA,
            wherein to the last of the one or more amino acids a blocking group Abl is attached, wherein Abl is selected from the group consisting of NH₂ and OH, wherein Abl preferably is NH₂, wherein if AA is glu, AA is optionally substituted by a bio-distribution modifier,
            wherein if AA is Lys, AA is optionally substituted by a chelator,
         (j) is a Z group,
            wherein the Z group comprises a chelator and a linker,
         (k) is a bio-distribution modifier optionally comprising a linker,
            or
         (1) is a blocking group Abl, wherein
            Abl is OH or NH₂,
            preferably Abl is NH₂,
      and wherein, if present, the C-terminal group C-term is bound to the C-atom of R^{11b} in Xaa11,
   wherein
   (i) if the cyclic peptide is of formula (Ia),
   under the proviso that Xaa11 is a residue of formula (XIIa), Yc is a structure of formula (XIIIa) or a structure of formula (XIIIb), more preferably Yc is a structure of formula (XIIIa) 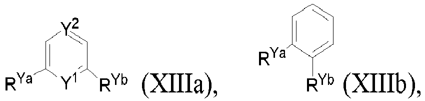 wherein
      - R^{Ya} and R^{Yb}: are each and independently -CH₂-,
      - Y¹: is selected from the group consisting of N and CH,
      - Y²: is selected from the group consisting of N and C-R^{Yc},
      - R^{Yc}: is selected from the group consisting of H and -CH₂-R^{Yd} and
      - R^{Yd}: is a structure of formula (XIIIc), (XIIId) or (XIIIe), 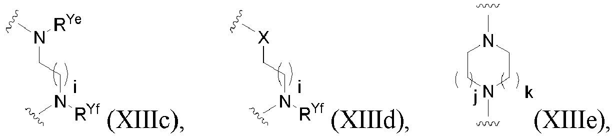
      wherein
      - R^{Ye} and R^{Yf}: are each and independently selected from the group consisting of H and (C1-C4)alkyl
      - i: is 1, 2, 3, 4, 5 or 6, preferably i is 1 or 2,
      - j and k: are each and independently 1, 2 or 3, and
      - X: is O or S, preferably X is S,
      wherein
      in formulae (XIIIc) and (XIIIe)
         one of the two nitrogen atoms is attached to -CH₂- of R^{Yc}
      and in formula (XIIId)
         -X- is attached to -CH₂- of R^{Yc}
      while the remaining nitrogen atom optionally connects to a Z group comprising a chelator and optionally a linker,
      preferably, if Yc is a structure of formulae (XIIIa), wherein R^{Yc} is -CH₂-R^{Yd}, R^{Yd} is a structure of formulae (XIIId), wherein X is S and R^{Yf} is H,
   and wherein
      one of R^{Ya} and R^{Yb}
         is linked to the S-atom of Xaa7 under the formation of a thioether, if R^{7a} in Xaa7 is SH, or,
         is linked to the N-atom of Xaa7 under the formation of an amine linkage, if R^{7a} in Xaa7 is NH₂,
      and the other one of R^{Ya} and R^{Yb}
         is linked to the S-atom of Xaa11 under the formation of a thioether if R^{11a} in Xaa11 is SH, or,
         is linked to the N-atom of Xaa11 under the formation of an amine linkage if R^{11a} in Xaa11 is NH₂,
      and the resulting generic structural formula corresponds to formula (Ic) 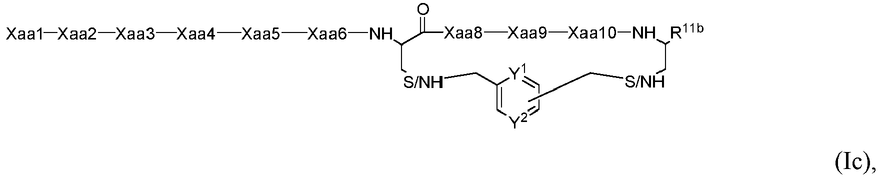 or wherein
   (j) if the cyclic peptide is of formula (Ia),
   under the proviso that
      R^{7a} in Xaa7 is NH₂ and Xaa11 is a residue of formula (XIIa) wherein R^{11a} is SH or NH₂,
      or
      Xaa11 is a residue of formula (XIIa) wherein R^{11a} is NH₂ and R^{7a} in Xaa7 is NH₂ or SH,
   Yc is a structure of formula (XIIIf), 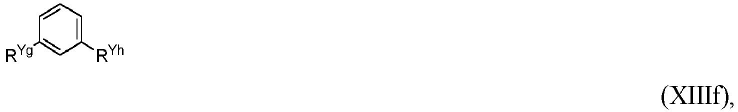 wherein
      - R^{Yg}: is -C(=O)- and
      - R^{Yh}: is -CH₂-,
      and wherein
      if R^{7a} in Xaa7 is NH₂ and Xaa11 is a residue of formula (XIIa) wherein R^{11a} is SH or NH₂,
      R^{Yg} is linked to the N-atom of R^{7a} in Xaa7 under the formation of an amide linkage
      and
      if R^{11a} in Xaa11 is SH, R^{Yh} is linked to said S-atom under the formation of a thioether linkage,
      or
      if R^{11a} in Xaa11 is NH₂, R^{Yh} is linked to said N-atom under the formation of an amine linkage
   or wherein
      if Xaa11 is a residue of formula (XIIa), wherein R^{11a} is NH₂ and R^{7a} in Xaa7 is either SH or NH₂,
         R^{Yg} is linked to the N-atom of R^{11a} in Xaa11 under the formation of an amide linkage
         and
         if R^{7a} in Xaa7 is SH, R^{Yh} is linked to said S-atom under the formation of a thioether linkage,
         or
         if R^{7a} in Xaa7 is NH₂, R^{Yh} is linked to said N-atom under the formation of an amine linkage,
      and the resulting generic structural formula corresponds to formula (Id) or formula (Ie) 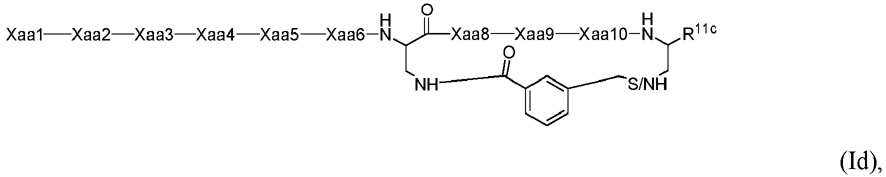 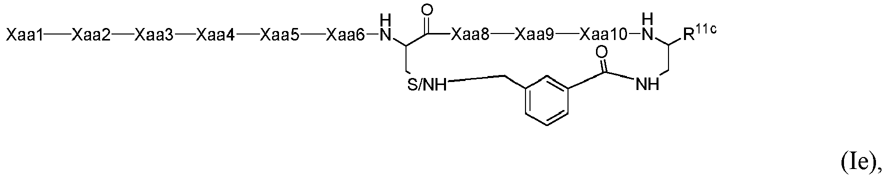
      or wherein
   (k) if the cyclic peptide is of formula (Ia),
   if R^{7a} in Xaa7 is SH and Xaa11 is a residue of formula (XIIa) wherein R^{11a} is SH,
   Yc is a structure of formula (XIIIg), 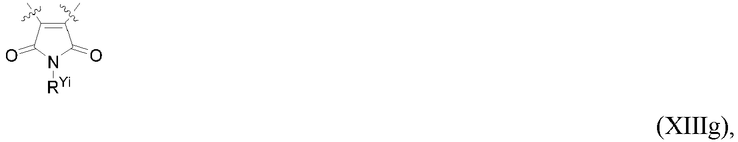 wherein
      R^{Yi} is selected from the group consisting of H and (C1-C6)alkyl and wherein the S-atom of Xaa7 is linked to Yc by a thioether linkage and the S-atom of Xaa11 is linked to Yc by a thioether linkage forming a cyclic structure with a generic formula corresponding to formula (If) 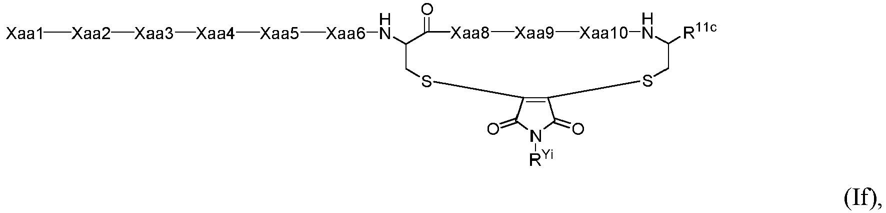 or wherein
   (1) if the cyclic peptide is of formula (Ia),
   if R^{7a} in Xaa7 is NH₂ and Xaa11 is a residue of an amino acid of the formula (XIIb),
   Yc is a structure of formula (XIIIh), 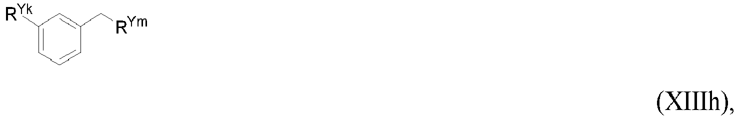 wherein,
      - R^{Yk}: is -C(=O)- and
      - R^{Ym}: is -NH-,
   wherein
      R^{Yk} is linked to the N-atom of R^{7a} in Xaa7 under the formation of an amide bond and R^{Ym} is linked to the carboxylic acid group of Xaa11 under the formation of an amide bond,
   and wherein
      the resulting generic structural formula corresponds to formula (Ig) 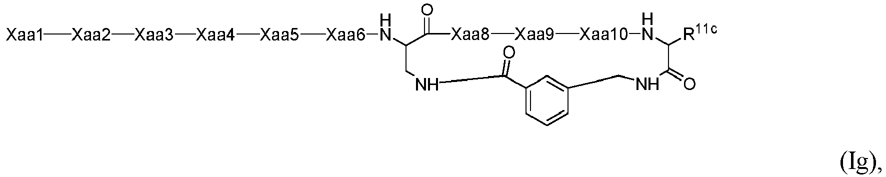
   and wherein
   if the cyclic peptide is of formula (Ib), the S atom of Xaa7 and the S atom of Xaa11 are covalently attached to each other forming a disulfide linkage.

According to the present invention, each and any embodiment of the compound of the first aspect is also an embodiment of the peptide of the second aspect, and vice versa.

The compound of the first aspect, including any embodiment thereof, and the peptide of the second aspect, including any embodiment thereof, are also referred to as the compound of the invention, compounds of the invention of compounds.

The problem underlying the present invention is also solved in a third aspect which is also a first embodiment of the third aspect, by the compound of the first aspect or the peptide of the second aspect, including each and any embodiment thereof, for the diagnosis of a disease.

The problem underlying the present invention is also solved in a fourth aspect which is also a first embodiment of the fourth aspect, by the compound of the first aspect or the peptide of the second aspect, including each and any embodiment thereof, for use in a method for the treatment of a disease.

The problem underlying the present invention is also solved in a fifth aspect which is also a first embodiment of the fifth aspect, by the compound of the first aspect or the peptide of the second aspect, including each and any embodiment thereof, for use in a method for the identification of a subject, wherein the subject is likely to respond or likely not to respond to a treatment of a disease, wherein the method for the identification of a subject comprises carrying out a method of diagnosis using the compound of the first aspect or the peptide of the second aspect, including each and any embodiment thereof.

The problem underlying the present invention is also solved in a sixth aspect which is also a first embodiment of the sixth aspect, by the compound of the first aspect or the peptide of the second aspect, including each and any embodiment thereof, for use in a method for the selection of a subject from a group of subjects, wherein the subject is likely to respond or likely not to respond to a treatment of a disease, wherein the method for the selection of a subject from a group of subjects comprises carrying out a method of diagnosis using the compound of the first aspect or the peptide of the second aspect including any embodiment thereof.

The problem underlying the present invention is also solved in a seventh aspect which is also a first embodiment of the seventh aspect, by the compound of the first aspect or the peptide of the second aspect, including each and any embodiment thereof, for use in a method for the stratification of a group of subjects into subjects which are likely to respond to a treatment of a disease, and into subjects which are not likely to respond to a treatment of a disease, wherein the method for the stratification of a group of subjects comprises carrying out a method of diagnosis using the compound of the first aspect or the peptide of the second aspect, including any embodiment thereof.

The problem underlying the present invention is solved in an eighth aspect by a composition, preferably a pharmaceutical composition, wherein the composition comprises the compound of the first aspect and/or the peptide of the second aspect, including any embodiment thereof, and a pharmaceutically acceptable excipient.

The problem underlying the present invention is solved in a ninth aspect by a kit comprising the compound of the first aspect and/or the peptide of the the second aspect<, including any embodiment thereof, one or more optional excipient(s) and optionally one or more device(s), whereby the device(s) is/are selected from the group comprising a labeling device, a purification device, a handling device, a radioprotection device, an analytical device or an administration device.

The present inventors have surprisingly found that the compounds of the invention show a high affinity to Gastric Inhibitory Peptide Receptor (GIPR). Furthermore, the present inventors have surprisingly found that compounds of the invention show other characteristics which predestine the compounds of the invention for use in the diagnosis and therapy of diseases involving Gastric Inhibitory Peptide Receptor (GIPR). Such other characteristics comprise high stability in plasma and selectivity for Gastric Inhibitory Peptide Receptor (GIPR). Another surprising characteristic of the compounds of the invention is a low retention in normal, i.e. non-diseased tissues.

According to the present invention the compounds of the invention may comprise one or more chelators which is either directly or by means of a linker attached to the compounds of the invention. It is, however, preferred that the compounds of the invention comprise only one chelator. More preferably, such one chelator is attached to Xaa8 or such one chelator is comprised by the N-terminal group A. Most preferably, such one chelator is directly attached to Xaa8 without any linker.

In an embodiment and as preferably used herein, a diagnostically active compound is a compound which is suitable for or useful in the diagnosis of a disease.

In an embodiment and as preferably used herein, a diagnostic agent or a diagnostically active agent is a compound which is suitable for or useful in the diagnosis of a disease.

In an embodiment and as preferably used herein, a therapeutically active compound is a compound which is suitable for or useful in the treatment of a disease.

In an embodiment and as preferably used herein, a therapeutic agent or a therapeutically active agent is a compound which is suitable for or useful in the treatment of a disease.

In an embodiment and as preferably used herein, a theragnostically active compound is a compound which is suitable for or useful in both the diagnosis and therapy of a disease.

In an embodiment and as preferably used herein, a theragnostic agent or a theragnostically active agent is a compound which is suitable for or useful in both the diagnosis and therapy of a disease.

In an embodiment and as preferably used herein, theragnostics is a method for the combined diagnosis and therapy of a disease; preferably, the combined diagnostically and therapeutically active compounds used in theragnostics are radiolabeled.

In an embodiment and as preferably used herein, treatment of a disease is treatment and/or prevention of a disease.

In an embodiment and as preferably used hrein, pEC50 is determined in a FACS binding assay, wherein the FACS binding assay is as described in the example part.

In an embodiment and as preferably used herein, pIC50 is determined in a FACS binding assay, wherein the FACS binding assay is as described in the example part.

In an embodiment and as preferably used herein, a disease involving GIPR is a disease where cells including but not limited to tumor cells expressing or pancreatic beta cells, preferably in an upregulated manner, GIPR and tissue either expressing GIPR, preferably in an upregulated manner respectively, are either a or the cause for the disease and/or the symptoms of the disease, or are part of the pathology underlying the disease. A preferred GIPR-expressing cell is a tumor cell. In an embodiment of the disease, preferably when used in connection with the treatment, treating and/or therapy of the disease, affecting the cells, the tissue and pathology, respectively, results in cure, treatment or amelioration of the disease and/or the symptoms of the disease. In an embodiment of the disease, preferably when used in connection with the diagnosis and/or diagnosing of the disease, labeling of the GIPR-expressing cells and/or of the GIPR-expressing tissue allows discriminating or distinguishing said cells and/or said tissue from healthy or GIPR -non-expressing cells and/or healthy or GIPR-non-expressing tissue. More preferably such discrimination or distinction forms the basis for said diagnosis and diagnosing, respectively. In an embodiment thereof, labeling means the interaction of a detectable label either directly or indirectly with the GIPR-expressing cells and/or with the GIPR-expressing tissue or tissue containing such GIPR-expressing cells; more preferably such interaction involves or is based on the interaction of the label or a compound bearing such label with GIPR.

In an embodiment and as preferably used herein, a target cell is a cell which is expressing GIPR and is a or the cause for a disease and/or the symptoms of a disease, or is part of the pathology underlying a disease.

In an embodiment and as preferably used herein, a non-target cell is a cell which is either not expressing GIPR and/or is not a or the cause for a disease and/or the symptoms of a disease, or is part of the pathology underlying a disease.

In an embodiment and as preferably used herein, a neoplasm is an abnormal new growth of cells. The cells in a neoplasm grow more rapidly than normal cells and will continue to grow if not treated. A neoplasm may be benign or malignant.

In an embodiment and as preferably used herein, a tumor is a mass lesion that may be benign or malignant.

In an embodiment and as preferably used herein, a cancer is a malignant neoplasm.

In an embodiment and as preferably used herein, a cell and/or tissue expressing GIPR is overexpressing GIPR. More preferably, GIPR is overexpressed if the extent of expression is one observed in a cell and/or tissue of a person diagnosed to suffer from the disease, preferably the disease being cancer and metabolic disease as disclosed herein.

Accordingly, the compounds of the invention are thus particularly suitable for and useful in the diagnosis and treatment, respectively, of these diseases. Insofar, the above indications are indications which can be treated by the compound of the invention. It will be understood by the person skilled in the art that also metastases and metastases of the above indications in particular can be treated and diagnosed by the compound of the invention and the methods of diagnosis and methods of treatment making use of the compound of the invention.

It is also within the present invention that the compound of the invention is used or is for use in a method for the treatment of a disease as disclosed herein. Such method, preferably, comprises the step of administering to a subject in need thereof a therapeutically effective amount of the compound of the invention. Such method includes, but is not limited to, curative or adjuvant cancer treatment. It is used as palliative treatment where cure is not possible and the aim is for local disease control or symptomatic relief or as therapeutic treatment where the therapy has survival benefit and it can be curative.

The method for the treatment of a disease as disclosed herein includes the treatment of the disease disclosed herein, including tumors and cancer as well as metabolic diseases, and may be used either as the primary therapy or as second, third, fourth or last line therapy. It is also within the present invention to combine the compound of the invention with further therapeutic approaches. It is well known to the person skilled in the art that the precise treatment intent including curative, adjuvant, neoadjuvant, therapeutic, or palliative treatment intent will depend on the tumor type, location, and stage, as well as the general health of the patient.

Without wishing to be bound by any theory, the therapeutic effect of the compounds of present invention is based on the delivery of a radionuclide to a diseased GIPR expressing cell or structure which is destroyed by the radiation emitted by the radionuclide. Preferably, GIPR is overexpressed by sich cell or structure.

Without wishing to be bound by any theory, the therapeutic use of the compounds of the invention arises from the binding of said compounds to GIPR expressing cells, cancer cells in particular, wherein said cells are killed by the radiation emitted by the radionuclide. It will also be appreciated by a person skilled in the art that GIPR is a pan-neuroendocrine-tumor target. Because of this, any respective cancer and tumor can be treated and diagnosed, respectively.

In an embodiment of the present invention, the disease is selected from the group comprising a cancer and tumor, respectively, expressing GIPR, preferably over-expressing GIPR. In a preferred embodiment, the cancer is a neuroendocrine cancer or neuroendocrine tumor, preferably each and any expressing or overexpressing GIPR.

In a further embodiment, the diseases is selected from the group comprising medullary thyroid cancer, gastrointestinal neuroendocrine neoplasms, ileal neuroendocrine neoplasms, pancreatic neuroendocrine neoplasms, nonfunctioning neuroendocrine neoplasms, Insulinomas, Gastrinomas, Glucagonomas, VIPomas, Somatostatinoma, ACTHoma, lung neuroendocrine neoplasms, typical carcinoid tumors, atypical carcinoid tumors, small cell carcinoma of the lung, large cell carcinoma of the lung, thymic neuroendocrine tumors, Merkel cell carcinoma, Pheochromocytoma of the adrenal gland, adrenal cancer, parathyroid cancer, paraganglioma, pituitary gland tumors, neuroendocrine tumors of the ovaries and neuroendocrine tumors of the testicles.

In a further embodiment, the disease is a metabolic disease. Preferably, the metabolic disease is one where GIPR is expressed, more preferably where GIPR is overexpressed in pancreatic beta cells. In a still further embodiment, the disease is selected from the group comprising metabolic diseases and disorders, preferably type 2 diabetes, type 1 diabetes, metabolic syndrome, insulin resistance, dyslipidemia, impaired fasting glucose, and impaired glucose tolerance.

In an embodiment, the compounds of the present invention are used to detect cells and tissues overexpressing the GIPR, whereby such detection is achieved by conjugating a detectable label to the compounds of the invention, preferably a detectable radionuclide. In a preferred embodiment, the cells and tissues detected are diseased cells and tissues and/or are either a or the cause for the disease and/or the symptoms of the disease, or are part of the pathology underlying the disease. In a further preferred embodiment, the diseased cells and tissues are causing and/or are part of an oncology indication (e.g. neoplasms, tumors, and cancers).

In another embodiment, the compounds of the present invention are used to treat cells and tissues overexpressing the GIPR. In a preferred embodiment, the cells and tissues treated are diseased cells and tissues and/or are either a or the cause for the disease and/or the symptoms of the disease, or are part of the pathology underlying the disease. In a further preferred embodiment, the diseased cells and tissues are causing and/or are part of an oncology indication (e.g. neoplasms, tumors, and cancers) and the therapeutic activity is achieved by conjugating therapeutically active effector to the compounds of the present invention, preferably a therapeutically active radionuclide.

An effective amount is a dosage of the compound sufficient to provide a therapeutically or medically desirable result or effect in the subject to which the compound is administered. The effective amount will vary with the particular condition being treated, the age and physical condition of the subject being treated, the severity of the condition, the duration of the treatment, the nature of the concurrent or combination therapy (if any), the specific route of administration and like factors within the knowledge and expertise of the health practitioner. For example, in connection with methods directed towards treating subjects having a condition characterized by abnormal cell proliferation, an effective amount to inhibit proliferation would be an amount sufficient to reduce or halt altogether the abnormal cell proliferation so as to slow or halt the development of or the progression of a cell mass such as, for example, a tumor. As used in the embodiments, "inhibit" embraces all of the foregoing.

In other embodiments, a therapeutically effective amount will be an amount necessary to extend the dormancy of micrometastases or to stabilize any residual primary tumor cells following surgical or drug therapy.

In a preferred embodiment, the compound of the present invention is for use in the treatment and/or prevention of a disease, whereby such treatment is targeted radionuclide therapy. Targeted radionuclide therapy is a form of radiation therapy (also called radiotherapy) using molecules labeled with a radionuclide to deliver a toxic level of radiation to sites of disease. Targeted radionuclide therapy may be applied systemically or locally. In contrast, in external beam radiation therapy a source outside of the body is producing a high-energy beam, which is then focused at sites of disease, passing through the skin into the body. It is as well distinguished from internal radiation therapy (brachytherapy), where a radioactive implant is placed at or near the site of disease.

Preferably, radionuclide therapy makes use of or is based on different forms of radiation emitted by a radionuclide. Such radiation can, for example, be any one of alpha (α), beta (β) or gamma (γ) radiation caused by the emission of photons, emission of electrons including but not limited to β⁻-particles and Auger-electrons, emission of protons, emission of neutrons, emission of positrons or emission of a-particles. Depending on the kind of particle or radiation emitted by said radionuclide, radionuclide therapy can, for example, be distinguished as β-particle radionuclide therapy, α-particle radionuclide therapy or Auger electron radionuclide therapy. All of these forms of radionuclide therapy are encompassed by the present invention, and all of these forms of radionuclide therapy can be realized by the compound of the invention, preferably under the proviso that the radionuclide attached to the compound of the invention, more preferably as an effector, is providing for this kind of radiation.

Radionuclide therapy preferably works by damaging the DNA of cells. The damage is caused by a β-particle, α-particle, or Auger electron directly or indirectly ionizing the atoms which make up the DNA chain. Indirect ionization happens as a result of the ionization of water, forming free radicals, notably hydroxyl radicals, which then damage the DNA.

In the most common forms of radionuclide therapy, most of the radiation effect is through free radicals. Because cells have mechanisms for repairing DNA damage, breaking the DNA on both strands proves to be the most significant technique in modifying cell characteristics. Because cancer cells generally are undifferentiated and stem cell-like, they reproduce more, and have a diminished ability to repair sub-lethal damage compared to most healthy differentiated cells. The DNA damage is inherited through cell division, accumulating damage to the cancer cells, causing them to die or reproduce more slowly.

Oxygen is a potent radiosensitizer, increasing the effectiveness of a given dose of radiation by forming DNA-damaging free radicals. Therefore, use of high-pressure oxygen tanks, blood substitutes that carry increased oxygen, hypoxic cell radiosensitizers such as misonidazole and metronidazole, and hypoxic cytotoxins, such as tirapazamine may be applied.

The total radioactive dose may be fractionated, i.e. spread out over time in one or more treatments for several important reasons. Fractionation allows normal cells time to recover, while tumor cells are generally less efficient in repair between fractions. Fractionation also allows tumor cells that were in a relatively radio-resistant phase of the cell cycle during one treatment to cycle into a sensitive phase of the cycle before the next fraction is given.

It is generally known that different cancers respond differently to radiation therapy. The response of a cancer to radiation is described by its radiosensitivity. Highly radiosensitive cancer cells are rapidly killed by modest doses of radiation. These include leukemias, most lymphomas, and germ cell tumors.

Radionuclide therapy is in itself painless. Many low-dose palliative treatments cause minimal or no side effects. Treatment to higher doses may cause varying side effects during treatment (acute side effects), in the months or years following treatment (long-term side effects), or after re-treatment (cumulative side effects). The nature, severity, and longevity of side effects depends on the organs that receive the radiation, the treatment itself (type of radionuclide, dose, fractionation, concurrent chemotherapy), and the patient.

It is within the present inventions that the method for the treatment of a disease of the invention may realize each and any of the above strategies which are as such known in the art, and which insofar constitute further embodiments of the invention.

It is also within the present invention that the compound of the invention is used in a method for the diagnosis of a disease as disclosed herein. Such method, preferably, comprises the step of administering to a subject in need thereof a diagnostically effective amount of the compound of the invention.

In accordance with the present invention, an imaging method is selected from the group consisting of scintigraphy, Single Photon Emission Computed Tomography (SPECT) and Positron Emission Tomography (PET).

Scintigraphy is a form of diagnostic test or method used in nuclear medicine, wherein radiopharmaceuticals are internalized by cells, tissues and/or organs, preferably internalized *in vivo*, and radiation emitted by said internalized radiopharmaceuticals is captured by external detectors (gamma cameras) to form and display two-dimensional images. In contrast thereto, SPECT and PET forms and displays three-dimensional images. Because of this, SPECT and PET are classified as separate techniques to scintigraphy, although they also use gamma cameras to detect internal radiation. Scintigraphy is unlike a diagnostic X-ray where external radiation is passed through the body to form an image.

Single Photon Emission Tomography (SPECT) scans are a type of nuclear imaging technique using gamma rays. They are very similar to conventional nuclear medicine planar imaging using a gamma camera. Before the SPECT scan, the patient is injected with a radiolabeled compound emitting gamma rays that can be detected by the scanner. A computer collects the information from the gamma camera and translates this into two-dimensional cross-sections. These cross-sections can be added back together to form a three-dimensional image of an organ or a tissue. SPECT involves detection of gamma rays emitted singly, and sequentially, by the radionuclide provided by the radiolabeled compound. To acquire SPECT images, the gamma camera is rotated around the patient. Projections are acquired at defined points during the rotation, typically every 3 - 6 degrees. In most cases, a full 360 degree rotation is used to obtain an optimal reconstruction. The time taken to obtain each projection is also variable, but 15 - 20 seconds is typical. This gives a total scan time of 15 - 20 minutes. Multi-headed gamma cameras are faster. Since SPECT acquisition is very similar to planar gamma camera imaging, the same radiopharmaceuticals may be used.

Positron Emitting Tomography (PET) is a non-invasive, diagnostic imaging technique for measuring the biochemical, physiological and pathophysiological processes within the human body. PET is unique since it is able to produce images of the body's basic biochemistry or functions. Traditional diagnostic techniques, such as X-rays, CT scans, or MRI, produce images of the body's anatomy or structure. The premise with these techniques is that any changes in structure or anatomy associated with a disease can be seen. Biochemical and physiological processes are also altered by a disease, and may occur before any gross changes in anatomy. PET is an imaging technique that can visualize some of these early biochemical and physiological changes. PET scanners rely on radiation emitted from the patient to create the images. Each patient is given a minute amount of a radioactive compound that either closely resembles a natural substance used by the body or binds specifically to a receptor or molecular structure. As the radioisotope undergoes positron emission decay (also known as positive beta decay), it emits a positron, the antiparticle counterpart of an electron. After traveling up to a few millimeters, the positron encounters an electron and annihilates, producing a pair of annihilation (gamma) photons moving in opposite directions. These are detected when they reach a scintillation material in the scanning device, creating a burst of light, which is detected by photomultiplier tubes or silicon avalanche photodiodes. The technique depends on simultaneous or coincident detection of the pair of photons. Photons that do not arrive in pairs, i.e., within a few nanoseconds, are ignored. All coincidences are forwarded to the image processing unit where the final image data is produced using image reconstruction procedures.

SPECT/CT and PET/CT is the combination of SPECT and PET with computed tomography (CT). The key benefits of combining these modalities are improving the reader's confidence and accuracy. With traditional PET and SPECT, the limited number of photons emitted from the area of abnormality produces a very low-level background that makes it difficult to anatomically localize the area. Adding CT helps determine the location of the abnormal area from an anatomic perspective and categorize the likelihood that this represents a disease.

It is within the present inventions that the method for the diagnosis of a disease of the invention may realize each and any of the above strategies which are as such known in the art, and which insofar constitute further embodiments of the invention.

In an embodiment and as preferably used herein, a linkage is an attachment of two atoms of two independent moieties. A preferred linkage is a chemical bond or a plurality of chemical bonds. More preferably a chemical bond is a covalent bond or a plurality of chemical bonds. Most preferably the linkage is a covalent bond or a coordinate bond. As preferably used herein, an embodiment of a coordinate bond is a bond or group of bonds as realized when a metal is bound by a chelator. Depending on the type of atoms linked and their atomic environment different types of linkages are created. These types of linkage are defined by the type of atom arrangements created by the linkage.

For instance, the linking of a moiety comprising an amine with a moiety comprising a carboxylic acid leads to a linkage named amide (which is also referred to as amide linkage, -CO-N-, -N-CO-). It will be acknowledged by a person skilled in the art that this and the following examples of creating linkages are only prototypical examples and are by no means limiting the scope of the instant application. It will be acknowledged by a person in the art that the linking of a moiety comprising an isothiocyanate with a moiety comprising an amine leads to thiourea (which is also referred to as a thiourea linkage, -N-CS-N-), and linking of a moiety comprising a C atom with a moiety comprising a thiol-group (-C-SH) leads to thioether (which is also referred to as a thioether linkage, -C-S-C-). A non-limiting list of linkages as preferably used in connection with the chelator and linker of the invention and their characteristic type of atom arrangement is presented Table 2.

**Table 2:**

| **Linkage** | **Characteristic atom arrangement** |
|---|---|
| Amide | 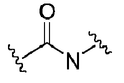 |
| Sulfonamide | 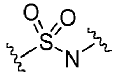 |
| Urea | 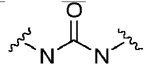 |
| Thioether | 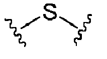 |
| Disulfide | 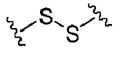 |
| Ether | 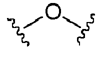 |
| Ester | 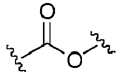 |
| Carbamate | 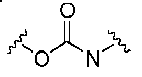 |
| Thiourea | 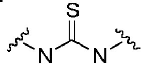 |
| Triazole | 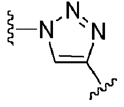 |

Examples of reactive groups which, in some embodiments of the invention, are used in the formation of linkages between the chelator and linker, directly between the chelator and the compound of the invention, the bio-distribution modifier and linker or the bio-distribution modifier and the compound of the invention are summarized in Table 3. It will, however, be understood by a person skilled in the art that neither the linkages which may be realized in embodiments for the formation of the conjugates of the invention are limited to the ones of Table 3 nor the reactive groups forming such linkages.

**Table 3:**

| **first reactive group** | **second reactive group** | **(type of) linkage** |
|---|---|---|
| amino | carboxylic acid | amide |
| amino | activated carboxylic acid | amide |
| amino | sulfonyl halide | sulfonamide |
| amino | isocyanate | urea |
| amino | p-nitrophenylcarbamate | urea |
| sulfhydryl | Michael acceptor (e.g. Maleimide) | thioether |
| bromo | sulfhydryl | thioether |
| isothiocyanate | amino | thiourea |
| hydroxyl | carboxylic acid | ester |
| azide | alkyne | triazole |
| sulfhydryl | sulfhydryl | disulfide |
| sulfhydryl | 2-Pyridine-di sulfide | disulfide |
| carbonate ester | amino | carbamate |
| chloroformate | amino | carbamate |
| bromo | hydroxy | ether |

The following are reactive groups and functionalities which are utilized or amenable of forming linkages between moieties or structures as used in embodiments of the conjugate of the invention:
Primary or secondary amino, carboxylic acid, activated carboxylic acid, chloro, bromo, iodo, sulfhydryl, hydroxyl, sulfonic acid, activated sulfonic acid, sulfonic acid esters like mesylate or tosylate, Michael acceptors, strained alkenes like *trans* cyclooctene, isocyanate, isothiocyanate, azide, alkyne and tetrazine.

As preferably used herein, the term "activated carboxylic acid" refers to a carboxylic acid group with the general formula -CO-X, wherein X is a leaving group. For example, activated forms of a carboxylic acid group may include, but are not limited to, acyl chlorides, symmetrical or unsymmetrical anhydrides, and esters. In some embodiments, the activated carboxylic acid group is an ester with pentafluorophenol, nitrophenol, benzotriazole, azabenzotriazole, thiophenol or N-hydroxysuccinimide (NHS) as leaving group.

As preferably used herein, the term "activated sulfonic acid" refers to a sulfonic acid group with the general formula -SO₂-X, wherein X is a leaving group. For example, activated forms of a sulfonic acid may include, but are not limited to, sulfonyl chlorides or sulfonic acid anhydrides. In some embodiments, the activated sulfonic acid group is sulfonylchloride with chloride as leaving group.

In an embodiment and as preferably used herein the term "mediating a linkage" means that a linkage or a type of linkage is established, preferably a linkage between two moieties. In a preferred embodiment the linkage and the type of linkage is as defined herein.

To the extent it is referred in the instant application to a range indicated by a lower integer and a higher integer such as, for example, 1-4, such range is a representation of the lower integer, the higher integer and any integer between the lower integer and the higher integer. Insofar, the range is actually an individualized disclosure of said integer. In said example, the range of 1-4 thus means 1, 2, 3 and 4.

In an embodiment and as preferably used herein, the expression alkyl refers each and individually to a saturated, straight-chain or branched hydrocarbon group and is usually accompanied by a qualifier which specifies the number of carbon atoms it may contain. For example the expression (C1-C6)alkyl means each and individually any of methyl, ethyl, n-propyl, isopropyl, n-butyl, isobutyl, *sec-*butyl, *tert*-butyl, n-pentyl, 1-methyl-butyl, 1-ethyl-propyl, 3-methyl-butyl, 1,2-dimethyl-propyl, 2-methyl-butyl, 1,1-dimethyl-propyl, 2,2-dimethylpropyl, n-hexyl, 1,1-dimethyl-butyl and any other isoform of alkyl groups containing six saturated carbon atoms.

In an embodiment and as preferably used herein, (C₁-C₂)alkyl means each and individually any of methyl and ethyl.

In an embodiment and as preferably used herein, (C₁-C₃)alkyl means each and individually any of methyl, ethyl, n-propyl and isopropyl.

In an embodiment and as preferably used herein, (C₁-C₄)alkyl means each and individually any of methyl, ethyl, n-propyl, isopropyl, n-butyl, isobutyl, *sec*-butyl and *tert*-butyl.

In an embodiment and as preferably used herein, (C₁-C₆)alkyl means each and individually any of methyl, ethyl, n-propyl, isopropyl, n-butyl, isobutyl, sec-butyl, tert-butyl, n-pentyl, 2-pentyl, 2-methyl-butyl, 3-methyl-butyl, 3-pentyl, 3-methyl-but-2-yl, 2-methyl-but-2-yl, 2,2-dimethylpropyl, n-hexyl, 2-hexyl, 2-methyl-pentyl, 3-methyl-pentyl, 4-methyl-pentyl, 3-hexyl, 2-ethyl-butyl, 2-methyl-pent-2-yl, 2,2-dimethyl-butyl, 3,3-dimethyl-butyl, 3-methyl-pent-2-yl, 4-methyl-pent-2-yl, 2,3-dimethyl-butyl, 3-methyl-pent-3-yl, 2-methyl-pent-3-yl, 2,3-dimethyl-but-2-yl and 3,3-dimethyl-but-2-yl.

In an embodiment and as preferably used herein, (C₁-C₈)alkyl refers to a saturated or unsaturated, straight-chain or branched hydrocarbon group having from 1 to 8 carbon atoms. Representative (C₁-C₈)alkyl groups include, but are not limited to, any of methyl, ethyl, n-propyl, isopropyl, n-butyl, isobutyl, sec-butyl, tert-butyl, n-pentyl, 2-pentyl, 2-methyl-butyl, 3-methyl-butyl, 3-pentyl, 3-methyl-but-2-yl, 2-methyl-but-2-yl, 2,2-dimethylpropyl, n-hexyl, 2-hexyl, 2-methyl-pentyl, 3-methyl-pentyl, 4-methyl-pentyl, 3-hexyl, 2-ethyl-butyl, 2-methyl-pent-2-yl, 2,2-dimethyl-butyl, 3,3-dimethyl-butyl, 3-methyl-pent-2-yl, 4-methyl-pent-2-yl, 2,3-dimethyl-butyl, 3-methyl-pent-3-yl, 2-methyl-pent-3-yl, 2,3-dimethyl-but-2-yl, 3,3-dimethyl-but-2-yl, n-heptyl, 2-heptyl, 2-methyl-hexyl, 3-methyl-hexyl, 4-methyl-hexyl, 5-methyl-hexyl, 3-heptyl, 2-ethyl-pentyl, 3-ethyl-pentyl, 4-heptyl, 2-methyl-hex-2-yl, 2,2-dimetyhl-pentyl, 3,3-dimetyhl-pentyl, 4,4-dimetyhl-pentyl, 3-methyl-hex-2-yl, 4-methyl-hex-2-yl, 5-methyl-hex-2-yl, 2,3-dimethyl-pentyl, 2,4-dimethyl-pentyl, 3,4-dimethyl-pentyl, 3-methyl-hex-3-yl, 2-ethyl-2-methyl-butyl, 4-methyl-hex-3-yl, 5-methyl-hex-3-yl, 2-ethyl-3-methyl-butyl, 2,3-dimethyl-pent-2-yl, 2,4-dimethyl-pent-2-yl, 3,3-dimethyl-pent-2-yl, 4,4-dimethyl-pent-2-yl, 2,2,3-trimethyl-butyl, 2,3,3-trimethyl-butyl, 2,3,3-trimethyl-but-2-yl, n-octyl, 2-octyl, 2-methyl-heptyl, 3-methyl-heptyl, 4-methyl-heptyl, 5-methyl-heptyl, 6-methyl-heptyl, 3-octyl, 2-ethyl-hexyl, 3-ethyl-hexyl, 4-ethyl-hexyl, 4-octyl, 2-propyl-pentyl, 2-methyl-hept-2-yl, 2,2-dimethyl-hexyl, 3,3-dimethyl-hexyl, 4,4-dimethyl-hexyl, 5,5-dimethyl-hexyl, 3-methyl-hept-2-yl, 4-methyl-hept-2-yl, 5-methyl-hept-2-yl, 6-methyl-hept-2-yl, 2,3-dimethyl-hex-1-yl, 2,4-dimethyl-hex-1-yl, 2,5-dimethyl-hex-1-yl, 3,4-dimethyl-hex-1-yl, 3,5-dimethyl-hex-1-yl, 3,5-dimethyl-hex-1-yl, 3-methyl-hept-3-yl, 2-ethyl-2-methyl-1-yl, 3-ethyl-3-methyl-1-yl, 4-methyl-hept-3-yl, 5-methyl-hept-3-yl, 6-methyl-hept-3-yl, 2-ethyl-3-methyl-pentyl, 2-ethyl-4-methyl-pentyl, 3-ethyl-4-methyl-pentyl, 2,3-dimethyl-hex-2-yl, 2,4-dimethyl-hex-2-yl, 2,5-dimethyl-hex-2-yl, 3,3-dimethyl-hex-2-yl, 3,4-dimethyl-hex-2-yl, 3,5-dimethyl-hex-2-yl, 4,4-dimethyl-hex-2-yl, 4,5-dimethyl-hex-2-yl, 5,5-dimethyl-hex-2-yl, 2,2,3-trimethyl-pentyl, 2,2,4-trimethyl-pentyl, 2,3,3-trimethyl-pentyl, 2,3,4-trimethyl-pentyl, 2,4,4-trimethyl-pentyl, 3,3,4-trimethyl-pentyl, 3,4,4-trimethyl-pentyl, 2,3,3-trimethyl-pent-2-yl, 2,3,4-trimethyl-pent-2-yl, 2,4,4-trimethyl-pent-2-yl, 3,4,4-trimethyl-pent-2-yl, 2,2,3,3-tetramethylbutyl, 3,4-dimethyl-hex-3-yl, 3,5-dimethyl-hex-3-yl, 4,4-dimethyl-hex-3-yl, 4,5-dimethyl-hex-3-yl, 5,5-dimethyl-hex-3-yl, 3-ethyl-3-methyl-pent-2-yl, 3-ethyl-4-methyl-pent-2-yl, 3-ethyl-hex-3-yl, 2,2-diethyl-butyl, 3-ethyl-3-methyl-pentyl, 4-ethyl-hex-3-yl, 5-methyl-hept-3-yl, 2-ethyl-3-methyl-pentyl, 4-methyl-hept-4-yl, 3-methyl-hept-4-yl, 2-methyl-hept-4-yl, 3-ethyl-hex-2-yl, 2-ethyl-2-methyl-pentyl, 2-isopropyl-pentyl, 2,2-dimethyl-hex-3-yl, 2,2,4-trimethyl-pent-3-yl and 2-ethyl-3-methyl-pentyl. A (C₁-C₈)alkyl group can be unsubstituted or substituted with one or more groups, including, but not limited to, (C₁-C₈)alkyl, -O-[(C₁-C₈)alkyl], -aryl, -CO-R', -O-CO-R', -CO-OR', -CO-NH₂, -CO-NHR', - CO-NR'₂, -NH-CO-R', -SO₂-R', -SO-R', -OH, -halogen, -N₃, -NH₂, -NHR', -NR'₂ and -CN; where each R' is independently selected from -(C₁-C₈)alkyl and aryl.

In an embodiment and preferably used herein, the expression alkylidene refers to a saturated straight chain or branched hydrocarbon group wherein two points of substitution are specified.

Simple alkyl chains wherein the two points of substitutions are in a maximal distance to each other like methane-1,1-diyl, ethane-1,2-diyl, propane-1,3-diyl, butane-1,4-diyl and pentane-1,5-diyl are also referred to as methylene (which is also referred to as methane-1,1-diyl), ethylene (which is also referred to as ethane-1,2-diyl), propylene (which is also referred to as propane-1,3-diyl), butylene (which is also referred to as butane-1,4-diyl) and pentylene (which is also referred to as pentane-1,5-diyl).

In an embodiment and as preferably used herein, (C₁-C₁₀)alkylidene means each and individually any of methylene, ethane-1,2-diyl, propane-1,3-diyl, propane-1,2-diyl, butane-1,4-diyl, butane-1,3-diyl, butane-1,2-diyl, 2-methyl-propane-1,2-diyl, 2-methyl-propane-1,3-diyl, pentane-1,5-diyl, pentane-1,4-diyl, pentane-1,3-diyl, pentane-1,2-diyl, pentane-2,3-diyl, pentane-2,4-diyl, any other isomer with 5 carbon atoms, hexane-1,6-diyl, any other isomer with 6 carbon atoms, heptane-1,7-diyl, any other isomer with 7 carbon atoms, octane-1,8-diyl, any other isomer with 8 carbon atoms, nonane-1,9-diyl, any other isomer with 9 carbon atoms, decane-1,10-diyl and any other isomer with 10 carbon atoms, preferably (C₁-C₁₀) alkylidene means each and individually any of methylene, ethane-1,2-diyl, propane-1,3-diyl, butane-1,4-diyl, pentane-1,5-diyl, hexane-1,6-diyl, heptane-1,7-diyl, octane-1,8-diyl, nonane-1,9-diyl and decane-1,10-diyl. A (C₁-C₁₀₎alkylidene group can be unsubstituted or substituted with one or more groups, including, but not limited to, (C₁-C₈)alkyl, -O-[(C₁-C₈)alkyl], -aryl, -CO-R', -O-CO-R', -CO-OR', -CO-NH₂, -CO-NHR', -CO-NR'₂, -NH-CO-R', -SO₂-R', -SO-R', -OH, - halogen, -N₃, -NH₂, -NHR', -NR'₂ and -CN; where each R' is independently selected from - (C₁-C₈)alkyl and aryl.

In an embodiment and as preferably used herein, "aliphatic bis-nucleophile" refers to a (C₂-C₃)alkyl, whereby each terminal carbon atom of the alkyl chain is individually substituted with a sulfur, oxygen or nitrogen atom. Examples of aliphatic bis-nucleophiles include, but are not limited to, 2-aminoethanethiol, 2-aminoethanol, ethylenediamine, 3-aminopropane-1-thiol, 3-amino-1-propanol and 1,3-diaminopropane.

In an embodiment and as preferably used herein, "aryl" refers to a carbocyclic aromatic group. Examples of aryl groups include, but are not limited to, phenyl, naphthyl and anthracenyl.

In an embodiment and as preferably used herein, (C₅-C₆)aryl refers to a 5 or 6 carbon atom comprising carbocyclic aromatic group. A carbocyclic aromatic group can be unsubstituted or substituted with one or more groups including, but not limited to, -(C₁-C₈)alkyl, -O-[(C₁-C₈)alkyl], -aryl, -CO-R', -O-CO-R', -CO-OR', -CO-NH₂, -CO-NHR', -CO-NR'₂, -NH-CO-R', -SO₂-R', -SO-R', -OH, -halogen, -N₃, -NH₂, -NHR', -NR'₂ and -CN; where each R' is independently selected from -(C₁-C₈)alkyl and aryl.

In an embodiment and as preferably used herein, "aromatic resiude" refers to a carbocyclic aromatic group. Examples of aromatic residues include, but are not limited to, phenyl, naphthyl and anthracenyl.

In an embodiment and as preferably used herein, (C₅-C₆)aryl refers to a 5 or 6 carbon atom comprising carbocyclic aromatic group. A carbocyclic aromatic group can be unsubstituted or substituted with one or more groups including, but not limited to, -(C₁-C₈)alkyl, -O-[(C₁-C₈)alkyl], -aryl, -CO-R', -O-CO-R', -CO-OR', -CO-NH₂, -CO-NHR', -CO-NR'₂, -NH-CO-R', -SO₂-R', -SO-R', -OH, -halogen, -N₃, -NH₂, -NHR', -NR'₂ and -CN; where each R' is independently selected from -(C₁-C₈)alkyl and aryl.

In an embodiment and as preferably used herein, "aromatic side chain" refers to a carbocyclic aromatic group. Examples of aromatic side chains include, but are not limited to, phenyl.

In an embodiment and as preferably used herein, (C₅-C₆)aromatic side chain refers to a 5 or 6 carbon atom comprising carbocyclic aromatic group. An aromatic side chain can be unsubstituted or substituted with one or more groups including, but not limited to, -(C₁-C₈)alkyl, -O-[(C₁-C₈)alkyl], -aryl, -CO-R', -O-CO-R', -CO-OR', -CO-NH₂, -CO-NHR', -CO-NR'₂, - NH-CO-R', -SO₂-R', -SO-R', -OH, -halogen, -N₃, -NH₂, -NHR', -NR'₂ and -CN; where each R' is independently selected from -(C₁-C₈)alkyl and aryl.

In an embodiment and as preferably used herein, "aromatic ring" refers to a carbocyclic aromatic group. Examples of aromatic rings include, but are not limited to, phenyl.

In an embodiment and as preferably used herein, (C₅-C₆)aromatic ring refers to a 5 or 6 carbon atom comprising carbocyclic aromatic group. An aromatic ring can be unsubstituted or substituted with one or more groups including, but not limited to, -(C₁-C₈)alkyl, -O-[(C₁-C₈)alkyl], -aryl, -CO-R', -O-CO-R', -CO-OR', -CO-NH₂, -CO-NHR', -CO-NR'₂, -NH-CO-R', -SO₂-R', -SO-R', -OH, -halogen, -N₃, -NH₂, -NHR', -NR'₂ and -CN; where each R' is independently selected from -(C₁-C₈)alkyl and aryl.

In an embodiment and as preferably used herein, "bicyclic aromatic side chain" refers to a carbocyclic aromatic group. Examples of bicyclic aromatic side chain include, but are not limited to naphthyl.

In an embodiment and as preferably used herein, (C₉-C₁₀)bicyclic aromatic side chain refers to a 9 or 10 carbon atom comprising a carbocyclic aromatic group. A bicyclic aromatic side chain can be unsubstituted or substituted with one or more groups including, but not limited to, -(C₁-C₈)alkyl, -O-[(C₁-C₈)alkyl], -aryl, -CO-R', -O-CO-R', -CO-OR', -CO-NH₂, -CO-NHR', -CO-NR'₂, -NH-CO-R', -SO₂-R', -SO-R', -OH, -halogen, -N₃, -NH₂, -NHR', -NR'₂ and -CN; where each R' is independently selected from -(C₁-C₈)alkyl and aryl.

In an embodiment and as preferably used herein, "bicyclic aromatic ring" refers to a carbocyclic aromatic group. Examples of aromatic rings include, but are not limited to, naphthyl.

In an embodiment and as preferably used herein, (C₉-C₁₀)bicyclic aromatic ring refers to a 9 or 10 carbon atom comprising carbocyclic aromatic group. A bicyclic aromatic ring can be unsubstituted or substituted with one or more groups including, but not limited to, -(C₁-C₈)alkyl, -O-[(C₁-C₈)alkyl], -aryl, -CO-R', -O-CO-R', -CO-OR', -CO-NH₂, -CO-NHR', -CO-NR'₂, - NH-CO-R', -SO₂-R', -SO-R', -OH, -halogen, -N₃, -NH₂, -NHR', -NR'₂ and -CN; where each R' is independently selected from -(C₁-C₈)alkyl and aryl.

In an embodiment and as preferably used herein, "heteroaryl" refers to a heterocyclic aromatic group. Examples of heteroaryl groups include, but are not limited to, furane, thiophene, pyridine, pyrimidine, benzothiophene, benzofurane and quinoline.

In an embodiment and as preferably used herein, (C₅-C₆)heteroaryl refers to a heterocyclic aromatic group consisting of 5 or 6 ring atoms wherein at least one atom is different from carbon, preferably nitrogen, sulfur or oxygen. A heterocyclic aromatic group can be unsubstituted or substituted with one or more groups including, but not limited to, -(C₁-C₈)alkyl, -O-[(C₁-C₈)alkyl], -aryl, -CO-R', -O-CO-R', -CO-OR', -CO-NH₂, -CO-NHR', -CO-NR'₂, - NH-CO-R', -SO₂-R', -SO-R', -OH, -halogen, -N₃, -NH₂, -NHR', -NR'₂ and -CN; where each R' is independently selected from -(C₁-C₈)alkyl and aryl.

In an embodiment and as preferably used herein, "heteroaromatic side chain" refers to a heterocyclic aromatic group. Examples of heteroaromatic side chains include, but are not limited to, furane, thiophene, pyridine and pyrimidine.

In an embodiment and as preferably used herein, (C₅-C₆)heteroaromatic side chain refers to a heterocyclic aromatic group consisting of 5 or 6 ring atoms wherein at least one atom is different from carbon, preferably nitrogen, sulfur or oxygen. A heteroaromatic side chain can be unsubstituted or substituted with one or more groups including, but not limited to, -(C₁-C₈)alkyl, -O-[(C₁-C₈)alkyl], -aryl, -CO-R', -O-CO-R', -CO-OR', -CO-NH₂, -CO-NHR', -CO-NR'₂, - NH-CO-R', -SO₂-R', -SO-R', -OH, -halogen, -N₃, -NH₂, -NHR', -NR'₂ and -CN; where each R' is independently selected from -(C₁-C₈)alkyl and aryl.

In an embodiment and as preferably used herein, "bicyclic heteroaromatic side chain" refers to a heterocyclic aromatic group. Examples of heteroaromatic side chains include, but are not limited to, benzothiophene, benzofurane and quinoline.

In an embodiment and as preferably used herein, (C₉-C₁₀)bicyclic heteroaromatic side chain refers to a heterocyclic aromatic group consisting of 9 or 10 ring atoms wherein at least one atom is different from carbon, preferably nitrogen, sulfur or oxygen. A bicyclic heteroaromatic side chain can be unsubstituted or substituted with one or more groups including, but not limited to, -(C₁-C₈)alkyl, -O-[(C₁-C₈)alkyl], -aryl, -CO-R', -O-CO-R', -CO-OR', -CO-NH₂, -CO-NHR', -CO-NR'₂, -NH-CO-R', -SO₂-R', -SO-R', -OH, -halogen, -N₃, -NH₂, -NHR', -NR'₂ and -CN; where each R' is independently selected from -(C₁-C₈)alkyl and aryl.

In an embodiment and as preferably used herein, "bicyclic heteroaromatic ring" refers to a heterocyclic aromatic group. Examples of bicyclic heteroaromatic rings include, but are not limited to, benzothiophene, benzofurane and quinoline.

In an embodiment and as preferably used herein, (C₉-C₁₀)bicyclic heteroaromatic ring refers to a heterocyclic aromatic group consisting of 9 or 10 ring atoms wherein at least one atom is different from carbon, preferably nitrogen, sulfur or oxygen. A bicyclic heteroaromatic ring can be unsubstituted or substituted with one or more groups including, but not limited to, -(Ci-C₈)alkyl, -O-[(C₁-C₈)alkyl], -aryl, -CO-R', -O-CO-R', -CO-OR', -CO-NH₂, -CO-NHR', -CO-NR'₂, -NH-CO-R', -SO₂-R', -SO-R', -OH, -halogen, -N₃, -NH₂, -NHR', -NR'₂ and -CN; where each R' is independently selected from -(C₁-C₈)alkyl and aryl.

In an embodiment and as preferably used herein, (C₃-C₈)heterocyclo refers to a (C₃-Cs)heterocycle group defined above wherein one of the carbocycles group hydrogen atoms is replaced with a bond. A (C₃-C₈)heterocyclo can be unsubstituted or substituted with up to six groups including, (C₁-C₈)alkyl, -O-[(C₁-C₈)alkyl], -aryl, -CO-R', -O-CO-R', -CO-OR', -CO-NH₂, -CO-NHR', -CO-NR'₂, -NH-CO-R', -SO₂-R', -SO-R', -OH, -halogen, -N₃, -NH₂, -NHR', -NR'₂ and -CN; where each R' is independently selected from -(C₁-C₈)alkyl and aryl.

In an embodiment and as preferably used herein, "bicyclic non-aromatic residue" refers to a bicyclic heterocyclic group. Examples of bicyclic non-aromatic residue include, but are not limited to, octahydroquinoline and octahydroisoquinoline.

In an embodiment and as preferably used herein, (C₉-C₁₀)bicyclic non-aromatic residue refers to a heterocyclic group consisting of 9 or 10 ring atoms wherein at least one atom is different from carbon, preferably nitrogen, sulfur or oxygen. A bicyclic non-aromatic residue can be unsubstituted or substituted with one or more groups including, but not limited to, -(C₁-C₈)alkyl, -O-[(C₁-C₈)alkyl], -aryl, -CO-R', -O-CO-R', -CO-OR', -CO-NH₂, -CO-NHR', -CO-NR'₂, - NH-CO-R', -SO₂-R', -SO-R', -OH, -halogen, -N₃, -NH₂, -NHR', -NR'₂ and -CN; where each R' is independently selected from -(C₁-C₈)alkyl and aryl.

In an embodiment and as preferably used herein, arylene refers to an aryl group which has two covalent bonds and can be in the *ortho*, *meta*, or *para* configurations as shown in the following structures: 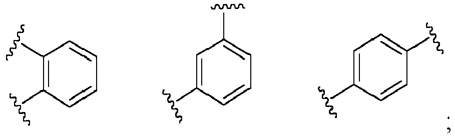 in which the phenyl group can be unsubstituted or substituted with four groups, including, but not limited to, (C₁-C₈)alkyl, -O-[(C₁-C₈)alkyl], -aryl, -CO-R', -O-CO-R', -CO-OR', -CO-NH₂, -CO-NHR', -CO-NR'₂, -NH-CO-R', -SO₂-R', -SO-R', -OH, -halogen, -N₃, -NH₂, -NHR', - NR'₂ and -CN; where each R' is independently selected from -(C₁-C₈)alkyl and aryl.

In an embodiment and as preferably used herein, the C-terminal group C-term is one or more amino acids AA. If C-term is two or more of such amino acids, the first of such two or amino acids is preferably the one at the N-terminus (assuming that the amino acids are linked to each other by a peptide bond) and the last of such two or amino acids is preferably the one at the C-terminus (assuming that the amino acids are linked to each other by a peptide bond).

In an embodiment and as preferably used herein, atoms with unspecified atomic mass numbers in any structural formula or in any passage of the instant specification including the claims are either of unspecified isotopic composition, naturally occurring mixtures of isotopes or individual isotopes. This applies in particular to carbon, oxygen, nitrogen, sulfur, phosphorus, halogens and metal atoms, including but not limited to C, O, N, S, F, P, Cl, Br, At, Sc, Cr, Mn, Co, Fe, Cu, Ga, Sr, Zr, Y, Mo, Tc, Ru, Rh, Pd, Pt, Ag, In, Sb, Sn, Te, I, Pr, Pm, Dy, Sm, Gd, Tb, Ho, Dy, Er, Yb, Tm, Lu, Sn, Re, Rd, Os, Ir, Au, Pb, Bi, Po, Fr, Ra, Ac, Th and Fm.

In an embodiment and as preferably used herein, a chelator is a compound which is capable of forming a chelate, whereby a chelate is a compound, preferably a cyclic compound where a metal or a moiety having an electron gap or a lone pair of electrons participates in the formation of the ring. More preferably, a chelator is a kind of compound where a single ligand occupies more than one coordination site at a central atom.

In an embodiment and as preferably used herein, a bio-distribution modifier is a compound the function of which can be the attenuation of unintended or undesired bio-distribution of the compound of the invention by altering physio-chemical properties of the compound of the invention. Alternatively or additionally, the function of the bio-distribution modifier can be the binding to components of the serum thus extending the serum half-life and/or the alteration of serum half-life by modulating renal clearance.

In an embodiment and as preferably used herein, "dicarboxylic acid reside" refers to a compound with two carboxylic acid groups, wherein the two carboxylic groups are attached to the terminal carbona atoms of a (C₁-C₆)alkyl. Examples of dicarboxylic acid resides residue include, but are not limited to, malonic acid, succinic acid, glutaric acid and pimelic acid.

Compounds of the invention typically contain amino acid sequences as provided herein. Conventional amino acids, also referred to as natural amino acids are identified according to their standard three-letter codes, as set forth in Table 4.

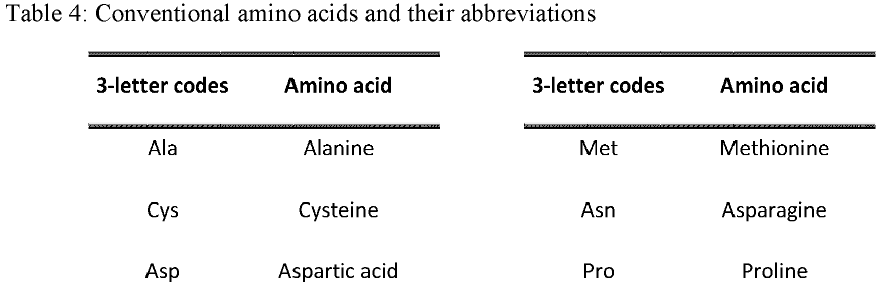

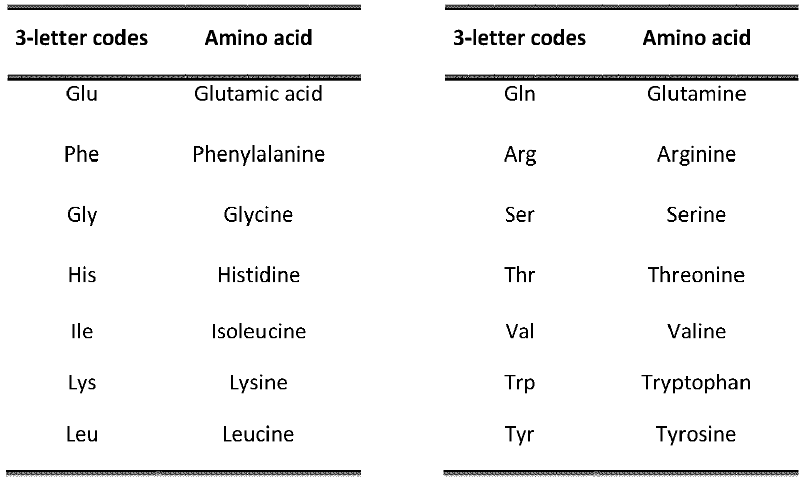

Non-conventional amino acids, also referred to as non-natural amino acids, are any kind of non-oligomeric compound which comprises an amino group and a carboxylic group and is not a conventional amino acid.

Examples of non-conventional amino acids and other building blocks as used for the construction compounds of the invention are identified according to their abbreviation or name found in Table 5. The structures of some building blocks are depicted with an exemplary reagent for introducing the building block into the peptide (e.g., as carboxylic acid like) or these building blocks are shown as residue which is completely attached to another structure like a peptide or amino acid. The structures of the amino acids are shown as residues of the amino acids how they are presented after implementation in the peptide sequence and not as explicit amino acids. Some larger chemical moieties consisting of more than one moiety are also shown for the reason of clarity.

**Table 5: Abbreviation, name and structure of non-natural amino-acid and other building blocks and chemical moieties**

| **Abbreviation** | **Name** | **Structure** |
|---|---|---|
| INi | 3 -(1 -naphthyl)-L-alanine | 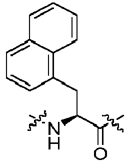 |
| 1Qi | 3-(4-quinoline)-L-alanine | 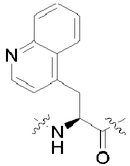 |
| 2Lut | 2,6-lutidylidene (derived from 2,6-lutidine) | 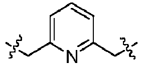 |
| 2MeBn | 2-Methylbenzylidene (derived from 1,2-Xylene) | 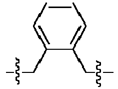 |
| 2Ni | 3 -(2-naphthyl)-L-alanine | 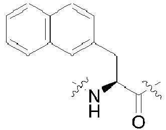 |
| 3CbBn | 3-Carboxybenzyl (derived from meta-methyl benzoic acid) | 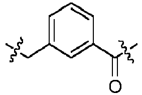 |
| 3Lut | 3,5-lutidylidene (derived from 3,5-lutidine) | 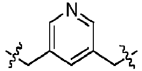 |
| 3MeBn | 3-Methylbenzylidene (derived from 1,3-Xylene) | 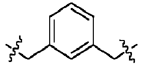 |
| 3MeBz | 3-methylbenzoyl (derived from meta-methyl benzoic acid) | 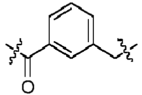 |
| 3Mh | 3-Methyl-L-histidine | 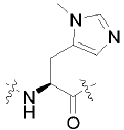 |
| 4Ap | 4-(*S*)-Amino-L-proline | 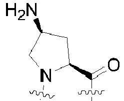 |
| 4ClPhp | 4-chlorophenyl propionic acid | 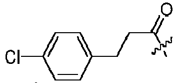 |
| 4Dfp | 4,4-Difluoro-L-proline | 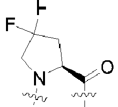 |
| 4FPhp | 4-fluorophenyl propionic acid | 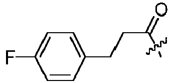 |
| 4MeBn | 4-Methylbenzylidene (derived from 1,4-Xylene) | 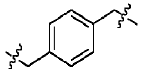 |
| 4OHPhp | 4-hydroxyphenyl propionic acid | 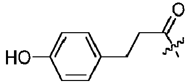 |
| 4Tfp | 4-(*R*)-Fluoro-L-proline | 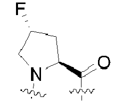 |
| 5Brw | 5-bromo-L-tryptophan | 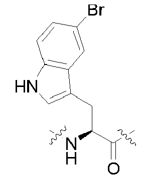 |
| 5Clw | 5-chloro-L-tryptophan | 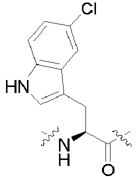 |
| 5Fw | 5 -fluoro-L-tryptophan | 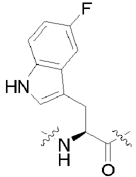 |
| 6Clw | 6-chloro-L-tryptophan | 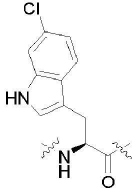 |
| 6Fw | 6-fluoro-L-tryptophan | 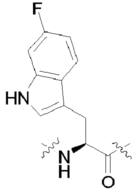 |
| 7Fw | 7 -fluoro-L-tryptophan | 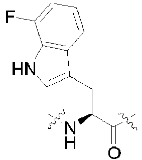 |
| 7Nw | 7-aza-L-tryptophan | 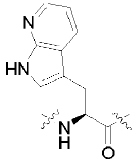 |
| Ac | Acetyl | 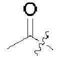 |
| Abu | 2*S*-aminobutyric acid | 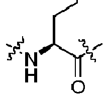 |
| AET | 2-aminoethane-1-thiol | 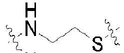 |
| AETO | 2-aminoethane-1-thiol oxidized | 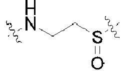 |
| ABOL | 4-Amino-butane-1,2,3-triol | 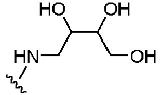 |
| AHOL | 6-Amino-hexane-1,2,3,4,5-pentaol | 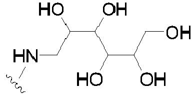 |
| AGLU | 1-amino-1-deoxy-D-glucitol | 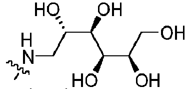 |
| Aib | 1-amino-isobutyric acid | 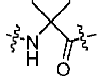 |
| Aic | 2-Aminoindane-2-carboxylic acid | 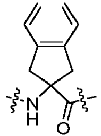 |
| ala | D-alanine | 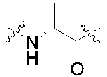 |
| Ala | L-alanine | 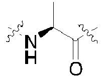 |
| alloThr | L-allo-threonine | 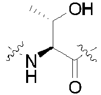 |
| Aml | α-methyl-L-leucine | 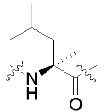 |
| Ams | α-methyl-L-serine | 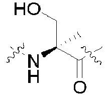 |
| APAc | 4-amino-1-carb oxymethylpiperidine | 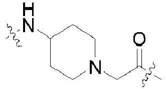 |
| Apc | 4-aminopiperidine-4-carboxylic acid | 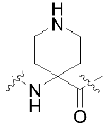 |
| Apc(DOTA) | 4-aminopiperidine-4-carboxylic acid DOTA conjugate | 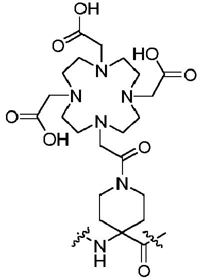 |
| Apc(DOTA-glu(AGLU)) | 4-aminopiperidine-4-carboxylic acid (glu(DOTA/AGLU)) conjugate | 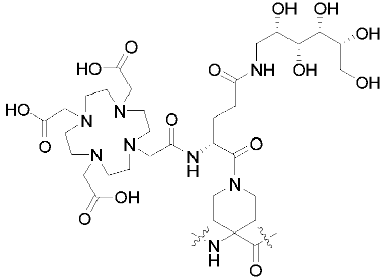 |
| Ape | 1,5 -diaminepentane | 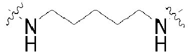 |
| APOL | 5-Amino-pentane-1,2,3,4-tetraol | 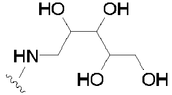 |
| APrOL | 3-Amino-propane-1,2-diol | 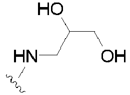 |
| Arg | L-arginine | 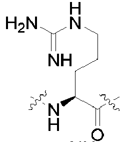 |
| Asn | L-asparagine | 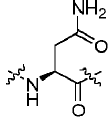 |
| asp | D-aspartic acid | 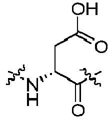 |
| Asp | L-aspartic acid | 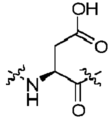 |
| Aze | (*S*)-Azetidine-2-carboxylic acid | 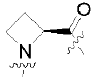 |
| Bhf | (*S*)-P-Homophenyl alanine | 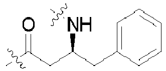 |
| Bio | D-(+)-Biotin | 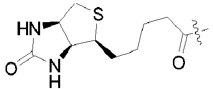 |
| Bta | 3-(3-benzothienyl)-L-alanine | 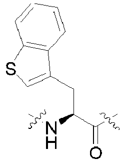 |
| Bzl | Benzyl | 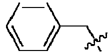 |
| Cha | L-cyclo-hexyl-alanine | 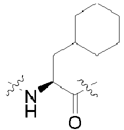 |
| Chg | L-cyclo-hexyl-glycine | 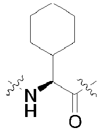 |
| Chy | 4-(*S*)-hydroxy-L-proline | 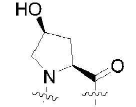 |
| Cfp | (2*S*,4*S*)-4-fluoro-pyrrolidine-2-carboxylic acid | 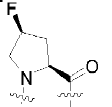 |
| Cit | L-Citrulline | 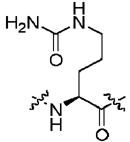 |
| Cpp | trans-3-Azabicyclo[3.1.0]hexane-2-carboxylic acid | 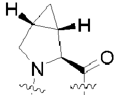 |
| Cy5SO3 | Cy5 dye (mono SO3) | 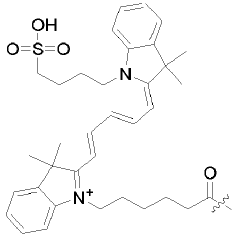 |
| Cya | L-cysteic acid | 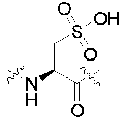 |
| cys | D-cysteine | 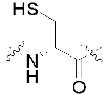 |
| Cys | L-cysteine | 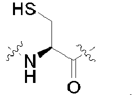 |
| Cys(2Lut) | L-cysteine bound to 2,6-lutidylidene | 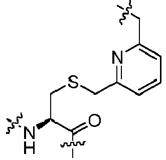 |
| Cys(3Lut) | L-cysteine bound to 3,5-lutidylidene | 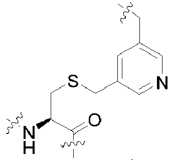 |
| Cys(3MeBn) | L-cysteine bound to 3-methylbenzylidene | 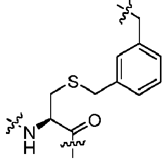 |
| Cys(tMeBn (DOTA-AET)) | L-cysteine conjugated to mesitylene conjugated to DOTA via 2-aminoethane-1-thiol | 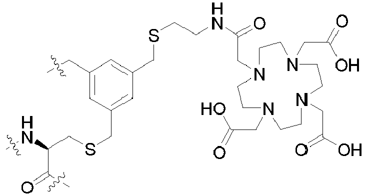 |
| Cys(tMeBn (H-AET)) | L-cysteine conjugated to mesitylene conjugated with 2-aminoethane-1-thiol linker | 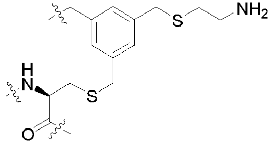 |
| Cys(tMeBn (DOTA-PP)) | L-cysteine conjugated to mesitylene conjugated to DOTA via 2-aminoethane-1-thiol | 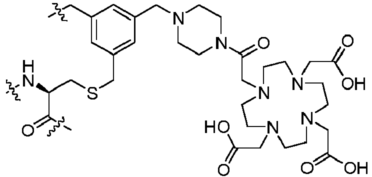 |
| Cys(tMeBn (H-PP)) | L-cysteine conjugated to mesitylene conjugated with piperazine linker | 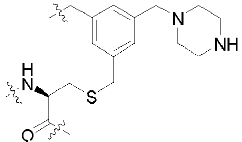 |
| Cysol | L-cysteinol | 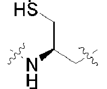 |
| Dab | (*S*)-2,4-Diaminobutyric acid | 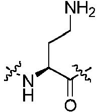 |
| Dap | (*S*)-2,3-Diaminopropionic acid | 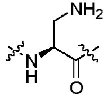 |
| Deg | Diethylgylcine | 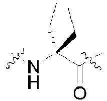 |
| Dfp | 4,4-Difluoro-L-proline | 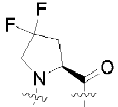 |
| Dmp | (*S*)-5,5-Dimethyl-proline | 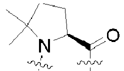 |
| DOTA | 2,2',2",2'"-(1,4,7,10-Tetraazacyclododecane-1,4,7,10-tetrayl)tetraacetic acid | 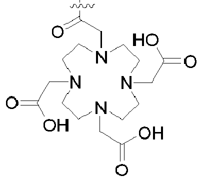 |
| Dtc | (*R*)-5,5-dimethyl-1,3-thiazolidine-4-carboxylic acid | 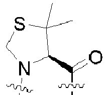 |
| Eaa | 3,4-Dichlor-L-phenylalanine | 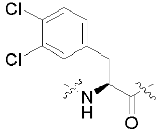 |
| Eaz | L-thiazolidine-4-carboxylic acid | 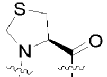 |
| Eca | 1-amino-1-cyclopentane carboxylic acid | 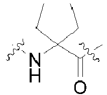 |
| Egd | (*S*)-ω,ω-Dimethyl-arginine | 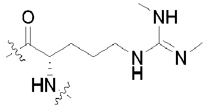 |
| Egm | 2,4-dichloro-L-phenylalanine | 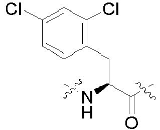 |
| Egn | 3,5-dichloro-L-phenylalanine | 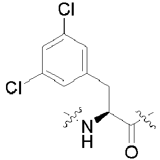 |
| Egp | 2,3 -di chl oro-L-pheny lalanine | 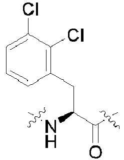 |
| Egz | 1-Amino-1-cyclohexan-carboxylic acid | 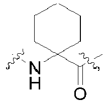 |
| en | Ethylendiamine | 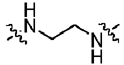 |
| EuDOTA | DOTA Europium(III) complex | 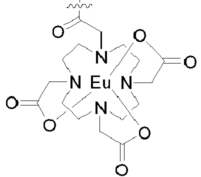 |
| GaDOTA | DOTA Gallium(III) complex | 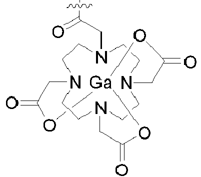 |
| GaNODAGA | NODAGA Gallium(III) complex | 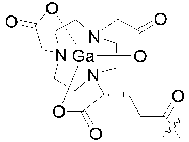 |
| Ghg | γ-Hydroxy-L-glutamic acid | 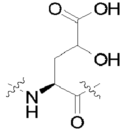 |
| gln | D-glutamine | 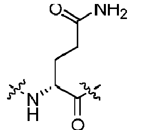 |
| Gln | L-glutamine | 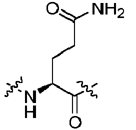 |
| glu | D-glutamic aicd | 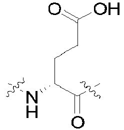 |
| Glu | L-glutamic aicd | 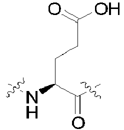 |
| glu(AGLU) | glu(AGLU) conjugate | 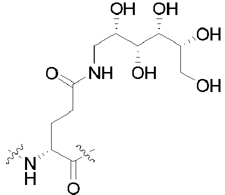 |
| Glutar | glutaric acid as linker | 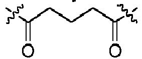 |
| Glutar | glutaric acid when present in the position of Xaa1 | 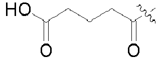 |
| Gly | glycine | 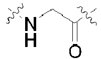 |
| H3p | 3 -(S)-hydroxy-L-proline | 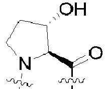 |
| Har | L-Homoarginine | 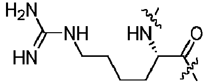 |
| Hci | L-Homocitrulline | 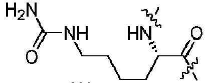 |
| Hcy | L-homocysteine | 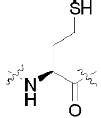 |
| Hga | L-homoglutamic acid | 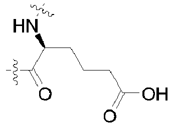 |
| Hfe | L-homophenylalanine | 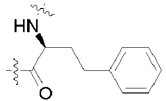 |
| Hle | L-homoleucine | 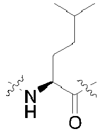 |
| Hse | L-homoserine | 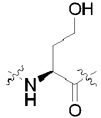 |
| Hth | L-homothreonine | 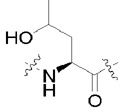 |
| Hym | (2*S*,4*R*)-4-Methyoxy-pyrrolidine-2-carboxylic acid | 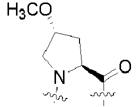 |
| Hyp | 4-(*R*)-hydroxy-L-proline | 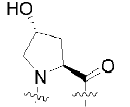 |
| Hyw | 5-hydroxy-L-tryptophane | 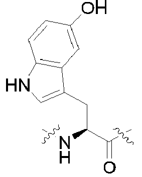 |
| Ile | L-isoleucine | 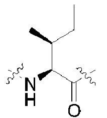 |
| InDOTA | DOTA Indium(III) complex | 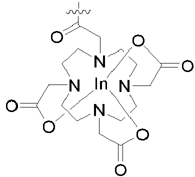 |
| leu | D-leucine | 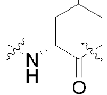 |
| Leu | L-leucine | 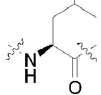 |
| LuDOTA | DOTA Lutetium(III) complex | 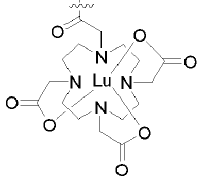 |
| lys | D-lysine | 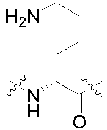 |
| Lys | L-lysine | 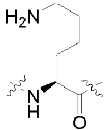 |
| Mamb | meta-aminomethyl benzoic acid | 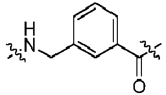 |
| Mcf | 3 -chl oro-L-phenyl al anine | 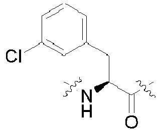 |
| MeO10kDaPEGAc | | 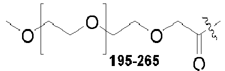 |
| MeO2kDaPEGAc | | 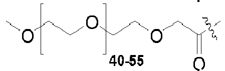 |
| MeO36PEG | Monodisperse polyethylene glycol 36 units conjugated with acetyl | 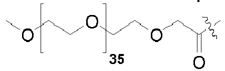 |
| Met | L-methionine | 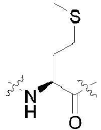 |
| mli | maleimide | 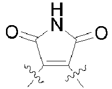 |
| Moo | (*S*)-Methionine sulfone | 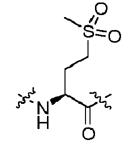 |
| N3Tap | (2*S*,4*R*)-4-azido-1-pyrrolidin-2-carboxylic acid | 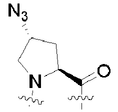 |
| N4Ac | Bis-(2-amino-ethylamine)-methylpropionic acid | 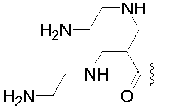 |
| Nglu | N-glutamic acid, N-carboxyethyl glycine | 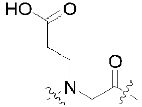 |
| Nle | L-norleucine | 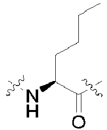 |
| Nleu | N-leucine, N-isobutyl glycine | 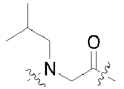 |
| Nlys | N-lysine, N-(4-aminobutyl) glycine | 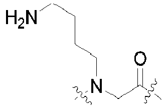 |
| nma | N-methyl-D-alanine | 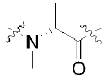 |
| Nma | N-methyl-L-alanine | 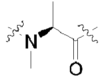 |
| Nmc | N-methyl-L-cysteine | 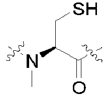 |
| Nmd | N-methyl-L-aspartic acid | 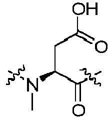 |
| Nme | N-methyl-L-glutamic acid | 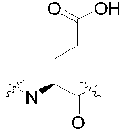 |
| Nmg | N-methyl-glycine | 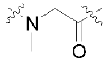 |
| Nml | N-methyl-L-leucine | 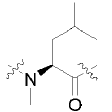 |
| Nmw | N-methyl-L-tryptophan | 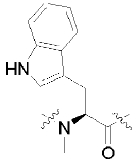 |
| NODAGA | (R)-1,4, 7-triazacyclononane, 1-glutaric acid-4,7-acetic acid | 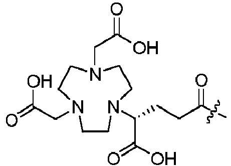 |
| NOPO | 1,4,7-triazacyclononane-1,4-bis [methylene (hydroxymethyl)phosphinic acid]-7- [methylene (2-carboxyethyl)phosphinic acid] | 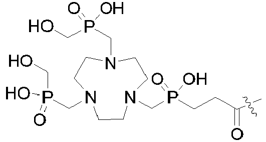 |
| Npg | L-neopentyl-glycine | 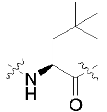 |
| Nphlp | 3-Naphthalen-1-yl-propionic acid | 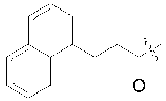 |
| Nph2p | 3-Naphthalen-2-yl-propionic acid | 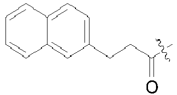 |
| Nphe | N-phenylalanine, N-benzyl-glycine | 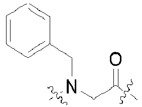 |
| Nva | L-norvaline | 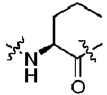 |
| O2Oc | 8-amino-3,6-dioxaoctanoic acid | 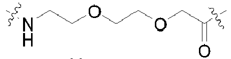 |
| Ocf | ortho-chloro-L-phenylalanine | 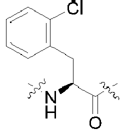 |
| Oic | (2*S*,3a*S*,7a*S*)-octahydroindolecarboxylic acid | 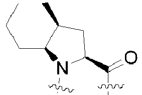 |
| Omr | ω-Methyl-L-arginine | 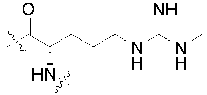 |
| Oxa | (*S*)-oxazolidine-4-carboxylic acid | 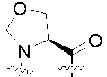 |
| Pcf | para-chl oro-L-pheny lalanine | 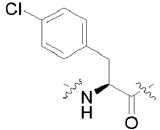 |
| PEG6 | 21-amino-4,7,10,13,16,19-hexaoxaheneicosanoic acid | 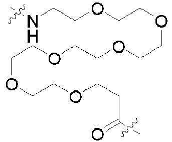 |
| Pen | L-penicillamine | 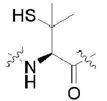 |
| Phe | L-phenylalanine | 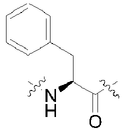 |
| Phg | L-phenylglycine | 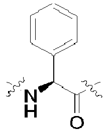 |
| Php | 3-phenyl-propionic acid | 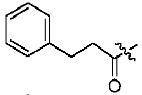 |
| Pip | (*S*)-Pipecolic Acid | 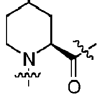 |
| PP | Piperazine | 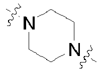 |
| pro | D-proline | 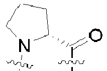 |
| Pro | L-proline | 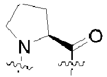 |
| Pse | L-phosphoserine | 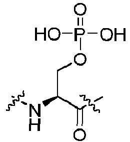 |
| Rni | (*R*)-nipecotic acid | 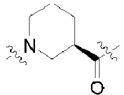 |
| Ser | L-serine | 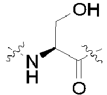 |
| Smc | S-methyl-L-cysteine | 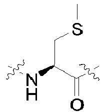 |
| Succinyl | succinic acid as linker | 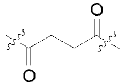 |
| Succinyl | succinic acid when present in the position of Xaa1 | 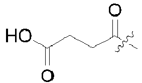 |
| Tap | (2*S*,4*R*)-4-amino-1-pyrrolidin-2-carboxylic acid | 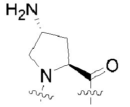 |
| Tap(AGLU-Glutar) | (2*S*,4*R*)-4-amino-1-pyrrolidin-2-carboxylic acid (AGLU-Glutar) conjugate | 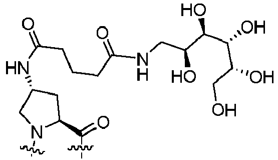 |
| Tfp | (2*S*,4*R*)-4-fluoro-pyrrolidine-2-carboxylic acid | 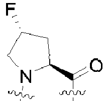 |
| Thi | L-thienylalanine | 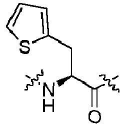 |
| Thp | 4-amino-tetrahydropyran-4-carboxylic acid | 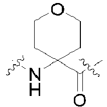 |
| Thr | L-threonine | 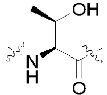 |
| Tie | (*S*)-α-t-butylglycine | 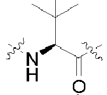 |
| tMeBn | tris-methylbenzyl, mesitylene | 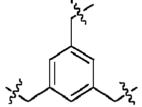 |
| tMeBn(DOTA-AET) | DOTA attached to mesitylene via 2-aminoethane-1-thiol | 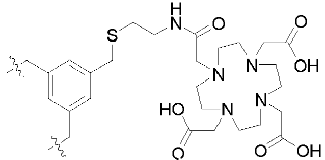 |
| tMeBn(DOTA-PP) | DOTA attached to mesitylene via piperazine | 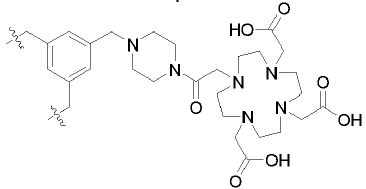 |
| trp | D-tryptophan | 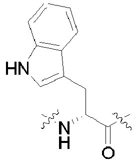 |
| Trp | L-tryptophan | 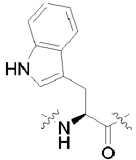 |
| Ttds | 1,13-diamino-4,7,10-trioxatridecan-succinamic acid | 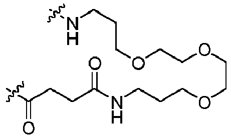 |
| tyr | D-tyrosine | 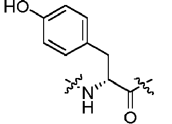 |
| Tyr | L-tyrosine | 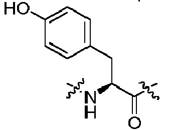 |
| val | D-valine | 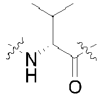 |
| Val | L-valine | 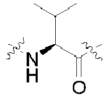 |
| ZnDOTA | DOTA Zink(II) complex | 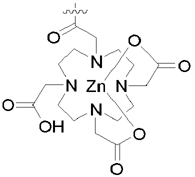 |

The amino acid sequences of the peptides provided herein are depicted in typical peptide sequence format, as would be understood by the ordinary skilled artisan. For example, the three-letter code of a conventional amino acid, or the code for a non-conventional amino acid or the abbreviations for additional building blocks, indicates the presence of the amino acid or building block in a specified position within the peptide sequence. The code for each amino acid or building block is connected to the code for the next and/or previous amino acid or building block in the sequence by a hyphen which (typically represents an amide linkage). If the hyphen stands before the abbreviation of the amino acid it usually symbolizes that the amino group of the amino acid is modified by a covalent bond and if the hyphen stands behind the amino acid abbreviation it usually symbolizes the modification of the former carboxyl group by a covalent bond. The remaing characteristic part of amino acids after modification by one or more covalent bonds is referred as residue of amino acid. Depending on the context the mentioning of the abbreviation of an amino acid or building block can symbolize either the full amino acid or building block or the residues of them. In connection with a use of a hyphen next to the abbreviation it is clearly specified that the residue of the amino acid or building block is adressed.

Depending on the spacing between the amino- and the carboxy group in amino acids they are classified into α-, β-, γ-, δ-, ε-, (and so forth) -amino acids, which means that these groups are typically are spaded apart by 1, 2, 3, 4, and 5 heavy-atoms (typically carbon), respectively.

For amino acids, in their abbreviations the first letter indicates the stereochemistry of the C-α-atom if applicable. For example, a capital first letter indicates that the L-form of the amino acid is present in the peptide sequence, while a lower case first letter indicating that the D-form of the correspondent amino acid is present in the peptide sequence. If the abbreviation starts with a number the first letter in the abbreviation will be characteristic for the stereochemistry, if applicable. However, for enhanced clarity it is at any abbreviation an option to further clearly specify the stereochemistry of an amino acid abreviation by adding for instance the prefix "D-". As example "lys", "D-Lys" or "D-lys" describe all a D-configured Lys.

For someone skilled in the art it is evident that many amino acids can be N-methylated at their amino group. These N-methyl amino acid feature can occur in combination with some other attributes like L-α- or D-α-N-methyl amino acids which are N-methylated L-α- or D-α-amino acids.

The term "α, α-dialkylamino acid" refers to amino acid which comprise indepently two alkyl groups at the α-carbon atom which maybe in some cases form a ring-structure with each other.

The term "cyclic" amino acid means that the amino acid comprises a cyclic structure wherein in some cases the amino nitrogen of the amino acid is part of a heterocyclic structure usually consisting of 4 to 7 ring members. Cyclic maybe combined with other amino acid attribute as for instance in cyclic D-α-amino acid or cyclic α,α-dialkylamino acid.

The term "aromatic amino acid" refers to amino acids which comprise an aromatic structure and this includes a heteroaromatic structure whereas the term "non-aromatic amino acid" refers to amino acids which are devoid of any aromatic structure.

The term "heteroaromatic amino acid" refers to amino acids which comprise any kind of heteroaromatic structure.

An "aliphatic amino acid" is a non-aromatic amino acid which consists of only C and H atoms apart from the amino and carboxy group.

A "polar amino acid" is any kind of amino acid which comprises apart from the amino and carboxy group functional groups or atoms selected from the group consisting of O, N, S, P, CONH₂, COOH, CN and tetrazole.

A "charged amino acid" is any kind of amino acid which comprises, apart from the amino and carboxy group, functional groups which can be charged at a certain pH, esp. in the range of 4-8, preferably selected from the group of COOH, phosphate, phosphonate, ammonium and amino nitrogen.

A neutral amino acid is any kind of amino acid which comprises apart from the amino and carboxy group, no further functional groups which can be charged at a certain pH, esp. in the range of 4-8.

If an amino acid contains more than one amino and/or carboxy group all orientations of this amino acid are in principle possible for formation of a covalent bond, but in α-amino acid the utilization of the α-amino and the α-carboxy group is preferred for the attachment to the neighbouring moieties and if other orientations are preferred they are explicitly specified.

Those skilled in the art will recognize if a stereocenter exists in the compounds disclosed herein irrespective thereof whether such stereocenter is part of an amino acid moiety or any other part or moiety of the compound of the invention. When a compound is desired as a single enantiomer or diastereomer, it may be obtained by stereospecific synthesis or by resolution of the final product or any convenient intermediate. Resolution of the final product, an intermediate, or a starting material may be affected by any suitable method known in the art. See, for example, "Stereochemistry of Organic Compounds" by E. L. Eliel, S. H. Wilen, and L. N. Mander (Wiley-lnterscience, 1994).

In an embodiment and as preferably used herein, an aromatic L-α-amino acid is any kind of L-α-amino acid which comprises an aryl group.

In an embodiment and as preferably used herein, a heteroaromatic L-α-amino acid is any kind of L-α-amino acid which comprises a heteroaryl group.

Unless indicated to the contrary, the amino acid sequences are presented herein in N- to C-terminus direction.

Derivatives of the amino acids constituting the peptides of the invention may be as set forth in Table 6. In any embodiment, one or more amino acids of the compounds of the invention are substituted with a derivative of the corresponding preferred amino acids.

**Table 6: Exemplary derivatives of preferred amino acids contained in the compound of the invention**

| **Amino Acid** | **Exemplary derivatives** |
|---|---|
| Ala | Aib, Bal, Abu, Gly, Nva, Nle |
| Cys | Hcy, Nmc |
| Asp | Glu, Asn, Gln, Cya |
| Glu | Asp, Asn, Gln, Cya, Homoglutamic acid, γ-Hydroxy-glutamic acid, |
| Phe | Hfe, Phg, Bhf, Thi, Bta, Bromophenylalanine, Iodophenylalanine, Chlorophenylalanine, Methylphenylalanine, Nitrophenylalanine, Tyr, Trp, N aphthy lalanine, Trifluoromethylphenylalanine |
| Gly | Ala, ala, Nmg |
| Nmg | Pro, Ala, ala, Gly, Nma, nma |
| Ile | Leu, Val, Hle, Nva, Nle, Chg |
| Lys | Arg, Dab, Dap, Har, Egd, Omr, Hci, Cit |
| Leu | Ile, Val, Hle, Nle, Nva, Moo |
| Met | Ile, Val, Hle, Nle, Nva, Moo |
| Nle | Ile, Val, Hle, Met, Nva, Moo |
| Asn | Asp, Glu, Gln, Cya, Thr |
| Pro | Aze, Pip, Hyp, Tfp, Cfp, Dmp, Tap, H3p, 4Ap, Cpp, Hym, Chy, Dfp |
| Gln | Asp, Asn, Glu, Cya, Thr, Hse |
| Arg | Arg, Dab, Dap, Har, Egd, Omr, Hci, Cit |
| Ser | Thr, Hse, *allo*-Threonine |
| Thr | Ser, Homothreonine, allo-Threonine |
| Val | Leu, Ile, Hle, Nva, Nle |
| Trp | Hfe, Phg, Bhf, Thi, Bta, Bromophenylalanine, Iodophenylalanine, Chlorophenylalanine, Methylphenylalanine, Nitrophenylalanine, Tyr, Trp, N aphthy lalanine, Trifluoromethylphenylalanine |
| Tyr | Hfe, Phg, Bhf, Thi, Bta, Bromophenylalanine, Iodophenylalanine, Chlorophenylalanine, Methylphenylalanine, Nitrophenylalanine, Tyr, Trp, N aphthy lalanine, Trifluoromethylphenylalanine |

A general linear peptide is typically written from the N-to C-terminal direction as shown below:
A-Xaa1-Xaa2-Xaa3-Xaa4-.......Xaan-C-term;

Therein
1. ,Xaax' is the abbreviation, descriptor or symbol for amino acids or building blocks at specific sequence position x as shown in Table 5,
2. ,A' is an N-terminal modification group, e.g., an abbreviation for a specific terminating carboxylic acid like ' Ac' for acetyl or other chemical group or structural formula of chemical groups linked to the N-terminal amino acid code (Xaa1) via a hyphen and
3. ,C-term' is a C-terminal group which is typically 'OH' or 'NH₂' (as terminal carboxylic acid or amide) or an abbreviation for a specific terminating amine linked to the C-terminal amino acid code (Xaan) via a hyphen.

### Branched peptides with side chains modified by specific building blocks or peptides

Linear, branched peptides are written from the N-to C-terminal direction as shown below:
1. A-Xaa1-Xaa2-Xaa3(*NT-Xab1-Xab2-.......Xabn*)-.......Xaan-C-term
   or
2. A-Xaa1-Xaa2-Xaa3(*Xac1*-*Xac2*-... ....*Xacn-C-term*)*-*.......Xaan-C-term

Therein, the statements 1. - 3. of the description of linear peptides for the specification of 'Xaax', ,A' and ,C-term' in the main chain of the branched peptide apply.

The position of a branch is specified by parentheses after an ,Xaax' abbreviation. Branches typically either occur
1. at lysine (Lys) residues or 4-aminopiperidine-4-carboxylic acid (Apc) (or similar), which means that the branch is attached to side chain ε-amino function of the lysine or to the nitrogen atom within the piperidine ring of 4-aminopiperidine-4-carboxylic acid via an amide bond,
   or
2. at glutamic acid (Glu) residues (or similar), which means that the branch is attached to γ-carboxy function of the glutamic acid.

The content of the parentheses describes the sequence/structure of the peptide branch.
1. Within an *'A-Xab1-Xab2-... ....Xabn'* branch, which is connected to lysine residues (or similar),
   1. *′Xabx'* is the abbreviation, descriptor or symbol for amino acids or building blocks at specific sequence position x of the branch as shown in Table 5,
   2. *,A'* is an N-terminal group, e.g. an abbreviation for a specific terminating carboxylic acid like 'Ac' for acetyl or other chemical group or structural formula of chemical groups linked to the N-terminal amino acid code (*Xabl*) via a hyphen and
   3. the last building block of the branch *′Xabn',* which connects the branch with the main chain by forming an amide bond with its own carboxyl function with the side chain amino function of this lysine or 4-aminopiperidine-4-carboxylic acid (or similar residue).
2. Within an *'Xac1-Xac2-... ....Xacn-C-term'* branch, which is connected to glutamic acid residues (or similar),
   1. *′Xacx'* is the abbreviation, descriptor or symbol for amino acids or building blocks at specific sequence position x of the branch as shown in Table 5,
   2. *′Xac1'* is the first building block of the branch, which connects the branch with the main chain by forming an amide bond with its own amino function with the side chain carboxylic acid function of the glutamic acid (or similar residue) and
   3. '*C-term'* is a C-terminal group which is typically 'OH' or 'NH₂' (as terminal carboxylic acid or amide) or an abbreviation for a specific terminating amine linked to the C-terminal amino acid code (*Xacn*) via a hyphen.

### Cyclic peptides

An exemplaric general cyclic peptide written from the N- to C-terminal direction is shown below:
A-Xaa1-[Xaa2-Xaa3-Xaa4-.......Xaan]-C-term;

Therein the statements 1. -3. of the description of linear peptides for the specification of ,Xaax', 'A' and ,C-term' in the main chain of the cyclic peptide apply. The characteristics of the peptide cycle are specified by square brackets.
1. The opening square bracket indicates the building block at whose side chain the cycle is initiated (*cycle initiation residue*) and
2. the closing square bracket indicates the building block at whose side chain the cycle is terminated (*cycle termination residue*)*.*

The chemical nature of the connection between these two residues is
1. an amide bond in case that among those indicated residues one residue contains an amino function its side chain (e.g. Lys) while the other contains a carboxyl function in its side chain (e.g. Glu) or
2. a disulphide bond in case that those indicated residues/amino acids contain sulfhydryl moieties (e.g., Cys).

### Cyclic peptides containing an additional cyclization element (Yc)

A general extended cyclic peptide written from the N-to C-terminal direction is shown below:
A-Xaa1-[Xaa2(Yc)-Xaa3-Xaa4-.......Xaan]-C-term;

Therein the statements 1. - 3. of the description of linear peptides for the specification of,Xaax', 'A' and ,C-term' in the main chain of the cyclic peptide apply. In addition, ,Yc' is the cyclization element. As in case of cyclic peptides, the characteristics of the cycle are specified by square brackets which indicate *cycle initiation residue* and *cycle termination residue* (statements 1. and 2. of cyclic peptides)

The content of the parentheses adjacent to the cycle initiation residue specifies the cyclization element Yc within the extended peptide cycle. The Yc element is linked to the side chain of said residue. Furthermore, the Yc element is linked to the side chain of the *cycle termination residue.* The chemical nature of the linkages between either of these residues the Yc element depend on side chain functionality of both corresponding amino acids Xaan. The linkage is a thioether if the side chain of Xaan contains a sulfhydryl group (e.g., Cys).

As non-limiting example for a branched, cyclic peptide containing an additional cyclization element peptide the structure of
AGLU-Glutar-Asp-Trp-5Clw-Hyp-Glu-leu-[Cys(2Lut)-Apc(AGLU-Glutar)-Leu-Ile-Cys]-APAc-Lys(DOTA)-NH₂
is depicted below.

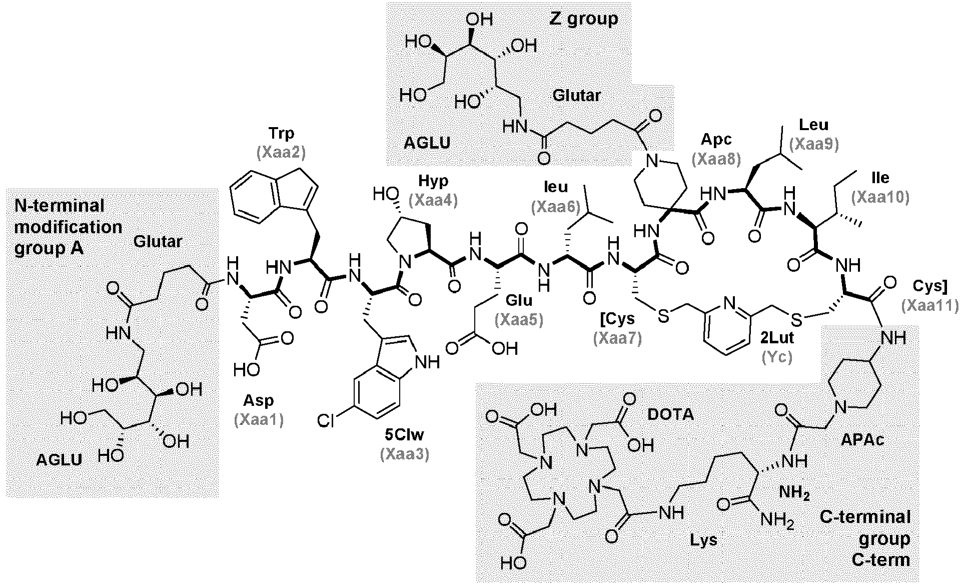

Therein
1. AGLU and Glutar bound to the N-terminal Asp (Xaa1) correspond to the ,N-terminal modification group A' in the general formula.
2. Asp, Trp, 5Clw, Hyp, Glu, leu, Cys, Apc, Leu, Ile, Cys correspond to ,Xaal' to 'Xaa11' in the general formula. For clarity the main chain is represented with bold line thickness.
3. APAc, Lys, DOTA and NH₂ correspond to the ,C-terminal group C-term' in the general formula.
4. The opening square bracket (,[') adjacent to the N-terminal cysteine (Xaa7) in the sequence indicates that at this residue the cycle is initiated (*cycle initiation residue*)*.*
5. The closing square bracket (,]') adjacent to the N-terminal cysteine (Xaa11) in the sequence indicates that at this residue the cycle is terminated (*cycle termination residue*)*.*
6. 2Lut within the parentheses adjacent to the Cys indicated as initiation residue specifies the cyclization element,Yc'. It is further bound to the Cys indicated as cycle termination residue. The Yc element is connected to said residues via thioether linkages.
7. To the amino group of the Apc residue a Z group is attached. The latter consist of the bio-distribution modifier AGLU with the linker Glutar.

In accordance with the present invention, the compound of the present invention may comprise one or more of a Z group. The Z group comprises a bio-distribution modifier with an optional linker, a chelator with an optional linker or a combination thereof. As preferably used, a linker is an element, moiety, or structure which separates two parts of a molecule. In the present invention, the linker forms a covalent bond with the chelator or the bio-distribution modifier or both and the respective part of the compounds of invention where Z is attached. A first functional group of the linker forming the bond with the chelator, the bio-distribution modifier or both of them may, in principle, be any chemical group which is capable of forming a bond, preferably a covalent bond with the chelator or the bio-distribution modifier group or both. In addition, the linker comprises a second functional group forming the bond, preferably a covalent bond with the part of the compounds of invention at the specified positions where Z is attached. Such second functional group may, in principle, be any chemical group which is capable of forming such bond with said part of the compound of invention at the specific positions where Z is attached.

An important property or feature of a linker is that it spaces apart the chelator or the bio-distribution modifier or both from the peptide part of the compound of invention. This is especially important in cases where the target binding ability of the peptide is compromised by the close proximity of the chelator. However, the overall linker length in its most extended conformer should not exceed 200 Å, preferably not more than 150 Å and most preferably not more than 100 Å.

A further important feature or property of a linker is that it enables the attachment of the chelator or the bio-distribution modifier or both to suitable positions of the peptide part of the compound of invention.

In an embodiment, a residue of a dicarboxylic acid represents a building block for a linker. Dicarboxylic acid-based linkers are used to enable the attachment of a chelator or a bio-distribution modifier to the N-terminal amine of the peptide of invention or an amino function of the side chain of an amino acid residue of the peptide of invention. Such N-terminal amine or amino side chain provides an amino function as conjugation group to which one of the at least two carboxy groups provided by the dicarboxylic acid building block is covalently attached forming an amide linkage. The bio-distribution modifier or chelator provides an amino function as conjugation group to which another of the at least two carboxy groups provided by the dicarboxylic acid building block is covalently attached forming an amide linkage, too. The very kind of dicarboxylic acid used as building block for a linker is dependent on the nature of the bio-distribution modifier or chelator and the attachment site on the peptide of invention and more specifically any functional group available at or provided by the bio-distribution modifier and chelator, respectively, which is reacted with one of the at least two carboxy groups of the dicarboxylic acid building block.

Preferred building blocks are dicarboxylic acids, which are selected from formulae (Lin1), (Lin2) or (Lin3)

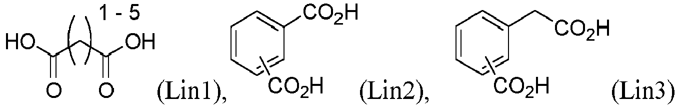

More preferred dicarboxylic acid building blocks such as formula (Lin1) are malonic acid, succinic acid, glutaric acid, adipic acid, wherein the two carboxylic acid groups are attached to ends of the aliphatic chain; such as formula (Lin2) phthalic acid, terephthalic acid, isophthalic acid and such as formula (Lin3) 2-, 3- or 4-carboxy-phenyl acetic acid. The most preferred dicarboxylic acid building block is glutaric acid (Glutar).

In an embodiment, a residue of a diamine represents a building block for a linker. Diamine based linkers are used to enable the attachment of a chelator or a bio-distribution modifier to the C-terminal carboxylic acid moiety of the peptide of invention or a carboxylic acid function of the side chain of an amino acid residue of the peptide of invention. Such C-terminal carboxylic acid or carboxylic acid side chain provides a carboxylic acid function as conjugation group to which one of the at least two amino groups provided by the diamine is covalently attached forming an amide linkage. The bio-distribution modifier or chelator provides a carboxylic acid as conjugation group to which another of the at least two amino groups provided by the diamine building block is covalently attached forming an amide linkage, too. The very kind of diamine used as building block for a linker is dependent on the nature of the bio-distribution modifier or chelator and the attachment site on the peptide of invention and more specifically any functional group available at or provided by the bio-distribution modifier and chelator, respectively, which is reacted with one of the at least two amino groups of the diamine building block.

Preferred building blocks are diamines, which are selected from formulae (Lin4), (Lin5), (Lin6) or (Lin7)

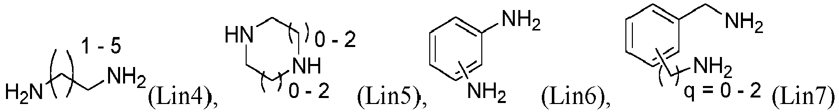

More preferred diamine building blocks are ethylenediamine (en), 1,4-diaminobutane, 1,5-diaminopentane (Ape), 1,6-diaminohexane, 1,3-dazetine, piperazine (PP), 1,3-diazinane, o-, m- and p-phenylenediamine, 4-aminobenzylamine, 1,4-bis(aminomethyl)benzene. The most preferred diamino building blocks are 1,5-diaminepentane (Ape), ethylenediamine (en) and piperazine (PP).

In an embodiment, a residue of an amino acid represents the building block for a linker. Amino acid-based linkers are used to enable the attachment of a chelator or a bio-distribution modifier to N-terminal amine, an amino function of the side chain of an amino acid residue or the C-terminal carboxylic acid moiety of the peptide of invention. Such N-terminal amine or amino side chain of the peptide of invention provides an amino function to which the carboxylic acid moiety of the amino acid linker is attached covalently by formation of an amide bond. The bio-distribution modifier or chelator provides a carboxylic acid as conjugation group, to which the amino function of the amino acid linker is attached covalently by formation of an amide bond.

Furthermore, to the amino function of the linker, the carboxylic acid moiety of a further linker can be attached covalently by an amide bond, which preferably is an alpha amino acid linker. As an alternative such C-terminal carboxylic acid moiety of the peptide of invention provides a carboxylic acid group to which the amino function of the amino acid linker is attached covalently by formation of an amide bond. In this case the bio-distribution modifier or chelator provides an amine as conjugation group to which the carboxylic acid moiety of the linker is attached covalently by formation of an amide bond. Furthermore, to the carboxylic acid moiety of the linker, the amino function of a further linker can be attached covalently by an amide bond, which preferably is an alpha amino acid linker.

Preferred building blocks are amino acids, which are selected from formulae (Lin8), (Lin9), (Lin10), Lin(11) or Lin(12).

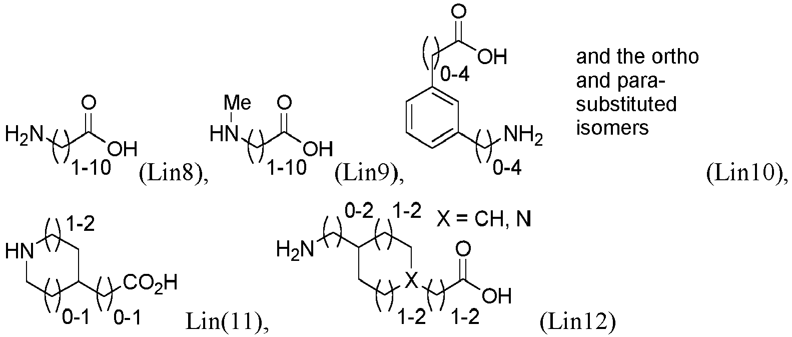

It is within the present invention that such amino acid linker is not further substituted. It is, however, also within the present invention that such amino acid linker is further substituted; preferably such substitution is CO-NH₂ and/or Ac-NH-.

Representatives of this kind of amino acid (structure Lin8), which can be used as a linker building block are glycine (Gly), beta-alanine (Bal), gamma-aminobutyric acid (GABA), 5-amino-pentanoic acid, 6-amino-hexanoic acid and homologs with up to 10 CH₂ groups.

Further representatives of this kind of amino acid (structure Lin9), which can be used as a linker building block are N-methylglycine (Nmg), N-methyl-beta-alanine, N-Methyl-gamma-aminobutyric acid, N-methyl-5-amino-pentanoic acid, N-methyl-6-amino-hexanoic acid and homologs with up to 10 CH₂ groups.

Further representatives of this kind of amino acid (structure Lin10), which can be used as a linker building block are 3-aminomethyl-benzoic acid, 4-aminomethyl-benzoic acid, anthranilic acid, 3-amino-benzoic acid and 4-amino-benzoic acid.

Further representatives of this kind of amino acid (structure Lin11), which can be used as a linker building block are 4-carboxymethyl-piperidine (Cmp), 3-piperidinecarboxylic acid (Nipecotic acid, Rni/Sni) and 4-piperidinecarboxylic acid (Isonipecotic acid, Inp).

Further representatives of this kind of amino acid (structure Lin12), which can be used as a linker building block are (4-aminopiperidin-1-yl)acetic acid (APAc), 2-(piperazin-1-yl)-acetic acid (PPAc) and 4-(aminomethyl)cyclohexanecarboxylic acid (4Amc).

It will be appreciated by a person skilled in the art that in case of amino acids with stereogenic centers all stereoisomeric forms may be used as such a linker.

In a further embodiment, the amino acid is an amino acid which contains, preferably as a backbone, a polyether. Preferably such polyether is polyethylene glycol and consists of up to 30 monomer units. Preferably, an amino acid comprising such polyether shows an increase in hydrophilicity compared to an amino acid not comprising such polyether. If incorporated as a linker the result is typically an increase in hydrophilicity. A preferred embodiment of this kind of amino acid is depicted in the following, wherein it will be acknowledged that such amino acid may comprise 0, 1, 2, 3, 4, 5, 6, 7, 8, 9, 10, 11 or 12 ethylene oxide moieties:

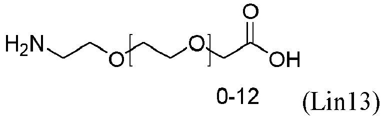

Preferred ethylene glycol containing amino acids are Ttds, O2Oc and PEG6.

In a further embodiment, a residue of an alpha amino acid represents a building block for a linker. Alpha amino acid-based linkers are used to enable the attachment of a chelator and/or a bio-distribution modifier to N-terminal amine, an amino function of the side chain of an amino acid residue of the peptide of invention or the C-terminal carboxylic acid moiety of the peptide of invention. Thereby simultaneous a chelator and a bio-distribution modifier or two bio-distribution modifiers can attached to one site of the peptide of the invention. Such N-terminal amine or amino side chain provides an amino function to which one of the one or more carboxy groups provided by the alpha amino acid building block is covalently attached forming an amide linkage. Such C-terminal carboxylic acid moiety provides a carboxylic acid function as conjugation group to which one of the one or more amino groups provided by the alpha amino acid covalently attached forming an amide linkage. In such cases wherein the alpha amino acid has more than one amino function the bio-distribution modifier or chelator provides a carboxylic acid moiety as conjugation group, which is attached covalently to said amino function of the alpha amino acid forming an amide linkage, while the other one of the amines is used as linkage to peptide of invention. In such cases wherein the alpha amino acid has more than one carboxylic acid moieties the bio-distribution modifier or chelator provides an amine as conjugation group, which is attached covalently to said carboxylic acid moiety of the alpha amino acid forming an amide linkage, while the other one of the carboxylic acid moieties is used as linkage to peptide of invention.

Preferred building blocks are alpha amino acids, which are selected from formula (Lin14) or (Lin15)

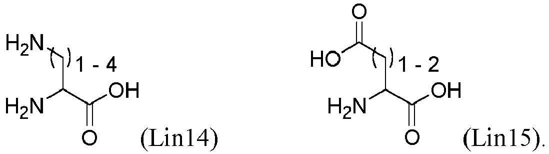

More preferred alpha amino acids building blocks are di-amino-propionic acid (Dap), di-aminobutyric acid (Dab), ornithine, lysine (Lys), aspartic acid and glutamic acid. The most preferred alpha amino acid building blocks are lysine and glutamic acid. It will be appreciated by a person skilled in the art that in case of alpha amino acids with stereogenic centers all stereoisomeric forms may be used as such a linker.

However, the use of linkers usually follows a purpose. In some circumstances it is necessary to space a larger moiety apart from a bioactive molecule in order to retain high bioactivity. In other circumstances introduction of a linker opens the chance to tune physicochemical properties of the molecule by introduction of polarity or multiple charges. In certain circumstances the site of attachment within the sequence of the peptide of invention and the chemical nature of the bio-distribution modifier or chelator necessitate the implementation of a linker. Further, in certain circumstances it might be a strength and achievement if one can combine the chelator with a bioactive compound without the need for such linkers. Compounds comprising N-terminal bio-distribution modifiers typically comprise dicarboxylic acids for the linkage of the bio-distribution modifier to the bioactive compound. Compounds comprising C-terminal bio-distribution modifiers typically comprise alpha amino acids for the linkage of the bio-distribution modifier to the bioactive compound. Compounds of the present invention where the chelator is directly attached to the side chain of a residue of the bioactive compound under the formation of an amide bond typically perform excellently without the use of any dedicated linker.

It will be acknowledged by a person skilled in the art that the radioactive nuclide which is or which is to be attached to the compound of the invention, is selected taking into consideration the disease to be treated and/or the disease to be diagnosed, respectively, and/or the particularities of the patient and patient group, respectively, to be treated and to be diagnosed, respectively.

In an embodiment of the present invention, the radioactive nuclide is also referred to as radionuclide. Radioactive decay is the process by which an atomic nucleus of an unstable atom loses energy by emitting ionizing particles (ionizing radiation). There are different types of radioactive decay. A decay, or loss of energy, results when an atom with one type of nucleus, called the parent radionuclide, transforms to an atom with a nucleus in a different state, or to a different nucleus containing different numbers of protons and neutrons. Either of these products is named the daughter nuclide. In some decays the parent and daughter are different chemical elements, and thus the decay process results in nuclear transmutation (creation of an atom of a new element). For example, the radioactive decay can be alpha decay, beta decay, and gamma decay. Alpha decay occurs when the nucleus ejects an alpha particle (helium nucleus). This is the most common process of emitting nucleons, but in rarer types of decays, nuclei can eject protons, or specific nuclei of other elements (in the process called cluster decay). Beta decay occurs when the nucleus emits an electron (β⁻-decay) or positron (β⁺-decay) and a type of neutrino, in a process that changes a proton to a neutron or the other way around. By contrast, there exist radioactive decay processes that do not result in transmutation. The energy of an excited nucleus may be emitted as a gamma ray in gamma decay, or used to eject an orbital electron by interaction with the excited nucleus in a process called internal conversion, or used to absorb an inner atomic electron from the electron shell whereby the change of a nuclear proton to neutron causes the emission of an electron neutrino in a process called electron capture (EC), or may be emitted without changing its number of proton and neutrons in a process called isomeric transition (IT). Another form of radioactive decay, the spontaneous fission (SF), is found only in very heavy chemical elements resulting in a spontaneous breakdown into smaller nuclei and a few isolated nuclear particles.

In a preferred embodiment of the present invention, the radionuclide can be used for labeling of the compound of the invention.

In an embodiment of the present invention, the radionuclide is suitable for complexing with a chelator, leading to a radionuclide chelate complex.

In a further embodiment one or more atoms of the compound of the invention are of non-natural isotopic composition, preferably these atoms are radionuclides; more preferably radionuclides of carbon, oxygen, nitrogen, sulfur, phosphorus and halogens: These radioactive atoms are typically part of amino acids, in some case halogen containing amino acids, and/or building blocks and in some cases halogenated building blocks each of the compound of the invention.

In a preferred embodiment of the present invention, the radionuclide has a half-life that allows for diagnostic and/or therapeutic medical use. Specifically, the half-life is between 1 min and 100 days.

In a preferred embodiment of the present invention, the radionuclide has a decay energy that allows for diagnostic and/or therapeutic medical use. Specifically, for γ-emitting isotopes, the decay energy is between 0.01 and 4 MeV, preferably between 0.08 and 0.4 MeV, for diagnostic use. For positron-emitting isotopes, the mean decay energy is between 0.2 and 3 MeV, preferably between 0.2 and 1 MeV, for diagnostic use. For β⁻-emitting isotopes, the mean decay energy is between 0.02 and 3 MeV, preferably between 0.1 and 1 MeV, for therapeutic use. For α-emitting isotopes, the decay energy is between 3 and 8 MeV, preferably between 3.9 and 6.4 MeV, for therapeutic use.

In a preferred embodiment of the present invention, the radionuclide is industrially produced for medical use. Specifically, the radionuclide is available in GMP quality.

In a preferred embodiment of the present invention, the daughter nuclide(s) after radioactive decay of the radionuclide are compatible with the diagnostic and/or therapeutic medical use. Furthermore, the daughter nuclides are either stable or further decay in a way that does not interfere with or even support the diagnostic and/or therapeutic medical use. Representative radionuclides which may be used in connection with the present invention are summarized in Table 7.

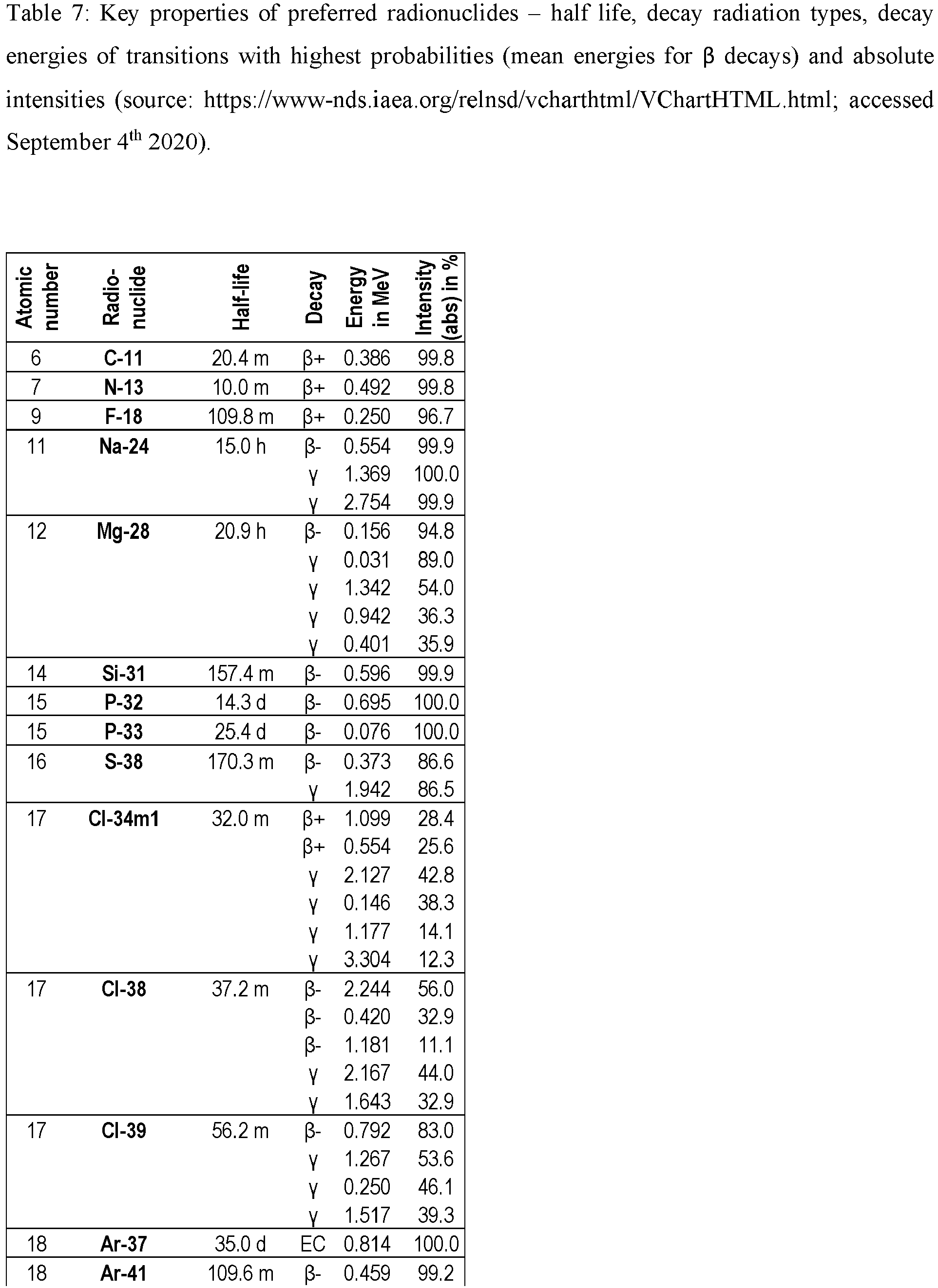

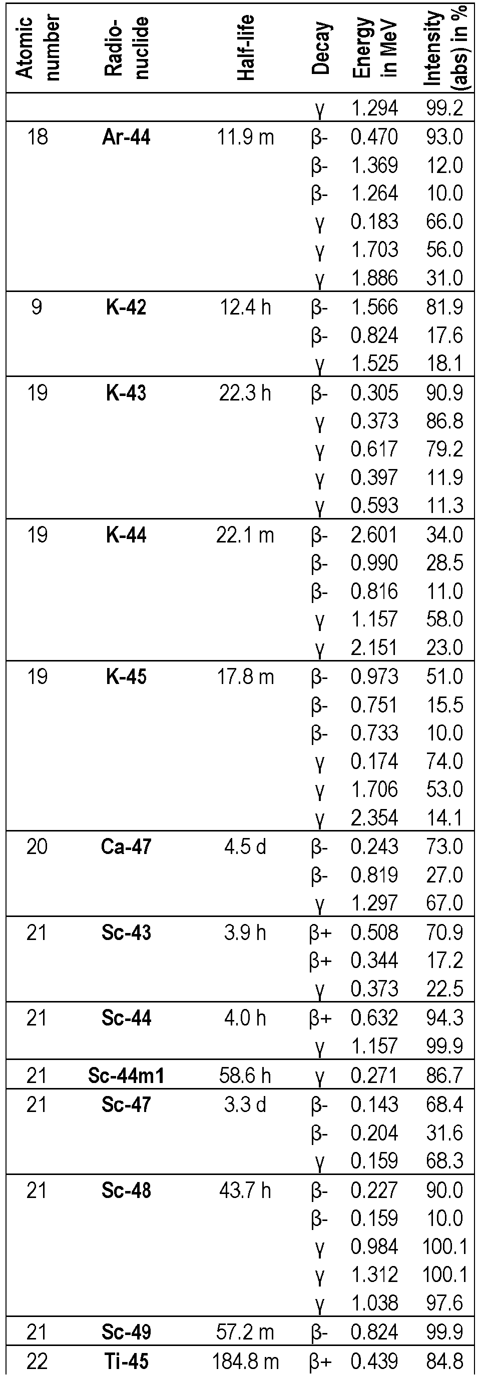

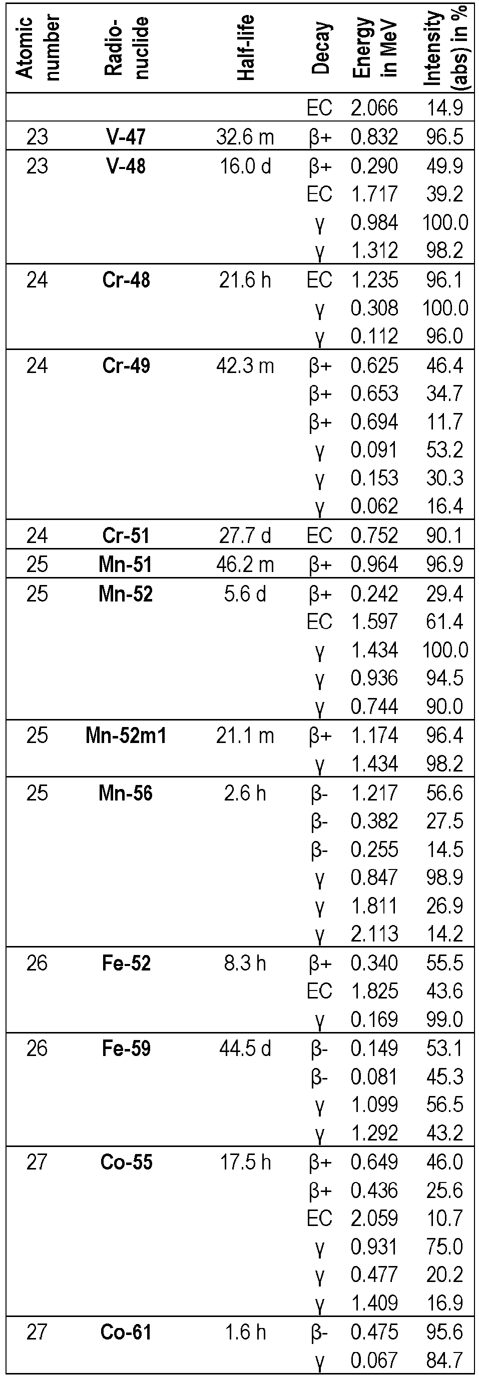

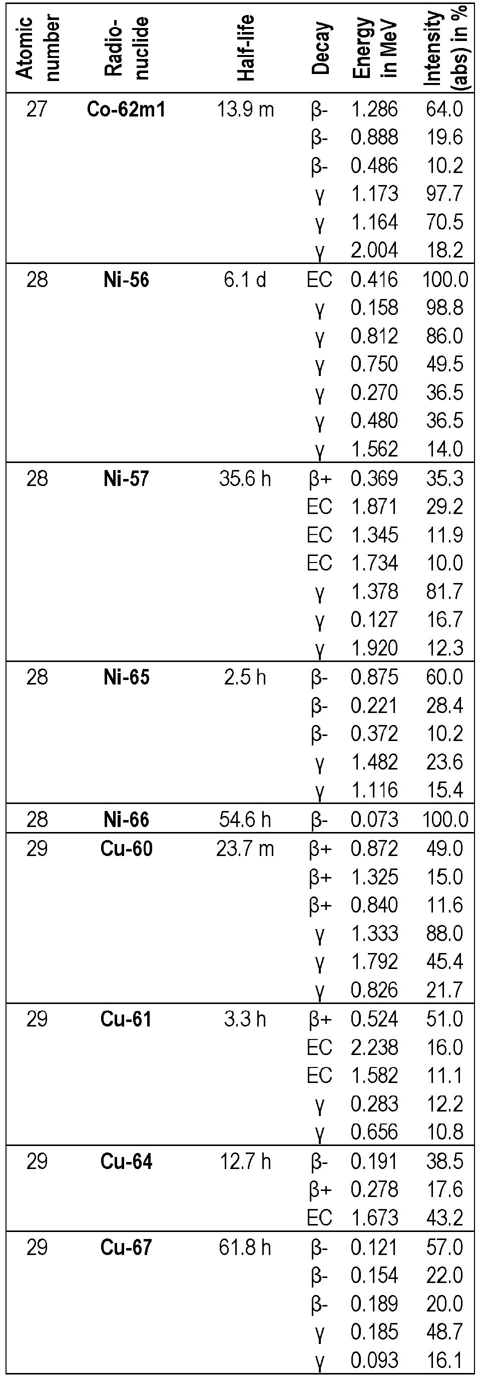

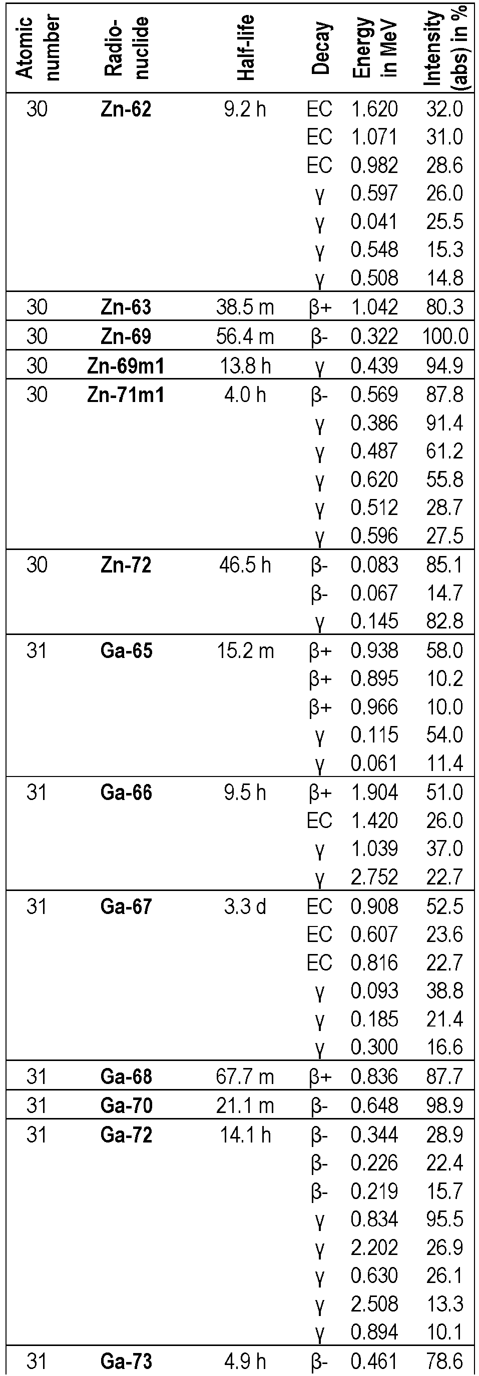

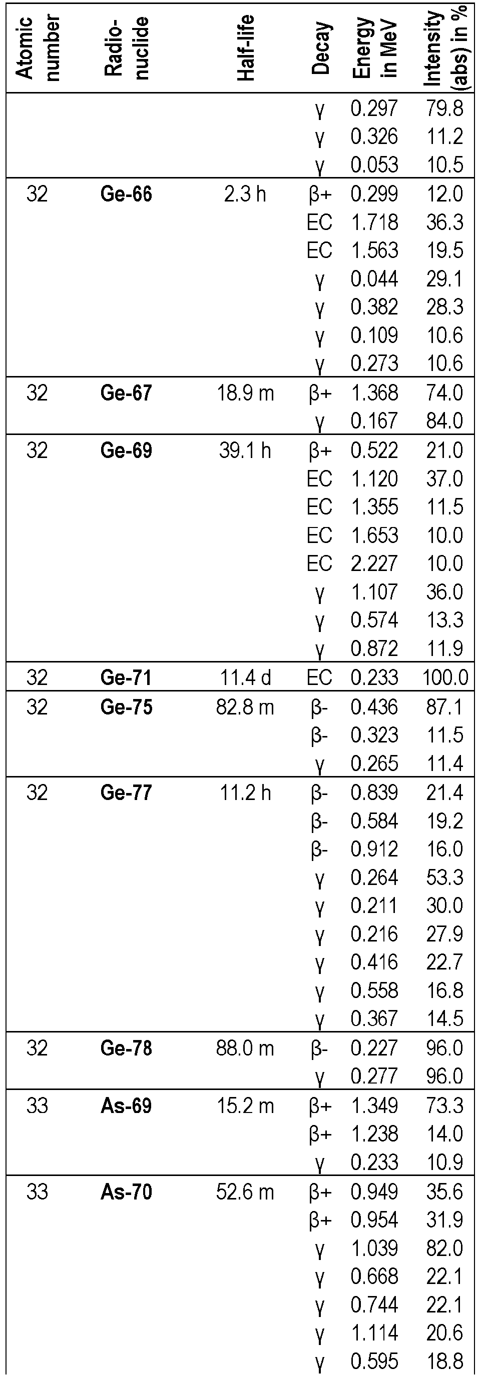

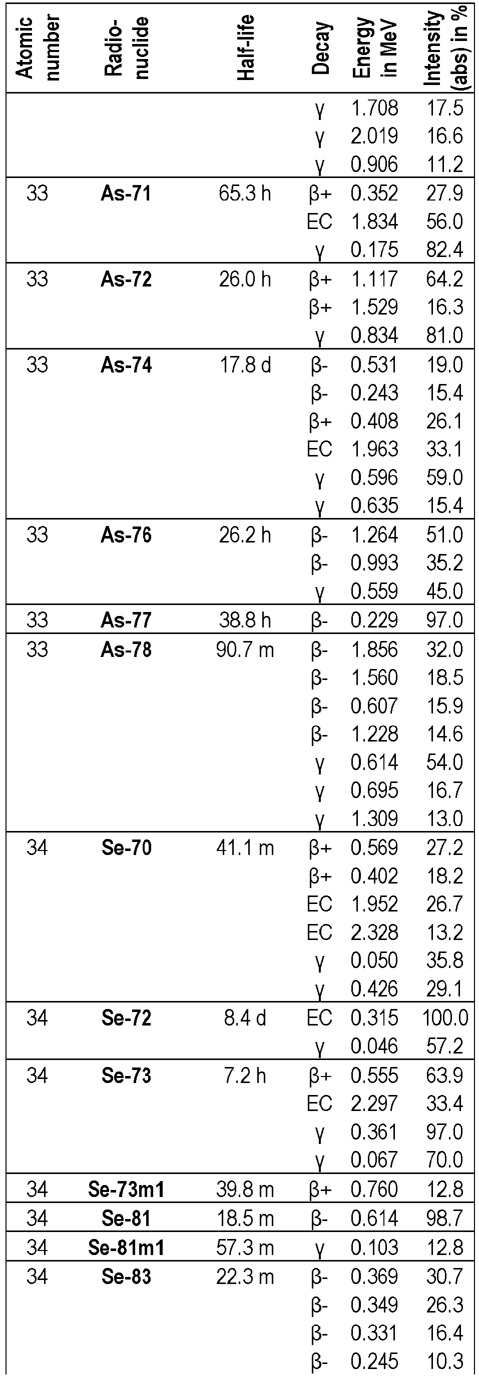

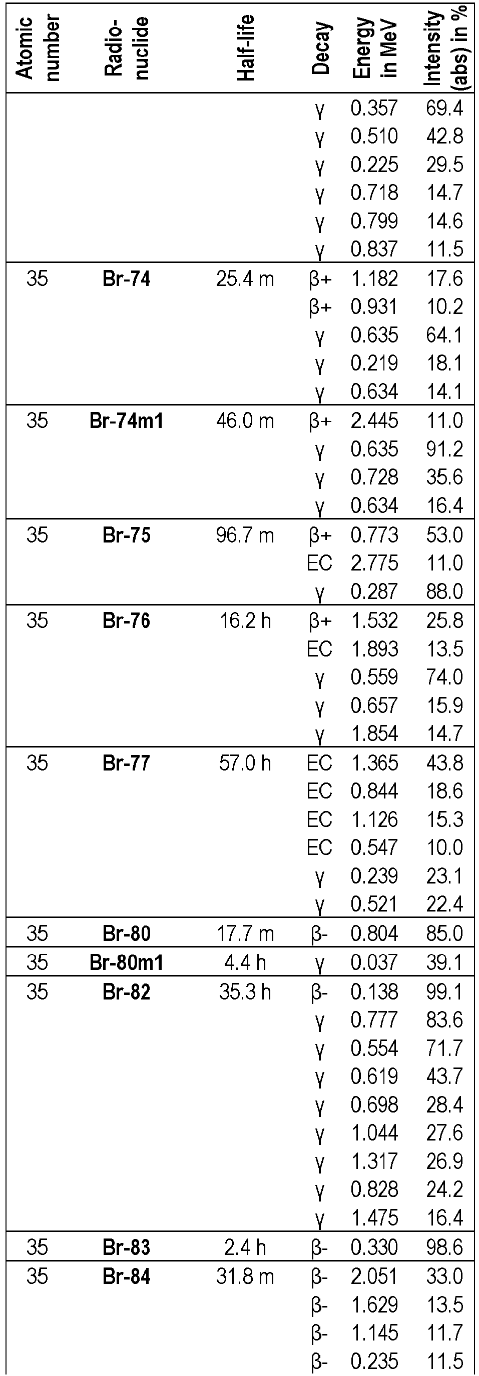

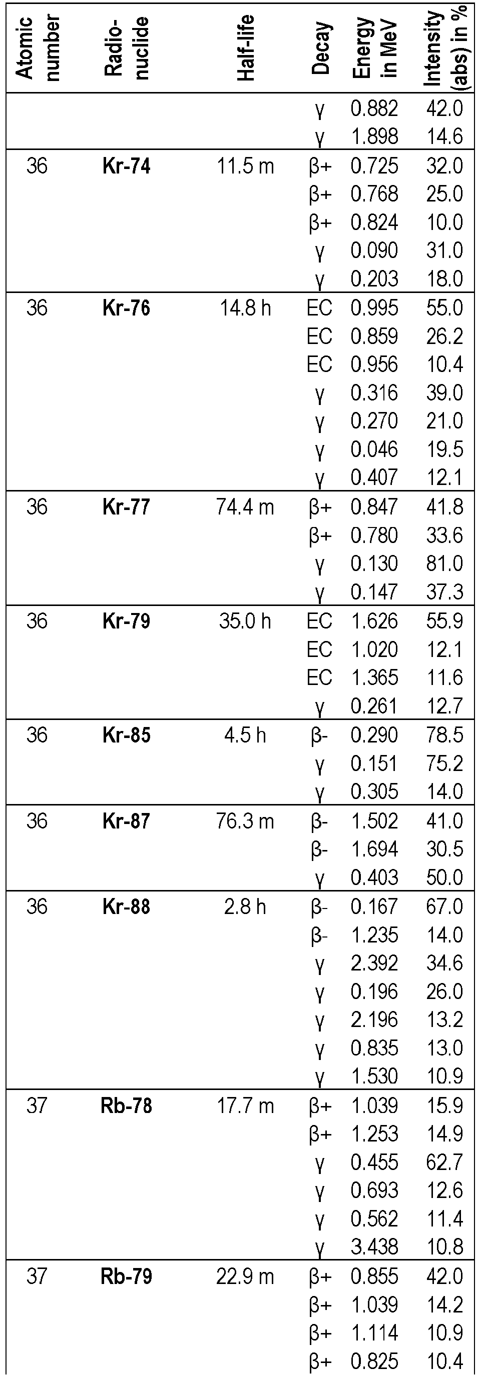

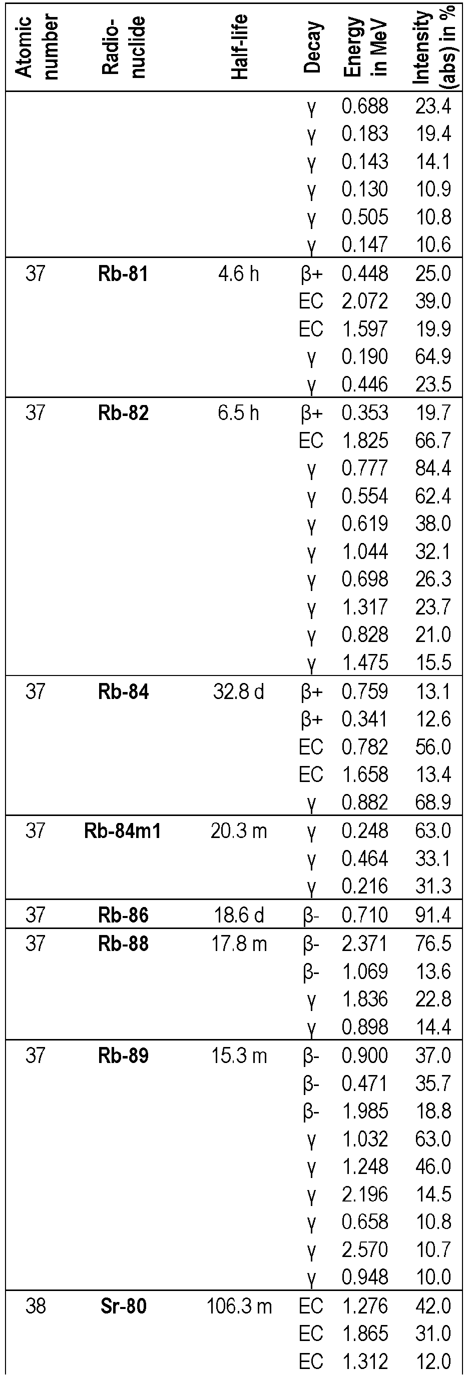

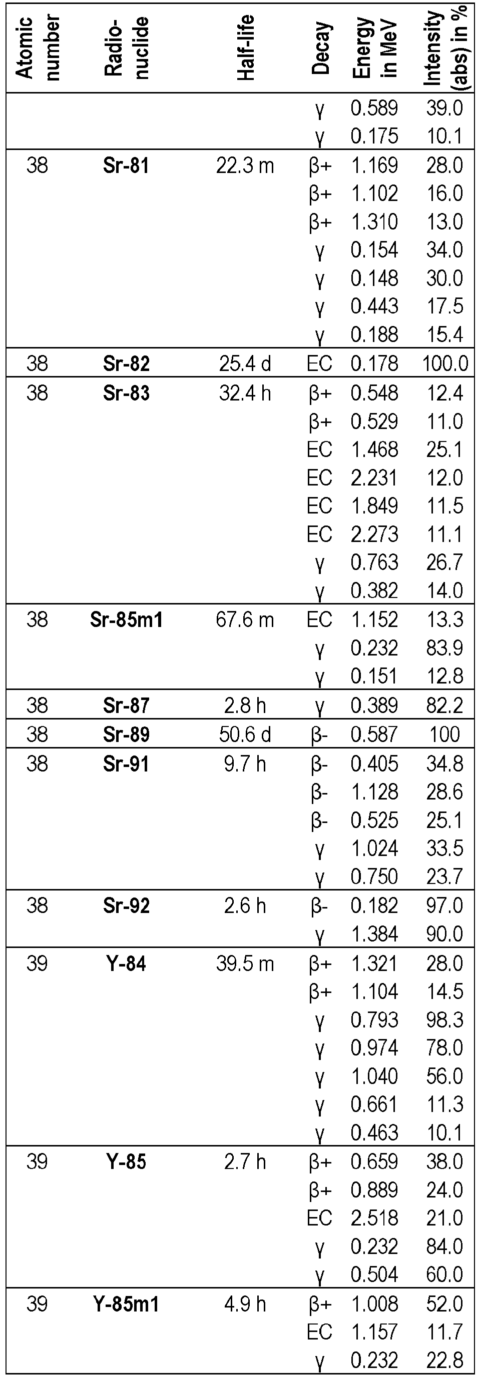

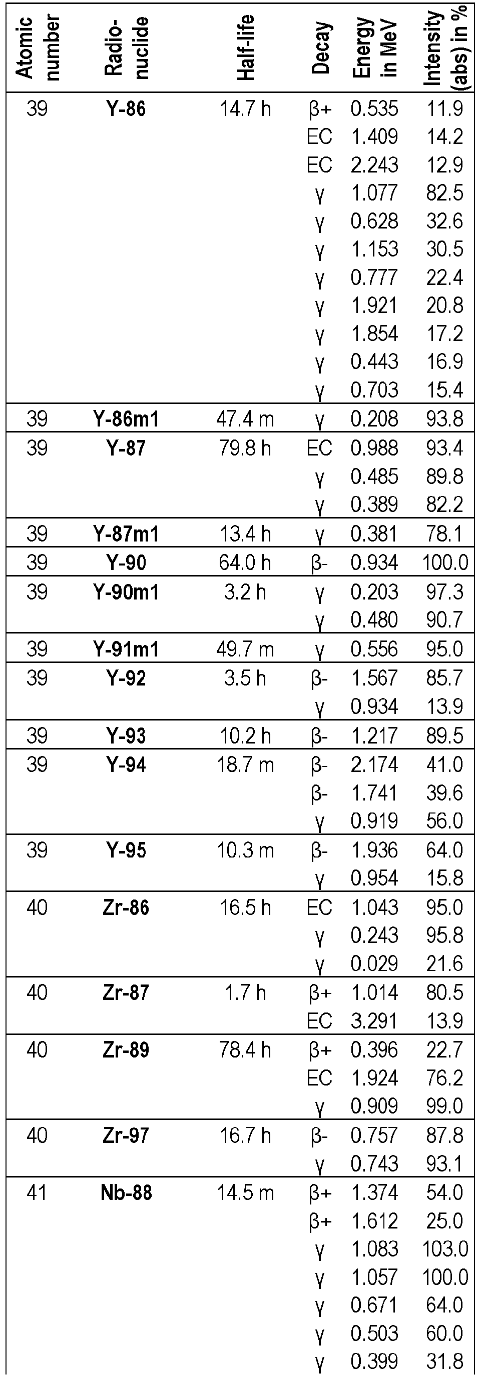

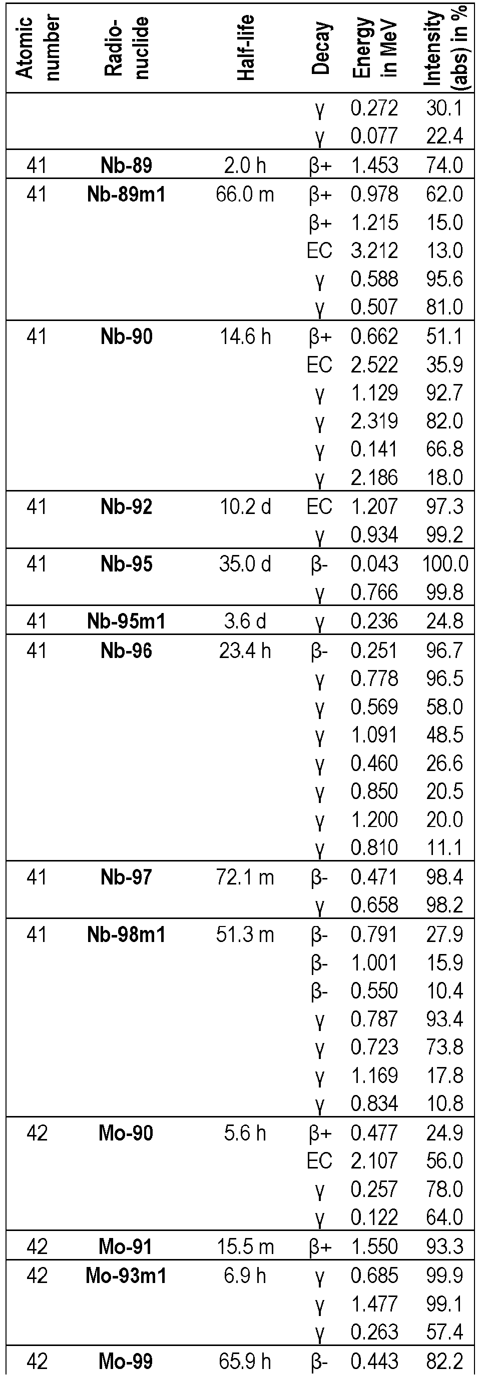

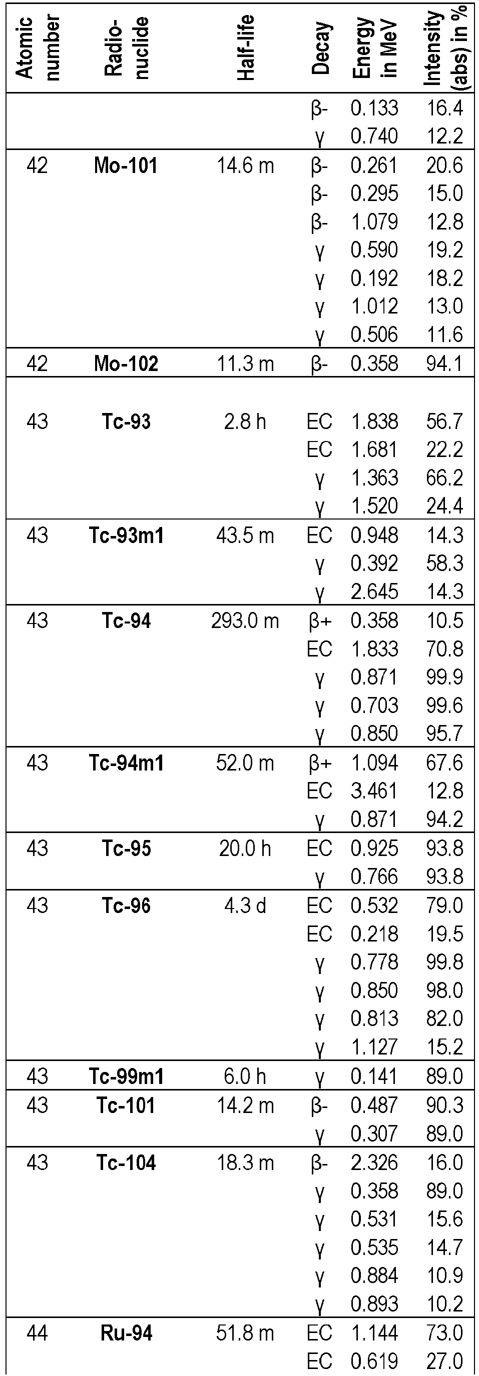

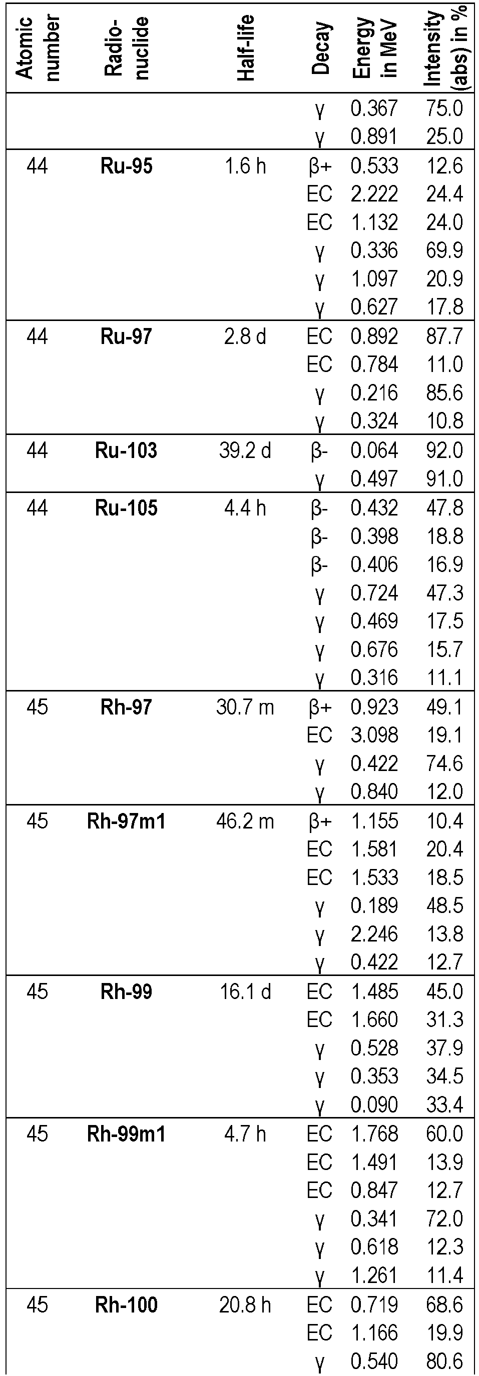

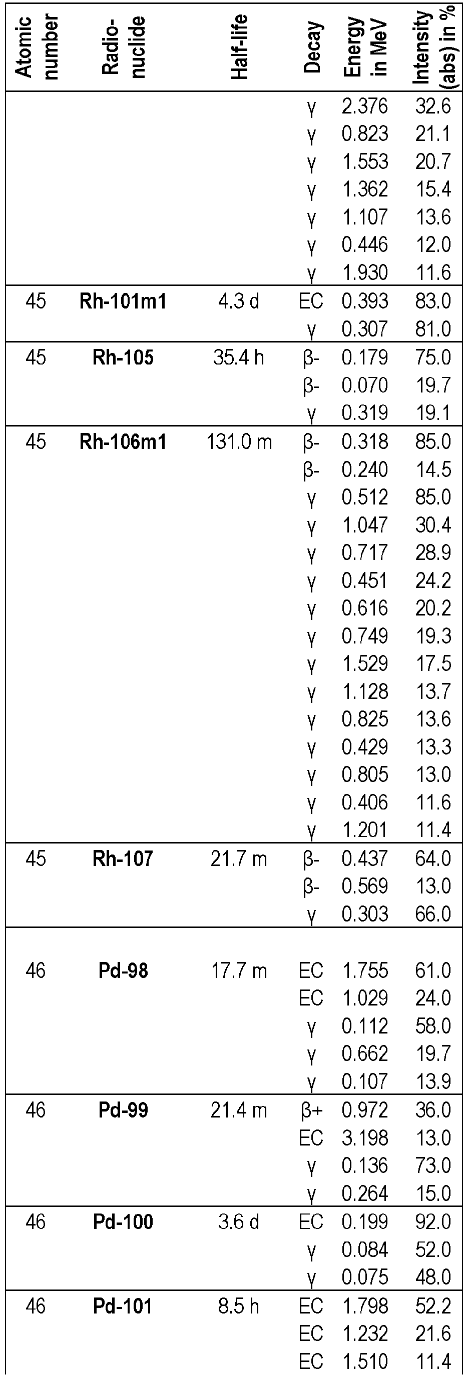

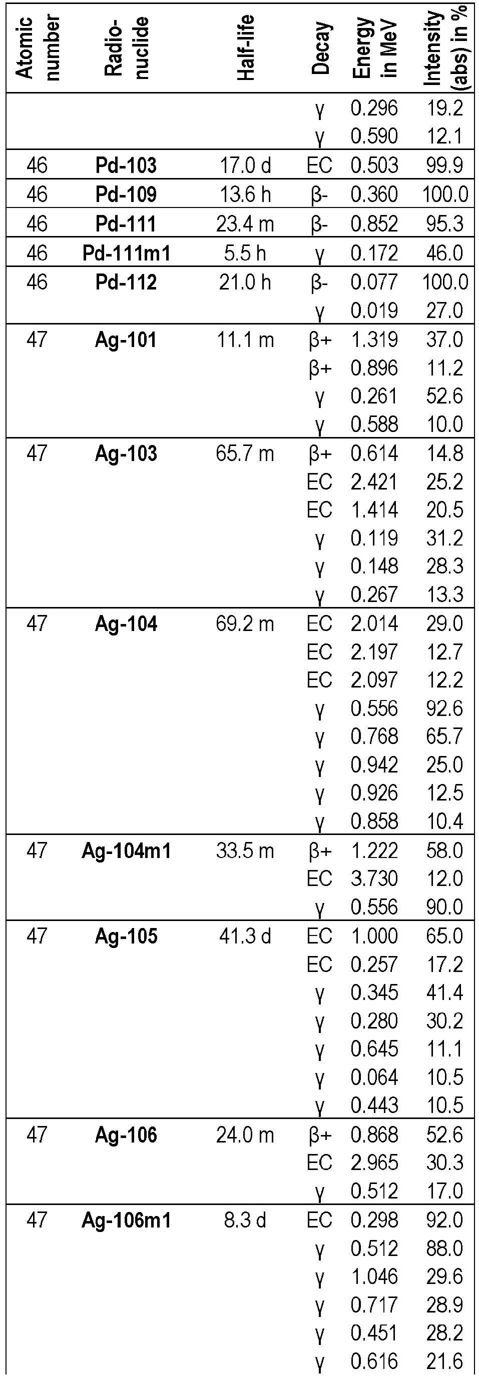

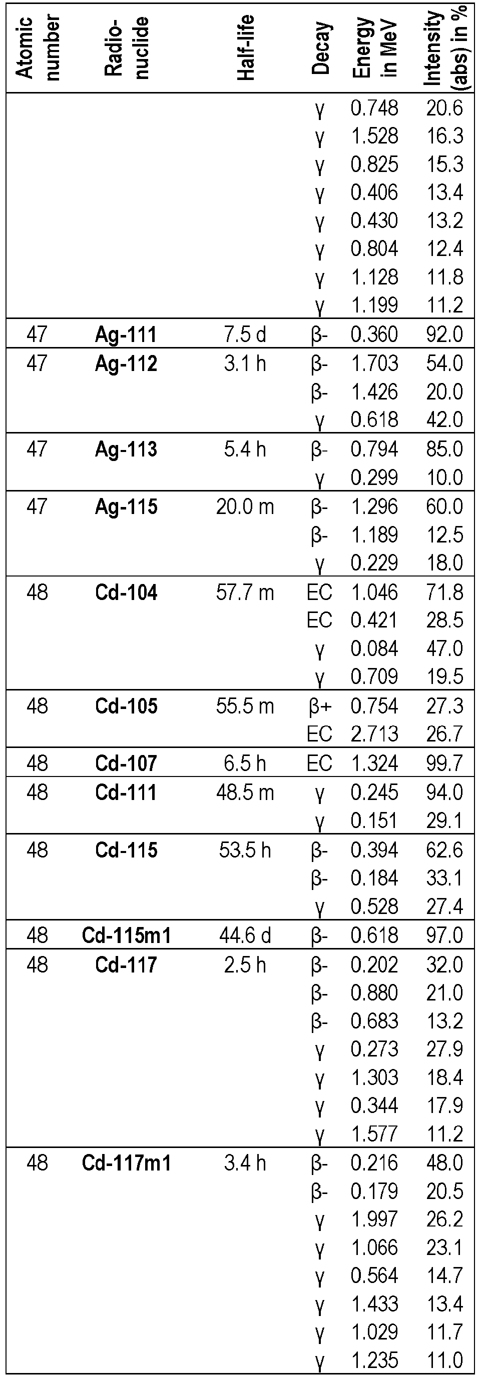

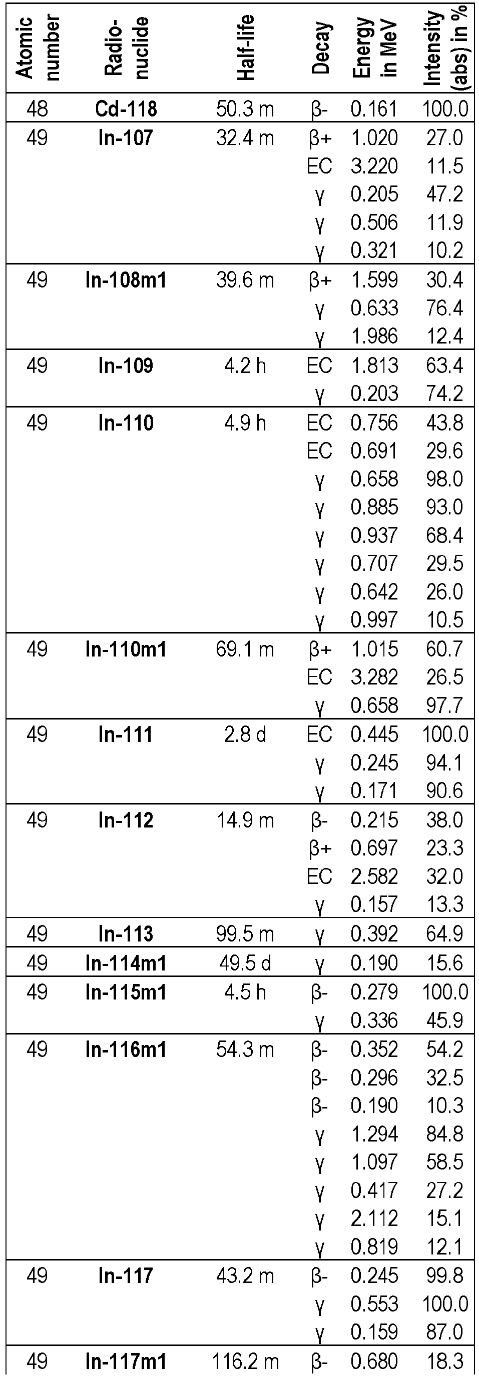

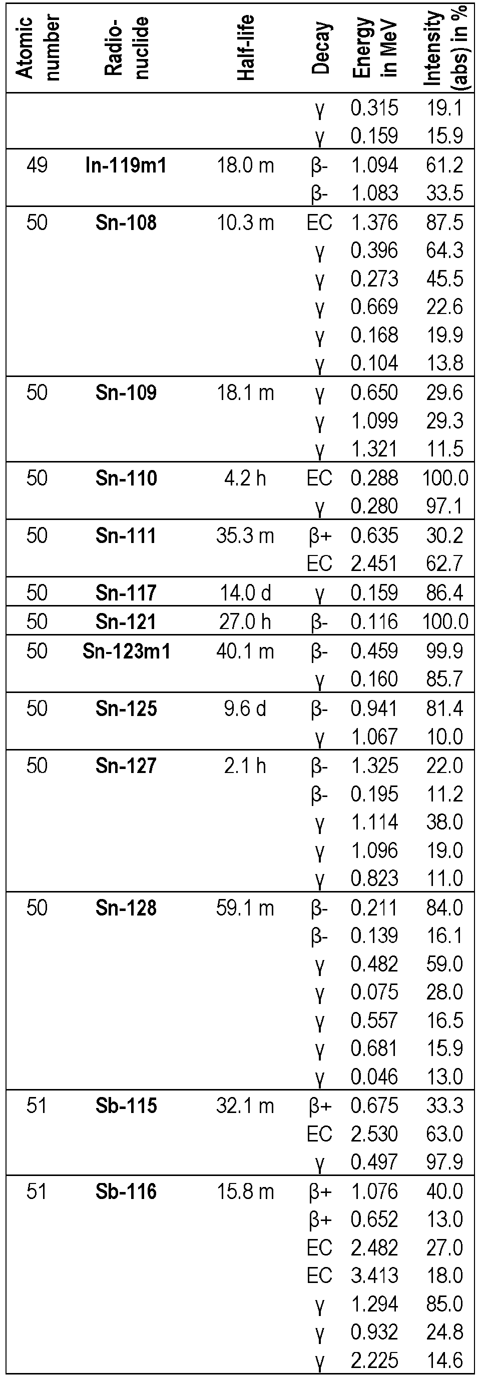

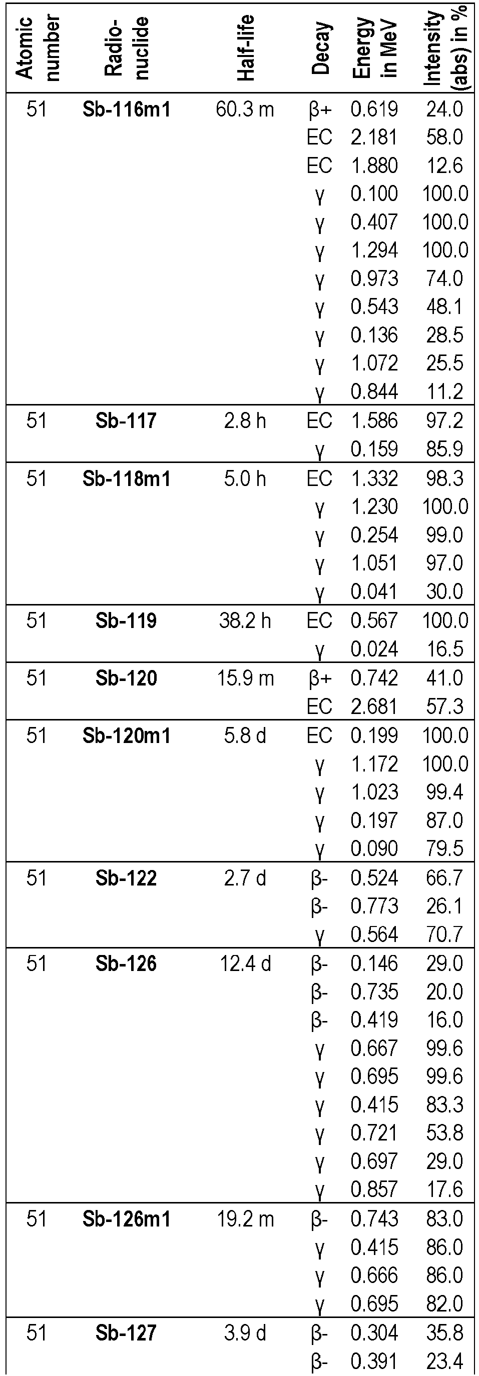

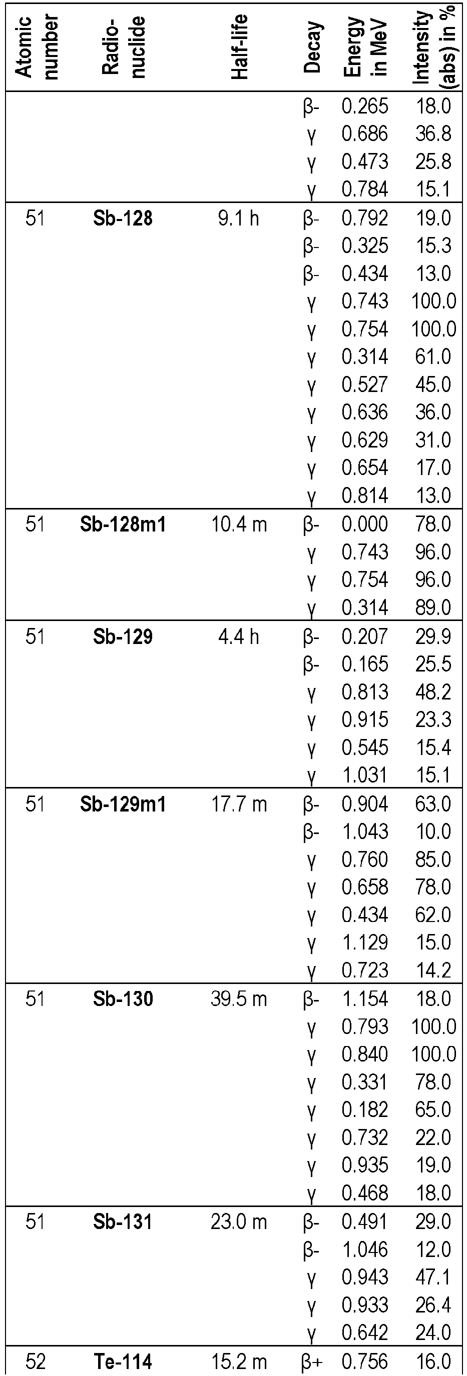

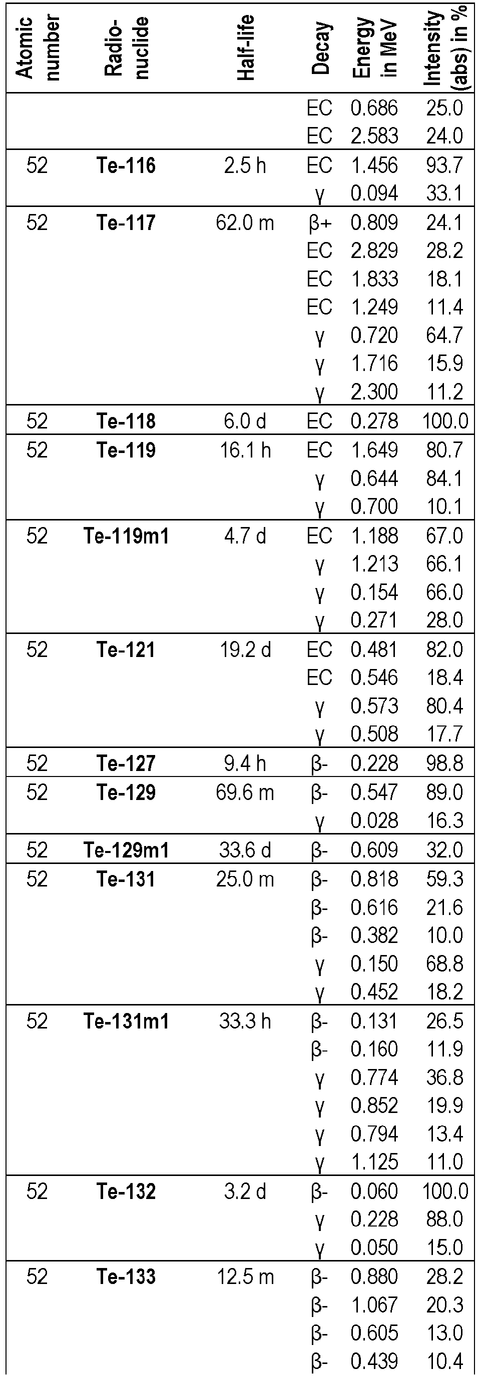

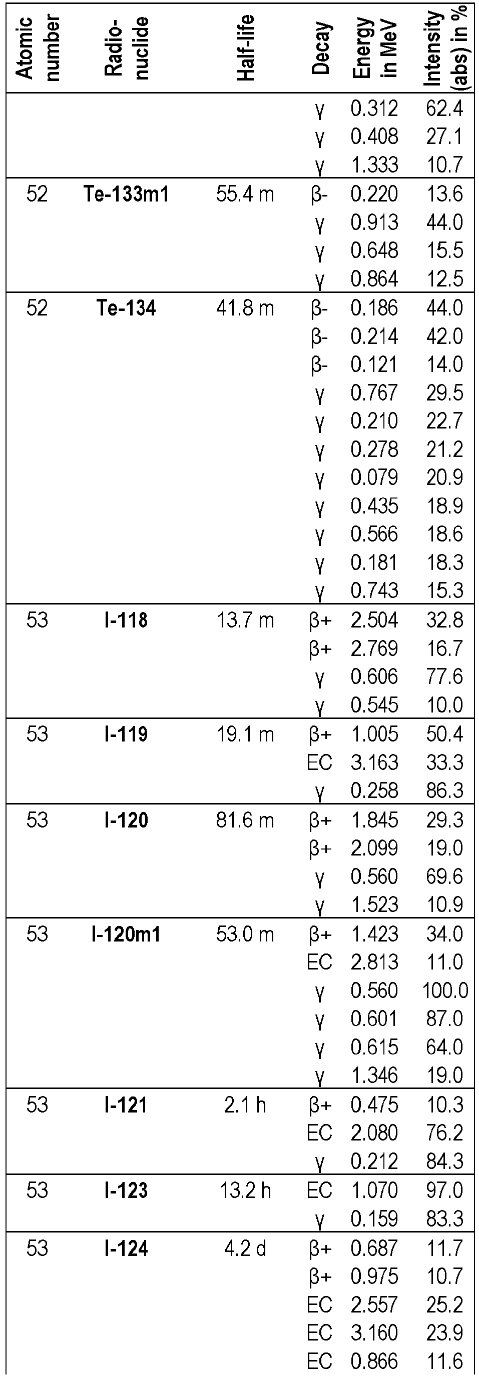

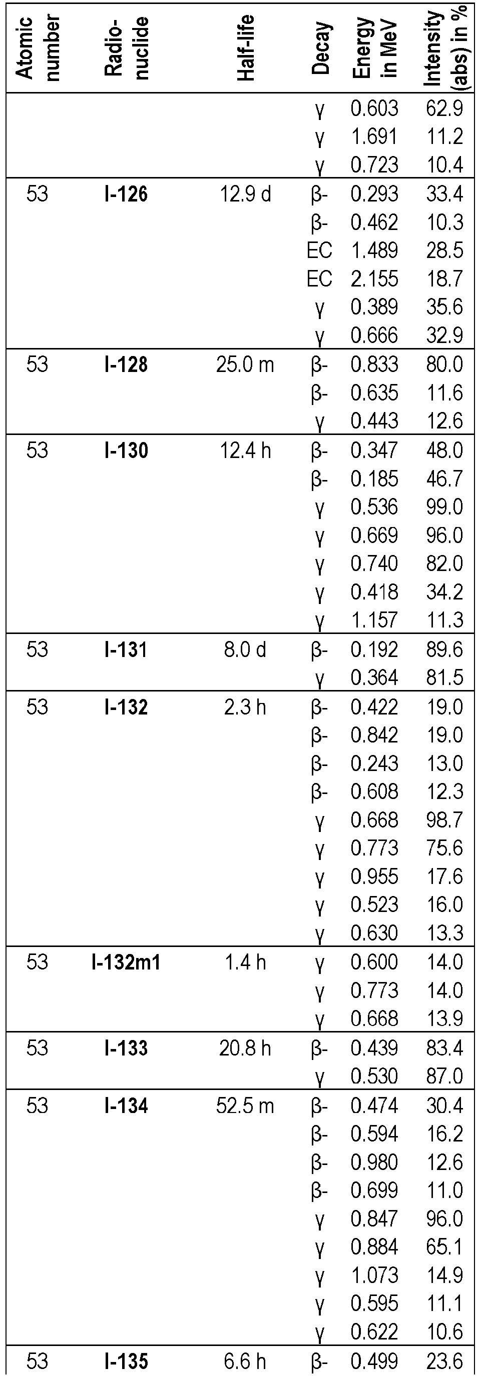

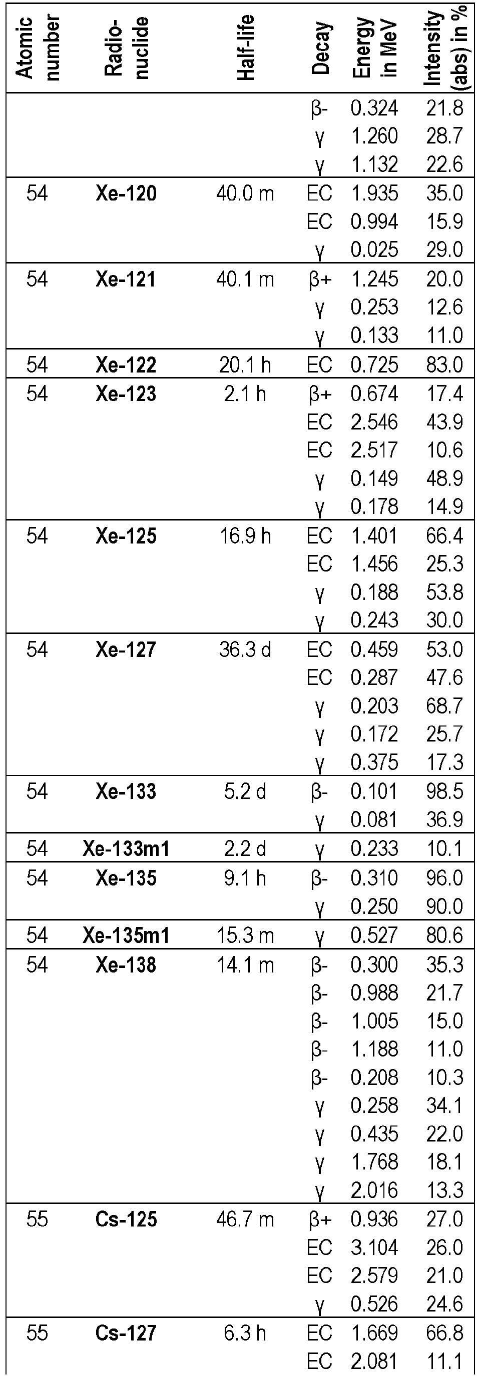

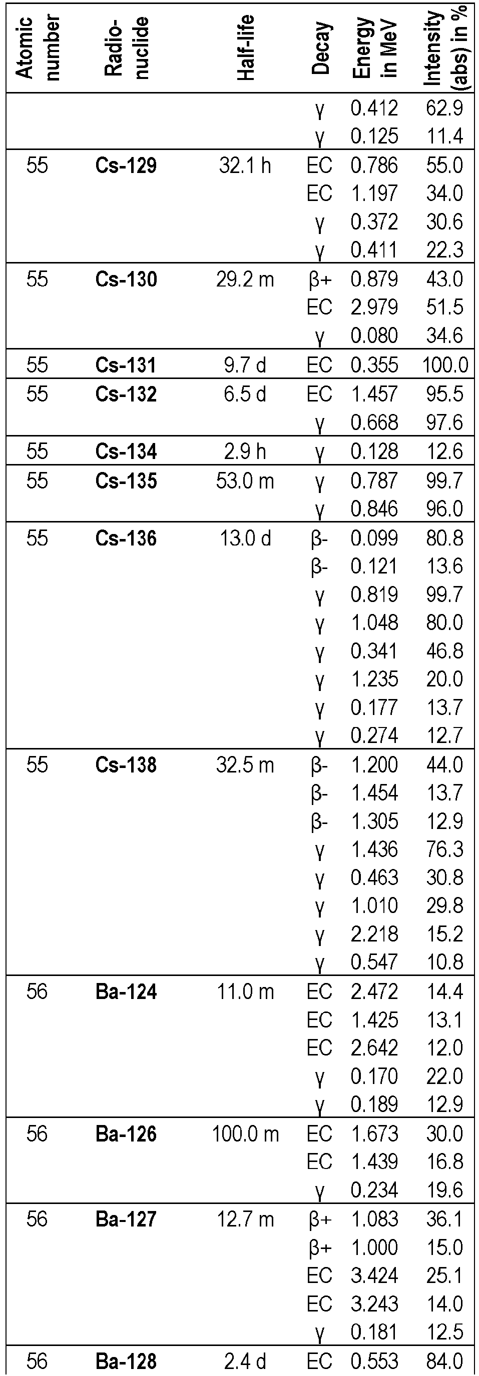

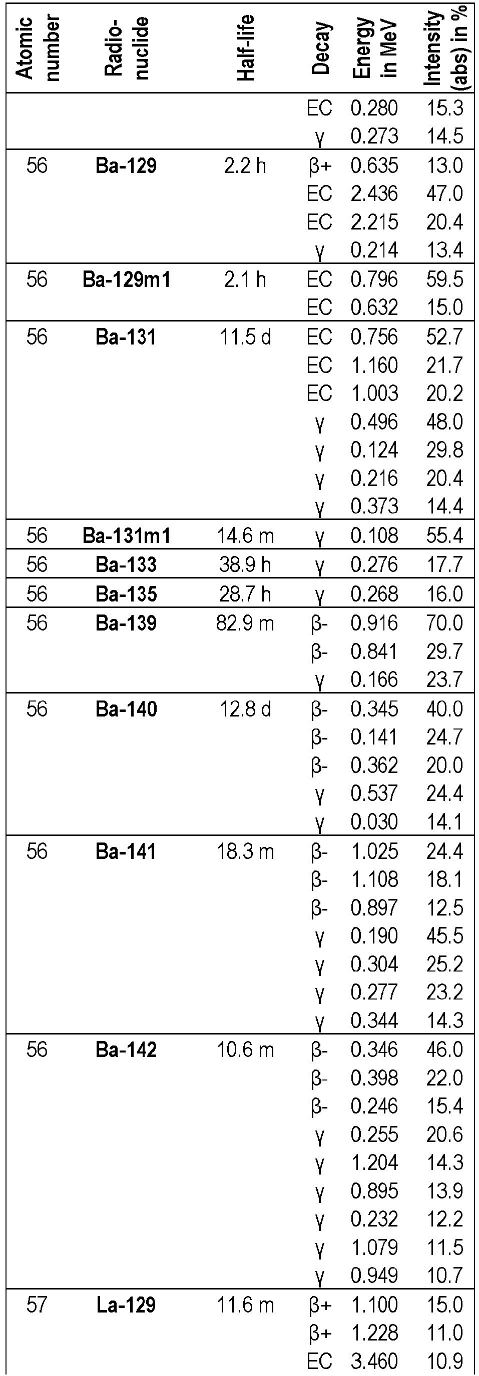

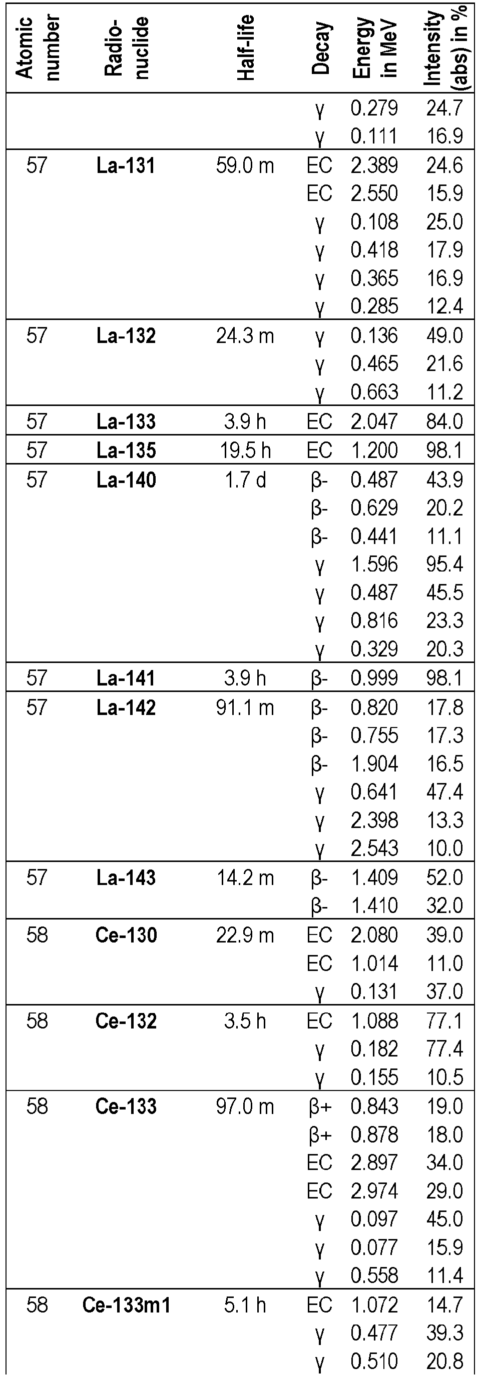

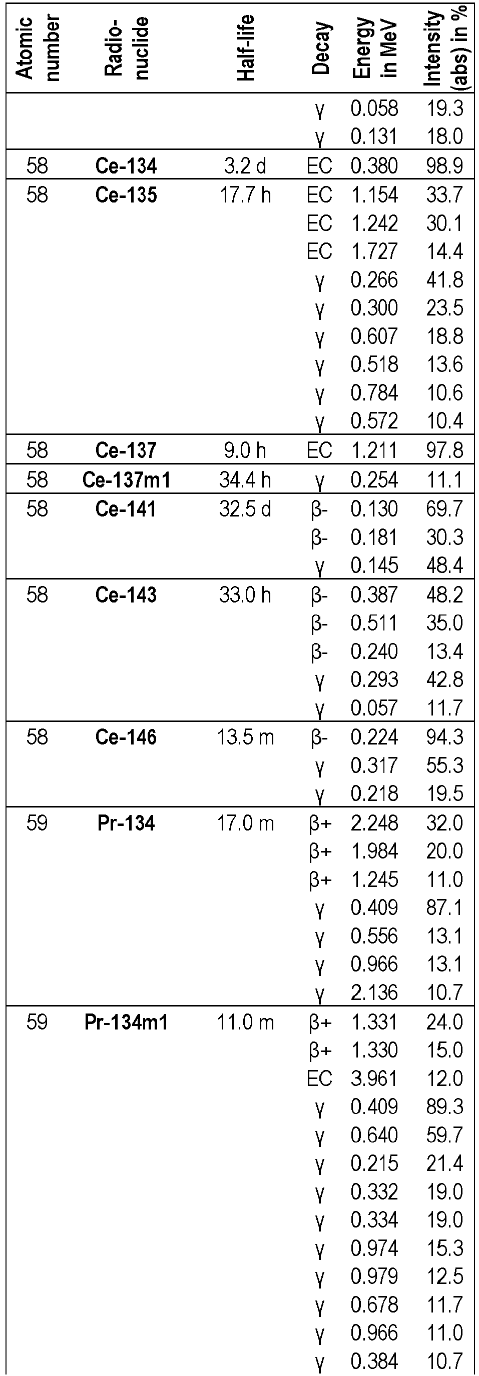

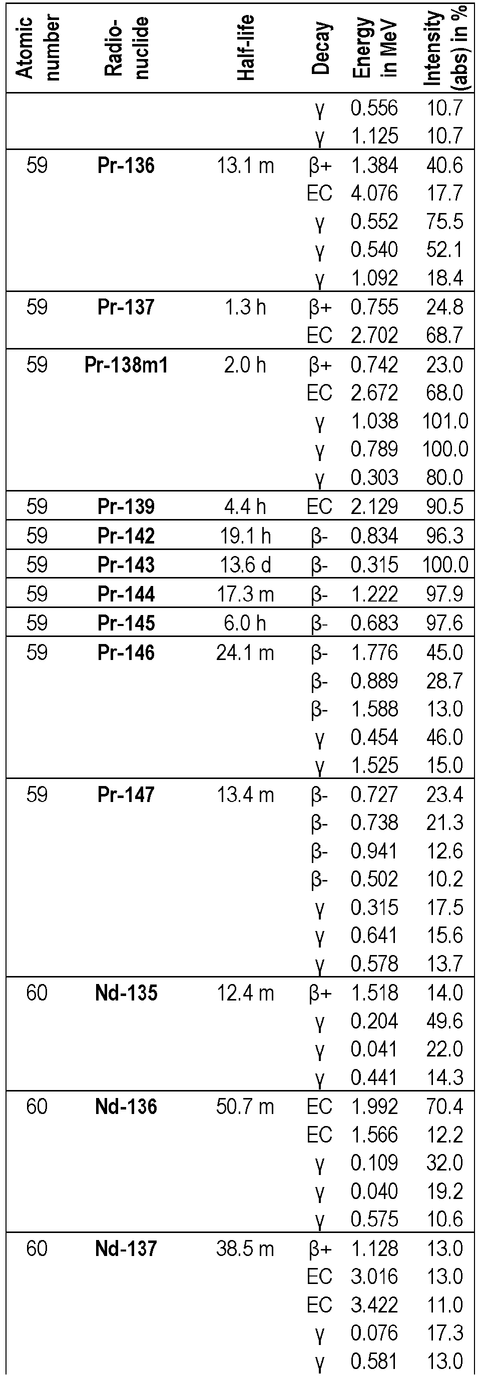

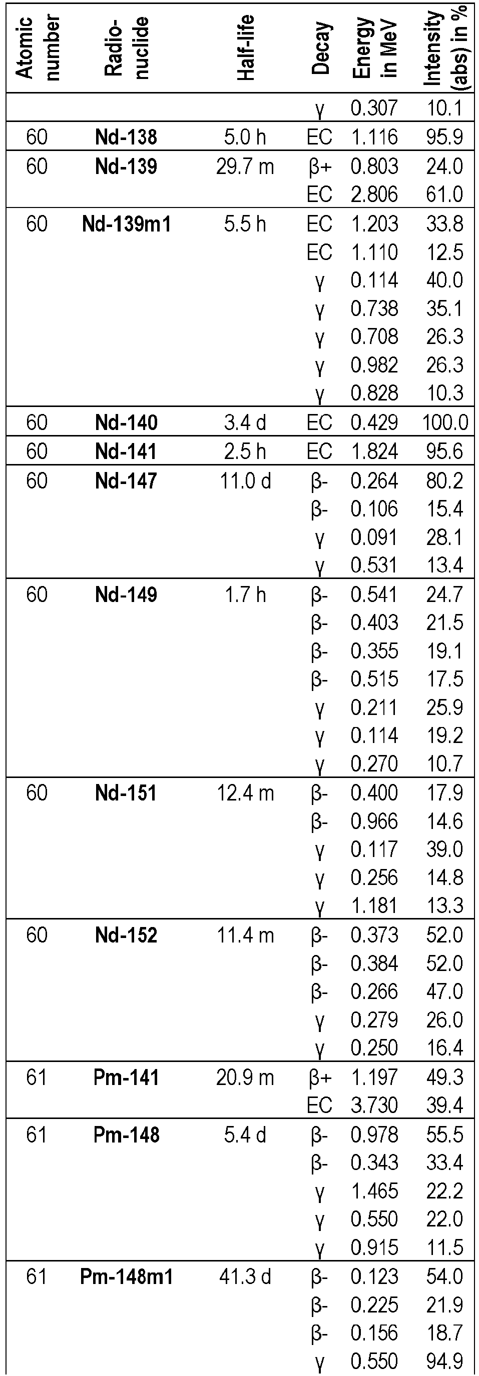

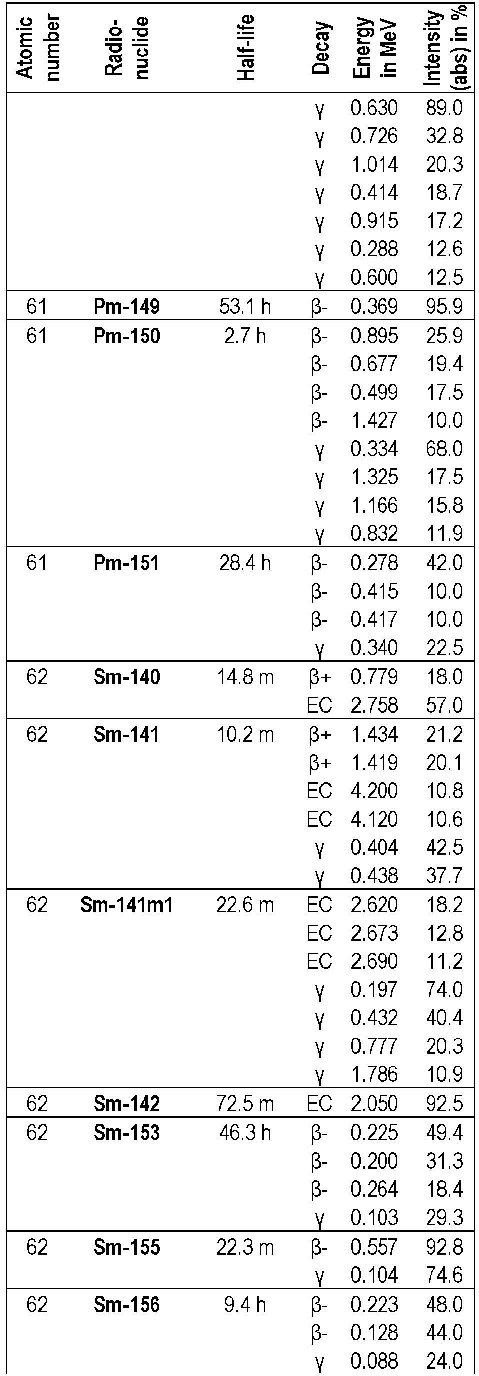

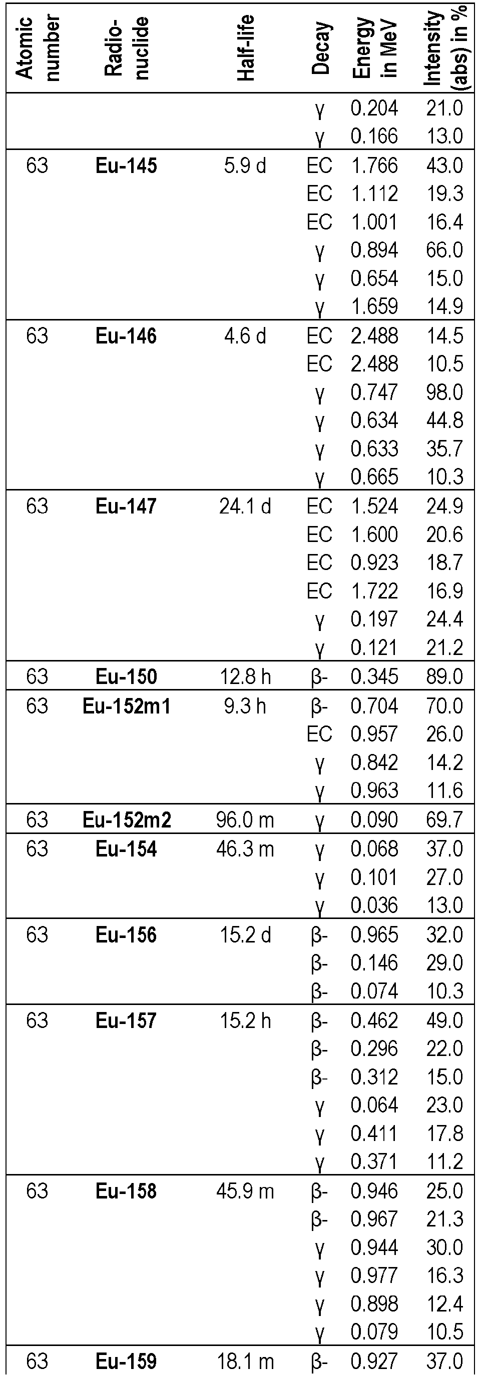

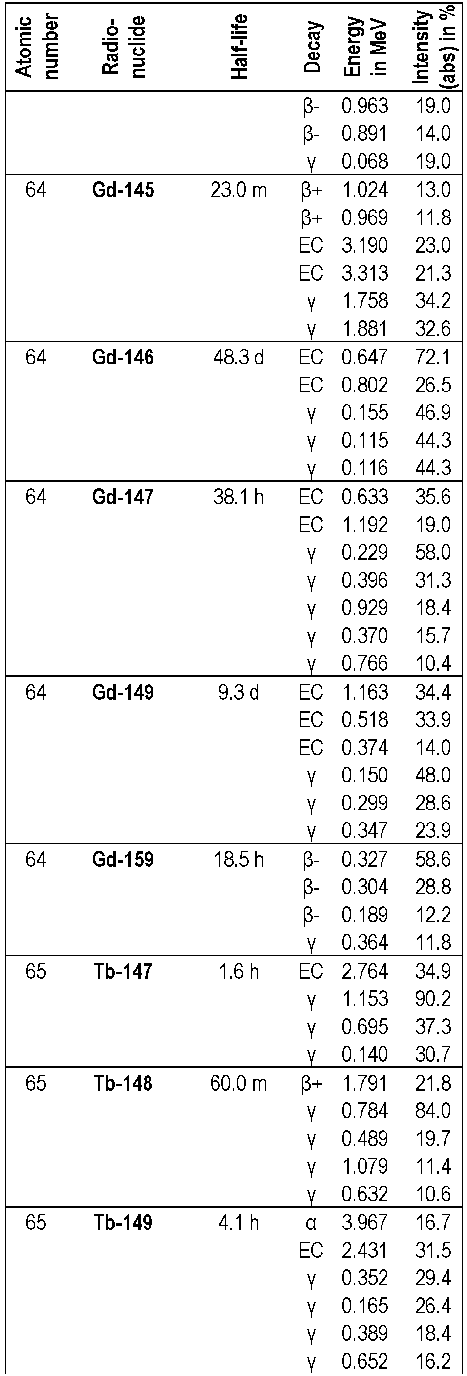

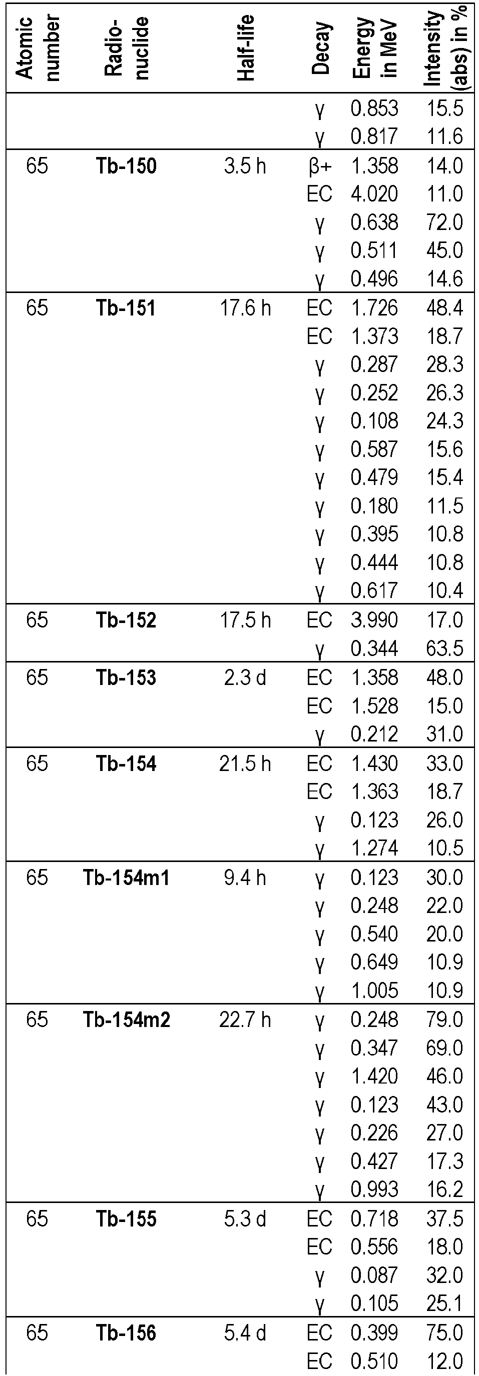

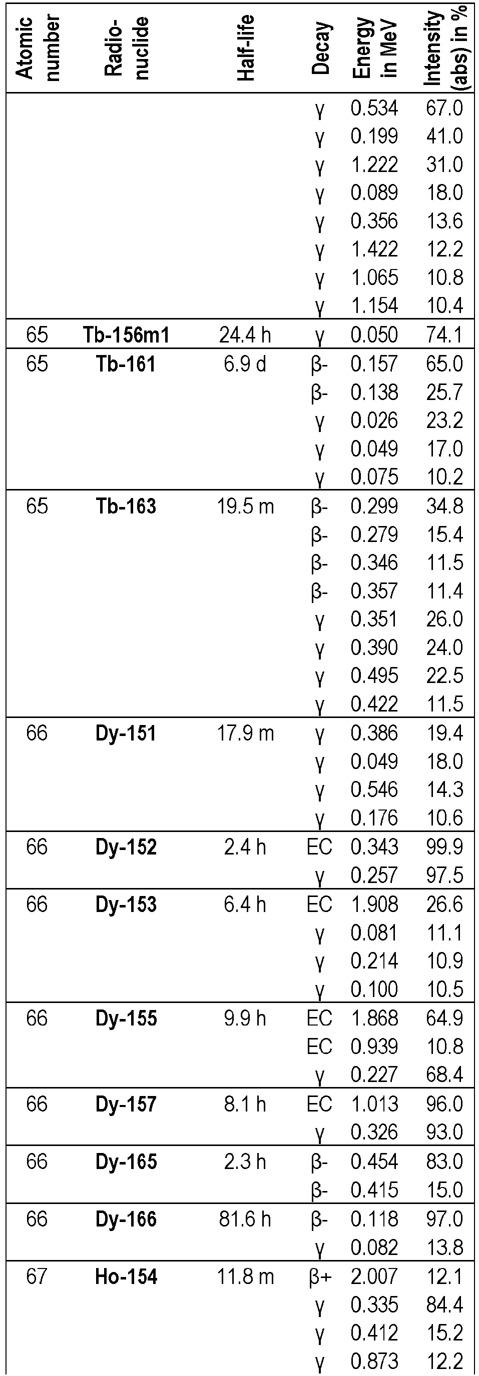

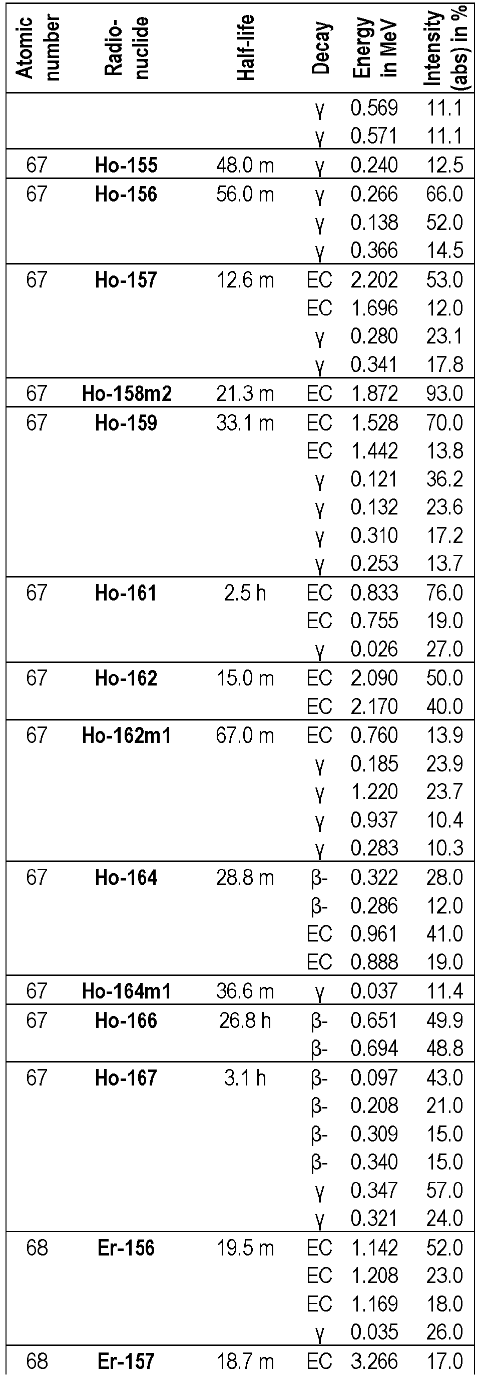

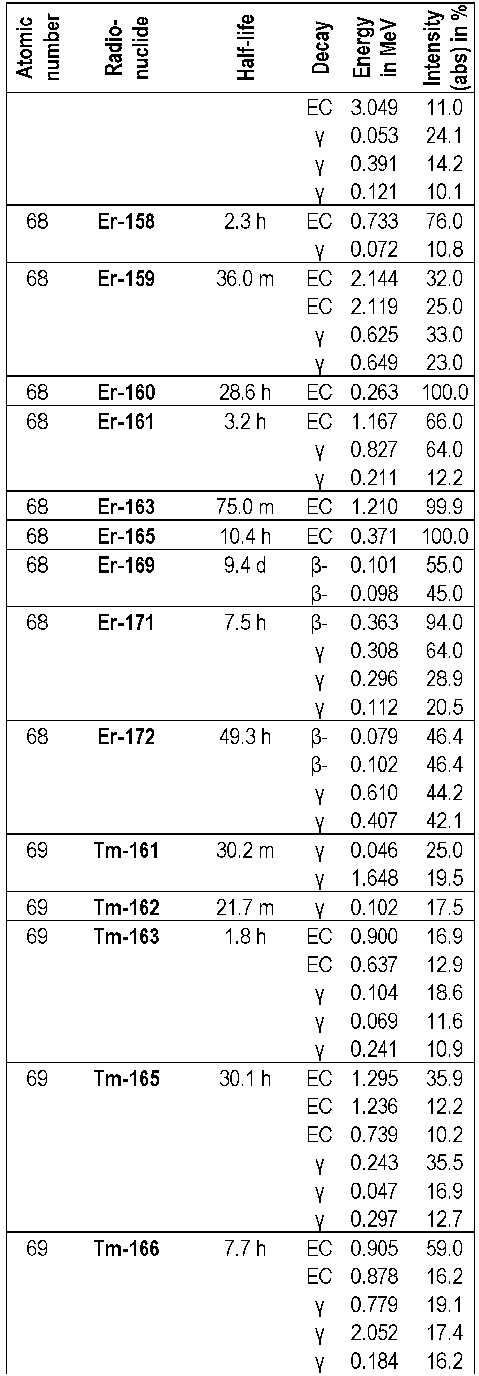

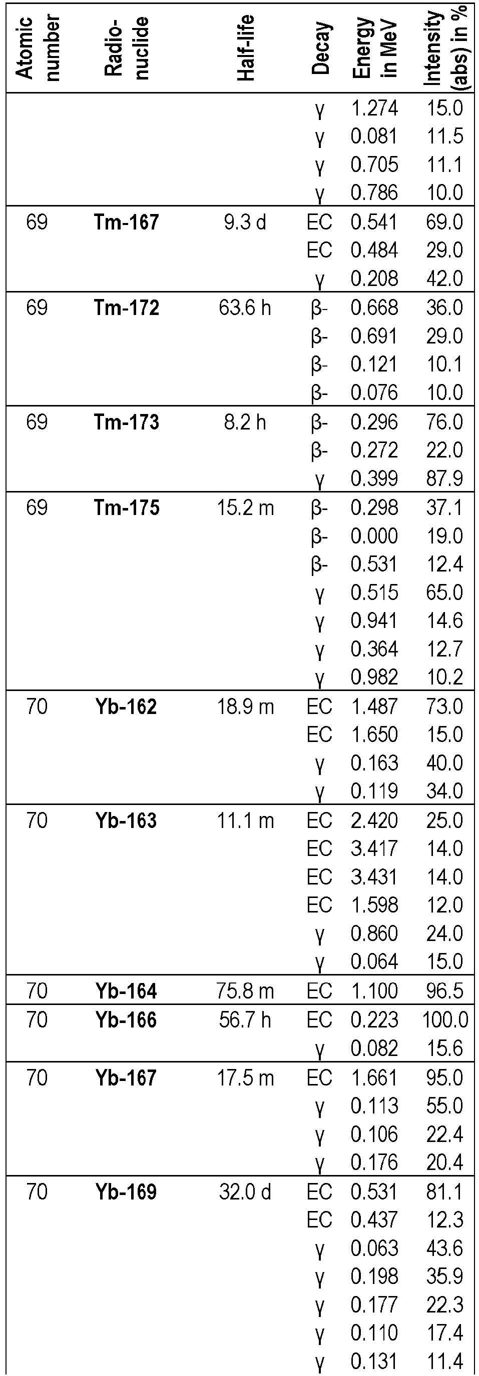

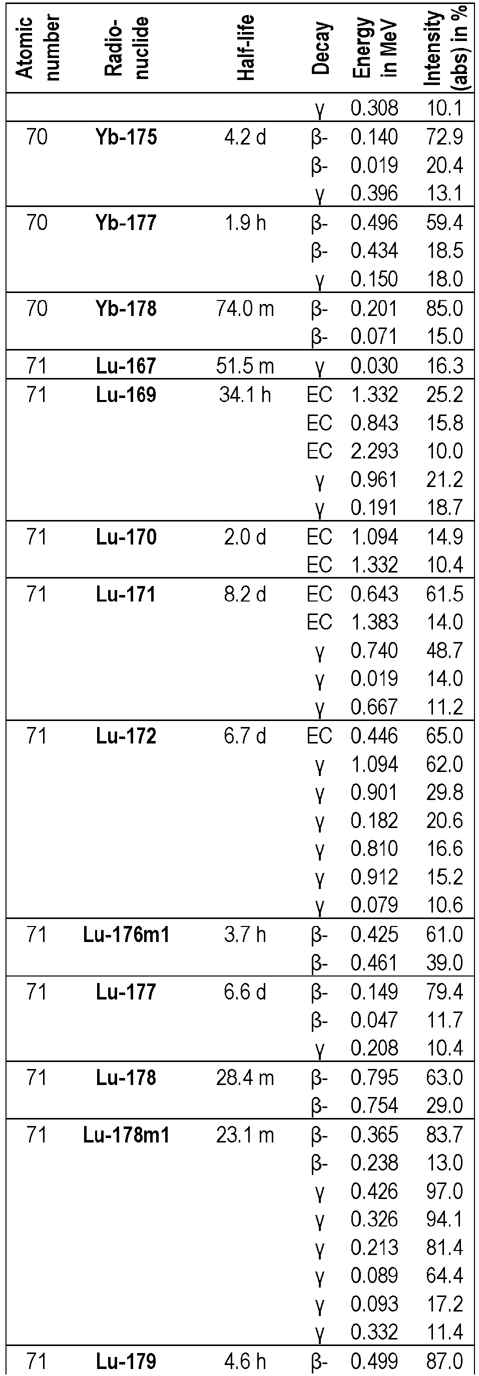

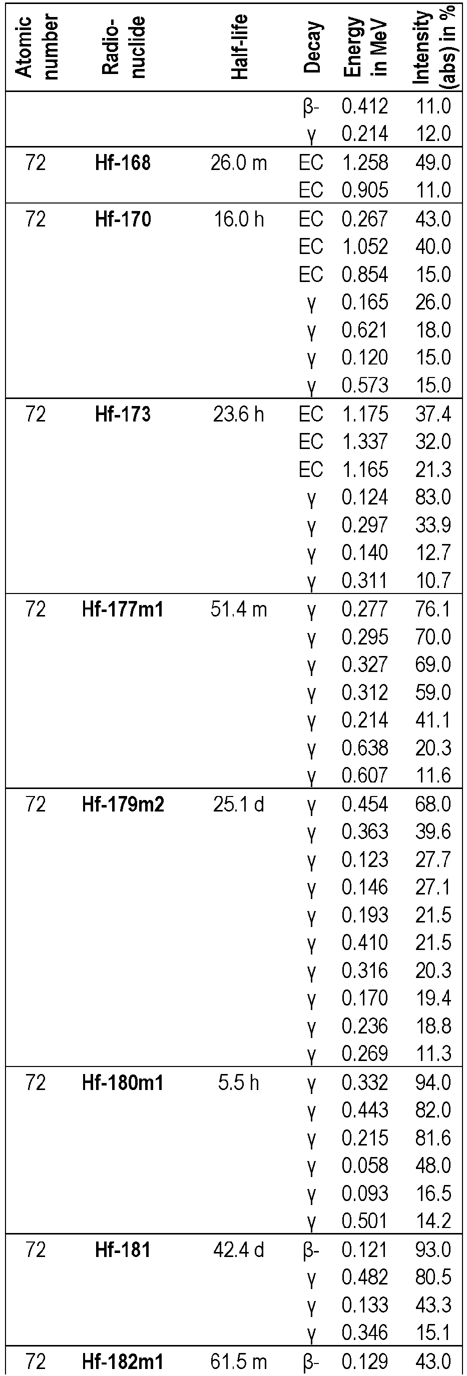

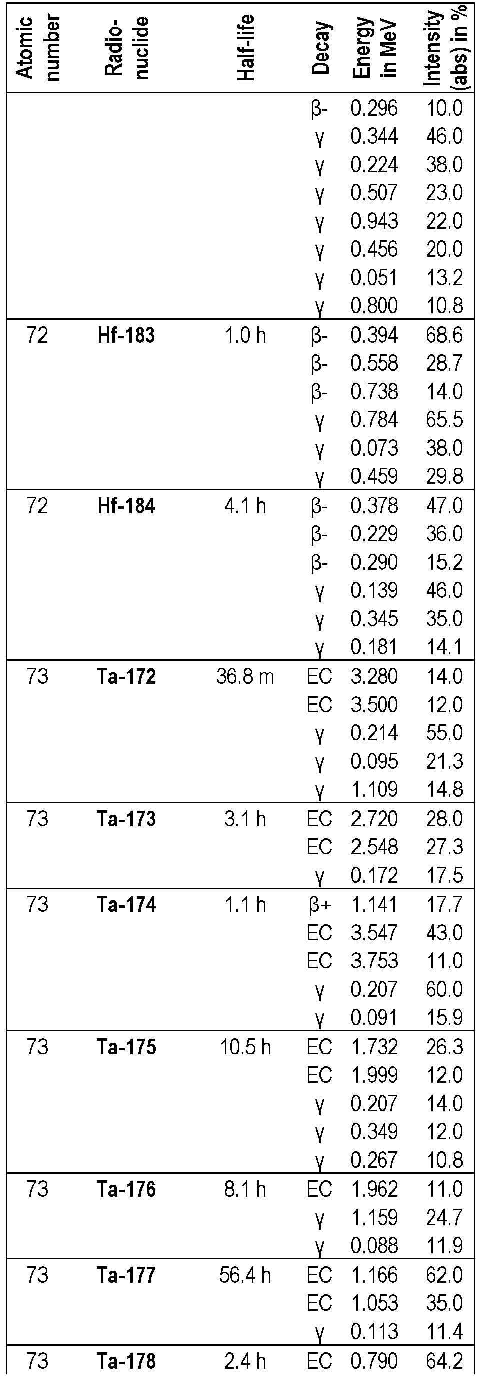

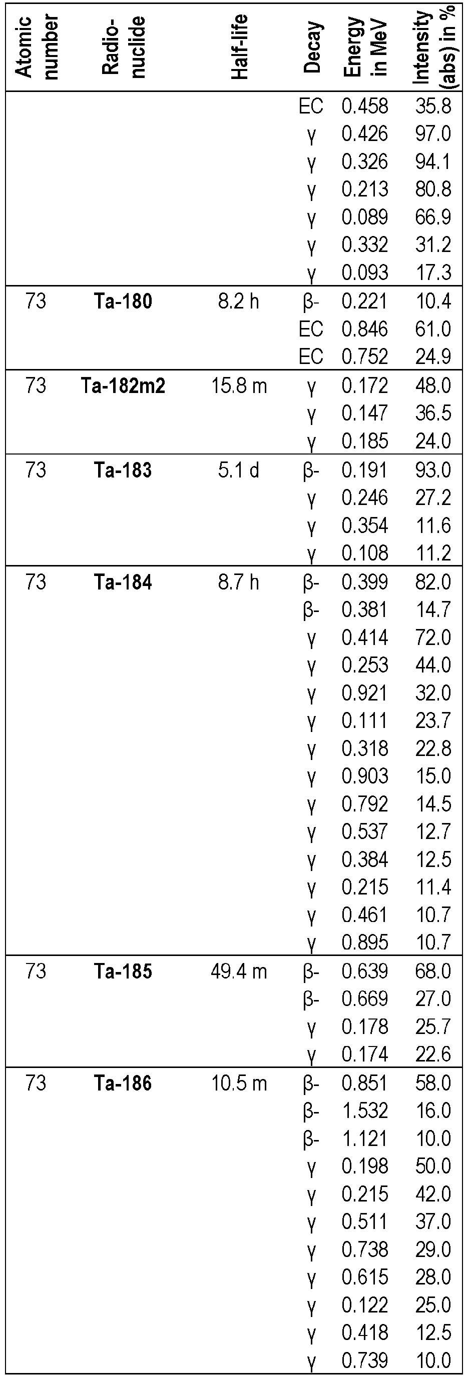

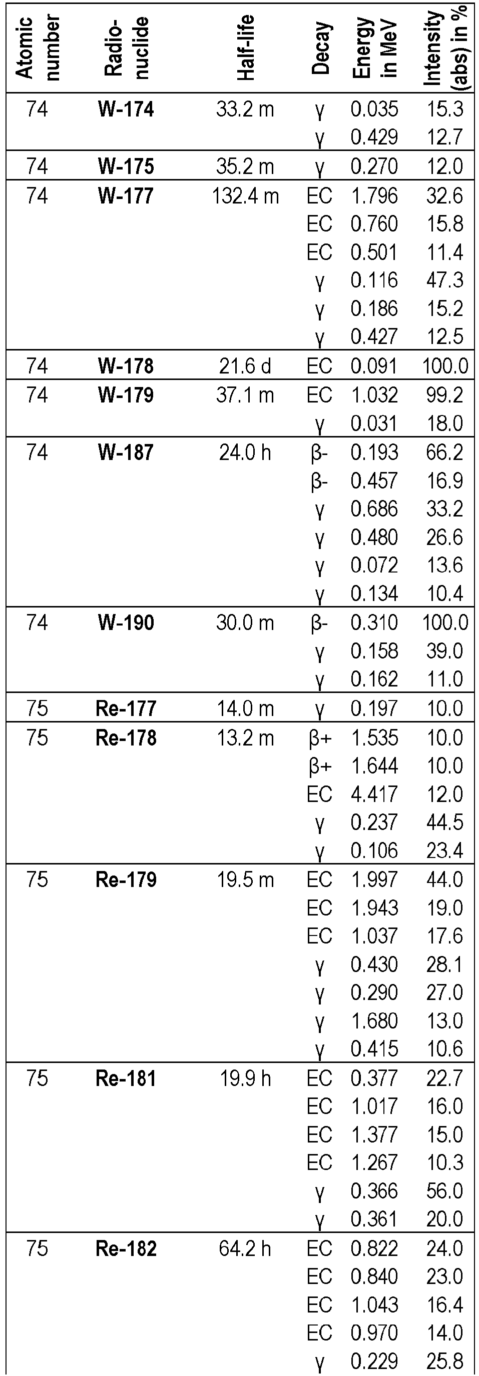

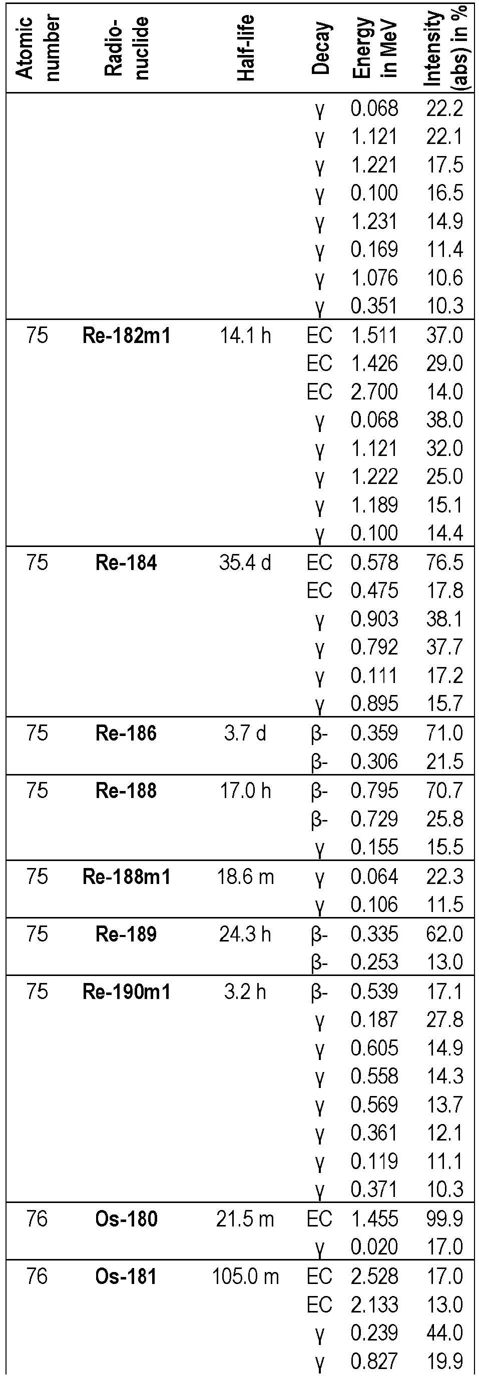

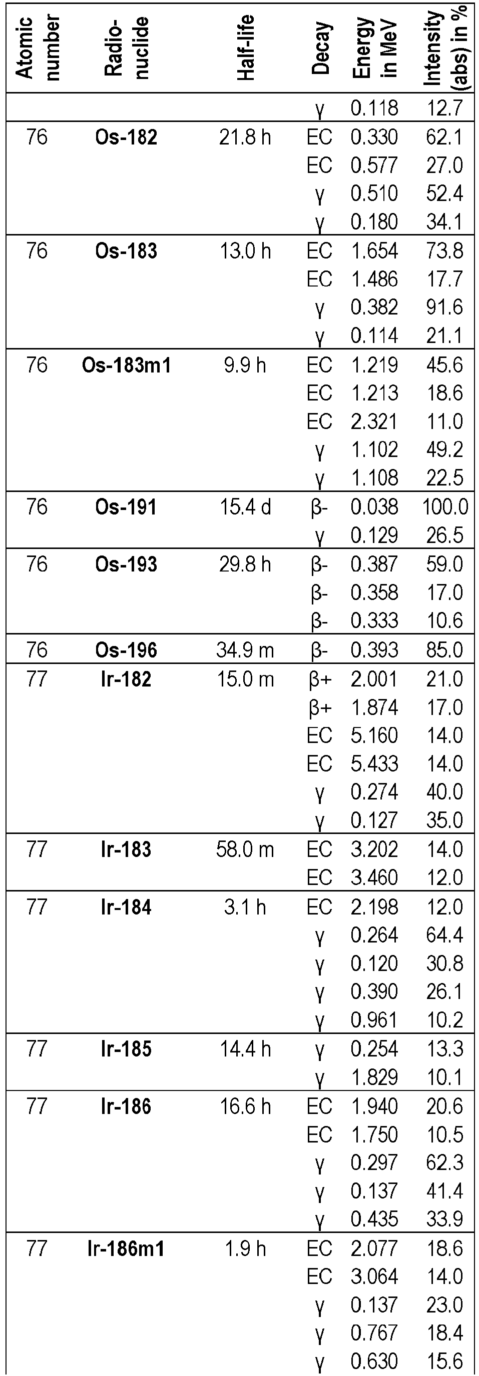

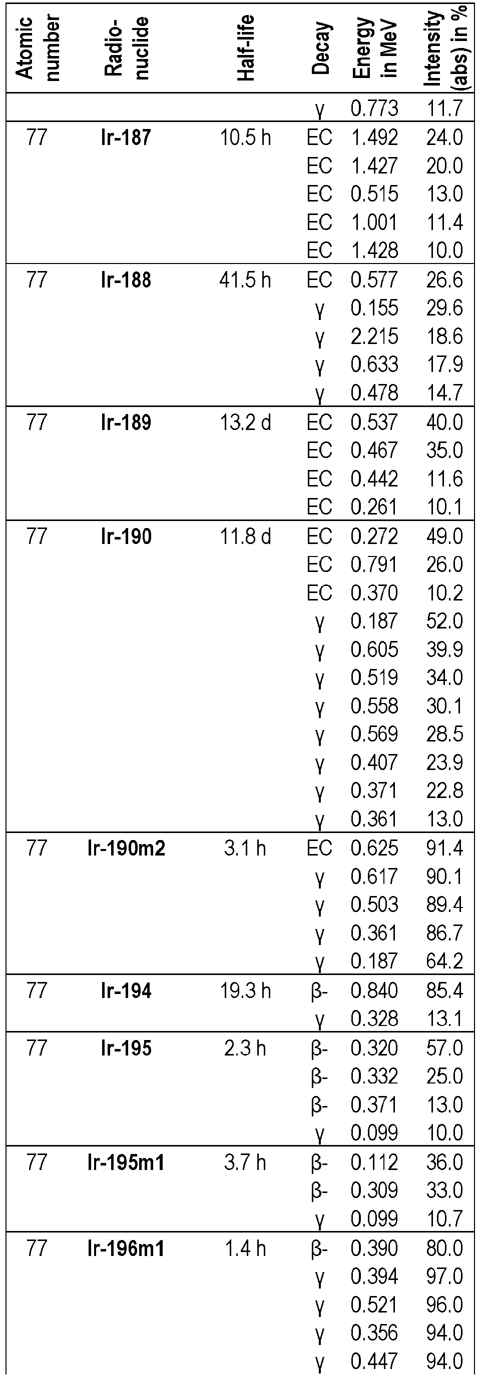

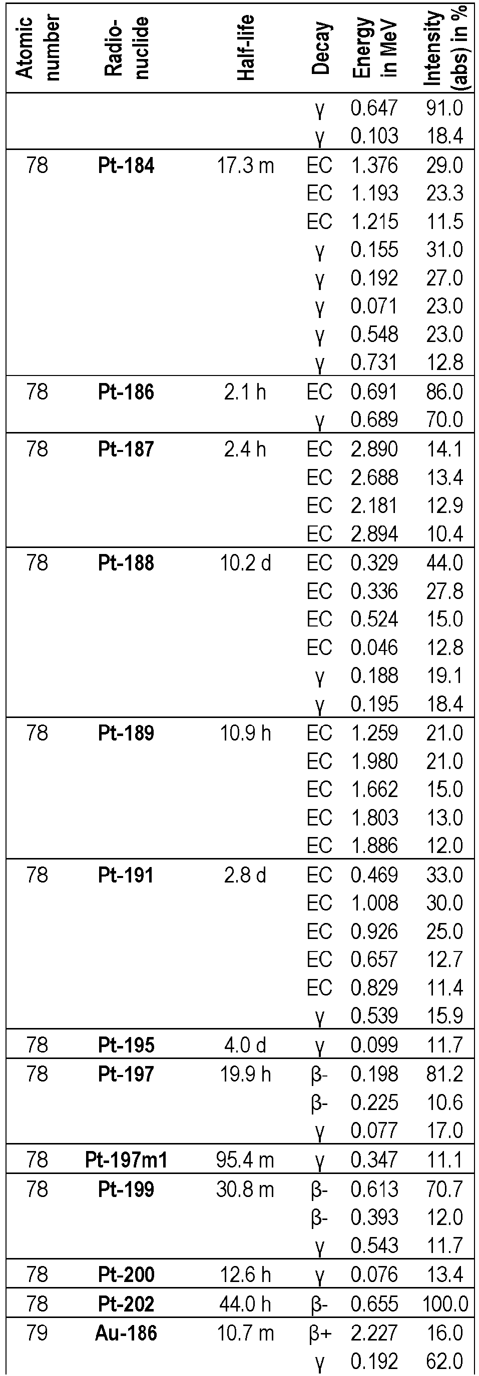

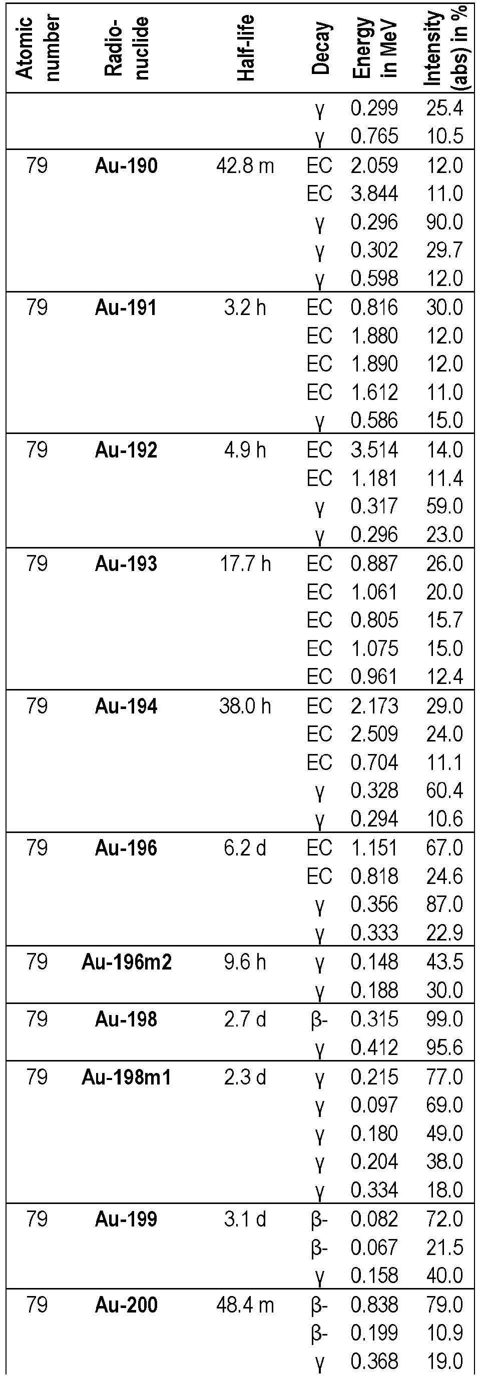

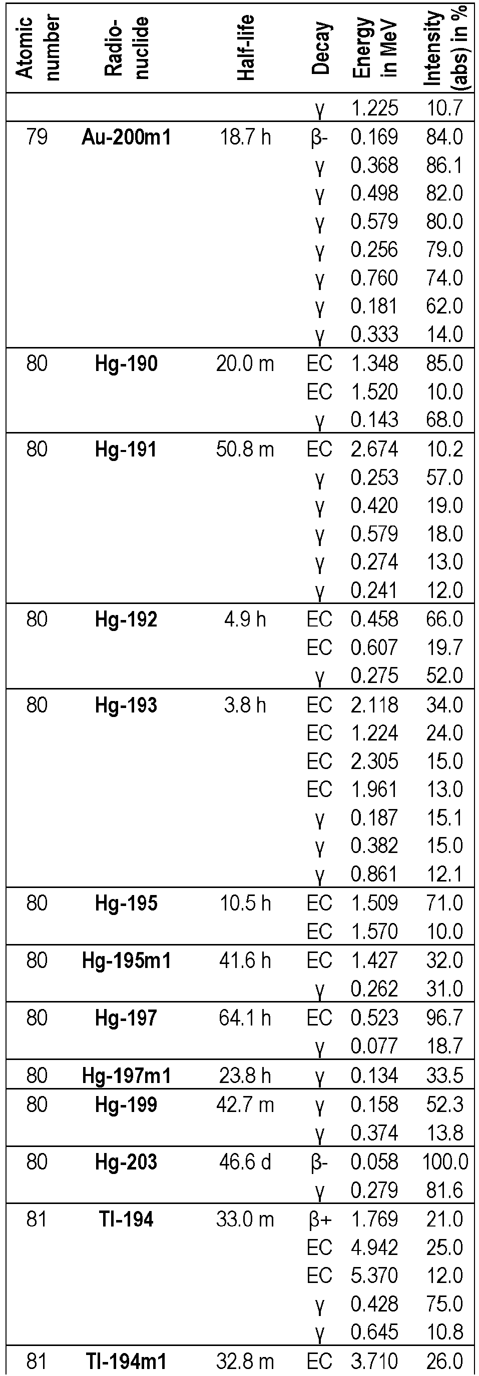

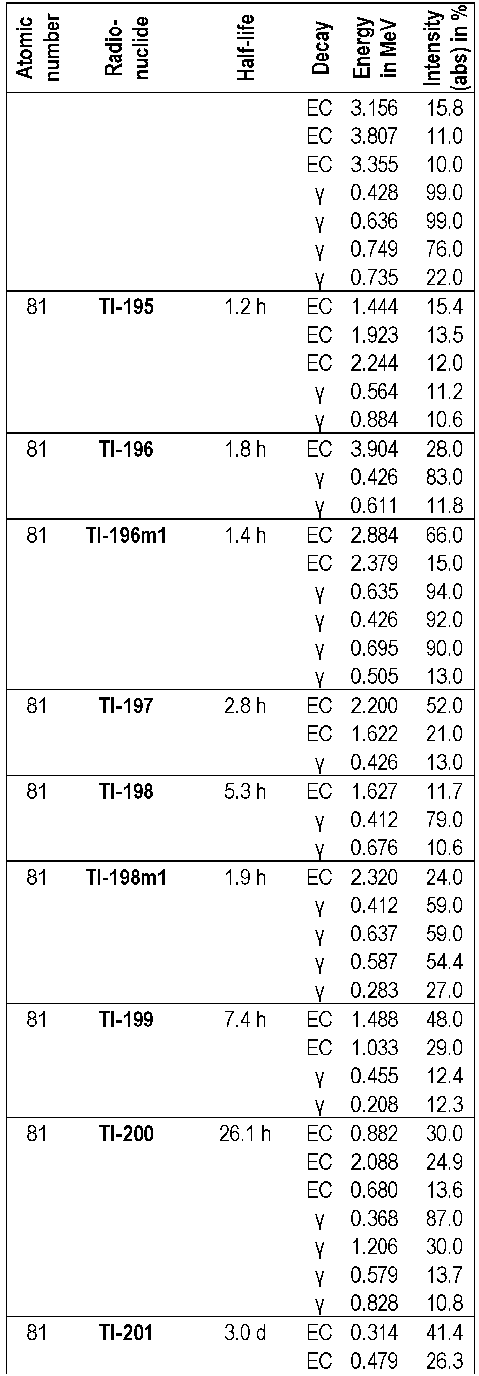

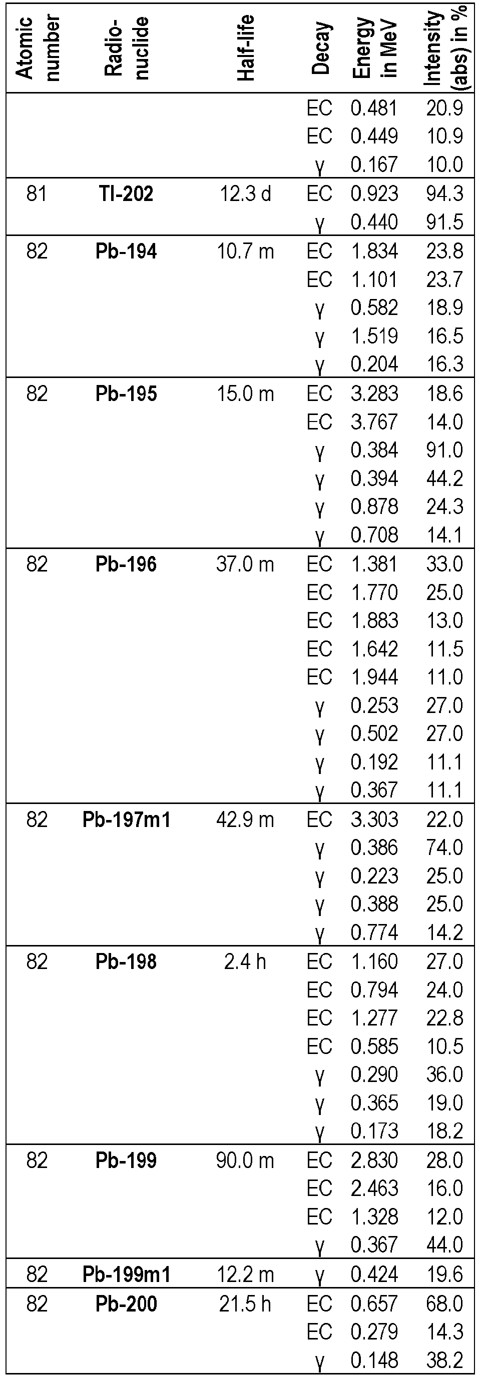

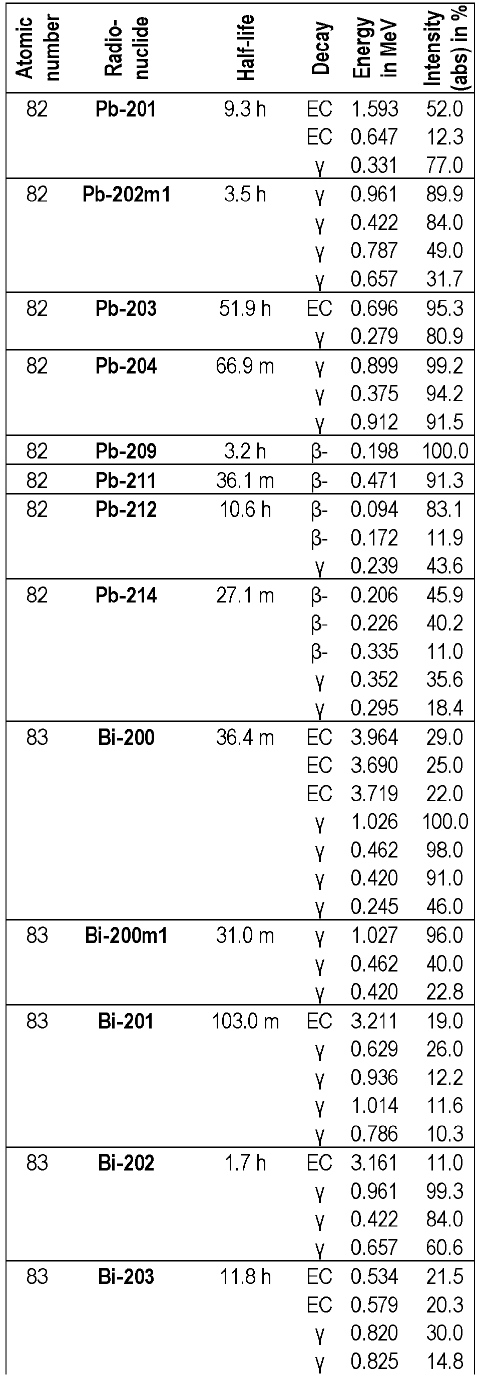

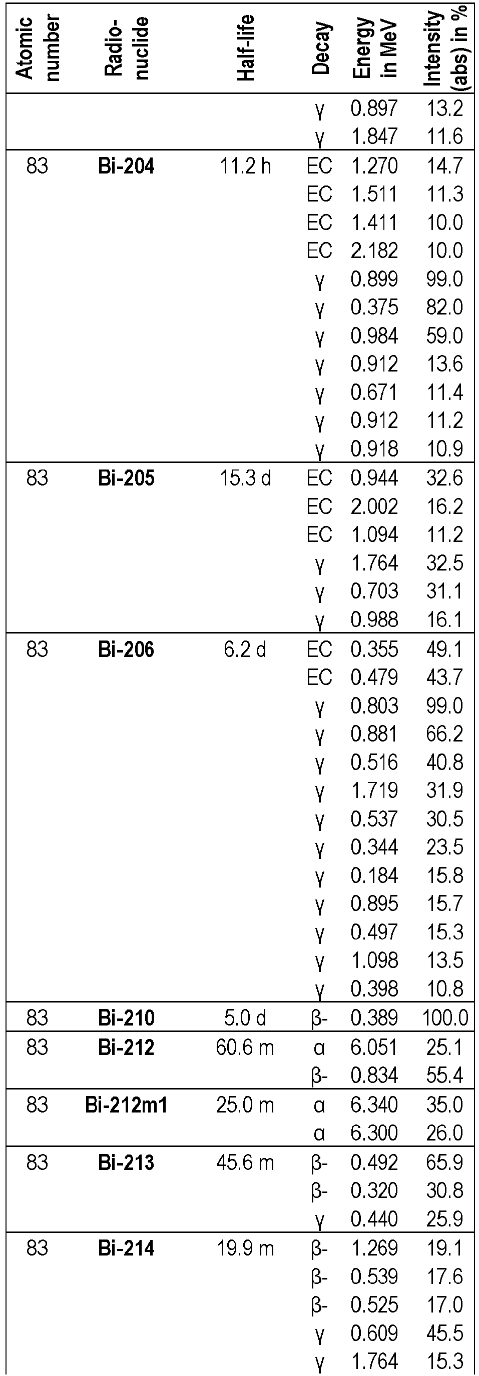

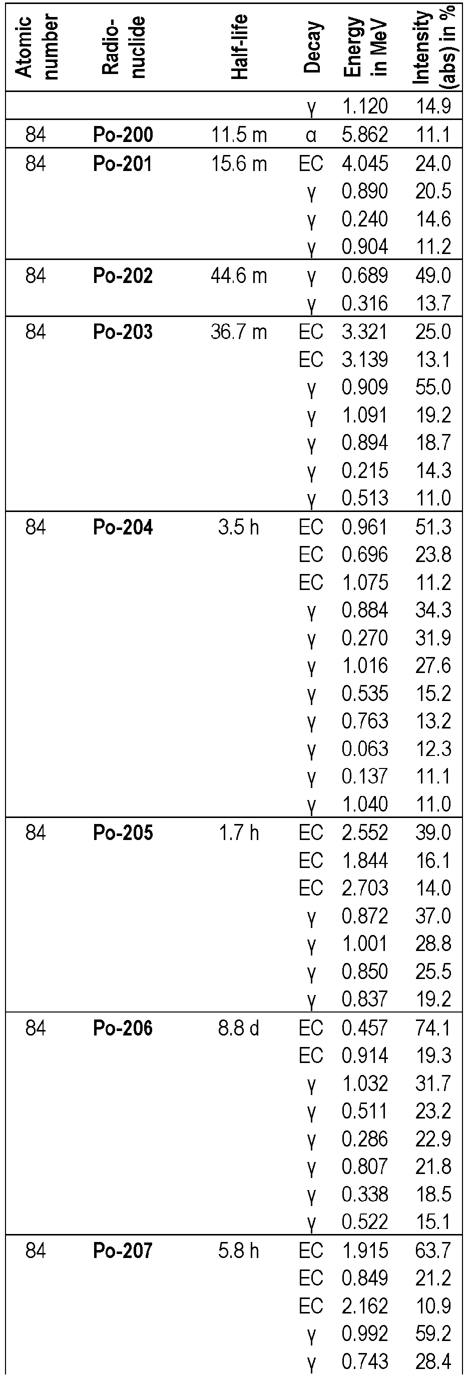

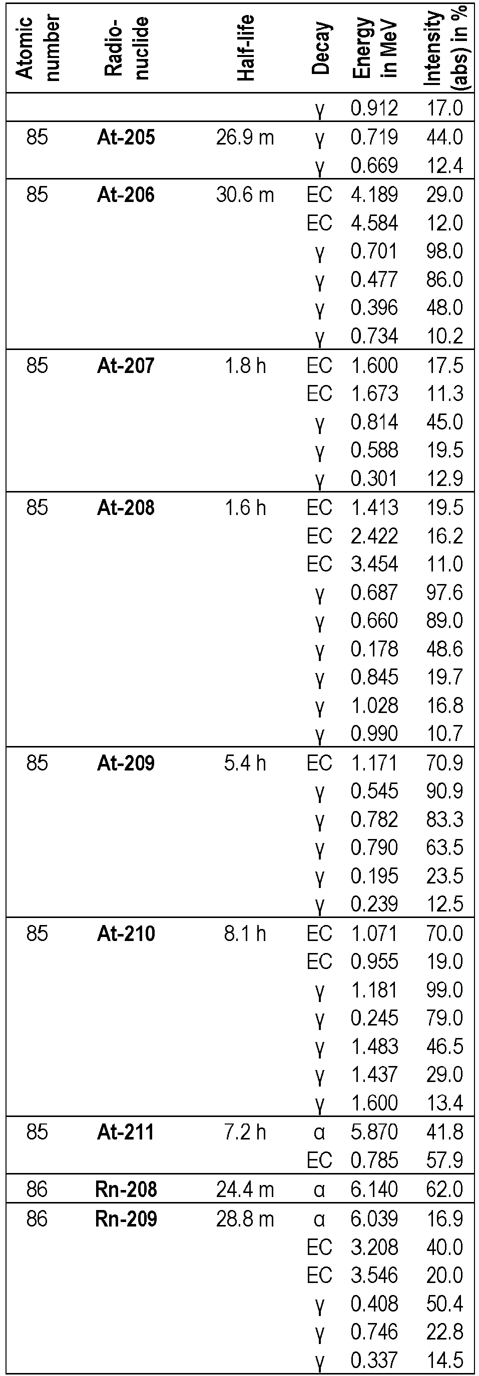

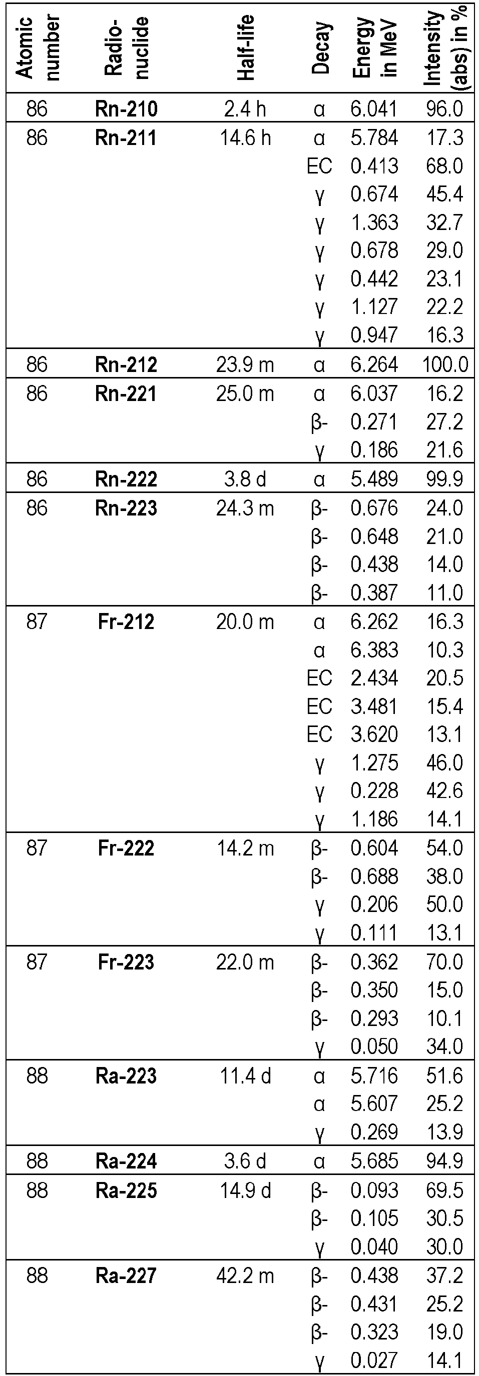

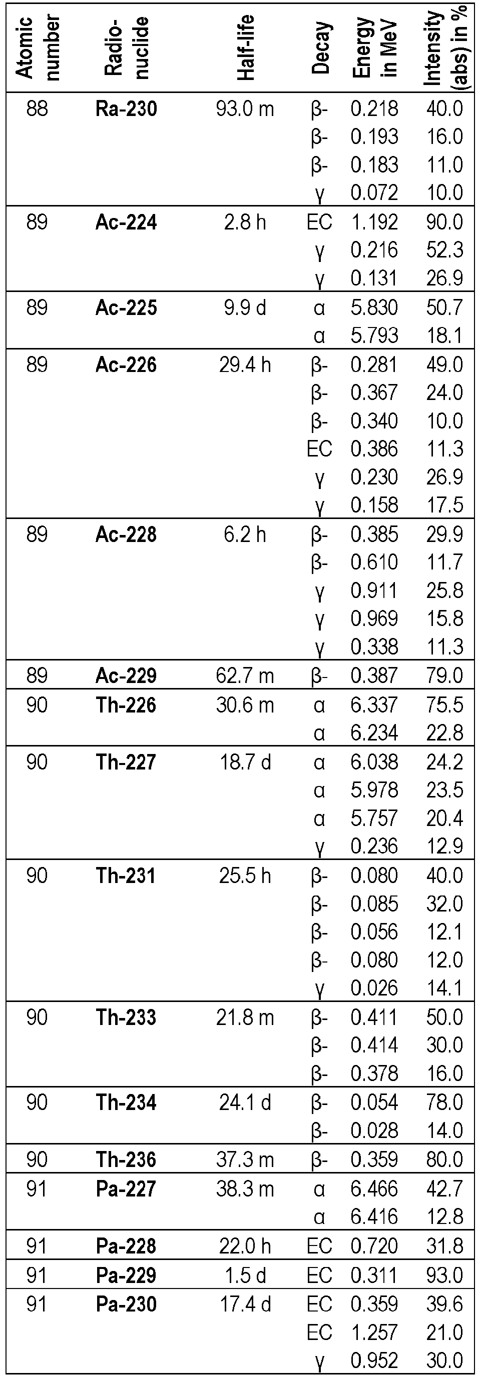

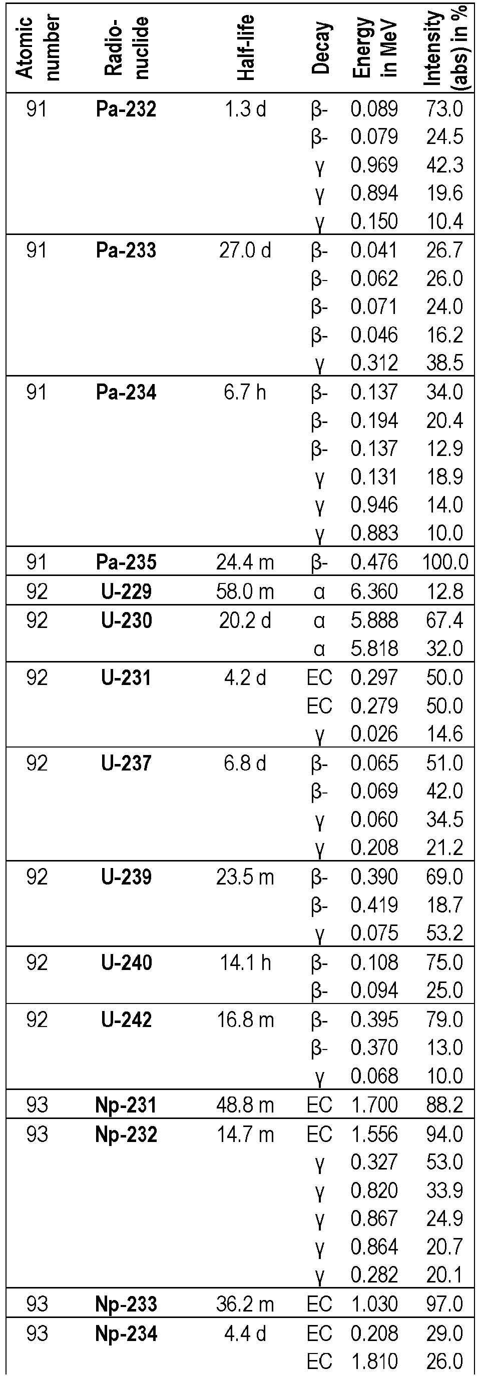

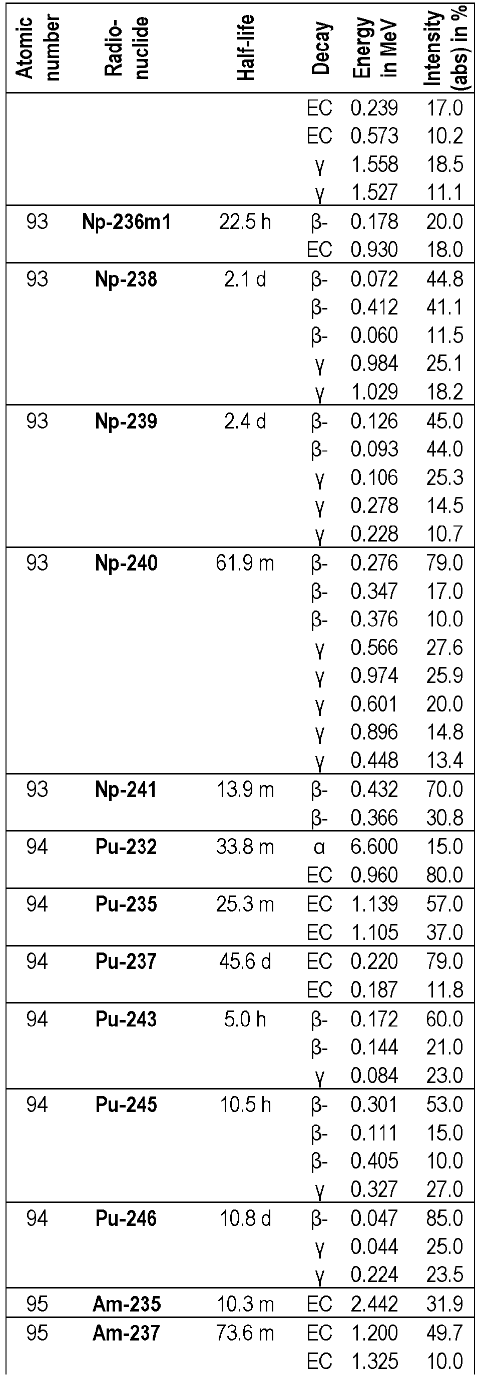

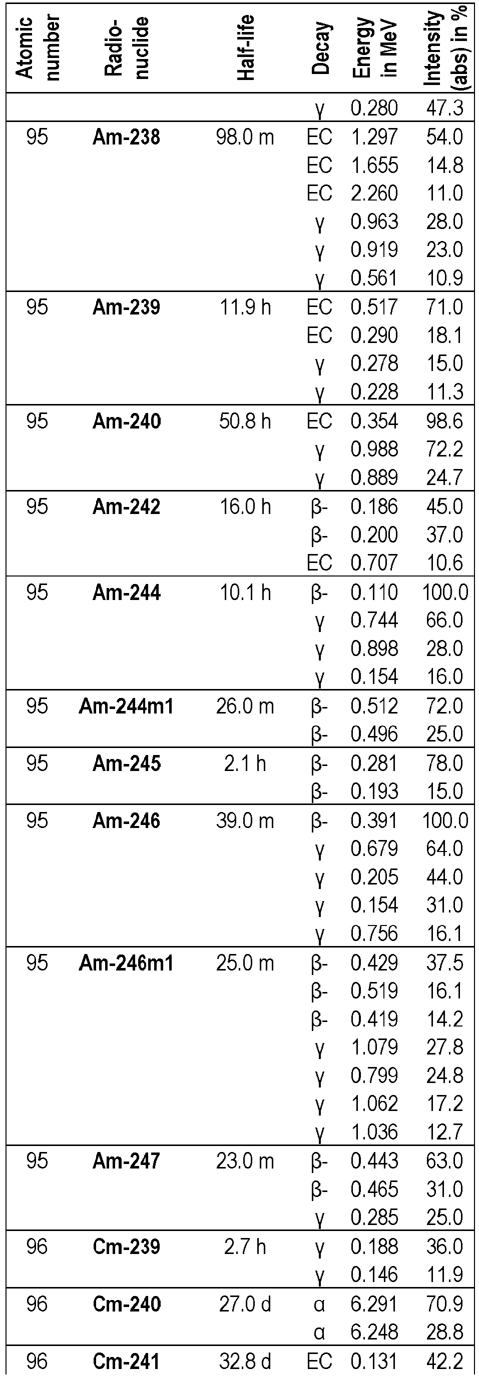

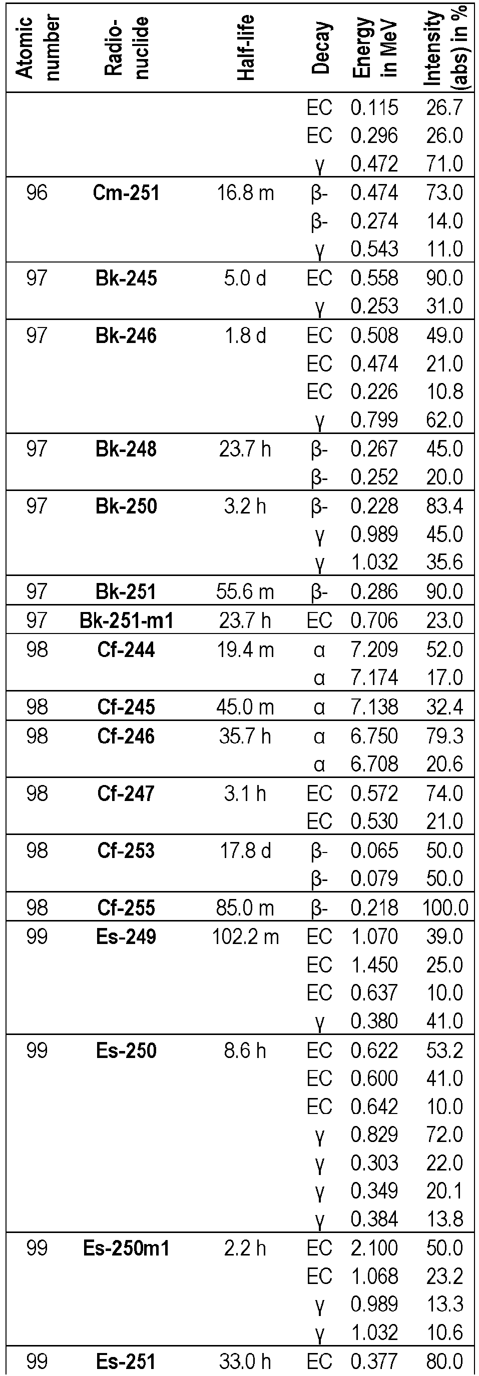

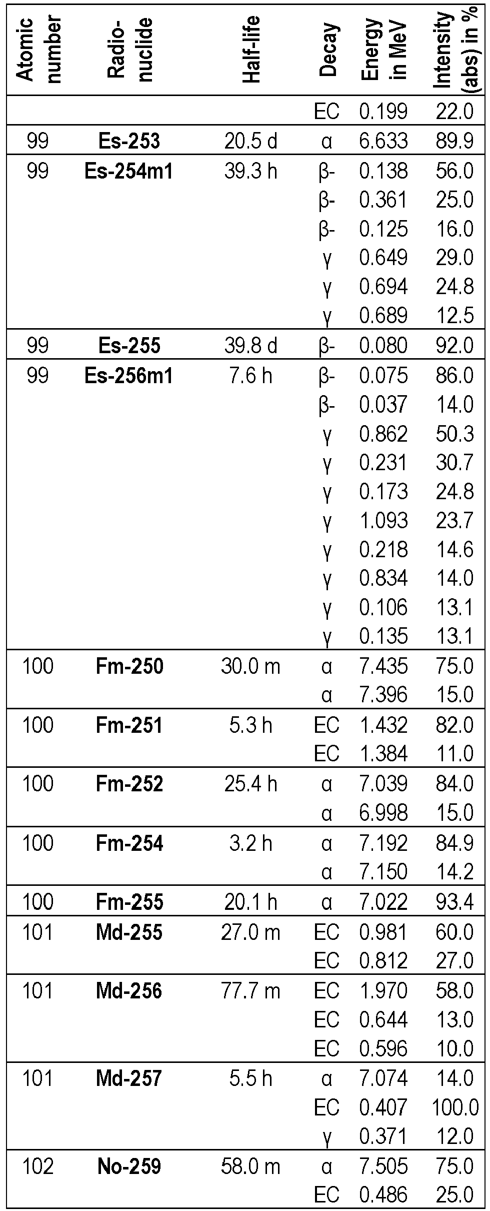

In an embodiment of the present invention, the radionuclide is used for diagnosis. Preferably, the radioactive isotope is selected from the group, but not limited to, comprising ⁴³Sc, ⁴⁴Sc, ⁵¹Mn , ⁵²Mn, ⁶⁴Cu, ⁶⁷Ga, ⁶⁸Ga, ⁸⁶Y, ⁸⁹Zr, ^{94m}Tc, ^{99m}Tc, ¹¹¹In, ¹⁵²Tb, ¹⁵⁵Tb, ¹⁷⁷Lu, ²⁰¹Tl, ²⁰³Pb. More preferably, the radionuclide is selected from the group comprising ⁴³Sc, ⁴⁴Sc, ⁶⁴Cu, ⁶⁷Ga, ⁶⁸Ga, ⁸⁶Y,⁸⁹Zr, ¹¹¹In, ¹⁵²Tb, ¹⁵⁵Tb, ²⁰³Pb. Even more preferably, the radionuclide is ⁶⁸Ga and ¹¹¹In. It will, however, also be acknowledged by a person skilled in the art that the use of said radionuclide is not limited to diagnostic purposes, but encompasses their use in therapy and theragnostics when conjugated to the compound of the invention.

In an embodiment of the present invention, the radionuclide is used for therapy. Preferably, the radioactive isotope is selected from the group comprising ⁴⁷Sc, ⁶⁷Cu, ⁸⁹Sr, ⁹⁰Y, ¹¹¹In, ¹⁵³Sm, ¹⁴⁹Tb, ¹⁶¹Tb, ¹⁷⁷Lu, ¹⁸⁶Re, ¹⁸⁸Re, ²¹²Pb, ²¹³Bi, ²²³Ra, ²²⁵Ac, ²²⁶Th, ²²⁷Th. More preferably, the radioactive isotope is selected from the group comprising ⁴⁷Sc, ⁶⁷Cu, ⁹⁰Y, ¹⁷⁷Lu, ²¹²Pb, ²¹³Bi, ²²⁵Ac, ²²⁷Th. Even more preferably, the radionuclide is selected from the group comprising ⁹⁰Y, ¹⁷⁷Lu, ²²⁵Ac and ²²⁷Th. It will, however, also be acknowledged by a person skilled in the art that the use of said radionuclide is not limited to therapeutic purposes, but encompasses their use in diagnostic and theragnostics when conjugated to the compound of the invention.

In an embodiment, the compound of the invention comprises a chelator. Preferably, the chelator is part of the compound of the invention, whereby the chelator is either directly or indirectly such as by a linker attached to the compound of the invention. A preferred chelator is a chelator which forms metal chelates preferably comprising at least one radioactive metal. The at least one radioactive metal is preferably useful in or suitable for diagnostic and/or therapeutic and/or theragnostic use and is more preferably useful in or suitable for imaging and/or radiotherapy.

Chelators in principle useful in and/or suitable for the practicing of the instant invention including diagnosis and/or therapy of a disease are known to the person skilled in the art. A wide variety of respective chelators is available and has been reviewed, e.g. by Banerjee et al. (Banerjee, et al., Dalton Trans, 2005, 24: 3886), and references therein (Price, et al., Chem Soc Rev, 2014, 43: 260; Wadas, et al., Chem Rev, 2010, 110: 2858). Such chelators include, but are not limited to linear, cyclic, macrocyclic, tetrapyridine, N3S, N2S2 and N4 chelators as disclosed in US 5,367,080 A, US 5,364,613 A, US, 5,021,556 A, US 5,075,099 A and US 5,886,142 A.

Representative chelators and their derivatives include, but are not limited to AAZTA, BAT, CDTA, DTA, DTPA, CY-DTA, DTCBP, CTA, cyclam, cyclen, TETA, sarcophagine, CPTA, TEAMA, Cyclen, DO3A, DO2A, TRITA, DATA, DFO, DATA(M), DATA(P), DATA(Ph), DATA(PPh), DEDPA, H4octapa, H2dedpa, H5decapa, H2azapa, H2CHX-DEDPA, DFO-Chx-MAL, DFO-p-SCN, DFO-1AC, DFO-BAC, p-SCN-Bn-DFO, DFO-pPhe-NCS, DFO-HOPO, DFC, diphosphine, DOTA, DOTAGA, DOTA-MFCO, DOTAM-mono-acid, nitro-DOTA, nitro-PA-DOTA, p-NCS-Bz-DOTA, PA-DOTA, DOTA-NCS, DOTA-NHS, CB-DO2A, PCTA, p-NH₂-Bn-PCTA, p-SCN-Bn-PCTA, p-SCN-Bn-DOTA, DOTMA, NB-DOTA, H4NB-DOTA, H4TCE-DOTA, HOPO, 2,3-HOPO, 3,4,3-(Li-1,2-HOPO), TREN(Me-3,2-HOPO), TCE-DOTA, DOTP, DOXP, p-NCS-DOTA, p-NCS-TRITA, TRITA, TETA, 3p-C-DEPA, 3p-C-DEPA-NCS, p-NH2-BN-OXO-DO3A, p-SCN-BN-TCMC, TCMC, 4-aminobutyl-DOTA, azido-mono-amide-DOTA, BCN-DOTA, butyne-DOTA, BCN-DOTA-GA, DOA3P, DO2a2p, DO2A(trans-H2do2a), H2DO2A, H2ODO2A, DO3A, DO3A-thiol, DO3AM-acetic acid, DO2AP, CB-DO2A, C3B-DO2A, HP-DO3A, DOTA-NHS-ester, maleimide-DOTA-GA, maleimido-mono-aminde-DOTA, maleimide-DOTA, NH₂-DOTA-GA, NH₂-PEG4-DOTA-GA, GA, p-NH₂-Bn-DOTA, p-NO₂-Bn-DOTA, p-SCN-Bn-DOTA, p-SCN-Bz-DOTA, TA-DOTA, TA-DOTA-GA, OTTA, DOXP, TSC, DTC, DTCBP, PTSM, ATSM, H2ATSM, H2PTSM, Dp44mT, DpC, Bp44mT, QT, hybrid thiosemicarbazone-benzothiazole, thiosemicarbazone-styrylpyridine tetradentate ligands H₂L²⁻⁴, HBED, HBED-CC, dmHBED, dmEHPG, HBED-nn, SHBED, Br-Me2HBED, BPCA, HEHA, BF-HEHA, deferiprone, THP, HYNIC (2-hydrazino nicotinamide), NHS-HYNIC, HYNIC-Kp-DPPB, HYNIC-Ko-DPPB, (HYNIC)(tricine)2, (HYNIC)(EDDA), p-EDDHA, AIM, AIM A,IAM B, MAMA, MAMA-DGal, MAMA-MGal, MAMA-DA, MAMA-HAD, macropa, macropaquin, macroquin-SO₃, NxS4-x, N2S2, N3S, N4, MAG3, NOTA, NODAGA, SCN-Bz-NOTA-R, NOT-P (NOTMP), MA-NOTMP, NOTAM, p-NCS-NOTA, TACN, TACN-TM, NETA, NETA-monoamine, p-SCN-PhPr-NE3TA, C-NE3TA-NCS, C-NETA-NCS, 3p-C-NETA, NODASA, NOPO, NODA, NO2A, N-benzyl-NODA, C-NOTA, BCNOT-monoamine, maleimido-mono-amide-NOTA, NO2A-azide, NO2A-butyne, NO2AP, NO3AP, N-NOTA, oxo-DO3A, p-NH₂-Bn-NOTA, p-NH₂-Bn-oxo-DO3A, p-NO₂-Bn-cyclen, p-SCN-Bn-NOTA, p-SCN-Bn-oxo-DO3A, TRAP, PEPA, BF-PEPA, pycup, pycup2A, pycup1A1Bn, pycup2Bn, SarAr-R, DiAmSar, AmBaSar-R, siamSar, Sar, Tachpyr, tachpyr-(6-Me), TAM A, TAM B, TAME, TAME-Hex, THP-Ph-NCS, THP-NCS, THP-TATE, NTP, H3THP, THPN, CB-TE2A, PCB-TE1A1P, TETA-NHS, CPTA, CPTA-NHS, CB-TE1A1P, CB-TE1K1P, CB-TE2A, TE2A, H2CB-TE2A, TE2P, CB-TE2P, MM-TE2A, DM-TE2A, 2C-TETA, 6C-TETA, BAT, BAT-6, NHS-BAT ester, SSBAT, SCN-CHX-A-DTPA-P, SCN-TETA, TMT-amine, p-BZ-HTCPP,DCMC, DEPA, H2ATSM, PCBA, PIH, wherein
2,3-HOPO stands for 3-hydroxypyridin-2-one,
2C-TETA stands for [4,8,11-tris-carboxymethyl-12-(4-isothiocyanato-benzyl)-1,4,8,11tetraaza-cyclotetradec-1-yl]-acetic acid,
3p-C-DEPA stands for 2-[(carboxymethyl)][5-(4-nitrophenyl-1-[4,7,10-tris(carboxymethyl)-1,4,7,10-tetraazacyclododecan-1-yl]pentan-2-yl)amino]acetic acid,
3p-C-DEPA-NCS stands for 2-[(carboxymethyl)][5-(4-thiocyanatophenyl-1-[4,7,10-tris(carboxymethyl)-1,4,7,10-tetraazacyclododecan-1-yl]pentan-2-yl)amino]acetic acid,
3p-C-NE3TA-NCS stands for {4-[2-(bis-carboxymethylamino)-5-(4-isothiocyanatophenyl) pentyl]-7-carboxymethyl[1,4,7] triazonan-1-yl}acetic acid,
3p-C-NETA stands for {4-[2-(bis-carboxymethylamino)-5-(4-nitrophenyl) pentyl]-7-carboxymethyl[1,4,7]triazonan-1-yl}acetic acid,
4-aminobutyl-DOTA stands for 1,4,7,10-tetraazacyclododecane-1,4,7-tris(acetic acid)-10-(4-aminobutyl)acetamide,
^{99m}Tc(CO)₃-chelators stands for bi- or tridendate chelators capable of forming stable complexes with technetium tricarbonyl fragments,
AAZTA stands for 6-amino-6-methylperhydro-1,4-diazepine-N,N',N",N"-tetraacetic acid,
AmBaSar stands for 4-((8-amino-3,6,10,13,16,19-hexaazabicyclo [6.6.6] icosane-1-ylamino) methyl) benzoic acid,
ATSM stands for diacetyl-bis(N4-methylthiosemicarbazone),
azido-mono-amide-DOTA stands for 1,4,7,10-tetraazacyclododecane-1,4,7-tris(acetic acid)-10-(azidopropyl ethylacetamide),
BAT stands for 3,15,27-triamino-7,19,31-trihydroxy-10,22,34-trimethyl-1,13,25-trioxa-7,19,31-triaza-cyclohexatriaconta-9,21,33-triene-2,8,14,20,26,32-hexaone,
BCN-DOTA-GA stands for 2,2,2"-(10-(4-((2-((((1R,8S,9s)-bicyclo[6.1.0]non-4-yn-9-ylmethoxy)carbonyl)amino)ethyl)amino)-1-carboxy-4-oxobutyl)-1,4,7,10-tetraazacyclododecane-1,4,7-triyl)triacetic acid,
BF-HEHA stands for 3-(4-isothicyanatobenzyl)-1,2,7,10,13-hexaazacyclooctadecane-1,4,7,10,13,16-hexaacetic acid,
BF-PEPA stands for 2-(4-thiocyanatobenzoyl)-1,4, 7, 10, 13-pentaazacyclopentadecane-N, N', N", N"', N""-pentaacetic acid,
Bp44mT stands for 2-benzoylpyridine-4,4-dimethyl-3-thiosemicarbazone,
BPCA stands for bipyridine-chelator,
Br-Me2HBED stands for N-(2-hydroxy-3,5-dimethylbenzyl)-Ar'-(2-hydroxy-5-(bromoacetamido)benzyl)ethylenediamine-N,- N'-diacetic acid,
butyne-DOTA stands for 1,4,7,10-tetraazacyclododecane-1,4,7-tris(acetic acid)-10-(3-butynylacetamide),
CB-DO2A stands for 4,10-bis(carboxymethyl)-1,4,7,10-tetraazabicyclo[5.5.2]tetradecane,
CB-TE1A1P stands for (1,8-diamino-3,6,10,13,16,19-hexaazabicyclo[6.6.6]icosane),
CB-TE1K1P stands for 6-amino-2-(11-phosphonomethyl-1,4,8,11-tetraazabicyclo[6.6.2]hexadec-4-yl)-hexanoic acid,
CB-TE2A stands for 4,11-bis-(carboxymethyl)-1,4,8,11-tetraazabicyclo[6.6.2]-hexadecane,
CB-TE2P stands for 1,4,8,11-tetraazacyclotetradecane-1,8-di(methanephosphonic acid),
CDTA stands for trans-1,2-diaminocyclohexane-N,N,N',N'-tetraacetic acid,
CHX-A"-DTPA stands for [(2-{[2-(bis-carboxymethyl-amino)-cyclohexyl]-carboxymethyl-amino}-ethyl)-carboxymethyl-amino]-acetic acid,
C-NOTA stands for [4,7-bis-carboxymethyl-2-(4-nitro-benzyl)-[1,4,7]triazonan-1-yl]-acetic acid,
CPTA stands for 4-((1,4,8,11-tetraazacyclotetradecan-1-yl)methyl)benzoic acid,
cyclam stands for 1,4,8,11-tetraazacyclotetradecane,
cyclen stands for 1,4,7,10-tetraazacyclododecane,
CY-DTA stands for trans-1,2-diaminocyclohexane-N,N,N',N'-tetraacetic acid, monohydrate,
DATA stands for [4-carboxymethyl-6-(carboxymethyl-methyl-amino)-6-methyl-[1,4]diazepan-1-yl]-acetic acid,
DCMC stands for 1,7-bis(carbamoylmethyl)-1,4,7,10- tetraazacyclodocane,
deferiprone (also called DMHP, CP20, L1) stands for 3-hydroxy-1,2-dimethyl-4(1H)-pyridone,
DEPA stands for 7-[2-(bis-carboxymethylamino)-ethyl]-4,10-bis-carboxymethyl-1,4,7,10-tetraaza-cyclododec-1-yl-acetic acid,
DEPA stands for diethylenetriamine pentaacetic acid,
DFO stands for the Desferal or Desferrioxamine type group of chelators, the chemical name of the non-limiting example is N-[5-({3-[5-(Acetyl-hydroxy-amino)-pentylcarbamoyl]-propionyl}-hydroxy-amino)-pentyl]-N'-(5-amino-pentyl)-N'-hydroxy-succinamide,
DFO-BAC stands for bromoacetyl-desferrioxamine,
DFO-HOPO stands for N1-hydroxy-N1-(5-(4-(hydroxy(5-(1-hydroxy-6-oxo-1,6-dihydropyridine-2- carboxamido)pentyl)amino)-4-oxobutanamido)pentyl)-N4-(5-(Nhydroxyacetamido)pentyl)succinamide,
DFO-pPhe-NCS (=p-SCN-Bn-DFO) stands for 1-(4-isothiocyanatophenyl)-3-[6,17-dihydroxy-7,10,18,21-tetraoxo-27-(N-acetylhydroxylamino)- 6,11,17, 22- tetraazaheptaeicosine] thiourea,
DiAmSar stands for 1,8-diamino-3,6,10,13,16,19-hexaazabicyclo[6.6.6]icosane,
dmEHPG stands for N,N-8-ethylene-bis(o-hydroxyphenylglycine) dimethyl ester,
dmHBED stands for N,N^{∗}- bis(o-hydroxybenzyl) ethylenediamine diacetic acid,
DM-TE2A stands for 1,8-N,N'-bis-(carboxymethyl)-4,11-N",N"'-bis-(methyl)-1,4,8,11-tetraazacyclotetra decane,
DO2A stands for 1,4,7,10-tetraazacyclododecane-1,7-diacetic acid,
DO2AP stands for 4-[phosphorylmethyl]-1,4,7,10-tetrazacyclododecane -1,7-diacetic acid,
DO2a2p stands for 1,4,7,10-tetraazacyclododecane-1,7-bis(acetic acid)-4,10-bis(methylenephosphonic acid),
DO3A stands for 1,4,7,10-tetraazacyclododecane-1,4,7-triacetic acid,
DO3AM-acetic acid stands for 2-(4,7,10-tris(2-amino-2-oxoethyl)-1,4,7,10-tetraazacyclododecan-1-yl)acetic acid,
DO3AP stands for 7-[phosphorylmethyl]-1,4,7,10-tetrazacyclododecane-1,4,10-triacetic acid,
DO3A-thiol stands for 1,4,7,10-tetraazacyclododecane-1,4,7-tris(acetic acid)-10-(2-thioethyl)acetamide,
DOTA (also called tetraxetan) stands for 1,4,7,10-tetrazacyclododecane-1,4,7,10-tetraacetic acid,
DOTAGA stands for 1,4,7,10-tetraazacyclodocecane,1-(glutaric acid)-4,7,10-triacetic acid,
DOTAM (also called TCMC) stands for 1,4,7,10-tetrakis[carbamoylmethyl]-1,4,7,10-tetracyclodecane,
DOTAM-mono-acid stands for 1,4,7,10-tetraazacyclododecane-1,4,7-tri(carbamoylmethyl)-10-acetic acid,
DOTA-NCS stands for [4,7,10-tris-carboxymethyl-6-(4-isothiocyanato-benzyl)-1,4,7,10tetraaza-cyclododec-1-yl]-acetic acid,
DOTA-NHS stands for [4,10-bis-carboxymethyl-7-(2,5-dioxo-pyrrolidin-1-yloxycarbonylmethyl)-1,4,7,10tetraaza-cyclododec-1-yl]-acetic acid,
DOTA-NHS-ester stands for 2,2',2"-(10-(2-((2,5-dioxopyrrolidin-l-yl)oxy)-2-oxoethyl)-1,4,7,10-tetraazacyclododecane-1,4,7-triyl)triacetic acid,
DOTMA stands for 1,4,7,10-tetraazacyclododecane-1,4,7,10-tetra(methylene phosphonic acid),
DOTP stands for 1,4,7,10-tetraazacyclododecane-1,4,7,10-tetra(methylene phosphonic acid),
DOXP stands for (di-2-pyridylketone-4,4-dimethyl-3-thiosemicarbazone,
Dp44mT stands for 2-(di-2-pyridinylmethylene)-N,N-dimethyl-hydrazinecarbothioamide, di-2-pyridylketone-4,4,-dimethyl-3-thiosemicarbazone,
DpC stands for di-2-pyridylketone-4-cyclohexyl-4-methyl-3-thiosemicarbazone,
DTC stands for diethyldithiocarbamate,
DTPA stands for diethylenetriaminepentaacetic acid,
EDDA stands for ethylenediaminediacetic acid,
FSC (also called fusarine C) stands for 3,15,27-triamino-7,19,31-trihydroxy-10,22,34-trimethyl-1,13,25-trioxa-7,19,31-triaza-cyclohexatriaconta-9,21,33-triene-2,8,14,20,26,32-hexaone,
H2ATSM stands for 1,4,8,11-tetraazacyclotetradecane-1-(methanephosphonic acid)-8-(methanecarboxylic acid),
H2CB-TE2A stands for 4,11-bis(carboxymethyl)-1,4,8,11-tetraazabicyclo[6.6.2]hexadecane,
H2CHX-DEDPA stands for *N*,*N*'-(6-carboxy-2-pyridylmethyl)-*N*,*N*'-diacetic acid-1,2-diaminoethane,
H2dedpa stands for 1,2-[{6-(carboxylato)pyridin-2-yl}methylamino]ethane,
H2DO2A stands for 1,4,7,10-tetraazacyclododecane-1,7-diacetic acid,
H2ODO2A stands for 1-oxa-4,7,10-triazacyclododecane-4,10-diacetic acid,
H2PTSM stands for pyruvaldehyde bis(methy-lthiosemicarbazone),
H4octapa stands for *N*,*N*'-(6-carboxy-2-pyridylmethyl)-*N*,*N*'-diacetic acid-1,2-diaminoethane,
HBED stands for bis(2-hydroxybenzyl) ethylenediaminediacetic acid,
HBED-CC stands for N,N'-bis[2-hydroxy-5-(carboxyethyl)benzyl]ethylenediamine-N,N'-diacetic acid,
HEHA stands for 1,4,7,10,13,16-hexaazacyclooctadecane-N,N′,Nʺ,N‴,N‴′N‴ʺ-hexaacetic acid,
HOPO stands for the octadentate hydroxypyridinone-type group of chelators,
HP-DO3A stands for 2,2',2"-[10-(2-hydroxypropyl)-1,4,7,10-tetraazacyclododecane-1,4,7-triyl]triacetic acid,
HYNIC stands for 6-hydrazino-nicotinic acid,
HYNIC-Ko-DPPB stands for N-ε-(2-(diphenylphosphino)benzoyl)-N-α-(6-(2-(2-Sulfonatobenzaldehyde)hydrazono)nicotinyl)lysine methyl ester,
HYNIC-Kp-DPPB stands for N-ε-(4-(diphenylphosphino)benzoyl)-N-α-(6-(2-(2-sulfonatobenzaldehyde)hydrazono)nicotinyl)lysine methyl ester,
macropa stands for N,N'-bis[(6-carboxy-2-pyridyl)methyl]-4,13-diaza-18-crown,
MAG3 stands for (N-hydroxysuccinimidyl S-acetylmercaptoacetyltriglycinate,
maleimide-DOTA stands for 2,2,2"-(10-(1-carboxy-4-((2-(2,5-dioxo-2,5-dihydro-1H-pyrrol-1-yl)ethyl)amino)-4-oxobutyl)-1,4,7,10-tetraazacyclododecane-1,4,7-triyl)triacetic acid,
maleimide-DOTA-GA stands for 2,2′,2"-(10-(1-carboxy-4-((2-(2,5-dioxo-2,5-dihydro-1H-pyrrol-1-yl)ethyl)amino)-4-oxobutyl)-1,4,7,10-tetraazacyclododecane-1,4,7-triyl)triacetic acid,
maleimido-mono-amide-NOTA stands for 1,4,7-triazacyclononane-1,4-bis-acetic acid-7-maleimidoethylacetamide,
maleimido-mono-amine-DOTA stands for 1,4,7,10-tetraazacyclododecane-1,4,7-tris-acetic acid-10-maleimidoethylacetamide,
MAMA stands for monoamine-monoamide dithiol,
MAMA-DA stands for N-[[[(2-mercaptoethyl)amino]carbonyl]methyl]-N-(2-mercaptoethyl)-6-aminododecanoic acid,
MAMA-HA stands for N-[[[(2-mercaptoethyl)amino]carbonyl]methyl]-N-(2-mercaptoethyl)-6-aminohexanoic acid,
MAMA-HAD stands for N-[[[(2-mercaptoethyl)amino]carbonyl]methyl]-N-(2-mercaptoethyl)-6-aminohexadecanoic acid,
MA-NOTMP stands for methylaminotriazacyclononane trimethylphosphinate,
MM-TE2A stands for 1,8-N,N'-bis-(carboxymethyl)-4-N"-(methyl)-1,4,8,11-tetraazacyclotetradecane,
N2S2 stands for N,N'-bis-(2-amino-ethyl)-propane-1,3-diamine,
N3OA stands for 4,7,10-tris(carbamoylmethyl)-,4,7,10-triaza-12-crown-ether,
N3S stands for (sulfanylidenehydrazinylidene)azanide,
N4 stands for N,N'-bis-(2-amino-ethyl)-propane-1,3-diamine,
NB-DOTA stands for {4-[2-(bis-carboxymethyl-amino)-ethyl]-7-carboxymethyl-[1,4,7]triazonan-1-yl}-acetic acid,
N-benzyl-NODA stands for 1-benzyl-1,4,7-triazonane-1,4-diyl)diacetic acid,
NE3TA stands for {4-carboxymethyl-7-[2-(carboxymethyl-amino)-ethyl]-[1,4,7]triazonan-1-yl}-acetic acid,
NETA stands for {4-[2-(bis-carboxymethyl-amino)-ethyl]-7-carboxymethyl-[1,4,7]triazonan-1-yl}-acetic acid,
NH2-DOTA-GA stands for 2,2,2"-(10-(4-((2-aminoethyl)amino)-1-carboxy-4-oxobutyl)-1,4,7,10-tetraazacyclododecane-1,4,7-triyl)triacetic acid,
NH2-PEG4-DOTA-GA stands for 2,2',2"-(10-(1-amino-19-carboxy-16-oxo-3,6,9,12-tetraoxa-15-azanonadecan-19-yl)-1,4,7,10-tetraazacyclododecane-1,4,7-triyl)triacetic acid,
NHS-BAT ester stands for N-hydroxysuccinimide ester of 6-(4'-(4"- carboxyphenoxy)butyl)-2, 10-dimercapto-2, 10-dimethyl-4,8- diazaundecane,
NHS-HYNIC stands for N-hydroxysuccinimidyl hydrazino nicotinate hydrochloride,
nitro-DOTA stands for 2-(g-nitrobenzyl)-1,4,7,10-tetraazacyclododecane-N,N',N",N‴-tetraacetic acid,
nitro-PA-DOTA stands for a-(2-(g-nitrophenyl)ethyl)-1,4,7,10-tetraazacyclododecane-1-acetic-4,7,10-tris(methylacetic) acid,
NO2A stands for 1,4,7-triazacyc1ononane-N,N',N"-triacetic acid,
NO2A-azide stands for 1,4,7-triazacyclononane-1,4-bis(acetic acid)-7-(3-azidopropylacetamide),
NO2A-butyne stands for 1,4,7-triazacyclononane-1,4-bis(acetic acid)-7-(3-butynylacetamide),
NO2AP stands for triazacyclononane,
NO3AP stands for 1,4,7-triazacyclononane-N-glutaric acid-N',N"-diacetic acid,
NODA stands for 4,10- bis(carbamoylmethyl)-4,10-diaza-12-crown-ether,
NODAGA stands for 1,4,7-triazacyclononane-N-glutaric acid-N',N"-diacetic acid,
NODA-MPAA stands for 1,4,7-triazacyclononane-1,4-diacetate-methyl phenylacetic acid,
NOPO stands for 3-(((4,7-bis((hydroxy(hydroxymethyl)phosphoryl)methyl)-1,4,7-triazonan-1-yl)methyl)(hydroxy)phosphoryl)propanoic acid,
NOTA stands for 1,4,7-triazacyclononanetriacetic acid,
NOTAM stands for 2,2',2"-(1,4,7-triazacyclononane-1,4,7-triyl)triacetamide,
NOTA-NHS stands for 3-hydroxy-2-oxopyridine,
NOTA-NHS ester stands for 2-(4,7,10-tris(2-(tert-butoxy)-2-oxoethyl)-1,4,7,10-tetraazacyclododecan-1-yl)acetic acid,
NOTP stands for 1,4,7-triazacyclononane-N,N'N"-tris(methylene phosphonic) acid),
NTP stands for {4-carboxymethyl-7-[2-(carboxymethyl-amino)-ethyl]-[1,4,7]triazonan-1-yl}-acetic acid,
NxS4-x (N4, N2S2, N3S) stands for a group of tetradentate chelators with N-atoms (basic amine or non-basic amide) and thiols as donors stabilizing Tc-complexes, especially Tc(V)-oxo complexes,
o,p-EDDHA stands for ethylenediamine-N(o-hydroxyphenylacetic)-N'(p-hydroxyphenylacetic) acid,
OPTT stands for 9-oxa-3,6,12,15,21-pentaazatricyclo[15,3,2,1]trieicos-1(21),17,19-triene-2,7,11,16-tetradione,
OTTA stands for 1-oxa-4,7,10-triazacyclododecane-N,N',N"-triacetic acid,
oxo-DO3A stands for 1-oxa-4,7,10-triazacyclododecane-4,7,10-triacetic acid,
PA-DOTA stands for 1,4,7,10-tetraaza-N-(1-carboxy-3-(4-nitrophenyl)propyl)-N',N",N‴-tris(acetic acid)cyclododecane,
p-BZ-HTCPP stands for 3,6,9,15-tetraazabicyclo[9.3.1]-pentadeca-1(15),11,13-triene-3,6,9,-triacetic acid,
PCBA stands for 1-[(1,4,7,10,13-pentaazacyclopentadec-1-yl)methyl]benzoic acid,
PCB-TE1A1P stands for 2-(11-(phosphonomethyl)-1,4,8,11-tetraazabicyclo[6.6.3]heptadecan-4-yl)acetic acid,
PCTA stands for 3,6,9,15-tetraazabicyclo[9.3.1]-pentadeca-1(15),11,13-triene-3,6,9,-triacetic acid,
PEPA stands for 1,4,7,10,13-pentaazacyclopentadecane-N, N', N", N‴, N‴′-pentaacetic acid,
PIH stands for pyridoxal isonicotinoyl hydrazone,
p-NCS-Bz-DFO stands for N1-hydroxy-N1-(5-(4-(hydroxy(5-(3-(4-thiocyanatobenzoyl)thioureido)pentyl)amino)-4-oxobutanamido)pentyl)-N4-(5-(N-hydroxyacetamido)pentyl)succinamide,
p-NCS-Bz-DOTA stands for S-2-(4-thiocyanatobenzoyl)-1,4,7,10-tetraazacyclododecane tetraacetic acid,
p-NH₂-Bn-DOTA stands for S-2-(4-aminobenzyl)-1,4,7,10-tetraazacyclododecane tetraacetic acid,
p-NH2-Bn-NOTA stands for 2-S-(4-aminobenzyl)-1,4,7-triazacyclononane-1,4,7-triacetic acid,
p-NH₂-Bn-oxo-DO3A stands for 1-oxa-4,7,10-tetraazacyclododecane-5-S-(4-aminobenzyl)-4,7,10-triacetic acid,
p-NH2-Bn-PCTA stands for 3,6,9,15-tetraazabicyclo[9.3.1] pentadeca-1(15),11,13-triene-4-S-(4-aminobenzyl)-3,6,9-triacetic acid,
p-NO2-Bn-cyclen stands for S-2-(4-nitrobenzyl)-1,4,7,10-tetraazacyclododecane,
p-NO2-Bn-DOTA stands for S-2-(4-nitrobenzyl)-1,4,7,10-tetraazacyclododecane tetraacetic acid,
p-SCN-Bn-DFO stands for N1-hydroxy-N1-(5-(4-(hydroxy(5-(3-(4-isothiocyanatophenyl)thioureido)pentyl)amino)-4-oxobutanamido)pentyl)-N4-(5-(N-hydroxyacetamido)pentyl)succinamide,
p-SCN-Bn-DOTA stands for S-2-(4-isothiocyanatobenzyl)-1,4,7,10-tetraazacyclododecane tetraacetic acid,
p-SCN-Bn-NOTA stands for 2-S-(4-isothiocyanatobenzyl)-1,4,7-triazacyclononane-1,4,7-triacetic acid,
p-SCN-Bn-oxo-DO3A stands for 1-oxa-4,7,10-tetraazacyclododecane-5-S-(4-isothiocyanatoobenzyl)-4,7,10-triacetic acid,
p-SCN-Bn-PCTA stands for 3,6,9,15-tetraazabicyclo[9.3.1] pentadeca-1(15),11,13-triene-4-S-(4-isothiocyanatobenzyl)-3,6,9-triacetic acid,
p-SCN-BN-TCMC stands for S-2-(4-isothiocyanatobenzyl)-1,4,7,10-tetraaza-1,4,7,10-tetra(2-carbamoylmethyl)cyclododecane,
p-SCN-Bz-DOTA stands for S-2-(4-isothiocyanatobenzoyl)-1,4,7,10-tetraazacyclododecane tetraacetic acid,
p-SCN-Bz-NOTA stands for 2-S-(4-isothiocyanatobenzoyl)-1,4,7-triazacyclononane-1,4,7-triacetic acid,
p-SCN-PhPr-NE3TA stands for 2,2'-(7-(2-((carboxymethyl)(3-(4-isothiocyanatophenyl)propyl)amino)ethyl)-1,4,7-triazonane-1,4-diyl)diacetic acid,
PTSM stands for pyruvaldehyde bis(N(4)-methylthiosemicarbazone),
pycup stands for 1,8-(2,6-pyridinedimethylene)-1,4,8,11-tetraazacyclo-tetradecane,
pycup2Bn stands for N1-hydroxy-N1-(5-(4-(hydroxy(5-(3-(4-isothiocyanatophenyl)thioureido)pentyl)amino)-4-oxobutanamido)pentyl)-N4-(5-(N-hydroxyacetamido)pentyl)succinamide,
Sar (also called Sarcophagine) stands for 3,6,10,13,16,19-hexaazabicyclo[6.6.6]icosane,
SarAr stands for (1-N-(4-aminobenzyl)-3,6,10,13,16,19-hexaazabicyclo[6.6.6] eicosane-1,8-diamine),
SCN-CHX-A-DTPA-P stands for [(R)-2-amino-3-(4-isothiocyanatophenyl)propyl]-trans-(S,S)-cyclohexane-1,2-diamine-pentaacetic acid,
SCN-TETA stands for 6-[p-(isothiocyanato)benzyl]-1,4,8, 11-tetraazacyclotetradecane-1,4,8,11-tetraacetic acid,
SHBED stands for 3,6,10,13,16,19-hexaazabicyclo(6,6,6)icosane,
Tachpyr stands for (N,N'N"-tris(2-pyridylmethyl)-cis,cis-1,3,5-triaminocyclohexane),
tachpyr-(5-Me) stands for 1,3,5-cis,cis,-triaminocyclohexane-N,N',N"-tri-(5-methyl-2-methylpyridineimine),
TACN stands for 1,4,7-triazacyclononane,
TACN-TM stands for 1,4,7-tris (2-mercaptoethyl)-1,4,7-triazacyclononane,
TA-DOTA stands for 2,2',2"-(10-(2-((2-(5-(1,2-dithiolan-3-yl)pentanamido)ethyl)amino)-2-oxoethyl)-1,4,7,10-tetraazacyclododecane-1,4,7-triyl)triacetic acid,
TA-DOTA-GA stands for 1,1,1-tris(aminomethyl)ethane,
TAM A stands for methyl-(2-methyl-3-methylamino-2-methylaminomethyl-propyl)-amine,
TAME stands for 1,1,1-tris-(aminomethyl)ethane,
TAME-Hex stands for (1,8-N,N'-bis(carboxymethyl)-1,4,8,11-tetraazacyclotetradecane,
TBPD stands for 3,6,9,15-tetraazabicyclo[9.3.1]pentadeca-1(15),11,13-triene-2,10-dione,
TCMC stands for 1,4,8,11-tetraazacyclotetradecane-1,4,8,11-tetraacetic acid,
TE2A stands for [4,8-bis-carboxymethyl-11-(2,5-dioxo-3-sulfo-pyrrolidin-1-yloxycarbonylmethyl)-1,4,8,11tetraaza-cyclotetradec-1-yl]-acetic acid,
TEAMA stands for 6-(p-bromoacetamidobenzyl)-1,4,8,11-tetraazacyclotetradecane-1,4,8,11-tetraacetic acid,
TETA stands for 1,4,8,11-tetraazacyclododecane-1,4,8,11-tetraacetic acid,
TETAM stands for 2,2',2",2‴-(1,4,8,11-tetraazacyclotetradecane-1,4,8,11-tetrayl)tetraacetamide,
THP stands for hexadentate tris(3,4-hydroxypyridinone),
THPN stands for 1,3-propanediamine-N,N,N',N'-tetrakis[(2-(aminomethyl)-3-hydroxy-1,6-dimethyl-4(1H)-pyridinone)acetamide],
THP-TATE stands for 3,3',3"-(((1,4,7-triazonane-1,4,7-triyl)tris(methylene))tris(hydroxyphosphoryl))tripropanoic acid,
TRAP stands for 3-({4,7-bis-[(2-carboxy-ethyl)-hydroxy-phosphinoylmethyl]-[1,4,7]triazonan-1-ylmethyl}-hydroxy-phosphinoyl)-propionic acid,
Triapine stands for dipyridyl thiosemicarbazone,
Tricine stands for picolylaminediacetic acid,
TRITA stands for 2,2′,2ʺ,2‴-(1,4,7,10-tetraazacyclotridecane-1,4,7,10-tetrayl)tetraacetic acid,
TRITRAM stands for 2,2',2"-(1,4,7,10-tetraazacyclotridecane-1,4,7-triyl)triacetamide.HYNIC, DTPA, EDTA, DOTA, TETA, bisamino bisthiol (BAT)-based chelators as disclosed in US 5,720,934; desferrioxamine (DFO) as disclosed in Doulias et al. (Doulias, et al., Free Radic Biol Med, 2003, 35: 719), tetrapyridine and N3S, N2S2 and N4 chelators as disclosed in US 5,367,080 A, US 5,364,613 A, US 5,021,556 A, US 5,075,099 A, US 5,886,142 A, whereby all of the references are included herein by reference in their entirety. 6-amino-6-methylperhydro-1,4-diazepine-*N*,*N*',*N*",*N*"-tetraacetic acid (AAZTA) is disclosed in Pfister et al. (Pfister, et al., EJNMMI Res, 2015, 5: 74), deferiprone, a 1,2-dimethyl-3,4-hydroxypyridinone and hexadentate tris(3,4-hydroxypyridinone) (THP) are disclosed in Cusnir et al. (Cusnir, et al., Int J Mol Sci, 2017, 18), monoamine-monoamide dithiol (MAMA)-based chelators are disclosed in Demoin et al. (Demoin, et al., NuclMedBiol, 2016, 43: 802), macropa and analogs are disclosed in Thiele et al. (Thiele, et al., Angew Chem Int Ed Engl, 2017, 56: 14712), 1,4,7,10,13,16-hexaazacyclohexadecane-N,N',N",N‴,N‴′,N‴ʺ-hexaacetic acid (HEHA) and PEPA analogs are disclosed in Price and Orvig (Price, et al., Chem Soc Rev, 2014, 43: 260), pycup and analogs are disclosed in Boros et al. (Boros, et al., MolPharm, 2014, 11: 617), N, N-bis(2-hydroxybenzyl)ethylenediamine-N,N-diacetic acid (HBED), 1,4,7,10-tetrakis (carbamoylmethyl)-1,4,7,10-tetraazacyclododecane (TCM), 2-[(carboxymethyl)]-[5-(4-nitrophenyl-1-[4,7,10-tris-(carboxymethyl)-1,4,7,10-tetraazacyclododecan-1-yl]pentan-2-yl)-amino]acetic acid (3p-C-DEPA), CB-TE2A, TE2A, TE1A1P, DiAmSar, 1-N-(4-aminobenzyl)-3,6,10,13,16,19-hexaazabicyclo[6.6.6]-eicosane-1,8-diamine (SarAr), NETA,, tris(2-mercaptoethyl)-1,4,7-triazacyclononane (TACN-TM), {4-[2-(bis-carboxymethyl-amino)-ethyl]-7-carboxymethyl-[1,4,7]triazonan-1-yl}-acetic acid (NETA), diethylenetriaminepentaacetic acid (DTP), 3-({4,7-bis-[(2-carboxy-ethyl)-hydroxy-phosphinoylmethyl]-[1,4,7]triazonan-1-ylmethyl}-hydroxy-phosphinoyl)-propionic acid (TRAP), NOPO, H4octapa, SHBED, BPCA, 3,6,9,15-tetraazabicyclo[9.3.1]-pentadeca-1(15),11,13-triene-3,6,9,-triacetic acid (PCTA), and 1,4,7,10,13-pentaazacyclopentadecane-N,N',N",N"',N""-pentaacetic acid (PEPA) are disclosed in Price and Orvig (Price, et al., Chem Soc Rev, 2014, 43: 260), 1-hydroxy-2-pyridone ligand (HOPO) is disclosed in Allott et al. (Allott, et al., Chem Commun (Camb), 2017, 53: 8529), [4-carboxymethyl-6-(carboxymethyl-methyl-amino)-6-methyl-[1,4]diazepan-1-yl]-acetic acid (DATA) is disclosed in Tornesello et al. (Tornesello, et al., Molecules, 2017, 22: 1282), tetrakis(aminomethyl)methane (TAM) and analogs are disclosed in McAuley 1988 (McAuley, et al., Canadian Journal of Chemistry, 1989, 67: 1657), hexadentate tris(3,4-hydroxypyridinone) (THP) and analogs are disclosed in Ma et al. (Ma, et al., Dalton Trans, 2015, 44: 4884).

The diagnostic and/or therapeutic use of some of the above chelators is described in the prior art. For example, 2-hydrazino nicotinamide (HYNIC) has been widely used in the presence of a coligand for incorporation of ^{99m}Tc and ^{186,188}Re (Schwartz, et al., Bioconjug Chem, 1991, 2: 333; Babich, et al., J Nucl Med, 1993, 34: 1964; Babich, et al., Nucl MedBiol, 1995, 22: 25); DTPA is used in Octreoscan^{®} for complexing ¹¹¹In and several modifications are described in the literature (Li, et al., Nucl Med Biol, 2001, 28: 145; Brechbiel, et al., Bioconjug Chem, 1991, 2: 187); DOTA-type chelators for radiotherapy applications are described by Tweedle et al. (US Pat 4,885,363); other polyaza macrocycles for chelating trivalent isotopes metals are described by Eisenwiener *et al.* (Eisenwiener, et al., Bioconjug Chem, 2002, 13: 530); and N4-chelators such as a ^{99m}Tc-N4-chelator have been used for peptide labeling in the case of minigastrin for targeting CCK-2 receptors (Nock, et al., J Nucl Med, 2005, 46: 1727).

In an embodiment the chelator is a metal chelator is selected from the group, but not limited to, comprising DOTA, DOTAGA, DOTAM, DOTP, NOTA, NODAGA, NODA-MPAA, HBED, TETA, CB-TE2A, DTPA, CHX-A"-DTPA, DFO, Macropa, HOPO, TRAP, THP, DATA, NOPO, NOTP, PCTA, sarcophagine, FSC, NETA, NE3TA, H4octapa, pycup, HYNIC, NxS4-x (N4, N2S2, N3S), ^{99m}Tc(CO)₃-chelators and their analogs. Preferably, the chelator additionally comprises one or more functional groups or functionalities allowing attachment to the compound of the invention.

The chemical structures thereof being as follows:

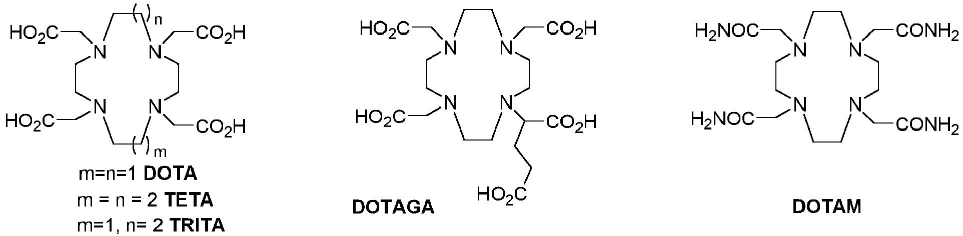

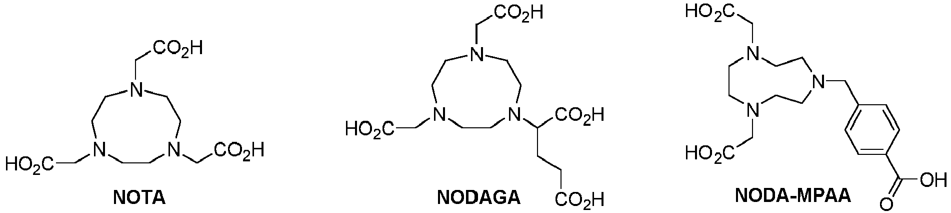

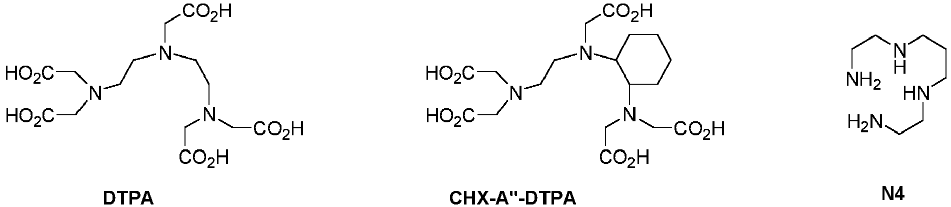

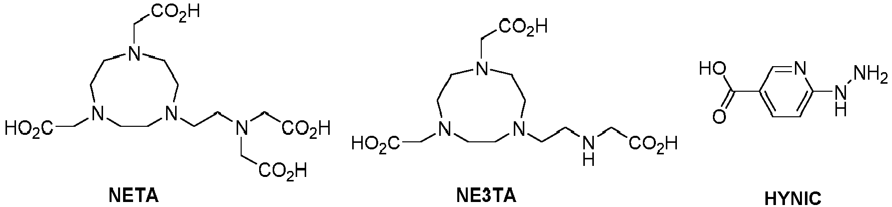

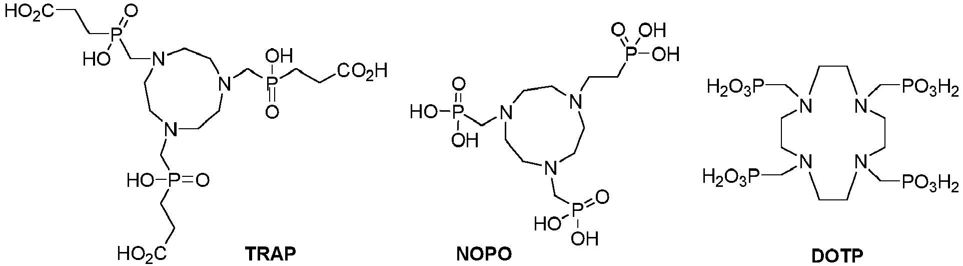

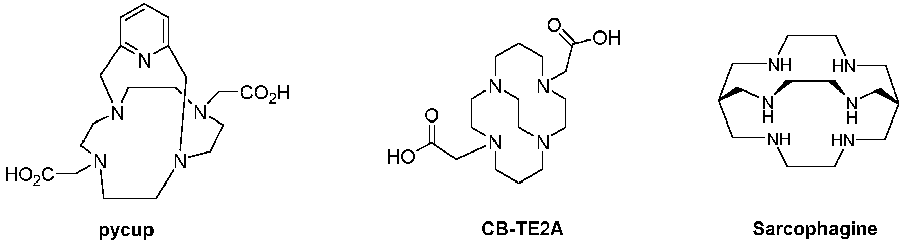

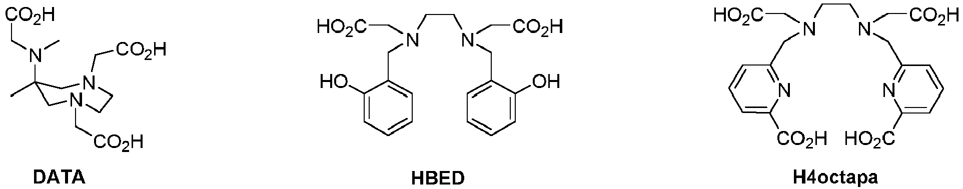

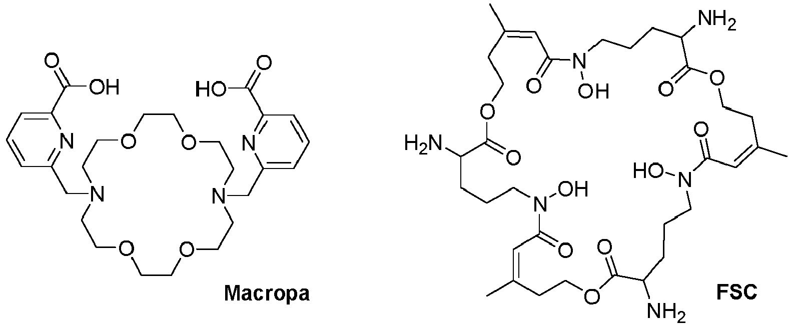

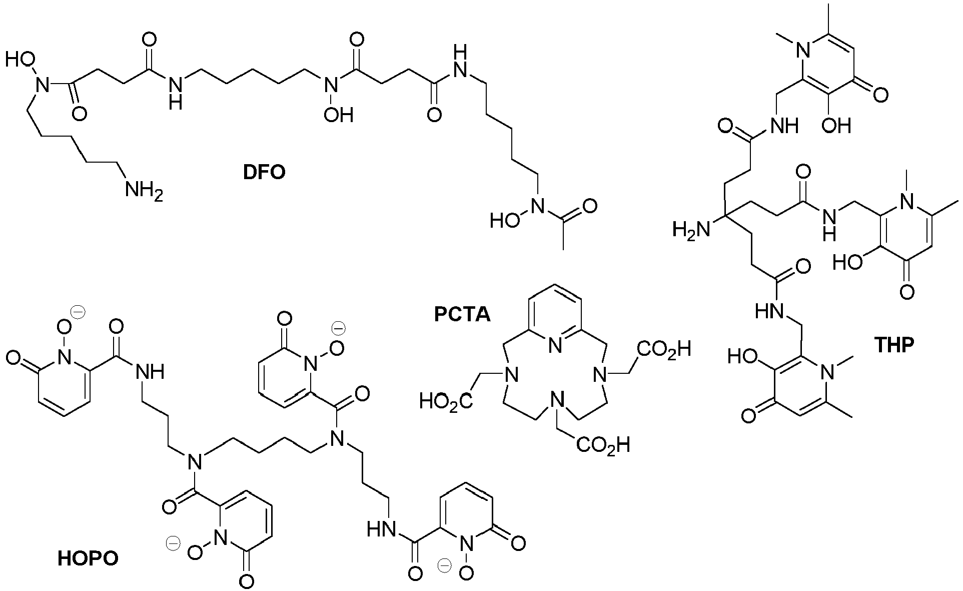

In a preferred embodiment, the metal chelator is selected from the group consisting of DOTA, DOTAGA, DOTAM, NOTA, NODAGA, NODA-MPAA, NOPO, HBED, DTPA, CHX-A"-DTPA, CB-TE2A, Macropa, PCTA, N4, and analogs thereof. Preferably, the chelator additionally comprises one or more functional groups or functionalities allowing attachment to the compounds of the invention.

In a more preferred embodiment, the metal chelator is selected from the group consisting of DOTA, DOTAGA, NODAGA, and macropa and their analogs thereof. Preferably, the chelator additionally comprises one or more functional groups or functionalities allowing attachment to the compounds of the invention.

It will be acknowledged by the persons skilled in the art that the chelator, in principle, may be used regardless whether the compound of the invention is used in or suitable for diagnosis or therapy. Such principle is, among others, outlined in international patent application WO 2009/109332 A1.

It will be further acknowledged by the persons skilled in the art that the presence of a chelator in the compound of the invention includes, if not stated otherwise, the possibility that the chelator is complexed to any metal complex partner, i.e. any metal which, in principle, can be complexed by the chelator. An explicitly mentioned chelator of a compound of the invention or the general term chelator in connection with the compound of the invention refers either to the uncomplexed chelator as such or to the chelator to which any metal complex partner is bound, wherein the metal complex partner is any radioactive or non-radioactive metal complex partner. Preferably the chelator-metal complex, i.e. the chelator to which the metal complex partner is bound, is a stable chelator-metal complex.

Non-radioactive chelator-metal complexes have several applications, e.g., for assessing properties like stability or activity which are otherwise difficult to determine. One aspect is that cold variants of the radioactive versions of the metal complex partner (e.g., non-radioactive indium complexes es described in the examples) can act as surrogates of the radioactive compounds. Furthermore, they are valuable tools for identifying metabolites *in vitro* or *in vivo,* as well as for assessing toxicity properties of the compounds of invention. Additionally, chelator-metal complexes can be used in binding assays utilizing the fluorescence properties of some metal complexes with distinct ligands (e.g., Europium salts).

Chelators can be synthesized or are commercially available with a wide variety of (possibly already activated) groups for the conjugation to peptides or amino acids.

Direct conjugation of a chelator to an amino-nitrogen of the respective compound of invention is well possible for chelators selected from the group consisting of DTPA, DOTA, DOTAGA, NOTA, NODAGA, NODA-MPAA, HBED, TETA, CB-TE2A, DFO, DATA, sarcophagine and N4, preferably DTPA, DOTA, DOTAGA, NOTA, NODAGA, NODA-MPAA, CB-TE2A, and N4. The preferred linkage in this respect is an amide linkage.

Direct conjugation of an isothiocyanate-functionalized chelator to an amino-nitrogen of the respective compound of invention is well possible for chelators selected from the group consisting of DOTA, DOTAGA, NOTA, NODAGA, DTPA, CHX-A"-DTPA, DFO, and THP, preferably DOTA, DOTAGA, NOTA, NODAGA, DTPA, and CHX-A"-DTPA. The preferred linkage in this respect is a thiourea linkage.

Functional groups at a chelator which are preferred precursors for the direct conjugation of a chelator to an amino-nitrogen are known to the person skilled in the art and include but are not limited to carboxylic acid, activated carboxylic acid, e.g., active ester like for instance NHS-ester, pentafluorophenol-ester, HOBt-ester, HOAt-ester, and isothiocyanate.

Functional groups at a chelator which are preferred precursors for the direct conjugation of a chelator to a carboxylic group are known to the person skilled in the art and include but are not limited to alkylamino and arylamino nitrogens. Respective chelator reagents are commercially available for some chelators, e.g., for DOTA with either alkylamino or arylamino nitrogen.

Functional groups at a chelator which are preferred precursors for the direct conjugation of a chelator to a thiol group are known to the person skilled in the art and include but are not limited to maleimide nitrogens. Respective chelator reagents are commercially available for some chelators, e.g., for DOTA with maleimide nitrogen.

Functional groups at a chelator which are preferred precursors for the direct conjugation of a chelator to an azide group are known to the person skilled in the art and include but are not limited to acyclic and cyclic alkynes. Respective chelator reagents are commercially available for some chelators, e.g., for DOTA with propargyl or butynyl.

Functional groups at a chelator which are preferred precursors for the direct conjugation of a chelator to an alkyne group are known to the person skilled in the art and include but are not limited to alkyl and aryl azines. Respective chelator reagents are commercially available for some chelators, e.g., for DOTA with azidopropyl.

In an embodiment, the compound of the invention comprises a bio-distribution modifier. Preferably a bio-distribution modifier is part of the compound of the invention, whereby the bio-distribution modifier is either directly or indirectly such as by a linker attached to the compound of the invention. More preferably a second bio-distribution modifier is part of the compound of the invention, whereby the second bio-distribution modifier is either directly or indirectly such as by a linker attached to the compound of the invention.

The function of a bio-distribution can be the attenuation of the unintended or undesired bio-distribution of the compound of the invention by altering physio-chemical properties of the compound of the invention. To that end elements with a multitude of hydroxyl groups are implemented within or are attached to the peptide of the invention (Wayua, et al., Mol Pharm, 2015, 12: 2477).

The function of a bio-distribution can be the alteration of serum half-life of the compound by modulation of the renal clearance (Du, et al., Bioconjugate Chem, 2020, 31: 241).

The function of a bio-distribution can be the modulation of serum half-life of the compound of the invention by binding to serum components. (Lorenz, et al., Bioorganic Med Chem Lett, 2013, 23: 4011)

In an embodiment an amino-oligo-alcohol, represents the building block for a bio-distribution modifier. The term oligo means that building block may contain 1 to 7 hydroxyl groups. Within the amino-oligo-alcohol the amino function is the conjugation group for the attachment to the peptide of the invention. Usually the amino-oligo-alcohol is linked to the peptide of invention via a dicarboxylic acid linker (such as glutaric acid) or an alpha amino acid linker (such as glutamic acid). Preferred amino-oligo-alcohol building blocks comply with formulae (bio01).

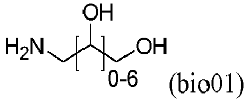

More preferred amino-oligo-alcohol building blocks such as of formula (bio01) are 3-Aminopropane-1,2-diol, 4-Amino-butane-1,2,3-triol, 5-Amino-pentane-1,2,3,4-tetraol, 6-Aminohexane-1,2,3,4,5-pentaol and 1-amino-1-deoxy-D-glucitol. The most preferred amino-oligo-alcohol building block such as formula (bio01) is 1-amino-1-deoxy-D-glucitol.

In an embodiment, conjugates consisting of two amino-oligo-alcohol building blocks such as of formulae (bio01) or (bio02) attached to the carboxylic acid moieties of an alpha amino acid linker such as glutamic acid, represents the building block for a bio-distribution modifier. The resulting construct is connected to the peptide of the invention by dicarboxylic acid linker. Preferably said dicarboxylic acid linker is glutaric acid. A preferred conjugate containing two amino-oligo-alcohol building blocks is a structure of formula (bio02)

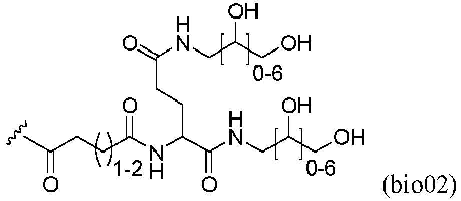

In an embodiment, an inosamine (1-amino-1-deoxy-inositol) represents the building block for a bio-distribution modifier. Within the inosamine the amino function is the conjugation group for the attachment to the peptide of the invention. Usually, the inosamine is linked to the peptide of invention via a dicarboxylic acid linker (such as glutaric acid) or an alpha amino acid linker (such as glutamic acid). Preferred inosamine building blocks comply with formulae (bio03), a more preferred inosamine building block is *scyllo*-inosamine (bio04).

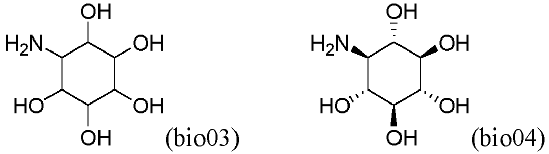

In an embodiment, a glyosylated building block represents the building block for a bio-distribution modifier. Preferred glycosyl building blocks comply with formula (bio05).

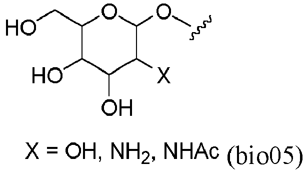

More preferred glycosyl building blocks are glucose, galactose, mannose, glucosamine, galactosamine, mannosamine, N-acetylglucosamine, N-acetylgalactosamine and N-acetylmannosamine.

The sugar moiety is attached to the side chain of an amino acid to from an O- or N-glycosylation as shown in formulae (bio06) and (bio07) to form conjugates which can be employed to attach the glycosyl moiety to the peptide of the invention.

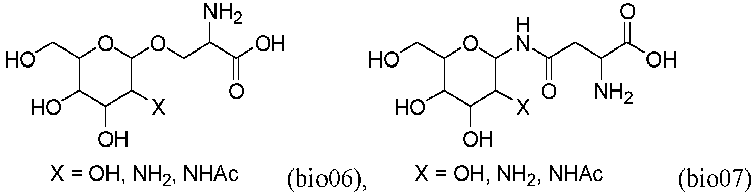

It will be appreciated by a person skilled in the art that the amino acids in formulae (bio06) and (bio07) can be replaced by omega-hydroxy carbonic acids and di-carbonic acids, respectively. The resulting conjugates can be employed to attach the glycosyl moiety to peptide of the invention likewise.

Furthermore, any suitable form of neo-glycosylation (Advanced Drug Delivery Reviews, Volume 171, April 2021, Pages 62-76) for example such as the attachment of the glycosyl residue via a thioether linkage and or via a triazole linkage may employed to attach a glycosyl moiety to the peptide of the invention.

In an embodiment, a linear monodisperse polyethylene glycol (PEG), represents the building block for a bio-distribution modifier. PEG is derived from ethylene glycol. PEGs with a precisely defined number of ethylene glycol units, which is also termed monodisperse, are preferred as building blocks for PEGylation as bio-distribution modifiers for the present invention. As a non-limiting example formula (bio08) is shown as a building block for a bio-distribution modifier.

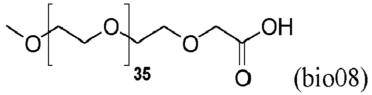

The carboxylic acid moiety in (bio08) is attached covalently to the amino function at the N-terminus of the peptide of invention or to an amino function of an amino acid residue of the peptide of invention. A linker may be interspersed between the monodisperse PEG building block and the peptide of invention.

In an embodiment, a linear polydisperse polyethylene glycol (PEG), represents the building block for a bio-distribution modifier. The synthesis of monodisperse PEGs is rather laborious and hence expensive. Therefore, usually also polydisperse PEGs are employed for the purpose of PEGylation, which means that the polydisperse PEG of a certain molecular weight contains a mixture of different PEGs with a discrete distribution numbers of polyethylene glycol units. For example, a polydiserse PEG with an average molecular weight of 2000 Da consists of a discrete mixture of PEG molecules ranging from about 40 ethylene glycol units to about 55 ethylene glycol units. In the present invention, also polydisperse PEGs can be employed as building blocks for a bio-distribution modifier.

Polydisperse PEG building blocks used as bio-distribution modifier have an average molecular weight of about 2000 Da (40 - 55 ethylen glycol units), about 5000 Da (95 - 135 ethylen glycol units), about 10000 Da (195 - 265 ethylen glycol units) and about 20000 Da (380 - 540 ethylene glycol units). As non-limiting examples formulae (bio09) - (bio12) are shown as building blocks for a bio-distribution modifier.

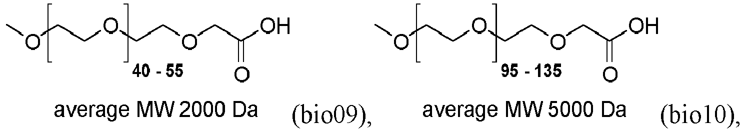

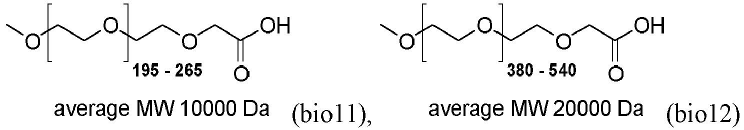

The carboxylic acid moiety in structure (bio08) is attached covalently to the amino function at the N-terminus of the peptide of invention or to an amino function of an amino acid residue of the peptide of invention. A linker may be interspersed between the monodisperse PEG building block and the peptide of the invention.

In an embodiment, a branched polyethylene glycol (PEG), represents the building block for a bio-distribution modifier. The branched PEG may consist of 2 or 3 linear PEG branches/arms/chains. The linear PEG branches/arms/chains implemented in the branched PEG can be monodisperse or polydispers. The individual linear PEG branches/arms/chains implemented in the branched PEG are usually equal. Exemplary branching elements structures are shown in bold in formulae (bio13) to (bio16).

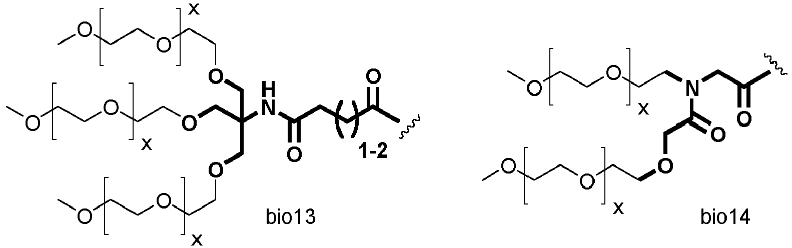

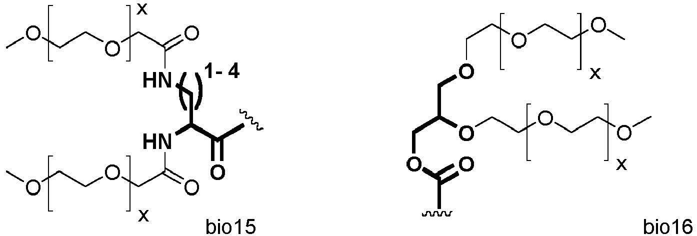

In an embodiment, an carboxylic acid with a long aliphatic chain or an alpha,omega dicarboxylic acid with a long aliphatic chain separating both of the carboxylic acid moieties within said building block, represents a building block for a bio-distribution modifier, which is bound to the peptide of the invention. In case a carboxylic acid is the building block for a bio-distribution modifier, the carboxylic acid moiety is attached covalently to the amino function at the N-terminus of the peptide of invention or to an amino function of an amino acid residue of the peptide of invention. In case a dicarboxylic acid is the building block for a bio-distribution modifier, one of the two carboxylic acid moieties is attached to the amino function at the N-terminus of the peptide of invention or to an amino function of an amino acid residue of the peptide of invention. A linker may be interspersed between either of the carboxylic acid or the dicarboxylic acid and the peptide of invention.

Preferred carboxylic acid building blocks comply with formula (bio17) and preferred carboxylic dicarboxylic acids comply with formula (bio18).

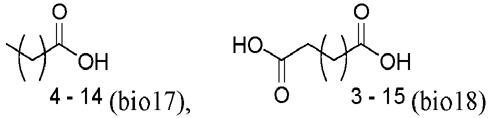

More preferred carboxylic acid building blocks such as formula (bio17) are hexanoic acid, octanoic acid, decanoic acid, lauric acid, myristic acid and palmitic acid. More preferred dicarboxylic acid building blocks such as formula (bio18) are suberic acid, sebacic acid, dodecanedioic acid, tetradecanedioic acid, hexadecanedioic acid and octadecanedioic acid.

In a further embodiment, a glutamic acid is interspersed between either the carboxylic acid or the dicarboxylic acid as shown in formulae (bio19) and (bio20).

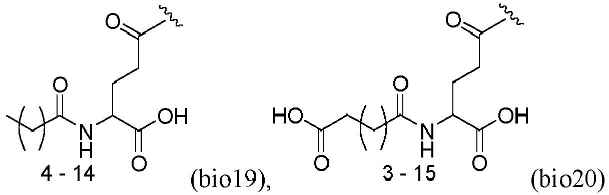

In case a glutamic acid is included in the bio-distribution modifier, a linker may be interspersed between the peptide of invention and the glutamic acid.

In an embodiment, the compound of the invention is present as a pharmaceutically acceptable salt.

A "pharmaceutically acceptable salt" of the compound of the present invention is preferably an acid salt or a base salt that is generally considered in the art to be suitable for use in contact with the tissues of human beings or animals without excessive toxicity or carcinogenicity, and preferably without irritation, allergic response, or other problem or complication. Such salts include mineral and organic acid salts of basic residues such as amines, as well as alkali or organic salts of acidic residues such as carboxylic acids. Compounds of the invention are capable of forming internal salts which are also pharmaceutically acceptable salts.

Suitable pharmaceutically acceptable salts include, but are not limited to, salts of acids such as hydrochloric, phosphoric, hydrobromic, malic, glycolic, fumaric, sulfuric, sulfamic, sulfanilic, formic, toluenesulfonic, methanesulfonic, benzene sulfonic, ethane disulfonic, 2-hydroxyethylsulfonic, nitric, benzoic, 2-acetoxybenzoic, citric, tartaric, lactic, stearic, salicylic, glutamic, ascorbic, pamoic, succinic, fumaric, maleic, propionic, hydroxymaleic, hydroiodic, phenylacetic, alkanoic such as acetic, HOOC-(CH₂)ₙ-COOH where n is any integer from 0 to *4, i.e.,* 0, 1, 2, 3, or 4, and the like. Similarly, pharmaceutically acceptable cations include, but are not limited to sodium, potassium, calcium, aluminum, lithium and ammonium. Those of ordinary skill in the art will recognize further pharmaceutically acceptable salts for the compounds provided herein. In general, a pharmaceutically acceptable acid or base salt can be synthesized from a parent compound that contains a basic or acidic moiety by any conventional chemical method. Briefly, such salts can be prepared by reacting the free acid or base forms of these compounds with a stoichiometric amount of the appropriate base or acid in water or in an organic solvent, or in a mixture of the two. Generally, the use of non-aqueous media, such as ether, ethyl acetate, ethanol, isopropanol or acetonitrile, is preferred.

A "pharmaceutically acceptable solvate" of the compound of the invention is preferably a solvate of the compound of the invention formed by association of one or more solvent molecules to one or more molecules of a compound of the invention. Preferably, the solvent is one which is generally considered in the art to be suitable for use in contact with the tissues of human beings or animals without excessive toxicity or carcinogenicity, and preferably without irritation, allergic response, or other problem or complication. Such solvent includes an organic solvent such as alcohols, ethers, esters and amines.

A "hydrate" of the compound of the invention is formed by association of one or more water molecules to one or more molecules of a compound of the invention. Such hydrate includes but is not limited to a hemi-hydrate, mono-hydrate, dihydrate, trihydrate and tetrahydrate. Independent of the hydrate composition all hydrates are generally considered as pharmaceutically acceptable.

The compound of the invention has a high binding affinity to the GIPR. The affinity is determined as described in example 27 by the FACS binding assay. High binding affinity is pIC₅₀ category preferably A-D, more preferably A-C, even more preferably A-B and most preferably A. Because of this high binding affinity, the compound of the invention is effective as, useful as and/or suitable as a targeting agent and, if conjugated to another moiety, as a targeting moiety. As preferably used herein a targeting agent is an agent which interacts with the target molecule which is in the instant case said GIPR. In terms of cells and tissues thus targeted by the compound of the invention any cell and tissue, respectively, expressing said GIPR is or may be targeted.

In an embodiment, the compound interacts with a gastric inhibitory polypeptide receptor (GIPR), preferably with human GIPR having an amino acid sequence of SEQ ID NO: 1 or a homolog thereof, wherein the amino acid sequence of the homolog has an identity of GIPR that is at least 85% to the amino acid sequence of SEQ ID NO: 1. In preferred embodiments, the identity is 90%, preferably 95 %, 96 %, 97 %, 98 % or 99%.

The identity between two nucleic acid molecules can be determined as known to the person skilled in the art. More specifically, a sequence comparison algorithm may be used for calculating the percent sequence homology for the test sequence(s) relative to the reference sequence, based on the designated program parameters. The test sequence is preferably the sequence or protein or polypeptide which is said to be identical or to be tested whether it is identical, and if so, to what extent, to a different protein or polypeptide, whereby such different protein or polypepetide is also referred to as the reference sequence and is preferably the protein or polypeptide of wild type, more preferably the human GIPR of SEQ ID NO: 1.

Optimal alignment of sequences for comparison can be conducted, e.g., by the local homology algorithm of Smith & Waterman (Smith, et al., Advances in Applied Mathematics, 1981, 2: 482), by the homology alignment algorithm of Needleman & Wunsch (Needleman, et al., JMol Biol, 1970, 48: 443), by the search for similarity method of Pearson & Lipman (Pearson, et al., Proc Natl Acad Sci U S A, 1988, 85: 2444), by computerized implementations of these algorithms (GAP, BESTFIT, FASTA, and TFASTA in the Wisconsin Genetics Software Package, Genetics Computer Group, 575 Science Dr., Madison, Wis.), or by visual inspection.

One example of an algorithm that is suitable for determining percent sequence identity is the algorithm used in the basic local alignment search tool (hereinafter "BLAST "), see, e.g. Altschul et al., 1990 (Altschul, et al., J Mol Biol, 1990, 215: 403) and Altschul et al., 1997 (Altschul, et al., Nucleic Acids Res, 1997, 25: 3389). Software for performing BLAST analyses is publicly available through the National Center for Biotechnology Information (hereinafter "NCBI"). The default parameters used in determining sequence identity using the software available from NCBI, e.g., BLASTN (for nucleotide sequences) and BLASTP (for amino acid sequences) are described in McGinnis et al. (McGinnis, et al., Nucleic Acids Res, 2004, 32: W20).

It is within the present invention that the compound of the invention is used or is for use in a method for the treatment of a disease as disclosed herein. Such method, preferably, comprises the step of administering to a subject in need thereof a therapeutically effective amount of the compound of the invention. Such method includes, but is not limited to, curative or adjuvant cancer treatment. It is used as palliative treatment where cure is not possible and the aim is for local disease control or symptomatic relief or as therapeutic treatment where the therapy has survival benefit and it can be curative.

The method for the treatment of a disease as disclosed herein includes the treatment of the disease disclosed herein, including tumors and cancer, and may be used either as the primary therapy or as second, third, fourth or last line therapy. It is also within the present invention to combine the compound of the invention with further therapeutic approaches. It is well known to the person skilled in the art that the precise treatment intent including curative, adjuvant, neoadjuvant, therapeutic, or palliative treatment intent will depend on the tumor type, location, and stage, as well as the general health of the patient.

In an embodiment, the diseases which may be treated by the compound of the invention is selected from the group comprising medullary thyroid cancer, gastrointestinal neuroendocrine neoplasms, ileal neuroendocrine neoplasms, pancreatic neuroendocrine neoplasms, nonfunctioning neuroendocrine neoplasms, Insulinomas, Gastrinomas, Glucagonomas, VIPomas, Somatostatinoma, ACTHoma, lung neuroendocrine neoplasms, typical carcinoid tumors, atypical carcinoid tumors, small cell carcinoma of the lung, large cell carcinoma of the lung, thymic neuroendocrine tumors, Merkel cell carcinoma, Pheochromocytoma of the adrenal gland, adrenal cancer, parathyroid cancer, paraganglioma, pituitary gland tumors, neuroendocrine tumors of the ovaries and neuroendocrine tumors of the testicles.

In a further embodiment, the disease which may be treated by the compound of the invention is a metabolic disease. Preferably, the metabolic disease is one where GIPR is expressed, more preferably where the GIPR expression is increased in pancreatic beta cells. In a still further embodiment, the disease is selected from the group comprising metabolic diseases and disorders, preferably type 2 diabetes, type 1 diabetes, metabolic syndrome, insulin resistance, dyslipidemia, impaired fasting glucose, and impaired glucose tolerance.

In an embodiment, the compounds of the present invention are used to detect cells and tissues overexpressing the GIPR, whereby such detection is achieved by conjugating a detectable label to the compounds of the invention, preferably a detectable radionuclide. In a preferred embodiment, the cells and tissues detected are diseased cells and tissues and/or are either a or the cause for the disease and/or the symptoms of the disease, or are part of the pathology underlying the disease. In a further preferred embodiment, the diseased cells and tissues are causing and/or are part of an oncology indication (e.g., neoplasms, tumors, and cancers) or a non-oncology indication (e.g., metabolic diseases, preferably type 2 diabetes, insulin restistance, impaired glucose tolerance).

In another embodiment, the compounds of the present invention are used to treat cells and tissues overexpressing the GIPR. In a preferred embodiment, the cells and tissues treated are diseased cells and tissues and/or are either a or the cause for the disease and/or the symptoms of the disease, or are part of the pathology underlying the disease. In a further preferred embodiment, the diseased cells and tissues are causing and/or are part of an oncology indication (e.g. neoplasms, tumors, and cancers) and the therapeutic activity is achieved by conjugating therapeutically active effector to the compounds of the present invention, preferably a therapeutically active radionuclide. In a further preferred embodiment, the diseased cells and tissues are causing and/or are part of a non-oncology indication (e.g., metabolic diseases, insulin resistance, type 1 and type 2 diabetes) and the therapeutic activity is either achieved by targeting of the GIPR or it is monitored by GIPR imaging.

In a further embodiment, particularly if the disease is a non-oncology disease or a non-oncology indication (e.g. metabolic diseases, insulin resistance, type 1 and type 2 diabetes), the compounds of the present invention are administered in therapeutically effective amounts; preferably the compound of the present invention does not comprise a therapeutically active nuclide such as a therapeutically active nuclide An effective amount is a dosage of the compound sufficient to provide a therapeutically or medically desirable result or effect in the subject to which the compound is administered. The effective amount will vary with the particular condition being treated, the age and physical condition of the subject being treated, the severity of the condition, the duration of the treatment, the nature of the concurrent or combination therapy (if any), the specific route of administration and like factors within the knowledge and expertise of the health practitioner. For example, in connection with methods directed towards treating subjects having a condition characterized by abnormal cell proliferation, an effective amount to inhibit proliferation would be an amount sufficient to reduce or halt altogether the abnormal cell proliferation so as to slow or halt the development of or the progression of a cell mass such as, for example, a tumor. As used in the embodiments, "inhibit" embraces all of the foregoing.

In other embodiments, a therapeutically effective amount will be an amount necessary to extend the dormancy of micrometastases or to stabilize any residual primary tumor cells following surgical or drug therapy.

Generally, when using an unconjugated compound without a therapeutically active radionuclide, a therapeutically effective amount will vary with the subject's age, condition, and sex, as well as the nature and extent of the disease in the subject, all of which can be determined by one of ordinary skill in the art. The dosage may be adjusted by the individual physician or veterinarian, particularly in the event of any complication. A therapeutically effective amount is typically, but not limited to, an amount in a range from 0.1 µg/kg to about 2000 mg/kg, or from 1.0 µg/kg to about 1000 mg/kg, or from about 0.1 mg/kg to about 500 mg/kg, or from about 1.0 mg/kg to about 100 mg/kg, in one or more dose administrations daily, for one or more days. If desired, the effective daily dose of the active compound may be administered as two, three, four, five, six, or more sub-doses for example administered separately at appropriate intervals throughout the day, optionally, in unit dosage forms. In some embodiments, the compounds are administered for more than 7 days, more than 10 days, more than 14 days and more than 20 days. In still other embodiments, the compound is administered over a period of weeks, or months. In still other embodiments, the compound is delivered on alternate days. For example, the agent is delivered every two days, or every three days, or every four days, or every five days, or every six days, or every week, or every month.

In a preferred embodiment, the compound of the present invention is for use in the treatment and/or prevention of a disease, whereby such treatment is radionuclide therapy.

Preferably, radionuclide therapy makes use of or is based on different forms of radiation emitted by a radionuclide. Such radiation can, for example, be any one of radiation of photons, radiation of electrons including but not limited to β⁻-particles and Auger-electrons, radiation of protons, radiation of neutrons, radiation of positrons, radiation of α-particles or an ion beam. Depending on the kind of particle or radiation emitted by said radionuclide, radionuclide therapy can, for example, be distinguished as photon radionuclide therapy, electron radionuclide therapy, proton radionuclide therapy, neutron radionuclide therapy, positron radionuclide therapy, α-particle radionuclide therapy or ion beam radionuclide therapy. All of these forms of radionuclide therapy are encompassed by the present invention, and all of these forms of radionuclide therapy can be realized by the compound of the invention, preferably under the proviso that the radionuclide attached to the compound of the invention, more preferably as an effector, is providing for this kind of radiation.

Radionuclide therapy preferably works by damaging the DNA of cells. The damage is caused by a photon, electron, proton, neutron, positron, α-particle or ion beam directly or indirectly ionizing the atoms which make up the DNA chain. Indirect ionization happens as a result of the ionization of water, forming free radicals, notably hydroxyl radicals, which then damage the DNA.

In the most common forms of radionuclide therapy, most of the radiation effect is through free radicals. Because cells have mechanisms for repairing DNA damage, breaking the DNA on both strands proves to be the most significant technique in modifying cell characteristics. Because cancer cells generally are undifferentiated and stem cell-like, they reproduce more, and have a diminished ability to repair sub-lethal damage compared to most healthy differentiated cells. The DNA damage is inherited through cell division, accumulating damage to the cancer cells, causing them to die or reproduce more slowly.

Oxygen is a potent radiosensitizer, increasing the effectiveness of a given dose of radiation by forming DNA-damaging free radicals. Therefore, use of high-pressure oxygen tanks, blood substitutes that carry increased oxygen, hypoxic cell radiosensitizers such as misonidazole and metronidazole, and hypoxic cytotoxins, such as tirapazamine may be applied.

Other factors that are considered when selecting a radioactive dose include whether the patient is receiving chemotherapy, whether radiation therapy is being administered before or after surgery, and the degree of success of surgery.

The total radioactive dose may be fractionated, i.e. spread out over time in one or more treatments for several important reasons. Fractionation allows normal cells time to recover, while tumor cells are generally less efficient in repair between fractions. Fractionation also allows tumor cells that were in a relatively radio-resistant phase of the cell cycle during one treatment to cycle into a sensitive phase of the cycle before the next fraction is given. Similarly, tumor cells that were chronically or acutely hypoxic and, therefore, more radioresistant, may reoxygenate between fractions, improving the tumor cell kill.

It is generally known that different cancers respond differently to radiation therapy. The response of a cancer to radiation is described by its radiosensitivity. Highly radiosensitive cancer cells are rapidly killed by modest doses of radiation. These include leukemias, most lymphomas, and germ cell tumors.

It is important to distinguish radiosensitivity of a particular tumor, which to some extent is a laboratory measure, from "curability" of a cancer by an internally delivered radioactive dose in actual clinical practice. For example, leukemias are not generally curable with radiotherapy, because they are disseminated through the body. Lymphoma may be radically curable if it is localized to one area of the body. Similarly, many of the common, moderately radioresponsive tumors can be treated with curative doses of radioactivity if they are at an early stage. This applies, for example, to non-melanoma skin cancer, head and neck cancer, non-small cell lung cancer, cervical cancer, anal cancer, prostate cancer.

The response of a tumor to radiotherapy is also related to its size. For complex reasons, very large tumors respond less well to radiation than smaller tumors or microscopic disease. Various strategies are used to overcome this effect. The most common technique is surgical resection prior to radiotherapy. This is most commonly seen in the treatment of breast cancer with wide local excision or mastectomy followed by adjuvant radiotherapy. Another method is to shrink the tumor with neoadjuvant chemotherapy prior to radical radionuclide therapy. A third technique is to enhance the radiosensitivity of the cancer by giving certain drugs during a course of radiotherapy. Examples of radiosensiting drugs include, but are not limited to Cisplatin, Nimorazole, and Cetuximab.

Introperative radiotherapy is a special type of radiotherapy that is delivered immediately after surgical removal of the cancer. This method has been employed in breast cancer (TARGeted Introperative radioTherapy), brain tumors and rectal cancers.

Radionuclide therapy is in itself painless. Many low-dose palliative treatments cause minimal or no side effects. Treatment to higher doses may cause varying side effects during treatment (acute side effects), in the months or years following treatment (long-term side effects), or after re-treatment (cumulative side effects). The nature, severity, and longevity of side effects depends on the organs that receive the radiation, the treatment itself (type of radionuclide, dose, fractionation, concurrent chemotherapy), and the patient.

It is within the present inventions that the method for the treatment of a disease of the invention may realize each and any of the above strategies which are as such known in the art, and which insofar constitute further embodiments of the invention.

It is also within the present invention that the compound of the invention is used in a method for the diagnosis of a disease as disclosed herein. Such method, preferably, comprises the step of administering to a subject in need thereof a diagnostically effective amount of the compound of the invention.

In accordance with the present invention, an imaging method is selected from the group consisting of scintigraphy, Single Photon Emission Computed Tomography (SPECT) and Positron Emission Tomography (PET).

Scintigraphy is a form of diagnostic test or method used in nuclear medicine, wherein radiopharmaceuticals are internalized by cells, tissues and/or organs, preferably internalized *in vivo*, and radiation emitted by said internalized radiopharmaceuticals is captured by external detectors (gamma cameras) to form and display two-dimensional images. In contrast thereto, SPECT and PET forms and displays three-dimensional images. Because of this, SPECT and PET are classified as separate techniques to scintigraphy, although they also use gamma cameras to detect internal radiation. Scintigraphy is unlike a diagnostic X-ray where external radiation is passed through the body to form an image.

Single Photon Emission Tomography (SPECT) scans are a type of nuclear imaging technique using gamma rays. They are very similar to conventional nuclear medicine planar imaging using a gamma camera. Before the SPECT scan, the patient is injected with a radiolabeled chemical emitting gamma rays that can be detected by the scanner. A computer collects the information from the gamma camera and translates this into two-dimensional cross-sections. These cross-sections can be added back together to form a three-dimensional image of an organ or a tissue. SPECT involves detection of gamma rays emitted singly, and sequentially, by the radionuclide provided by the radiolabeled chemical. To acquire SPECT images, the gamma camera is rotated around the patient. Projections are acquired at defined points during the rotation, typically every 3 - 6 degrees. In most cases, a full 360 degree rotation is used to obtain an optimal reconstruction. The time taken to obtain each projection is also variable, but 15 - 20 seconds is typical. This gives a total scan time of 15 - 20 minutes. Multi-headed gamma cameras are faster. Since SPECT acquisition is very similar to planar gamma camera imaging, the same radiopharmaceuticals may be used.

Positron Emitting Tomography (PET) is a non-invasive, diagnostic imaging technique for measuring the biochemical status or metabolic activity of cells within the human body. PET is unique since it produces images of the body's basic biochemistry or functions. Traditional diagnostic techniques, such as X-rays, CT scans, or MRI, produce images of the body's anatomy or structure. The premise with these techniques is that any changes in structure or anatomy associated with a disease can be seen. Biochemical processes are also altered by a disease, and may occur before any gross changes in anatomy. PET is an imaging technique that can visualize some of these early biochemical changes. PET scanners rely on radiation emitted from the patient to create the images. Each patient is given a minute amount of a radioactive pharmaceutical that either closely resembles a natural substance used by the body or binds specifically to a receptor or molecular structure. As the radioisotope undergoes positron emission decay (also known as positive beta decay), it emits a positron, the antiparticle counterpart of an electron. After traveling up to a few millimeters, the positron encounters an electron and annihilates, producing a pair of annihilation (gamma) photons moving in opposite directions. These are detected when they reach a scintillation material in the scanning device, creating a burst of light, which is detected by photomultiplier tubes or silicon avalanche photodiodes. The technique depends on simultaneous or coincident detection of the pair of photons. Photons that do not arrive in pairs, i.e., within a few nanoseconds, are ignored. All coincidences are forwarded to the image processing unit where the final image data is produced using image reconstruction procedures.

SPECT/CT and PET/CT is the combination of SPECT and PET with computed tomography (CT). The key benefits of combining these modalities are improving the reader's confidence and accuracy. With traditional PET and SPECT, the limited number of photons emitted from the area of abnormality produces a very low-level background that makes it difficult to anatomically localize the area. Adding CT helps determine the location of the abnormal area from an anatomic perspective and categorize the likelihood that this represents a disease.

It is within the present inventions that the method for the diagnosis of a disease of the invention may realize each and any of the above strategies which are as such known in the art, and which insofar constitute further embodiments of the invention.

Compounds of the present invention are useful to stratify patients, i.e., to create subsets within a patient population that provide more detailed information about how the patient will respond to a given drug. Stratification can be a critical component to transforming a clinical trial from a negative or neutral outcome to one with a positive outcome by identifying the subset of the population most likely to respond to a novel therapy.

Stratification includes the identification of a group of patients with shared "biological" characteristics to select the optimal management for the patients and achieve the best possible outcome in terms of risk assessment, risk prevention and achievement of the optimal treatment outcome.

A compound of the present invention may be used to assess or detect, a specific disease as early as possible (which is a diagnostic use), the risk of developing a disease (which is a susceptibility/risk use), the evolution of a disease including indolent vs. aggressive (which is a prognostic use) and it may be used to predict the response and the toxicity to a given treatment (which is a predictive use).

It is also within the present invention that the compound of the invention is used in a theragnostic method. The concept of theragnostics is to combine a therapeutic agent with a corresponding diagnostic test that can increase the clinical use of the therapeutic drug. The concept of theragnostics is becoming increasingly attractive and is widely considered the key to improving the efficiency of drug treatment by helping doctors identify patients who might profit from a given therapy and hence avoid unnecessary treatments.

The concept of theragnostics is to combine a therapeutic agent with a diagnostic test that allows doctors to identify those patients who will benefit most from a given therapy. In an embodiment and as preferably used herein, a compound of the present invention is used for the diagnosis of a patient, i.e. identification and localization of the primary tumor mass as well as potential local and distant metastases. Furthermore, the tumor volume can be determined, especially utilizing three-dimensional diagnostic modalities such as SPECT or PET. Only those patients having GIPR-positive tumor masses and who, therefore, might profit from a given therapy are selected for a particular therapy and hence unnecessary treatments are avoided. Preferably, such therapy is a GIPR-targeted therapy using a compound of the present invention. In one particular embodiment, chemically identical tumor-targeted diagnostics, preferably imaging diagnostics for scintigraphy, PET or SPECT and radiotherapeutics are applied. Such compounds only differ in the radionuclide and therefore usually have a very similar if not identical pharmacokinetic profile. This can be realized using a chelator and a diagnostic or therapeutic radiometal. Alternatively, this can be realized using a precursor for radiolabeling and radiolabeling with either a diagnostic or a therapeutic radionuclide. In one embodiment diagnostic imaging is used preferably by means of quantification of the radiation of the diagnostic radionuclide and subsequent dosimetry which is known to those skilled in the art and the prediction of drug concentrations in the tumor compared to vulnerable side effect organs. Thus, a truly individualized drug dosing therapy for the patient is achieved.

In an embodiment and as preferably used herein, the theragnostic method is realized with only one theragnostically active compound such as a compound of the present invention labeled with a radionuclide emitting diagnostically detectable radiation (e.g. positrons or gamma rays) as well as therapeutically effective radiation (e.g. electrons or alpha particles).

The invention also contemplates a method of intraoperatively identifying/disclosing diseased tissues expressing GIPR in a subject. Such method uses a compound of the invention, whereby such compound of the invention preferably comprises as Effector a diagnostically active agent.

According to a further embodiment of the invention, the compound of the invention, particularly if complexed with a radionuclide, may be employed as adjunct or adjuvant to any other tumor treatment including, surgery as the primary method of treatment of most isolated solid cancers, radiation therapy involving the use of ionizing radiation in an attempt to either cure or improve the symptoms of cancer using either sealed internal sources in the form of brachytherapy or external sources, chemotherapy such as alkylating agents, antimetabolites, anthracyclines, plant alkaloids, topoisomerase inhibitors, and other antitumor agents, hormone treatments that modulate tumor cell behavior without directly attacking those cells, targeted agents which directly target a molecular abnormality in certain types of cancer including monoclonal antibodies and tyrosine kinase inhibitors, angiogenesis inhibitors, immunotherapy, cancer vaccination, palliative care including actions to reduce the physical, emotional, spiritual, and psycho-social distress to improve the patient's quality of life and alternative treatments including a diverse group of health care systems, practices, and products that are not part of conventional medicine.

In an embodiment of the methods of the invention, the subject is a patient. In an embodiment, a patient is a subject which has been diagnosed as suffering from or which is suspected of suffering from which or which is at risk of suffering from or developing a disease, whereby the disease is a disease as described herein and preferably a disease involving the GIPR.

Dosages employed in practicing the methods for treatment and diagnosis, respectively, where a radionuclide is used and more specifically attached to or part of the compound of the invention will vary depending e.g., on the particular condition to be treated, for example the known radiosensitivity of the tumor type, the volume of the tumor and the therapy desired. In general, the dose is calculated on the basis of radioactivity distribution to each organ and on observed target uptake. A γ-emitting complex may be administered once or at several times for diagnostic imaging. In animals, an indicated dose range may be from 0.1 µg/kg to 5 mg/kg of the compound of the invention complexed e.g., with 1 to 200 MBq of ¹¹¹In or ⁸⁹Zr. A β-emitting complex of the compound of the invention may be administered at several time points e.g., over a period of 1 to 3 weeks or longer. In animals, an indicated dosage range may be of from 0.1 µg/kg to 5 mg/kg of the compound of the invention complexed e.g., with 1 to 200 MBq ⁹⁰Y or ¹⁷⁷Lu. In larger mammals, for example humans, an indicated dosage range is from 0.1 to 100 µg/kg of the compound of the invention complexed with e.g., 10 to 400 MBq ¹¹¹In or ⁸⁹Zr. In larger mammals, for example humans, an indicated dosage range is of from 0.1 to 100 µg/kg of the compound of the invention complexed with e.g., 10 to 5000 MBq ⁹⁰Y or ¹⁷⁷Lu.

In a further aspect, the instant invention is related to a composition and a pharmaceutical composition in particular, comprising the compound of the invention.

The pharmaceutical composition of the present invention comprises at least one compound of the invention and, optionally, one or more carrier substances, excipients and/or adjuvants. The pharmaceutical composition may additionally comprise, for example, one or more of water, buffers such as, *e.g.,* neutral buffered saline or phosphate buffered saline, ethanol, mineral oil, vegetable oil, dimethylsulfoxide, carbohydrates such as *e.g.,* glucose, mannose, sucrose or dextrans, mannitol, proteins, adjuvants, polypeptides or amino acids such as glycine, antioxidants, chelating agents such as EDTA or glutathione and/or preservatives. Furthermore, one or more other active ingredients may, but need not, be included in the pharmaceutical composition of the invention.

The pharmaceutical composition of the invention may be formulated for any appropriate route of administration, including, for example, topical such as, e.g., transdermal or ocular, oral, buccal, nasal, vaginal, rectal or parenteral administration. The term parenteral as used herein includes subcutaneous, intradermal, intravascular such as, e.g., intravenous, intramuscular, intrathecal and intraperitoneal injection, as well as any similar injection or infusion technique. A preferred route of administration is intravenous administration.

In an embodiment of the invention the compound of the invention comprising a radionuclide is administered by any conventional route, in particular intravenously, e.g., in the form of injectable solutions or suspensions. The compound of the invention may also be administered advantageously by infusion, e.g., by an infusion of 30 to 60 min.

Depending on the site of the tumor, the compound of the invention may be administered as close as possible to the tumor site, e.g., by means of a catheter. Such administration may be carried out directly into the tumor tissue or into the surrounding tissue or into the afferent blood vessels. The compound of the invention may also be administered repeatedly in doses, preferably in divided doses.

According to a preferred embodiment of the invention, a pharmaceutical composition of the invention comprises a stabilizer, e.g., a free radical scavenger, which inhibits autoradiolysis of the compound of the invention. Suitable stabilizers include, e.g., serum albumin, ascorbic acid, retinol, gentisic acid or a derivative thereof, or an amino acid infusion solution such, e.g., used for parenteral protein feeding, preferably free from electrolyte and glucose, for example a commercially available amino acid infusion such as Proteinsteril^{®} KE Nephro. Ascorbic acid and gentisic acid are preferred.

A pharmaceutical composition of the invention may comprise further additives, e.g., an agent to adjust the pH between 7.2 and 7.4, e.g., sodium or ammonium acetate or Na₂HPO₄. Preferably, the stabilizer is added to the non-radioactive compound of the invention and introduction of the radionuclide, for instance the complexation with the radionuclide, is performed in the presence of the stabilizer, either at room temperature or, preferably, at a temperature of from 40 to 120° C. The complexation may conveniently be performed under air free conditions, e.g., under N₂ or Ar. Further stabilizer may be added to the composition after complexation.

Excretion of the compound of the invention, particularly if the Effector is a radionuclide, essentially takes place through the kidneys. Further protection of the kidneys from radioactivity accumulation may be achieved by administration of lysine or arginine or an amino acid solution having a high content of lysine and/or arginine, e.g., a commercially available amino acid solution such as Synthamin^{®}-14 or -10, prior to the injection of or together with the compound of the invention, particularly if the Effector is a radionuclide. Protection of the kidneys may also be achieved by administration of plasma expanders such as e.g., gelofusine, either instead of or in addition to amino acid infusion. Protection of the kidneys may also be achieved by administration of diuretics providing a means of forced diuresis which elevates the rate of urination. Such diuretics include high ceiling loop diuretics, thiazides, carbonic anhydrase inhibitors, potassium-sparing diuretics, calcium-sparing diuretics, osmotic diuretics and low ceiling diuretics. A pharmaceutical composition of the invention may contain, apart from a compound of the invention, at least one of these further compounds intended for or suitable for kidney protection, preferably kidney protection of the subject to which the compound of the invention is administered.

It will be understood by a person skilled in the art that the compound of the invention is disclosed herein for use in various methods. It will be further understood by a person skilled in the art that the composition of the invention and the pharmaceutical composition of the invention can be equally used in said various methods. It will also be understood by a person skilled in the art that the composition of the invention and the pharmaceutical composition are disclosed herein for use in various methods. It will be equally understood by a person skilled in the art that the compound of the invention can be equally used in said various methods.

It will be acknowledged by a person skilled in the art that the composition of the invention and the pharmaceutical composition of the invention contain one or more further compounds in addition to the compound of the invention. To the extent that such one or more further compounds are disclosed herein as being part of the composition of the invention and/or of the pharmaceutical composition of the invention, it will be understood that such one or more further compounds can be administered separately from the compound of the invention to the subject which is exposed to or the subject of a method of the invention. Such administration of the one or more further compounds can be performed prior, concurrently with or after the administration of the compound of the invention. It will also be acknowledged by a person skilled in the art that in a method of the invention, apart from a compound of the invention, one or more further compound may be administered to a subject. Such administration of the one or more further compounds can be performed prior, concurrently with or after the administration of the compound of the invention. To the extent that such one or more further compounds are disclosed herein as being administered as part of a method of the invention, it will be understood that such one or more further compounds are part of a composition of the invention and/or of a pharmaceutical composition of the invention. It is within the present invention that the compound of the invention and the one or more further compounds may be contained in the same or a different formulation. It is also within the present invention that the compound of the invention and the one or more further compounds are not contained in the same formulation, but are contained in the same package containing a first formulation comprising a compound of the invention, and a second formulation comprising the one or more further compounds, whereby the type of formulation may be the same or may be different.

It is within the present invention that more than one type of a compound of the invention is contained in the composition of the invention and/or the pharmaceutical composition of the invention. It is also within the present invention that more than one type of a compound of the invention is used, preferably administered, in a method of the invention.

It will be acknowledged that a composition of the invention and a pharmaceutical composition of the invention may be manufactured in conventional manner.

Radiopharmaceuticals have decreasing content of radioactivity with time, as a consequence of the radioactive decay. The physical half-life of the radionuclide is often short for radiopharmaceutical diagnostics. In these cases, the final preparation has to be done shortly before administration to the patient. This is in particular the case for positron emitting radiopharmaceuticals for tomography (PET radiopharmaceuticals). It often leads to the use of semi-manufactured products such as radionuclide generators, radioactive precursors and kits.

Preferably, a kit of the invention comprises apart from one or more than one compounds of the invention typically at least one of the followings: instructions for use, final preparation and/or quality control, one or more optional excipient(s), one or more optional reagents for the labeling procedure, optionally one or more radionuclide(s) with or without shielded containers, and optionally one or more device(s), whereby the device(s) is/are selected from the group comprising a labeling device, a purification device, an analytical device, a handling device, a radioprotection device or an administration device.

Shielded containers known as "pigs" for general handling and transport of radiopharmaceutical containers come in various configurations for holding radiopharmaceutical containers such as bottles, vials, syringes, etc. One form often includes a removable cover that allows access to the held radiopharmaceutical container. When the pig cover is in place, the radiation exposure is acceptable.

A labeling device is selected from the group of open reactors, closed reactors, microfluidic systems, nanoreactors, cartridges, pressure vessels, vials, temperature controllable reactors, mixing or shaking reactors and combinations thereof.

A purification device is preferably selected from the group of ion exchange chromatography columns or devices, size-exclusion chromatography columns or devices, affinity chromatography columns or devices, gas or liquid chromatography columns or devices, solid phase extraction columns or devices, filtering devices, centrifugations vials columns or devices.

An analytical device is preferably selected from the group of tests or test devices to determine the identity, radiochemical purity, radionuclidic purity, content of radioactivity and specific radioactivity of the radiolabelled compound.

A handling device is preferably selected from the group consisting of devices for mixing, diluting, dispensing, labeling, injecting and administering radiopharmaceuticals to a subject.

A radioprotection device is used in order to protect doctors and other personnel from radiation when using therapeutic or diagnostic radionuclides. The radioprotection device is preferably selected from the group consisting of devices with protective barriers of radiation-absorbing material selected from the group consisting of aluminum, plastics, wood, lead, iron, lead glass, water, rubber, plastic, cloth, devices ensuring adequate distances from the radiation sources, devices reducing exposure time to the radionuclide, devices restricting inhalation, ingestion, or other modes of entry of radioactive material into the body and devices providing combinations of these measures.

An administration device is preferably selected from the group of syringes, shielded syringes, needles, pumps, and infusion devices. Syringe shields are commonly hollow cylindrical structures that accommodate the cylindrical body of the syringe and are constructed of lead or tungsten with a lead glass window that allows the handler to view the syringe plunger and liquid volume within the syringe.

It is within the presen invention that the term "in an embodiment" or "in an embodiment of the invention" refers to each and any aspect of the invention, including any embodiment thereof.

The present invention is now further illustrated by reference to the following figures and examples from which further features, embodiments and advantages, may be taken, wherein
- Fig. 1: shows the amino acid sequence of human GIPR (SEQ ID NO: 1);
- Fig. 2: shows a representative blank corrected sensorgram for compound GIP-008; blank and reference cell corrected response (RU, Response Unit) data is shown as line; fitted curve shown as dotted black line;
- Fig. 3: shows SPECT/CT images of ¹¹¹In-GIP-001, ¹¹¹In-GIP-002, ¹¹¹In-GIP-003, ¹¹¹In-GIP-004, ¹¹¹In-GIP-005, ¹¹¹In-GIP-006, ¹¹¹In-GIP-007 3 h post injection (see, Example 32);
- Fig. 4: shows, indicated as %ID/g, quantified tumor uptake as determined by SPECT-imaging of ¹¹¹In-GIP-001, ¹¹¹In-GIP-002, ¹¹¹In-GIP-003, ¹¹¹In-GIP-004, ¹¹¹In-GIP-005, ¹¹¹In-GIP-006, ¹¹¹In-GIP-007 3 h post injection (see, Example 32); and
- Fig. 5: shows, indicated as %ID/g, quantified kidney uptake as determined by SPECT-imaging of ¹¹¹In-GIP-001, ¹¹¹In-GIP-002, ¹¹¹In-GIP-003, ¹¹¹In-GIP-004, ¹¹¹In-GIP-005, ¹¹¹In-GIP-006, ¹¹¹In-GIP-007 3 h post injection (see, Example 32).

The following Examples have been included to provide guidance to one of ordinary skill in the art for practicing representative embodiments of the presently disclosed subject matter. In light of the present disclosure and the general level of skill in the art, those of skill can appreciate that the following Examples are intended to be exemplary only and that numerous changes, modifications, and alterations can be employed without departing from the scope of the presently disclosed subject matter. The synthetic descriptions and specific examples that follow are only intended for the purposes of illustration, and are not to be construed as limiting in any manner to make compounds of the disclosure by other methods.

### EXAMPLES

Abbreviations used in the instant application and the following examples in particular are as follows:
- °C: means degree celsius
- ¹¹¹InCl₃: means Indium-111 chloride
- ¹⁷⁷LuCl₃: means Lutetium-177 chloride
- 4PL: means four parameter logistic
- ACN: means acetonitrile
- ADCC: means antibody-dependent cellular cytotoxicity
- AGLU: means 1-amino-1-deoxy-D-glucitol
- Alloc: means allyloxycarbonyl
- amu: means atomic mass unit
- aq.: means aqueous
- AUC: means area under the curve
- Bio: means D-Biotin
- BSA: means bovine serum albumin
- Calc: means Calculated
- cAMP: means cyclic adenosine monophosphate
- CAR-T: means chimeric antigen receptor T
- cDNA: means complementary DNA
- CHO: means chinese hamster ovarian
- CM: means ChemmatrixTM
- CMV: means cytomegalovirus
- CO₂: means carbon dioxide
- CT: means computed tomography
- Da: means Dalton
- DAD: means diode array detector
- DCM: means dichloromethane
- Dde: means N-(1-(4,4-dimethyl-2,6-dioxocyclohexylidene)ethyl)
- DEG: means di(ethylene glycol)
- DIC: means N,N'-Diisopropylcarbodiimide
- DICOM: means digital Imaging and Communications in Medicine
- DIPEA: means N,N-Diisopropylethylamine
- DMEM: means Dulbecco's Modified Eagle Medium
- DMF: means dimethylformamide
- DMSO: means dimethyl sulfoxide
- DOTA: means dodecane tetraacetic acid - 2,2′,2ʺ,2‴-(1,4,7,10-Tetraazacyclododecane-1,4,7,10-tetrayl)tetraacetic acid
- DOTA(OtBu)₃-OH: means DOTA-tris(tert-butyl ester), Tri-tert-butyl 1,4,7,10-tetraazacyclododecane-1,4,7,10-tetraacetate
- DOTA-NHS: means DOTA N-hydroxysuccinimide ester
- DPP4: means dipeptidyl peptidase-4
- DTPA: means Diethylenetriamine pentaacetate
- DTT: means Dithiothreitol
- e.g.: means for example (exempli gratia)
- EC₅₀: means half-maximal excitatory concentration
- EDT: means 1,2-ethanedithiol
- EDTA: means ethylenediaminetetraacetic acid
- ELSD: means Evaporative Light Scattering Detector
- eq.: means equivalent
- ESI: means electrospray ionization
- Et2O: means diethylether
- EtOAc: means ethylacetate
- FACS: means fluorescence-activated cell sorting
- FCS: means fetal calf serum
- Fmoc: means 9-Fluorenyloxycarbonyl
- GBq: means gigabecquerel
- GHRH: means Growth hormone-releasing hormone
- GIP: means glucose-dependent insulinotropic polypeptide
- GIPR: means glucose-dependent insulinotropic polypeptide receptor
- GLP-1: means Glucagon-like peptide-1
- GLP-2: means Glucagon-like peptide-2
- G-PCR: means G-protein coupled receptor
- h: means hour(s)
- H₂O: means water
- HATU: means Hexafluorophosphate Azabenzotriazole Tetramethyl Uronium-1-[Bis(dimethylamino)methylene]-1H-1,2,3-triazolo[4,5-b]pyridinium 3-oxide hexafluorophosphate
- HCl: means Hydrogen chloride
- HEK: means Human embryonic kindey
- HEPES: means 4-(2-hydroxyethyl)-1-piperazineethanesulfonic acid
- HFIP: means hexafluoro isopropanol
- HOAc: means acetic acid
- HPLC: means high performance liquid chromatography
- i.e.: means that is (id est)
- IC50: means half-maximal inhibitory concentration
- ID: means injected dose
- ID/g: means injected dose per gram
- IDBS: means ID Business Solutions
- K_{D}: means dissociation constant
- kDa: means 1000 Dalton or Kilo
- kDa: means kilo Dalton
- Kᵢ: means inhibitory constant
- k_{off}: means dissociation rate
- kₒₙ: means association rate
- kVp: means peak kilovoltage
- LC: means Liquid chromatography
- LC/TOF-MS: means liquid chromatography time-of-flight mass spectrometry
- LC-MS: means Liquid chromatography mass spectrometry
- M: means molar or mol per Liter
- m/z: means mass divided by charge
- MAPK: means mitogen-activated protein kinase
- max.: means maximum
- MBq: means megabecquerel
- MeCN: means acetonetrile
- MeOH: means methanol
- MeV: means mega electron volt
- MFI: means Median flourescence intensities
- min: means minute(s)
- MTBE: means methyl-tert-butyl ether
- Mtt: means 4-Methyltrityl
- MW: means molecular weight
- n.d.: means not determined
- N3Tap: means L-(4R)-Azidoproline
- NaCl: means sodium chloride
- NaHCO₃: means sodium hydrogencarbonate
- NEP: means neutral endopeptidase
- NET: means neuroendocrine tumor
- NHS: means N-hydroxysuccinimide
- NMP: means N-Methyl-2-pyrrolidone
- NMR: means Nuclear Magnetic Resonance
- OtBu: means tert Butyl ester as protecting group
- p.a.: means for analytical purpose (quality grade)
- PARP: means Poly(ADP-ribose) polymerases
- PBS: means phosphate buffered saline
- PBST: means phosphate buffered saline containing Tween
- pEC₅₀: means the negative log of the EC50 value when converted to molar
- PET: means positron emission tomography
- pH: means potential of hydrogen
- pIC₅₀: means the negative log of the IC50 value when converted to molar
- pK_{D}: means negative decadic logarithm of dissociation constant
- ppm: means parts per million
- prep.: means preparative
- PRRT: means peptide receptor radionuclide therapy
- PS: means Polystyrene
- Q-TOF: means quadrupole time-of-flight
- RFU: means relative fluorescence unit
- RLU: means relative luminescence values
- RP: means reversed phase
- RPM: means rounds per minute
- RPMI: means Roswell Park Memorial Institute
- RT: means room temperature
- Rₜ: means retention time
- RU: means resonance units
- SCID: means severe combined immunodeficiency
- SCK: means Single cycle kinetic
- sec: means second
- SPECT: means single photon emission computed tomography
- SPPS: means solid phase peptide synthesis
- SPR: means surface plasmon resonance
- t_{1/2}: means Dissociation half-life, equals In 2/k_{off} for 1^{st} order dissociation process
- tBu: means tert Butyl ether as protecting group
- tBuO: means tert Butyl ester as protecting group
- TFA: means trifluoroacetate or trifluoroacetic acid
- TG: means tentagel
- TIPS: means triisopropyl silane
- TLC: means thin layer chromatography
- TOF-MS: means tof-of-flight mass spectrometry
- UHPLC: means ultrahigh performance liquid chromatography
- UV: means ultraviolet
- VGT: means vertical gene transfer

### Example 1: Material and Methods

The materials and methods as well as general methods are further illustrated by the following examples.

### Solvents:

Solvents were used in the specified quality without further purification. Acetonitrile (Super Gradient, HPLC, VWR - for analytical purposes; PrepSolv, Merck - for preparative purposes); dichloromethane (synthesis, Roth); ethyl acetate (synthesis grade, Roth); N,N-dimethylformamide (peptide synthesis grade, Biosolve); 1-methyl-2-pyrolidone (peptide grade, IRIS BioTech) 1,4-dioxane (reinst, Roth); methanol (p. a., Merck).

Water: Milli-Q Plus, Millipore, demineralized.

### Chemicals:

Chemicals were synthesized according to or in analogy to literature procedures or purchased from Sigma-Aldrich-Merck (Deisenhofen, Germany), Bachem (Bubendorf, Switzerland), VWR (Darmstadt, Germany), Novabiochem (Merck Group, Darmstadt, Germany), Acros Organics (distribution company Fisher Scientific GmbH, Schwerte, Germany), Iris Biotech (Marktredwitz, Germany), Amatek Chemical (Jiangsu, China), Roth (Karlsruhe, Deutschland), Molecular Devices (Chicago, USA), Biochrom (Berlin, Germany), Peptech (Cambridge, MA, USA), Synthetech (Albany, OR, USA), Pharmacore (High Point, NC, USA), PCAS Biomatrix Inc (Saint-Jean-sur-Richelieu, Quebec, Canada), Alfa Aesar (Karlsruhe, Germany), Tianjin Nankai Hecheng S&T Co., Ltd (Tianjin, China), CheMatech (Dijon, France), JenKem Technology (Plano, TX, USA), Trimen Chemicals (Lodz, Poland) and Anaspec (San Jose, CA, USA) or other companies and used in the assigned quality without further purification.

Boc₄N4Ac-OH was synthesized according to a literature procedure (Maecke et al. Chem. Eur. J., 2010, 16, 7, 2115).

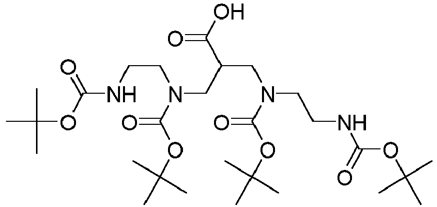

AGLU^{∗}-OH was synthesized according to a literature procedure (Vlahov et al. J. Org. Chem., 2010, 75, 3685).

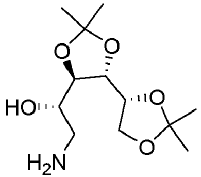

β-alanine allyl ester hydrochlorid (H₂N-CH₂CH₂-CO₂Allyl) was synthesized as follows:

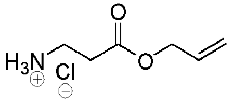

2g β-alanine (22 mmol) were dissolved in 40 mL of allyl alcohol in a 100 mL three-neck-flask. The reaction flask was equipped with a capillary dipping into the solution which was further connected with suitable tubing to a two-neck flask filled with sodium chloride (approx. 100 g) via a washing bottle. The sodium chloride filled two-neck flask was equipped with a dropping funnel filled with 30 mL sulphuric acid. The sulphuric acid was then slowly added dropwise to the sodium chloride thereby releasing gaseous HCl which was guided into the stirred solution of β-alanine in allyl alcohol. After the addition of the sulphuric acid was complete the solution of β-alanine was stirred overnight. Then all volatiles were evaporated, the remainder lyophilized from water and acetonitrile to yield 3.5g (95%) of the target compound which was used without any further purification. ¹H-NMR: (500 MHz, CDCl₃): δ 2.90 (2H, t, *J* = 6.7 Hz), 3.30 (2H, t, *J* = 6.7 Hz), 4.55 (2H, d, *J* = 4.5 Hz), 5.15 (1H, dd, *J* = 9.5, 0.9 Hz), 5.27 (1H, dd, *J* = 16.19, 0.9 Hz), 5.85 (1H, ddt, *J* = 16.19, 9.5, 4.5 Hz).

### HPLC/MS analyses

HPLC/MS analyses were performed by injection of 5 µL of a solution of the sample, using a 2 step gradient for all chromatograms (5-65% B in 12 min, followed by 65-90% in 0.5 min, A: 0.1% TFA in water and B: 0.1% TFA in ACN). RP columns were from Agilent (Type Poroshell 120, 2.7µm, EC-C18, 50 × 3.00 mm, flow 0.8 mL, HPLC at room temperature); Mass spectrometer: Agilent 6230 LC/TOF-MS, ESI ionization. MassHunter Qualitative Analysis B.07.00 SP2 was used as software. UV detection was done at λ = 230 nm. Retention times (Rₜ) are indicated in the decimal system (e.g. 1.9 min = 1 min 54 s) and are referring to detection in the UV spectrometer. For the evaluation of observed compound masses the 'Find Compounds by Formula'-feature was used. In particular, the individual 'neutral mass of a compound (in units of Daltons)'-values and the corresponding isotope distribution pattern were used to confirm compound identity. The accuracy of the mass spectrometer was approx. ± 5 ppm.

### HPLC/ELSD analyese:

HPLC/ELSD analyses of PEGylated peptides with a polydisperse PEG were performed by injection of 25 µL of a solution of the sample, using a 2 step gradient for all chromatograms (20-60% B in 20 min, followed by 60-90% in 5 min, A: 0.1% TFA in water and B: 0.1% TFA in ACN). RP columns were from Vydac (Type PROTEIN & PEPTIDE C18, 5µm, 150 × 2.1 mm, flow 0.8 mL, HPLC at 50 °C). UV detection was done at λ = 230 nm. For ELSD detection of the PEGylation reagent an Alltech 3300 ELSD device (operating temperature 40 °C) was employed. Retention times (Rₜ) are indicated in the decimal system (e.g. 1.9 min = 1 min 54 s) and are referring to detection in the UV spectrometer.

### Preparative HPLC:

Preparative HPLC separations were done with reversed phase columns (General: Kinetex 5µ XB-C18 100 Å, 150 × 30 mm from Phenomenex; for PEGylated peptides Pursuit 5 C18 200 Å, 150 × 30 mm from Varian) as stationary phase. As mobile phase 0.1% TFA in water (A) and 0.1% TFA in ACN (B) were used which were mixed in linear binary gradients. The gradients are described as: "10 to 40% B in 30 min", which means a linear gradient from 10% B (and correspondingly 90% A) to 40% B (and correspondingly 60% A) was run within 30min. Flowrates were within the range of 30 to 50 mL/min. A typical gradient for the purification of the compounds of the invention started at 5-25% B and ended after 30 min at 35-50% B and the difference between the percentage B at end and start was at least 10%. A commonly used gradient was "15 to 40% B in 30 min". Samples were dissolved preferably in mixtures of HOAc and water or DMSO.

### Solid phase extraction:

In case of solid phase extraction 250 mg Varian Bondesil-ENV was placed in a 15 mL polystyrene syringe, pre-washed with methanol (1 × 5 mL) and water (2 × 5 mL). Then the reaction solution or the solution containing the product to be purified was applied to the column. Thereafter elution was performed with water (2 × 5 mL - to remove excess salt), 5 mL of 50% ACN in water as first fraction and each of the next fractions were eluted with 5 mL of 50% ACN in water containing 0.1% TFA.

### General procedures for Automated/Semi-automated Solid-Phase Synthesis:

Automated solid-phase of peptides and polyamides was performed on a Tetras Peptide Synthesizer (Advanced ChemTech) in 50 µmol and 100 µmol scales. Manual steps were performed in plastic syringes equipped with frits (material PE, Roland Vetter Laborbedarf OHG, Ammerbuch, Germany). The amount of reagents in the protocols described corresponds to the 100 µmol scale, unless stated otherwise.

Solid-phase synthesis was performed on polystyrene (cross linked with 1,4-divinylbenzene (PS) or di (ethylene glycol) dimethacrylate (DEG)), ChemMatrix (CM) or TentaGel (TG) resin. Resin linkers were trityl, wang and rink amide.

### Resin loading:

In case of the trityl linker the attachment of the first building block (resin loading) was performed as follows. The resin (polystyrene (PS) trityl chloride, initial loading: 1.8 mmol/g) was swollen in DCM (5 mL) for 30 min and subsequently washed with DCM (3 mL, 1 min). Then the resin was treated with a mixture of the corresponding building block (0.5 mmol, 5 eq.) and DIPEA (350 µL, 3.5 mmol, 35 eq.) in DCM (4 mL) for 1 hour. Afterwards the resin was washed with methanol (5 mL, 5 min) and DMF (3 mL, 2x 1 min).

In case of the Wang linker pre-loaded resins (polystyrene (PS) and TentaGel (TG)) were employed.

In case of the rink amide linker the attachment of the first residue the resin (CM, DEG) was performed with the same procedure as for the chain assembly as described below.

### AlloclAllyl-deprotection:

After swelling in DMF, the resin was washed with DMF and DCM. DCM was de-oxygenated by passing a stream of nitrogen through the stirred solvent. The oxygen-free solvent was used to wash the resin trice. Then 2 mL of a 2 M solution of barbituric acid in oxygen-free DCM and 1 mL of a 25 µM solution of Tetrakis(triphenylphosphine)palladium(0) in oxygen-free DCM were added to the resin. The resin was agitated for 1 hour and then washed with DCM, MeOH, DMF, 0.5% DIPEA in DMF, 0.5% dithiocarbamate in DMF, DMF and DCM (each washing step was repeated 3 times with 3 mL, 1 min).

### Fmoc-deprotection:

After swelling in DMF, the resin was washed with DMF and then treated with piperidine/DMF (1:4, 3 mL, 2 and 20 min) and subsequently washed with DMF (3 mL, 5× 1 min).

### Dde-deprotection:

After swelling in DMF, the resin was washed with DMF and then treated with hydrazine-hydrate/DMF (2/98, 3 mL 2x 10 min) and subsequently washed with DMF (3 mL, 5× 1 min).

### Mtt-deprotection:

After swelling in DCM, the resin was washed with DCM and then treated with HFIP/DCM (7/3, 4 - 6 mL, 4 hours) and subsequently washed with DCM (3 mL, 3× 1 min), DMF (3 mL, 3× 1 mL) and DIPEA (0.9 M in DMF, 3 mL, 1 min).

### Solutions of reagents:

Building Blocks (0.3 M in DMF or NMP), DIPEA (0.9 M in DMF), HATU (0.4 M in DMF), Acetic anhydride (0.75 M in DMF), DIC (3.2 M in DMF)

### Coupling: Coupling of building blocks/amino acids (chain assembly):

Unless otherwise stated, coupling of building blocks was performed as follows: After subsequent addition of solutions of the corresponding building block (0.9 mL, 5eq.), DIPEA solution (0.6 mL, 10 eq.) and HATU solution (0.65 mL, 5 eq.) the resin was shaken for 45 min. If necessary, the resin was washed with DMF (3 mL, 1 min) and the coupling step was repeated.

In case DOTA(OtBu)₃-OH was coupled as a building block the coupling was performed for 90 min. After this timespan DIC solution was added (0.2 mL, 12.5 eq.) was added and the reaction mixture was agitated for further 90 min.

### Terminal acetylation:

After addition of DIPEA solution (1.75 mL, 16 eq.) and acetic anhydride solution (1.75 mL, 13 eq.) the resin was shaken for 10 min. Afterwards the resin was washed with DMF (3 mL, 6x 1 min).

### Cleavage method A: Cleavage of protected fragments from hyper-acid labile resin:

After the completion of the assembly of the sequence the resin was finally washed with DCM (3 mL, 4x 1 min) and then dried in the vacuum. Then the resin was treated with HFIP/DCM (7/1, 4 mL, 4 hours) and the collected solution evaporated to dryness. The residue was purified with preparative HPLC or used without further purification.

### Cleavage method B: Cleavage of unprotected fragments (complete resin cleavage):

After the completion of the assembly of the sequence the resin was finally washed with DCM (3 mL, 4× 1 min), dried in the vacuum overnight and treated with TFA, EDT, water and TIPS (94/2.5/2.5/1) for 4 h (unless otherwise stated). Afterwards the cleavage solution was poured into a chilled mixture of MTBE and cyclohexane (1/1, 10-fold excess compared to the volume of cleavage solution), centrifuged at 4 °C for 5 min and the precipitate collected and dried in the vacuum. The residue was lyophilized from water/acetonitrile prior to purification or further modification.

### Cleavage method C: Cleavage of protective groups of peptides in solution

The protected/partially protected compound was dissolved in TFA, water and TIPS (95/2.5/2.5) for 2 h (unless otherwise stated). Afterwards the cleavage solution was poured into a chilled mixture of MTBE and cyclohexane (1/1, 10-fold excess compared to the volume of cleavage solution), centrifuged at 4 °C for 5 min and the precipitate collected and dried in the vacuum. The residue was lyophilized from water/acetonitrile prior to purification or further modification.

### Cleavate method D: Cleavage of unprotected fragments with Reagent K (complete resin cleavage):

After the completion of the assembly of the sequence the resin was finally washed with DCM (3 mL, 4× 1 min), dried in the vacuum overnight and treated with TFA, phenol, water, thioanisole and EDT (82.5/5/5/5/2.5) for 4 h (unless otherwise stated). Afterwards the cleavage solution was poured into a chilled mixture of MTBE and cyclohexane (1/1, 10-fold excess compared to the volume of cleavage solution), centrifuged at 4 °C for 5 min and the precipitate collected and dried in the vacuum. The residue was lyophilized from water/acetonitrile prior to purification or further modification.

More relevant Fmoc-solid-phase-peptide synthesis methods are described in detail in "Fmoc Solid Phase Peptide Synthesis" Editors W. Chan, P. White, Oxford University Press, USA, 2000. Compounds were named using MestreNova version 12 Mnova IUPAC Name plugin (Mestrelab Research, S.L.), or AutoNom version 2.2 (Beilstein Informationssysteme Copyright^{©} 1988-1998, Beilstein Institut für Literatur der Organischen Chemie licensed to Beilstein Chemiedaten and Software GmbH, where appropriate.

### Solution Cyclization method A: Dibromoxylene or bis-(bromomethyl)-pyridin cyclization

The crude peptide material was dissolved in a 1:1 mixture of ammonium bicarbonate solution (50 mM, pH = 8.5) and acetonitrile. To the resulting mixture a solution of α,α'-dibromo-m-xylene or 2,6-bis(bromomethyl)pyridine in acetonitrile was added. Upon completition of the cyclization reaction which was judged by analytical LC-MS, DTT was added and the solution stirred for a further hour. Then TFA was added and the reaction solution subjected to lyophilization. The volume of solvent, amount of α,α'-dibromo-m-xylene or 2,6-bis(bromomethyl)pyridine, DTT and volume of TFA used in the reaction depended on the amount of resin used for the synthesis of the linear peptide precursor - per 50 µmol of initially used 60 mL of the solvent mixture, 14.5 mg (55 µmol) of α,α'-dibromo-m-xylene or 14.5 mg (55 µmol), 15.4 mg (100 µmol) of DTT and 50 µL of TFA were used.

### Solution Cyclization method B: Cyclization with Maleimide Derivatives

The crude peptide material was dissolved in a 1:1 mixture of ammonium bicarbonate (0.1 M, pH = 8) and acetonitrile. To the resulting mixture a solution of the corresponding 3,4-dibromomaleimide in acetonitrile was added. Upon completion of the cyclization reaction which was judged by LC-MS, TFA was added and the reaction solution subjected to lyophilization. The volume of solvent, amount of 3,4-dibromomaleimide and volume of TFA used in the reaction depended on the amount of resin used for the synthesis of the linear peptide precursor - per 50 µmol of initially used resin 60 mL of the solvent mixture, 14.0 mg (55 µmol) of 3,4-dibromomaleimide and 50 µL of TFA were used.

### Solution Cyclization method C: Disulfide cyclization

The crude peptide material was dissolved in a 1:1 mixture of acetonitrile and ammonium acetate buffer (0.1M, pH 6). To the solution [Pt(en)₂Cl₂]Cl₂ (Dichlorobis-(ethylendiamine)-platinum(IV) chloride) was added. Upon completition of the cyclization reaction which was judged by analytical LC-MS, TFA was added and the reaction solution subjected to lyophilization. The volume of solvent, amount of Pt-reagent and volume of TFA used in the reaction depended on the amount of resin used for the synthesis of the linear peptide precursor - per 50 µmol of initially used 60 mL of the solvent mixture, 22.8 mg (50 µmol) of Pt-reagent and 50 µL of TFA were used.

### Preparation of compounds:

Specific embodiments for the preparation of compounds of the invention are prepared by using the preferred general methods disclosed above and other published or other methods which are known to persons skilled in the art Unless otherwise specified, all starting materials and reagents are of standard commercial grade, and are used without further purification, or are readily prepared from such materials by routine methods. Those skilled in the art of organic synthesis will recognize in light of the instant disclosure that starting materials and reaction conditions may be varied including additional steps employed to produce compounds encompassed by the present invention. Since one important step is the cyclization towards different cyclic structures these general methods are disclosed below in a separate section.

### Example 2: Synthesis of H-Met-Tyr-Asp-Trp-Trp-Pro-Glu-Leu-[Cys(3MeBn)-Ala-Leu-Ile-Cys]-Ttds-Lys(Bio)-NH₂ (GIP-057)

The linear sequence (H-Met-Tyr-Asp-Trp-Trp-Pro-Glu-Leu-[Cys(3MeBn)-Ala-Leu-Ile-Cys]-Ttds-Lys(Bio)-NH₂) of the peptide was assembled according to the 'General procedures for Automated/Semi-automated Solid-Phase Synthesis' in a 100 µmol scale on a Rink amide resin. After performing the steps of *'Cleavage method B'* (2 hours) the lyophilized crude peptide residue was subjected to '*Solution Cyclization method A'* with dibromoxylene as cyclization reagent. After lyophilization the crude product was purified by preparative HPLC (35 to 65% B in 30 min - Kinetex) to yield 9.38 mg of the pure title compound (15.0%). HPLC t_{R} = 9.24 min. LC/TOF-MS: exact mass 2399.129 (calculated 2399.123). C₁₁₅H₁₆₆N₂₂O₂₆S₄ (MW = 2400.949).

### Example 3: Synthesis of DOTA-Ttds-Tyr-Asp-Trp-Trp-Pro-Glu-Leu-[Cys(3MeBn)-Ala-Leu-Ile-Cys]-NH₂ (GIP-126)

The linear sequence (DOTA-Ttds-Tyr-Asp-Trp-Trp-Pro-Glu-Leu-Cys-Ala-Leu-Ile-Cys-NH₂) of the peptide was assembled according to the 'General procedures for Automated/Semi-automated Solid-Phase Synthesis' in a 50 µmol scale on a Rink amide resin. After performing the steps of *'Cleavage method B'* the lyophilized crude peptide residue was subjected to '*Solution Cyclization method A'* with dibromoxylene as cyclization reagent. After lyophilization the crude product was purified by preparative HPLC (35 to 60% B in 30 min-Kinetex) to yield 16.11 mg of the pure title compound (14.0%). HPLC t_{R} = 9.29 min. LC/TOF-MS: exact mass 2300.097 (calculated 2300.090). C₁₁₀H₁₅₇N₂₁O₂₉S₂ (MW = 2301.683).

### Example 4: Synthesis of Ac-Tyr-Asp-Trp-Trp-Pro-Glu-Leu-[Cys(3MeBn)-Ala-Leu-Ile-Cys]-Ttds-Lys(DOTA)-NH₂ (GIP-127)

The linear sequence (Ac-Tyr-Asp-Trp-Trp-Pro-Glu-Leu-Cys-Ala-Leu-Ile-Cys-Ttds-Lys(Mtt)-NH₂) of the peptide was assembled according to the 'General procedures for Automated/Semi-automated Solid-Phase Synthesis' in a 50 µmol scale on a Rink amide resin. The Mtt-group was removed by performing the '*Mtt-deprotection*' as described in the 'General procedures for Automated/Semi-automated Solid-Phase Synthesis', DOTA(OtBu)₃-OH was coupled and the peptide cleaved from the resin by performing the steps of *'Cleavage method B'* (4 hours cleavage time). The lyophilized crude peptide residue was subjected to '*Solution Cyclization method A* ' with dibromoxylene as cyclization reagent. After lyophilization the crude product was purified by preparative HPLC (35 to 60% B in 30 min - Kinetex) to yield 10.37 mg of the pure title compound (8.4%). HPLC t_{R} = 9.37 min. LC/TOF-MS: exact mass 2470.202 (calculated 2470.195). C₁₁₈H₁₇₁N₂₃O₃₁S₂ (MW = 2471.893).

### Example 5: Synthesis of DOTA-Ttds-Asp-Trp-Trp-Pro-Glu-leu-[Cys(3MeBn)-Aib-Leu-Ile-AET] (GIP-129)

The linear sequence (DOTA-Ttds-Asp-Trp-Trp-Pro-Glu-leu-Cys-Aib-Leu-Ile-AET-H) of the peptide was assembled according to the 'General procedures for Automated/Semi-automated Solid-Phase Synthesis' in a 50 µmol scale on a Cysteamine-4-methoxytrityl resin. After performing the steps of *'Cleavage method B'* the lyophilized crude peptide residue was subjected to '*Solution Cyclization method A'* with dibromoxylene as cyclization reagent. After lyophilization the crude product was purified by preparative HPLC (30 to 60% B in 30 min-Kinetex) to yield 43.69 mg of the pure title compound (41.4%). HPLC t_{R} = 8.83 min. LC/TOF-MS: exact mass 2108.044 (calculated 2108.036). C₁₀₁H₁₄₉N₁₉O₂₆S₂ (MW = 2109.512).

### Example 6: Synthesis of 4OHPhp-Asp-Trp-Trp-Pro-Glu-leu-[Cys(3MeBn)-Aib-Leu-Ile-Cys]-Ttds-Lys(DOTA)-NH₂ (GIP-137)

The linear sequence (4OHPhp-Asp-Trp-Trp-Pro-Glu-leu-Cys-Aib-Leu-Ile-Cys-Ttds-Lys(Mtt)-NH₂) of the peptide was assembled according to the 'General procedures for Automated/Semi-automated Solid-Phase Synthesis' in a 50 µmol scale on a Rink amide resin. After the coupling of 3-(4-Hyrdoxyphenyl) propionic acid, the Mtt-group was removed by performing the '*Mtt-deprotection*' as described in the 'General procedures for Automated/Semi-automated Solid-Phase Synthesis', DOTA(OtBu)₃-OH was coupled and the peptide cleaved from the resin by performing the steps of *'Cleavage method B'* (4 hours cleavage time). The lyophilized crude peptide residue was subjected to '*Solution Cyclization method A'* with dibromoxylene as cyclization reagent. After lyophilization the crude product was purified by preparative HPLC (35 to 65% B in 30 min - Kinetex) to yield 14.21 mg of the pure title compound (11.7%). HPLC t_{R} = 9.04 min. LC/TOF-MS: exact mass 2427.198 (calculated 2427.190). C₁₁₇H₁₇₀N₂₂O₃₀S₂ (MW = 2428.868).

### Example 7: Synthesis of HO-Succinyl-Trp-1Ni-Pro-Glu-Leu-[Cys(3MeBn)-Aib-Leu-Ile-Cys]-Ttds-Lys(DOTA)-NH₂ (GIP-085)

The linear sequence (tBuO-Succinyl-Trp-1Ni-Pro-Glu-Leu-Cys-Aib-Leu-Ile-Cys-Ttds-Lys(Mtt)-NH₂) of the peptide was assembled according to the 'General procedures for Automated/Semi-automated Solid-Phase Synthesis' in a 50 µmol scale on a Rink amide resin. After the coupling of mono-tert.-butyl succinate, the Mtt-group was removed by performing the '*Mtt-deprotection*' as described in the 'General procedures for Automated/Semi-automated Solid-Phase Synthesis', DOTA(OtBu)₃-OH was coupled and the peptide cleaved from the resin by performing the steps of *'Cleavage method B*'. The lyophilized crude peptide residue was subjected to '*Solution Cyclization method A* ' with dibromoxylene as cyclization reagent. After lyophilization the crude product was purified by preparative HPLC (35 to 65% B in 30 min-Kinetex) to yield 15.17 mg of the pure title compound (13.3%). HPLC t_{R} = 10.40 min. LC/TOF-MS: exact mass 2275.133 (calculated 2275.131). C₁₁₀H₁₆₂N₂₀O₂₈S₂ (MW = 2276.717).

### Example 8: Synthesis of DOTA-APAc-Asp-Trp-5Clw-Hyp-Glu-leu-[Dap(2Lut)-Thp-Leu-Ile-AET] (GIP-106)

The linear sequence (DOTA-Ttds-Asp-Trp-Trp-Pro-Glu-leu-Dap-Aib-Leu-Ile-AET-H) of the peptide was assembled according to the 'General procedures for Automated/Semi-automated Solid-Phase Synthesis' in a 100 µmol scale on a Cysteamine-4-methoxytrityl resin. After performing the steps of *'Cleavage method B'* the lyophilized crude peptide residue was subjected to '*Solution Cyclization method A'* with bis-(bromomethyl)-pyridin as cyclization reagent. After lyophilization the crude product was purified by preparative HPLC (20 to 45% B in 30 min - Kinetex) to yield 10.58 mg of the pure title compound (5.2%). HPLC t_{R} = 7.57 min. LC/TOF-MS: exact mass 2021.894 (calculated 2021.948). C₉₅H₁₃₆ClN₂₁O₂₄S (MW = 2023.745).

### Example 9: Synthesis of HO-Succinyl-Trp-5Clw-Hyp-Glu-leu-[Cys(2Lut)-Apc(DOTA)-Leu-Ile-AET] (GIP-110)

The linear sequence (tBuO-Succinyl-Trp-5Clw-Hyp-Glu-leu-Cys-Apc-Leu-Ile-AET-H) of the peptide was assembled according to the 'General procedures for Automated/Semi-automated Solid-Phase Synthesis' in a 100 µmol scale on a Cysteamine-4-methoxytrityl resin. After performing the steps of *'Cleavage method B'* the lyophilized crude peptide residue was subjected to '*Solution Cyclization method A'* with bis-(bromomethyl)-pyridin as cyclization reagent. Prior to the coupling of DOTA the cyclic intermediate peptide (HO-Succinyl-Trp-5Clw-Hyp-Glu-leu-[Cys(2Lut)-Apc-Leu-Ile-AET]) was purified by purified by preparative HPLC (25 to 55% B in 30 min - Kinetex) to yield 83.96 mg of the peptide precursor (56.0%). 40 mg of the intermediate peptide (26.7 µmol) were dissolved in DMSO (400 µL) and the pH value of the solution adjusted to 8 by addition of DIPEA (8 µL). DOTA-NHS (32.3 mg, 40 µmol, 1.5 eq.) was added and the pH value re-adjusted to 8 by addition of further DIPEA. After completion of the reaction the solution was subjected to preparative HPLC (25 to 50% B in 30 min - Kinetex) to yield 28.8 mg of the pure title compound (57.2% - yield for coupling of DOTA - overall yield: 32.0%). HPLC t_{R} = 8.68 min. LC/TOF-MS: exact mass 1882.827 (calculated 1882.819). C₈₈H₁₂₃ClN₁₈O₂₂S₂ (MW = 1884.614).

### Example 10: Synthesis of AGLU-Glutar-Asp-Trp-5Clw-Hyp-Glu-leu-[Cys(2Lut)-Thp-Leu-Ile-Cys]-APAc-glu(AGLU)-Lys(DOTA)-NH₂ (GIP-004)

The linear sequence (HO-Glutar-Asp-Trp-5Clw-Hyp-Glu-leu-Cys-Thp-Leu-Ile-Cys-APAc-glu(OAll)-Lys(Dde)-NH₂) of the peptide was assembled according to the 'General procedures for Automated/Semi-automated Solid-Phase Synthesis' in a 50 µmol scale on a Rink amide resin. The N-terminal Glutaric acid was introduced by reaction of glutaric acid anhydride (5 eq., incl. DIPEA 10 eq.) with peptide resin. The side chain of the C-terminal lysine was liberated from the Dde-group by executing the '*Dde-deprotection*' followed by the coupling of DOTA(OtBu)₃-OH to the previously liberated amino function. In contrast to the general procedure in the present case during the coupling of the DOTA building block no DIC was added. Afterwards, the allyl ester protecting group was removed acid by performing the steps of the '*Alloc*/*Allyl-deprotection*' from D-glutamic acid. AGLU^{∗} was coupled simultaneously to the side chain of the latter and the free N-terminal carboxylic acid function of the peptide resin (AGLU^{∗} (195 mg, 7.5 eq.), Oxyma (107 mg, 7.5 eq.) and DIC (116 µL, 7.5 eq.), DMF, overnight at 50 °C). Thereafter the steps of *'Cleavage method B*' where performed to cleave the peptide from the resin. The lyophilized crude peptide residue was subjected to '*Solution Cyclization method A'* with bis-(bromomethyl)-pyridin as cyclization reagent. After lyophilization the crude product was purified by preparative HPLC (25 to 40% B in 30 min-Kinetex) to yield 10.29 mg of the pure title compound (7.4%). HPLC t_{R} = 7.02 min. LC/TOF-MS: exact mass 2779.260 (calculated 2779.253). C₁₂₄H₁₈₇ClN₂₆O₄₀S₂ (MW = 2781.552).

### Example 11: Synthesis of HO-Succinyl-Trp-5Clw-Hyp-Glu-leu-[Cys(2Lut)-Apc(AGLU-Glutar)-Leu-Ile-Cys]-APAc-Lys(DOTA)-NH₂ (GIP-073)

The linear sequence (HO-Glutar-Asp-Trp-5Clw-Hyp-Glu-leu-Cys-Apc(Alloc)-Leu-Ile-Cys-APAc-Lys(Mtt)-NH₂) of the peptide was assembled according to the 'General procedures for Automated/Semi-automated Solid-Phase Synthesis' in a 50 µmol scale on a Rink amide resin. After the coupling of mono-tert.-butyl succinate, the Mtt-protecting group was removed by performing the '*Mtt-deprotection*' as described in the 'General procedures for Automated/Semi-automated Solid-Phase Synthesis', DOTA(OtBu)₃-OH was coupled and the Alloc protecting group removed from the side chain of the Apc building block. Glutaric acid was coupled to Apc side chain by reaction of the peptide resin with glutaric acid anhydride (31 mg, 5 eq., DIPEA (48 µL, 5eq.), DMF, 45 min, room temperature). AGLU^{∗} was coupled to the carboxylic acid function of the glutaric acid (AGLU^{∗} (195 mg, 7.5 eq.), Oxyma (107 mg, 7.5 eq.) and DIC (116 µL, 7.5 eq.), DMF, overnight at 50 °C). Thereafter the steps of '*Cleavage method B*' where performed to cleave the peptide from the resin. The lyophilized crude peptide residue was subjected to '*Solution Cyclization method A'* with bis-(bromomethyl)-pyridin as cyclization reagent. After lyophilization the crude product was purified by preparative HPLC (25 to 50% B in 30 min - Kinetex) to yield 14.15 mg of the pure title compound (11.4%). HPLC t_{R} = 7.78 min. LC/TOF-MS: exact mass 2471.139 (calculated 2471.131). C₁₁₃H₁₆₇ClN₂₄O₃₂S₂ (MW = 2473.266).

### Example 12: Synthesis of AGLU-Glutar-Asp-Trp-5Clw-Hyp-Glu-leu-[Cys(2Lut)-Apc(DOTA)-Leu-Ile-Cys]-APAc-glu(AGLU)-NH₂ (GIP-002)

The linear sequence (Fmoc-Asp-Trp-5Clw-Hyp-Glu-leu-Cys-Apc-Leu-Ile-Cys-APAc-glu(OAll)-NH₂) of the peptide was assembled according to the 'General procedures for Automated/Semi-automated Solid-Phase Synthesis' in a 100 µmol scale on a Rink amide resin. The allyl ester protecting group was removed acid by performing the steps of the '*Alloc*/*Allyl-deprotection*' from D-glutamic acid. The N-terminal Fmoc-group was removed and glutaric acid was coupled to N-terminus by reaction of the peptide resin with glutaric acid anhydride (57 mg, 5 eq., DIPEA (171 µL, 10eq.), DMF, 150 min, room temperature). AGLU^{∗} was coupled simultaneously to the side chain of the latter and the free N-terminal carboxylic acid function of the peptide resin (AGLU^{∗} (195 mg, 7.5 eq.), Oxyma (107 mg, 7.5 eq.) and DIC (116 µL, 7.5 eq.), DMF, overnight at 50 °C). Thereafter the steps of *'Cleavage method B*' where performed to cleave the peptide from the resin. The lyophilized crude peptide residue was subjected to '*Solution Cyclization method A'* with bis-(bromomethyl)-pyridin as cyclization reagent. Prior to the coupling of DOTA the cyclic intermediate peptide (AGLU-Glutar-Asp-Trp-5Clw-Hyp-Glu-leu-[Cys(2Lut)-Apc-Leu-Ile-Cys]-APAc-glu(AGLU)-NH₂) was purified by purified by preparative HPLC (20 to 50% B in 30 min - Kinetex) to yield 31.04 mg of the peptide precursor (13.7%). The purified peptide was dissolved in DMSO (300 µL) and the pH value of the solution adjusted to 8 by addition of DIPEA (20 µL). DOTA-NHS (15.6 mg, 20.5 µmol, 1.5 eq.) was added and the pH value re-adjusted to 8 by addition of further DIPEA. After completion of the reaction the solution was subjected to preparative HPLC (25 to 50% B in 30 min - Kinetex) to yield 21.4 mg of the pure title compound (58.9% - yield for coupling of DOTA - overall yield: 8.1%). HPLC t_{R} = 7.08 min. LC/TOF-MS: exact mass 2650.178 (calculated 2650.174). C₁₁₈H₁₇₆ClN₂₅O₃₈S₂ (MW = 2652.394).

### Example 13: Synthesis of AGLU-Glutar-Asp-Trp-5Clw-Tap-Glu-leu-[Cys(2Lut)-Apc(DOTA)-Leu-Ile-Cys]-APAc-glu(AGLU)-NH₂ (GIP-001)

### Synthesis with coupling of DOTA-NHS and reduction of azide function in solution

The linear sequence (Fmoc-Asp-Trp-5Clw-N3Tap-Glu-leu-Cys-Apc-Leu-Ile-Cys-APAc-glu(OAll)-NH₂) of the peptide was assembled according to the 'General procedures for Automated/Semi-automated Solid-Phase Synthesis' in a 100 µmol scale on a Rink amide resin. The allyl ester protecting group was removed acid by performing the steps of the '*Alloc*/*Allyl-deprotection*' from D-glutamic acid. The N-terminal Fmoc-group was removed and glutaric acid was coupled to N-terminus by reaction of the peptide resin with glutaric acid anhydride (57 mg, 5 eq., DIPEA (171 µL, 10eq.), DMF, 150 min, room temperature). AGLU^{∗} was coupled simultaneously to the side chain of the latter and the free N-terminal carboxylic acid function of the peptide resin (AGLU^{∗} (195 mg, 7.5 eq.), Oxyma (107 mg, 7.5 eq.) and DIC (116 µL, 7.5 eq.), DMF, overnight at 50 °C). Thereafter the steps of *'Cleavage method B*' where performed to cleave the peptide from the resin. The lyophilized crude peptide residue was subjected to '*Solution Cyclization method A'* with bis-(bromomethyl)-pyridin as cyclization reagent. Prior to the coupling of DOTA the cyclic intermediate peptide (AGLU-Glutar-Asp-Trp-5Clw-N3Tap-Glu-leu-[Cys(2Lut)-Apc-Leu-Ile-Cys]-APAc-glu(AGLU)-NH₂) was purified by purified by preparative HPLC (20 to 50% B in 30 min - Kinetex) to yield 30.94 mg of the peptide precursor (13.5%). The purified peptide was dissolved in DMSO (300 µL) and the pH value of the solution adjusted to 8 by addition of DIPEA (20 µL). DOTA-NHS (15.4 mg, 20.3 µmol, 1.5 eq.) was added and the pH value re-adjusted to 8 by addition of further DIPEA. After completion of the DOTA a solution of triscarboxylethylenephosphane HCl (53 mg in 200 µL DMSO) were added and the solution stirred for 80 min. After completion of the reduction of the azide to the amine the solution was subjected to preparative HPLC (15 to 45% B in 30 min - Kinetex) to yield 17.72 mg of the pure title compound (49.5% - yield for coupling of DOTA - overall yield: 6.7%). HPLC t_{R} = 6.57 min. LC/TOF-MS: exact mass 2649.198 (calculated 2649.190). C₁₁₈H₁₇₇ClN₂₆O₃₇S₂ (MW = 2651.410).

### Synthesis with AGLU*-Glutar-OH and Fmoc-glu(AGLU*)-OH building blocks

The linear sequence (AGLU*-Glutar-Asp-Trp-5Clw-Tap-Glu-leu-Cys-Apc(Alloc)-Leu-Ile-Cys-APAc-glu(AGLU^{∗})-NH₂) of the peptide was assembled according to the 'General procedures for Automated/Semi-automated Solid-Phase Synthesis' in a 100 µmol scale on a Rink amide resin. The alloc protection group was removed from the Apc side chain by performing the steps of the '*Alloc*/*Allyl-deprotection*'. DOTA(OtBu)₃-OH was coupled and the peptide cleaved from the resin Thereafter the steps of *'Cleavage method D'* where performed to cleave the peptide from the resin. The lyophilized crude peptide residue was subjected to '*Solution Cyclization method A'* with bis-(bromomethyl)-pyridin as cyclization reagent. After lyophilization the crude product was purified by preparative HPLC (15 to 40% B in 30 min-Kinetex) to yield 43.62 mg of the pure title compound (16.5%). HPLC t_{R} = 6.57 min. LC/TOF-MS: exact mass 2649.198 (calculated 2649.190). C₁₁₈H₁₇₇ClN₂₆O₃₇S₂ (MW = 2651.410).

### Synthesis with AGLU*-Glutar-OH and Fmoc-glu(AGLU*)-OH building blocks and alternative cyclization conditions

The linear sequence (AGLU^{∗}-Glutar-Asp-Trp-5Clw-Tap-Glu-leu-Cys-Apc(Alloc)-Leu-Ile-Cys-APAc-glu(AGLU^{∗})-NH₂) of the peptide was assembled according to the 'General procedures for Automated/Semi-automated Solid-Phase Synthesis' in a 50 µmol scale on a Rink amide resin. The alloc protection group was removed from the Apc side chain by performing the steps of the '*Alloc*/*Allyl-deprotection*'. DOTA(OtBu)₃-OH was coupled and the peptide cleaved from the resin Thereafter the steps of *'Cleavage method D'* where performed to cleave the peptide from the resin. The lyophilized crude peptide residue was subjected to '*Solution Cyclization method A'* with bis-(bromomethyl)-pyridin as cyclization reagent with the exception that as a solvent for the cyclization reaction a mixture of ethanol and acetonitrile (60 mL, 1:1, adjusted to pH 8 by addition of DIPEA). After evaporation of the volatiles the crude product was purified by preparative HPLC (15 to 40% B in 30 min - Kinetex) to yield 6.8 mg of the pure title compound (5.1%). HPLC t_{R} = 6.57 min. LC/TOF-MS: exact mass 2649.198 (calculated 2649.190). C₁₁₈H₁₇₇ClN₂₆O₃₇S₂ (MW = 2651.410).

### Synthesis with AGLU*-Glutar-OH and Fmoc-glu(AGLU*)-OH building blocks and on resin cyclization

The linear sequence (AGLU^{∗}-Glutar-Asp-Trp-5Clw-Tap-Glu-leu-Cys(SDmp)-Apc(Alloc)-Leu-Ile-Cys(SDmp)-APAc-glu(AGLU^{∗})-NH₂) of the peptide was assembled according to the 'General procedures for Automated/Semi-automated Solid-Phase Synthesis' in a 50 µmol scale on a Rink amide resin. The SDmp-disulfide protecting groups were removed from the cysteine side chains by treating the peptide resin with a 5% DTT solution (500 mg DTT in 10 mL 0.1 N-methylmorpholine in DMF, 3x 10 min). The cyclization was achieved by adding bis-(bromomethyl)-pyridin (13.25 mg, 1 eq., DIPEA (43 µL, 5 eq.), DMF, overnight, room temperature) to the resin. The alloc protection group was removed from the Apc side chain by performing the steps of the '*Alloc*/*Allyl-deprotection*'. DOTA(OtBu)₃-OH was coupled and the peptide cleaved from the resin employing the steps of *'Cleavage method D*'. After lyophilization the crude product was purified by preparative HPLC (15 to 40% B in 30 min-Kinetex) to yield 1.36 mg of the pure title compound (1.0%). HPLC t_{R} = 6.57 min. LC/TOF-MS: exact mass 2649.198 (calculated 2649.190). C₁₁₈H₁₇₇ClN₂₆O₃₇S₂ (MW = 2651.410).

### Example 14: Synthesis of AGLU-Glutar-Asp-Trp-5Clw-Tap-Glu-leu-[Cys(2Lut)-Apc(NODAGA-Gly)-Leu-Ile-Cys]-APAc-glu(AGLU)-NH₂ (GIP-031)

The linear sequence (AGLU^{∗}-Glutar-Asp-Trp-5Clw-Tap-Glu-leu-Cys-Apc(Alloc)-Leu-Ile-Cys-APAc-glu(AGLU^{∗})-NH₂) of the peptide was assembled according to the 'General procedures for Automated/Semi-automated Solid-Phase Synthesis' in a 50 µmol scale on a Rink amide resin. The alloc protection group was removed from the Apc side chain by performing the steps of the '*Alloc*/*Allyl-deprotection*'. Fmoc-Gly-OH and then (*R*)-NODAGA(tBu)₃-OH was coupled and the peptide cleaved from the resin Thereafter the steps of '*Cleavage method* D' where performed to cleave the peptide from the resin. The lyophilized crude peptide residue was subjected to '*Solution Cyclization method A'* with bis-(bromomethyl)-pyridin as cyclization reagent. After lyophilization the crude product was purified by preparative HPLC (15 to 45% B in 30 min - Kinetex) to yield 11.5 mg of the pure title compound (8.6%). HPLC t_{R} = 7.10 min. LC/TOF-MS: exact mass 2677.281 (calculated 2677.185). C₁₁₉H₁₇₇ClN₂₆O₃₈S₂ (MW = 2679.420).

### Example 15: Synthesis of AGLU-Glutar-APAc-Asp-Trp-5Clw-Hyp-Glu-leu-[Cys(2Lut)-Apc(DOTA)-Leu-Ile-Cys]-lys(Me036PEG)-NH₂ (GIP-039)

Initially the C-terminal part (Alloc-Apc(Fmoc)-Leu-Ile-Cys]-lys(Dde)-NH₂) of the linear sequence of the peptide was assembled according to the 'General procedures for Automated/Semi-automated Solid-Phase Synthesis' in a 50 µmol scale on a Rink amide resin. The Fmoc-group was removed, DOTA(OtBu)₃-OH was coupled and the alloc protection group cleaved from the N-terminus of the peptide resin by performing the steps of the '*Alloc*/*Allyl-deprotection*'. Thereafter the N-terminal part of the peptide sequence was assembled until the glutaric acid moiety which was introduced by the reaction of the peptide resin with glutaric acid anhydride (31 mg, 5 eq., 0.6 mL 0.9M DIPEA in DMF, 150 min, room temperature). AGLU^{∗} was coupled to the free N-terminal carboxylic acid function of the peptide resin (AGLU^{∗} (98 mg, 7.5 eq.), Oxyma (54 mg, 7.5 eq.) and DIC (58 µL, 7.5 eq.), DMF, overnight at 50 °C). The side chain of the C-terminal lysine was liberated from the Dde-group by executing the '*Dde-deprotection*' followed by the coupling the monodisperse MeO36PEG-OH PEG-building block (169 mg, 2 eq., Oyxma (21.5 mg, 3 eq.), DIC (23.4 µL, 3 eq.)). The resin was agitated at 50 °C for 2.5 hours, the addition of DIC repeated and the resin agitated overnight. Thereafter the steps of *'Cleavage method B'* where performed to cleave the peptide from the resin. The lyophilized crude peptide residue was subjected to '*Solution Cyclization method A*' with bis-(bromomethyl)-pyridin as cyclization reagent. After lyophilization the crude product was purified by preparative HPLC (25 to 50% B in 30 min - Kinetex) to yield 11.86 mg of the pure title compound (5.7%). HPLC t_{R} = 7.66 min. LC/TOF-MS: exact mass 4157.127 (calculated 4157.122). C₁₈₉H₃₁₈ClN₂₅O₇₀S₂ (MW = 4160.265).

### Example 16: Synthesis of Ac-Asp-Trp-5Clw-Hyp-Glu-leu-[Cys(2Lut)-Thp-Leu-Ile-Cys]-OH (GIP-181)

The linear sequence (AGLU^{∗}-Glutar-Asp-Trp-5Clw-Tap-Glu-leu-Cys-Apc(Alloc)-Leu-Ile-Cys-APAc-glu(AGLU^{∗})-NH₂) of the peptide was assembled according to the 'General procedures for Automated/Semi-automated Solid-Phase Synthesis' in a 50 µmol scale on a Trityl resin. Thereafter the steps of *'Cleavage method B'* where performed to cleave the peptide from the resin. The lyophilized crude peptide residue was subjected to '*Solution Cyclization method A'* with bis-(bromomethyl)-pyridin as cyclization reagent. After lyophilization the crude product was purified by preparative HPLC (30 to 60% B in 30 min - Kinetex) to yield 34.09 mg of the pure title compound (42.6%). HPLC t_{R} = 8.83 min. LC/TOF-MS: exact mass 1598.643 (calculated 1598.634). C₇₅H₉₉ClN₁₄O₁₉S₂ (MW = 1600.259).

### Example 17: Synthesis of AGLU-Glutar-APAc-Asp-Trp-5Clw-Tap(AGLU-Glutar)-Glu-leu-[Cys(2Lut)-Apc(DOTA-glu(AGLU))-Leu-Ile-Cys]-APAc-glu(AGLU)-NH₂ (GIP-007)

Initially the C-terminal part (Dde-Apc(Fmoc)-Leu-Ile-Cys-APAc-glu(OAll)-NH₂) of the linear sequence of the peptide was assembled according to the 'General procedures for Automated/Semi-automated Solid-Phase Synthesis' in a 100 µmol scale on a Rink amide resin. The Fmoc-group was removed, Fmoc-glu(OAll)-OH and then DOTA(OtBu)₃-OH were coupled. The N-terminus was released from the protection group by executing the '*Dde-deprotection'* to yield the linear intermediate H-Apc(DOTA-glu(OAll))-Leu-Ile-Cys]-APAc-glu(OAll)-NH₂ peptide resin. Afterwards the linear precursor peptide was assembled until the N-terminal APAc moiety to yield Fmoc-APAc-Asp-Trp-5Clw-Tap(Alloc)-Glu-leu-Cys-Apc(DOTA-glu(OAll))-Leu-Ile-Cys-APAc-glu(OAll)-NH₂. The alloc and allyl ester protection groups were release simulteanously, the Fmoc group was removed and the N-terminal glutaric acid coupled by the reaction of the peptide resin with glutaric acid anhydride (57 mg, 5 eq., 1.15 mL 0.9M DIPEA in DMF, 150 min, room temperature) to yield HO-Glutar-APAc-Asp(OtBu)-Trp-5Clw-Tap(HO-Glutar)-Glu(OtBu)-leu-Cys-Apc(DOTA-glu)-Leu-Ile-Cys-APAc-glu-NH₂. AGLU^{∗} was coupled to the four free carboxylic acid functions by treating the peptide resin with the mixture of AGLU^{∗} (392 mg, 15 eq.), Oxyma (214 mg, 15 eq.) and DIC (232 µL, 15 eq.) (in 1.7 mL DMF) for 1.5 hours at 50 °C. The addition of DIC was repeated and the resin left to agitate overnight at 50 °C. , DMF, overnight at 50 °C). Thereafter the steps of *'Cleavage method B'* where performed to cleave the peptide from the resin. The lyophilized crude peptide residue was subjected to '*Solution Cyclization method A'* with bis-(bromomethyl)-pyridin as cyclization reagent. After lyophilization the crude product was purified by preparative HPLC (25 to 50% B in 30 min - Kinetex) to yield 4.16 mg of the pure title compound (1.2%). HPLC t_{R} = 6.20 min. LC/TOF-MS: exact mass 3358.542 (calculated 3358.528). C₁₄₇H₂₂₈ClN₃₁O₅₂S₂ (MW = 3361.151).

### Example 18: Synthesis of Ac-Asp-Trp-5Clw-Nglu(AGLU)-Glu-leu-[Cys(2Lut)-Thp-Leu-Ile-Cys]-NH₂ (GIP-176)

Initially the C-terminal part (BrAc-Glu-leu-Cys-Thp-Leu-Ile-Cys-NH₂) of the linear sequence of the peptide was assembled according to the 'General procedures for Automated/Semi-automated Solid-Phase Synthesis' in a 100 µmol scale on a Rink amide resin. The peptide resin was reacted with β-alanine allyl ester hydrochloride (H₂N-CH₂CH₂-CO₂Allyl·Cl, 200 mg, 15 eq., DIPEA (348 µL, 20 eq.)) in a nucleophilc substitution to yield H-Nglu(OAll)-Glu-leu-[Cys(2Lut)-Thp-Leu-Ile-Cys]-NH₂ as an intermediate. The sequence assembly was finalize and the allyl ester protection removed. AGLU^{∗} was coupled to the four free carboxylic acid functions by treating the peptide resin with the mixture of AGLU^{∗} (195 mg, 7.5 eq.), Oxyma (106 mg, 7.5 eq.) and DIC (116 µL, 7.5 eq.) (in 1.7 mL DMF) for 1.5 hours at 50 °C. The addition of DIC was repeated and the resin left to agitate for further 90 min at 50 °C. Thereafter the steps of *'Cleavage method B'* where performed to cleave the peptide from the resin. The lyophilized crude peptide residue was subjected to '*Solution Cyclization method A*' with bis-(bromomethyl)-pyridin as cyclization reagent. After lyophilization the crude product was purified by preparative HPLC (25 to 55% B in 30 min - Kinetex) to yield 30.9 mg of the pure title compound (17.4%). HPLC t_{R} = 8.36 min. LC/TOF-MS: exact mass 1776.731 (calculated 1776.729). C₈₁H₁₁₃ClN₁₆O₂₃S₂ (MW = 1778.446).

### Example 19: Synthesis of AGLU-Glutar-Asp-Trp-5Clw-Tap-Glu-leu-[Cys(2Lut)-Apc(NOPO)-Leu-Ile-Cys]-APAc-glu(AGLU)-NH₂ (GIP-029)

Initially the C-terminal part (AGLU-Glutar-Asp-Trp-5Clw-Tap-Glu-leu-Cys-Apc(Alloc)-Leu-Ile-Cys-APAc-glu(AGLU)-NH₂) of the linear sequence of the peptide was assembled according to the 'General procedures for Automated/Semi-automated Solid-Phase Synthesis' in a 20 µmol scale on a Rink amide resin. The alloc protection group cleaved from the Apc side chain by performing the steps of the '*Alloc*/*Allyl-deprotection*'. The NOPO chelator was coupled to the Apc side chain by adding a pre-activated solution of NOPO-OH (50 mg, 5 eq.), HATU (76 mg, 10 eq.) and DIPEA (71 µL, 20 eq.) in DMSO (1 mL) to resin. After 90 min at room temperature, the resin was under conditions described in *'Cleavage method D*'. The lyophilized crude peptide residue was subjected to '*Solution Cyclization method A'* with bis-(bromomethyl)-pyridin as cyclization reagent. After lyophilization the crude product was purified by preparative HPLC (20 to 35% B in 30 min - Kinetex) to yield 12.63 mg of the pure title compound (23.0%). HPLC t_{R} = 7.22 min. LC/TOF-MS: exact mass 2740.127 (calculated 2740.129). C₁₁₆H₁₈₁ClN₂₅O₃₉P₃S₂ (MW = 2742.333).

### Example 20: Synthesis of Ac-Asp-Trp-5Clw-Hyp-Glu-leu-[Cys(mli)-Thp-Leu-Ile-Hcy]-NH₂ (GIP-361)

The linear sequence (Ac-Asp-Trp-5Clw-Hyp-Glu-leu-Cys-Thp-Leu-Ile-Hcy-NH₂) of the peptide was assembled according to the 'General procedures for Automated/Semi-automated Solid-Phase Synthesis' in a 50 µmol scale on a Rink amide resin. Thereafter the steps of *'Cleavage method B'* where performed to cleave the peptide from the resin. The lyophilized crude peptide residue was subjected to '*Solution Cyclization method B: Cyclization with Maleimide Derivatives'.* After lyophilization the crude product was purified by preparative HPLC (30 to 60% B in 30 min - Kinetex) to yield 11.16 mg of the pure title compound (13.9%). HPLC t_{R} = 9.69 min. LC/TOF-MS: exact mass 1601.631 (calculated 1601.609). C₇₃H₉₆ClN₁₅O₂₀S₂ (MW = 1603.220).

### Example 21: Synthesis of Ac-Asp-Trp-5Clw-Hyp-Glu-leu-[Hcy-Thp-Leu-Ile-Hcy]-NH₂ (GIP-363)

The linear sequence (Ac-Asp-Trp-5Clw-Hyp-Glu-leu-Hcy-Thp-Leu-Ile-Hcy-NH₂) of the peptide was assembled according to the 'General procedures for Automated/Semi-automated Solid-Phase Synthesis' in a 50 µmol scale on a Rink amide resin. Thereafter the steps of *'Cleavage method B'* where performed to cleave the peptide from the resin. The lyophilized crude peptide residue was subjected to '*Solution Cyclization method C: Disulfide cyclization'.* After lyophilization the crude product was purified by preparative HPLC (30 to 60% B in 30 min - Kinetex) to yield 14.19 mg of the pure title compound (18.6%). HPLC t_{R} = 10.59 min. LC/TOF-MS: exact mass 1520.639 (calculated 1520.624). C₇₀H₉₇ClN₁₄O₁₈S₂ (MW = 1522.190).

### Example 22: Synthesis of Ac-Asp-Trp-5Clw-Hyp-Glu-leu-[Cys(tMeBn(DOTA-AET))-Thp-Leu-Ile-Cys]-NH₂ (GIP-124)

The linear sequence (Ac-Asp-Trp-5Clw-Hyp-Glu-leu-Cys-Thp-Leu-Ile-Cys-NH₂) of the peptide was assembled according to the 'General procedures for Automated/Semi-automated Solid-Phase Synthesis' in a 100 µmol scale on a Rink amide resin. Thereafter the steps of *'Cleavage method B'* where performed to cleave the peptide from the resin. The lyophilized crude peptide residue was dissolved in 120 mL of a 1:1 mixture of ammonium bicarbonate solution (50 mM, pH = 8.5) and acetonitrile. 1,3,5-Tris(bromomethyl)benzene (53.5 mg, 1.5 eq compared to initial resin loading) was added to the solution, which was then left to stir for 1 hour. Then 2-aminoethanthiol (77 mg, 10 eq.) was added to the solution, which was stirred for further 5 hours. After addition of TFA (100 µL) the solution was lyophilized and the remainder subjected to HPLC purification (30 to 70% B in 30 min - Kinetex) to yield 41.15 mg (24.6%) of the peptide precursor Ac-Asp-Trp-5Clw-Hyp-Glu-leu-[Cys(tMeBn(H-AET))-Thp-Leu-Ile-Cys]-NH₂. 15 mg of the latter where dissolved in DMSO and the pH of the solution adjusted to 8 by addition of DIPEA (7 µL). DOTA-NHS (10.1 mg, 1.5 eq.) was added and the pH value re-adjusted to 8 by addition of further DIPEA (7 µL). After completion of the reaction the solution was subjected to preparative HPLC (25 to 50% B in 30 min - Kinetex) to yield 12.57 mg of the pure title compound (68.1% - yield for coupling of DOTA - overall yield: 16.7%). HPLC t_{R} = 9.53 min. LC/TOF-MS: exact mass 2071.894 (calculated 2071.865). C₉₅H₁₃₄CIN₁₉O₂₅S₃ (MW = 2073.848).

### Example 23: Synthesis of DOTA-Ttds-Asp-Trp-5Clw-Pro-Glu-leu-[Cys(3CbBn)-Aib-Leu-Ile-en] (GIP-359)

The linear sequence (DOTA(OtBu)₃-Ttds-Asp(OtBu)-Trp(Boc)-5Clw-Pro-Glu(OtBu)-leu-Cys(Mmt)-Aib-Leu-Ile-en-H) of the protected peptide was assembled according to the 'General procedures for Automated/Semi-automated Solid-Phase Synthesis' in a 50 µmol scale on a 1,2-diaminoethane trityl resin. The protected peptide was cleaved from the resin by performing the steps of '*Cleavage method A*'. To the C-terminal 1,2-diaminoethane moiety 3-chloromethyl-benzoic acid was coupled (conditions: 2 eq. 3-chloromethyl-benzoic acid, 2 eq. Oxyma, 2 eq. DIC in DMF/DCM (3:1, 600 µL), overnight). The solvent was evapotated and all protecting groups were removed by execution of 'C*leavage method C*'. The reaction of the previously introduced benzyl chloride and the sulfhydryl group of the cysteine side chain to form the peptide cycle was allowed to proceed overnight in a sodium acetat buffer (pH 8.0). Afterwards, the crude product was isolated from the reaction buffer by a solid phase extraction. The lyophilized crude product was purified by preparative HPLC (30 to 60% B in 30 min - Kinetex) to yield 11.6 mg of the pure title compound (10.8%). HPLC t_{R} = 8.50 min. LC/TOF-MS: exact mass 2139.017 (calculated 2139.015). C₁₀₁H₁₄₇ClN₂₀O₂₇S (MW = 2140.889).

### Example 24: Synthesis of DOTA-Ttds-Asp-Trp-5Clw-Pro-Glu-leu-[Dap(Mamb)-Aib-Leu-Ile-gln] (GIP-366)

The linear sequence (DOTA(OtBu)₃-Ttds-Asp(OtBu)-Trp(Boc)-5Clw-Pro-Glu(OtBu)-leu-Dap(Alloc)-Aib-Leu-Ile-gln-OH) of the protected peptide was assembled according to the 'General procedures for Automated/Semi-automated Solid-Phase Synthesis' in a 50 µmol scale on a trityl resin. The Alloc-protection group was removed from the Dap side chain by performing the steps of the '*Alloc*/*Allyl-deprotection*'. To the liberated amino function Fmoc-Mamb-OH was coupled and the Fmoc-group of the latter removed. The protected peptide was cleaved from the resin by performing the steps of '*Cleavage method A*' to yield the intermediate partially-protected precursor (DOTA(OtBu)₃-Ttds-Asp(OtBu)-Trp(Boc)-5Clw-Pro-Glu(OtBu)-leu-Dap(H-Mamb)-Aib-Leu-Ile-gln-OH). The reaction of the amino function of the Mamb building block and the C-terminus of the peptide was achieved by activation with DIC/Oxyma (conditions: 2eq. Oxyma, 2 eq. DIC in DMF/DCM (3:1, 5 mL), overnight). The solvent was evapotated and all protecting groups were removed by execution of '*Cleavage method C*'. After lyophilization the crude product was subjected to preparative HPLC (20 to 45% B in 30 min - Kinetex) to yield 31.56 mg of the pure title compound (28.6%). HPLC t_{R} = 7.60 min. LC/TOF-MS: exact mass 2207.077 (calculated 2207.071). C₁₀₄H₁₅₁ClN₂₂O₂₉ (MW = 2208.899).

### Example 25: Synthesis of HO-Succinyl-Trp-5Clw-Hyp-Glu-leu-[Cys(2Lut)-Apc(MeO10kDaPEGAc)-Leu-Ile-Cys]-APAc-Lys(DOTA)-NH₂ (GIP-142)

The linear sequence (tBuO-Succinyl-Trp-5Clw-Hyp-Glu-leu-Cys-Apc-Leu-Ile-Cys-APAc-Lys(Alloc)-NH₂) was assembled according to the 'General procedures for Automated/Semi-automated Solid-Phase Synthesis' in a 50 µmol scale on a Rink amide resin. The Alloc-protection group was removed from the Lys side chain by performing the steps of the '*Alloc*/*Allyl-deprotectior*'*.* DOTA(OtBu)₃-OH was coupled and the peptide cleaved from the resin employing the steps of *'Cleavage method B'.* The lyophilized crude peptide residue was subjected to '*Solution Cyclization method A'* with bis-(bromomethyl)-pyridin as cyclization reagent. After lyophilization the crude product was purified by preparative HPLC (20 to 50% B in 30 min - Kinetex) to yield 30.8 mg of the cyclic intermediate peptide (28.1%). HPLC t_{R} = 8.36 min. LC/TOF-MS: exact mass 2194.019 (calculated 2194.015). C₁₀₂H₁₄₈ClN₂₃O₂₅S₂ (MW = 2195.995). 12.5 mg of the intermediate peptide (5.7 µmol) were dissolved in DMSO (600 µL) and the pH-value adjusted to roughly pH = 8.0 by addition of DIPEA (2 µL). 59.3 mg Methoxy PEG Succinimidyl Carboxymethyl Ester (5.7 µmol; 10 kDa average mass, JenKem M-SCM-10K) were dissolved in acetonitrile (400 µL) and added to the solution of the peptide. The pH-value was adjusted to pH = 8 by addition of small portions (0.5 µL) of DIPEA. After 3 hours the acetonitrile fraction of the reaction solution was evaporated and the remaining solution subjected to preparative HPLC to yield 8.87 mg of the pure title compound (12.7%). HPLC t_{R} = 12.30 min. Average MW = 12196.

### Example 26: Preparation of Transition metal complexes of compounds of the invention

### A. General procedure for the preparation of a peptide comprising Chelator-transition metal-complexes from corresponding peptides comprising uncomplexed Chelator

A 0.1 mM solution of the peptide comprised by uncomplexed Chelator in
- 0.4 M sodium acetate, pH = 5 (Buffer A) (in case of In(III), Lu(III) or Ga(III) complexes) or
- 0.1 M ammonium acetate, pH = 8 (Buffer B) (in case of Eu(III) complexes)
was diluted with a solution 0.1 mM solution of the corresponding metal salt in water whereby the molar ratio of peptide to metal was adjusted to 1 : 3. The solution was stirred
- at 50 °C for 20 min (also referred to herein as Condition A) (in case of In(III), Lu(III) or Ga(III) complexes)
or
- at room temperature overnight (also referred to herein as Condition B) (in case of Eu(III) complexes).

The solution was then applied to
- HPLC purification (also referred to herein as Purification A) or
- solid phase extraction (also referred to herein as Purification B).

In either case (HPLC purification or solid phase extraction) fractions containing the pure product were pooled and freeze dried.

### B. Indium-complex of AGLU-Glutar-Asp-Trp-5Clw-Hyp-Glu-leu-[Cys(2Lut)-Apc(DOTA)-Leu-Ile-Cys]-APAc-glu(AGLU)-NH₂ (GIP-065)

The complex was prepared starting from 15 mg peptide (5.7 µmol) dissolved in Buffer A, diluted with a solution of InCl₃ × 4 H₂O which was treated with Condition A. In the purification step 'Purification B' was employed to yield 12.85 mg of the pure title compound (82.3%). HPLC t_{R} = 7.13 min. LC/TOF-MS: exact mass 2760.063 (calculated 2762.054). C₁₁₈H₁₇₃ClInN₂₅O₃₈S₂ (MW = 2764.189).

### C. Lutetium-complex of AGLU-Glutar-Asp-Trp-5Clw-Hyp-Glu-leu-[Cys(2Lut)-Apc(DOTA)-Leu-Ile-Cys]-APAc-glu(AGLU)-NH₂ (GIP-066)

The complex was prepared starting from 15 mg peptide (5.7 µmol) dissolved in Buffer A, diluted with a solution of LuCl₃ which was treated with Condition A. In the purification step 'Purification B' was employed to yield 10.87 mg of the pure title compound (68.1%). HPLC t_{R} = 7.15 min. LC/TOF-MS: exact mass 2822.092 (calculated 2822.091). C₁₁₈H₁₇₃ClLuN₂₅O₃₈S₂ (MW = 2824.337).

### D. Gallium-complex of AGLU-Glutar-Asp-Trp-5Clw-Tap-Glu-leu-[Cys(2Lut)-Apc(DOTA)-Leu-Ile-Cys]-APAc-glu(AGLU)-NH₂ (GIP-069)

The complex was prepared starting from 25 mg peptide (9.5 µmol) dissolved in Buffer A, diluted with a solution of Ga(NO₃)₃ × H₂O which was treated with Condition A. In the purification step 'Purification B' to yield 16.42 mg of the pure title compound (63.6%). HPLC t_{R} = 6.91 min. LC/TOF-MS: exact mass 2715.104 (calculated 2715.092). C₁₁₈H₁₇₄ClGaN₂₆O₃₇S₂ (MW = 2718.109).

### E. Europium-complex of DOTA-glu(AGLU)-APAc-Asp-Trp-5Clw-Hyp-Glu-leu-[Cys(2Lut)-Thp-Leu-Ile-Cys]-APAc-glu(AGLU)-NH₂ (GIP-064)

The complex was prepared starting from 6 mg peptide (2.1 µmol) dissolved in Buffer B, diluted with a solution of EuCl₃ × 6 H₂O which was treated with Condition B. In the purification step 'Purification B' was employed to yield 4.88 mg of the pure title compound (77.1%). HPLC t_{R} = 6.80 min. LC/TOF-MS: exact mass 2954.158 (calculated 2956.161). C₁₂₅H₁₈₅ClEuN₂₇O₄₀S₂ (MW = 2957.518).

### E. Gallium-complex Synthesis of AGLU-Glutar-Asp-Trp-5Clw-Tap-Glu-leu-[Cys(2Lut)-Apc(NODAGA)-Leu-Ile-Cys]-APAc-glu(AGLU)-NH₂ (GIP-071)

The complex was prepared starting from 8 mg peptide (3 µmol) dissolved in Buffer A, diluted with a solution of Ga(NO₃)₃ × H₂O which was treated with Condition A. In the purification step 'Purification B' was employed to yield 6.16 mg of the pure title compound (75.1%). HPLC t_{R} = 6.93 min. LC/TOF-MS: exact mass 2686.071 (calculated 2686.065). C₁₁₇H₁₇₁ClGaN₂₅O₃₇S₂ (MW = 2689.068).

### Example 27: FACS Binding Assay

In order to determine binding of compounds according to the present invention to GIPR-expressing cells, a FACS binding assay was established.

CHO-K1-hGIPR cells (GenScript Biotech, #M00486) were cultured in Ham's F12 medium (Sigma-Aldrich, #N4888) including 10% fetal calf serum (FCS), 2 mM L-glutamine, 100 U/mL penicillin, 100 µg/mL streptomycin, 200µg/mL Zeocin and 100µg/mL HygromycinB. Cells were detached with Accutase (Biolegend, #BLD-423201) and washed in FACS buffer (PBS including 1% FCS). Cells were diluted in FACS buffer to a final concentration of 500.000 cells per mL. 200 µL of the cell suspension were transferred to a u-shaped non-binding 96-well plate (Greiner) and cells were washed in ice-cold FACS buffer.

For EC₅₀ determination, cells were incubated with various concentrations of biotinylated compound (GIP-057) at 4°C for 1 hour. The determined pEC₅₀ value of GIP-057 was 8.5. For IC₅₀ determination, cells were incubated with 20 nM GIP-057 (H-Met-Tyr-Asp-Trp-Trp-Pro-Glu-Leu-[Cys(3MeBn)-Ala-Leu-Ile-Cys]-Ttds-Lys(Bio)-NH₂) in the presence of increasing concentrations of non-labeled test compounds at 4°C for 1 hour. Cells were washed twice in FACS buffer. An additional incubation step with 1 µg/mL APC-Streptavidin (Miltenyi; #130-106-791) in 50 µL FACS buffer for 30 min on ice was performed followed by two washes with FACS buffer.

Cells were analyzed in an Attune NxT flow cytometer. Median fluorescence intensities (MFI) of the APC/Cy5-channel were calculated by Attune NxT software. MFI values were plotted against peptide concentration and four parameter logistic (4PL) curve fitting and EC₅₀/pEC₅₀ or IC₅₀/pIC₅₀ calculations were performed using ActivityBase software.

The results of the IC₅₀ assay is shown in Table 8. pIC₅₀ category A stands for pIC₅₀ values >8.0, category B for pIC₅₀ values between 7.1 and 8.0, category C for pIC₅₀ values between 6.1 and 7.0, and category D for pIC₅₀ values ≤ 6.0.

**Table 8: Compound ID, sequence, exact calculated mass, exact mass found, retention time in min as determined by HPLC and pIC₅₀ category of FACS binding assay.**

| **Compound ID** | **Sequence** | **Exact Mass (Calc)** | **Exact Mass (Found)** | **Rt (HPLC) [min]** | **pIC₅₀ Category** |
|---|---|---|---|---|---|
| GIP-001 | AGLU-Glutar-Asp-Trp-5Clw-Tap-Glu-leu- [Cys(2Lut)-Apc(DOTA)-Leu-Ile-Cys]-APAc- glu(AGLU)-NH2 | 2649.190 | 2649.198 | 6.6 | A |
| GIP-002 | AGLU-Glutar-Asp-Trp-5Clw-Hyp-Glu-leu- [Cys(2Lut)-Apc(DOTA)-Leu-Ile-Cys]-APAc- glu(AGLU)-NH2 | 2650.174 | 2650.178 | 7.1 | A |
| GIP-003 | DOTA-glu(AGLU)-APAc-Asp-Trp-5Clw-Hyp-Glu- leu-[Cys(2Lut)-Apc(AGLU-Glutar)-Leu-Ile-Cys]- APAc-glu(AGLU)-NH2 | 3082.396 | 3082.398 | 6.5 | A |
| GIP-004 | AGLU-Glutar-Asp-Trp-5Clw-Hyp-Glu-leu- [Cys(2Lut)-Thp-Leu-Ile-Cys]-APAc-glu(AGLU)- Lys(DOTA)-NH2 | 2779.253 | 2779.260 | 7.0 | A |
| GIP-005 | DOTA-glu(AGLU)-APAc-Asp-Trp-5Clw-Hyp-Glu- leu-[Cys(2Lut)-Thp-Leu-Ile-Cys]-APAc- glu(AGLU)-NH2 | 2806.264 | 2806.273 | 6.7 | A |
| GIP-006 | AGLU-Glutar-APAc-Asp-Trp-5Clw-Tap(AGLU- Glutar)-Glu-leu-[Cys(2Lut)-Apc(DOTA)-Leu-Ile- Cys]-APAc-glu(AGLU)-NH2 | 3066.401 | 3066.416 | 6.3 | A |
| GIP-007 | AGLU-Glutar-APAc-Asp-Trp-5Clw-Tap(AGLU- Glutar)-Glu-leu-[Cys(2Lut)-Apc(DOTA- glu(AGLU))-Leu-Ile-Cys]-APAc-glu(AGLU)-NH2 | 3358.528 | 3358.542 | 6.2 | B |
| GIP-008 | AGLU-Glutar-APAc-Asp-Trp-5Clw-Tap-Glu-leu- [Cys(2Lut)-Apc(DOTA-glu(AGLU))-Leu-Ile-Cys]- APAc-glu(AGLU)-NH2 | 3081.412 | 3081.423 | 6.1 | A |
| GIP-009 | DOTA-glu(AGLU)-O2Oc-Asp-Trp-5Clw-Tap-Glu- leu-[Cys(2Lut)-Apc(AGLU-Glutar)-Leu-Ile-Cys]- APAc-glu(AGLU)-NH2 | 3086.391 | 3086.411 | 6.7 | A |
| GIP-010 | AGLU-Glutar-Asp-Trp-5Clw-Hyp-Glu-leu- [Cys(2Lut)-Apc(AGLU-Glutar)-Leu-Ile-Cys]- APAc-Lys(DOTA)-NH2 | 2763.258 | 2763.274 | 7.2 | A |
| GIP-011 | AGLU-Glutar-APAc-Asp-Trp-5Clw-Hyp-Glu-leu- [Cys(2Lut)-Apc(DOTA)-Leu-Ile-Cys]-APAc- glu(AGLU)-NH2 | 2790.269 | 2790.274 | 6.6 | A |
| GIP-012 | DOTA-APAc-Asp-Trp-5Clw-Hyp-Glu-leu- [Cys(2Lut)-Apc(AGLU-Glutar)-Leu-Ile-Cys]- APAc-glu(AGLU)-NH2 | 2790.269 | 2790.270 | 6.8 | A |
| GIP-013 | AGLU-Glutar-APAc-Asp-Trp-5Clw-Tap-Glu-leu- [Cys(2Lut)-Apc(DOTA)-Leu-Ile-Cys]-APAc- glu(AGLU)-NH2 | 2789.285 | 2789.309 | 6.6 | A |
| GIP-014 | AGLU-Glutar-APAc-Asp-Trp-5Brw-Tap-Glu-leu- [Cys(2Lut)-Apc(DOTA)-Leu-Ile-Cys]-APAc- glu(AGLU)-NH2 | 2833.234 | 2833.261 | 6.7 | A |
| GIP-015 | AGLU-Glutar-Asp-Trp-5Brw-Tap-Glu-leu- [Cys(2Lut)-Apc(DOTA)-Leu-Ile-Cys]-APAc- glu(AGLU)-NH2 | 2693.139 | 2693.131 | 6.8 | A |
| GIP-016 | AGLU-Glutar-Hse-Trp-5Brw-Tap-Glu-leu- [Cys(2Lut)-Apc(DOTA)-Leu-Ile-Cys]-APAc- glu(AGLU)-NH2 | 2679.160 | 2679.150 | 6.7 | A |
| GIP-017 | DOTA-glu(AGLU)-Asp-Trp-5Clw-Tap-Glu-leu- [Cys(2Lut)-Thp-Leu-Ile-Cys]-APAc-glu(AGLU)- NH2 | 2665.185 | 2665.197 | 6.7 | A |
| GIP-018 | DOTA-O2Oc-glu(AGLU)-Asp-Trp-5Clw-Tap-Glu- leu-[Cys(2Lut)-Thp-Leu-Ile-Cys]-APAc- glu(AGLU)-NH2 | 2810.259 | 2810.260 | 6.7 | A |
| GIP-019 | DOTA-glu(AGLU)-PEG6-Asp-Trp-5Clw-Tap-Glu- leu-[Cys(2Lut)-Thp-Leu-Ile-Cys]-APAc- glu(AGLU)-NH2 | 3000.379 | 3000.379 | 7.0 | A |
| GIP-020 | DOTA-glu(AGLU)-O2Oc-Asp-Trp-5Clw-Tap-Glu- leu-[Cys(2Lut)-Thp-Leu-Ile-Cys]-APAc- glu(AGLU)-NH2 | 2810.259 | 2810.247 | 6.9 | A |
| GIP-021 | HO-Succinyl-Trp-5Clw-Hyp-Glu-leu-[Cys(2Lut)- Thp-Leu-Ile-Cys]-APAc-glu(AGLU)-Lys(DOTA)- NH2 | 2487.126 | 2487.135 | 7.7 | A |
| GIP-022 | HO-Succinyl-Trp-5Clw-Tap-Glu-leu-[Cys(2Lut)- Apc-Leu-Ile-Cys]-APAc-glu(AGLU)-Lys(DOTA)- NH2 | 2485.158 | 2485.165 | 7.0 | A |
| GIP-023 | HO-Succinyl-Trp-5Clw-Tap-Glu-leu-[Cys(2Lut)- Apc(DOTA)-Leu-Ile-Cys]-APAc-glu(AGLU)-NH2 | 2357.063 | 2357.080 | 7.3 | A |
| GIP-024 | AGLU-Glutar-Asp-Trp-5Clw-Tap-Glu-leu- [Cys(2Lut)-Lys(DOTA)-Leu-Ile-Cys]-APAc- glu(AGLU)-NH2 | 2651.205 | 2651.200 | 6.8 | A |
| | AGLU-Glutar-Asp-Trp-5Clw-Tap-Glu-leu- [Cys(2Lut)-lys(DOTA)-Leu-Ile-Cys]-APAc- glu(AGLU)-NH2 | 2651.205 | 2651.194 | 6.5 | B |
| GIP-026 | AGLU-Glutar-Asp-Trp-5Clw-Tap-Glu-leu- [Cys(2Lut)-Dab(DOTA)-Leu-Ile-Cys]-APAc- glu(AGLU)-NH2 | 2623.174 | 2623.163 | 6.7 | A |
| g<se< | AGLU-Glutar-Asp-Trp-5Clw-Tap-Glu-leu- [Cys(2Lut)-dab(DOTA)-Leu-Ile-Cys]-APAc- glu(AGLU)-NH2 | 2623.174 | 2623.168 | 6.4 | B |
| GIP-028 | AGLU-Glutar-Asp-Trp-5Clw-Tap-Glu-leu- [Cys(2Lut)-Apc(NODAGA)-Leu-Ile-Cys]-APAc- glu(AGLU)-NH₂ | 2620.163 | 2620.134 | 6.7 | A |
| <se< | AGLU-Glutar-Asp-Trp-5Clw-Tap-Glu-leu- [Cys(2Lut)-Apc(NOPO)-Leu-Ile-Cys]-APAc- glu(AGLU)-NH2 | 2740.129 | 2740.127 | 7.2 | A |
| g<se< | AGLU-Glutar-Asp-Trp-5Clw-Tap-Glu-leu- [Cys(2Lut)-Apc(N4Ac)-Leu-Ile-Cys]-APAc- glu(AGLU)-NH2 | 2449.158 | 2449.225 | 6.4 | A |
| GIP-031 | AGLU-Glutar-Asp-Trp-5Clw-Tap-Glu-leu- [Cys(2Lut)-Apc(NODAGA-Gly)-Leu-Ile-Cys]- APAc-glu(AGLU)-NH2 | 2677.185 | 2677.281 | 7.1 | A |
| GIP-032 | HO-Succinyl-Trp-5Clw-Hyp-Glu-leu-[Cys(2Lut)- Thp-Leu-Ile-Cys]-APAc-glu(AGLU)-glu(AGLU)- Lys(DOTA)-NH2 | 2779.253 | 2779.264 | 7.4 | A |
| GIP-033 | AGLU-Glutar-APAc-Asp-Trp-5Clw-Hyp-Glu-leu- [Cys(2Lut)-Apc(DOTA)-Leu-Ile-Cys]-glu(AGLU)- NH2 | 2650.174 | 2650.186 | 6.6 | A |
| GIP-034 <s< | AGLU-Glutar-Hse-Trp-5Clw-Tap-Glu-leu- [Cys(2Lut)-Apc(DOTA)-Leu-Ile-Cys]-APAc- glu(AGLU)-NH2 | 2635.211 | 2635.203 | 6.6 | A |
| GIP-035 <se< | AGLU-Glutar-Asp-Trp-5Clw-Tap-Glu-Leu- [Cys(2Lut)-Apc(DOTA)-Leu-Ile-Cys]-APAc- glu(AGLU)-NH2 | 2649.190 | 2649.214 | 7.3 | A |
| GIP-036 | DOTA-glu(AGLU)-Asp-Trp-5Clw-Hyp-Glu-leu- [Cys(2Lut)-Thp-Leu-Ile-Cys]-APAc-glu(AGLU)- NH2 | 2666.169 | 266.180 | 7.1 | A |
| GIP-037 300.379 3000.379 6.8 A | DOTA-PEG6-glu(AGLU)-Asp-Trp-5Clw-Tap-Glu- leu-[Cys(2Lut)-Thp-Leu-Ile-Cys]-APAc- glu(AGLU)-NH2 | 3000.379 | 3000.379 | 6.8 | A |
| GIP-038 | AGLU-Glutar-Asp-Trp-5Clw-Tap-Glu-leu- [Cys(2Lut)-Apc(DOTA)-Leu-Ile-cys]-APAc- glu(AGLU)-NH₂ | 2649.190 | 2649.227 | 7.0 | A |
| GIP-039 | AGLU-Glutar-APAc-Asp-Trp-5Clw-Hyp-Glu-leu- [Cys(2Lut)-Apc(DOTA)-Leu-Ile-Cys]- lys(MeO36PEG)-NH2 | 4157.122 | 4157.127 | 7.7 | B |
| GIP-040 | MeO36PEG-APAc-Asp-Trp-5Clw-Hyp-Glu-leu- [Cys(2Lut)-Apc(DOTA)-Leu-Ile-Cys]-glu(AGLU)- NH2 | 4044.038 | 4044.039 | 7.5 | B |
| GIP-041 | MeO36PEG-APAc-Asp-Trp-5Clw-Hyp-Glu-leu- [Cys(2Lut)-Apc(DOTA)-Leu-Ile-Cys]-APAc- glu(AGLU)-NH2 | 4184.133 | 4184.152 | 7.9 | B |
| GIP-042 | MeO36PEG-APAc-Asp-Trp-5Clw-Tap-Glu-leu- [Cys(2Lut)-Apc(DOTA)-Leu-Ile-Cys]-APAc- glu(AGLU)-NH2 | 4183.149 | 4183.165 | 7.5 | B |
| GIP-043 | AGLU-Glutar-APAc-Asp-Trp-5Clw-Hyp-Glu-leu- [Cys(2Lut)-Apc(DOTA-glu(AGLU))-Leu-Ile-Cys]- APAc-glu(AGLU)-NH2 | 3082.396 | 3082.412 | 6.9 | A |
| GIP-044 | DOTA-O2Oc-glu(AGLU)-Asp-Trp-5Clw-Tap-Glu- leu-[Cys(2Lut)-Apc(AGLU-Glutar)-Leu-Ile-Cys]- APAc-glu(AGLU)-NH2 | 3086.391 | 3086.414 | 6.6 | A |
| GIP-045 | DOTA-glu(AGLU)-Asp-Trp-5Clw-Tap-Glu-leu- [Cys(2Lut)-Apc(AGLU-Glutar)-Leu-Ile-Cys]- APAc-glu(AGLU)-NH2 | 2941.317 | 2941.324 | 6.5 | A |
| GIP-046 | AGLU-Glutar-APAc-Ser-Trp-5Clw-Tap(AGLU- Glutar)-Hse-leu-[Cys(2Lut)-Apc(DOTA)-Leu-Ile- Cys]-APAc-glu(AGLU)-NH2 | 3010.411 | 3010.414 | 6.4 | B |
| GIP-047 | AGLU-Glutar-APAc-Hse-Trp-5Clw-Hyp-Glu-leu- [Cys(2Lut)-Apc(DOTA-glu(AGLU))-Leu-Ile-Cys]- APAc-glu(AGLU)-NH2 | 3068.417 | 3068.435 | 6.8 | B |
| GIP-048 | AGLU-Glutar-APAc-Ser-Trp-5Clw-Tap-Hse-leu- [Cys(2Lut)-Apc(DOTA)-Leu-Ile-Cys]-APAc- glu(AGLU)-NH2 | 2733.295 | 2733.307 | 6.2 | B |
| GIP-049 | AGLU-Glutar-APAc-Hse-Trp-5Clw-Hyp-Glu-leu- [Cys(2Lut)-Apc(DOTA)-Leu-Ile-Cys]-APAc- glu(AGLU)-NH2 | 2776.290 | 2776.322 | 6.9 | B |
| GIP-050 | AGLU-Glutar-APAc-Hse-Trp-5Clw-Tap-Glu-leu- [Cys(2Lut)-Apc(DOTA)-Leu-Ile-Cys]-APAc- glu(AGLU)-NH2 | 2775.306 | 2775.316 | 6.5 | B |
| GIP-051 | AGLU-Glutar-Asp-Trp-5Clw-Tap-Glu-leu- [cys(2Lut)-Apc(DOTA)-Leu-Ile-Cys]-APAc- glu(AGLU)-NH2 | 2649.190 | 2649.232 | 7.0 | B |
| GIP-052 | AGLU-Glutar-Asp-Trp-5Clw-Tap-Glu-leu- [cys(2Lut)-Apc(DOTA)-Leu-Ile-cys]-APAc- glu(AGLU)-NH2 | 2649.190 | 2649.226 | 6.6 | B |
| GIP-053 | DOTA-Ttds-Asp-Trp-5Clw-Pro-Glu-leu- [Cys(3MeBn)-Apc(DOTA)-Leu-Ile-AET] | 2569.204 | 2569.215 | 8.5 | A |
| GIP-054 | DOTA-Ttds-Asp-Trp-5Clw-Hyp-Glu-leu- [Cys(2Lut)-Thp-Leu-Ile-Cys]-Lys(DOTA)-NH2 | 2758.279 | 2758.351 | 7.9 | A |
| GIP-055 | DOTA-Ttds-Asp-Trp-5Clw-Hyp-Glu-leu- [Cys(2Lut)-Apc(DOTA)-Leu-Ile-Cys]-Lys(DOTA)- NH2 | 3143.475 | 3143.537 | 7.8 | A |
| GIP-056 | DOTA-Ttds-Asp-Trp-5Clw-Tap(DOTA)-Glu-leu- [Cys(2Lut)-Apc(DOTA)-Leu-Ile-Cys]-Lys(DOTA)- NH2 | 3528.671 | 3528.788 | 7.4 | A |
| GIP-059 | AGLU-Glutar-APAc-Asp-Trp-5Clw-Tap-Glu-leu- [Cys(2Lut)-Apc(GaDOTA-glu(AGLU))-Leu-Ile- Cys]-APAc-glu(AGLU)-NH2 | 3147.314 | 3147.320 | 6.4 | A |
| GIP-060 | AGLU-Glutar-APAc-Asp-Trp-5Clw-Tap-Glu-leu- [Cys(2Lut)-Apc(InDOTA-glu(AGLU))-Leu-Ile- Cys]-APAc-glu(AGLU)-NH2 | 3191.292 | 3191.303 | 6.7 | A |
| GIP-061 | AGLU-Glutar-APAc-Asp-Trp-5Clw-Tap-Glu-leu- [Cys(2Lut)-Apc(LuDOTA-glu(AGLU))-Leu-Ile- Cys]-APAc-glu(AGLU)-NH2 | 3253.329 | 3253.341 | 6.5 | A |
| GIP-062 | LuDOTA-glu(AGLU)-APAc-Asp-Trp-5Clw-Hyp- Glu-leu-[Cys(2Lut)-Thp-Leu-Ile-Cys]-APAc- glu(AGLU)-NH2 | 2978.181 | 2978.182 | 6.8 | A |
| GIP-063 | InDOTA-glu(AGLU)-APAc-Asp-Trp-5Clw-Hyp- Glu-leu-[Cys(2Lut)-Thp-Leu-Ile-Cys]-APAc- glu(AGLU)-NH2 | 2916.144 | 2916.143 | 6.8 | A |
| GIP-064 | EuDOTA-glu(AGLU)-APAc-Asp-Trp-5Clw-Hyp- Glu-leu-[Cys(2Lut)-Thp-Leu-Ile-Cys]-APAc- glu(AGLU)-NH2 | 2954.160 | 2954.158 | 6.8 | A |
| GIP-065 | AGLU-Glutar-Asp-Trp-5Clw-Hyp-Glu-leu- [Cys(2Lut)-Apc(InDOTA)-Leu-Ile-Cys]-APAc- glu(AGLU)-NH2 | 2760.054 | 2760.063 | 7.1 | A |
| GIP-066 | AGLU-Glutar-Asp-Trp-5Clw-Hyp-Glu-leu- [Cys(2Lut)-Apc(LuDOTA)-Leu-Ile-Cys]-APAc- glu(AGLU)-NH2 | 2822.091 | 2822.092 | 7.2 | A |
| GIP-067 | AGLU-Glutar-Asp-Trp-5Clw-Tap-Glu-leu- [Cys(2Lut)-Apc(LuDOTA)-Leu-Ile-Cys]-APAc- glu(AGLU)-NH2 | 2821.107 | 2821.116 | 6.8 | A |
| GIP-068 | AGLU-Glutar-Asp-Trp-5Clw-Tap-Glu-leu- [Cys(2Lut)-Apc(InDOTA)-Leu-Ile-Cys]-APAc- glu(AGLU)-NH2 | 2759.070 | 2759.003 | 7.2 | A |
| GIP-069 | AGLU-Glutar-Asp-Trp-5Clw-Tap-Glu-leu- [Cys(2Lut)-Apc(GaDOTA)-Leu-Ile-Cys]-APAc- glu(AGLU)-NH2 | 2715.092 | 2715.104 | 6.9 | A |
| GIP-070 | AGLU-Glutar-Asp-Trp-5Clw-Hyp-Glu-leu- [Cys(2Lut)-Apc(GaDOTA)-Leu-Ile-Cys]-APAc- glu(AGLU)-NH2 | 2716.076 | 2716.087 | 7.5 | A |
| GIP-071 | AGLU-Glutar-Asp-Trp-5Clw-Tap-Glu-leu- [Cys(2Lut)-Apc(GaNODAGA)-Leu-Ile-Cys]- APAc-glu(AGLU)-NH2 | 2686.065 | 2686.071 | 6.9 | A |
| GIP-072 | AGLU-Glutar-Asp-Trp-5Clw-Hyp-Glu-leu- [Cys(2Lut)-Apc(DOTA)-Leu-Ile-AET] | 2174.946 | 2174.966 | 7.8 | A |
| GIP-073 | HO-Succinyl-Trp-5Clw-Hyp-Glu-leu-[Cys(2Lut)- Apc(AGLU-Glutar)-Leu-Ile-Cys]-APAc- Lys(DOTA)-NH2 | 2471.131 | 2471.139 | 7.8 | A |
| GIP-074 | Ac-Asp-Trp-1Ni-Pro-Glu-Leu-[Cys(3MeBn)-Aib- Leu-Ile-Cys]-Ttds-Lys(DOTA)-NH2 | 2332.152 | 2332.163 | 9.9 | A |
| GIP-075 | Ac-Asp-Trp-Bta-Pro-Glu-Leu-[Cys(3MeBn)-Aib- Leu-Ile-Cys]-Ttds-Lys(DOTA)-NH2 | 2338.109 | 2338.116 | 9.9 | A |
| GIP-076 | Ac-Asp-Trp-5Clw-Pro-Glu-Leu-[Cys(3MeBn)- Aib-Leu-Ile-Cys]-Ttds-Lys(DOTA)-NH2 | 2355.109 | 2355.114 | 9.5 | A |
| GIP-077 | DOTA-Ttds-Asp-Trp-5Clw-Pro-Glu-leu- [Cys(3MeBn)-Aib-Leu-Ile-AET] | 2141.997 | 2142.002 | 9.2 | A |
| GIP-078 | Ac-Asp-Trp-1Ni-Pro-Glu-leu-[Cys(3MeBn)-Aib- Leu-Ile-Cys]-Ttds-Lys(DOTA)-NH2 | 2332.152 | 2332.157 | 9.3 | A |
| GIP-079 | Ac-Asp-Trp-5Clw-Pro-Glu-leu-[Cys(3MeBn)- Aib-Leu-Ile-Cys]-Ttds-Lys(DOTA)-NH2 | 2355.109 | 2355.113 | 9.1 | A |
| GIP-080 | Ac-Asp-Trp-1Ni-Pro-Glu-leu-[Cys(3MeBn)-Egz- Leu-Ile-Cys]-Ttds-Lys(DOTA)-NH2 | 2372.184 | 2372.206 | 9.5 | A |
| GIP-081 | Ac-Asp-Trp-5Fw-Pro-Glu-leu-[Cys(3MeBn)-Aib- Leu-Ile-Cys]-Ttds-Lys(DOTA)-NH2 | 2339.138 | 2339.144 | 9.2 | A |
| GIP-082 | Ac-Asp-Trp-5Brw-Pro-Glu-leu-[Cys(3MeBn)- Aib-Leu-Ile-Cys]-Ttds-Lys(DOTA)-NH2 | 2399.058 | 2399.066 | 9.5 | A |
| GIP-083 | Ac-Asp-5Fw-1Ni-Pro-Glu-leu-[Cys(3MeBn)-Aib- Leu-Ile-Cys]-Ttds-Lys(DOTA)-NH2 | 2350.143 | 2350.142 | 9.6 | A |
| GIP-084 | Ac-Asp-6Fw-1Ni-Pro-Glu-leu-[Cys(3MeBn)-Aib- Leu-Ile-Cys]-Ttds-Lys(DOTA)-NH2 | 2350.143 | 2350.141 | 9.5 | A |
| GIP-085 | HO-Succinyl-Trp-1Ni-Pro-Glu-Leu- [Cys(3MeBn)-Aib-Leu-Ile-Cys]-Ttds-Lys(DOTA)- NH2 | 2275.131 | 2275.133 | 10.4 | A |
| GIP-086 | DOTA-Ttds-Asp-Trp-5Clw-Pro-Glu-leu- [Cys(3MeBn)-Egz-Leu-Ile-AET] | 2182.029 | 2182.034 | 9.7 | A |
| GIP-087 | DOTA-Ttds-Asp-Trp-5Clw-Pro-Glu-leu- [Cys(3MeBn)-Thp-Leu-Ile-AET] | 2184.008 | 2184.017 | 9.3 | A |
| GIP-088 | DOTA-Ttds-Asp-Trp-5Clw-Pro-Glu-leu- [Cys(3MeBn)-Apc-Leu-Ile-AET] | 2183.024 | 2183.034 | 8.8 | A |
| GIP-089 | DOTA-APAc-Asp-Trp-5Clw-Pro-Glu-leu- [Cys(3MeBn)-Aib-Leu-Ile-AET] | 1979.908 | 1979.915 | 8.8 | A |
| GIP-090 | DOTA-Ttds-Asp-Trp-5Clw-Pro-Glu-leu- [Cys(2Lut)-Aib-Leu-Ile-AET] | 2142.993 | 2143.001 | 8.0 | A |
| GIP-091 | DOTA-Ttds-Asp-Trp-5Clw-Pro-Glu-leu- [Cys(3Lut)-Aib-Leu-Ile-AET] | 2142.993 | 2143.002 | 7.6 | A |
| GIP-092 | DOTA-Ttds-Asp-Trp-5Clw-Pro-Glu-leu- [Dap(3MeBn)-Aib-Leu-Ile-AET] | 2125.036 | 2125.043 | 8.0 | A |
| GIP-093 | DOTA-Ttds-Asp-Trp-5Clw-Pro-Glu-leu- [Cys(3MeBn)-Aib-Leu-Ile-en] | 2125.036 | 2125.043 | 8.4 | A |
| GIP-094 | DOTA-Ttds-Asp-Trp-5Clw-Hyp-Glu-leu- [Cys(3MeBn)-Aib-Leu-Ile-AET] | 2157.992 | 2158.000 | 8.8 | A |
| GIP-095 | DOTA-Ttds-Asp-Trp-5Clw-Tap-Glu-leu- [Cys(3MeBn)-Aib-Leu-Ile-AET] | 2157.008 | 2157.015 | 8.2 | A |
| GIP-096 | DOTA-Ttds-Asp-Trp-5Clw-4Ap-Glu-leu- [Cys(3MeBn)-Aib-Leu-Ile-AET] | 2157.008 | 2157.014 | 8.2 | A |
| GIP-097 | DOTA-Ttds-Asp-Trp-5Clw-Pro-Glu-leu- [Cys(3MeBn)-Apc(Ac)-Leu-Ile-AET] | 2225.034 | 2225.037 | 9.2 | A |
| GIP-098 | DOTA-Ttds-Asp-Trp-5Clw-Pro-Glu-leu- [Dap(3MeBn)-Aib-Leu-Ile-AETO] | 2141.031 | 2141.039 | 7.2 | A |
| GIP-099 | DOTA-Ttds-Asp-Trp-5Clw-Pro-Glu-leu- [Cys(3MeBn)-Aib-Hle-Ile-AET] | 2156.013 | 2156.020 | 9.5 | A |
| GIP-100 | DOTA-Ttds-Asp-Trp-5Clw-Nmg-Glu-leu- [Cys(3MeBn)-Aib-Leu-Ile-AET] | 2115.982 | 2115.987 | 9.1 | A |
| GIP-101 | DOTA-Ttds-Asp-Trp-5Clw-Pro-Glu-leu- [Cys(3MeBn)-Ams-Leu-Ile-AET] | 2157.992 | 2157.997 | 9.0 | A |
| GIP-102 | DOTA-Ttds-Asp-Trp-5Clw-H3p-Glu-leu- [Cys(3MeBn)-Aib-Leu-Ile-AET] | 2157.992 | 2157.989 | 8.8 | A |
| GIP-103 | DOTA-APAc-Asp-Trp-5Clw-Hyp-Glu-leu- [Cys(2Lut)-Thp-Leu-Ile-AET] | 2038.909 | 2038.919 | 7.6 | A |
| GIP-104 | DOTA-Ttds-Asp-6Fw-5Clw-Pro-Glu-leu- [Cys(3MeBn)-Aib-Leu-Ile-AET] | 2159.988 | 2159.993 | 9.2 | A |
| GIP-105 | DOTA-Ttds-Asp-6Fw-5Brw-Pro-Glu-leu- [Cys(3MeBn)-Aib-Leu-Ile-AET] | 2203.937 | 2203.941 | 9.3 | A |
| GIP-106 | DOTA-APAc-Asp-Trp-5Clw-Hyp-Glu-leu- [Dap(2Lut)-Thp-Leu-Ile-AET] | 2021.948 | 2021.894 | 7.6 | A |
| GIP-107 | DOTA-APAc-Asp-Trp-5Clw-Hyp-Glu-leu- [Dap(3MeBn)-Thp-Leu-Ile-AET] | 2020.952 | 2020.958 | 7.6 | A |
| GIP-108 | HO-Succinyl-Trp-5Clw-Hyp-Glu-leu-[Cys(2Lut)- Thp-Leu-Ile-Cys]-Ttds-Lys(DOTA)-NH2 | 2357.088 | 2357.091 | 8.2 | A |
| GIP-109 | HO-Succinyl-Trp-5Clw-Hyp-Glu-leu-[Cys(2Lut)- Thp-Leu-Ile-Cys]-APAc-Lys(DOTA)-NH2 | 2194.999 | 2195.005 | 7.9 | A |
| GIP-110 | HO-Succinyl-Trp-5Clw-Hyp-Glu-leu-[Cys(2Lut)- Apc(DOTA)-Leu-Ile-AET] | 1882.819 | 1882.827 | 8.7 | A |
| GIP-111 | DOTA-Ttds-Asp-Trp-5Clw-4Ap(Ac)-Glu-leu- [Cys(3MeBn)-Aib-Leu-Ile-AET] | 2199.019 | 2199.023 | 8.7 | A |
| GIP-112 | DOTA-Ttds-Asp-Trp-5Clw-Tap-Glu-leu- [Cys(2Lut)-Apc-Leu-Ile-AET] | 2199.030 | 2199.039 | 7.2 | A |
| GIP-113 | DOTA-Ttds-Asp-Trp-5Clw-Tap-Glu-leu- [Dap(2Lut)-Apc-Leu-Ile-AET] | 2182.069 | 2182.079 | 6.8 | A |
| GIP-114 | HO-Succinyl-Trp-5Clw-Hyp-Glu-leu-[Cys(2Lut)- Apc-Leu-Ile-Cys]-APAc-Lys(DOTA)-NH2 | 2194.015 | 2194.019 | 7.9 | A |
| GIP-115 | HO-Succinyl-Trp-5Clw-Hyp-Glu-leu-[Cys(2Lut)- Thp-Leu-Ile-Cys]-Lys(DOTA)-NH2 | 2054.904 | 2054.907 | 8.2 | A |
| GIP-116 | HO-Succinyl-Trp-5Clw-Hyp-Glu-leu-[Cys(2Lut)- Thp-Leu-Ile-Cys]-lys(DOTA)-NH2 | 2054.904 | 2054.907 | 8.0 | A |
| GIP-117 | HO-Succinyl-Trp-5Clw-Hyp-Glu-leu-[Cys(2Lut)- Thp-Leu-Ile-Cys]-Ape-DOTA | 2011.898 | 2011.902 | 8.3 | A |
| GIP-118 | HO-Succinyl-Trp-5Clw-Hyp-Glu-leu-[Cys(2Lut)- Thp-Leu-Ile-Cys]-PP-DOTA | 1995.867 | 1995.874 | 8.4 | A |
| GIP-119 | DOTA-Ttds-Asp-Trp-5Clw-Pro-Glu-leu- [Cys(2Lut)-Apc-Leu-Ile-AET] | 2184.019 | 2184.029 | 7.9 | A |
| GIP-120 | MeO2kDaPEGAc-Asp-Trp-5Clw-Hyp-Glu-leu- [Cys(2Lut)-Thp-Leu-Ile-Cys]-lys(DOTA)-NH2 | n.d. | n.d. | 11.6^{∗} | A |
| GIP-121 | Ac-Asp-Trp-5Clw-Tap(DOTA)-Glu-leu- [Cys(2Lut)-Thp-Leu-Ile-Cys]-NH2 | 1982.846 | 1982.856 | 7.9 | A |
| GIP-122 | Ac-Asp-Trp-5Clw-Hyp-Glu-leu-[Cys(2Lut)- Apc(DOTA)-Leu-Ile-Cys]-NH2 | 1982.846 | 1982.867 | 8.8 | A |
| GIP-123 | Ac-Asp-Trp-5Clw-Tap-Glu-leu-[Cys(2Lut)- Apc(DOTA)-Leu-Ile-Cys]-NH2 | 1981.862 | 1981.885 | 8.2 | A |
| GIP-124 | Ac-Asp-Trp-5Clw-Hyp-Glu-leu- [Cys(tMeBn(DOTA-AET))-Thp-Leu-Ile-Cys]-NH2 | 2071.865 | 2071.894 | 9.5 | A |
| GIP-125 | DOTA-Asp-Trp-5Clw-Hyp-Glu-leu-[Cys(2Lut)- Thp-Leu-Ile-Cys]-NH2 | 1941.820 | 1941.816 | 8.2 | A |
| GIP-126 | DOTA-Ttds-Tyr-Asp-Trp-Trp-Pro-Glu-Leu- [Cys(3MeBn)-Ala-Leu-Ile-Cys]-NH₂ | 2300.090 | 2300.097 | 9.3 | B |
| GIP-127 | Ac-Tyr-Asp-Trp-Trp-Pro-Glu-Leu-[Cys(3MeBn)- Ala-Leu-Ile-Cys]-Ttds-Lys(DOTA)-NH2 | 2470.195 | 2470.202 | 9.4 | B |
| GIP-128 | DOTA-Ttds-Asp-Trp-Trp-Pro-Glu-leu- [Cys(3MeBn)-Aib-Leu-Ile-Cys]-NH2 | 2151.042 | 2151.049 | 8.7 | B |
| GIP-129 | DOTA-Ttds-Asp-Trp-Trp-Pro-Glu-leu- [Cys(3MeBn)-Aib-Leu-Ile-AET] | 2108.036 | 2108.044 | 8.8 | B |
| GIP-130 | DOTA-Ttds-Nmd-Trp-Trp-Pro-Glu-Leu- [Cys(3MeBn)-Aib-Leu-Ile-AET] | 2122.052 | 2122.058 | 9.2 | B |
| GIP-131 | Ac-Asp-Trp-Trp-Pro-Glu-Leu-[Cys(3MeBn)-Aib- Leu-Ile-Cys]-Ttds-Lys(DOTA)-NH2 | 2321.148 | 2321.157 | 9.2 | B |
| GIP-132 | Ac-Asp-Trp-Trp-Pro-Glu-Npg-[Cys(3MeBn)-Aib- Leu-Ile-Cys]-Ttds-Lys(DOTA)-NH2 | 2335.163 | 2335.169 | 9.5 | B |
| GIP-133 | Ac-Asp-Trp-Trp-Pro-Glu-Leu-[Cys(3MeBn)-Aib- Npg-Ile-Cys]-Ttds-Lys(DOTA)-NH2 | 2335.163 | 2335.171 | 9.2 | B |
| GIP-134 | Ac-Asp-Trp-Trp-Pro-Glu-Leu-[Cys(3MeBn)-Aib- Cha-Ile-Cys]-Ttds-Lys(DOTA)-NH2 | 2361.179 | 2361.188 | 9.7 | B |
| GIP-135 | Php-Asp-Trp-Trp-Pro-Glu-leu-[Cys(3MeBn)- Aib-Leu-Ile-Cys]-Ttds-Lys(DOTA)-NH2 | 2411.195 | 2411.201 | 9.7 | B |
| GIP-136 | 4FPhp-Asp-Trp-Trp-Pro-Glu-leu-[Cys(3MeBn)- Aib-Leu-Ile-Cys]-Ttds-Lys(DOTA)-NH2 | 2429.185 | 2429.189 | 9.8 | B |
| GIP-137 | 4OHPhp-Asp-Trp-Trp-Pro-Glu-leu- [Cys(3MeBn)-Aib-Leu-Ile-Cys]-Ttds-Lys(DOTA)- NH2 | 2427.190 | 2427.198 | 9.0 | B |
| GIP-138 | Ac-Asp-Trp-1Ni-Pro-Glu-leu-[Cys(3MeBn)-Ala- Leu-Val-Cys]-Ttds-Lys(DOTA)-NH2 | 2304.121 | 2304.128 | 9.0 | B |
| GIP-139 | Ac-Asp-Trp-1Ni-Pro-Glu-leu-[Cys(3MeBn)-Deg- Leu-Ile-Cys]-Ttds-Lys(DOTA)-NH2 | 2360.184 | 2360.198 | 9.3 | B |
| GIP-140 | Ac-Asp-Trp-6Fw-Pro-Glu-leu-[Cys(3MeBn)-Aib- Leu-Ile-Cys]-Ttds-Lys(DOTA)-NH2 | 2339.138 | 2339.122 | 9.5 | B |
| GIP-141 | DOTA-Ttds-Asp-6Clw-Trp-Pro-Glu-leu- [Cys(3MeBn)-Aib-Leu-Ile-AET] | 2141.997 | 2142.007 | 9.2 | B |
| GIP-142 | HO-Succinyl-Trp-5Clw-Hyp-Glu-leu-[Cys(2Lut)- Apc(MeO10kDaPEGAc)-Leu-Ile-Cys]-APAc- Lys(DOTA)-NH2 | n.d. | n.d. | 12.3^{∗} | B |
| GIP-143 | HO-Succinyl-Trp-5Clw-Hyp-Glu-leu-[Cys(2Lut)- Apc(MeO2kDaPEGAc)-Leu-Ile-Cys]-APAc- Lys(DOTA)-NH2 | n.d. | n.d. | 11.6^{∗} | B |
| GIP-144 | HO-Succinyl-Trp-5Clw-Hyp-Glu-leu-[Cys(2Lut)- Thp-Leu-Ile-Cys]-APAc-lys(MeO2kDaPEGAc)- Lys(DOTA)-NH2 | n.d. | n.d. | 11.5^{∗} | B |
| GIP-145 | Ac-Asp-Trp-5Clw-Hyp-Glu-leu- [Cys(tMeBn(DOTA-PP))-Thp-Leu-Ile-Cys]-NH2 | 2080.919 | 2080.900 | 8.8 | B |
| GIP-146 | AGLU-Glutar-Asp-Trp-5Clw-Hyp-Glu-leu- [Cys(2Lut)-Apc(InDOTA)-Leu-Ile-AET] | 2284.827 | 2284.827 | 7.7 | A |
| GIP-147 | AGLU-Glutar-Asp-Trp-5Clw-Hyp-Glu-leu- [Cys(2Lut)-Apc(ZnDOTA)-Leu-Ile-AET] | 2236.860 | 2236.861 | 7.9 | A |
| GIP-148 | InDOTA-Ttds-Asp-Trp-5Clw-Pro-Glu-leu- [Cys(3MeBn)-Aib-Leu-Ile-AET] | 2251.878 | 2251.886 | 9.2 | A |
| GIP-149 | InDOTA-Ttds-Asp-Trp-5Clw-Pro-Glu-leu- [Cys(3MeBn)-Ams-Leu-Ile-AET] | 2267.873 | 2267.879 | 9.0 | A |
| GIP-150 | InDOTA-Ttds-Asp-Trp-5Clw-Pro-Glu-leu- [Cys(3MeBn)-Thp-Leu-Ile-AET] | 2293.888 | 2293.894 | 9.3 | A |
| GIP-151 | InDOTA-Ttds-Asp-Trp-5Clw-Hyp-Glu-leu- [Cys(3MeBn)-Aib-Leu-Ile-AET] | 2267.873 | 2267.880 | 8.9 | A |
| GIP-152 | InDOTA-Ttds-Asp-Trp-5Clw-Nmg-Glu-leu- [Cys(3MeBn)-Aib-Leu-Ile-AET] | 2225.862 | 2225.869 | 9.0 | A |
| GIP-153 | InDOTA-APAc-Asp-Trp-5Clw-Pro-Glu-leu- [Cys(3MeBn)-Aib-Leu-Ile-AET] | 2089.789 | 2089.794 | 8.8 | A |
| GIP-154 | InDOTA-Ttds-Asp-Trp-5Clw-Pro-Glu-leu- [Cys(2Lut)-Aib-Leu-Ile-AET] | 2252.873 | 2252.880 | 8.0 | A |
| GIP-155 | InDOTA-APAc-Asp-Trp-5Clw-Hyp-Glu-leu- [Cys(2Lut)-Thp-Leu-Ile-AET] | 2148.789 | 2148.796 | 7.7 | A |
| GIP-156 | Ac-Asp-Trp-5Clw-Pro-Glu-leu-[Cys(3MeBn)- Aib-Leu-Ile-Cys]-Ttds-Lys(LuDOTA)-NH2 | 2527.026 | 2527.028 | 9.1 | A |
| GIP-157 | LuDOTA-Ttds-Asp-Trp-5Clw-Pro-Glu-leu- [Cys(3MeBn)-Ams-Leu-Ile-AET] | 2329.910 | 2329.914 | 9.0 | A |
| GIP-158 | LuDOTA-Ttds-Asp-Trp-5Clw-Pro-Glu-leu- [Cys(3MeBn)-Thp-Leu-Ile-AET] | 2355.925 | 2355.927 | 9.4 | A |
| GIP-159 | LuDOTA-Ttds-Asp-Trp-5Clw-Hyp-Glu-leu- [Cys(3MeBn)-Aib-Leu-Ile-AET] | 2329.910 | 2329.912 | 8.9 | A |
| GIP-160 | LuDOTA-Ttds-Asp-Trp-5Clw-Nmg-Glu-leu- [Cys(3MeBn)-Aib-Leu-Ile-AET] | 2287.899 | 2287.903 | 9.0 | A |
| GIP-161 | LuDOTA-APAc-Asp-Trp-5Clw-Pro-Glu-leu- [Cys(3MeBn)-Aib-Leu-Ile-AET] | 2151.825 | 2151.829 | 8.9 | A |
| GIP-162 | LuDOTA-APAc-Asp-Trp-5Clw-Hyp-Glu-leu- [Cys(2Lut)-Thp-Leu-Ile-AET] | 2210.826 | 2210.827 | 7.7 | A |
| GIP-163 | LuDOTA-Ttds-Asp-Trp-5Clw-Pro-Glu-leu- [Cys(2Lut)-Aib-Leu-Ile-AET] | 2314.910 | 2314.912 | 8.1 | A |
| GIP-164 | Ac-Asp-Trp-5Clw-Pro-Glu-leu-[Cys(3MeBn)- Aib-Leu-Ile-Cys]-Ttds-Lys(InDOTA)-NH2 | 2464.989 | 2464.997 | 9.1 | A |
| GIP-165 | Ac-Asp-Trp-1Ni-Pro-Glu-leu-[Cys(3MeBn)-Aib- Leu-Ile-Cys]-Ttds-Lys(InDOTA)-NH2 | 2442.033 | 2442.038 | 9.3 | A |
| GIP-166 | Ac-Asp-Trp-1Ni-Pro-Glu-leu-[Cys(3MeBn)-Egz- Leu-Ile-Cys]-Ttds-Lys(InDOTA)-NH2 | 2482.064 | 2482.067 | 9.6 | A |
| GIP-167 | LuDOTA-Ttds-Asp-Trp-5Clw-Pro-Glu-leu- [Cys(3MeBn)-Aib-Leu-Ile-AET] | 2313.915 | 2313.919 | 9.2 | A |
| GIP-168 | HO-Succinyl-Trp-5Clw-Hyp-Glu-leu-[Cys(2Lut)- Thp-Leu-Ile-Cys]-APAc-Lys(InDOTA)-NH2 | 2304.879 | 2304.882 | 8.0 | A |
| GIP-169 | HO-Succinyl-Trp-5Clw-Hyp-Glu-leu-[Cys(2Lut)- Apc(InDOTA)-Leu-Ile-AET] | 1992.700 | 1992.701 | 8.7 | A |
| GIP-170 | HO-Succinyl-Trp-5Clw-Hyp-Glu-leu-[Cys(2Lut)- Thp-Leu-Ile-Cys]-APAc-Lys(LuDOTA)-NH2 | 2366.916 | 2366.920 | 8.1 | A |
| GIP-171 | HO-Succinyl-Trp-5Clw-Hyp-Glu-leu-[Cys(2Lut)- Apc(LuDOTA)-Leu-Ile-AET] | 2054.736 | 2054.738 | 8.7 | A |
| GIP-172 | Ac-Asp-Trp-1Ni-Pro-Glu-leu-[Cys(3MeBn)-Aib- Leu-Ile-Cys]-Ttds-Lys(LuDOTA)-NH2 | 2504.070 | 2504.072 | 9.3 | B |
| GIP-173 | Ac-Asp-Trp-1Ni-Pro-Glu-leu-[Cys(3MeBn)-Egz- Leu-Ile-Cys]-Ttds-Lys(LuDOTA)-NH2 | 2544.101 | 2544.104 | 9.7 | B |
| GIP-174 | AGLU-Glutar-Asp-Trp-5Clw-Hyp-Glu-leu- [Cys(2Lut)-Thp-Leu-Ile-Cys]-APAc-glu(AGLU)- NH2 | 2264.978 | 2264.987 | 7.4 | A |
| GIP-175 | Ac-Asp-Trp-5Clw-Tap(AGLU-Glutar)-Glu-leu- [Cys(2Lut)-Thp-Leu-Ile-Cys]-NH2 | 1873.782 | 1873.784 | 8.3 | A |
| GIP-176 | Ac-Asp-Trp-5Clw-Nglu(AGLU)-Glu-leu- [Cys(2Lut)-Thp-Leu-Ile-Cys]-NH2 | 1776.729 | 1776.731 | 8.4 | A |
| GIP-177 | Ac-Asp-Trp-5Clw-Hyp-Glu-leu-[Cys(2Lut)-Thp- Leu-Ile-Cys]-APAc-glu(AGLU)-NH2 | 2029.872 | 2029.882 | 8.1 | A |
| GIP-178 | AGLU-Glutar-Asp-Trp-5Clw-Hyp-Glu-leu- [Cys(2Lut)-Thp-Leu-Ile-Cys]-NH2 | 1832.756 | 1832.770 | 8.3 | A |
| GIP-179 | Ac-Cya-Trp-5Clw-Hyp-Glu-leu-[Cys(2Lut)-Aib- Leu-Ile-AET] | 1548.601 | 1548.603 | 8.7 | A |
| GIP-180 | Ac-Asp-Trp-5Clw-Hyp-Glu-leu-[Cys(2Lut)-Thp- Leu-Ile-Cys]-NH2 | 1597.650 | 1597.659 | 9.0 | A |
| GIP-181 | Ac-Asp-Trp-5Clw-Hyp-Glu-leu-[Cys(2Lut)-Thp- Leu-Ile-Cys]-OH | 1598.634 | 1598.643 | 8.8 | A |
| GIP-182 | Ac-Asp-Trp-5Clw-Hyp-Glu-leu-[Cys(2Lut)-Thp- Leu-Ile-Cysol]-OH | 1584.655 | 1584.662 | 8.9 | A |
| GIP-183 | Ac-Asp-Trp-5Clw-Nmg-Glu-leu-[Cys(2Lut)-Thp- Leu-Ile-Cys]-NH2 | 1555.640 | 1555.641 | 8.8 | A |
| GIP-184 | Ac-Asp-Trp-5Clw-Nleu-Glu-leu-[Cys(2Lut)-Thp- Leu-Ile-Cys]-NH2 | 1597.687 | 1597.690 | 9.6 | A |
| GIP-185 | Ac-Asp-Trp-5Clw-Nphe-Glu-leu-[Cys(2Lut)- Thp-Leu-Ile-Cys]-NH2 | 1631.671 | 1631.674 | 9.7 | A |
| GIP-186 | Ac-Asp-Trp-5Clw-Nlys-Glu-leu-[Cys(2Lut)-Thp- Leu-Ile-Cys]-NH2 | 1612.697 | 1612.701 | 8.1 | A |
| GIP-187 | Ac-Asp-Trp-5Clw-4Tfp-Glu-leu-[Cys(2Lut)-Thp- Leu-Ile-Cys]-NH2 | 1599.646 | 1599.659 | 9.4 | A |
| GIP-188 | Ac-Ser-Trp-5Clw-Hyp-Glu-leu-[Cys(2Lut)-Thp- Leu-Ile-Cys]-NH2 | 1569.655 | 1569.666 | 8.9 | A |
| GIP-189 | Ac-Cya-Trp-5Clw-Hyp-Glu-leu-[Cys(2Lut)-Thp- Leu-Ile-Cys]-NH2 | 1633.617 | 1633.627 | 8.7 | A |
| GIP-190 | Ac-Asn-Trp-5Clw-Hyp-Glu-leu-[Cys(2Lut)-Thp- Leu-Ile-Cys]-NH2 | 1596.666 | 1596.677 | 8.9 | A |
| GIP-191 | Ac-Glu-Trp-5Clw-Hyp-Glu-leu-[Cys(2Lut)-Thp- Leu-Ile-Cys]-NH2 | 1611.666 | 1611.677 | 8.9 | A |
| GIP-192 | Ac-Asp-Trp-5Clw-Hyp-Asp-leu-[Cys(2Lut)-Thp- Leu-Ile-Cys]-NH2 | 1583.634 | 1583.646 | 8.8 | A |
| GIP-193 | Ac-Asp-Trp-5Clw-Hyp-Gln-leu-[Cys(2Lut)-Thp- Leu-Ile-Cys]-NH2 | 1596.666 | 1596.679 | 8.7 | A |
| GIP-194 | Ac-Asp-Trp-5Clw-Hyp-Hse-leu-[Cys(2Lut)-Thp- Leu-Ile-Cys]-NH2 | 1569.655 | 1569.667 | 8.9 | A |
| GIP-195 | Ac-Hse-Trp-5Clw-Hyp-Glu-leu-[Cys(2Lut)-Thp- Leu-Ile-Cys]-NH2 | 1583.671 | 1583.686 | 8.9 | A |
| GIP-196 | Ac-Asp-Trp-5Clw-Nglu-Glu-leu-[Cys(2Lut)-Thp- Leu-Ile-Cys]-NH2 | 1613.645 | 1613.648 | 9.0 | A |
| GIP-197 | Ac-Hse-Trp-5Brw-Hyp-Glu-leu-[Cys(2Lut)-Thp- Leu-Ile-Cys]-NH2 | 1627.620 | 1627.637 | 9.4 | A |
| GIP-198 | HO-Succinyl-Trp-5Clw-Pro-Glu-leu- [Cys(3MeBn)-Aib-Leu-Ile-Cys]-NH2 | 1481.628 | 1481.631 | 11.3 | A |
| GIP-199 | HO-Succinyl-Trp-5Clw-Pro-Glu-leu- [Cys(3MeBn)-Thp-Leu-Ile-Cys]-NH2 | 1523.638 | 1523.642 | 11.5 | A |
| GIP-200 | Ac-Asp-Trp-5Clw-Hyp-Glu-leu-[Hcy(2Lut)-Thp- Leu-Ile-Cys]-NH2 | 1611.666 | 1611.680 | 9.5 | A |
| GIP-201 | Ac-Asp-Trp-5Clw-Hyp-Glu-leu-[Cys(2Lut)-Thp- Leu-Ile-Hcy]-NH2 | 1611.666 | 1611.684 | 9.6 | A |
| GIP-202 | Ac-Asp-Trp-5Clw-Hyp-Glu-leu-[Hcy(2Lut)-Thp- Leu-Ile-Hcy]-NH2 | 1625.681 | 1625.697 | 9.7 | A |
| GIP-203 | Ac-Asp-Trp-5Clw-Hyp-Glu-leu-[Cys(2Lut)-Eca- Leu-Ile-Cys]-NH2 | 1581.655 | 1581.672 | 9.5 | A |
| GIP-204 | Ac-Gln-Trp-5Clw-Hyp-Glu-leu-[Cys(2Lut)-Thp- Leu-Ile-Cys]-NH2 | 1610.682 | 1610.700 | 9.2 | A |
| GIP-205 | Ac-Asp-Trp-5Clw-Hyp-Asn-leu-[Cys(2Lut)-Thp- Leu-Ile-Cys]-NH2 | 1582.650 | 1582.669 | 8.9 | A |
| GIP-206 | Ac-Asp-Trp-5Clw-Hyp-Glu-leu-[Cys(2Lut)-Aic- Leu-Ile-Cys]-NH2 | 1629.655 | 1629.675 | 9.9 | A |
| GIP-207 | Ac-Pse-Trp-5Clw-Hyp-Glu-leu-[Cys(2Lut)-Thp- Leu-Ile-Cys]-NH2 | 1649.622 | 1649.641 | 9.1 | A |
| GIP-208 | Ac-Asp-Trp-5Clw-Hyp-Pse-leu-[Cys(2Lut)-Thp- Leu-Ile-Cys]-NH2 | 1635.606 | 1635.620 | 8.3 | A |
| GIP-209 | Ac-Asp-Mcf-5Clw-Hyp-Glu-leu-[Cys(2Lut)-Thp- Leu-Ile-Cys]-NH2 | 1592.600 | 1592.611 | 10.0 | A |
| GIP-210 | Ac-Asp-Pcf-5Clw-Hyp-Glu-leu-[Cys(2Lut)-Thp- Leu-Ile-Cys]-NH2 | 1592.600 | 1592.609 | 10.0 | A |
| GIP-211 | Ac-Asp-Eaa-5Clw-Hyp-Glu-leu-[Cys(2Lut)-Thp- Leu-Ile-Cys]-NH2 | 1626.561 | 1626.569 | 10.4 | A |
| GIP-212 | Ac-Asp-Egm-5Clw-Hyp-Glu-leu-[Cys(2Lut)-Thp- Leu-Ile-Cys]-NH2 | 1626.561 | 1626.571 | 10.5 | A |
| GIP-213 | Ac-Asp-Trp-Eaa-Hyp-Glu-leu-[Cys(2Lut)-Thp- Leu-Ile-Cys]-NH2 | 1592.600 | 1592.613 | 9.9 | A |
| GIP-214 | HO-Glutar-Trp-5Clw-Hyp-Glu-leu-[Cys(2Lut)- Thp-Leu-Ile-Cys]-NH2 | 1554.644 | 1554.657 | 9.5 | A |
| GIP-215 | Ac-Asp-Trp-5Clw-Dtc-Glu-leu-[Cys(2Lut)-Thp- Leu-Ile-Cys]-NH2 | 1627.643 | 1627.654 | 10.2 | A |
| GIP-216 | Ac-Asp-Trp-5Clw-Oxa-Glu-leu-[Cys(2Lut)-Thp- Leu-Ile-Cys]-NH2 | 1583.634 | 1583.639 | 9.6 | A |
| GIP-217 | Ac-Asp-Trp-5Clw-Hyp-Nme-Leu-[Cys(2Lut)- Thp-Leu-Ile-Cys]-NH2 | 1611.666 | 1611.672 | 9.4 | A |
| GIP-218 | Ac-Asp-Trp-5Clw-Hyp-Glu-Ile-[Cys(2Lut)-Thp- Leu-Ile-Cys]-NH2 | 1597.650 | 1597.656 | 10.2 | A |
| GIP-219 | Ac-Asp-Trp-5Clw-Hyp-Glu-Val-[Cys(2Lut)-Thp- Leu-Ile-Cys]-NH2 | 1583.634 | 1583.639 | 9.9 | A |
| GIP-220 | Ac-Asp-Hyw-5Clw-Hyp-Glu-leu-[Cys(2Lut)-Thp- Leu-Ile-Cys]-NH2 | 1613.645 | 1613.668 | 8.7 | A |
| GIP-221 | Ac-Asp-7Fw-5Clw-Hyp-Glu-leu-[Cys(2Lut)-Thp- Leu-Ile-Cys]-NH2 | 1615.641 | 1615.661 | 9.7 | A |
| GIP-222 | Ac-Asp-Trp-5Clw-Eaz-Glu-leu-[Cys(2Lut)-Thp- Leu-Ile-Cys]-NH2 | 1599.612 | 1599.619 | 9.8 | A |
| GIP-223 | Ac-Asp-Trp-5Clw-Hyp-glu-Leu-[Cys(2Lut)-Thp- Leu-Ile-Cys]-NH2 | 1597.650 | 1597.664 | 9.8 | A |
| GIP-224 | Ac-Asp-Phe-5Clw-Hyp-Glu-leu-[Cys(2Lut)-Thp- Leu-Ile-Cys]-NH2 | 1558.639 | 1558.647 | 9.5 | A |
| GIP-225 | Ac-Asp-1Qi-5Clw-Hyp-Glu-leu-[Cys(2Lut)-Thp- Leu-Ile-Cys]-NH2 | 1609.650 | 1609.671 | 8.1 | A |
| GIP-226 | Ac-Asp-Egp-5Clw-Hyp-Glu-leu-[Cys(2Lut)-Thp- Leu-Ile-Cys]-NH2 | 1626.561 | 1626.575 | 10.3 | A |
| GIP-227 | Ac-Asp-Trp-7Fw-Hyp-Glu-leu-[Cys(2Lut)-Thp- Leu-Ile-Cys]-NH2 | 1581.680 | 1581.692 | 9.2 | A |
| GIP-228 | Ac-Asp-Trp-5Clw-Hyp-Glu-leu-[Cys(2Lut)-Phe- Leu-Ile-Cys]-NH2 | 1617.655 | 1617.652 | 9.6 | A |
| GIP-229 | Ac-Asp-Trp-5Clw-Hyp-Glu-leu-[Cys(2Lut)-Thp- Ile-Ile-Cys]-NH₂ | 1597.650 | 1597.653 | 9.5 | A |
| GIP-230 | Ac-Asp-Trp-5Clw-Hyp-Glu-leu-[Cys(2Lut)-Thp- Arg-Ile-Cys]-NH2 | 1640.667 | 1640.675 | 7.7 | A |
| GIP-231 | Ac-Asp-Trp-5Clw-Hyp-Glu-leu-[Cys(2Lut)-Thp- Glu-Ile-Cys]-NH2 | 1613.609 | 1613.622 | 8.1 | A |
| GIP-232 | Ac-Asp-Trp-5Clw-Hyp-Glu-leu-[Cys(2Lut)-Thp- Phe-Ile-Cys]-NH2 | 1631.634 | 1631.649 | 9.3 | A |
| GIP-233 | Ac-Asp-Trp-5Clw-Hyp-Glu-leu-[Cys(2Lut)-Thp- Trp-Ile-Cys]-NH2 | 1670.645 | 1670.675 | 9.3 | A |
| GIP-234 | Ac-Asp-Trp-5Clw-Hyp-Glu-leu-[Cys(2Lut)-Trp- Leu-Ile-Cys]-NH2 | 1656.666 | 1656.700 | 9.5 | A |
| GIP-235 | Ac-Asp-Trp-5Clw-Hyp-Glu-leu-[Cys(2Lut)-Glu- Leu-Ile-Cys]-NH2 | 1599.629 | 1599.628 | 9.1 | A |
| GIP-236 | Ac-Asp-Trp-5Clw-Hyp-Glu-leu-[Cys(2Lut)-Thp- Ser-Ile-Cys]-NH2 | 1571.598 | 1571.623 | 8.0 | A |
| GIP-237 | Ac-Asp-Trp-5Clw-Hyp-Glu-leu-[Cys(2Lut)-Thp- Leu-Leu-Cys]-NH2 | 1597.650 | 1597.659 | 9.5 | A |
| GIP-238 | Ac-Asp-Trp-5Clw-Hyp-Glu-leu-[Cys(2Lut)-Thp- Leu-Tle-Cys]-NH2 | 1597.650 | 1597.697 | 9.5 | A |
| GIP-239 | Ac-Asp-Trp-5Clw-Tap-Glu-leu-[Cys(2Lut)-Thp- Leu-Ile-Cys]-NH2 | 1596.666 | 1596.638 | 8.5 | A |
| GIP-240 | Ac-Tyr-Asp-Trp-Trp-Pro-Glu-Leu-[Cys(3MeBn)- Ala-Leu-Ile-Cys]-NH2 | 1653.736 | 1653.738 | 10.7 | B |
| GIP-241 | H-Met-Tyr-Asp-Trp-Trp-Pro-Glu-Leu- [Cys(3MeBn)-Gln-Leu-Ile-Cys]-NH2 | 1799.787 | 1799.790 | 9.7 | B |
| GIP-242 | H-Met-Tyr-Asp-Trp-Trp-Pro-Glu-Leu- [Cys(3MeBn)-Thr-Leu-Ile-Cys]-NH2 | 1772.777 | 1772.780 | 9.7 | B |
| GIP-243 | H-Met-Tyr-Asp-Trp-Trp-Pro-Glu-Leu- [Cys(3MeBn)-Lys-Leu-Ile-Cys]-NH₂ | 1799.824 | 1799.829 | 9.3 | B |
| GIP-244 | Ac-Asp-Trp-Trp-Pro-Glu-Leu-[Cys(3MeBn)-Ala- Leu-Ile-Cys]-NH2 | 1490.673 | 1490.676 | 10.6 | B |
| GIP-245 | H-Met-Tyr-Asp-Trp-Trp-Pro-glu-Leu- [Cys(3MeBn)-Ala-Leu-Ile-Cys]-NH2 | 1742.766 | 1742.790 | 9.5 | B |
| GIP-246 | H-Met-tyr-Asp-Trp-Trp-Pro-Glu-Leu- [Cys(3MeBn)-Ala-Leu-Ile-Cys]-NH2 | 1742.766 | 1742.769 | 9.8 | B |
| GIP-247 | H-Met-Tyr-Asp-Trp-Trp-Pro-Glu-leu- [Cys(3MeBn)-Ala-Leu-Ile-Cys]-NH₂ | 1742.766 | 1742.769 | 9.5 | B |
| GIP-248 | H-Met-Tyr-Asp-Trp-Trp-Pro-Glu-Leu- [Cys(3MeBn)-ala-Leu-Ile-Cys]-NH2 | 1742.766 | 1742.765 | 9.5 | B |
| GIP-249 | H-Met-Ala-Asp-Trp-Trp-Pro-Glu-Leu- [Cys(3MeBn)-Ala-Leu-Ile-Cys]-NH2 | 1650.740 | 1650.743 | 9.6 | B |
| GIP-250 | Ac-Asp-Trp-Trp-Pro-Glu-Leu-[Cys(3MeBn)-ala- Leu-Ile-AET] | 1447.667 | 1447.670 | 10.4 | B |
| GIP-251 | Ac-Asp-Trp-Trp-Pro-Glu-Leu-[Cys(3MeBn)-Aib- Leu-Ile-AET] | 1461.683 | 1461.685 | 10.9 | B |
| GIP-252 | Ac-Asp-Trp-1Ni-Oic-Glu-Leu-[Cys(3MeBn)-Aib- Leu-Ile-Cys]-NH2 | 1569.740 | 1569.742 | 11.8 | B |
| GIP-253 | Ac-Asp-Trp-1Ni-Pip-Glu-Leu-[Cys(3MeBn)-Aib- Leu-Ile-Cys]-NH2 | 1529.709 | 1529.711 | 11.6 | B |
| GIP-254 | H-Met-Tyr-Asp-Trp-Trp-Pro-Glu-Leu- [Cys(3MeBn)-Ala-Leu-Ile-Cys]-NH₂ | 1742.766 | 1742.776 | 9.8 | B |
| GIP-255 | HO-Succinyl-Trp-Trp-Pro-Glu-Leu- [Cys(3MeBn)-Ala-Leu-Ile-Cys]-NH2 | 1433.651 | 1433.667 | 11.5 | B |
| GIP-256 | HO-Succinyl-Trp-Trp-Pro-Glu-Leu- [Cys(3MeBn)-Ala-Leu-Ile-AET] | 1390.645 | 1390.659 | 11.6 | B |
| GIP-257 | HO-Succinyl-Trp-Trp-Pro-Glu-Leu-[Cys(2Lut)- Ala-Leu-Ile-Cys]-NH2 | 1434.647 | 1434.653 | 9.8 | B |
| GIP-258 | HO-Succinyl-Trp-Trp-Pro-Glu-leu- [Cys(3MeBn)-Aib-Leu-Ile-Cys]-NH2 | 1447.667 | 1447.672 | 10.9 | B |
| GIP-259 | HO-Succinyl-Trp-Trp-Pro-Glu-Leu- [Cys(3MeBn)-Aib-Leu-Ile-Cys]-NH2 | 1447.667 | 1447.674 | 11.6 | B |
| GIP-260 | HO-Succinyl-Trp-Trp-Pro-Glu-Leu- [Cys(3MeBn)-Aib-Hle-Ile-Cys]-NH2 | 1461.683 | 1461.686 | 12.1 | B |
| GIP-261 | HO-Succinyl-Trp-Trp-Pro-Glu-Leu- [Cys(3MeBn)-Thp-Leu-Ile-Cys]-NH2 | 1489.677 | 1489.683 | 11.6 | B |
| GIP-262 | Ac-Asp-Ocf-5Clw-Hyp-Glu-leu-[Cys(2Lut)-Thp- Leu-Ile-Cys]-NH2 | 1592.600 | 1592.615 | 9.9 | B |
| GIP-263 | Ac-Asp-Egn-5Clw-Hyp-Glu-leu-[Cys(2Lut)-Thp- Leu-Ile-Cys]-NH2 | 1626.561 | 1626.569 | 10.5 | B |
| GIP-264 | Ac-Asp-Trp-Egn-Hyp-Glu-leu-[Cys(2Lut)-Thp- Leu-Ile-Cys]-NH2 | 1592.600 | 1592.606 | 9.9 | B |
| GIP-265 | Ac-Asp-Trp-Egp-Hyp-Glu-leu-[Cys(2Lut)-Thp- Leu-Ile-Cys]-NH2 | 1592.600 | 1592.612 | 9.6 | B |
| GIP-266 | Ac-Asp-Trp-5Clw-Hyp-Glu-leu-[Cys(2Lut)-Thp- Leu-Phe-Cys]-NH2 | 1631.634 | 1631.674 | 9.5 | B |
| GIP-267 | Ac-Asp-Trp-7Nw-Hyp-Glu-leu-[Cys(2Lut)-Thp- Leu-Ile-Cys]-NH2 | 1564.684 | 1564.729 | 7.4 | B |
| GIP-268 | DOTA-Ttds-Asp-Trp-Trp-Pro-Glu-leu- [Cys(3MeBn)-ala-Leu-Ile-Cys]-NH2 | 2137.026 | 2137.035 | 8.3 | C |
| GIP-269 | DOTA-Ttds-Asp-Trp-Trp-Pro-glu-Leu- [Cys(3MeBn)-ala-Leu-Ile-Cys]-NH2 | 2137.026 | 2137.034 | 8.5 | C |
| GIP-270 | DOTA-Ttds-Asp-Trp-Trp-Pro-glu-Leu- [Cys(3MeBn)-Aib-Leu-Ile-Cys]-NH2 | 2151.042 | 2151.050 | 8.8 | C |
| GIP-271 | DOTA-Ttds-asp-Trp-Trp-Pro-Glu-Leu- [Cys(3MeBn)-ala-Leu-Ile-Cys]-NH2 | 2137.026 | 2137.034 | 8.8 | C |
| GIP-272 | Ac-Asp-2Ni-Trp-Pro-Glu-Leu-[Cys(3MeBn)-Aib- Leu-Ile-Cys]-Ttds-Lys(DOTA)-NH2 | 2332.152 | 2332.160 | 9.8 | C |
| GIP-273 | Ac-Asp-Bta-Trp-Pro-Glu-Leu-[Cys(3MeBn)-Aib- Leu-Ile-Cys]-Ttds-Lys(DOTA)-NH2 | 2338.109 | 2338.121 | 9.7 | C |
| GIP-274 | Ac-Asp-Trp-Trp-Pro-Nme-Leu-[Cys(3MeBn)- Aib-Leu-Ile-Cys]-Ttds-Lys(DOTA)-NH2 | 2335.163 | 2335.168 | 8.8 | C |
| GIP-275 | Ac-Asp-Trp-Trp-Pro-Glu-Tle-[Cys(3MeBn)-Aib- Leu-Ile-Cys]-Ttds-Lys(DOTA)-NH2 | 2321.148 | 2321.153 | 9.3 | C |
| GIP-276 | Ac-Asp-Trp-Trp-Pro-Glu-Leu-[Cys(3MeBn)-Aib- Tle-Ile-Cys]-Ttds-Lys(DOTA)-NH2 | 2321.148 | 2321.153 | 9.2 | C |
| GIP-277 | Ac-Asp-5Clw-Trp-Pro-Glu-Leu-[Cys(3MeBn)- Aib-Leu-Ile-Cys]-Ttds-Lys(DOTA)-NH2 | 2355.109 | 2355.116 | 9.5 | C |
| GIP-278 | Ac-Asp-Trp-Trp-Pro-Glu-Leu-[Cys(3MeBn)-Aib- Leu-Cha-Cys]-Ttds-Lys(DOTA)-NH2 | 2361.179 | 2361.187 | 9.9 | C |
| GIP-279 | 4ClPhp-Asp-Trp-Trp-Pro-Glu-leu-[Cys(3MeBn)- Aib-Leu-Ile-Cys]-Ttds-Lys(DOTA)-NH2 | 2445.156 | 2445.159 | 10.2 | C |
| GIP-280 | Nph1p-Asp-Trp-Trp-Pro-Glu-leu-[Cys(3MeBn)- Aib-Leu-Ile-Cys]-Ttds-Lys(DOTA)-NH2 | 2461.210 | 2461.215 | 10.2 | C |
| GIP-281 | Nph2p-Asp-Trp-Trp-Pro-Glu-leu-[Cys(3MeBn)- Aib-Leu-Ile-Cys]-Ttds-Lys(DOTA)-NH2 | 2461.210 | 2461.215 | 10.3 | C |
| GIP-282 | Ac-Asp-Trp-6Clw-Pro-Glu-leu-[Cys(3MeBn)- Aib-Leu-Ile-Cys]-Ttds-Lys(DOTA)-NH2 | 2355.109 | 2355.114 | 9.1 | C |
| GIP-283 | DOTA-Ttds-Asp-6Fw-Trp-Pro-Glu-leu- [Cys(3MeBn)-Aib-Leu-Ile-AET] | 2126.027 | 2126.033 | 8.9 | C |
| GIP-284 | DOTA-APAc-Asp-Gly-5Clw-Hyp-Glu-leu- [Cys(2Lut)-Thp-Leu-Ile-AET] | 1909.851 | 1909.853 | 7.3 | C |
| GIP-285 | DOTA-Ttds-Asp-Trp-Trp-Pro-Glu-leu- [Cys(3MeBn)-ala-Leu-Ile-Cys]-OH | 2138.010 | 2138.015 | 8.3 | D |
| GIP-286 | HO-Succinyl-Trp-Trp-Pro-Glu-leu- [Cys(tMeBn(DOTA-PP))-ala-Leu-Ile-Cys]-NH2 | 1917.916 | 1917.917 | 7.3 | D |
| GIP-287 | HO-Succinyl-Trp-Trp-Pro-Glu-leu- [Cys(tMeBn(DOTA-AET))-ala-Leu-Ile-Cys]-NH2 | 1908.861 | 1908.867 | 8.0 | D |
| GIP-288 | HO-Succinyl-Trp-Trp-Pro-Glu-leu- [Cys(3MeBn)-ala-Leu-Ile-Cys]-Ttds-Lys(DOTA)- NH2 | 2250.111 | 2250.115 | 8.5 | D |
| GIP-289 | Ac-Asp-Trp-Nmw-Pro-Glu-Leu-[Cys(3MeBn)- Aib-Leu-Ile-Cys]-Ttds-Lys(DOTA)-NH2 | 2335.163 | 2335.172 | 8.8 | D |
| GIP-290 | Ac-Asp-Trp-Trp-Pro-Glu-Nml-[Cys(3MeBn)- Aib-Leu-Ile-Cys]-Ttds-Lys(DOTA)-NH2 | 2335.163 | 2335.175 | 8.5 | D |
| GIP-291 | Ac-Asp-Trp-Trp-Pro-Glu-Leu-[Nmc(3MeBn)- Aib-Leu-Ile-Cys]-Ttds-Lys(DOTA)-NH2 | 2335.163 | 2335.170 | 8.8 | D |
| GIP-292 | Ac-Asp-Trp-Trp-Pro-Glu-Leu-[Cys(3MeBn)-Aib- Leu-Ile-Nmc]-Ttds-Lys(DOTA)-NH2 | 2335.163 | 2335.169 | 8.7 | D |
| GIP-293 | Ac-Asp-1Ni-Trp-Pro-Glu-Leu-[Cys(3MeBn)-Aib- Leu-Ile-Cys]-Ttds-Lys(DOTA)-NH2 | 2332.152 | 2332.162 | 9.8 | D |
| GIP-294 | Ac-Asp-Trp-2Ni-Pro-Glu-Leu-[Cys(3MeBn)-Aib- Leu-Ile-Cys]-Ttds-Lys(DOTA)-NH2 | 2332.152 | 2332.159 | 9.8 | D |
| GIP-295 | Ac-Asp-Trp-Trp-Pro-Glu-Cha-[Cys(3MeBn)-Aib- Leu-Ile-Cys]-Ttds-Lys(DOTA)-NH2 | 2361.179 | 2361.187 | 9.8 | D |
| GIP-296 | Ac-Asp-Trp-Trp-Pro-Glu-Aml-[Cys(3MeBn)-Aib- Leu-Ile-Cys]-Ttds-Lys(DOTA)-NH2 | 2335.163 | 2335.167 | 9.5 | D |
| GIP-297 | Ac-Asp-Nmw-Trp-Pro-Glu-Leu-[Cys(3MeBn)- Aib-Leu-Ile-Cys]-Ttds-Lys(DOTA)-NH2 | 2335.163 | 2335.170 | 9.4 | D |
| GIP-298 | Ac-1Ni-Pro-Glu-Leu-[Cys(3MeBn)-Aib-Leu-Ile- Cys]-Ttds-Lys(DOTA)-NH2 | 2031.046 | 2031.064 | 9.9 | D |
| GIP-299 | Nph1p-Pro-Glu-Leu-[Cys(3MeBn)-Aib-Leu-Ile- Cys]-Ttds-Lys(DOTA)-NH2 | 1974.025 | 1974.031 | 11.0 | D |
| GIP-300 | Ac-Asp-Trp-1Ni-Pro-Glu-leu-[Cys(3MeBn)-Ala- Leu-val-Cys]-Ttds-Lys(DOTA)-NH2 | 2304.121 | 2304.128 | 8.2 | D |
| GIP-301 | Ac-Trp-1Ni-Pro-Glu-Leu-[Cys(3MeBn)-Aib-Leu- Ile-Cys]-Ttds-Lys(DOTA)-NH2 | 2217.125 | 2217.133 | 10.2 | D |
| GIP-302 | Ac-Asp-Trp-Hyw-Pro-Glu-leu-[Cys(3MeBn)- Aib-Leu-Ile-Cys]-Ttds-Lys(DOTA)-NH2 | 2337.143 | 2337.145 | 8.2 | D |
| GIP-303 | Ac-Asp-7Nw-1Ni-Pro-Glu-leu-[Cys(3MeBn)- Aib-Leu-Ile-Cys]-Ttds-Lys(DOTA)-NH2 | 2333.148 | 2333.153 | 8.0 | D |
| GIP-304 | DOTA-Ttds-Trp-Asp-Pro-5Clw-leu-Glu- [Cys(3MeBn)-Leu-Ile-Aib-AET] | 2141.997 | 2142.001 | 9.1 | D |
| GIP-305 | DOTA-Ttds-leu-Asp-Pro-5Clw-Trp-Leu- [Cys(3MeBn)-Glu-Aib-Ile-AET] | 2141.997 | 2142.001 | 9.5 | D |
| GIP-306 | DOTA-Ttds-5Clw-Pro-Glu-leu-Asp-Trp- [Cys(3MeBn)-Ile-Leu-Aib-AET] | 2141.997 | 2142.003 | 8.7 | D |
| GIP-307 | H-Met-Tyr-asp-Trp-Trp-Pro-Glu-Leu- [Cys(3MeBn)-Ala-Leu-Ile-Cys]-NH2 | 1742.766 | 1742.778 | 9.5 | C |
| GIP-308 | H-Met-Tyr-Asp-trp-Trp-Pro-Glu-Leu- [Cys(3MeBn)-Ala-Leu-Ile-Cys]-NH2 | 1742.766 | 1742.771 | 10.1 | C |
| GIP-309 | H-Met-Tyr-Asp-Trp-Trp-Pro-Glu-Leu- [cys(3MeBn)-Ala-Leu-Ile-Cys]-NH2 | 1742.766 | 1742.770 | 9.7 | C |
| GIP-310 | H-Met-Tyr-Asp-Trp-Trp-Pro-Glu-Leu- [Cys(3MeBn)-Ala-Leu-Ile-cys]-NH2 | 1742.766 | 1742.770 | 9.6 | C |
| GIP-311 | H-Met-Tyr-Asp-Trp-Trp-Ala-Glu-Leu- [Cys(3MeBn)-Ala-Leu-Ile-Cys]-NH2 | 1716.750 | 1716.752 | 10.5 | C |
| GIP-312 | H-Met-Tyr-Asp-Trp-Trp-Pro-Glu-Leu- [Cys(3MeBn)-Ala-Ala-Ile-Cys]-NH2 | 1700.719 | 1700.723 | 9.2 | C |
| GIP-313 | H-Met-Tyr-Asp-Trp-Trp-Pro-Glu-Leu- [Cys(2MeBn)-Ala-Leu-Ile-Cys]-NH2 | 1742.766 | 1742.770 | 9.6 | C |
| GIP-314 | HO-Succinyl-Trp-Trp-Pro-Glu-Leu- [Cys(3MeBn)-ala-Leu-Ile-Cys]-NH2 | 1433.651 | 1433.654 | 10.4 | C |
| GIP-315 | Ac-Asp-Trp-1Ni-Pro-Glu-Hle-[Cys(3MeBn)-Aib- Leu-Ile-Cys]-NH2 | 1529.709 | 1529.712 | 12.0 | C |
| GIP-316 | Ac-Cya-Gly-5Clw-Hyp-Glu-leu-[Cys(2Lut)-Aib- Leu-Ile-AET] | 1419.543 | 1419.545 | 7.5 | C |
| GIP-317 | Ac-Cya-Trp-Trp-Pro-Glu-Leu-[Cys(3MeBn)-Ala- Leu-Ile-Cys]-NH2 | 1526.640 | 1526.649 | 11.3 | C |
| GIP-318 | HO-Succinyl-Trp-Trp-Pro-Glu-leu- [Cys(3MeBn)-ala-Leu-Ile-Cys]-NH2 | 1433.651 | 1433.657 | 10.2 | C |
| GIP-319 | HO-Succinyl-Trp-Trp-Hyp-Glu-Leu- [Cys(3MeBn)-Aib-Leu-Ile-Cys]-NH2 | 1463.662 | 1463.665 | 11.3 | C |
| GIP-320 | Ac-Asp-Trp-Mcf-Hyp-Glu-leu-[Cys(2Lut)-Thp- Leu-Ile-Cys]-NH2 | 1558.639 | 1558.653 | 9.4 | C |
| GIP-321 | Ac-Asp-Trp-1Qi-Hyp-Glu-leu-[Cys(2Lut)-Thp- Leu-Ile-Cys]-NH2 | 1575.689 | 1575.700 | 7.6 | C |
| GIP-322 | Ac-Asp-Trp-5Clw-Hyp-Glu-leu-[Cys(2Lut)-Thp- Leu-Ser-Cys]-NH2 | 1571.598 | 1571.637 | 8.0 | C |
| GIP-323 | Ac-Asp-Eaa-Eaa-Hyp-Glu-leu-[Cys(2Lut)-Thp- Leu-Ile-Cys]-NH2 | 1621.511 | 1621.535 | 10.6 | C |
| GIP-324 | Ac-Trp-Trp-Pro-Glu-Leu-[Cys(3MeBn)-Ala-Leu- Ile-Cys]-NH2 | 1375.646 | 1375.648 | 11.0 | D |
| GIP-325 | Ac-Trp-Pro-Glu-Leu-[Cys(3MeBn)-Ala-Leu-Ile- Cys]-NH2 | 1189.566 | 1189.569 | 10.7 | D |
| GIP-326 | Ac-Pro-Glu-Leu-[Cys(3MeBn)-Ala-Leu-Ile-Cys]- NH2 | 1003.487 | 1003.489 | 10.1 | D |
| GIP-327 | Ac-Glu-Leu-[Cys(3MeBn)-Ala-Leu-Ile-Cys]-NH₂ | 906.434 | 906.436 | 9.0 | D |
| GIP-328 | H-Met-Tyr-Asp-Trp-Trp-pro-Glu-Leu- [Cys(3MeBn)-Ala-Leu-Ile-Cys]-NH2 | 1742.766 | 1742.771 | 9.6 | D |
| GIP-329 | H-Met-Tyr-Asp-Trp-trp-Pro-Glu-Leu- [Cys(3MeBn)-Ala-Leu-Ile-Cys]-NH2 | 1742.766 | 1742.768 | 10.1 | D |
| GIP-330 | H-Met-Tyr-Asp-Trp-Trp-Pro-Glu-Leu- [Cys(3MeBn)-Ala-leu-Ile-Cys]-NH2 | 1742.766 | 1742.768 | 8.9 | D |
| GIP-331 | H-Met-Tyr-Asp-Trp-Trp-Pro-Glu-Leu- [Cys(4MeBn)-Ala-Leu-Ile-Cys]-NH2 | 1742.766 | 1742.770 | 9.8 | D |
| GIP-332 | H-Met-Tyr-Ala-Trp-Trp-Pro-Glu-Leu- [Cys(3MeBn)-Ala-Leu-Ile-Cys]-NH2 | 1698.776 | 1698.784 | 9.8 | D |
| GIP-333 | H-Met-Tyr-Asp-Ala-Trp-Pro-Glu-Leu- [Cys(3MeBn)-Ala-Leu-Ile-Cys]-NH₂ | 1627.724 | 1627.727 | 9.1 | D |
| GIP-334 | H-Met-Tyr-Asp-Trp-Ala-Pro-Glu-Leu- [Cys(3MeBn)-Ala-Leu-Ile-Cys]-NH2 | 1627.724 | 1627.729 | 8.9 | D |
| GIP-335 | H-Met-Tyr-Asp-Trp-Trp-Pro-Ala-Leu- [Cys(3MeBn)-Ala-Leu-Ile-Cys]-NH2 | 1684.760 | 1684.765 | 10.0 | D |
| GIP-336 | H-Met-Tyr-Asp-Trp-Trp-Pro-Glu-Ala- [Cys(3MeBn)-Ala-Leu-Ile-Cys]-NH2 | 1700.719 | 1700.723 | 8.9 | D |
| GIP-337 | H-Met-Tyr-Asp-Trp-Trp-Pro-Glu-Leu- [Cys(3MeBn)-Ala-Leu-Ala-Cys]-NH2 | 1700.719 | 1700.723 | 8.9 | D |
| GIP-338 | Ac-Asp-Trp-Trp-Pro-Glu-Leu-[Pen(3MeBn)-Aib- Leu-Ile-Cys]-NH2 | 1532.720 | 1532.722 | 10.9 | D |
| GIP-339 | Ac-Asp-Trp-Trp-Pro-Glu-Leu-[Cys(3MeBn)-Aib- Leu-Ile-Pen]-NH2 | 1532.720 | 1532.722 | 10.4 | D |
| GIP-340 | Ac-Asp-Trp-1Ni-Pro-Glu-Asn-[Cys(3MeBn)-Aib- Leu-Ile-Cys]-NH2 | 1516.652 | 1516.655 | 8.6 | D |
| GIP-341 | Ac-Asp-Trp-1Ni-4Dfp-Glu-Leu-[Cys(3MeBn)- Aib-Leu-Ile-Cys]-NH2 | 1551.674 | 1551.676 | 11.8 | D |
| GIP-342 | Ac-Asp-Trp-1Ni-Rni-Glu-Leu-[Cys(3MeBn)-Aib- Leu-Ile-Cys]-NH2 | 1529.709 | 1529.712 | 10.9 | D |
| GIP-343 | Ac-Asp-Trp-1Ni-Pro-Glu-Thi-[Cys(3MeBn)-Aib- Leu-Ile-Cys]-NH2 | 1555.634 | 1555.636 | 11.1 | D |
| GIP-344 | Ac-Asp-Trp-1Ni-Pro-Glu-Phe-[Cys(3MeBn)-Aib- Leu-Ile-Cys]-NH2 | 1549.677 | 1549.679 | 11.4 | D |
| GIP-345 | Ac-Asp-Trp-1Ni-Pro-Glu-Lys-[Cys(3MeBn)-Aib- Leu-Ile-Cys]-NH2 | 1530.704 | 1530.711 | 8.2 | D |
| GIP-346 | Ac-Asp-Trp-1Ni-Pro-Glu-Nle-[Cys(3MeBn)-Aib- Leu-Ile-Cys]-NH2 | 1515.693 | 1515.697 | 10.2 | D |
| GIP-347 | Ac-Asp-Trp-1Ni-Pro-Glu-Cit-[Cys(3MeBn)-Aib- Leu-Ile-Cys]-NH2 | 1559.694 | 1559.698 | 8.8 | D |
| GIP-348 | Ac-Asp-Trp-1Ni-Pro-Glu-Dap(Ac)-[Cys(3MeBn)- Aib-Leu-Ile-Cys]-NH2 | 1530.668 | 1530.670 | 9.1 | D |
| GIP-349 | Ac-Asp-Trp-Ocf-Hyp-Glu-leu-[Cys(2Lut)-Thp- Leu-Ile-Cys]-NH2 | 1558.639 | 1558.649 | 9.1 | D |
| GIP-350 | Ac-Asp-Trp-Pcf-Hyp-Glu-leu-[Cys(2Lut)-Thp- Leu-Ile-Cys]-NH2 | 1558.639 | 1558.653 | 9.5 | D |
| GIP-351 | Ac-Asp-Trp-Egm-Hyp-Glu-leu-[Cys(2Lut)-Thp- Leu-Ile-Cys]-NH2 | 1592.600 | 1592.614 | 9.8 | D |
| GIP-352 | Ac-Asp-Trp-Phe-Hyp-Glu-leu-[Cys(2Lut)-Thp- Leu-Ile-Cys]-NH2 | 1524.678 | 1524.684 | 9.0 | D |
| GIP-353 | Ac-Asp-Trp-5Clw-Hyp-Glu-leu-[Cys(2Lut)-Thp- Leu-Arg-Cys]-NH2 | 1640.667 | 1640.682 | 7.0 | D |
| GIP-354 | Ac-Asp-Trp-5Clw-Hyp-Glu-leu-[Cys(2Lut)-Thp- Leu-Glu-Cys]-NH2 | 1613.609 | 1613.646 | 7.6 | D |
| GIP-355 | Ac-Asp-Trp-5Clw-Hyp-Glu-leu-[Cys(2Lut)-Thp- Leu-Trp-Cys]-NH2 | 1670.645 | 1670.691 | 8.9 | D |
| GIP-356 | Ac-Asp-Eaa-Eaa-Oic-Glu-leu-[Cys(2Lut)-Thp- Leu-Ile-Cys]-NH2 | 1659.563 | 1659.620 | 11.3 | D |
| GIP-357 | Ac-Asp-Eaa-Eaa-Nmg-Glu-leu-[Cys(2Lut)-Thp- Leu-Ile-Cys]-NH2 | 1579.501 | 1579.555 | 10.5 | D |
| GIP-358 | DOTA-Ttds-Asp-Trp-5Clw-Pro-Glu-leu- [Dap(3MeBz)-Aib-Leu-Ile-AET] | 2139.015 | 2139.027 | 8.3 | A |
| GIP-359 | DOTA-Ttds-Asp-Trp-5Clw-Pro-Glu-leu- [Cys(3CbBn)-Aib-Leu-Ile-en] | 2139.015 | 2139.017 | 8.5 | B |
| GIP-360 | Ac-Asp-Trp-5Clw-Hyp-Glu-leu-[Cys(mli)-Thp- Leu-Ile-Cys]-NH2 | 1587.593 | 1587.601 | 9.2 | A |
| GIP-361 | Ac-Asp-Trp-5Clw-Hyp-Glu-leu-[Cys(mli)-Thp- Leu-Ile-Hcy]-NH2 | 1601.609 | 1601.631 | 9.7 | A |
| GIP-362 | Ac-Asp-Trp-5Clw-Hyp-Glu-leu-[Hcy(mli)-Thp- Leu-Ile-Cys]-NH2 | 1601.609 | 1601.627 | 10.8 | B |
| GIP-363 | Ac-Asp-Trp-5Clw-Hyp-Glu-leu-[Hcy-Thp-Leu- Ile-Hcy]-NH2 | 1520.624 | 1520.639 | 10.6 | B |
| GIP-364 | Ac-Asp-Trp-5Clw-Hyp-Glu-leu-[Cys-Thp-Leu- Ile-Cys]-NH2 | 1492.592 | 1492.604 | 9.1 | C |
| GIP-365 | DOTA-Ttds-Asp-Trp-5Clw-Pro-Glu-leu- [Dap(Mamb)-Aib-Leu-Ile-Gly] | 2136.034 | 2136.040 | 7.9 | B |
| GIP-366 | DOTA-Ttds-Asp-Trp-5Clw-Pro-Glu-leu- [Dap(Mamb)-Aib-Leu-Ile-gln] | 2207.071 | 2207.077 | 7.6 | B |
| GIP-367 | DOTA-Ttds-Asp-Trp-5Clw-Pro-Glu-leu- [Dap(Mamb)-Aib-Leu-Ile-Gln] | 2207.071 | 2207.075 | 7.4 | D |
| GIP-368 | H-Met-Tyr-Asp-Trp-Trp-Pro-Glu-Leu-Cys(Bzl)- Ala-Leu-Ile-Smc-NH₂ | 1744.782 | 1744.790 | 9.6 | C |
| GIP-369 | H-Met-Tyr-Asp-Trp-Trp-Pro-Glu-Leu-Smc-Ala- Leu-Ile-Cys(Bzl)-NH2 | 1744.782 | 1744.789 | 9.8 | D |

| | | | | | |
|---|---|---|---|---|---|
| ^{∗}HPLC analyses were performed by HPLC/ELSD | | | | | |

### Example 28: Surface Plasmon Resonance (SPR) based Binding Assay

Surface plasmon resonance (SPR) studies were performed using a Biacore^{™} T200 SPR system. Briefly, polarized light is directed towards a gold-labeled sensor surface, and minimum intensity reflected light is detected. The angle of reflected light changes as molecules bind and dissociate.

Biotinylate extracellular of the human GIP receptor (bioGIPR, Proteros biostructures GmbH, lot#02/2b) was captured on a biotin capture chip (Biotin CAPture Kit, Series S, GE Healthcare, Cat#28-9202-34). bioGIPR was diluted in Running Buffer (HBST from Xantec Analytics; 150 nM NaCl, 0.05% Tween20, 0.1% DMSO) to a final concentration 100 nM and than flushed over the Fc-capture chip to immobilized ~3500 RUs.

Stock solutions of test compounds were prepared by dissolving each compound in DMSO. DMSO stock solution were diluted 1:1000 in Running Buffer without DMSO. Further sequencial dilutions were made with Running Buffer containing 0.1% DMSO. SPR binding analyses were performed in Single Cycle Kinetic (SCK) mode at 25°C. Flow cell without immobilized bioGIPR served as reference flowcell. After each SCK run, bioGIRP and the Biotin Capture Reagent was removed with Regeneration Solution (6 M Guanidium, 0.75 M NaOH).

In between every three SCK measurements, a blank run with Running Buffer instead of test compound was included to correct for baseline drifts (double blanking methode).

Table 9 describes the protocol steps for bioGIPR capturing and assessment of the binding kinetics.

**Table 9: SPR protocol steps with bioGIPR.**

| **Step** | **Injected solution** | **Contact time** | **Flow rate** |
|---|---|---|---|
| Startup cycle (3x): Washing & surface regeneration | Running Buffer 6 M Guanidin-HCl | 60 s 5 s | 30 µL/min |
| Immobilize Biotin Capture Reagent (Streptavidin-oligo fusion) | Biotin Capture Reagent | 300 s | 2 µL/min |
| Wash | Running Buffer | 60 s | 30 µL/min |
| Capture bioGIPR | bioGIPR (100 nM) | 300 s | 10 µL/min |
| 1. Binding kinetics of test compound | Dilution no. 5 (0.78 nM) | 120 s | 30 µL/min |
| 2. Binding kinetics of test compound | Dilution no. 4 (3.125 nM) | 120 s | 30 µL/min |
| 3. Binding kinetics of test compound | Dilution no. 3 (12.5 nM) | 120 s | 30 µL/min |
| 4. Binding kinetics of test compound | Dilution no. 2 (50 nM) | 120 s | 30 µL/min |
| 5. Binding kinetics of test compound | Dilution no. 1 (100 nM) | 120 s | 30 µL/min |
| Dissociation cycle | Running Buffer | 2400 s | 30 µL/min |
| Regeneration (2x) | Regeneration solution | 120 s | 5 µL/min |

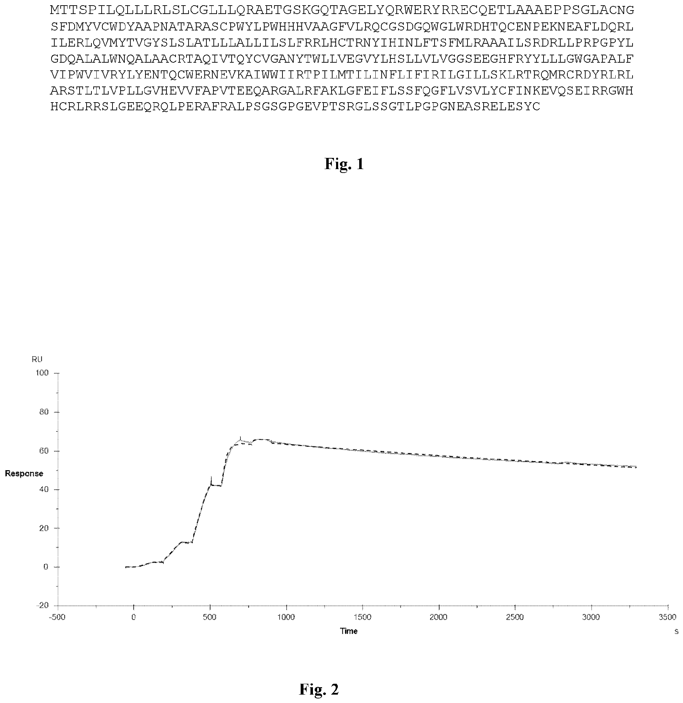

For each test compound, SPR raw data in the form of resonance units (RU) were plotted as sensorgrams using the Biacore^{™} T200 control software. The signal from the blank sensorgram was subtracted from that of the test compound sensorgram (blank corrected). The blank corrected sensorgram was corrected for baseline drift by subtracting the sensorgram of a SCK run without the test compound (running buffer only). The association rate (kₒₙ), dissociation rate (k_{off}), dissociation constant (K_{D}), and t_{1/2} were calculated from blank corrected SPR data using the 1:1 Langmuir binding model from the Biacore^{™} T200 evaluation software. Raw data and fit results were imported as text files in IDBS. The pK_{D} value (negative decadic logarithm of dissociation constant) was calculated in the IDBS excel template. Figure 2 shows a representative sensorgram for compound PSM-01.

The results of this assay for a selection of compounds according to the present invention are presented in Table 10. pK_{D} category A stands for pK_{D} values > 9.0, category B for pK_{D} values between 8.1 and 9.0, category C for pK_{D} values between 7.1 and 8.0, and category D for pK_{D} values ≤ 7.0. t_{1/2} category A stands for t_{1/2} values > 15 min, category B for t_{1/2} between 15 min and 5.1 min, category C for t_{1/2} between 5 min and 2.1 min, and category D for t_{1/2} < 2.0 min.

The results of this assay for a selection of compounds according to the present invention are presented in Table 10.

**Table 10: Compound ID, pK_{D}-category and t_{1/2}-category.**

| **Compound ID** | **pK_{D} Category** | **t_{1/2} Category** | **Compound ID** | **pK_{D} Category** | **t_{1/2} Category** |
|---|---|---|---|---|---|
| **GIP-001** | A | A | **GIP-103** | A | A |
| **GIP-002** | A | A | **GIP-106** | B | A |
| **GIP-004** | A | A | **GIP-107** | A | A |
| **GIP-005** | A | A | **GIP-108** | A | A |
| **GIP-006** | A | A | **GIP-109** | A | A |
| **GIP-007** | A | A | **GIP-110** | A | A |
| **GIP-008** | A | A | **GIP-111** | B | A |
| **GIP-010** | A | A | **GIP-112** | A | A |
| **GIP-011** | A | A | **GIP-113** | A | A |
| **GIP-021** | A | A | **GIP-114** | A | A |
| **GIP-022** | A | A | **GIP-117** | A | A |
| **GIP-032** | A | A | **GIP-118** | A | A |
| **GIP-046** | B | D | **GIP-119** | A | A |
| **GIP-048** | B | D | **GIP-121** | A | A |
| **GIP-049** | B | D | **GIP-142** | B | A |
| **GIP-065** | A | A | **GIP-146** | A | A |
| **GIP-066** | A | A | **GIP-151** | A | A |
| **GIP-067** | A | A | **GIP-153** | A | A |
| **GIP-068** | A | A | **GIP-155** | A | A |
| **GIP-069** | A | A | **GIP-167** | A | A |
| **GIP-072** | A | A | **GIP-169** | A | A |
| **GIP-077** | A | A | **GIP-179** | A | A |
| **GIP-079** | A | A | **GIP-188** | A | A |
| **GIP-095** | A | A | **GIP-194** | A | A |
| **GIP-096** | A | A | **GIP-197** | A | A |
| **GIP-097** | A | A | **GIP-359** | B | C |

### Example 29: Plasma stability assay

In order to determine the stability of selected compounds of the invention in human and mouse plasma, a plasma stability assay was carried out. Such plasma stability assay measures degradation of compounds of the present invention in blood plasma. This is an important characteristic of a compound as compounds, with the exception of pro-drugs, which rapidly degrade in plasma, generally show poor *in vivo* efficacy. The results show that those compounds are highly stable in human and mouse plasma. The stability is sufficient for the diagnostic, therapeutic and theragnostic use of these compounds according to the present invention.

The plasma stability samples were prepared by spiking 50 µL plasma aliquots (all K2EDTA) with 1 µL of a 0.5 mM compound stock solution in DMSO. After vortexing the samples were incubated in a Thermomixer at 37°C for 0, 4 and 24 hours. After incubation the samples were stored on ice until further treatment. All samples were prepared in duplicates.

A suitable internal standard was added to each sample (1 µL of a 0.5 mM stock solution in DMSO). Protein precipitation was performed by addition of 250 µL of acetonitrile containing 1% trifluoroacetic acid. After incubation at room temperature for 30 min the precipitate was separated by centrifugation and 150 µL of the supernatant was diluted with 150 µL of 1% aqueous formic acid.

The determination of the analyte in the clean sample solutions was performed on an Agilent 1290 UHPLC system coupled to an Agilent 6530 Q-TOF mass spectrometer. The chromatographic separation was carried out on a Phenomenex BioZen XB-C18 HPLC column (50 × 2 mm, 1.7 µm particle size) with gradient elution using a mixture of 0.1% formic acid in water as eluent A and acetonitrile as eluent B (2% B to 41% in 7 min, 800 µL/min, 40°C). Mass spectrometric detection was performed in positive ion ESI mode by scanning the mass range from m/z 50 to 3000 with a sampling rate of 2 / sec.

From the mass spectrometric raw data, the ion currents for the double or triple charged monoisotopic signal was extracted for both, the compound and the internal standard.

Quantitation was performed by external matrix calibration with internal standard using the integrated analyte signals.

Additionally, recovery was determined by spiking a pure plasma sample that only contained the internal standard after treatment with a certain amount of the compound.

Carry-over was evaluated by analysis of a blank sample (20% acetonitrile) after the highest calibration sample.

The results of this assay performed on some of the compounds according to the present invention are given in the following Table 11. The result is stated as "% intact compound remaining after 4 h or 24 h" and means that from the amount of material at the start of the experiment the stated percentage is detected as unchanged material at the end of the experiment by LC-MS quantification. Stability category A stands for > 80% intact after 24 hours, category B stands for > 80% intact after 4 hours, category C stands for ≥ 50% intact after 4 hours and category D stands for < 50% intact after 4 hours. Compounds with more than 50% intact after at least 4 h they are considered as stable enough for diagnostic and therapeutic applications.

**Table 11: Results of the plasma stability assay**

| **Compound** | **Human plasma stability category** | **Mouse plasma stability category** |
|---|---|---|
| GIP-001 | A | A |
| GIP-005 | n.d. | A |
| GIP-126 | n.d. | B |
| GIP-127 | n.d. | B |
| GIP-128 | n.d. | B |
| GIP-129 | n.d. | B |
| GIP-240 | B | B |
| GIP-241 | B | C |
| GIP-242 | B | C |
| GIP-244 | B | B |
| GIP-245 | B | D |
| GIP-246 | B | B |
| GIP-247 | C | D |
| GIP-248 | B | D |
| GIP-270 | n.d. | B |

### Example 30: Proteolytic stability assay against NEP

Peptides are often sensitive to proteolytic cleavage in the blood (Werle et al., Amino Acids, 2006, 30, 351-367). If peptides degrade rapidly, in vivo elimination could be dominated by metabolism. Since metabolites often demonstrate poor binding to the target, performance of the compound during radiopharmaceutical use (diagnostic or therapeutic) can be significantly decreased. Therefore, evaluating the stability of the compound against proteases in the early stages of compound development is essential.

Two classes of proteases and peptidases can be found in the blood circulation: soluble and membrane-bound proteases. The conduction of a plasma stability study only covers the impact of the soluble fraction on compound stability, since all membrane-bound proteases are separated during blood collection (proteins expressed on the surface of blood vessels) or during plasma generation (proteins expressed on blood cells). Thus, the result of a plasma stability assay is not always predictive for in vivo behavior.

Membrane-bound peptidases are mainly responsible for the activation and deactivation of bioactive peptides, such as hormones, cytokines, and neurohormones (Antczak et al., Bioessays, 2001, 23, 251-260). They activate hormones by cleavage of a pre-hormone and deactivate them through another cleavage step. Activation is usually very specific, as well as the activation peptidases, including ACE, ECE, and DPPIV. With a specific activation, signaling remains specific even if deactivation is more unspecific. Thus, several peptidases that are mainly responsible for hormone deactivation reveal a broad substrate pattern (e.g. neutral endopeptidase, meprines). It is not possible to assess all peptidases during early compound development. Therefore, more importance should be focused on those peptidases with broad substrate patterns, since it is more likely that the peptide sequence fits into this pattern.

NEP (neutral endopeptidase, EC 3.4.24.11, CD10, CALLA, endopeptidase 24.11, enkephalinase, neprilysin, membrane metallopeptidase A) is a membrane-bound metallopeptidase. It is expressed on neutrophils and highly active in blood (Antczak et al., Bioessays, 2001, 23, 251-260). In addition, the cleavage pattern of NEP is very broad, covering many different peptide hormones, with more than 50 different natural substrates already described (Bayes-Genis et al., Journal of the American College of Cardiology, 2016, 68, 639-653). NEP preferably cleaves amide bonds between a hydrophilic and hydrophobic amino acid (preferably leucine or phenylalanine), even in small cyclic peptides such as CNP. Plamboeck et al. (2005) showed a significant impact of NEP caused cleavage on the pharmacokinetic behavior of peptides (Plamboeck et al., Diabetologia, 2005, 48, 1882-1890).

To assess the stability of compounds against NEP, the compounds were subjected to an in vitro assay based on the protocol described by Edelson et al. (Edelson et al., Pulmonary pharmacology & therapeutics, 2013, 26, 229-238).

Recombinant soluble human NEP (BioTechne, Wiesbaden, Germany) at a concentration of 100 ng/mL was mixed with the compound (10 µM) and a stable internal standard (10 µM) and incubated at 37°C. After several time points, samples were taken and analyzed with LC-MS.

The determination of the analyte in the clean sample solutions was performed on an Agilent 1290 UHPLC system coupled to an Agilent 6530 Q-TOF mass spectrometer. The chromatographic separation was carried out on a Phenomenex BioZen XB-C18 HPLC column (50 × 2 mm, 1.7 µm particle size) with gradient elution using a mixture of 0.1% formic acid in water as eluent A and acetonitrile as eluent B (2% B to 41% in 7 min, 800 µL/min, 40°C). Mass spectrometric detection was performed in positive ion ESI mode by scanning the mass range from m/z 50 to 3000 with a sampling rate of 2 / sec.

From the mass spectrometric raw data the ion currents for the double or triple charged monoisotopic signal was extracted for both, the compound and the internal standard.

Quantitation was performed by external matrix calibration with internal standard using the integrated analyte signals.

The activity of NEP was examined using a commercially available chromogenic substrate of NEP.

The results of this assay performed on some of the compounds according to the present invention are given in the following Table 12. The result is stated as "% intact compound remaining after 24 h incubation" and means that from the amount of material at the start of the experiment the stated percentage is detected as unchanged material at the end of the experiment by LC-MS quantification. Stability category A stands for > 80% intact after 24 hours, category B stands for > 50% intact after 24 hours, category C stands for ≥ 50% intact after 4 hours and category D stands for < 50% intact after 4 hours. Compounds with more than 50% intact after at least 24 h they are considered as stable enough for diagnostic and therapeutic applications.

Since all compounds are more than 50% intact after 24 h they are considered as stable enough for diagnostic and therapeutic applications.

**Table 12: Results of the NEP stability assay**

| **Compound** | **NEP stability category** |
|---|---|
| GIP-001 | A |
| GIP-003 | A |
| GIP-039 | A |
| GIP-040 | A |
| GIP-126 | A |
| GIP-127 | A |
| GIP-128 | A |
| GIP-244 | A |
| GIP-270 | A |

### Example 31: ¹¹¹In- and ¹⁷⁷Lu-labeling of selected compounds

In order to serve as a diagnostically, therapeutically, or theragnostically active agent, a compound needs to be labeled with a radioactive isotope. The labeling procedure needs to be appropriate to ensure a high radiochemical yield and purity of the radiolabeled compound of the invention. This example shows that the compounds of the present invention are appropriate for radiolabeling and can be labeled in high radiochemical yield and purity.

30-105 MBq of ¹¹¹InCl₃ (in 0.02 M HCl) were mixed with 1 nmol of compound (200 µM stock solution in 0.1 M HEPES pH 7) per 30 MBq and buffer (A: 1 M sodium acetate buffer pH 5, containing 20 mg/mL gentisic acid, B: 1 M sodium acetate / ascorbic acid buffer pH 5 containing 25 mg/mL methionine or C: 1 M sodium acetate buffer pH 5 containing 25 mg/mL methionine) at a final buffer concentration of 0.1 M. The mixture was heated to 80 °C for 25 min. After cooling down, DTPA and TWEEN-20 were added at a final concentration of 0.2 mM and 0.1%, respectively (Formulation A). In some instances, 20 µL of a 200 mg/mL ascorbic acid solution per 100 µL reaction mixture was additionally added at the end of synthesis (Formulation B).

2.0 ± 0.1 GBq ¹⁷⁷LuCl₃ (in 0.04 M HCl) were mixed with 1 nmol of compound (200 µM stock solution in 0.1 M HEPES pH 7) per 60 - 90 MBq and buffer (1 M sodium acetate / ascorbic acid buffer pH 5 containing 25 mg/mL methionine) at a final buffer concentration of ~0.4 M. The mixture was heated for 20 min at 90 °C. After cooling down, DTPA and TWEEN-20 were added at a final concentration of 0.2 mM and 0.1%, respectively (Formulation A). In some instances, 9 volumes of a 100 mg/mL ascorbic acid solution containing 5 mg/mL methionine and 0.8 mM DTPA were added instead (Formulation C).

Radiochemical purity was analyzed by HPLC. 5 µL of diluted labeling solution was analyzed with a Poroshell SB-C18 2.7 µm (Agilent). Eluent A: H₂O, 0.1 % TFA, eluent B: MeCN; gradient from 5 % B to 70 % B within 15 min, flow rate 0.5 mL/min; detector: NaI (Raytest), DAD 230 nm. The peak eluting with the dead volume represents free radionuclide, the peak eluting with the peptide-specific retention time as determined with an unlabeled sample represents radiolabeled compound.

Radiochemical purity was ≥ 92 % at end of synthesis. Exemplary radiochemical purities for selected ¹¹¹In-labeled compounds are shown in Table 13. ¹⁷⁷Lu-labeled compounds maintained a radiochemical purity of ≥ 85 % up 6 days post synthesis (Table 13).

**Table 13: Radiochemical purity of ¹¹¹In-labeled compounds as measured by HPLC.**

| | **Labeling buffer** | **Formulation** | **HPLC retention time [min]** | **HPLC Area% at end of synthesis** |
|---|---|---|---|---|
| ¹¹¹In-GIP-001 | B | A | 8.8 | > 97.0 |
| ¹¹¹In-GIP-002 | B | A | 9.1 | > 97.0 |
| ¹¹¹In-GIP-003 | B | A | 8.6 | 99.0 |
| ¹¹¹In-GIP-003 | C | B | 8.6 | 96.6 |
| ¹¹¹In-GIP-004 | A | A | 8.9 | 97.9 |
| ¹¹¹In-GIP-005 | B | A | 8.8 | 98.2 |
| ¹¹¹In-GIP-005 | C | B | 8.8 | 98.3 |
| ¹¹¹In-GIP-006 | B | A | 8.4 | 99.3 |
| ¹¹¹In-GIP-007 | B | A | 8.2 | 100.0 |
| ¹¹¹In-GIP-008 | C | B | 8.3 | > 99.0 |
| ¹¹¹In-GIP-009 | C | B | 8.7 | 97.4 |
| ¹¹¹In-GIP-010 | B | A | 9.0 | 98.3 |
| ¹¹¹In-GIP-011 | C | B | 8.8 | 95.5 |
| ¹¹¹In-GIP-012 | B | A | 8.8 | 97.6 |
| ¹¹¹In-GIP-015 | C | B | 8.9 | 98.2 |
| ¹¹¹In-GIP-016 | C | B | 8.8 | 98.4 |
| ¹¹¹In-GIP-017 | C | B | 8.9 | 97.8 |
| ¹¹¹In-GIP-018 | C | B | 8.6 | 98.9 |
| ¹¹¹In-GIP-019 | C | B | 8.9 | 98.8 |
| ¹¹¹In-GIP-020 | C | B | 8.8 | 98.2 |
| ¹¹¹In-GIP-021 | B | A | 9.8 | 96.5 |
| ¹¹¹In-GIP-022 | B | A | 11.2 | 98.2 |
| ¹¹¹In-GIP-023 | C | B | 9.4 | 98.8 |
| ¹¹¹In-GIP-032 | A | A | 9.5 | 98.6 |
| ¹¹¹In-GIP-077 | A | A | 11.6 | > 97.0 |
| ¹¹¹In-GIP-079 | A | A | 11.2 | 98.4 |
| ¹¹¹In-GIP-080 | A | A | 11.7 | 92.9 |
| ¹¹¹In-GIP-082 | A | A | 11.3 | 99.5 |
| ¹¹¹In-GIP-088 | A | A | 11.3 | 98.0 |
| ¹¹¹In-GIP-089 | A | A | 11.2 | 98.5 |
| ¹¹¹In-GIP-093 | A | A | 10.7 | 97.4 |
| ¹¹¹In-GIP-094 | A | | 11.3 | > 98.2 |
| ¹¹¹In-GIP-095 | A | A | 10.8 | 97.5 |
| ¹¹¹In-GIP-096 | A | A | 10.9 | 97.1 |
| ¹¹¹In-GIP-097 | A | A | 11.6 | 97.5 |
| ¹¹¹In-GIP-103 | A | A | 10.0 | 96.1 |
| ¹¹¹In-GIP-106 | A | A | 9.6 | > 98.3 |
| ¹¹¹In-GIP-109 | A | A | 10.0 | > 98.3 |
| ¹¹¹In-GIP-112 | A | A | 9.7 | 98.5 |
| ¹¹¹In-GIP-142 | B | A | 11.2 | 99.3 |
| ¹¹¹In-GIP-359 | A | A | 11.7 | 96.2 |

Radiochemical purity of ¹⁷⁷Lu-labeled compounds as measured by HPLC.

| | **Formulation** | **HPLC retention time [min]** | **HPLC Area% at end of synthesis** | **HPLC Area% appr. 6 d post end of synthesis** |
|---|---|---|---|---|
| ¹⁷⁷Lu-GIP-001 | C | 8.8 | 97.4 | 94.8 |
| ¹⁷⁷Lu-GIP-002 | A | 9.1 | 97.7 | 85.0 |

### Example 32: Imaging studies

Radioactively labeled compounds can be detected by imaging methods such as SPECT and PET. Furthermore, the data acquired by such techniques can be confirmed by direct measurement of radioactivity contained in the individual organs prepared from an animal injected with a radioactively labeled compound of the invention. Thus, the biodistribution (the measurement of radioactivity in individual organs) of a radioactively labeled compound can be determined and analyzed. This example shows that the compounds of the present invention show a biodistribution appropriate for diagnostic imaging of tumors.

All animal experiments were conducted in compliance with the German animal protection laws. Male SCID beige mice (6- to 8-week-old, Charles River Laboratories, France) were inoculated with 5 × 10⁶ target-positive CHO-K1-hGIPR (GenScript Biotech, #M00486) and target negative HT-29 cells (DSMZ, Germany) subcutaneously on opposite shoulders in the same mouse. When tumors reached a size between 150 mm³ and 500 mm³ mice received ~30 MBq ¹¹¹In-labeled compounds of the invention (diluted to 100 µL with PBS) administered intravenously via the tail vein. Images were obtained on a NanoSPECT/CT system (Mediso Medical Imaging Systems, Budapest, Hungary) using exemplarily the following acquisition and reconstruction parameters (Table 14).

**Table 14: Acquisition and reconstruction parameters of NanoSPECT/CT imaging**

| **Acquisition parameters SPECT** | |
|---|---|
| System | NanoSPECT/CT^{™} |
| Scan range | whole body, 3-bed holder (mouse hotel) |
| Time per projection | 60s |
| Aperture model, pinhole diameter | Aperture #2, 1,5 mm |

| **Reconstruction parameters** | |
|---|---|
| Method | HiSPECT (Scivis), iterative reconstruction |
| Smoothing | 35% |
| Iterations | 9 |
| Voxel size | 0.15 mm × 0.15 mm × 0.15 mm |

| **Acquisition parameters CT** | |
|---|---|
| System | NanoSPECT/CT ^{™} |
| Scan range | whole body, 3-bed holder (mouse hotel) |
| Scan duration | 7 min |
| Tube voltage | 45 kVp |
| Exposure time | 500 ms |
| Number of projections | 240 |

Imaging data were saved as DICOM files and analyzed using VivoQuant^{™} software (Invicro, Boston, USA). Results are expressed as a percentage of injected dose per gram of tissue (%ID/g). Two animals were treated with each compound.

Table 15 states the tumor and kidney uptake after 1 h p.i. to 24 h p.i. calculated as area under the curve (AUC) (GraphPad Prism). Tumor uptake category A stands for AUC > 250 %ID/g, category B for AUC between 250 %ID/g and 150 %ID/g, and category C for AUC < 150 %ID/g.

Kidney uptake category A stands for AUC < 150 %ID/g, category B for AUC between 150 %ID/g and 200 %ID/g, and category C for AUC > 200 %ID/g.

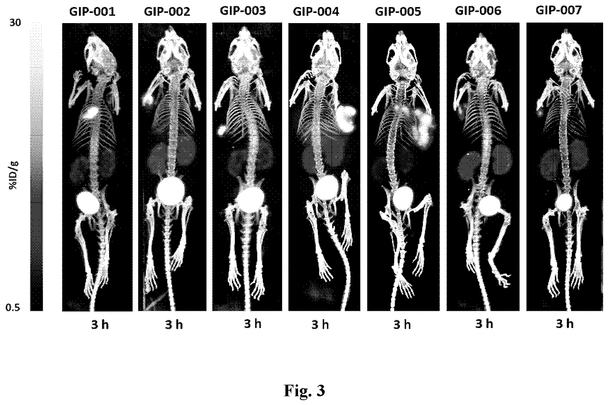

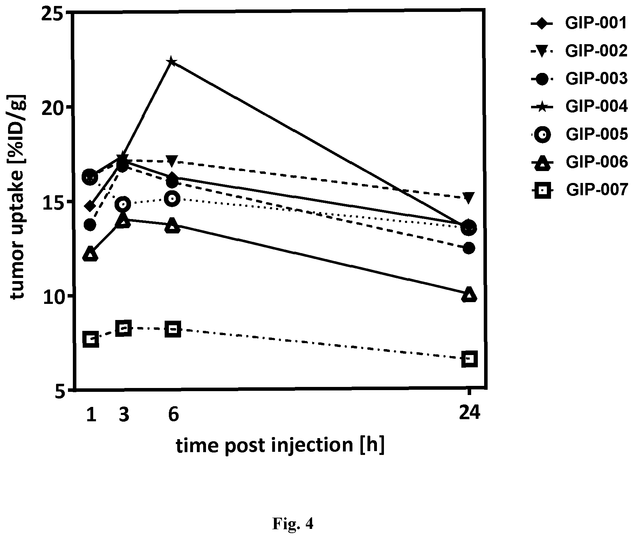

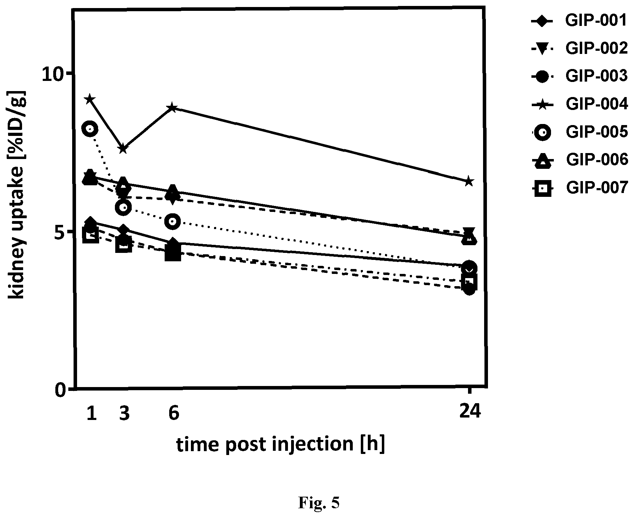

The results of the imaging studies for selected compounds are shown in Figs. 3, 4 and 5.

**Table 15**

| **Compound ID** | **AUC_{1-24 h} Tumor** | **AUC_{1-24 h} Kidneys** |
|---|---|---|
| ¹¹¹In-GIP-001 | A | A |
| ¹¹¹In-GIP-002 | A | A |
| ¹¹¹In-GIP-003 | A | A |
| ¹¹¹In-GIP-004 | A | B |
| ¹¹¹In-GIP-005 | A | B |
| ¹¹¹In-GIP-006 | A | A |
| ¹¹¹In-GIP-007 | B | A |
| ¹¹¹In-GIP-008 | A | A |
| ¹¹¹In-GIP-009 | B | A |
| ¹¹¹In-GIP-010 | A | A |
| ¹¹¹In-GIP-011 | A | A |
| ¹¹¹In-GIP-012 | B | A |
| ¹¹¹In-GIP-013 | B | A |
| ¹¹¹In-GIP-014 | B | A |
| ¹¹¹In-GIP-015 | A | A |
| ¹¹¹In-GIP-016 | A | A |
| ¹¹¹In-GIP-017 | B | B |
| ¹¹¹In-GIP-018 | B | C |
| ¹¹¹In-GIP-019 | A | B |
| ¹¹¹In-GIP-020 | B | C |
| ¹¹¹In-GIP-021 | A | C |
| ¹¹¹In-GIP-022 | A | A |
| ¹¹¹In-GIP-023 | A | C |
| ¹¹¹In-GIP-077 | A | A |
| ¹¹¹In-GIP-079 | A | B |
| ¹¹¹In-GIP-080 | B | A |
| ¹¹¹In-GIP-109 | A | C |
| ¹¹¹In-GIP-142 | C | A |

### Example 33: Radioligand binding assay

In order to assess the affinity of ¹¹¹In-labeled compounds to the GIPR receptor of different species, a radioligand binding assay was established.

CHO-K1 cells (GenScript Biotech, #M00486) were stably transfected with the human GIPR. CHO-K1-hGIPR cells were cultured in Ham's F12 medium (Sigma-Aldrich, #N4888) including 10% fetal calf serum (FCS), 2 mM _{L}-glutamine, 100 U/mL penicillin, 100 µg/mL streptomycin, 200 µg/mL Zeocin and 100 µg/mL HygromycinB. To evaluate the species cross-reactivity, CHO-VGT cells were transfected with the human, dog, rat and mouse GIPR (Inscreenex GmbH), which were cultured in DMEM/Ham's F12 Medium 1:1 (Biochrom, #F485) including 5 % fetal calf serum (FCS), 2 mM _{L}-glutamine, 100 U/mL penicillin and 100 µg/mL streptomycin. Cells were detached with Accutase (Biolegend, #BLD-423201) and counted using a particle counter (CASY Model TT; Schärfe Systems). Cell concentrations were adjusted to 3 × 10⁵ mL⁻¹, and 1.000 µL of the suspension per well were dispensed into flat-clear-bottom 24 well plates.

Approximately 24 hours after re-seeding, the medium was aspirated and the cells were washed once with 700 µL assay medium (Ham's medium with 20 mM HEPES). To determine total binding, 700 µL of assay medium and 100 µL of the radioligand dilutions were added to the wells in triplicates. To determine non-specific binding, 600 µL of binding medium, 100 µL of the radioligand dilutions and 100 µL of a blocking solution containing 8 µM non-labeled compound were added to the wells in triplicates.

For determination of the equilibrium time, cells were incubated with 0.1 nM and 0.2 nM radiolabeled compound for 1 h, 3 h, 6 h, and 8 h at 37°C under standard cell culture conditions (5% CO₂). For determination of the dissociation constant (K_{D}) cells were incubated with increasing concentrations of ¹¹¹In-labeled compound radiolabeled compound for 6 h at 37°C under standard cell culture conditions (5% CO₂).

At the end of incubation time, cells were washed and subsequently harvested in 300 µL RIPA buffer containing PIC. Radioactivity of the cell lysate of each well was counted using a gamma counter (Perkin Elmer). An aliquot of each radioligand dilution was included in the gamma counter measurements to allow for determination of their actual concentrations via their known specific activity.

The results of the radioligand binding assay are shown in Table 16. pK_{D} category A stands for pK_{D} values > 9.5, category B for pK_{D} between 9.0 and 9.5 and category C for pK_{D} < 9.0.

**Table 16 Results of the radioligand binding assay**

| **Compound ID** | **Species** | **Category** |
|---|---|---|
| GIP-001 | Human GIPR | A |
| GIP-001 | Dog GIPR | A |
| GIP-001 | Rat GIPR | B |
| GIP-001 | Mouse GIPR | C |
| GIP-002 | Human GIPR | A |

### Example 34: cAMP Assay

In order to determine the inhibitory or stimulatory activity of compounds according to the present invention of GIPR-expressing cells, a cAMP activity assay was established.

HEK-GIPR cells were generated by InSCREENeX GmbH (Germany) using the targeting vector pTAR to insert the minimal CMV promoter upstream of the human GIPR cDNA by recombinase-mediated cassette exchange into the pre-tagged HEK293 cell line.

HEK-GIPR cells were cultured in RPMI-1640 medium (Sigma-Aldrich, #R0883) including 10% fetal calf serum (FCS), 8 mM L-glutamine, 100 U/mL penicillin, 100 µg/mL streptomycin and 1 mg/mL Geneticin. Cells were detached with Accutase (Biolegend, #BLD-423201) and diluted in culture medium to a final concentration of 100,000 cells per mL. 100 µL of the cell suspension were transferred to white poly-L-lysine-coated 96-well plates with optical bottom (Nunc, #165306) and incubated overnight in a humidified atmosphere containing 5% CO₂ at 37°C.

For EC₅₀ determination, cells were incubated in 20 µL induction buffer (RPMI-1640 growth medium including 100 µM Ro 20-1724 (Sigma-Aldrich, #B8279) and 500 µM IBMX (Sigma-Aldrich, #17018)) containing various concentrations of compound at 37°C for 30 min.

For IC₅₀ determination, cells were incubated in 20 µL induction buffer (RPMI-1640 growth medium including 100 µM Ro 20-1724 (Sigma-Aldrich, #B8279) and 500 µM IBMX (Sigma-Aldrich, #17018)) containing various concentrations of antagonistic compound and 3 pM GIP(1-30) agonist (H-YAEGTFISDYSIAMDKIHQQDFVNWLLAQK-NH₂; 3BP in-house synthesis) at 37°C for 30 min.

For cAMP detection, the cAMP-Glo^{™} Assay (Promega, #V1501) was employed. Following the incubation period, 20 µL cAMP Lysis Buffer (Promega, #V1501) was added to each well and the plate was shaken at 1,000 RPM for 15 min at room temperature. 40 µL of the cAMP detection solution (Promega, #V1501) was added to each well and the plate was shaken at 850 RPM for 20 min at room temperature. 80 µL of the Kinase-Glo-Reagent Solution (Promega, #V1501) was added to each well and mixed at 850 RPM for 30 sec. After 10 min of incubation, luminescence was read using a SpectraMax M5 Plate Reader (Molecular Devices).

Relative luminescence values (RLU) were plotted against peptide concentration and four parameter logistic (4PL) curve fitting and calculations of EC₅₀/pEC₅₀ and IC₅₀/pIC₅₀, respectively, were performed using ActivityBase software.

The result of the EC₅₀ assay is shown in Table 17 and the combined results of the EC₅₀ and IC₅₀ assays in Table 18.

**Table 17**

| **Compound ID/ description** | **Sequence** | **Mean pEC₅₀ ± SD** |
|---|---|---|
| GIP(1-30) | H-Tyr-Ala-Glu-Gly-Thr-Phe-Ile-Ser-Asp-Tyr-Ser-Ile-Ala-Met-Asp-Lys-Ile-His-Gln-Gln-Asp-Phe-Val-Asn-Trp-Leu-Leu-Ala-Gln-Lys-NH₂ | 11.8 ± 0.1 |
| GIP(1-30) C-ter. DOTA | H-Tyr-Ala-Glu-Gly-Thr-Phe-Ile-Ser-Asp-Tyr-Ser-Ile-Ala-Met-Asp-Lys-Ile-His-Gln-Gln-Asp-Phe-Val-Asn-Trp-Leu-Leu-Ala-Gln-Lys(DOTA-Ahx)-NH₂ | 12.1 ± 0.1 |
| GIP(1-30) C-ter. ^{nat}InDOTA | H-Tyr-Ala-Glu-Gly-Thr-Phe-Ile-Ser-Asp-Tyr-Ser-Ile-Ala-Met-Asp-Lys-Ile-His-Gln-Gln-Asp-Phe-Val-Asn-Trp-Leu-Leu-Ala-Gln-Lys(^{nat}InDOTA-Ahx)-NH2 | 12.0 ± 0.1 |

**Table 18**

| **Compound ID** | **pIC₅₀ Category** | **pEC₅₀ Category** |
|---|---|---|
| GIP-001 | A | D^{∗} |
| GIP-002 | A | D^{∗} |
| GIP-028 | B | D^{∗} |
| GIP-065 | A | D^{∗} |
| GIP-067 | A | D^{∗} |
| GIP-071 | A | D^{∗} |

| | | |
|---|---|---|
| ^{∗}Not stimulatory in a wide concentration range between 0.1 pM and 10 µM. Full antagonist. | | |

## Claims

1. A compound comprising a cyclic peptide of formula (Ia) 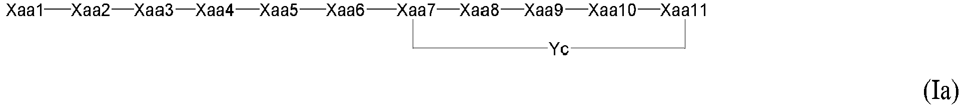 or a cyclic peptide of formula (Ib) 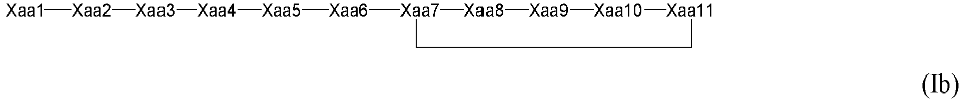
optionally an N-terminal modification group A attached to Xaa1, and
optionally a C-terminal group C-term attached to Xaa11,
wherein
the peptide sequence is drawn from left to right in N- to C-terminal direction,
Xaa1 is a residue of an α-amino acid with an acidic or polar group within the side chain or an aliphatic carboxylic acid with an additional acidic or polar group at the ω-position, preferably Xaa1 is a residue of an amino acid of formula (IIa), (IIb) or (IIc), more preferably Xaa1 is a residue of an amino acid formula (IIa), 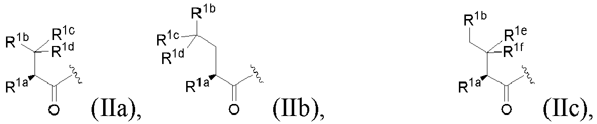 wherein
R^{1a} is selected from the group consisting of NH, H, NH(C1-C4)alkyl, OH, and (C1-C4)alkyl, wherein if R^{1a} is selected from the group consisting of NH and NH(C1-C4)alkyl, the N-terminal modification group is covalently attached to the nitrogen atom of R^{1a}, and if R^{1a} is selected from the group consisting of H, OH or (C1-C4)alkyl the N-terminal modification group is absent,
R^{1b} is selected from the group consisting of CO₂H, SO₃H, OPO₃H₂, CONH₂ and OH,
R^{1c} is selected from the group consisting of H, OH and (C1-C2)alkyl under the proviso that R^{1b} is selected from the group consisting of CO₂H, SO₃H and CONH₂, or is selected from the group consisting of H and (C1-C2)alkyl under the proviso that R^{1b} is selected from the group consisting of OH and OPO₃H₂,
R^{1d} is selected from the group consisting of H and (C1-C2)alkyl; preferably if R^{1c} is (C1-C2)alkyl, R^{1d} is the same (C1-C2)alkyl,
R^{1e} is selected from the group consisting of H, OH and (C1-C2)alkyl,
R^{1f} is selected from the group consisting of H and (C1-C2)alkyl; preferably if R^{1e} is (C1-C2)alkyl, R^{1f} is the same (C1-C2)alkyl,
the carbonyl group of Xaa1 is covalently attached to the α-nitrogen atom of Xaa2,
Xaa2 is a residue of an α-amino acid with a substituted or unsubstituted bicyclic aromatic or heteroaromatic side chain, preferably Xaa2 is a residue of an amino acid of formula (IIIa) or (IIIb), more preferably a residue of an amino acid of formula (IIIa), 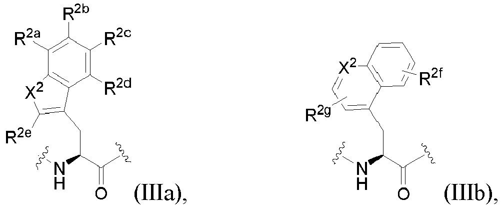 wherein
X² is selected from the group consisting of NH, NCH₃ and S; preferably selected from the group consisting of NH and S, if Xaa2 is an amino acid of formula (IIIa), or is selected from the group consisting of N, CH, CF and C-CH₃; preferably selected from the group consisting of N and CH, if Xaa2 is an amino acid of formula (IIIb),
R^{2a} is selected from the group consisting of H, F, Cl, Br, CH₃ and OCH₃, preferably R^{2a} is selected from the group consisting of H and F,
R^{2b} is selected from the group consisting of H, a halogen, CH₃ and OCH₃, preferably the halogen is selected from the group consisting of Cl and Br, more preferably R^{2b} is selected from the group consisting of H, Cl and Br,
R^{2c} is selected from the group consisting of H, OH, a halogen, CH₃ and OCH₃, preferably the halogen is selected from the group consisting of F, Cl and Br, more preferably R^{2c} is selected from the group consisting of H, F and OH,
R^{2d} is selected from the group consisting of H, a halogen, CH₃ and OCH₃, wherein the halogen is selected from the group consisting of F, Cl and Br, preferably R^{2d} is H,
R^{2e} is selected from the group consisting of H, a halogen and CH₃, preferably the halogen is Cl, more preferably R^{2e} is H,
R^{2f} is selected from the group consisting of H and OCH₃, preferably R^{2f} is H,
R^{2g} is selected from the group consisting of H, OH, CH₃ and OCH₃, preferably R^{2g} is H,
or
Xaa2 is a residue of an α-amino acid with a substituted or unsubstituted aromatic or heteroaromatic side chain, wherein Xaa2 is preferably a residue of an amino acid of formula (IIIc) 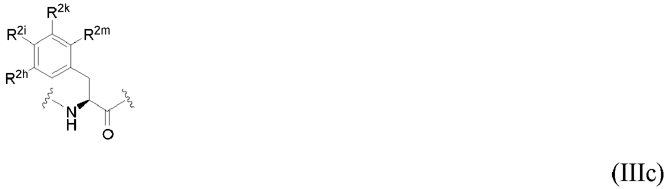 wherein
R^{2h} is selected from the group consisting of H, CH₃, CF₃, OH, OCH₃, OCF₃, CONH₂ and a halogen, preferably the halogen is selected from the group consisting of Cl and Br, more preferably R^{2h} is H, Cl or Br,
R^{2 i} is selected from the group consisting of H, CH₃, C(CH₃)₃, CF₃, OH, OCH₃, OCH₂CH₃, OCF₃, CONH₂, CO₂H and a halogen, preferably the halogen is selected from the group consisting of Cl and Br, more preferably R²ⁱ is selected from the group consisting of H, Cl and Br,
R^{2k} is selected from the group consisting of H, CH₃, CF₃, OH, OCH₃, OCF₃, CONH₂ and a halogen, preferably the halogen is selected from the group consisting of Cl and Br, more preferably R^{2k} is selected from the group consisting of H, Cl and Br,
R^{2m} is selected from the group consisting of H, CH₃, CF₃, OH, OCH₃, OCF₃, CONH₂ and a halogen, preferably the halogen is selected from the group consisting of Cl and Br, more preferably R^{2m} is selected from the group consisting of H, Cl and Br,
the carbonyl group of Xaa2 is covalently attached to the α-nitrogen atom of Xaa3,
Xaa3 is an α-amino acid with a substituted or unsubstituted bicyclic aromatic or heteroaromatic side chain, preferably Xaa3 is a residue of an amino acid of formula (IVa) or (IVb), more preferably Xaa3 is a residue of an amino acid of formula (IVa), 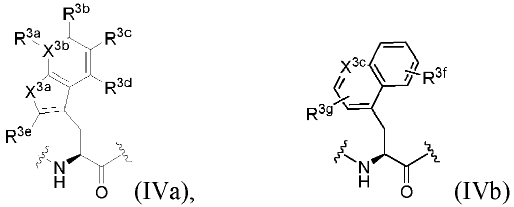 wherein
X^{3a} is selected from the group consisting of NH, NCH₃ and S, preferably selected from the group consisting of NH and S,
X^{3b} is selected from the group consisting of C and N, preferably X^{3b} is C,
X^{3c} is selected from the group consisting of C and N, preferably X^{3c} is C,
R^{3a} is, under the proviso that X^{3b} is C, selected from the group consisting of H, a halogen, CH₃ and OCH₃, preferably the halogen is F, or is absent if X^{3b} is N,
R^{3b} is selected from the group consisting of H, a halogen, CH₃ and OCH₃, preferably the halogen is selected from the group consisting of F and Cl, more preferably R^{3b} is selected from the group consisting of H and F,
R^{3c} is selected from the group consisting of H, a halogen, CH₃ and OCH₃, preferably the halogen is selected from the group consisting of Cl, Br and F, more preferably R^{3C} is selected from the group consisting of Cl and Br,
R^{3d} is selected from the group consisting of H, a halogen, CH₃ and OCH₃, preferably the halogen is F,
R^{3e} is selected from the group consisting of H, a halogen and CH₃, preferably the halogen is Cl,
R^{3f} is selected from the group consisting of H and OCH₃,
R^{3g} is selected from the group consisting of H, F, OH, CH₃ and OCH₃,
or
Xaa3 is a residue of an α-amino acid with a substituted or unsubstituted aromatic or heteroaromatic side chain, preferably Xaa3 is a residue of an amino acid of formula (IVc) 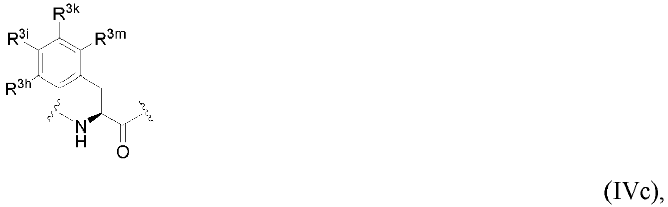 wherein
R^{3h} is selected from the group consisting of H, a halogen, OH, CH₃, CF₃, OCH₃, OCF₃ and CONH₂, preferably the halogen is selected from the group consisting of Cl and Br,
R³¹ is selected from the group consisting of H, a halogen, OH, CH₃, CF₃, OCH₃, OCF₃ and CONH₂, preferably the halogen is selected from the group consisting of Cl and Br,
R^{3k} is selected from the group consisting of H, a halogen, OH, CH₃, CF₃, OCH₃, OCF₃ and CONH₂, preferably the halogen is selected from the group consisting of Cl and Br, and
R^{3m} is selected from the group consisting of H, a halogen, OH, CH₃, CF₃, OCH₃, OCF₃ and CONH₂, preferably the halogen is selected from the group consisting of Cl and Br, preferably R^{3m} is selected from the group consisting of H and Cl,
the carbonyl group of Xaa3 is covalently attached to the α-nitrogen atom of Xaa4,
Xaa4 is a residue of a cyclic non-aromatic amino acid, preferably Xaa4 is a residue of an amino acid of formula (Va) 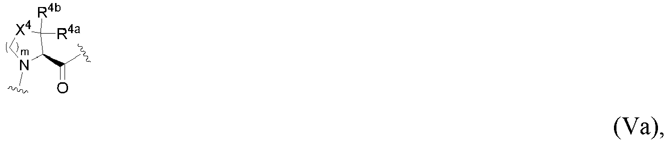 wherein
R^{4a} is selected from the group consisting of H, OH, CH₃ and CF₃
R^{4b} is selected from the group consisting of H and CH₃, preferably R^{4b} is CH₃ if R^{4a} is CH₃,
X⁴ is selected from the group consisting of CHR^{4c}, CH₂, S and O,
wherein R^{4c} is absent or is selected from the group consisting of NH₂, OH, H, NHR^{4d}, CH₃ and F,
wherein R^{4d} is absent or is selected from the group consisting of H, Ac, a Z group, wherein the Z group comprises a bio-distribution modifier with an optional linker, a chelator with an optional linker or a combination thereof, preferably the chelator is DOTA, the bio-distribution modifier is AGLU comprising the linker Glutar
m is 1 or 2, preferably m is 1
or
Xaa4 is a residue of an N-alkylated amino acid, preferably Xaa4 is a residue of an amino acid of formula (Vb) 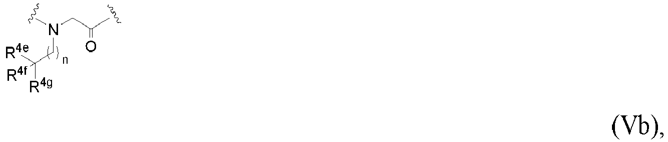 wherein
n is 0, 1, 2, 3, 4, or 5, preferably n is 0, 1 or 3,
R^{4e} is, if n is 0, selected from the group consisting of H, CH₃, a substituted or unsubstituted aromatic residue, COOH, CONH₂ and C(=O)R^{4h}, or is, if n is 1, 2, 3, 4 or 5, selected from the group consisting of H, CH₃, a substituted or unsubstituted aromatic residue, OH, NH₂, COOH, CONH₂ and C(=O)R^{4h},
R^{4f} is selected from the group consisting of H, CH₃ and CH₂CH₃,
R^{4g} is selected from the group consisting of H and CH₃,
R^{4h} is AGLU,
or
Xaa4 is a residue of a bicyclic non-aromatic amino acid, preferably Xaa4 is a residue of an amino acid of formula (Vc) 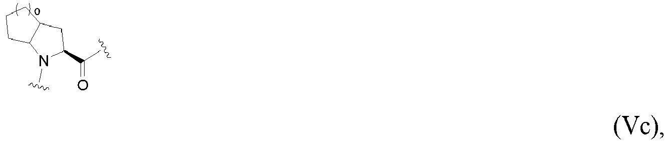 wherein
o is 1 or 2,
the carbonyl group of Xaa4 is covalently attached to the α-nitrogen atom of Xaa5,
Xaa5 a residue of an α-amino acid with a polar, preferably an acidic group within the side chain, preferably Xaa5 is a residue of an amino acid of formula (VIa), (VIb) or (VIc), more preferably Xaa5 is a residue of an amino acid formula (VIa) or (VIb), 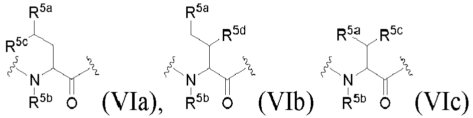 wherein
R^{5a} is selected from the group consisting of COOH, CONH₂, OPO₃H₂, OH and SO₃H,
R^{5b} is selected from the group consisting of H and (C1-C4)alkyl,
R^{5c} is selected from the group consisting of H, OH and CH₃ under the proviso that R^{5A} is selected from the group consisting of COOH, CONH₂, OPO₃H₂ and SO₃H, or is selected from the group consisting of H and CH₃ under the proviso that R^{5a} is selected from the group consisting of COOH, CONH₂, OPO₃H₂, OH and SO₃H,
R^{5d} is selected from the group consisting of H, OH and CH₃
the carbonyl group of Xaa5 is covalently attached to the α-nitrogen atom of Xaa6,
Xaa6 is a residue of an α-amino acid with a branched aliphatic side chain, preferably Xaa6 is a residue of an amino acid of formula (VIIa) 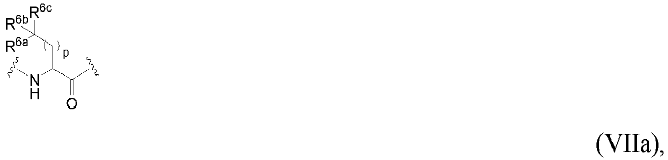 wherein
p is 1, 0 or 2, preferably p is 1,
R^{6a} is selected from the group consisting of CH₃ and CH₂CH₃,
R^{6b} is CH₃,
R^{6c} is H, if p is 0, and is selected from the group consisting of H and CH₃, if p is 1 or 2,
the carbonyl group of Xaa6 is covalently attached to the α-nitrogen atom of Xaa7,
Xaa7 is a residue of an amino acid of formula (VIIIa) 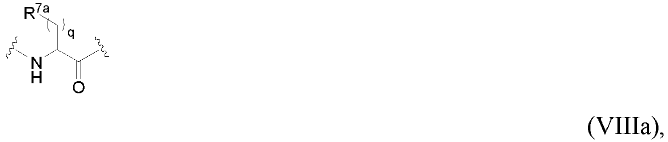 wherein
R^{7a} is selected from the group consisting of SH and NH₂,
q is 1 or 2, preferably q is 1,
Xaa8 is a residue of an aliphatic cyclic α-amino acid, of an aliphatic heterocyclic α-amino acid or of an aliphatic cyclic α-amino acid with an annulated aromatic ring, preferably Xaa8 is a residue of an amino acid of formula (IXa) or (IXb), more preferably Xaa8 is a residue of an amino acid of formula (IXa), 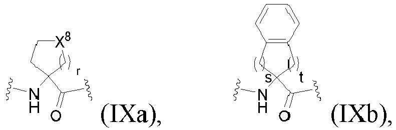 wherein
X⁸ is selected from the group consisting of NR^{8a}, O, NH, and CH₂, wherein R^{8a} is a Z group, wherein the Z group comprises a bio-distribution modifier with an optional linker, a chelator with an optional linker or a combination thereof, preferably the bio-distribution modifier is AGLU and the linker is Glutar, the chelator is DOTA, or if AGLU and DOTA are combined the linker is glu, or R^{8a} is acetyl,
r is 2 or 1, preferably r is 2,
s is 1 or 2, preferably s is 1,
t is 1 or 2, preferably t is 1 if s is 1, and t is 2 if s is 2,
or
Xaa8 is a residue of an α-amino acid or an α-alkyl-α-amino acid, wherein preferably Xaa8 is a residue of an amino acid of formula (IXc), 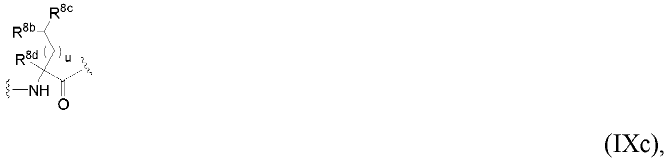 wherein
R^{8b} is selected from the group consisting of H, OH, NH₂, NHR^{8e}, CH₃, CH₂CH₃, COOH, CONH₂, a substituted aromatic ring, an unsubstituted aromatic ring, a substituted bicyclic aromatic ring system, a substituted bicyclic hetero aromatic ring system, an unsubstituted bicyclic aromatic ring system and an unsubstituted bicyclic hetero aromatic ring system, preferably the aromatic ring is phenyl and the bicycylic heteroaromatic ring system is 3-indol, wherein R^{8e} is a Z group, wherein the Z group comprises a bio-distribution modifier with an optional linker, a chelator with an optional linker or a combination thereof,
R^{8c} is selected from the group consisting of H and CH₃, if R^{8b} is selected from the group consisting of H, OH, NH₂, CH₃, CH₂CH₃, COOH, CONH₂, a substituted aromatic ring, an unsubstituted aromatic ring, a substituted bicyclic aromatic system, a substituted bicyclic hetero aromatic ring system, an unsubstituted bicyclic aromatic ring system and an unsubstituted bicyclic hetero aromatic ring system, or is selected from the group consisting of H, CH₃ and OH, if R^{8b} is selected from the group consisting of H, CH₃, CH₂CH₃, COOH, CONH₂, a substituted aromatic ring, an unsubstituted aromatic ring, a substituted bicyclic aromatic ring system, a substituted bicyclic hetero aromatic ring system, an unsubstituted bicyclic aromatic ring system and an unsubstituted bicyclic hetero aromatic ring system,
R^{8d} is selected from the group consisting of H, CH₃ and CH₂CH₃,
u is 0, 1, 2, 3, 4, or 5, preferably u is 0, 1 or 3,
the carbonyl group of Xaa8 is covalently attached to the α-nitrogen atom of Xaa9,
Xaa9 is a residue of an α-amino acid, preferably Xaa9 is a residue of an amino acid of formula (Xa) 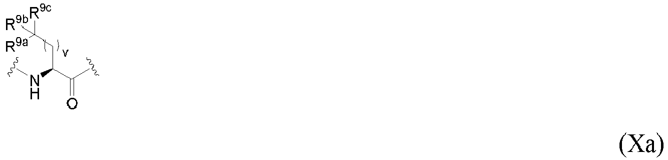 wherein
R^{9a} is selected from the group consisting of CH₃, H, CH₂CH₃, OH, NH₂, NHC(=NH)NH₂, cyclo-hexyl, a substituted aromatic ring, an unsubstituted aromatic ring, a substituted bicyclic aromatic ring system, a substituted bicyclic hetero aromatic ring system, an unsubstituted bicyclic aromatic ring system and an unsubstituted bicyclic hetero aromatic ring system, preferably the aromatic ring is phenyl and the heteroaromatic ring system is 3-indol,
R^{9b} is selected from the group consisting of CH₃ and H,
R^{9c} is selected from the group consisting of H and CH₃,
v is 0, 1 or 2, preferably v is 1,
the carbonyl group of Xaa9 is covalently attached to the α-nitrogen atom of Xaa10,
Xaa10 is a residue of an α-amino acid, preferably Xaa10 is a residue of an amino acid of formula (XIa) 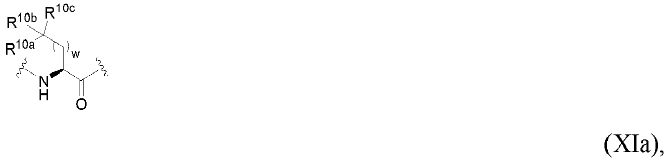 wherein
R^{10a} is selected from the group consisting of CH₂CH₃, CH₃, phenyl, cyclohexyl and OH,
R^{10b} is selected from the group consisting of CH₃ and H,
R^{10c} is selected from the group consisting of H and CH₃,
w is 0 or 1,
the carbonyl group of XaalO is covalently attached to the nitrogen of Xaa11,
Xaa11 is a residue of an α-amino acid with a nucleophilic group within the side chain or a residue of an aliphatic bis-nucleophile, preferably Xaa11 is a residue of an amino acid of formula (XIIa) 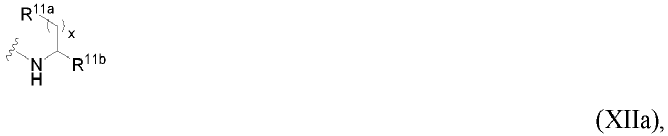 wherein
R^{11a} is selected from the group consisting of SH and NH₂,
R^{11b} is selected from the group consisting of C(=O)R, CONH₂, CO₂H, CH₂OH and H, wherein R is C-term, NH₂ or OH,
x is 1 or 2,
or
Xaa11 is a residue of an amino acid of the formula (XIIb) 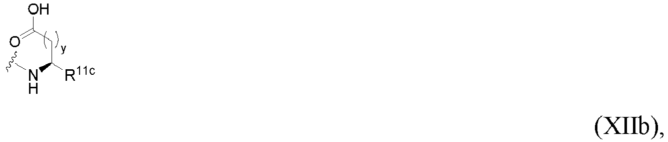 wherein
R^{11c} is selected from the group consisting of H and CH₂CH₂CONH₂,
y is 0 or 1
wherein
if R^{1a} in Xaa1 is selected from the group consisting of NH and NH(C1-C4)alkyl, the N-terminal modification group A
(a) is a Z group or a bio-distribution modifier optionally comprising a linker, wherein the Z group comprises a chelator and optionally a linker,
(b) is a residue of an amino acid Aaa, wherein an optional linker is interspersed between Aaa and Xaa1,
wherein the amino acid Aaa is optionally substituted by a Z group, and wherein said Z group comprises a chelator and optionally a linker,
wherein if Aaa is glu, Aaa is optionally substituted by a bio-distribution modifier,
or
(c) is selected from the group consisting of acetyl, a blocking group Abl of a structure of formula (Abl-Ia) or of a structure of formula (Abl-Ib) 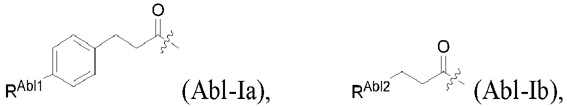 wherein
R^{Abl1} is selected from the group consisting of H, OH and a halogen, preferably the halogen is selected from the group consisting of F and Cl, preferably R^{Abl1} is OH
R^{Abl2} is selected from the group consisting of 1-naphtyl and 2-naphthyl,
preferably Abl is of formula (Abl-Ia),
and wherein, if present, the N-terminal modification group A is bound to the α-N atom of R^{1a} in Xaa1,
wherein
if Xaa11 is a residue of an amino acid of formula (XIIa), R^{11b} is C(=O)R, and R is C-term,
the C-terminal group C-term
(a) is one of more amino acids AA,
wherein at least one or more of the one or more amino acids AA is optionally substituted by a Z group and/or a bio-distribution modifier optionally comprising a linker, wherein said Z group comprises a chelator and optionally a linker,
wherein an optional linker is interspersed between Xaa11 and the first of the one or more amino acids AA,
wherein an optional linker is interspersed between any of the more than one amino acids AA,
wherein to the last of the one or more amino acids a blocking group Abl is attached, wherein Abl is selected from the group consisting of NH₂ and OH, wherein Abl preferably is NH₂, wherein if AA is glu, AA is optionally substituted by a bio-distribution modifier,
wherein if AA is Lys, AA is optionally substituted by a chelator,
(b) is a Z group,
wherein the Z group comprises a chelator and a linker,
(c) is a bio-distribution modifier optionally comprising a linker,
or
(d) is a blocking group Abl, wherein
Abl is OH or NH₂,
preferably Abl is NH₂,
and wherein, if present, the C-terminal group C-term is bound to the C-atom of R^{11b} in Xaa11,
wherein
(a) if the cyclic peptide is of formula (Ia),
under the proviso that Xaa11 is a residue of formula (XIIa), Yc is a structure of formula (XIIIa) or a structure of formula (XIIIb), more preferably Yc is a structure of formula (XIIIa) 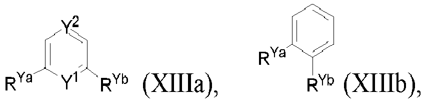
wherein
R^{Ya} and R^{Yb} are each and independently -CH₂-,
Y¹ is selected from the group consisting of N and CH,
Y² is selected from the group consisting of N and C-R^{Yc},
R^{Yc} is selected from the group consisting of H and -CH₂-R^{Yd} and
R^{Yd} is a structure of formula (XIIIc), (XIIId) or (XIIIe), 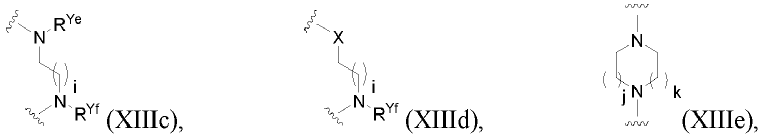
wherein
R^{Ye} and R^{Yf} are each and independently selected from the group consisting of H and (C1-C4)alkyl
i is 1, 2, 3, 4, 5 or 6, preferably i is 1 or 2,
j and k are each and independently 1, 2 or 3, and
X is O or S, preferably X is S,
wherein
in formulae (XIIIc) and (XIIIe) one of the two nitrogen atoms is attached to -CH₂- of R^{Yc}
and in formula (XIIId) -X- is attached to -CH₂- of R^{Yc}
while the remaining nitrogen atom optionally connects to a Z group comprising a chelator and optionally a linker,
preferably, if Yc is a structure of formulae (XIIIa), wherein R^{Yc} is -CH₂-R^{Yd}, R^{Yd} is a structure of formulae (XIIId), wherein X is S and R^{Yf} is H,
and wherein
one of R^{Ya} and R^{Yb}
is linked to the S-atom of Xaa7 under the formation of a thioether, if R^{7a} in Xaa7 is SH, or,
is linked to the N-atom of Xaa7 under the formation of an amine linkage, if R^{7a} in Xaa7 is NH₂,
and the other one of R^{YA} and R^{Yb}
is linked to the S-atom of Xaa11 under the formation of a thioether if R^{11a} in Xaa11 is SH, or,
is linked to the N-atom of Xaa11 under the formation of an amine linkage if R^{11a}in Xaa11 is NH₂,
and the resulting generic structural formula corresponds to formula (Ic), 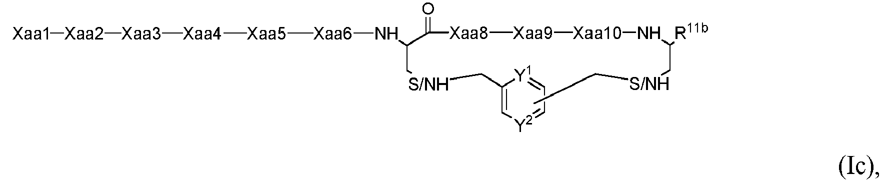
or wherein
(b) if the cyclic peptide is of formula (Ia),
under the proviso that
R^{7a} in Xaa7 is NH₂ and Xaa11 is a residue of formula (XIIa) wherein R^{11a} is SH or NH²,
or
Xaa11 is a residue of formula (XIIa) wherein R₁₁ₐ is NH₂ and R^{7a} in Xaa7 is NH₂ or SH,
Yc is a structure of formula (XIIIf), 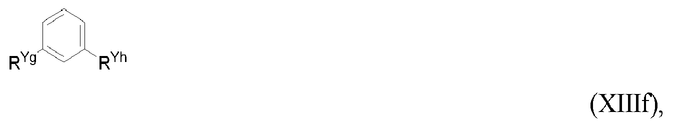
wherein
R^{Yg} is -C(=O)- and
R^{Yh} is -CH₂-,
and wherein
if R^{7a} in Xaa7 is NH₂ and Xaa11 is a residue of formula (XIIa) wherein R^{11a} is SH or NH₂,
R^{Yg} is linked to the N-atom of R^{7a} in Xaa7 under the formation of an amide linkage
and
if R^{11a} in Xaa11 is SH, R^{Yh} is linked to said S-atom under the formation of a thioether linkage,
or
if R^{11a} in Xaa11 is NH₂, R^{Yh} is linked to said N-atom under the formation of an amine linkage
or wherein
if Xaa11 is a residue of formula (XIIa), wherein R^{11a} is NH₂ and R^{7a} in Xaa7 is either SH or NH₂,
R^{Yg} is linked to the N-atom of R^{11a} in Xaa11 under the formation of an amide linkage
and
if R^{7a} in Xaa7 is SH, R^{Yh} is linked to said S-atom under the formation of a thioether linkage,
or
if R^{7a} in Xaa7 is NH₂, R^{Yh} is linked to said N-atom under the formation of an amine linkage,
and the resulting generic structural formula corresponds to formula (Id) or formula (Ie), 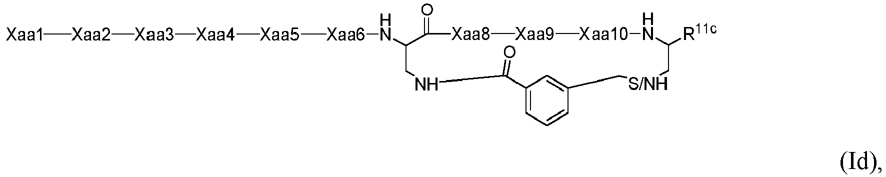 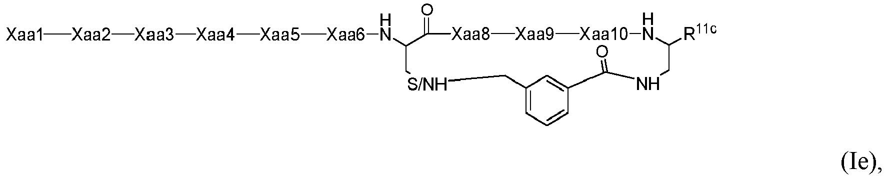
or wherein
(c) if the cyclic peptide is of formula (Ia),
if R^{7a} in Xaa7 is SH and Xaa11 is a residue of formula (XIIa) wherein R^{11a} is SH,
Yc is a structure of formula (XIIIg), 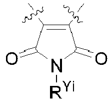 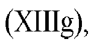
wherein
R^{Yi} is selected from the group consisting of H and (C1-C6)alkyl and wherein the S-atom of Xaa7 is linked to Yc by a thioether linkage and the S-atom of Xaa11 is linked to Yc by a thioether linkage forming a cyclic structure with a generic formula corresponding to formula (If) 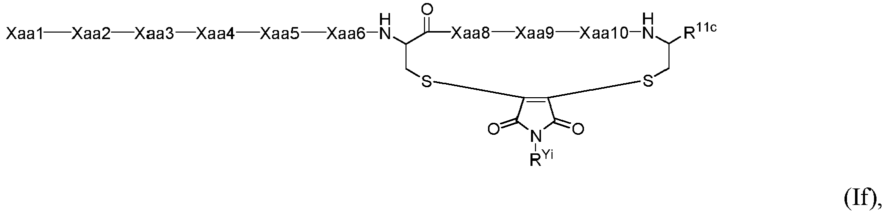
or wherein
(d) if the cyclic peptide is of formula (Ia),
if R^{7a} in Xaa7 is NH₂ and Xaa11 is a residue of an amino acid of the formula (XIIb),
Yc is a structure of formula (XIIIh), 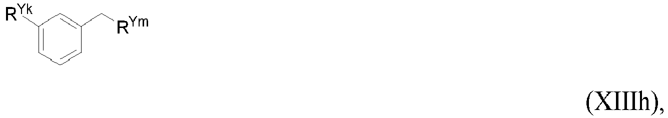 wherein,
R^{Yk} is -C(=O)- and
R^{Ym} is -NH-,
wherein
R^{Yk} is linked to the N-atom of R^{7a} in Xaa7 under the formation of an amide bond and R^{Ym} is linked to the carboxylic acid group of Xaa11 under the formation of an amide bond,
and wherein
the resulting generic structural formula corresponds to formula (Ig), 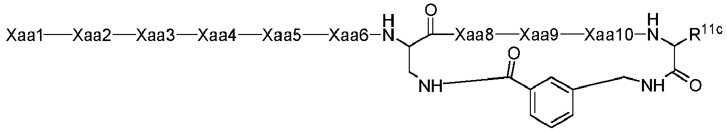 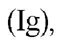
and wherein
if the cyclic peptide is of formula (Ib), the S atom of Xaa7 and the S atom of Xaa11 are covalently attached to each other forming a disulfide linkage.

2. The compound of claim 1, wherein Xaa1 is a residue of an α-amino acid with an acidic or polar within the side chain or an aliphatic carboxylic acid with an additional acidic or polar group at the ω-position, of formula (IId) or (IIe);
preferably Xaa1 is a residue of an amino acid of formula (IId), 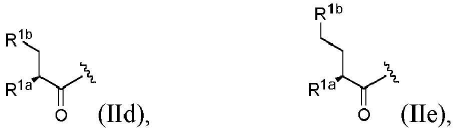 wherein
R^{1a} is NH,
R^{1b} is CO₂H or CONH₂,
the N-terminal modification group is covalently attached to the α-nitrogen atom of Xaa1,
the carbonyl group of Xaa1 is covalently attached to the α-nitrogenatom of Xaa2;
more preferably Xaa1 is an L-amino acid residue selected from the group consisting of Asp, Asn, Glu, and Gln, more preferably Xaa1 is an Asp residue.

3. The compound of any one of claims 1 to 2, wherein Xaa2 is a residue of an amino acid of formula (IIId), 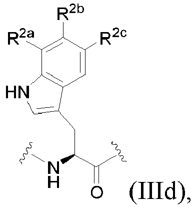 wherein
R^{2a} is H or F,
R^{2b} is H or Cl,
R^{2c} is selected from the group consisting of H, F and OH,
the carbonyl group of Xaa2 is covalently attached to the α-nitrogenatom of Xaa3;
preferably Xaa2 is an L-amino acid residue selected from the group consisting of Trp, Hyw, 5Fw, 6Clw, 7Fw, Bta, 5Clw, 5Brw and 6Fw;
more preferably Xaa2 is a Trp residue.

4. The compound of any one of claims 1 to 3, wherein Xaa3 is a residue of an amino acid of formula (IVd) or (IVe), 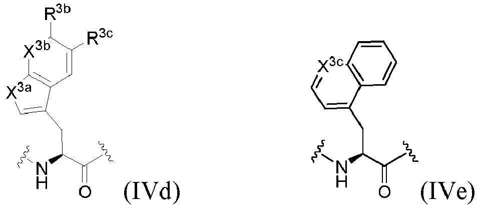 wherein
R^{3b} is H or F,
R^{3c} is selected from the group consisting of Cl, Br, H and F, and
x^{3a} is NH or S,
X^{3b} is selected from the group consisting of CH, CF and N,
X^{3c} is CH or N, preferably X^{3c} is CH,
the carbonyl group of Xaa3 is covalently attached to the α-nitrogenatom of Xaa4;
preferably Xaa3 is an L-amino acid residue selected from the group consisting of 5Clw, 5Brw, Trp, INi, Bta, 5Fw, 6Fw and 7Fw;
more preferably Xaa3 is a 5Clw residue.

5. The compound of any one of claims 1 to 4, wherein Xaa4 is a residue of an amino acid of formula (Ve) 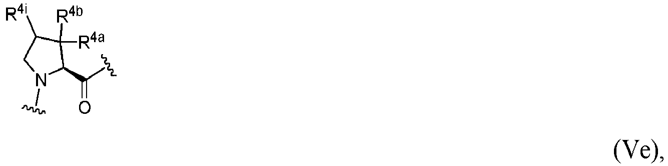 wherein
R^{4a} is selected from the group consisting of H, OH and CH₃,
R^{4b} is H or CH₃, preferably R^{4b} is CH₃ if R^{4a} is CH₃,
R⁴¹ is selected from the group consisting of NH₂, OH, NHR^{4d}, H and F, wherein R^{4d} is H or Ac, or R^{4d} is a chelator, preferably the chelator is DOTA, optionally comprising a linker or R^{4d} is a bio-distribution modifier with an optional linker, preferably the bio-distribution modifier is AGLU with Glutar as linker,
the carbonyl group of Xaa4 is covalently attached to the α-nitrogenatom of Xaa5;
preferably Xaa4 is a residue of an amino acid of formula (Vf) 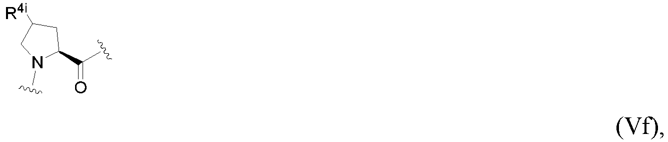 wherein
R⁴¹ is selected from the group consisting of NH₂, OH and H,
the carbonyl group of Xaa4 is covalently attached to the α-nitrogen atom of Xaa5;
more preferably Xaa4 is a Hyp residue or a Tap residue.

6. The compound of any one of claims 1 to 5, wherein Xaa5 is a residue of an amino acid of formula (VId) or (VIe);
preferably Xaa5 is a residue of an amino acid of formula (IVd), 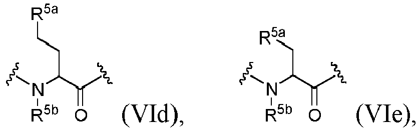 wherein
R^{5a} is selected from the group consisting of COOH, CONH₂, OPO₃H₂, OH and SO₃H,
R^{5b} is H or CH₃,
the carbonyl group of Xaa5 is covalently attached to the α-nitrogen atom of Xaa6;
more preferably Xaa5 is an amino acid residue selected from the group consisting of Glu, Gln, glu, Nme, Asp, Asn, Pse, Hse and Hca;
most preferably Xaa5 is a Glu residue.

7. The compound of any one of claims 1 to 6, wherein Xaa6 is a residue of an amino acid of formula (VIIa) 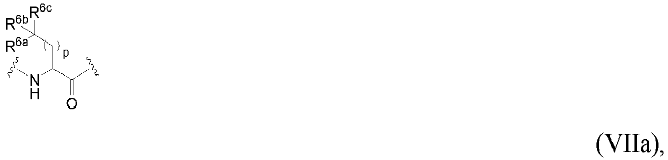 wherein
p is 1 or 0, preferably p is 1,
R^{6a} is CH₃ or CH₂CH₃,
R^{6b} is CH₃,
R^{6c} is H if p is 0, and is H or CH₃, if p is 1,
the carbonyl group of Xaa6 is covalently attached to the α-nitrogen atom of Xaa7;
preferably Xaa6 is an amino acid residue selected from the group consisting of leu, Leu, Npg, Val, Ile and Hle;
more preferably Xaa6 is a leu residue.

8. The compound of any one of claims 1 to 7, wherein Xaa7 is an L-amino acid residue selected from the group of Cys, cys, Hcy and Dap; preferably Xaa7 is a Cys residue.

9. The compound of any one of claims 1 to 8, wherein Xaa8 is a residue of an amino acid of formula (IXa), 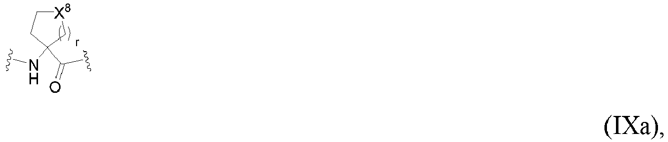 wherein
r is 2 or 1, preferably r is 2,
X⁸ is selected from the group consisting of CH₂, O, NH and NR^{8a}, wherein R^{8a} is a Z group, wherein the Z group comprises a bio-distribution modifier with an optional linker, a chelator with an optional linker or a combination thereof, preferably the bio-distribution modifier is AGLU and the linker is Glutar, the chelator is DOTA, or if AGLU and DOTA are combined the linker is glu, or R^{8a} is acetyl,
the carbonyl group of Xaa8 is covalently attached to the α-nitrogen atom of Xaa9;
preferably X⁸ is NR^{8a} or O,
wherein R^{8a} is a Z group, wherein the Z group comprises a bio-distribution modifier with an optional linker, a chelator with an optional linker or a combination thereof, preferably the bio-distribution modifier is AGLU and the linker is Glutar, the chelator is DOTA, or if AGLU and DOTA are combined the linker is glu, or R^{8a} is acetyl,
the carbonyl group of Xaa8 is covalently attached to the α-nitrogen atom of Xaa9; more preferably Xaa8 is an Apc(R^{8a}) residue, wherein R^{8a} is a Z group, wherein the Z group comprises a chelator with an optional linker, preferably the chelator is DOTA.

10. The compound of any one of claims 1 to 9, wherein Xaa9 is a residue of an amino acid of formula (Xa) 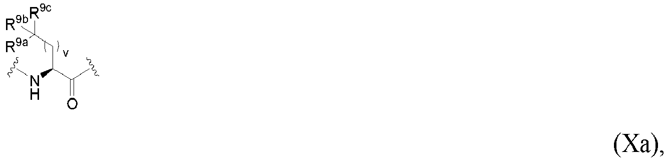 wherein
R^{9a} is selected from the group consisting of CH₃, CH₂CH₃, OH, NH(=NH)NH₂, CO₂H, phenyl and 3-indol,
R^{9b} is CH₃ or H,
R^{9c} is H or CH₃, and
v is 0, 1, 2, preferably v is 1,
the carbonyl group of Xaa9 is covalently attached to the α-nitrogen atom of Xaa10;
preferably Xaa9 is an L-amino acid residue selected from the group consisting of Leu, Hle, Ile, Arg, Trp, Glu, Phe, Ser, Cha, Npg, Tie and Ala;
more preferably Xaa9 is a Leu residue.

11. The compound of any one of claims 1 to 10, wherein Xaa10 is a residue of an amino acid of formula (XVI) 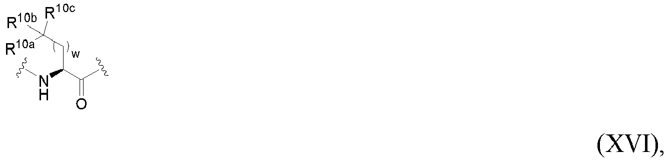 wherein
R^{10a} is CH₂CH₃ or CH₃
R^{10b} is CH₃ or H,
R^{10c} is H or CH₃,
w is 0 or 1,
the carbonyl group of XaalO is covalently attached to the α-nitrogen atom of Xaa11;
preferably Xaa10 is an L-amino acid residue selected from the group consisting of Ile, Leu, Tle, Val, Phe, Cha and Ser;
more preferably Xaa10 is an Ile residue.

12. The compound of any one of claims 1 to 11, wherein Xaa11 is a residue of an amino acid with a nucleophilic group within the side chain or a residue of an aliphatic bis-nucleophile, preferably Xaa11 is a residue of an amino acid of formula (XIIc) 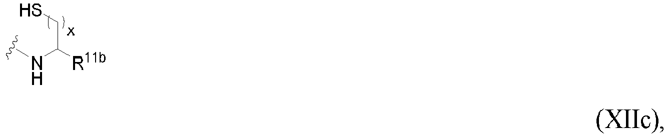 wherein
R^{11b} is selected from the group consisting of C(=O)R, CONH₂ and H, wherein R is NH₂ or C-term,
x is 1 or 2, preferably 1;
preferably Xaa11 is a residue selected from the group consisting of Cys, cys, Hcy, AET, en and Cysol;
more preferably Xaa11 is a Cys residue.

13. The compound of any one of claims 1 to 12, wherein if R^{1a} in Xaa1 is selected from the group consisting of NH and NHCH₃, the N-terminal modification group A is a Z group or a bio-distribution modifier optionally comprising a linker, wherein the Z group comprises a chelator and optionally a linker, preferably the bio-distribution modifier is AGLU in combination with Glutar as linker, the chelator is DOTA, the optional linker is selected from the group consisting of Ttds and APAc;
preferably, if in Xaa1 R^{1a} is NH, the N-terminal modification group A is a bio-distribution modifier optionally comprising a linker;
more preferably the bio-distribution modifier is AGLU in combination with Glutar as linker.

14. The compound of any of claims 1 to 13, wherein, if Xaa11 is a residue of an amino acid of formula (XIIa) and R^{11b} is C(=O)R, whereby R is C-term,
the C-terminal group C-term
is one amino acid AA,
wherein the amino acid AA is substituted with a bio-distribution modifier optionally comprising a linker,
wherein an optional linker is interspersed between Xaa11 and the amino acid AA,
wherein to the amino acid AA NH₂ is attached as a blocking group Abl;
preferably
the amino acid is glu with AGLU as bio-distribution modifier attached to the side chain of said glu, and
the linker interspersed between Xaa11 and the amino acid is APAc.

15. The compound of any one of claims 1 to 14, wherein the cyclic peptide is a cyclic peptide of formula (Ia), wherein Yc is a structure of formula (XIIIa) 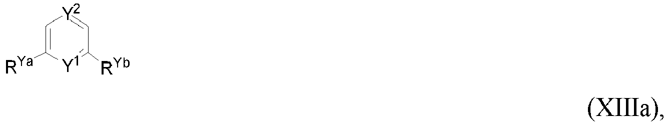 wherein
R^{Ya} and R^{Yb} are each and independently -CH₂-,
Y¹ is N or CH,
Y² is CH or N;
preferably the cyclic peptide is a cyclic peptide of formula (Ia), wherein Yc is selected from the group consisting of 2Lut, 3MeBn, 3Lut, tMeBn(DOTA-AET) and tMeBn(DOTA-PP);
more preferably Yc is 2Lut.

16. The compound of any one of claims 1 to 15, wherein the compound is selected from the group consisting of
compound AGLU-Glutar-Asp-Trp-5Clw-Tap-Glu-leu-[Cys(2Lut)-Apc(DOTA)-Leu-Ile-Cys]-APAc-glu(AGLU)-NH₂ (GIP-001) of the following formula 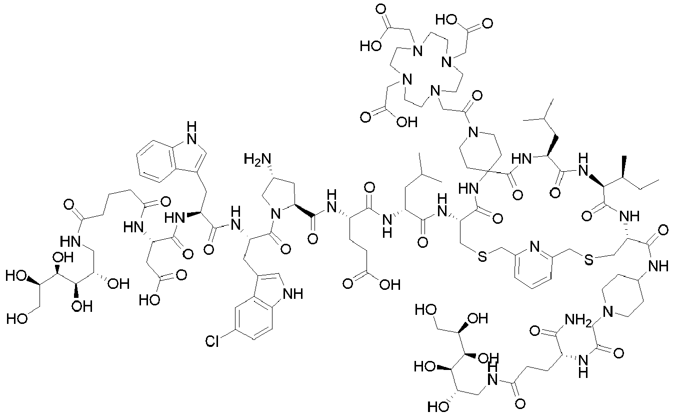 and
compound AGLU-Glutar-Asp-Trp-5Clw-Hyp-Glu-leu-[Cys(2Lut)-Apc(DOTA)-Leu-Ile-Cys]-APAc-glu(AGLU)-NH₂ (GIP-002) of the following formula
